(19) **Europäisches Patentamt / European Patent Office / Office européen des brevets**

(11) **EP 1 649 062 B1**

(12) # EUROPEAN PATENT SPECIFICATION

(45) Date of publication and mention of the grant of the patent:
**09.12.2009 Bulletin 2009/50**

(21) Application number: **04777581.2**

(22) Date of filing: **02.07.2004**

(51) Int Cl.:
***C12Q 1/68*** (2006.01)

(86) International application number:
**PCT/US2004/021543**

(87) International publication number:
**WO 2005/007892 (27.01.2005 Gazette 2005/04)**

(54) **METHODS AND SYSTEMS FOR DIAGNOSIS OF NON-CENTRAL NERVOUS SYSTEM (CNS) DISEASES IN CNS SAMPLES**

VERFAHREN UND SYSTEME ZUR DIAGNOSE VON NICHT DAS ZENTRALE NERVENSYSTEM (ZNS) BETREFFENDEN KRANKHEITEN IN ZNS-PROBEN

METHODES ET SYSTEMES POUR DIAGNOSTIQUER DES MALADIES DU SYSTEME NERVEUX NON CENTRAL (SNC) DANS DES ECHANTILLONS CNS

(84) Designated Contracting States:
**AT BE BG CH CY CZ DE DK EE ES FI FR GB GR HU IE IT LI LU MC NL PL PT RO SE SI SK TR**

(30) Priority: **03.07.2003 US 484683 P**
**03.07.2003 US 484726 P**

(43) Date of publication of application:
**26.04.2006 Bulletin 2006/17**

(73) Proprietor: **Gentron, LLC.**
**New Castle, DE 19720 (US)**

(72) Inventors:
• **PODHAJCER, Osvaldo, L.**
**1405 Buenos Aires (AR)**
• **PITOSSI, Fernando, Juan**
**1188 Buenos Aires (AR)**
• **RUBINSTEIN, Marcelo**
**1425 Buenos Aires (AR)**

(74) Representative: **Adams, Harvey Vaughan John**
**Mathys & Squire LLP**
**120 Holborn**
**London**
**EC1N 2SQ (GB)**

(56) References cited:
**WO-A-00/50889      WO-A-00/70340**
**WO-A-02/24956      WO-A-03/040404**

• **PETRICOIN E F ET AL: "Use of proteomic patterns in serum to identify ovarian cancer" LANCET, XX, XX, vol. 359, no. 9306, 16 February 2002 (2002-02-16), pages 572-577, XP004339269 ISSN: 0140-6736 cited in the application**
• **PETRICOIN E F III ET AL: "Serum proteomic patterns for detection of prostate cancer" JOURNAL OF THE NATIONAL CANCER INSTITUTE, US DEPT. OF HEALTH, EDICATIONAND WELFARE, PUBLIC HEALTH, US, vol. 94, no. 20, 16 October 2002 (2002-10-16), pages 1576-1578, XP002975918 ISSN: 0027-8874 cited in the application**
• **KAMINSKI N ET AL: "Global analysis of gene expression in pulmonary fibrosis reveals distinct programs regulating lung inflammation and fibrosis" PROCEEDINGS OF THE NATIONAL ACADEMY OF SCIENCES OF USA, NATIONAL ACADEMY OF SCIENCE. WASHINGTON, US, vol. 97, no. 4, 15 February 2000 (2000-02-15), pages 1778-1783, XP002237872 ISSN: 0027-8424**
• **KECHA-KAMOUN O ET AL: "THYMIC EXPRESSION OF INSULIN-RELATED GENES IN AN ANIMAL MODEL OF AUTOIMMUNE TYPE 1 DIABETES" DIABETES/METABOLISM RESEARCH AND REVIEWS, WILEY, LONDON,, GB, vol. 17, no. 2, March 2001 (2001-03), pages 146-152, XP008025928 ISSN: 1520-7552**

**(Cont. next page)**

**EP 1 649 062 B1**

• SORIANO M A ET AL: "PARALLEL GENE EXPRESSION MONITORING USING OLIGONUCLEOTIDE PROBE ARRAYS OF MULTIPLE TRANSCRIPTS WITH AN ANIMAL MODEL OF FOCAL ISCHEMIA" JOURNAL OF CEREBRAL BLOOD FLOW AND METABOLISM, RAVEN PRESS, LTD., NEW YORK, NY, US, vol. 20, no. 7, July 2000 (2000-07), pages 1045-1055, XP009012666 ISSN: 0271-678X

**Description**

**CROSS-REFERENCE TO RELATED APPLICATIONS**

This applications claims priority from U.S. Provisional Patent Application Nos. 60/484,683 and 60/484,726, both filed on July 3, 2003.

**FIELD OF THE INVENTION**

The invention relates to methods and compositions for risk assessment, identification, diagnosis, prognosis, and/or monitoring of disease, and for early therapeutic intervention.

**BACKGROUND OF THE INVENTION**

It is axiomatic that early diagnosis and concomitant early therapeutic intervention is the key to successful treatment and/or management of most human disorders. However, many disorders cannot be diagnosed until the pathological process is already advanced. For example, many solid tumors are usually not clinically detectable before they can be palpated or visualized by tissue imaging techniques (i.e., when they are at least 0.5 cm in size), at which time neoplasia may have been present for years. Similarly, the diagnostic criterion for diabetes mellitus (increased fasting plasma glucose levels or hyperglycemia) identifies the disorder when glucose intolerance (the underlying cause of hyperglycemia) is already present. In another example, rheumatoid arthritis (RA) is diagnosed by the presence of joint stiffness and soreness and the presence of positive rheumatoid factor, all factors that indicate RA is already present and may be advanced.

Diagnostic Disease Markers

In cancer, progression from preneoplasia to malignancy is accompanied by the accumulation of genetic changes in the neoplastic cells that lead to histopathological modifications. In some circumstances, when such a genetic change corresponds to an increase in a protein made by the tumor cells, such a protein can be detected in the tumor or in body fluids (if secreted from the tumor), and used as a biological tumor marker. Most tumors have been associated with one or more such tumor markers. Such markers have been evaluated as potential tools to diagnose cancer, determine prognosis, and/or monitor cancer progression. However, many tumor markers are detectable only after neoplasia has already progressed to the stage of formation of a tumor. In some cases, a tumor marker may not be detectable until a tumor is already malignant. Thus, many of the most widely used tumor markers are used primarily to monitor disease progression or response to treatment rather than for early diagnosis.
In rheumatoid arthritis, anti-cyclic citrullinated peptide (anti-CCP) antibodies, anti-keratin antibodies (AKA) and IgM rheumatoid factors have been suggested as markers for rheumatoid arthritis (Bas et al., Rheumatology (Oxford), 2002, 41(7):809-14). However, the value of such markers remains inconclusive (Scott, Rheumatology (Oxford), 2000, 39(Supp) 1:24-9). Similarly, while several protein and gene markers have been found to correlate with the presence of active diabetes, the use of markers as diagnostic or predictive has not been proven valuable at this time for either type I or type 2 diabetes (see the National Academy of Clinical Biochemistry (NACB) Laboratory Medicine Practice Guidelines: Guidelines and Recommendations for Laboratory Analysis in the Diagnosis and Management of Diabetes Mellitus 2002, available online at the NACB web site).

Genomics and Proteomics Tools for Disease Diagnosis

The development of high throughput screening approaches such as functional genomics and proteomics has provided a new biological platform to search for molecules associated with different disorders. Gene-expression profiles based on microarray analysis have been of some use to predict survival of patients with lung carcinoma (Beer et al., 2002, Nat. Med., 8(8):816-24). A similar approach identified a group of genes that were said to be useful to predict the clinical outcome of diffuse large B-cell lymphoma following combination chemotherapy (Shipp et al., 2002, Nat. Med., 8(1):68-74). In addition, comparison of the proteomic profile of patients with ovary or prostate cancer compared to non cancerous volunteers was said to have provided a set of serum proteins that might be useful for early cancer detection (Petricoin et al., 2002, Lancet, 2002, 359(9306):572-7; Petricoin et al., 2002, J. Natl. Cancer Inst., 94(20):1576-8).
At present, most functional genomics studies in cancer have used cancer samples obtained from patients to generate cancer-associated gene expression profiles (either by a genomics or a proteomics approach).
A need remains for methods to detect and diagnose disease. Particularly needed are predictive methods and markers for early stage or very early stage disease detection and risk assessment.

## SUMMARY OF THE INVENTION

The methods and systems described herein are based, at least in part, on the discovery that the central nervous system (CNS) exhibits specific changes in gene expression (e.g., changes in patterns of gene expression) in response to the presence of a peripheral (non-CNS) disease or disorder (e.g., a hyperproliferative disorder such as a non-CNS tumor or cancer, an immunological disorder, an inflammatory disorder, a metabolic disorder, or a pathogenic infection). While not bound by any theory, the inventors believe that specific changes in gene expression in the CNS, e.g., in the brain, occur in response to the presence of peripheral disease at an early stage in the development of the disease, e.g., before the disorder is clinically detectable and/or before the subject is symptomatic. Thus, peripheral disorders can be diagnosed at an early stage and targeted for early therapeutic intervention by analyzing changes or patterns in gene expression in the CNS.

Accordingly, in one aspect, the invention features methods of diagnosing a non-CNS disorder in a subject, such as a human. The non-CNS disorder can be, e.g., a hyperproliferative disorder, e.g., a non-CNS tumor or cancer; an immunological disorder, e.g., rheumatoid arthritis; an inflammatory or allergic disorder, e.g., asthma; a metabolic disorder, e.g., diabetes or obesity; or a pathogenic infection, e.g., a viral infection. The methods include detecting expression of a gene in a CNS sample of the subject, e.g., a brain tissue or cell (such as a tissue or cell of the hypothalamus, the cerebellum, the midbrain, the hippocampus, the prefrontal cortex or the striatum) or a sample of cerebrospinal fluid (CSF) or any other bodily fluid where the CNS gene product (or derivatives from it) could be detected. The method optionally includes a step of obtaining the CNS sample. A change in gene expression compared to a reference value, e.g., a control or basal value, is correlated with the presence of a non-CNS disorder. The method is not limiting in that it can be used to detect the risk or presence of any non-CNS disorder. In one embodiment, the non-CNS disorder is not lymphoma.

The subject can be a human. In one embodiment, the human is not symptomatic for the disorder to be diagnosed. In another embodiment, the disorder is not clinically detectable, e.g., it is not detectable by a routine general clinical exam.

Detecting expression of a gene in a CNS sample, or any other bodily fluid where the CNS gene product (or derivatives from it) could be detected, can include detecting or determining a value for one or more of the level of mRNA, rate of transcription, amount of a gene product, and activity of a gene product. In some embodiments, expression of a single gene in the CNS may be detected, where a change in gene expression in that gene is associated with the presence of a non-CNS disorder. In other embodiments, expression of a plurality of genes (e.g., a panel or cluster of genes) may be evaluated, where a specific profile of gene expression of the plurality of genes is associated with the presence of a particular non-CNS disorder.

The method can include correlating the result of the detecting step to the presence or absence of a non-CNS disorder. "Correlating" means identifying the probability, based on the result of a detecting step, that the subject has or does not have, or will develop or will not develop at some future time, a non-CNS disorder. Correlating can include generating a dataset from, or providing a record of, the detecting step, e.g., a printed or computer readable record such as a laboratory record or dataset. The record can include other information, such as a specific subject identifier, a sample identifier for the CNS sample, a date, the identity of the operator of the method, and/or other information. The record can be used to provide or store information about the subject. For example, the record can be used to provide information (e.g., to the subject, a health care provider, the government, or insurance company). The record or information derived from the record can be used, e.g., to identify the subject as suitable or unsuitable for a particular therapy or a particular clinical trial group.

In the methods described herein, gene expression of a CNS gene can be detected by any technique available to the skilled artisan, e.g., genomics or proteomics microarray analysis of a CNS biological sample, such as brain tissue, CSF, or any other bodily fluid where the CNS gene product (or derivatives from it) could be detected; or brain imaging techniques that detect changes in gene expression. In one embodiment, the method involves detecting a CNS gene product released or secreted into the CSF. In such embodiments, an agent (such as an antibody, e.g., a labeled antibody) for detecting the gene product can be immobilized on a solid phase, e.g., in a dipstick format.

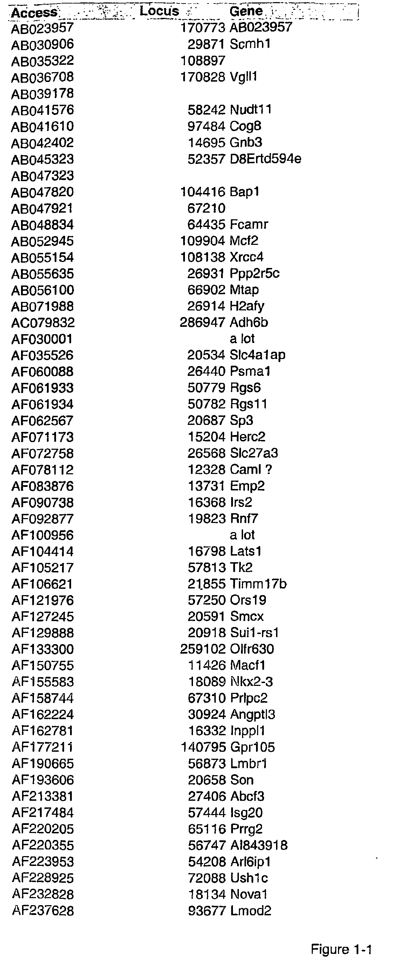

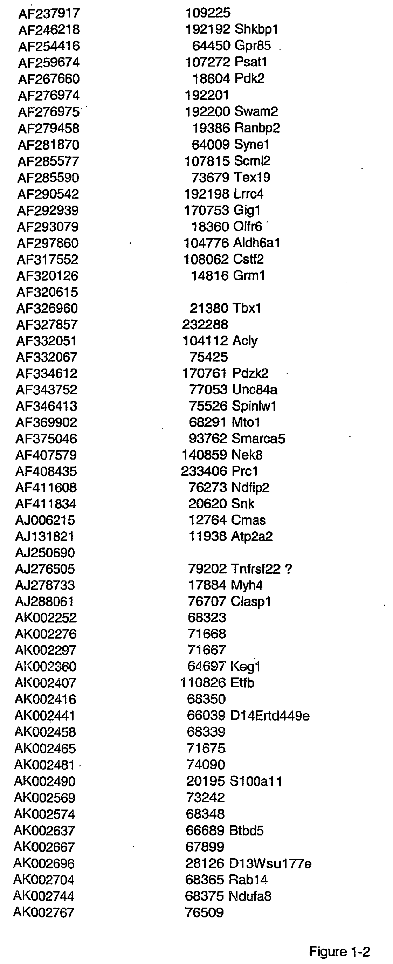

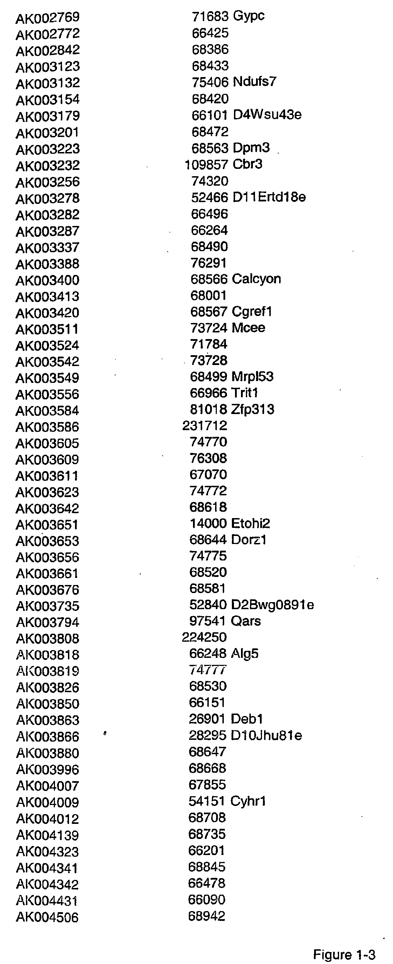

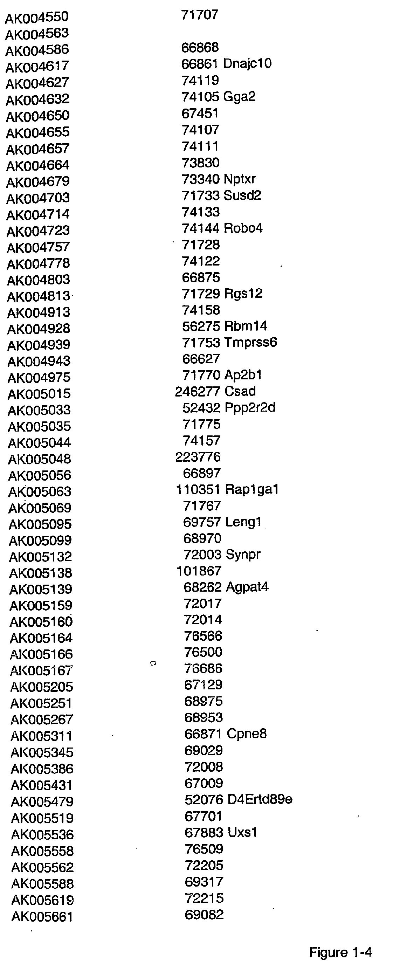

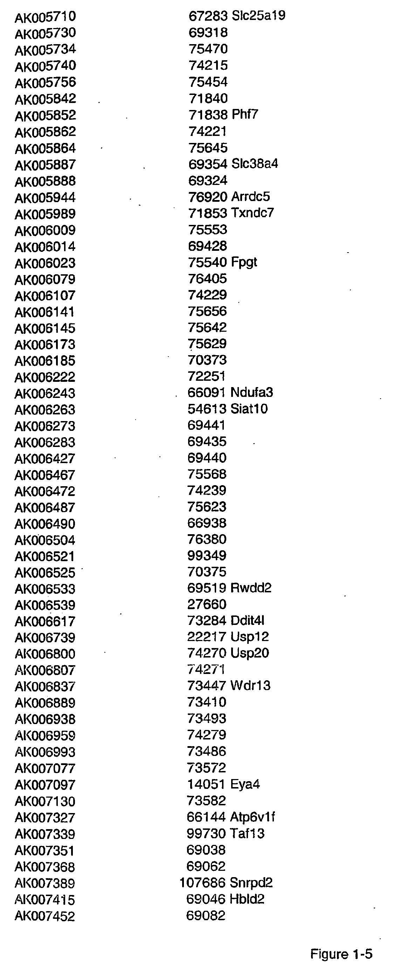

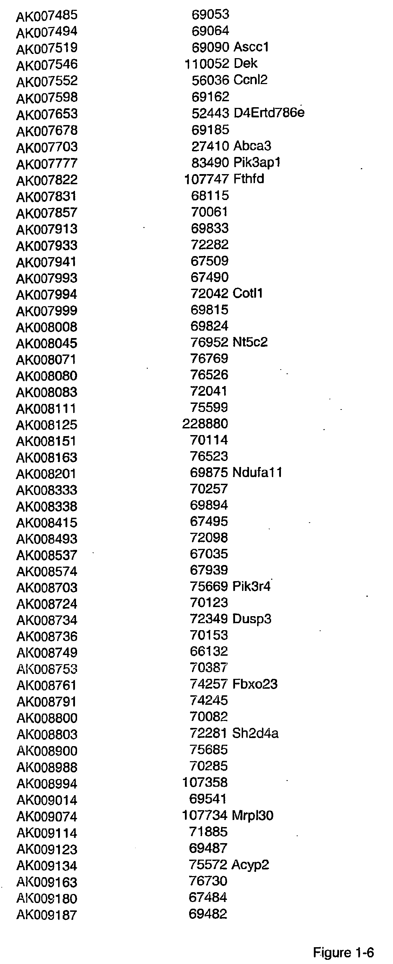

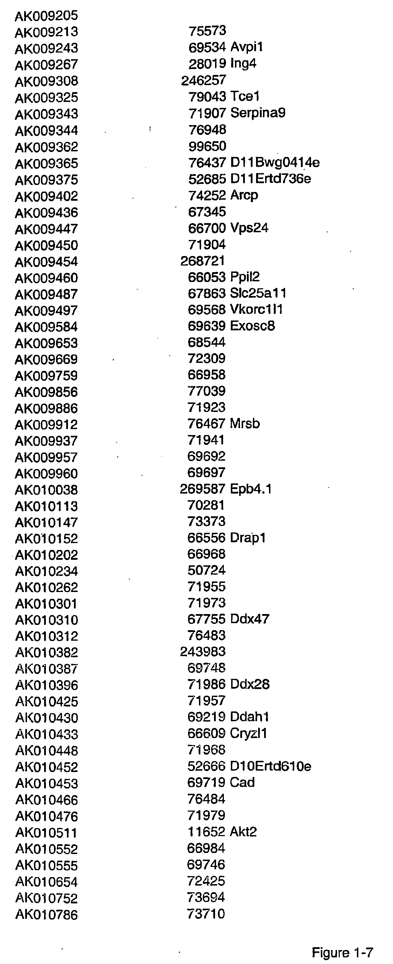

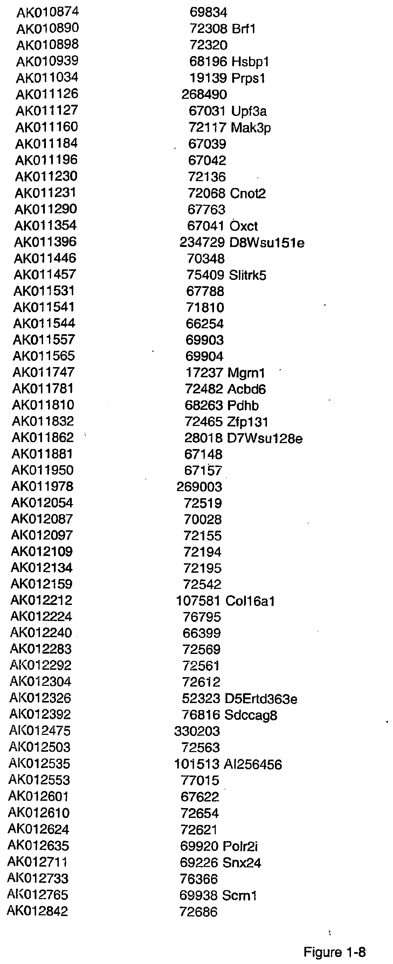

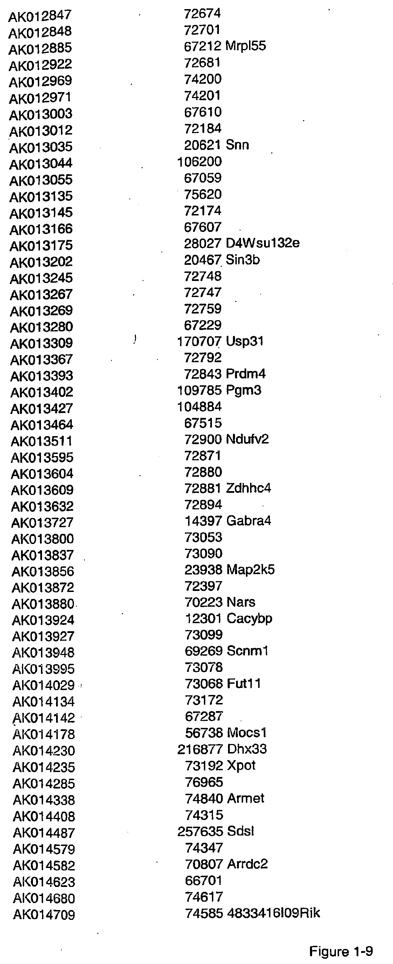

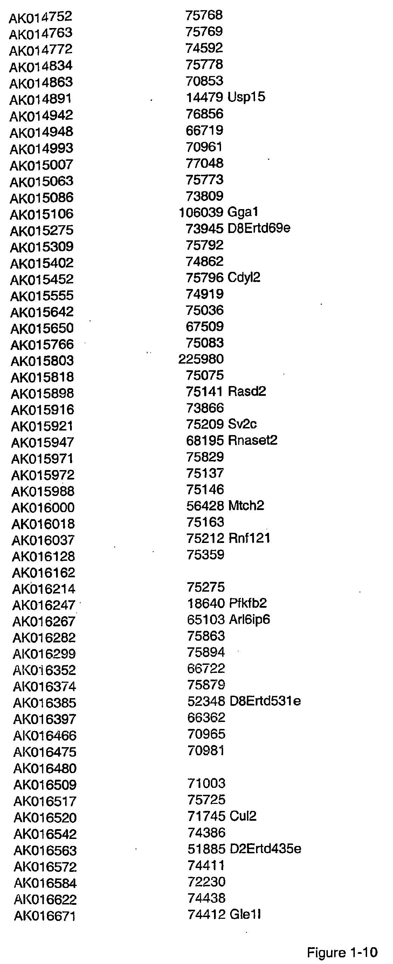

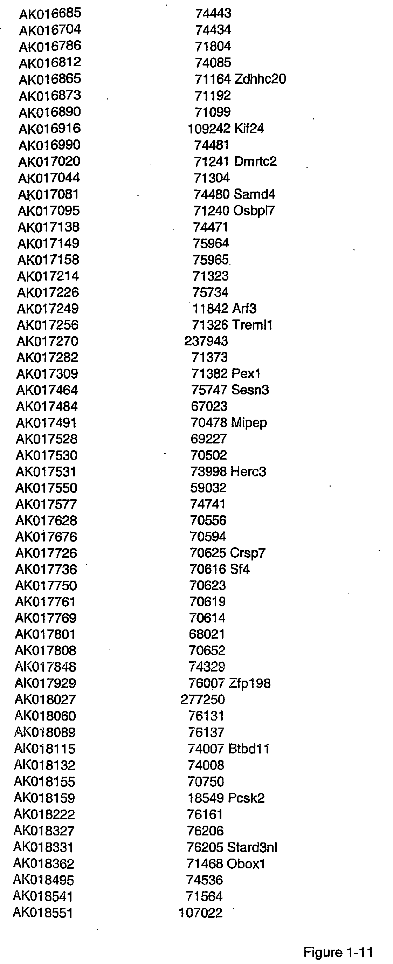

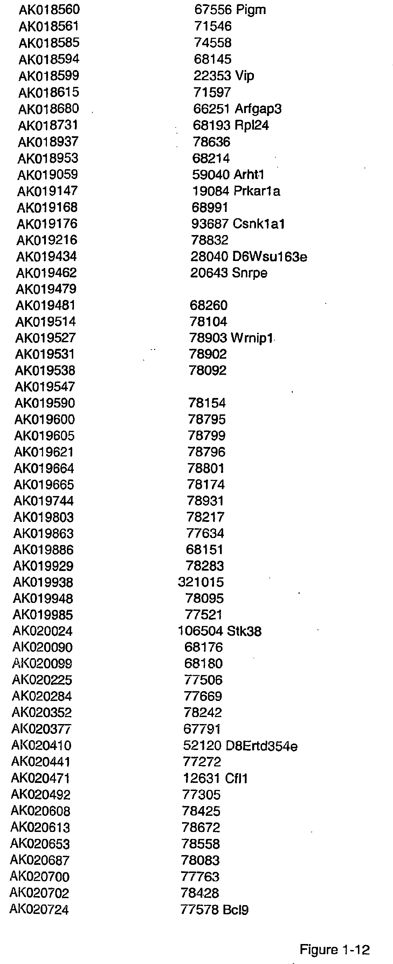

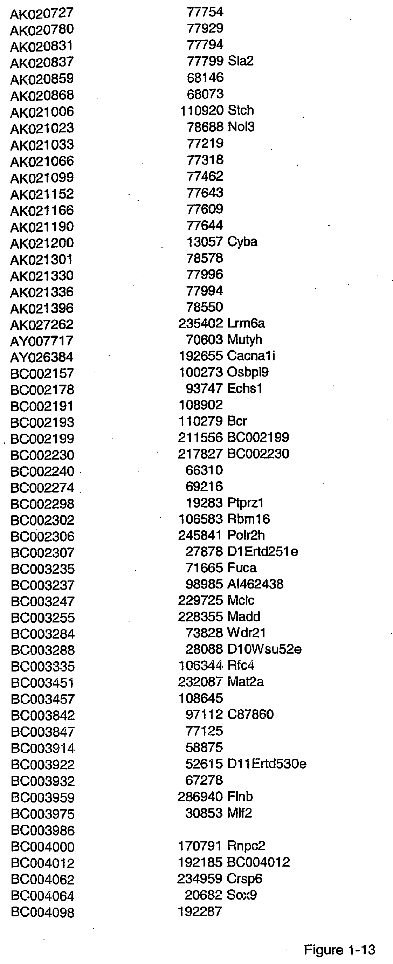

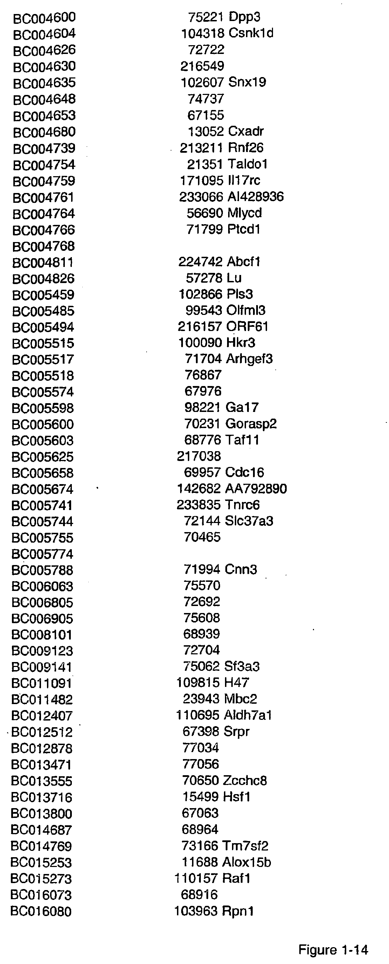

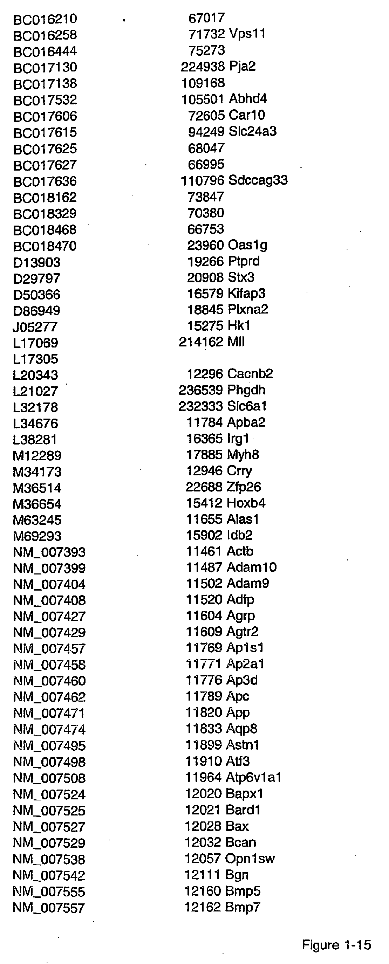

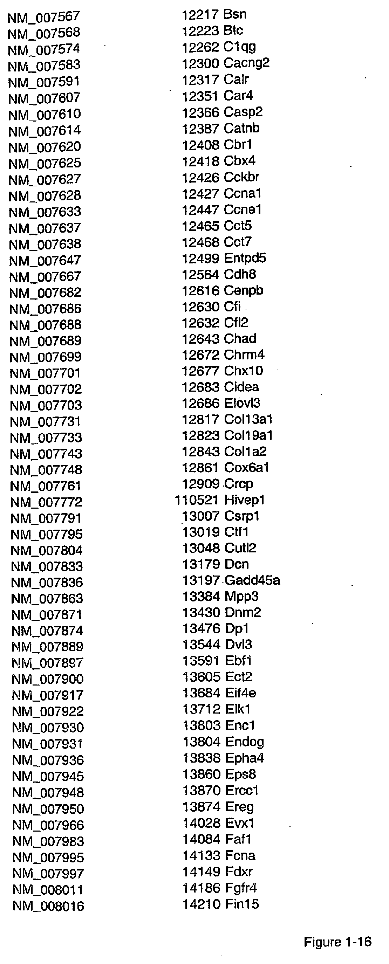

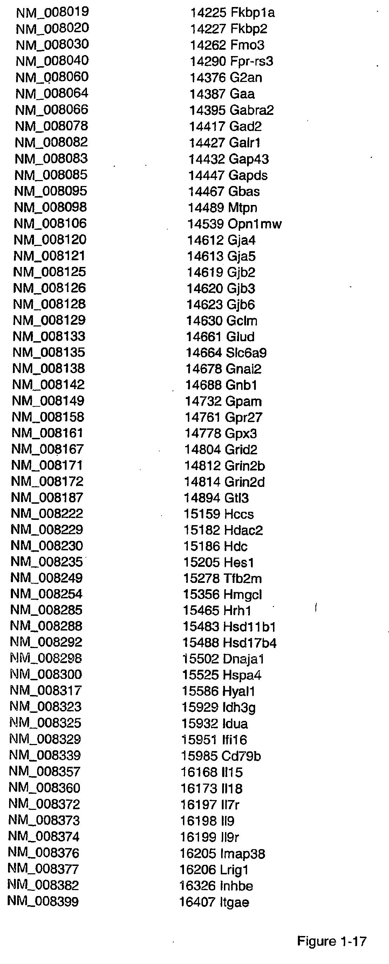

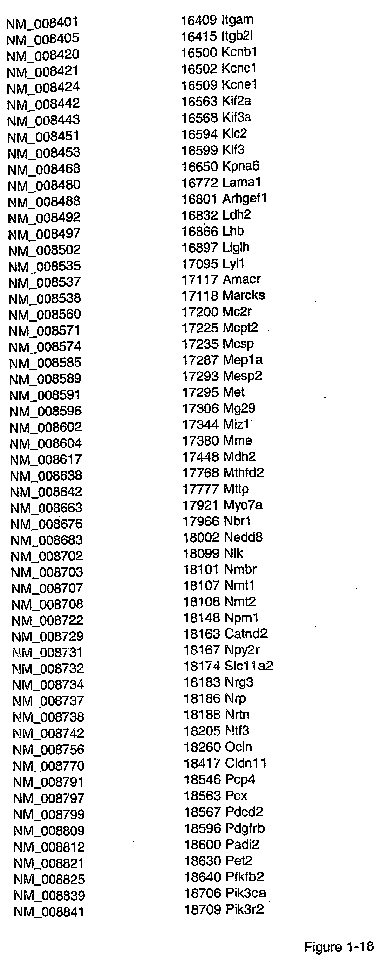

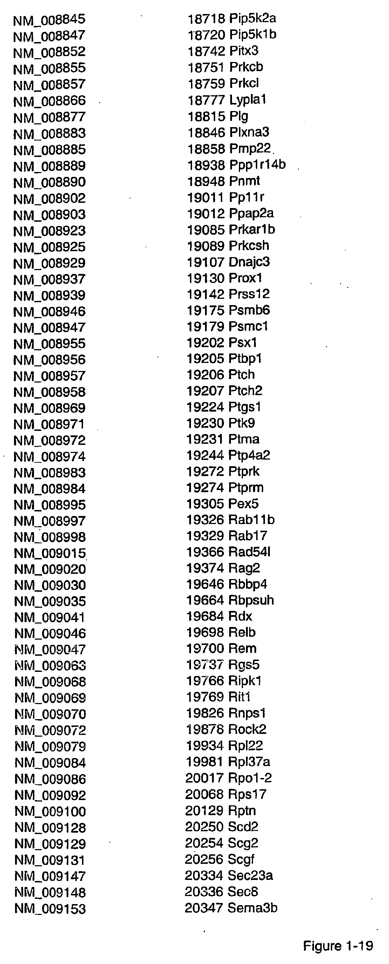

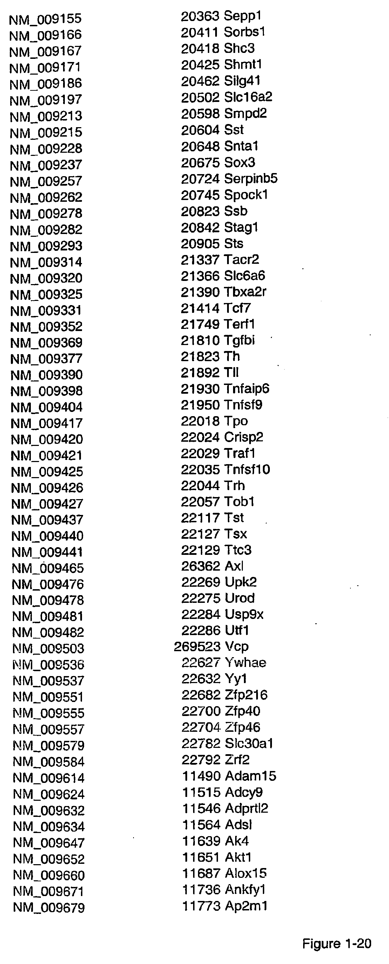

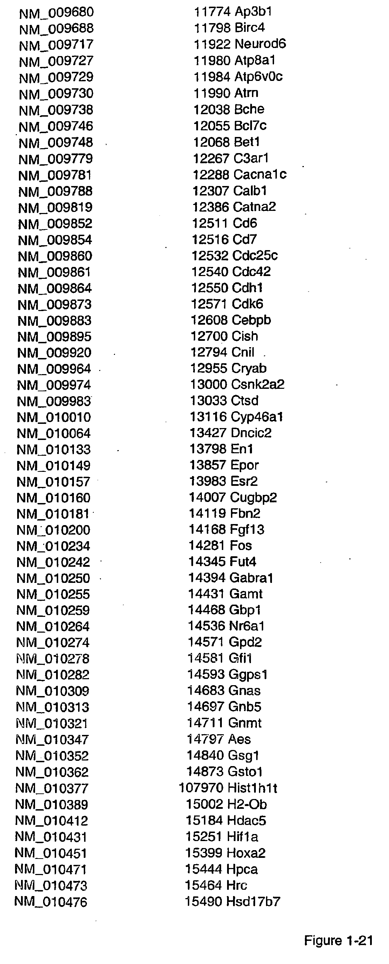

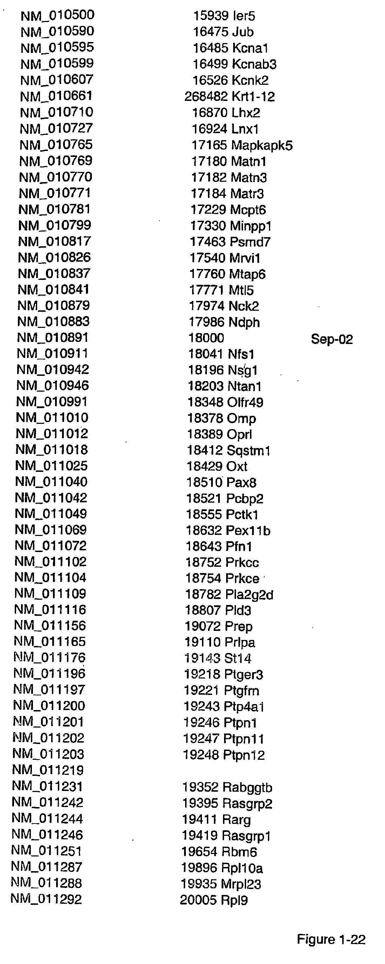

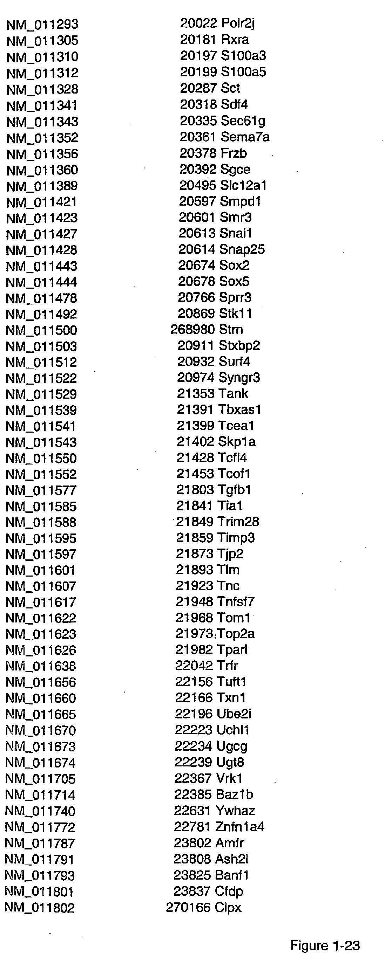

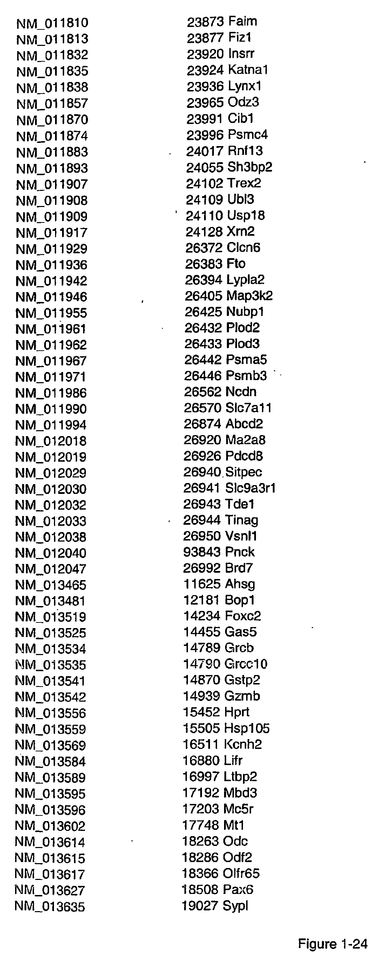

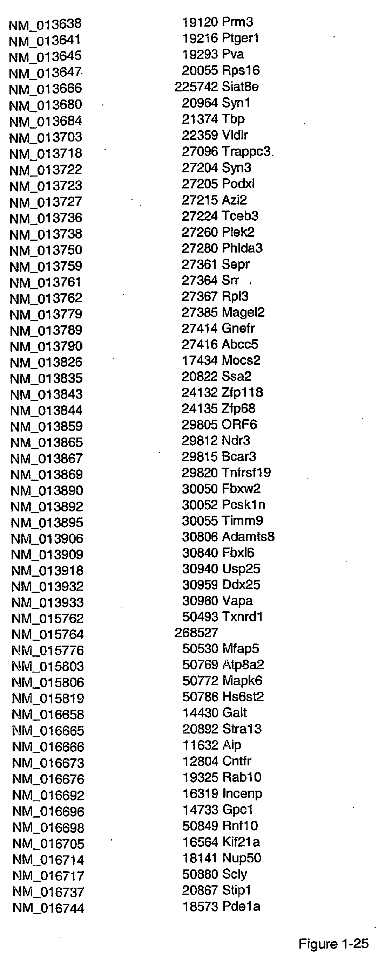

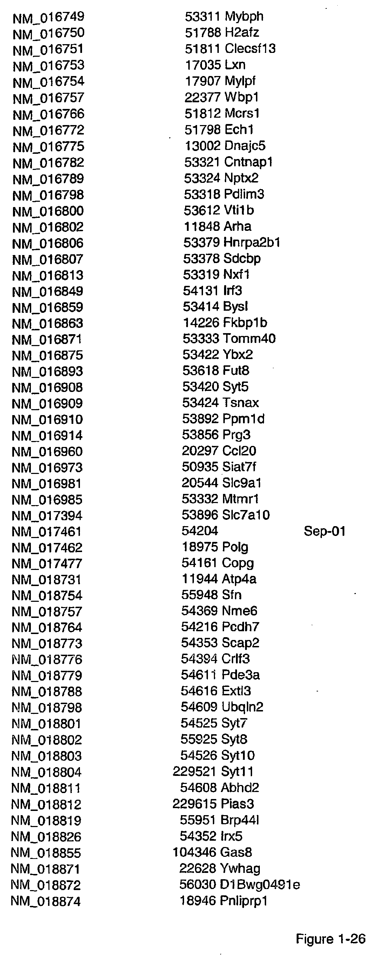

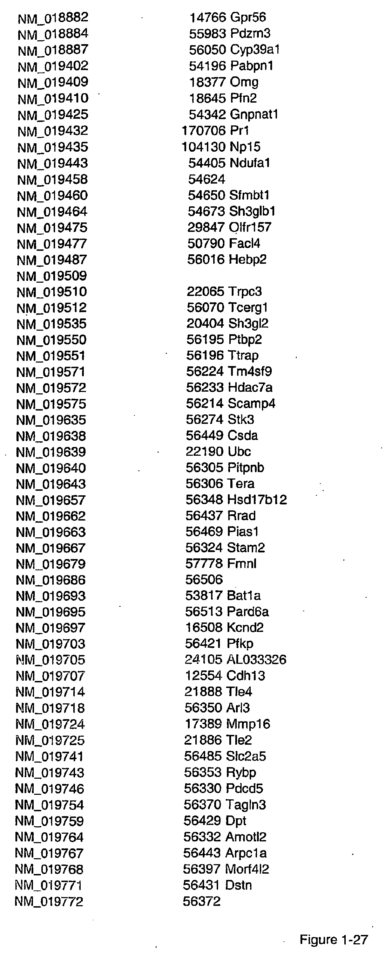

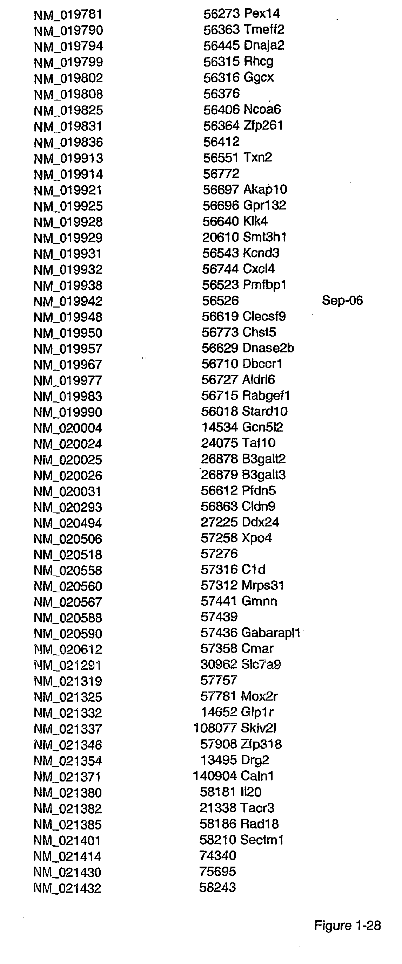

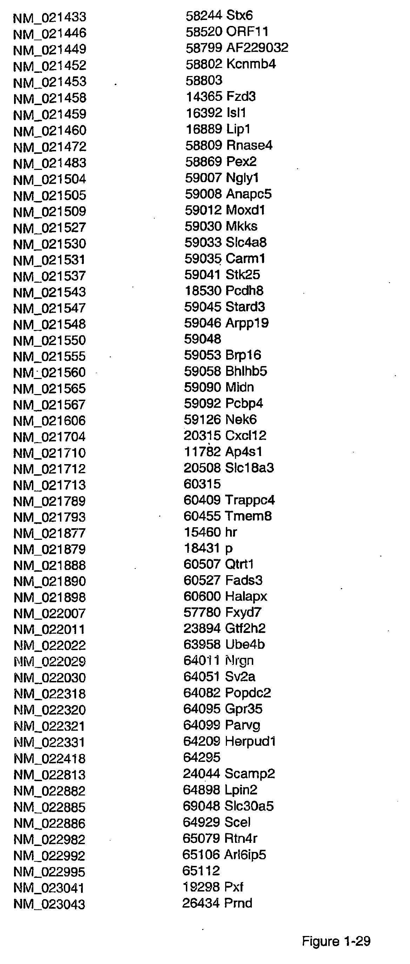

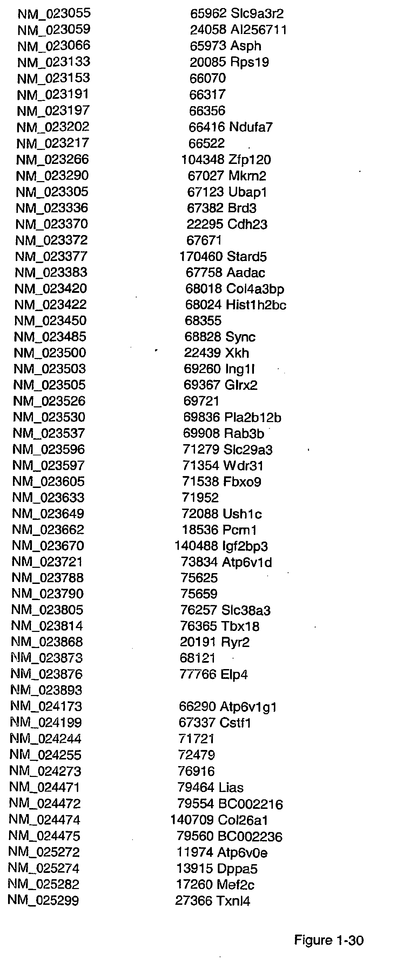

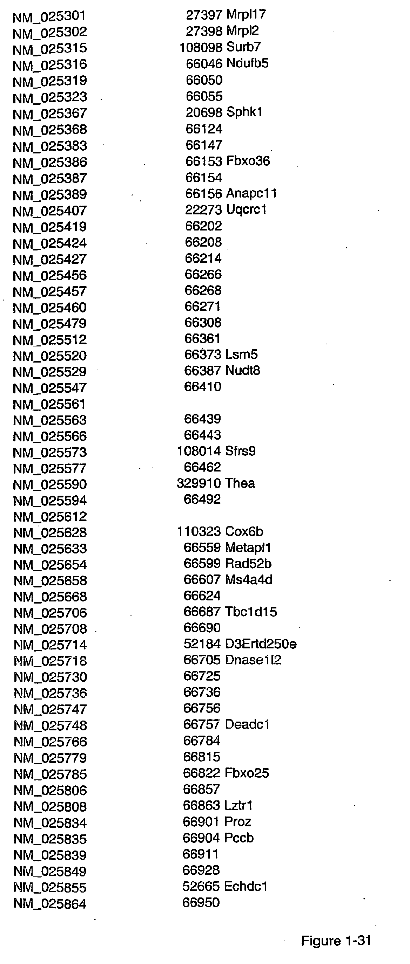

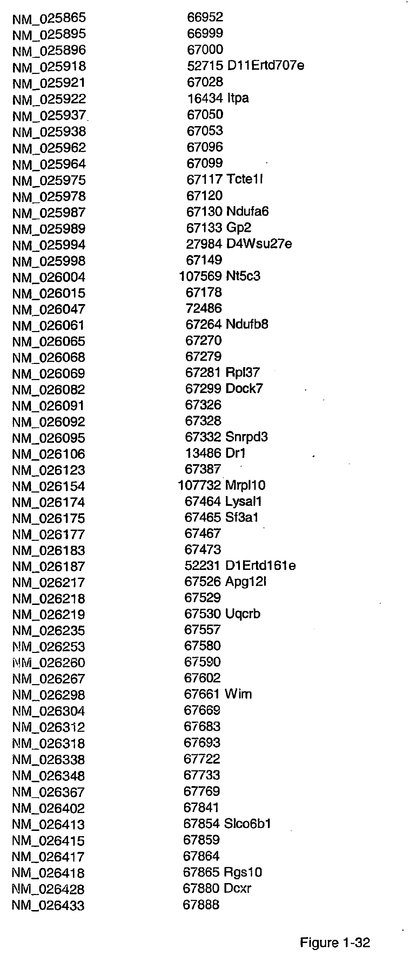

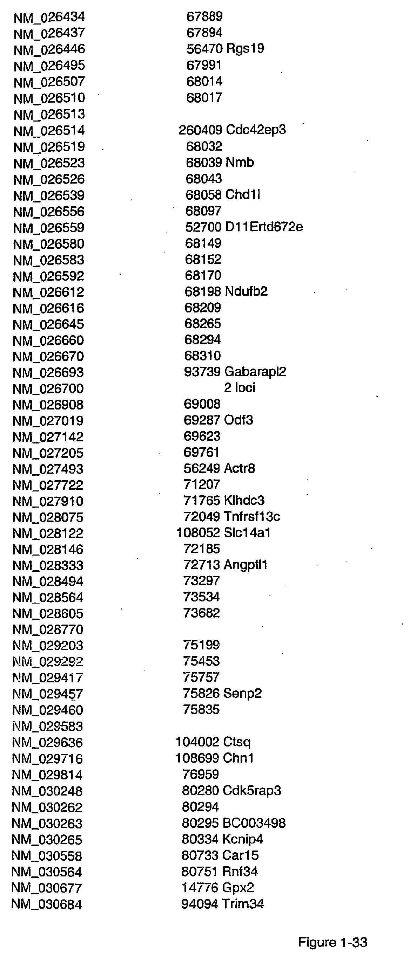

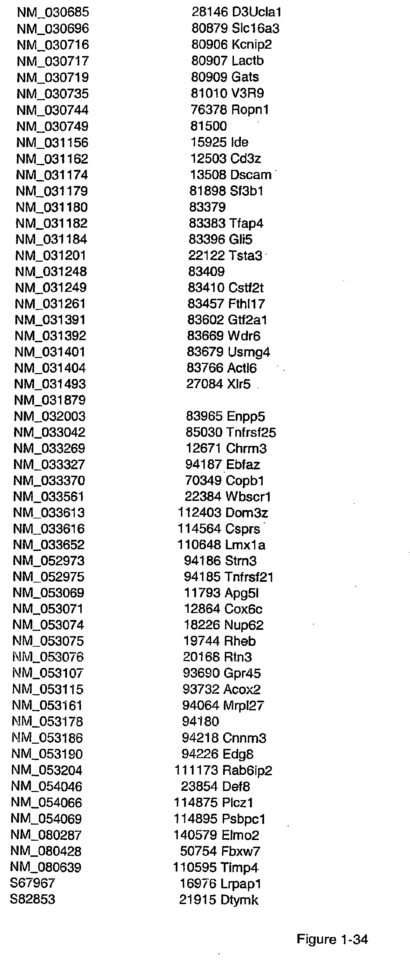

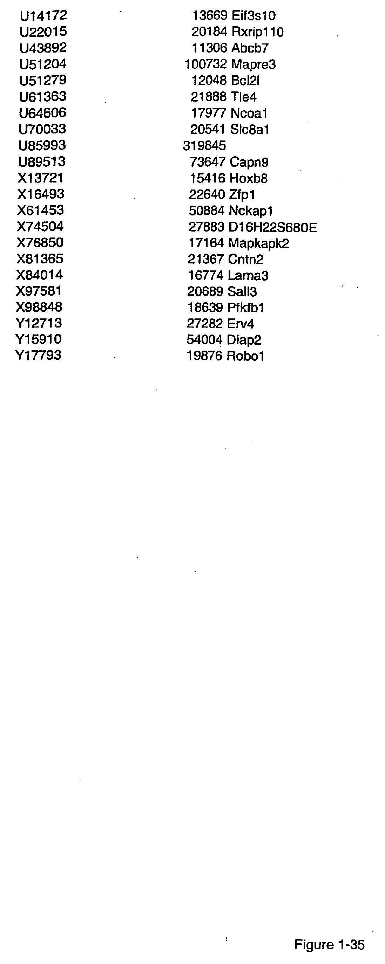

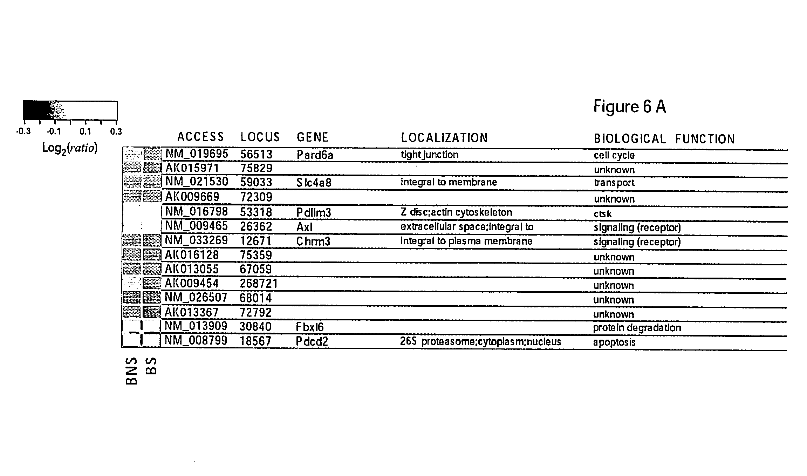

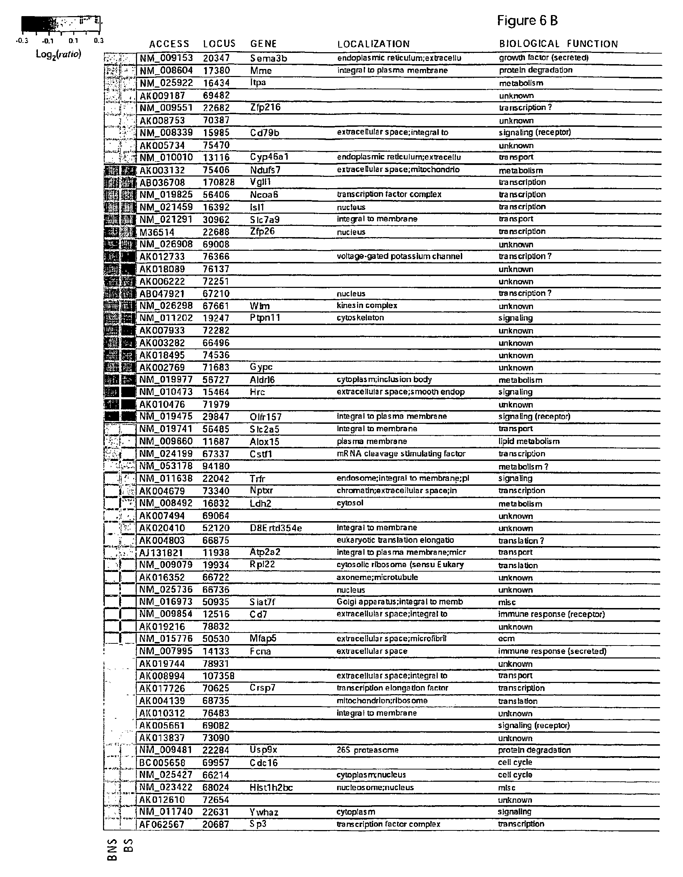

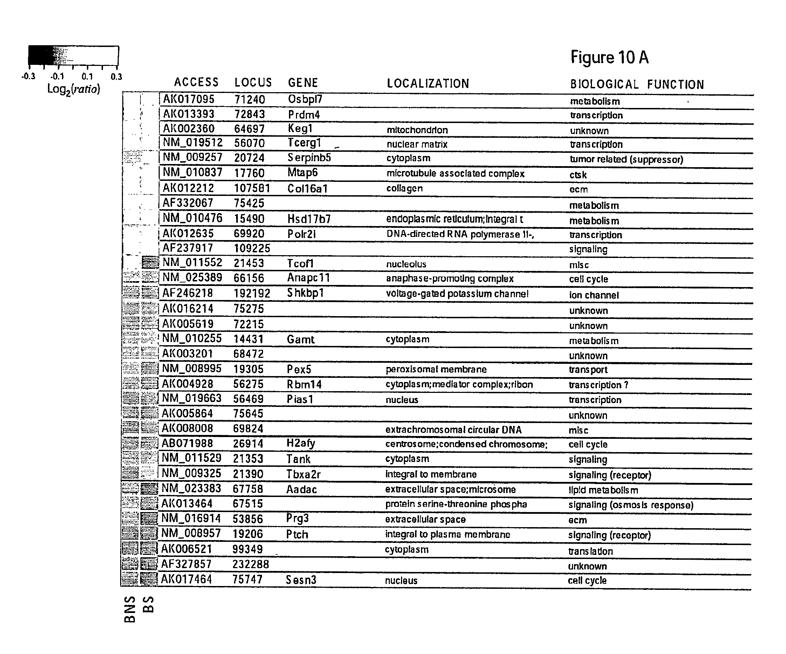

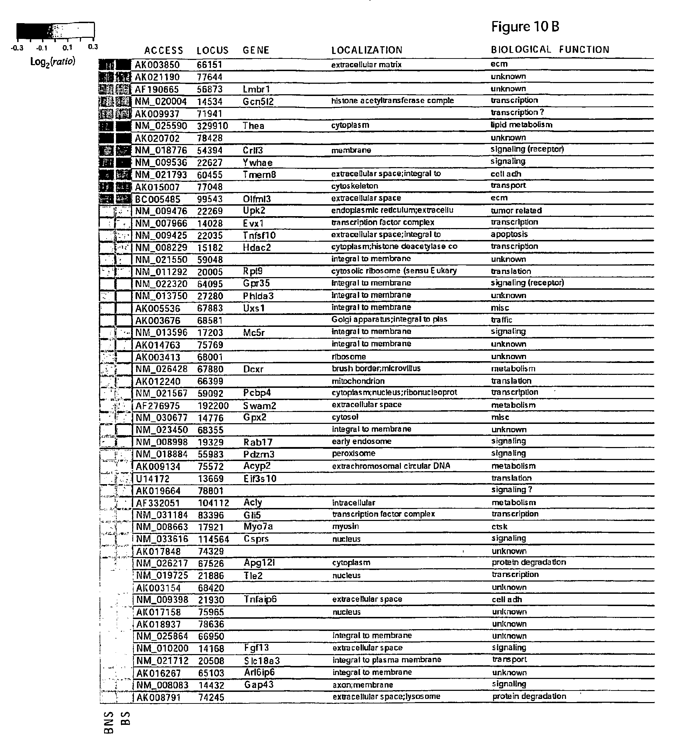

The gene or genes to be evaluated will depend on the specific gene or profile of gene expression associated with a particular disorder (reference gene expression profile). For example, exemplary genes (or profiles or clusters of genes) that are regulated in response to the presence of cancer cells (or particular types of cancer cells) are shown in FIGs. 1-29, *infra.* Such genes are also referred to herein as CNS "marker genes" or "disease surveillance genes" for non-CNS disorders. The exemplary CNS marker genes are not limiting, as the methods described herein can include the detection of other genes or gene products determined to exhibit a change in expression associated with the presence of a peripheral non-CNS disorder. CNS marker genes can include, *inter alia,* genes encoding hormones, growth factors, immune system components, and cytokines.

In another aspect, the invention features systems for diagnosing non-CNS disorders in a subject. The systems include a sampling device to obtain a CNS sample; a gene expression detection device that generates gene expression data for one or more genes in the CNS sample; a reference gene expression profile for a specific non-CNS disorder;

and a comparator that receives and compares the gene expression data with the reference gene expression profile. The invention also includes kits that can be used with such systems. The kits include the sampling device or containers for the sample, and the reference gene expression profile for a specific disorder. The profile can be in the form of a digital data set in a computer-readable medium, or an analog profile in electronic form.

Other systems included herein for diagnosing non-CNS disorders include an imaging device (e.g., PET or TRI device) to obtain an image of gene expression of one or more genes in the CNS and generate gene expression data for the one or more genes; a reference gene expression profile for a specific non-CNS disorders; and a comparator that receives and compares the gene expression data with the reference gene expression profile.

In other aspects, the invention also includes methods of diagnosing non-CNS disorders in a subject, by detecting expression of one or more genes in a CNS sample of the subject; generating gene expression data from the detected expression; obtaining a reference gene expression profile for a specific non-CNS disorders; and comparing the gene expression data with the reference gene expression profile, wherein a match of the CNS sample gene expression data to the reference gene expression profile indicates the subject has or will develop the non-CNS disorder.

In these systems and methods, the CNS sample can be a cerebrospinal fluid (CSF) sample, and the gene expression data can corresponds to a protein in the CSF. Alternatively, the CNS sample can be a bodily fluid sample that contains a protein expressed by a gene in the CNS, and the gene expression data corresponds to the presence or level of the protein in the sample. The CNS sample can also be a bodily fluid sample that contains a protein whose presence or level in the sample is affected by a gene expressed in the CNS, and the gene expression data corresponds to the presence or level of the protein in the sample. For example, the protein can be selected from a hormone, a growth factor, an immune system component, and a cytokine. The protein can be encoded by any of the genes listed in any of FIGS. 1, 50, and 54, or a human or other mammalian homolog thereof. Human homologs of the genes named herein can be easily obtained from publicly available databases, e.g., on the Internet, such as GenBank.

Specific genes encode a gene product (e.g., protein) selected from the group consisting of hepatocyte growth factor (HGF), apherin A3, chemokine (C-C motif) ligand 4, growth differentiation factor-9b (GDF-9b); bone morphogenetic protein 15 (BMP 15), neuroblastoma suppressor of tumorigenicity 1, melanocyte proliferating gene 1, and fibroblast growth factor 22 (FGF 22).

The CNS sample can also be one or more cells from the brain, and the gene expression data can correspond to a nucleic acid molecule (e.g., mRNA corresponding to the gene) or protein in the sample. The brain cells can be selected from the hypothalamus, the midbrain, the prefrontal cortex, or the striatum.

In these systems and methods, two or more reference gene expression profiles can be used, each specific for a different non-CNS disorder. The non-CNS disorder can be, for example, cancer, rheumatoid arthritis, asthma, diabetes, or obesity. For example, the non-CNS disorder can be a solid tumor less than 0.5 cm in diameter. The gene expression data can contain data for a plurality of genes in the CNS sample, and comprises a gene expression profile.

The methods herein can also include obtaining a control gene expression profile corresponding to one or more healthy subjects; and comparing the gene expression data with the control gene expression profile, wherein a match of the CNS sample gene expression data to the control gene expression profile indicates the subject does not have and will not develop the non-CNS disorder.

In the new systems and methods, gene expression can be detected using a microarray assay, and the subject can be a human.

In another aspect, the invention includes methods of diagnosing non-CNS disorders by obtaining a test gene expression profile for two or more CNS genes from the subject; obtaining a reference gene expression profile for a specific non-CNS disorder; and comparing the test gene expression profile with a reference gene expression profile, wherein a test gene expression profile that matches the reference gene expression profile indicates the subject has or will develop the non-CNS disorder.

The methods and systems herein can include generating a record of the result of the comparing step; and optionally transmitting the record to the subject, health care provider, or other party.

In yet another aspect, the invention features a computer-readable medium that contains a data set corresponding to a reference gene expression profile including expression data of 5 or more genes (e.g., 10, 15, 20, 50, or more), wherein each of the 5 or more genes is differentially expressed in a central nervous system (CNS) sample of a mammal having a specific non-CNS disorder compared to the same 5 or more genes in a mammal not having the specific non-CNS disorder; wherein the data set is used to diagnose a non-CNS disorder.

For example, in some embodiments, the computer-readable medium contains a reference gene expression profile that includes expression data of 5 or more (e.g., 10, 15, 20, 50, or more) genes selected from any of the genes listed in one or more of FIGS. 29-1 to 29-6; 32-1 to 32-6; or 35-1 to 35-6 for breast cancer; FIGs. 30-1 to 30-6; 33-1 to 33-6; or 36-1 to 36-6 for colon cancer; FIGs. 31-1 to 31-6; 34-1 to 34-6; or 37-1 to 37-6 for lung cancer; FIG. 50 for arthritis; or FIG. 54 for asthma.

The genes can also be selected from any one of the following groups of genes:

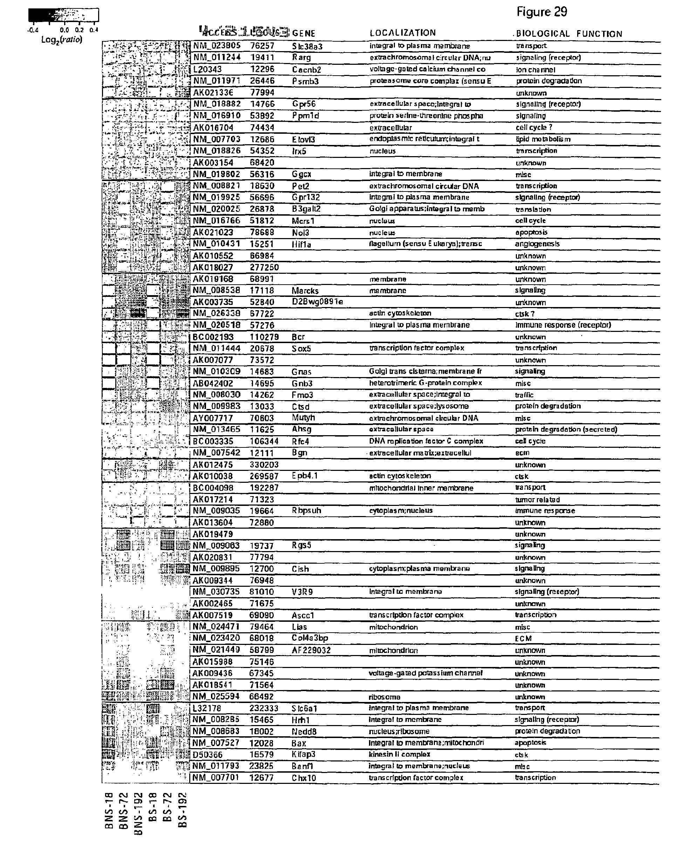

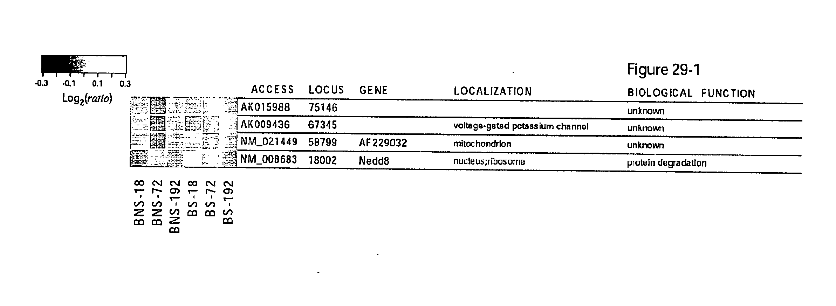

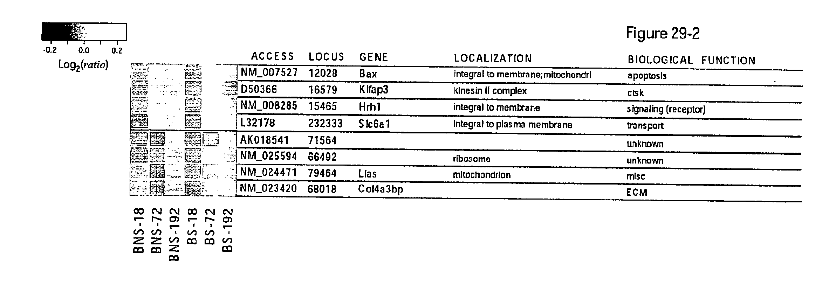

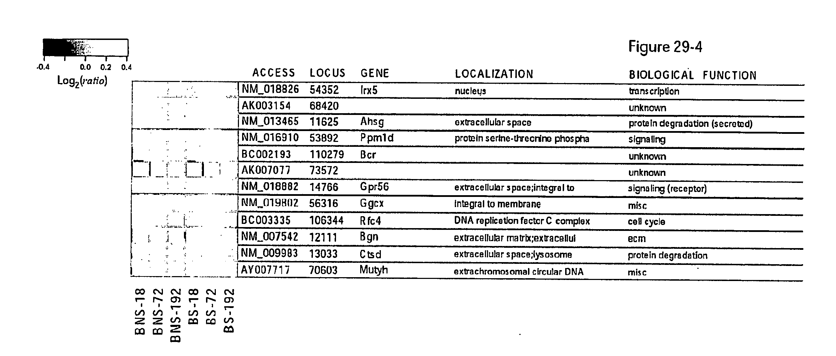

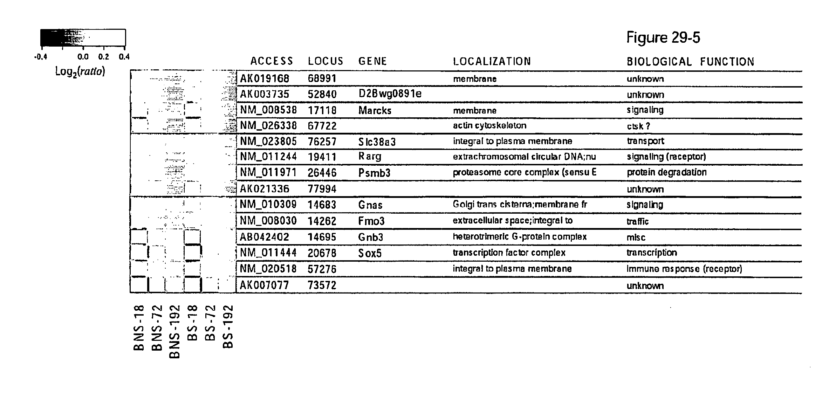

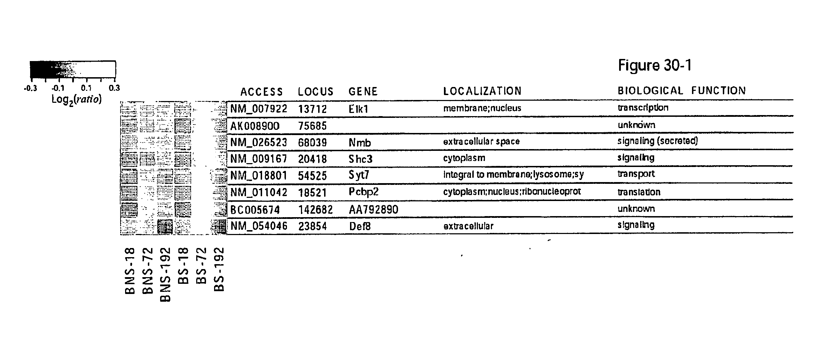

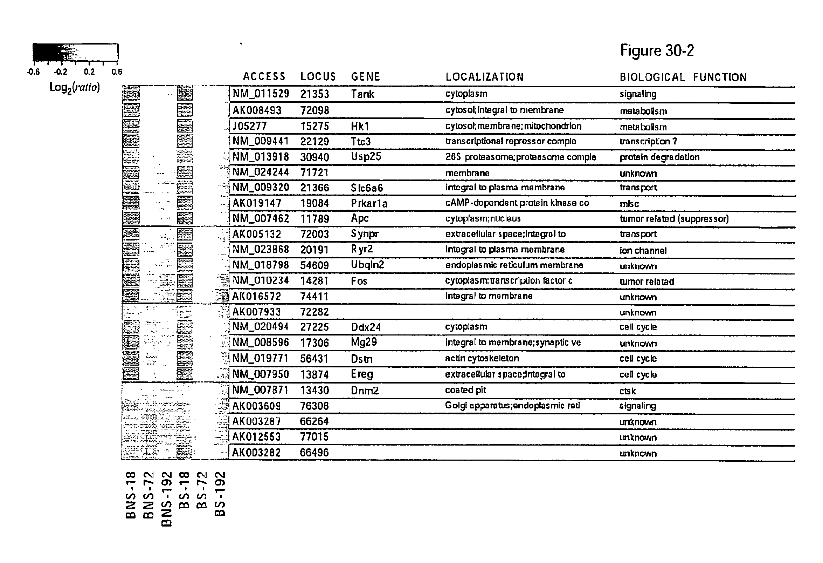

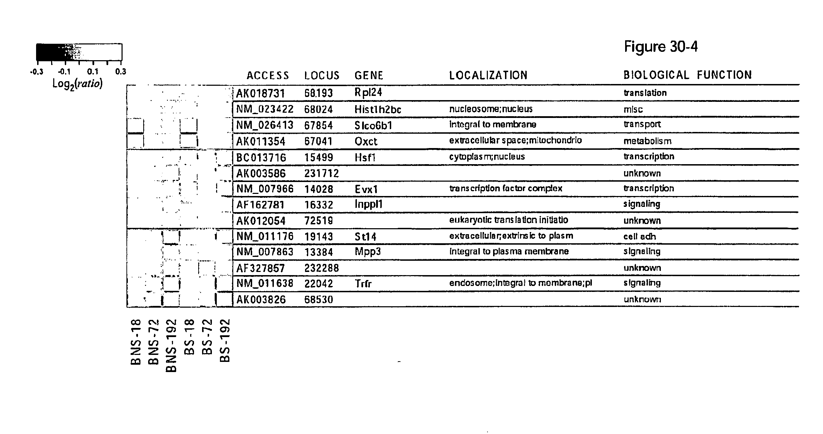

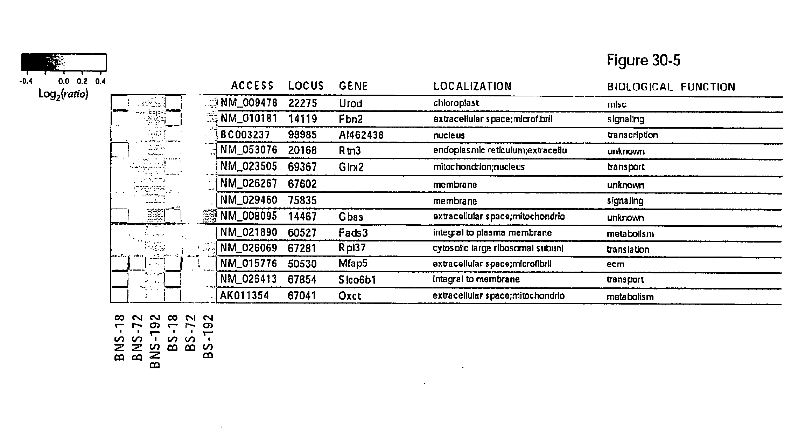

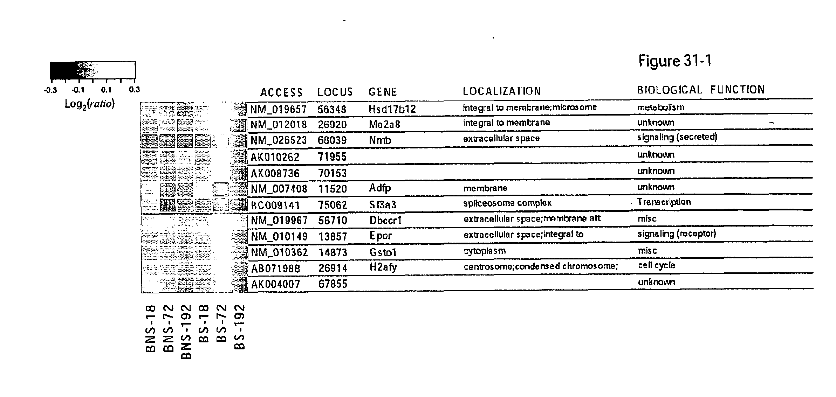

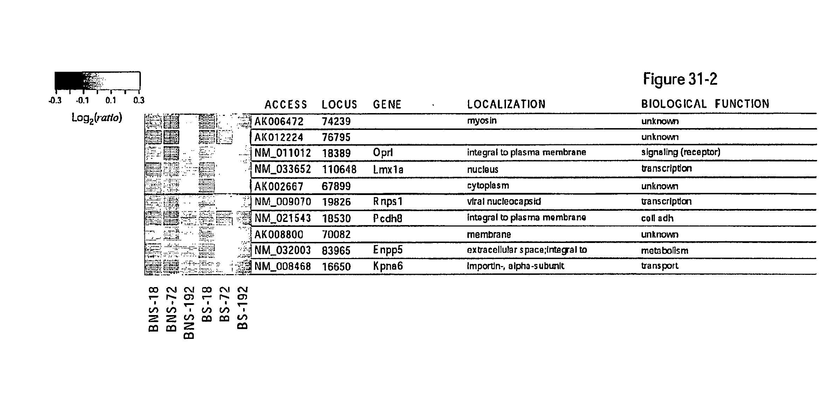

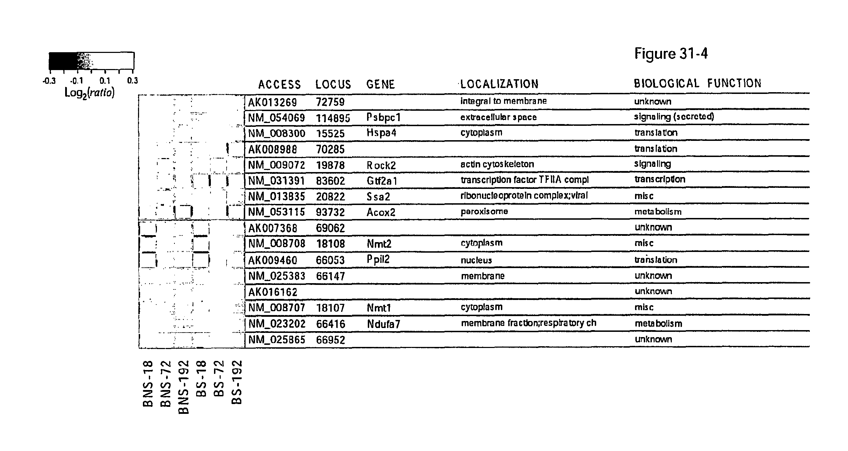

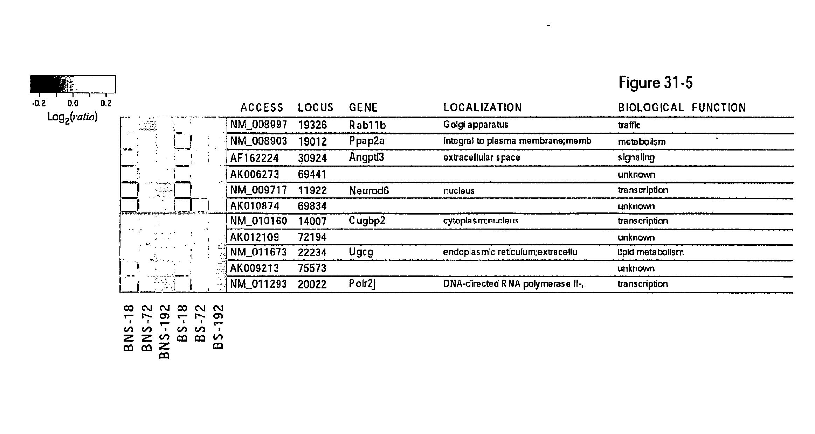

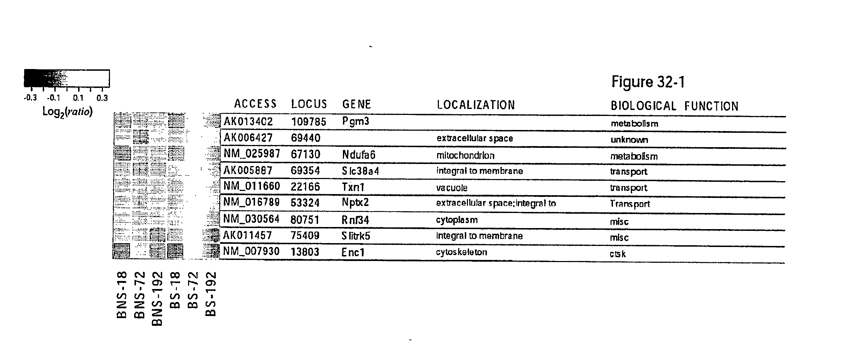

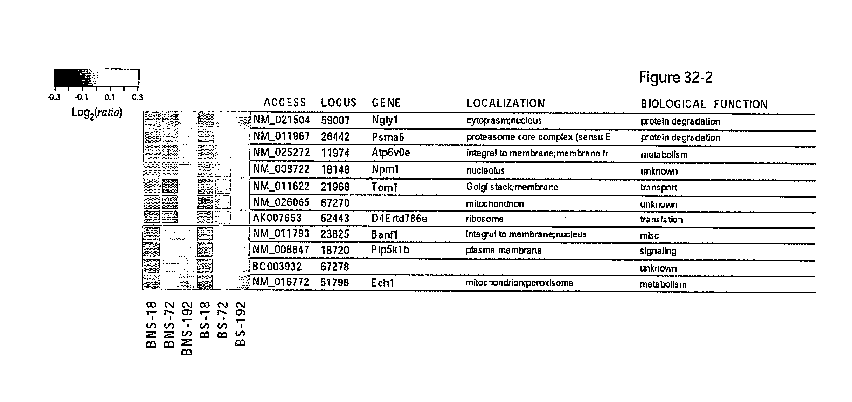

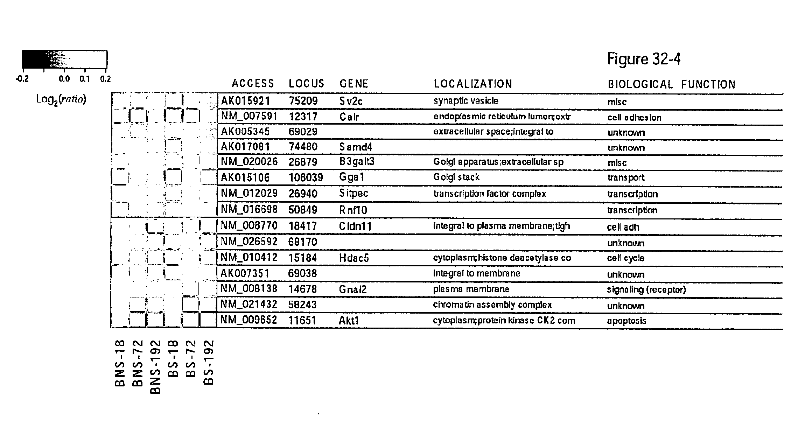

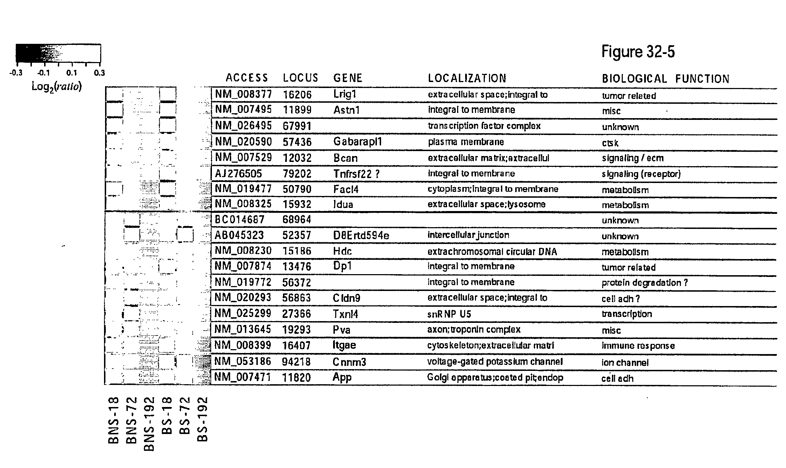

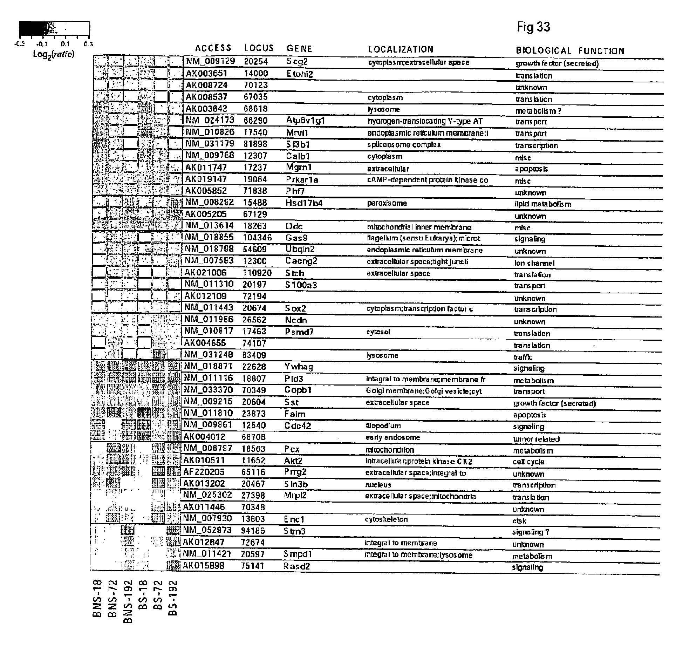

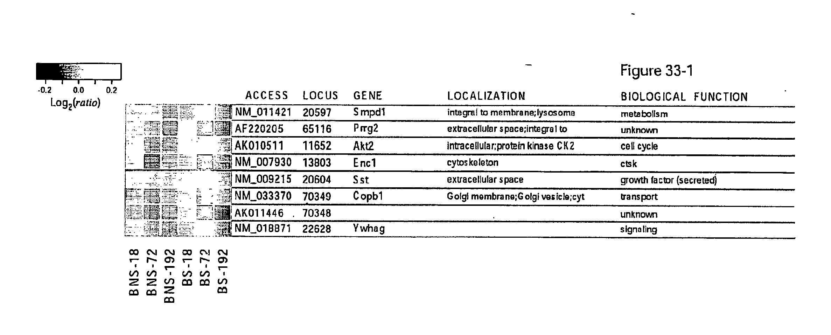

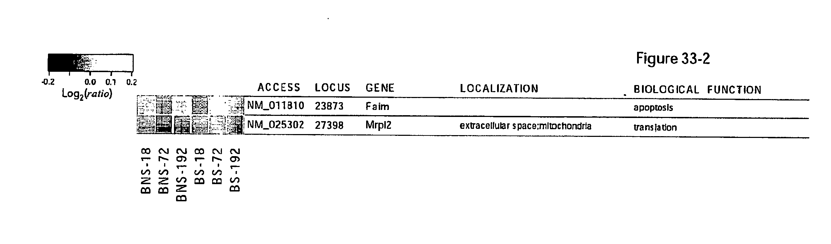

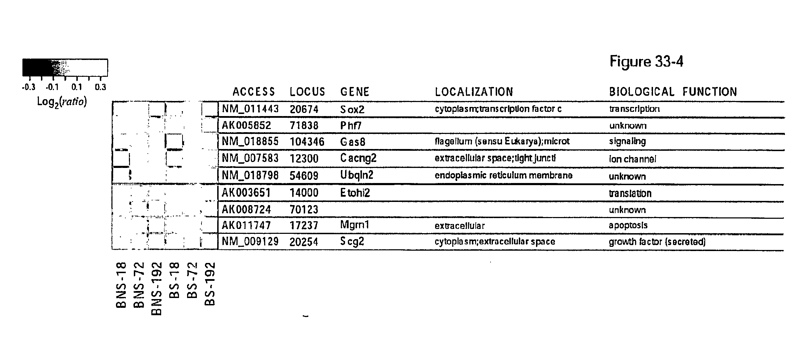

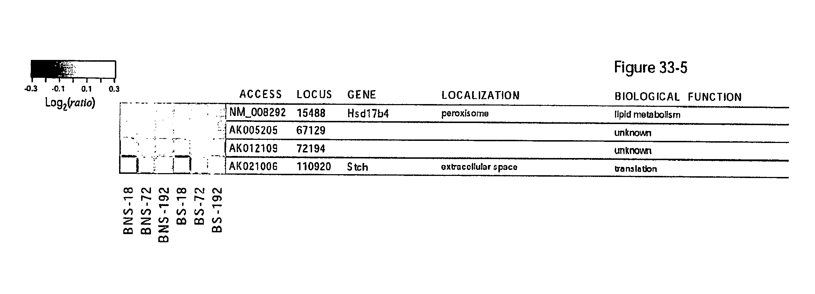

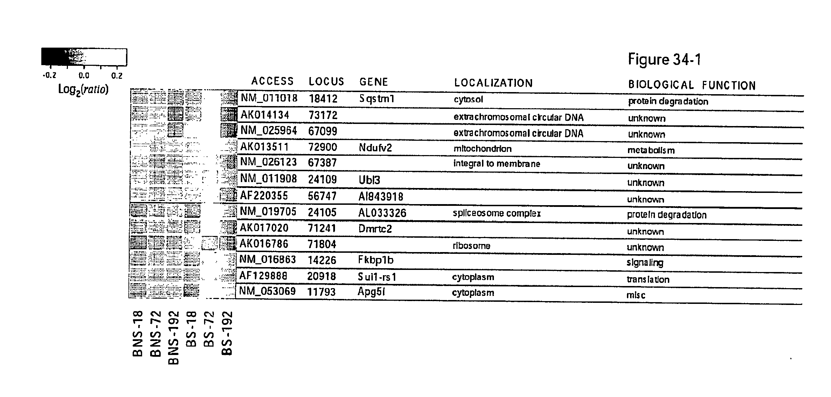

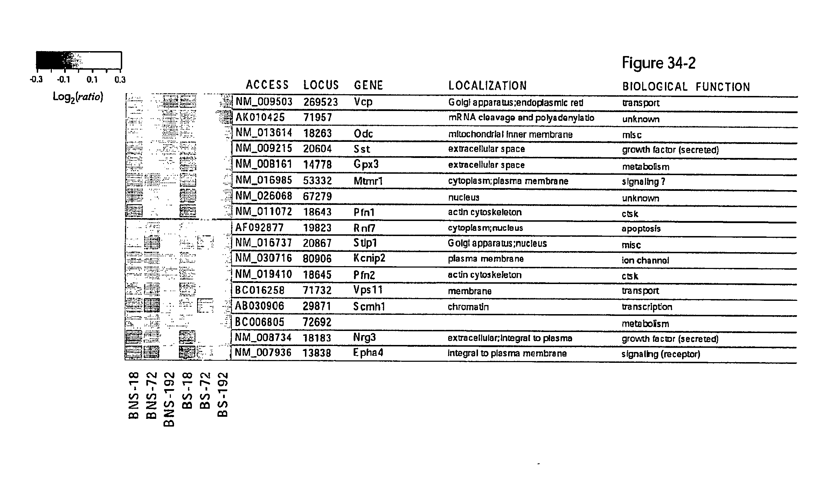

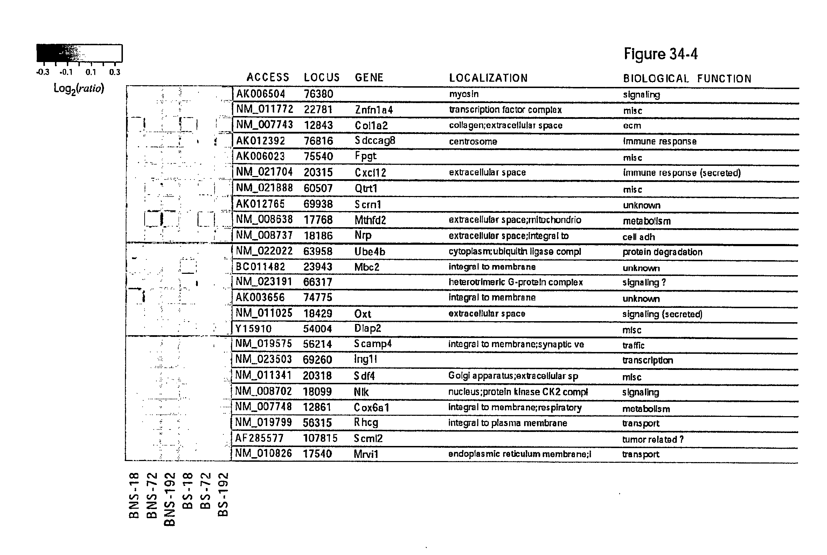

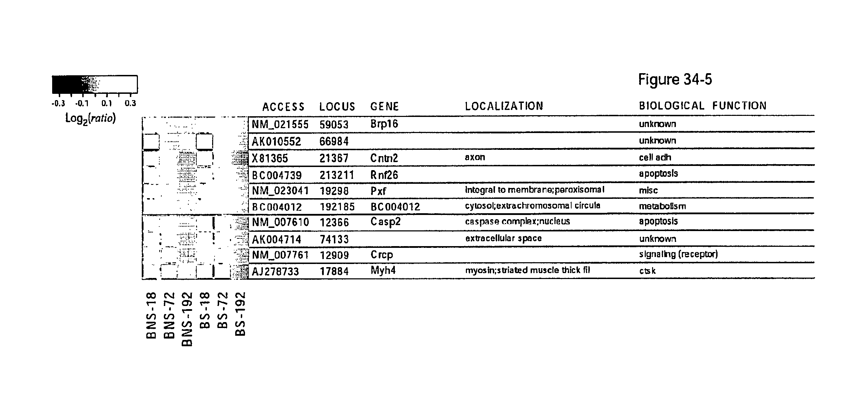

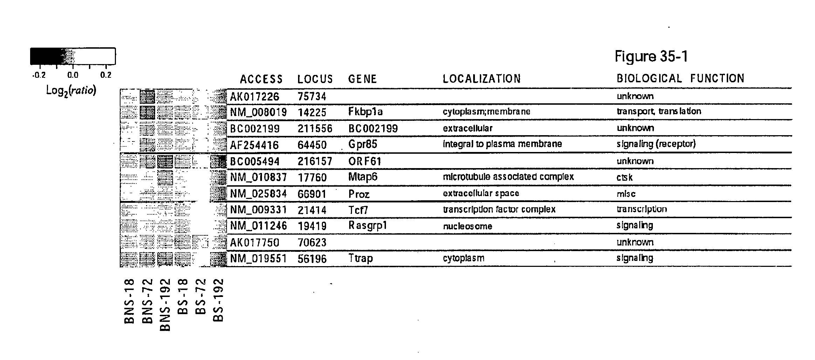

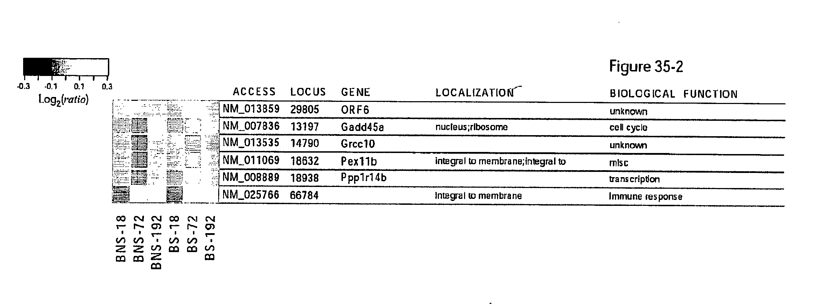

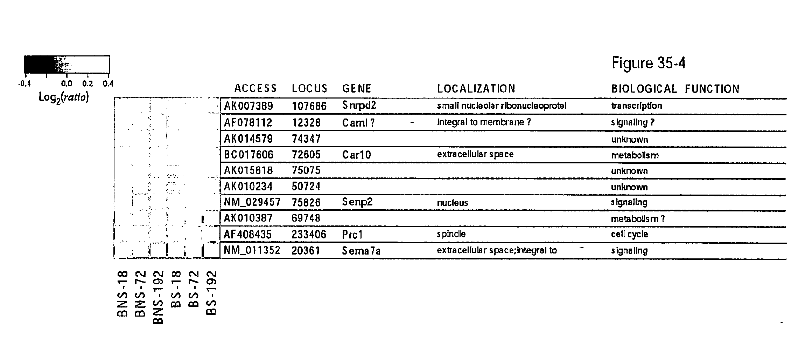

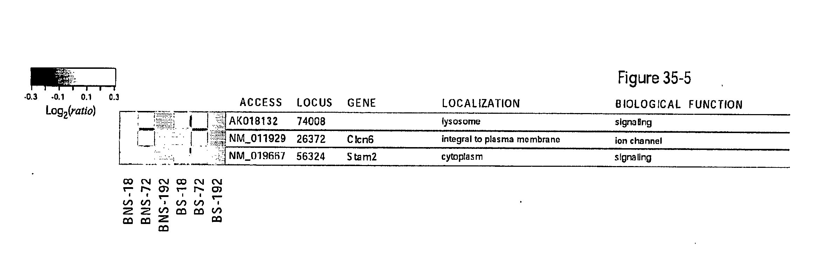

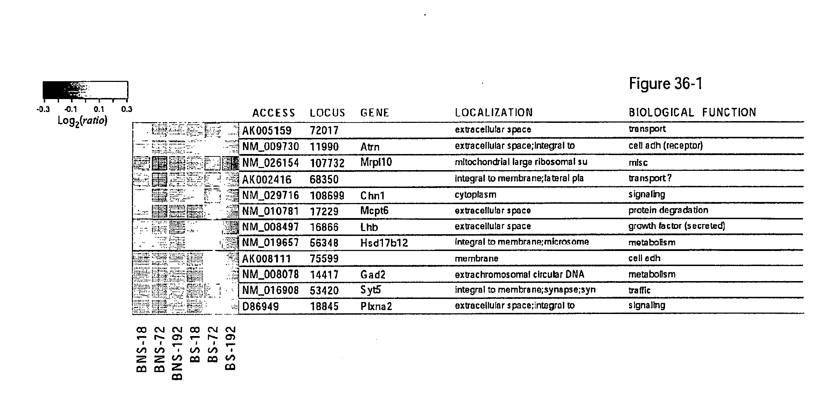

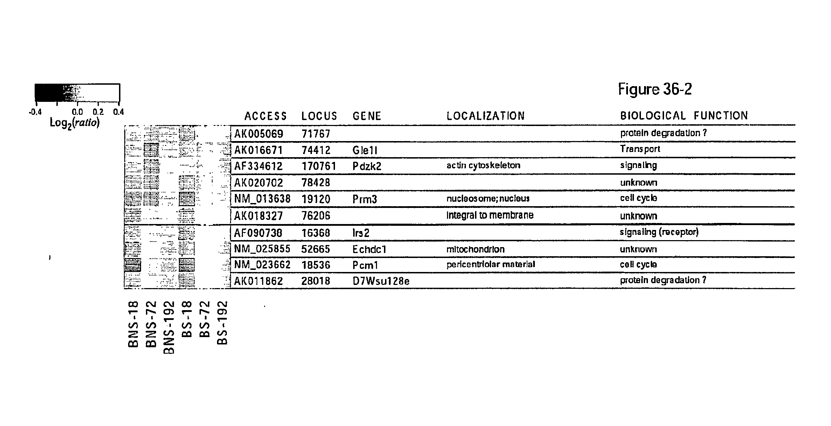

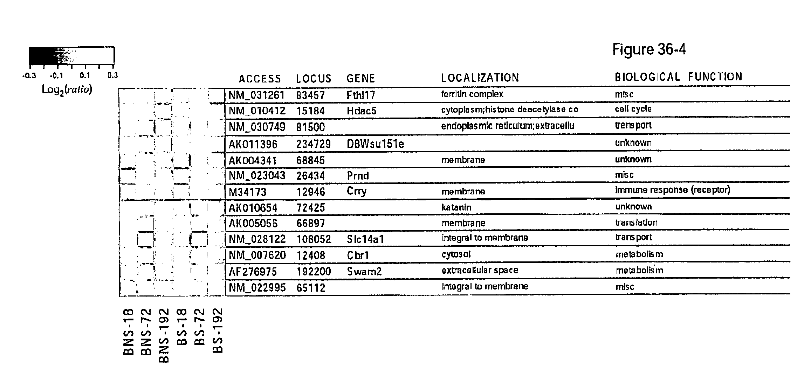

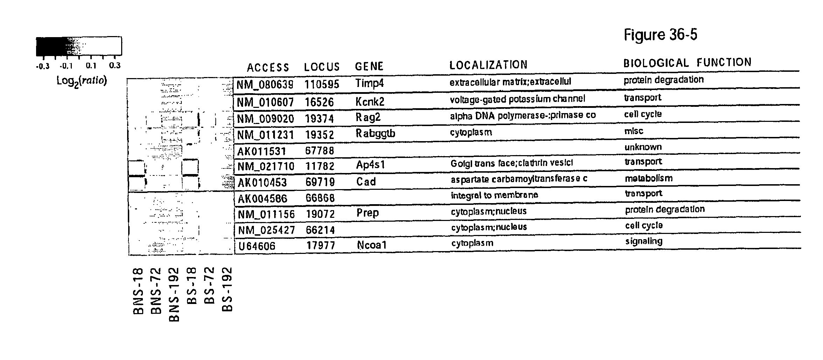

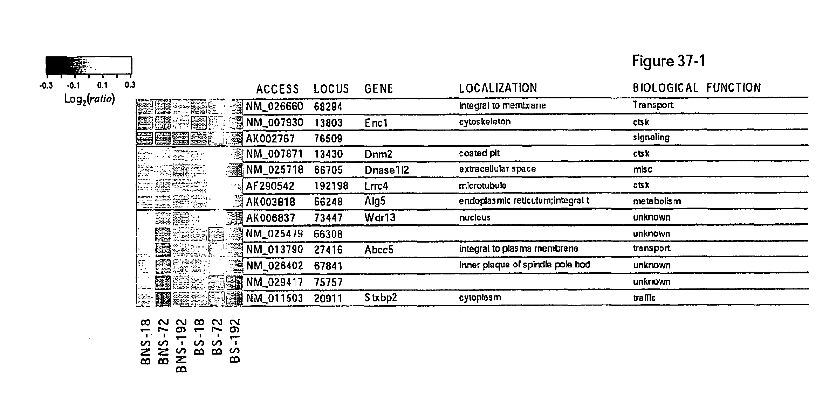

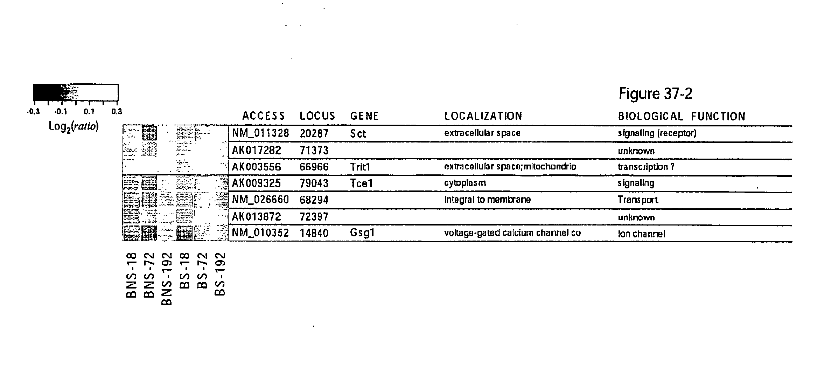

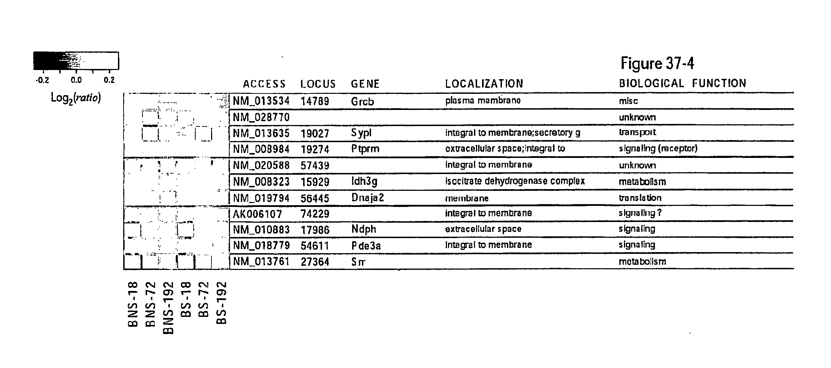

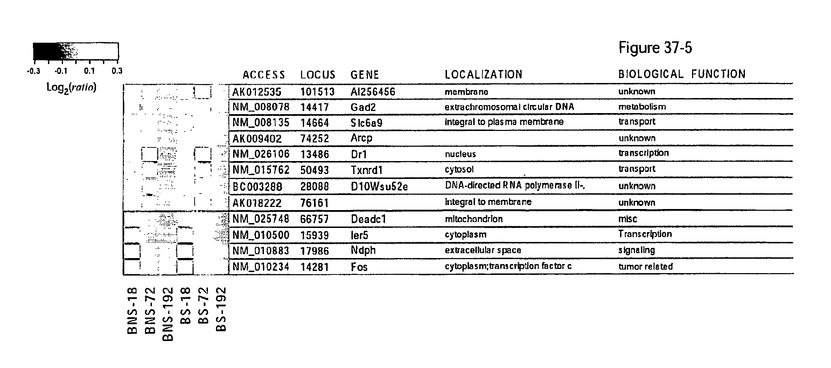

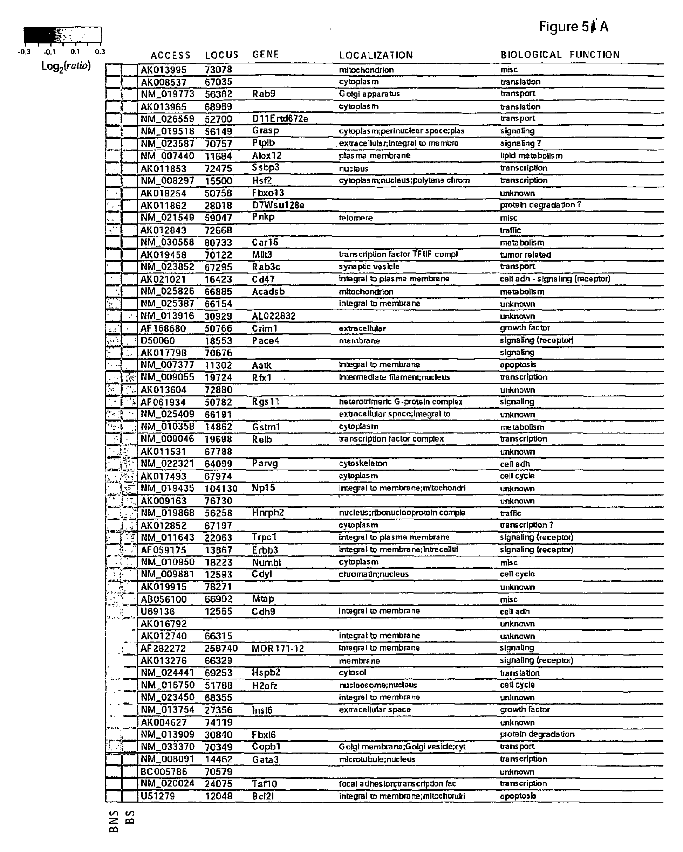

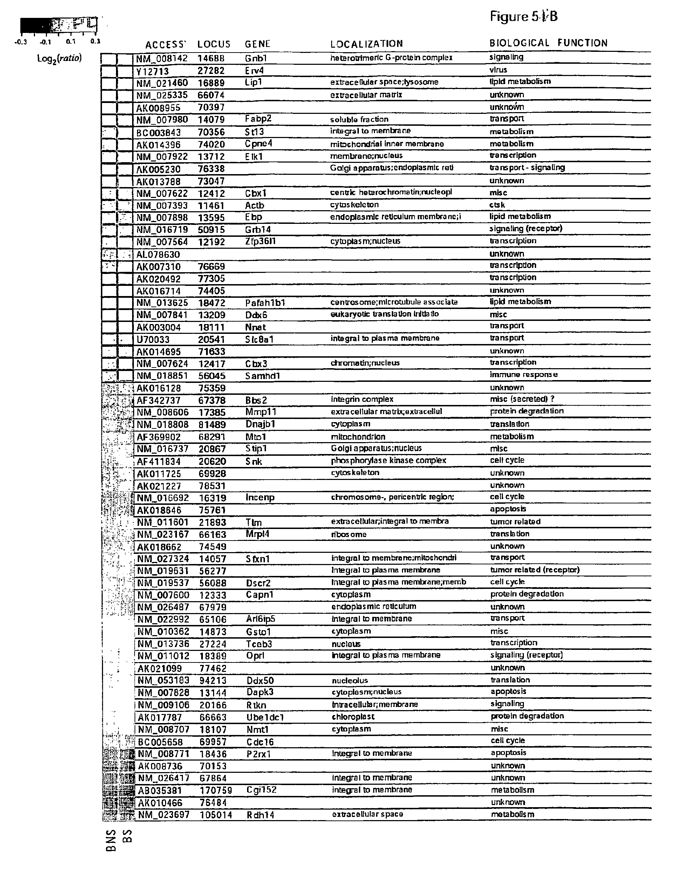

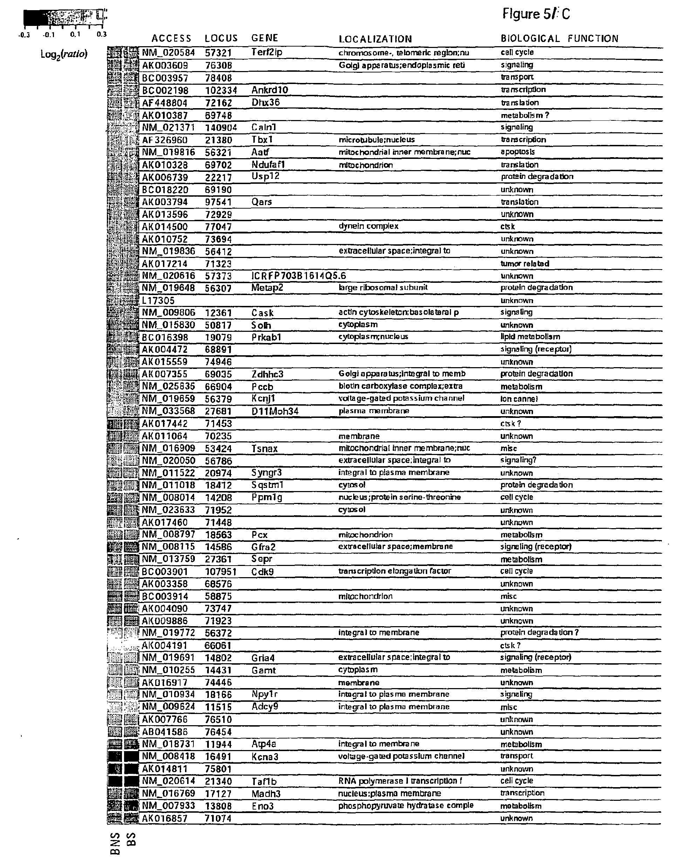

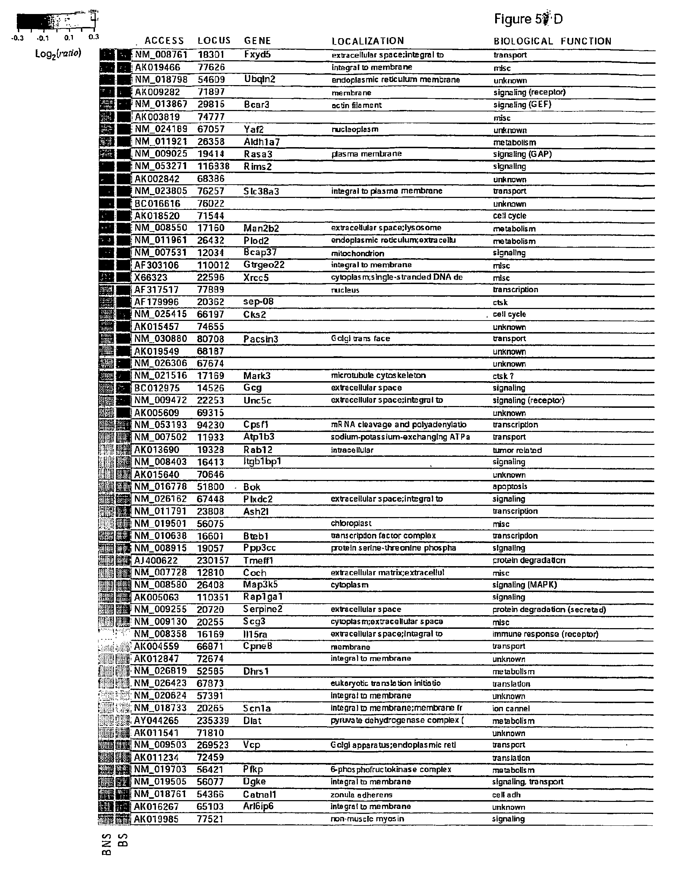

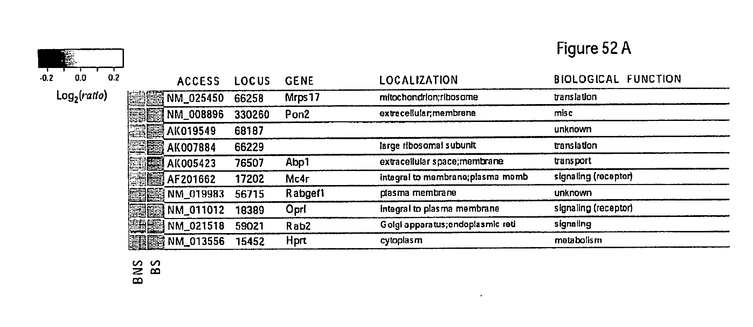

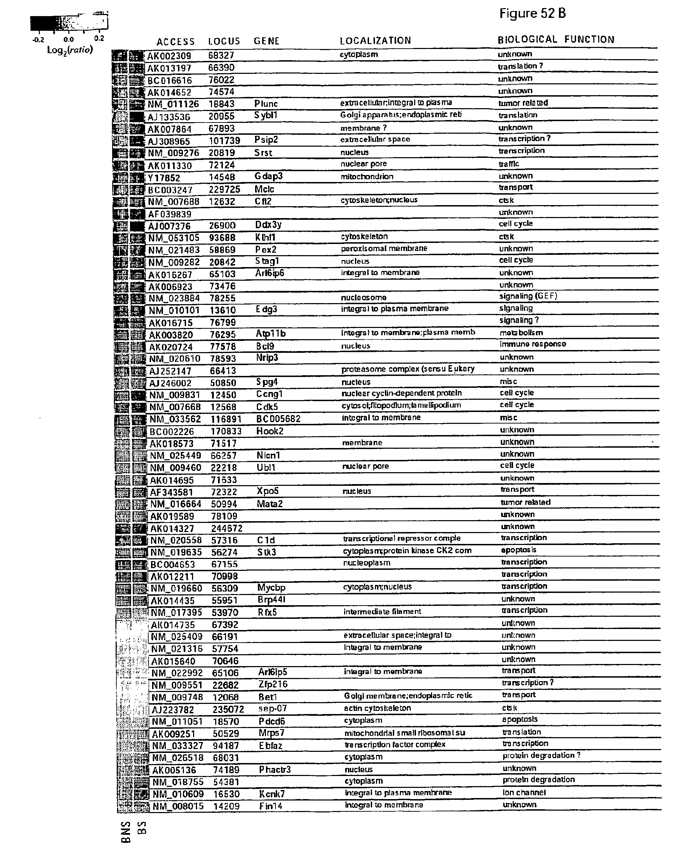

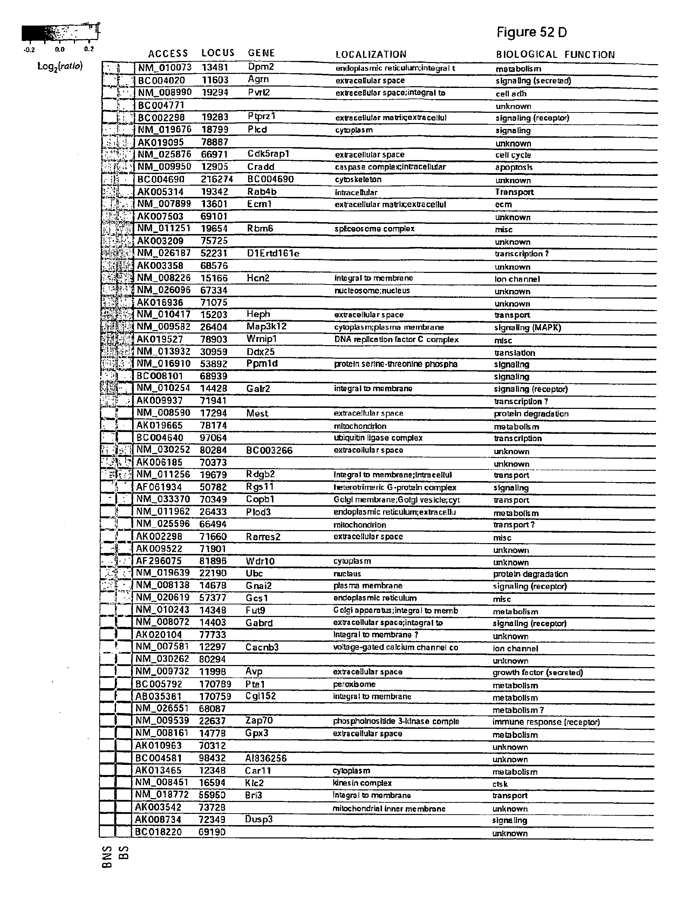

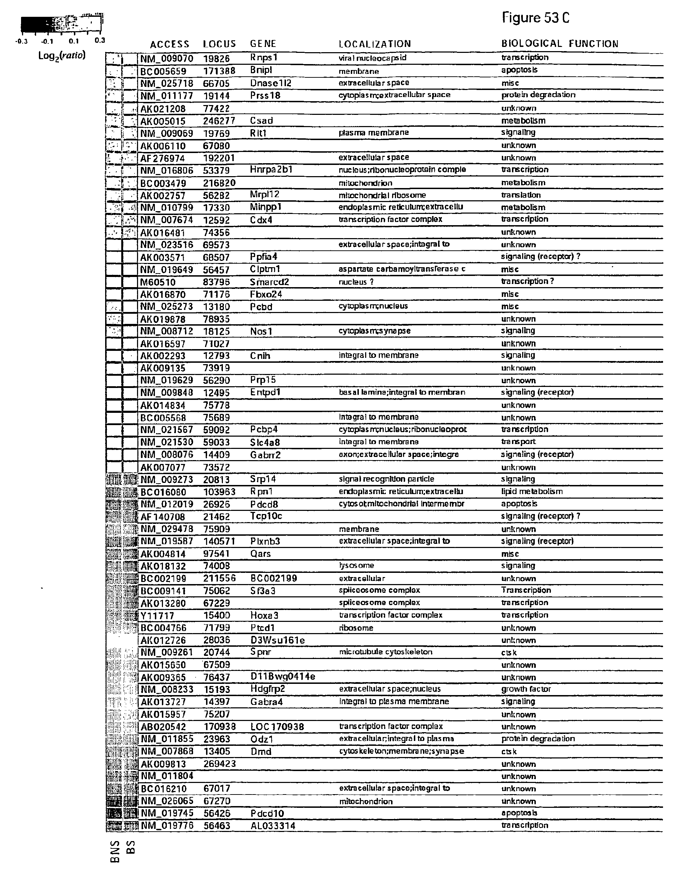

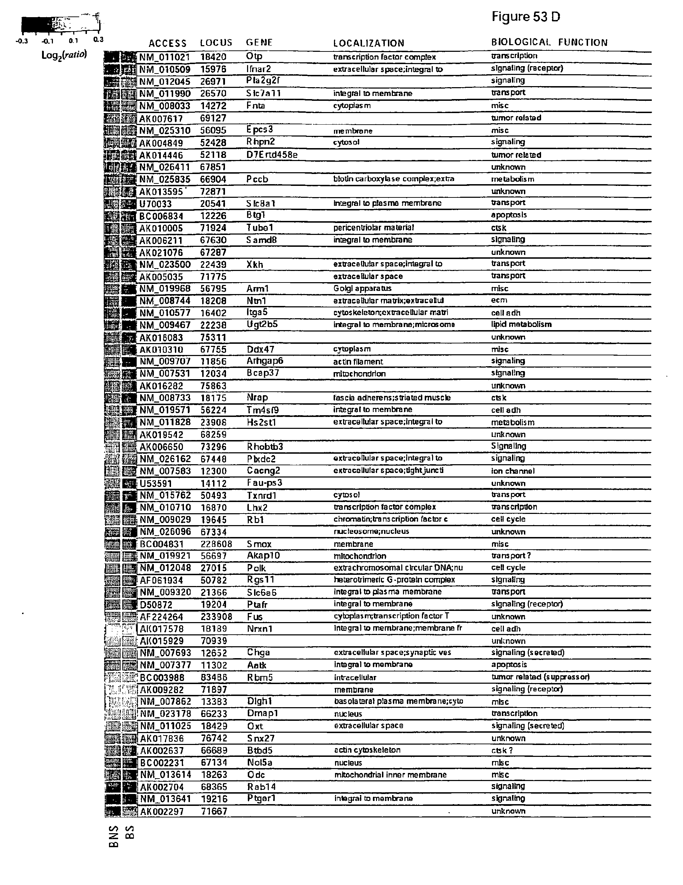

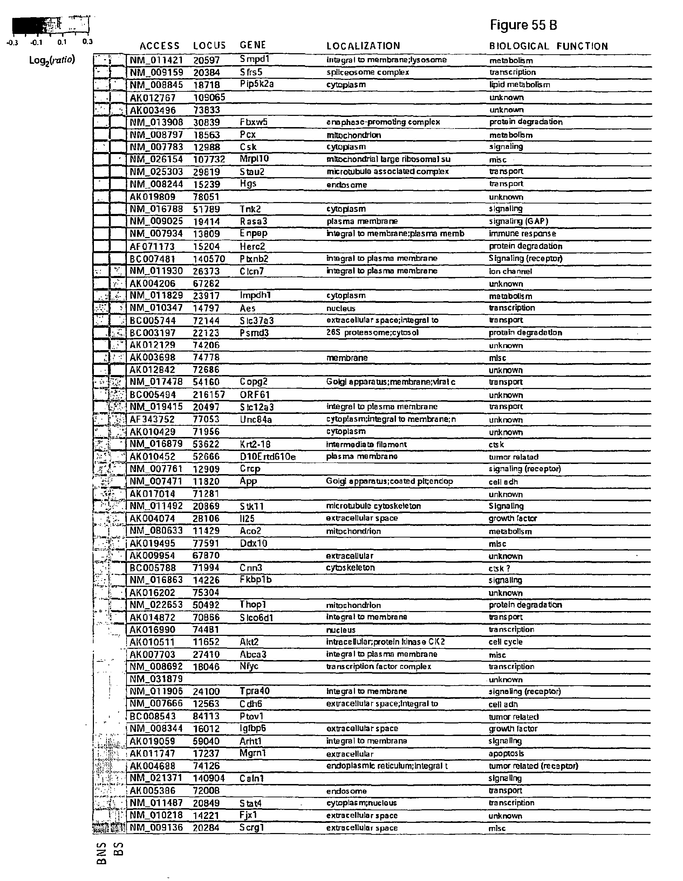

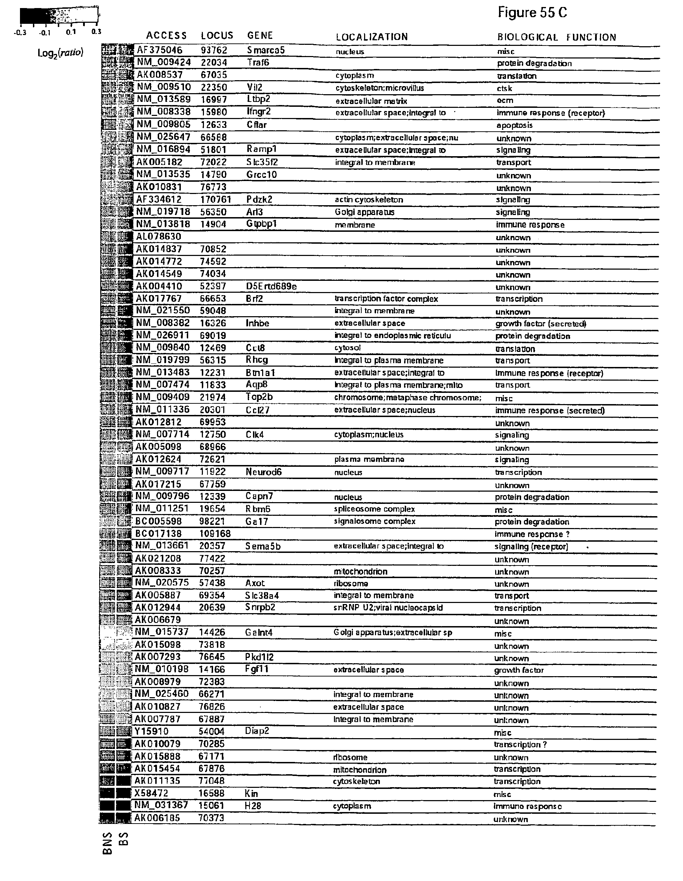

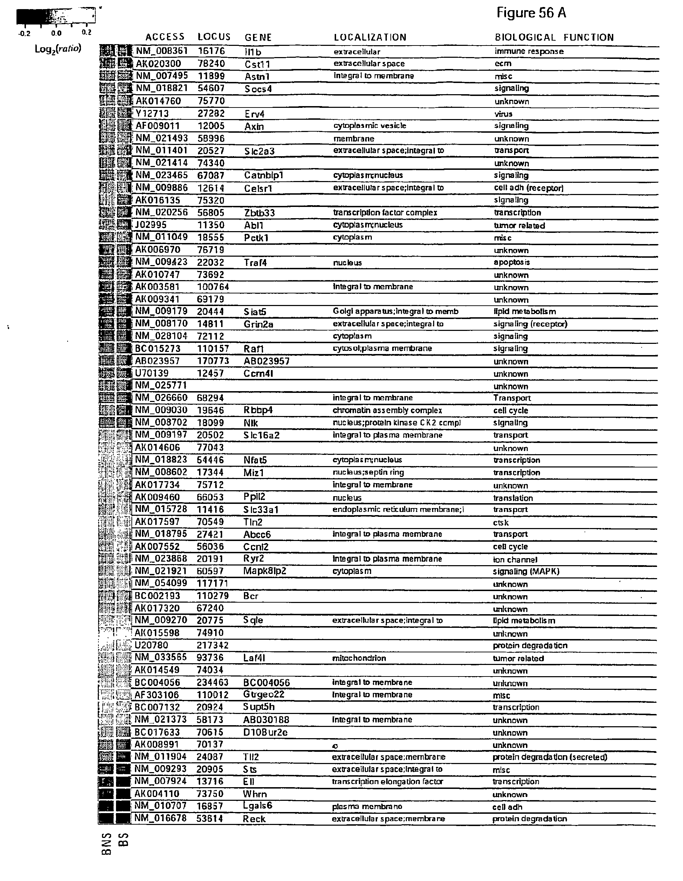

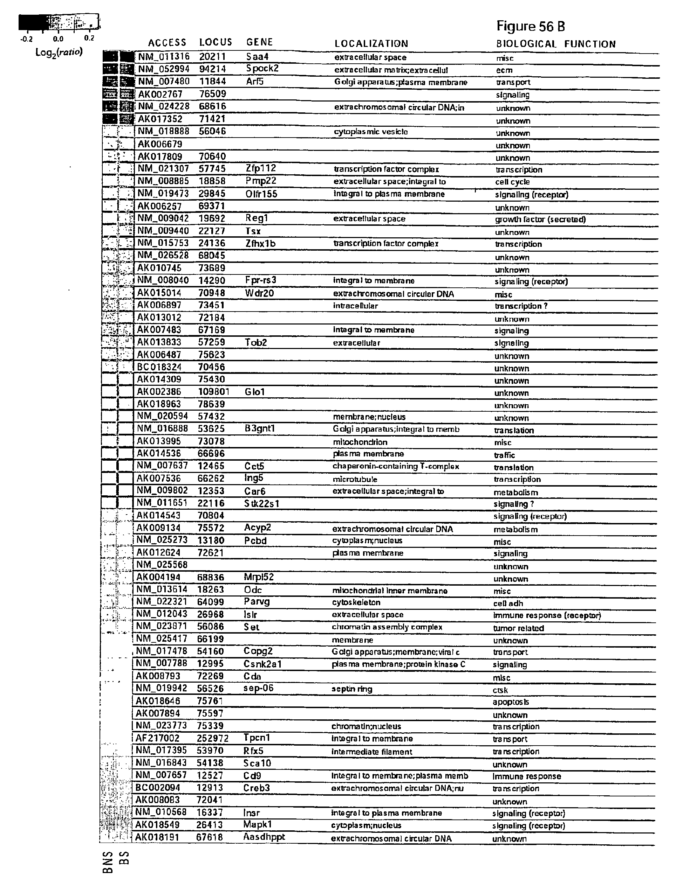

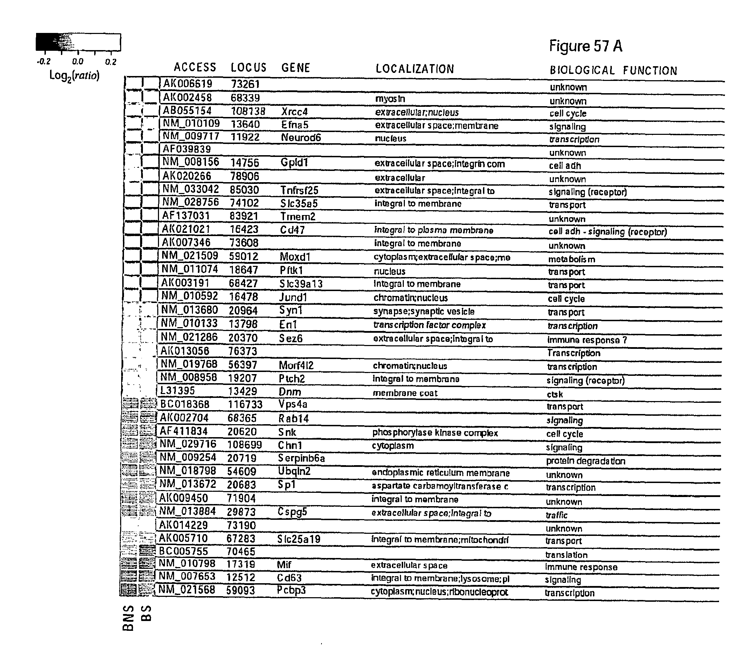

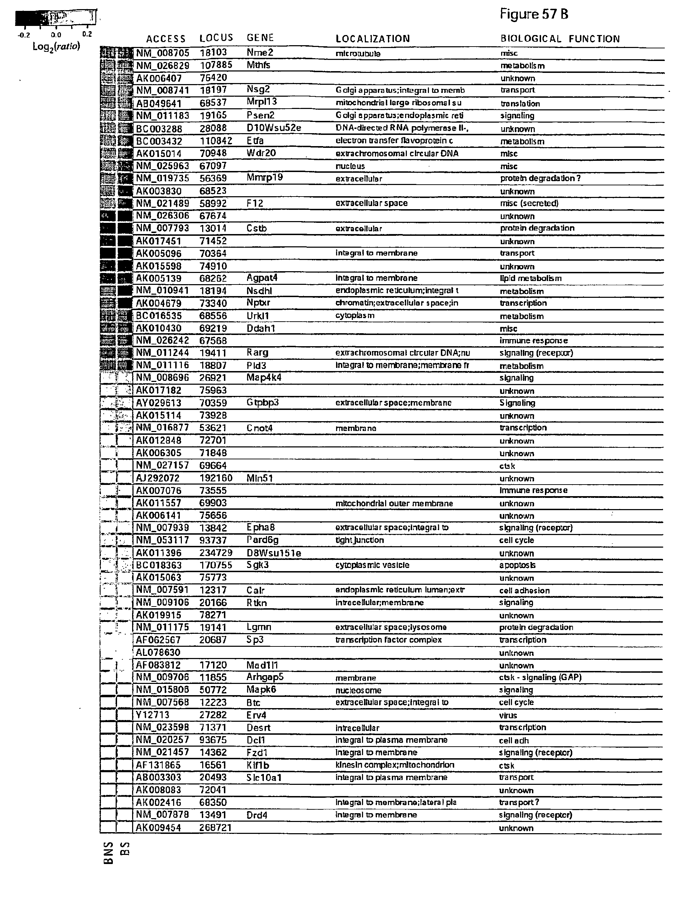

Breast Cancer: Nedd8 (FIG. 29-1), Co14a3bp (FIG. 29-2), Bgn (FIG. 29-4), Sox5 (FIG. 29-5), Slc38a4 (FIG. 32-1), Tom1 (FIG. 32-2), Calr (FIG. 32-4), Itgae (FIG. 32-5),
Ttrap (FIG. 35-1), P ex11b (FIG. 35-2), Sema7a (FIG. 35-4), and Stam2 (FIG. 35-5);
Colon Cancer: Nmb (FIG. 30-1), Ryr2 (FIG. 30-2), Trfr (FIG. 30-4), Mfap5 (FIG. 30-5), Prrg2 (FIG. 33-1), Faim (FIG. 33-2), Mum1 (FIG. 33-4), Stch (FIG. 33-5), Lhb (FIG. 36-1), Prm3 (FIG. 36-2), Crry (FIG. 36-4), and Timp4 (FIG. 36-5);
Lung cancer: Nmb (FIG. 31-1), Pcdh8 (FIG. 31-2), Rock2 (FIG. 31-4), Angptl3 (FIG. 31-5), Sqstm1 (FIG. 34-1), Kcnip2 (FIG. 34-2), Oxt (FIG. 34-4), Myh4 (FIG. 34-5), Enc1 (FIG. 37-1), Gsg1 (FIG. 37-2), Srr (FIG. 37-4), and Ndph (FIG. 37-5);
Arthritis: Bcl21 (FIG. 51A), P2rx1 (FIG. 51B), Pafah1b1 (FIG. 51B), Kcna3 (FIG. 51C), Taf1b (FIG. 51C), Slc38a3 (FIG. 51D), Hprt (FIG. 52A), C1d (FIG. 52B), Car1 (FIG. 52D), Dusp3 (FIG. 52D), Gabrr2 (FIG. 53C), and Aatk (FIG. 53D); and
Asthma: Rasa3 (FIG. 55B), Tnk2 (FIG. 55B), H28 (FIG. 55C), Diap2 (FIG. 55C), Lgals6 (FIG. 56A), Reck (FIG. 56A), Whrn (FIG. 56A), Stk22s1 (FIG. 56B), CD47 (FIG. 57A), Jund1 (FIG. 57A), Cstb (FIG. 57B), and Desrt (FIG. 57B).

In another embodiment, the invention includes methods of identifying a disease surveillance gene for non-CNS disorders in a human, by inducing a non-CNS disorder in a test experimental non human animal; comparing expression of a gene in a CNS sample from the test experimental animal to expression of the gene in a CNS sample from a control experimental animal; and selecting as a disease surveillance gene a human homolog of a gene that is differentially expressed in the CNS sample from the test experimental animal compared to the CNS sample from the control experimental animal. In some embodiments, a non-CNS neoplasm is induced by chemical or radiation mutagenesis, or by administering a neoplastic cell to the experimental animal, and the experimental animal is an animal model (e.g., a mouse or non-human primate) of rheumatoid arthritis, diabetes, asthma, obesity, or diabetes.

In the new systems and methods, the subject can lack a clinical sign of a disorder as evaluated by imaging analysis, can have a family history of the disorder, and/or can be a carrier of a gene associated with an increased risk of developing the disorder (such as the BRCA1, BRCA2, hMSH2, hMLH1, or hMSH6 gene).

In another aspect, the invention features methods of generating a reference gene expression profile of one or more genes that are differentially expressed in a CNS sample of a mammal having a specific non-CNS disorder, by obtaining a control mammal not having the specific non-CNS disorder; obtaining a diseased mammal of the same type as the control mammal that has the specific non-CNS disorder; obtaining a first CNS sample from the control mammal and a second CNS sample from the diseased mammal; generating a first gene expression profile from the first CNS sample and a second genetic expression profile from the second CNS sample; comparing the first and second genetic expression profiles; selecting a set of genes from the second genetic expression profile that are differentially expressed; and preparing the reference gene expression profile from expression data from the selected genes.

The invention also features, e.g., in electronic digital or analog format a reference gene expression profile corresponding to the presence of a non-central nervous system (non-CNS) disorder in a mammal, comprising expression data of 5 or more genes, wherein each of the 5 or more genes is differentially expressed in a central nervous system (CNS) sample of a mammal having a specific non-CNS disorder compared to the same 5 or more genes in a mammal not having the specific non-CNS disorder.

The invention also includes methods of treating a subject by diagnosing a non-central nervous system (non-CNS) disorder according to the methods or using the systems described herein; and administering to the subject a therapeutic agent for the disorder. For example, the therapeutic agent can be a chemotherapeutic agent, such as an antitubulin/antimicrotubule drug, a topoisomerase I inhibitor, an antimetabolite, and an alkylating agent.

In another aspect, the invention features methods of determining whether a subject (e.g., a human) has, or is at risk for developing, a peripheral (non-CNS) disorder. The method involves providing or obtaining a test gene expression profile for one, two, or more CNS genes in the subject; and comparing the test gene expression profile with a reference gene expression profile (e.g., a reference gene expression profile described herein), wherein the reference gene expression profile is associated with the presence of a particular non-CNS disorder. Non-limiting examples of reference gene expression profiles (e.g., associated with colon, breast or lung carcinoma), are disclosed herein. In one embodiment, the method includes generating a record of the result (e.g., a laboratory record or dataset) of the comparing step; and, optionally, transmitting the record (e.g., by print or computer readable material) to the subject, the subject's health care provider or another party. As with other methods described herein, various techniques can be used to provide a gene expression profile and various types of disorder scan be detected.

The methods described herein are useful, *inter alia,* for risk assessment for a variety of disorders, for early detection and diagnosis of disease, for monitoring of progression of disease, for monitoring efficacy of treatment for a disease, and/or evaluation of clinical status.

As used herein a "disorder" or "disease" is an alteration in the state of the body or of some of its cells, tissues, or organs, that threatens health. The two terms are meant to encompass all stages of an illness, including the very early stages of an illness (e.g., early alterations in the body that may not be detectable by the subject or a health care provider, but nonetheless set in motion a disease process). For example, the terms "disorder" and "disease" encompass the state of neoplasia, before a neoplasm or tumor is formed; early immunological reactions to an antigen, e.g., in the development of rheumatoid arthritis or asthma, before inflammation or allergy are symptomatic; and early changes in energy metabolism that promote weight gain, before weight gain is produced.

As used herein, "neoplasia" is an unregulated and progressive proliferation of cells under conditions that would not elicit, or would cause cessation of, proliferation of normal cells. Neoplasia can result in the formation of a "neoplasm," a new and abnormal growth of tissue. If the abnormally proliferating cells form a mass, a neoplasm is generally referred to as a "tumor." A neoplasm may be benign or malignant (cancerous).

As used herein, the term "matches", "matching" or "match" if at least 75% of the genes in a test gene expression profile are either up- or down- regulated in the same manner as the genes in the reference expression profile. For example, if genes 1 through 5 are up regulated and genes 6 through 10 are down regulated in the reference expression profile, then a test profile where genes 1 through 10 are down regulated would not be a match, whereas a test profile where genes 1, 2, 3, 4 & 6 are up-regulated and genes 5, 7, 8, 9 & 10 are down-regulated would be a match. A "high level match" would mean that at least 75% of the genes come within at least plus or minus 50% of the expression level (or Log2 ratio of expression level) of the gene in the reference expression profile. For example, in the reference expression profile: for gene A the Log2 ratio of expression level in the presence of a disorder to the expression level in the absence of the disorder is +0.4; for gene B the ratio is -0.4; for gene C the ratio is +0.2; and for gene D the ratio is -0.2. A test profile with the following values (A = +0.3; B = □0.3; C = +0.1; D = +0.3) is a high level match because genes A, B, C in the test profile (75% of the genes in the reference profile) are within $\pm 50\%$ of the ratios for those genes in the reference profile.

A "subject" is a human or animal that is tested for the presence of a possible disorder. The animal can be a mammal, e.g., a domesticated animal such as a dog, cat, horse, pig, cow or goat; an experimental animal such as an experimental rodent (e.g., a mouse, rat, guinea pig, or hamster); a rabbit; or an experimental primate, e.g., a chimpanzee or monkey.

Although methods and materials similar or equivalent to those described herein can be used in the practice or testing of the present invention, suitable methods and materials are described below.

## BRIEF DESCRIPTION OF THE DRAWINGS

FIGs. 1-1 to 1-35 are a table showing all the cancer disease surveillance genes (differentially expressed at p < 0.01) identified in prefrontal cortex, hypothalamus, and midbrain of relevant animal models for breast, colon, and lung carcinoma. Data corresponds to genes differentially expressed in mice harboring tumors compared to control mice. Samples correspond to 18, 72, and 192 hours post tumor cell injection.

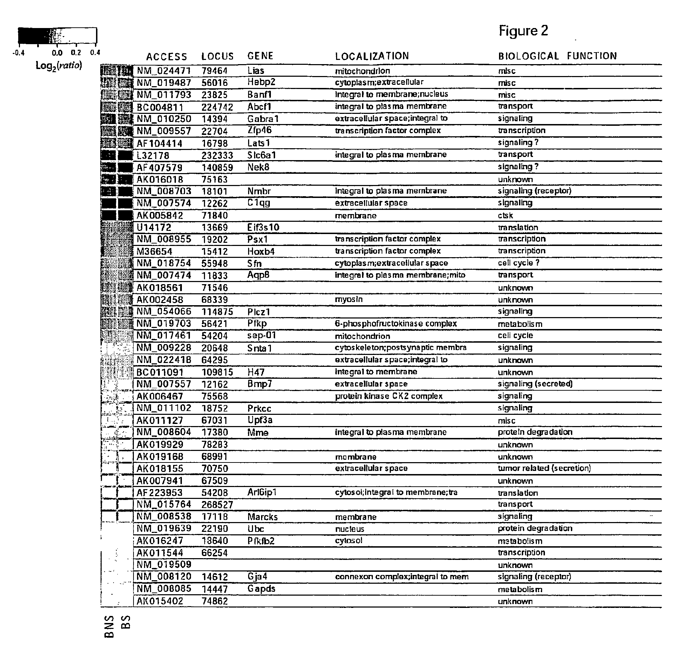

The following figures 2 to 28 are tables showing the differentially expressed genes (p < 0.01) in either the prefrontal cortex, the hypothalamus, or the midbrain of mice harboring either breast, lung or colon carcinoma. Samples correspond to either 18, 72, or 192 hours post tumor cell injection. Differentially expressed genes were identified by a mixed model ANalysis Of VAriance (ANOVA), with tumor (or control) as fixed effect. The base 2 logarithm of tumor vs. control ratio is shown as a gray scale. BNS corresponds to data obtained without background subtraction, and BS corresponds to data obtained after background subtraction.

FIG. 2 shows the differentially expressed genes in the prefrontal cortex of mice harboring breast carcinoma at 18 hours.

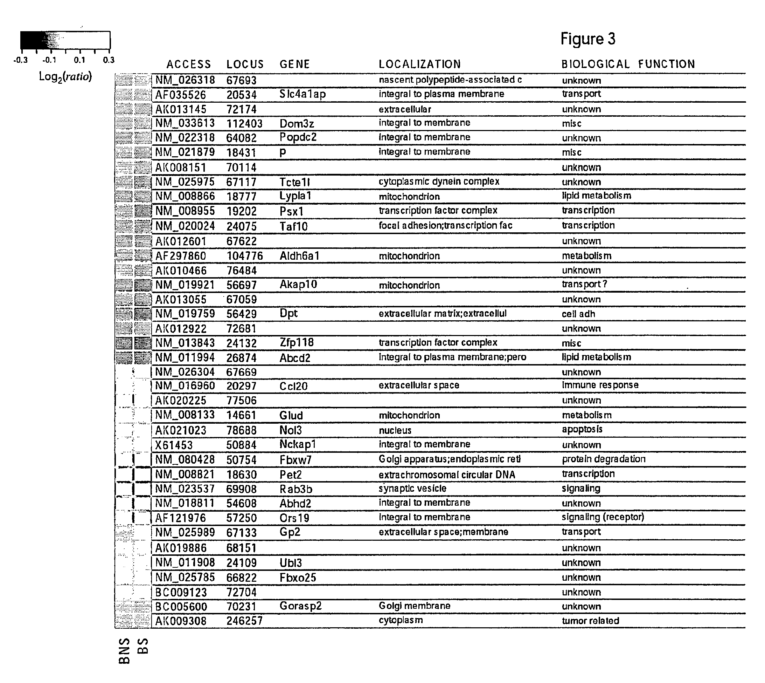

FIG. 3 shows the differentially expressed genes in prefrontal cortex of mice harboring breast carcinoma at 72 hours.

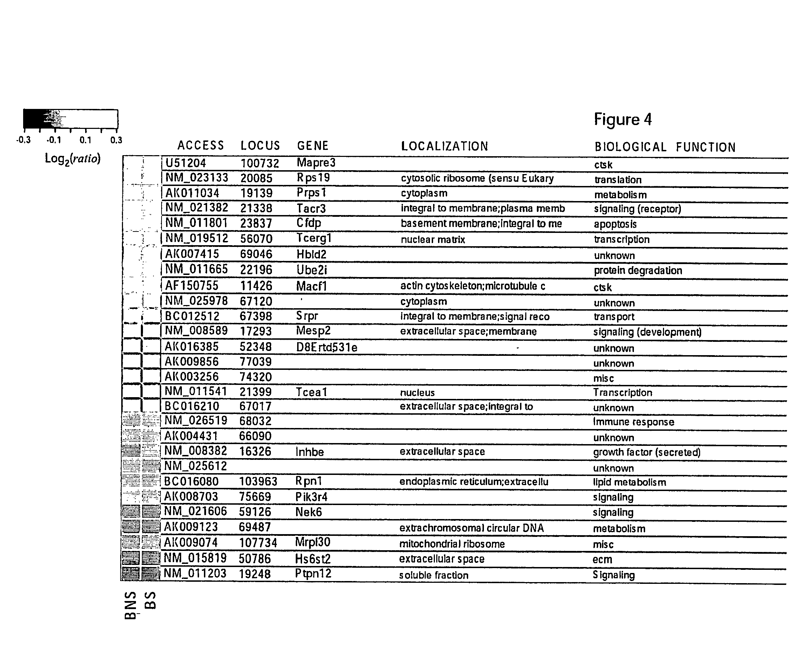

FIG. 4 shows the differentially expressed genes in prefrontal cortex of mice harboring breast carcinoma at 192 hours.

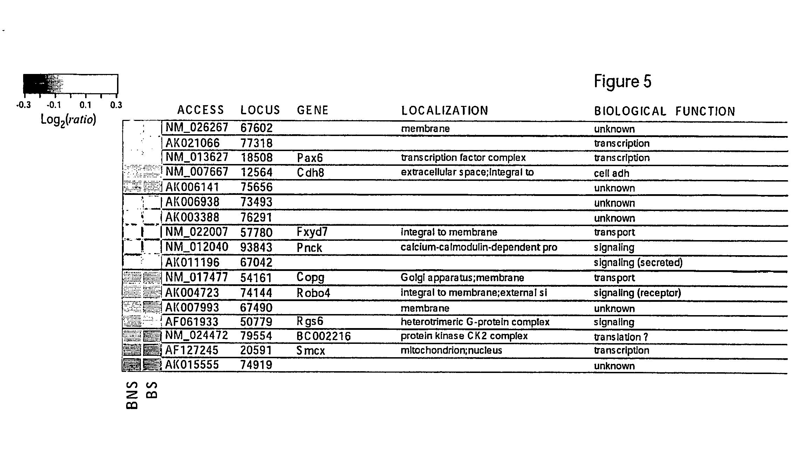

FIG. 5 shows the differentially expressed genes in prefrontal cortex of mice harboring colon carcinoma at 18 hours.

FIG.s. 6A & 6B show the differentially expressed genes in prefrontal cortex of mice harboring colon carcinoma at 72 hours.

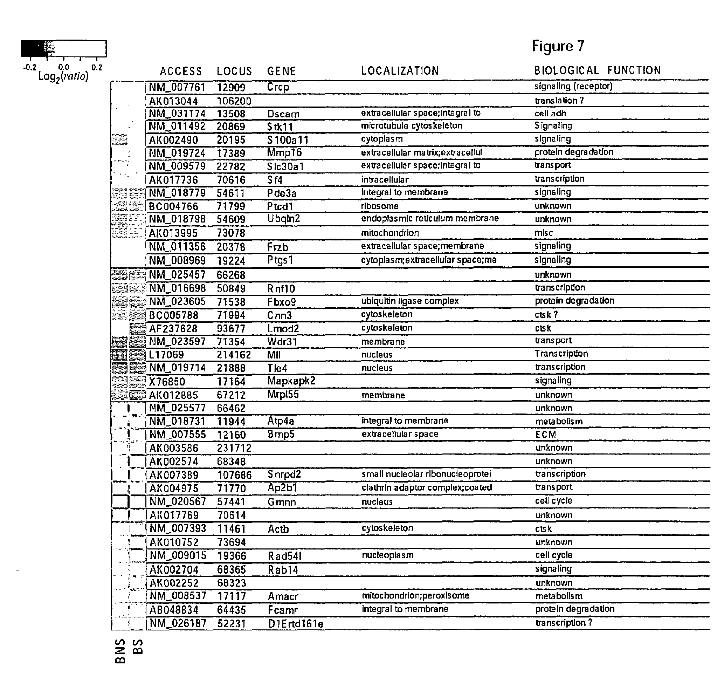

FIG. 7 shows the differentially expressed genes in prefrontal cortex of mice harboring colon carcinoma at 192 hours.

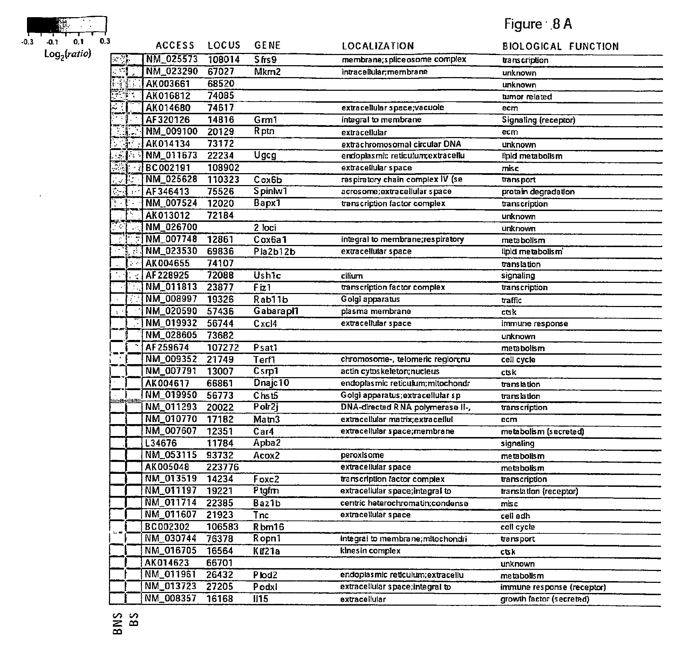

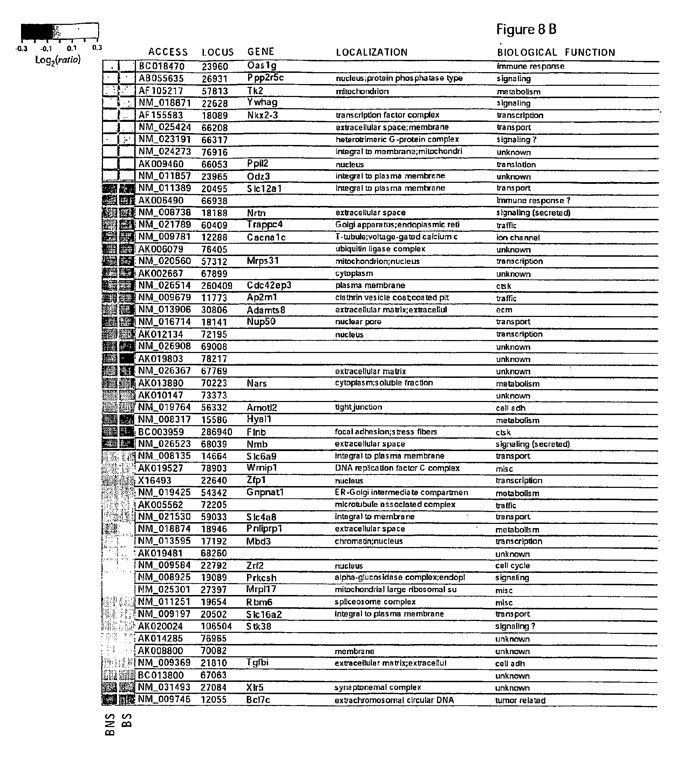

FIGS. 8A & 8B show the differentially expressed genes in prefrontal cortex of mice harboring lung carcinoma at 18 hours.

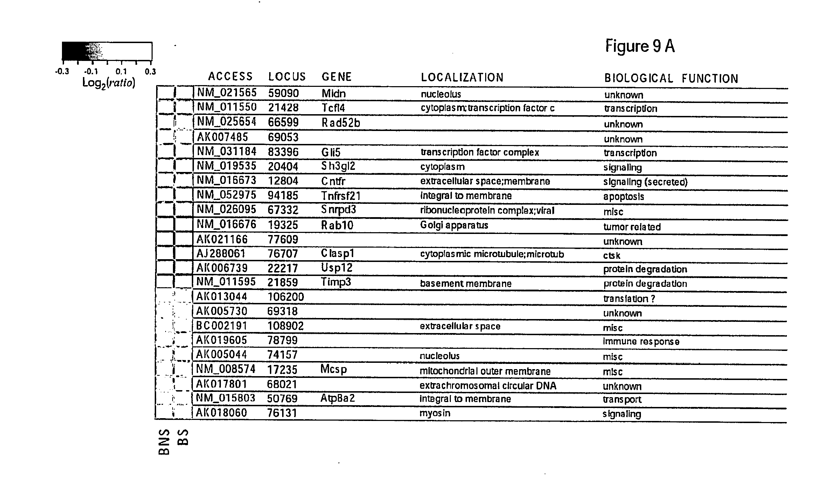

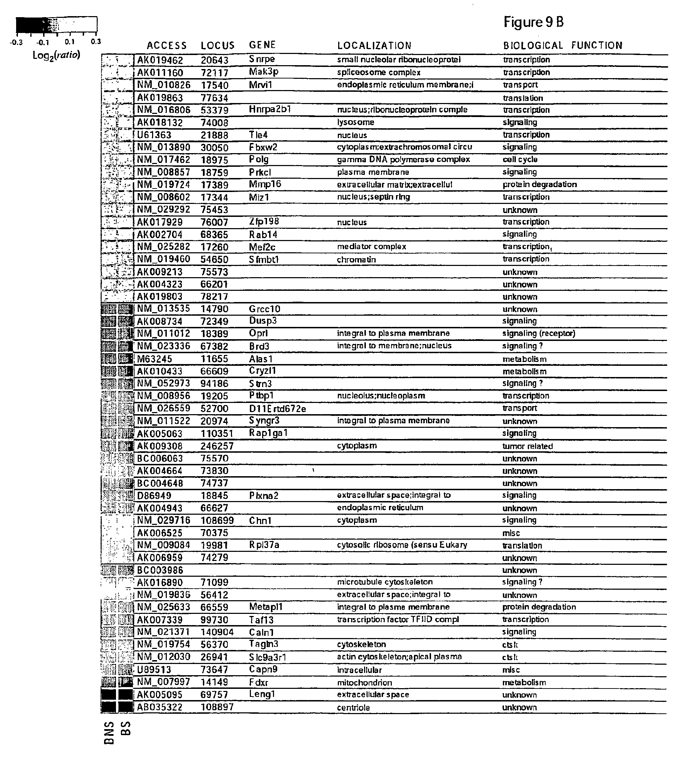

FIGS. 9A & 9B show the differentially expressed genes in prefrontal cortex of mice harboring lung carcinoma at 72 hours.

FIGS . 10A & 10B show the differentially expressed genes in prefrontal cortex of mice harboring lung carcinoma at 192 hours.

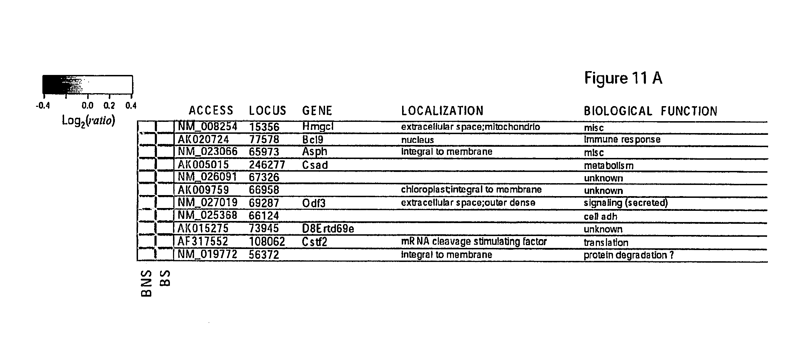

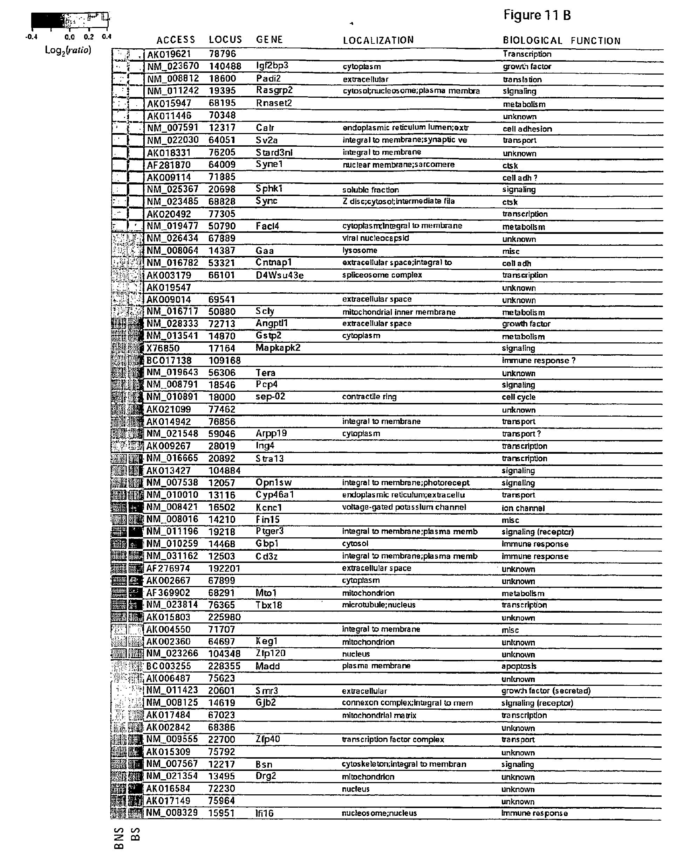

FIGS. 11A & 11B show the differentially expressed genes in hypothalamus of mice harboring breast carcinoma at 18 hours.

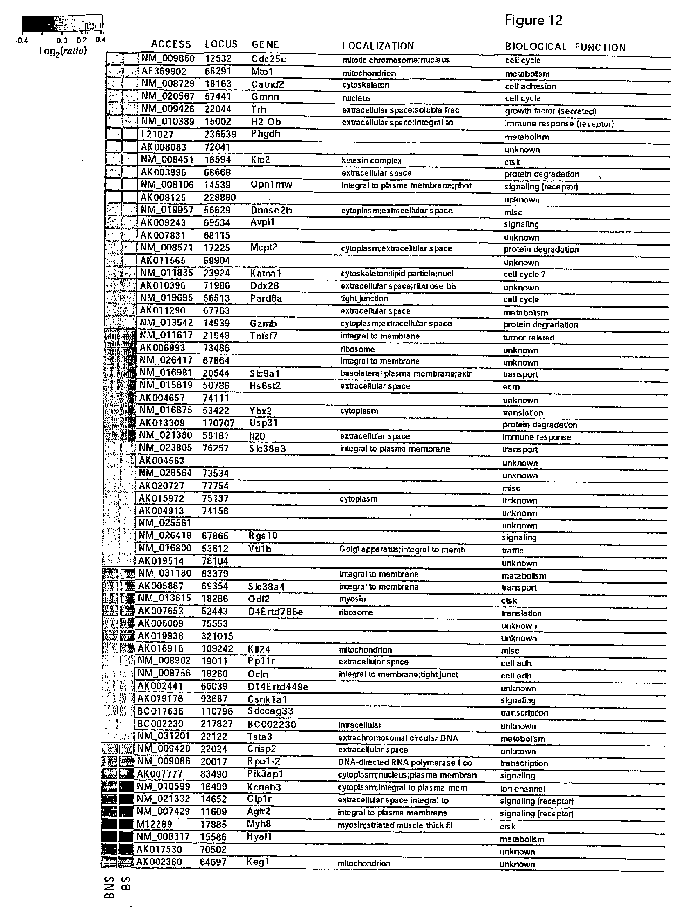

FIG. 12 shows the differentially expressed genes in hypothalamus of mice harboring breast carcinoma at 72 hours.

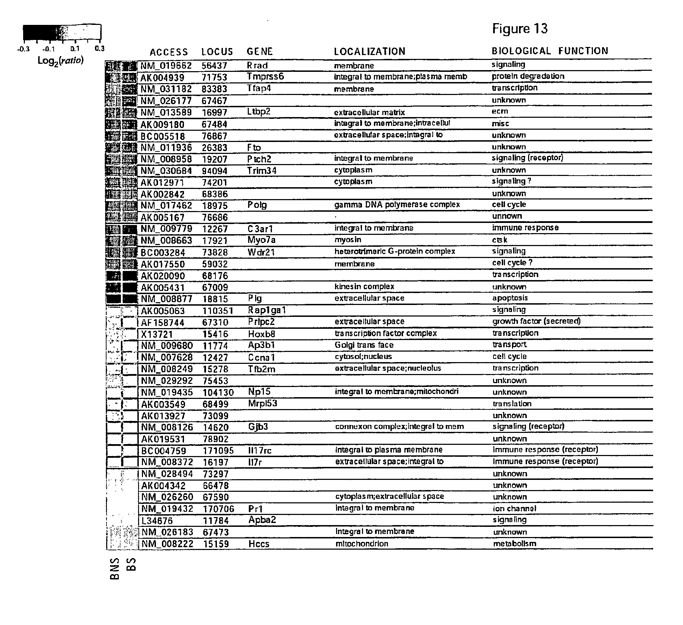

FIG. 13 shows the differentially expressed genes in hypothalamus of mice harboring breast carcinoma at 192 hours.

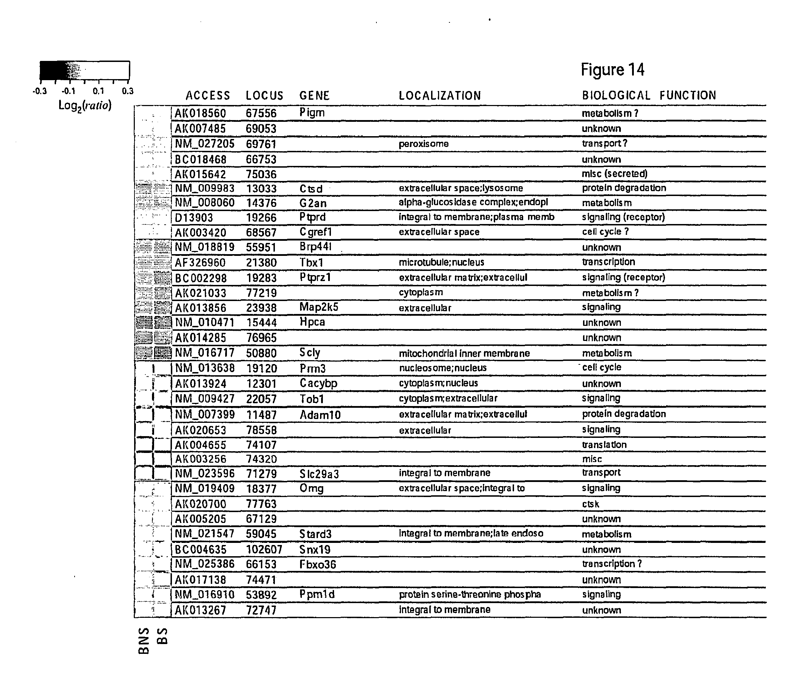

FIG. 14 shows the differentially expressed genes in hypothalamus of mice harboring colon carcinoma at 18 hours.

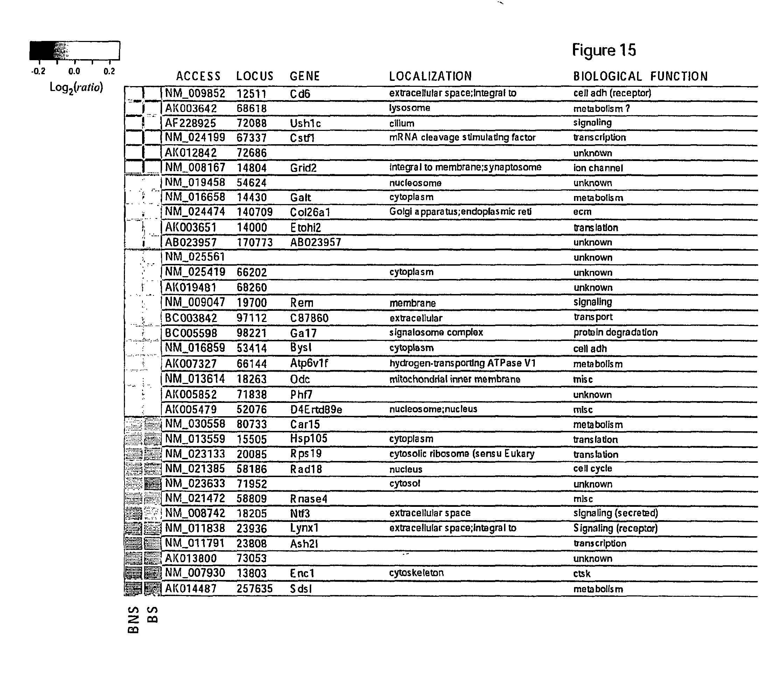

FIG. 15 shows the differentially expressed genes in hypothalamus of mice harboring colon carcinoma at 72 hours.

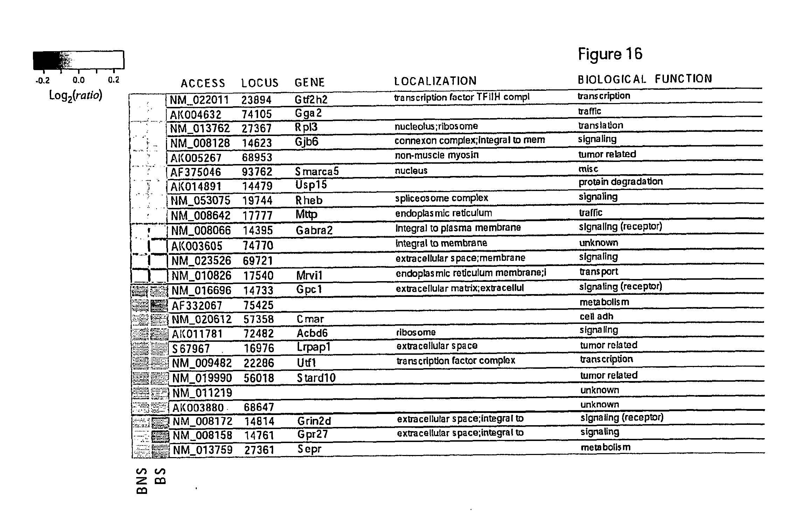

FIG. 16 shows the differentially expressed genes in hypothalamus of mice harboring colon carcinoma at 192 hours.

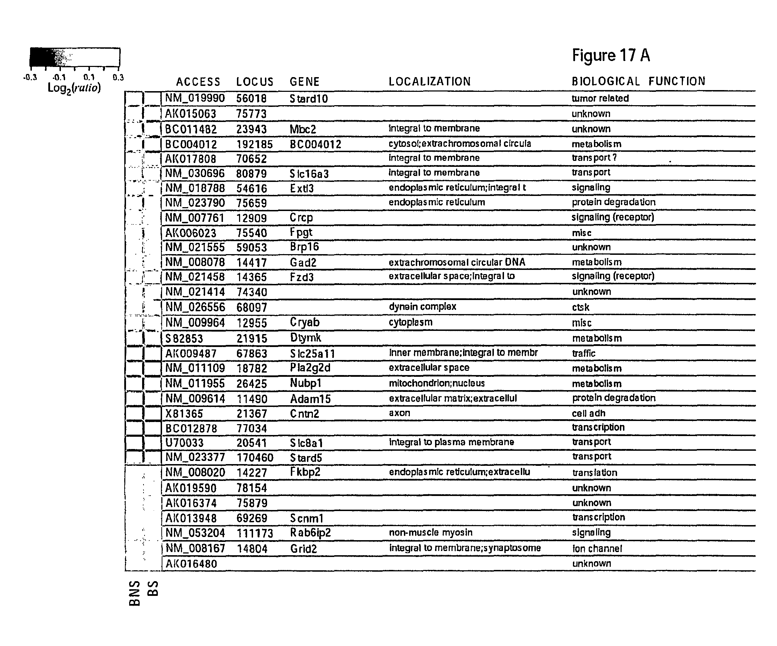

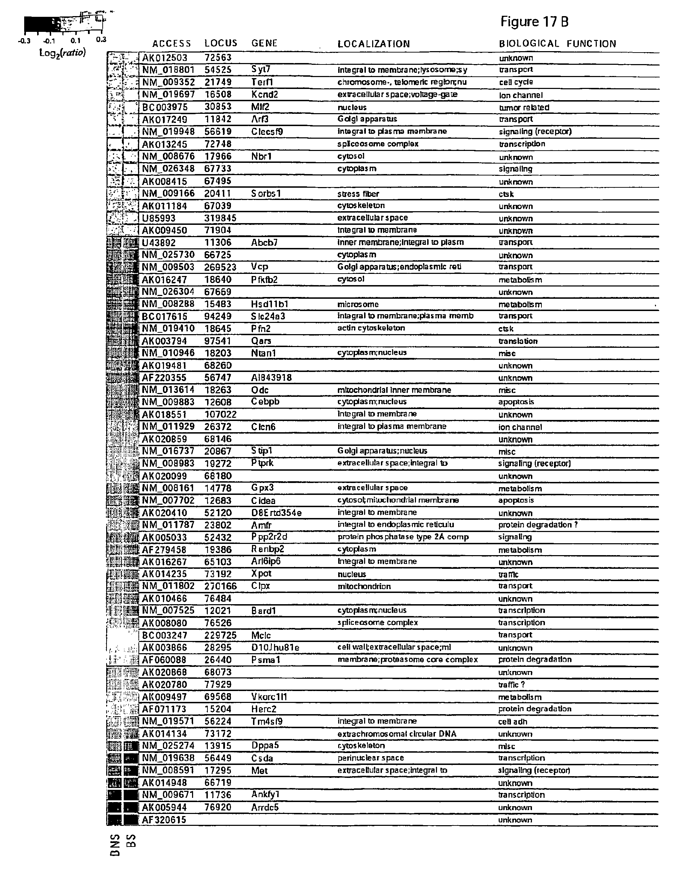

FIGS. 17A & 17B show the differentially expressed genes in hypothalamus of mice harboring lung carcinoma at 18 hours.

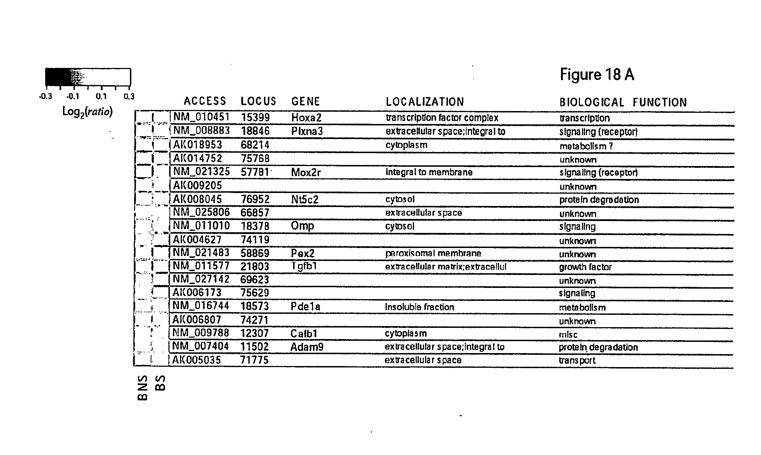

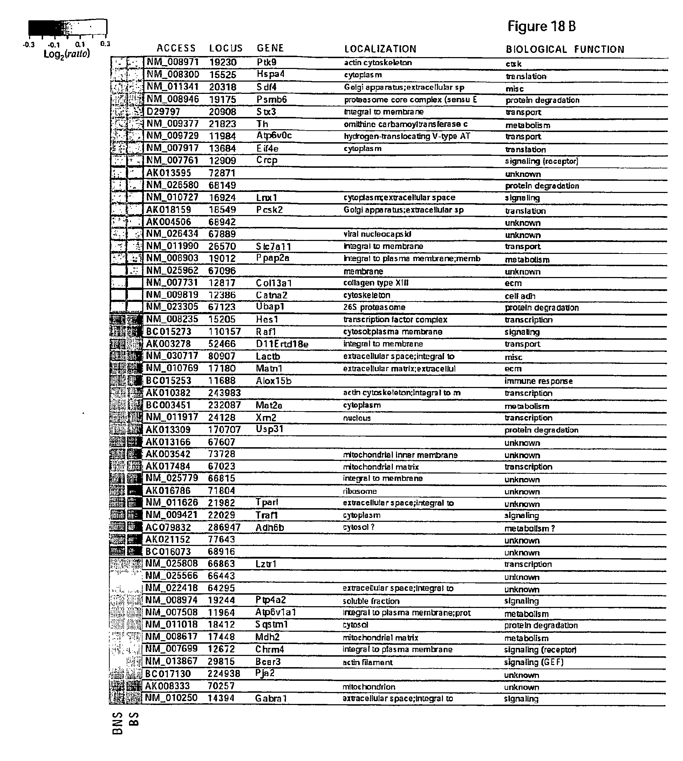

FIGS. 18A & 18B show the differentially expressed genes in hypothalamus of mice harboring lung carcinoma at 72 hours.

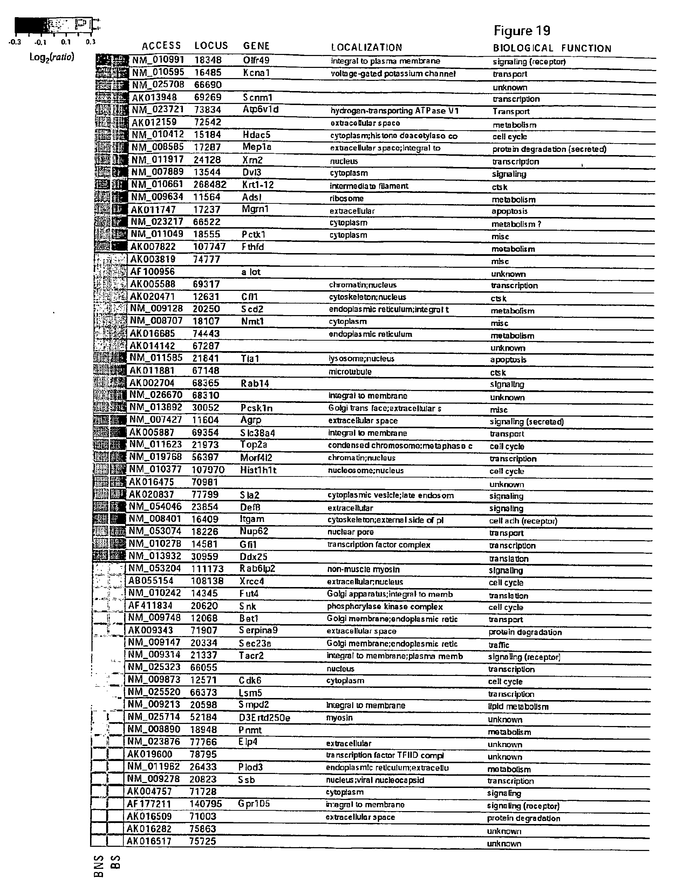

FIG. 19 shows the differentially expressed genes in hypothalamus of mice harboring lung carcinoma at 192 hours.

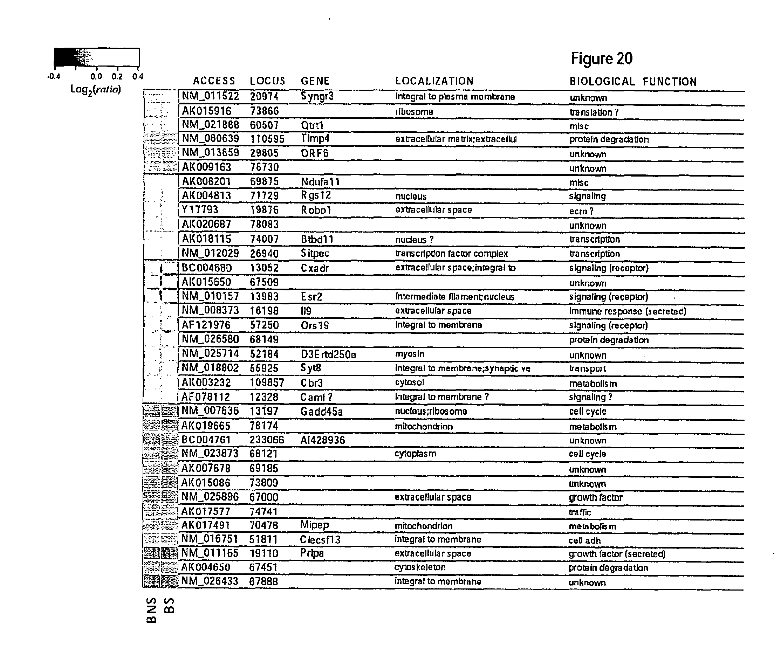

FIG. 20 shows the differentially expressed genes in midbrain of mice harboring breast carcinoma at 18 hours.

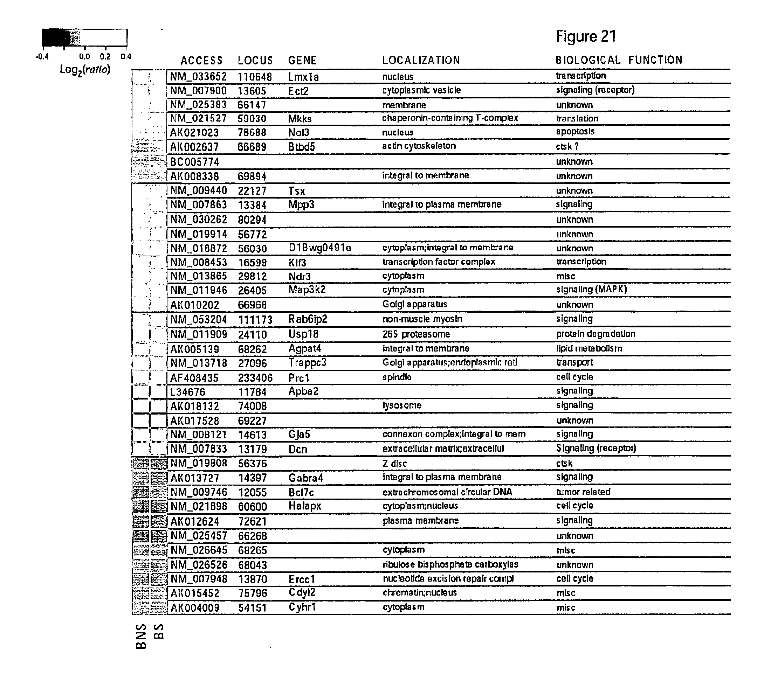

FIG. 21 shows the differentially expressed genes in midbrain of mice harboring breast carcinoma at 72 hours.

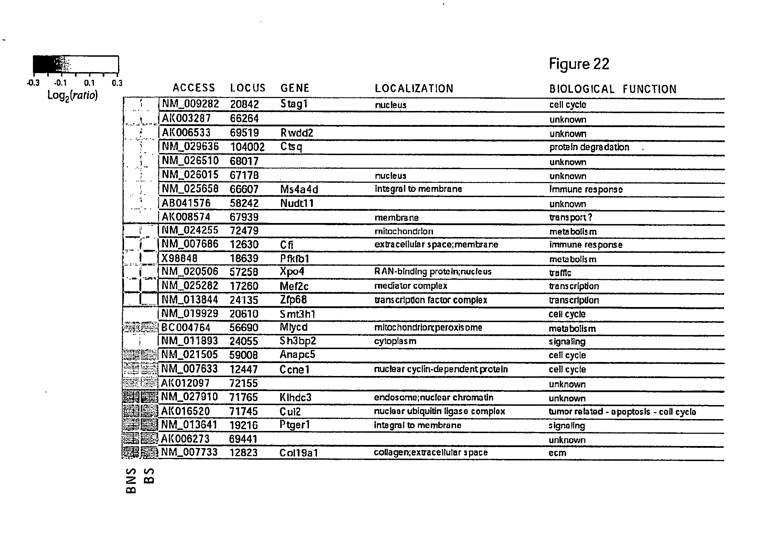

FIG. 22 shows the differentially expressed genes in midbrain of mice harboring breast carcinoma at 192 hours.

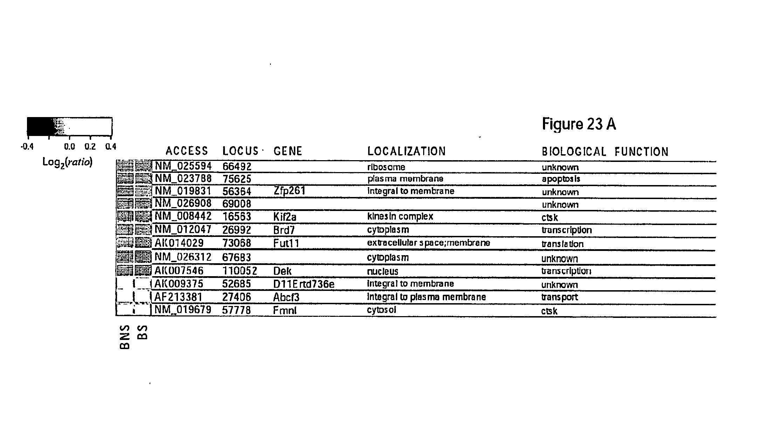

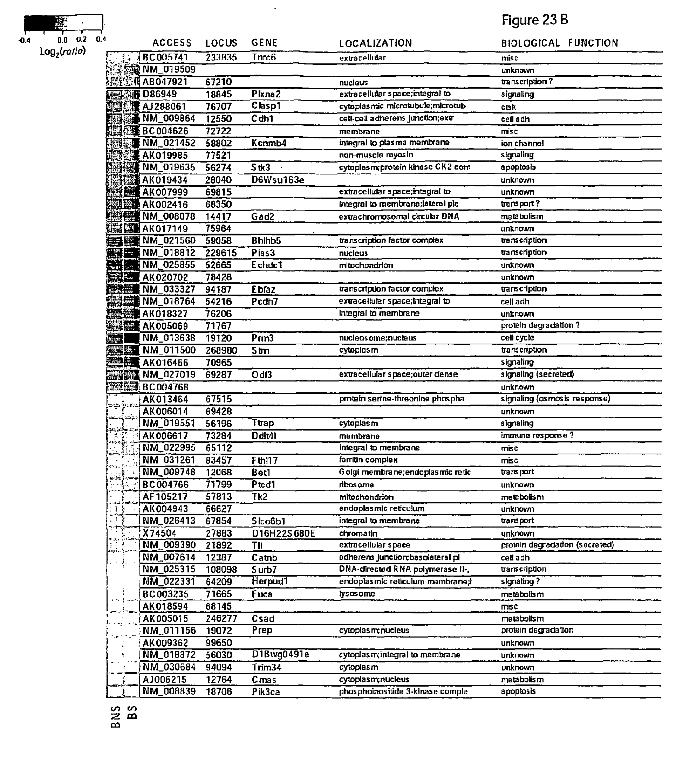

FIGS. 23A & 23B show the differentially expressed genes in midbrain of mice harboring colon carcinoma at 18 hours.

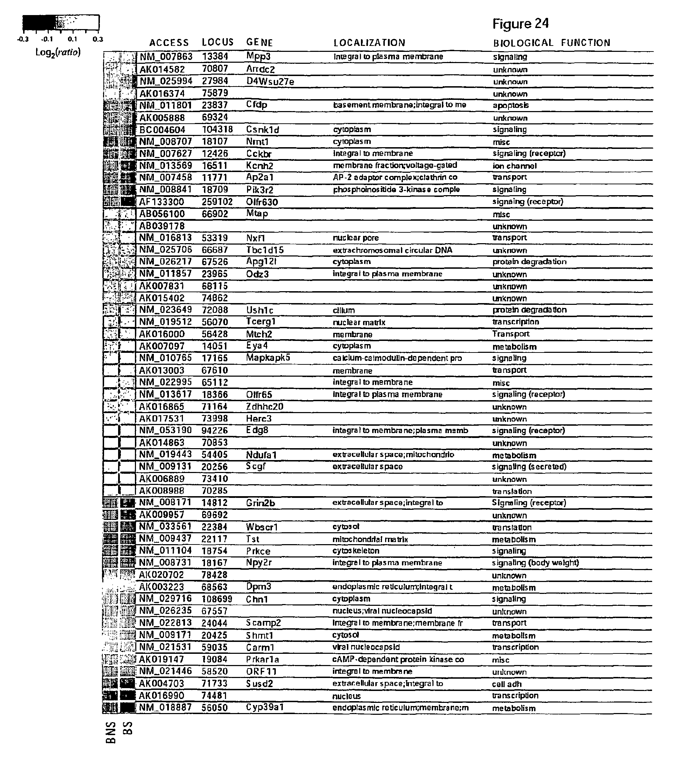

FIG. 24 shows the differentially expressed genes in midbrain of mice harboring colon carcinoma at 72 hours.

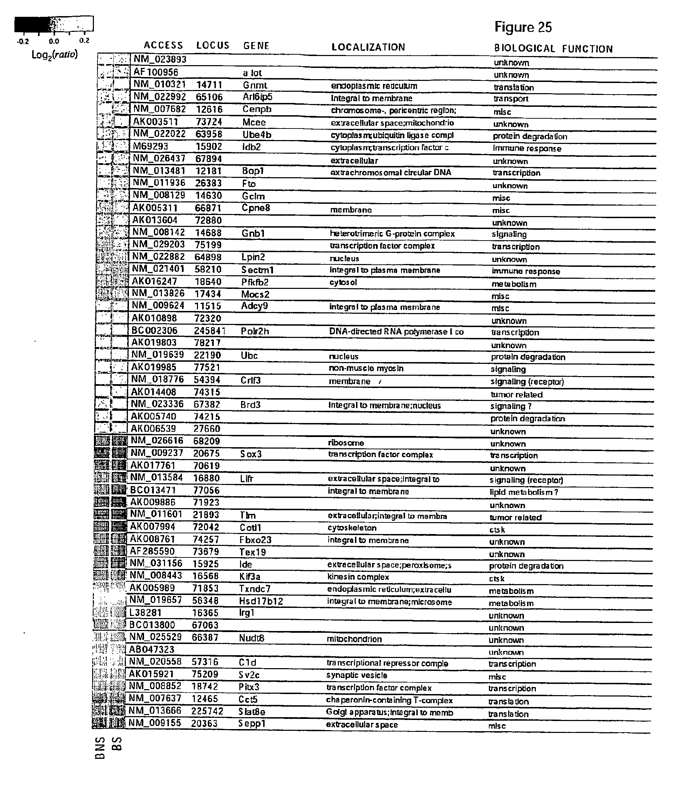

FIG. 25 shows the differentially expressed genes in midbrain of mice harboring colon carcinoma at 192 hours.

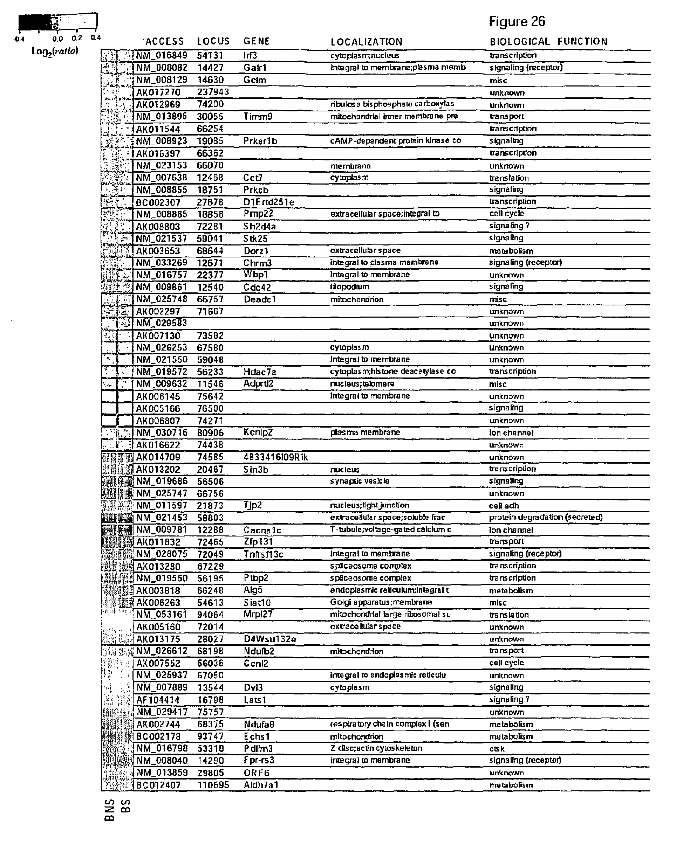

FIG. 26 shows the differentially expressed genes in midbrain of mice harboring lung carcinoma at 18 hours.

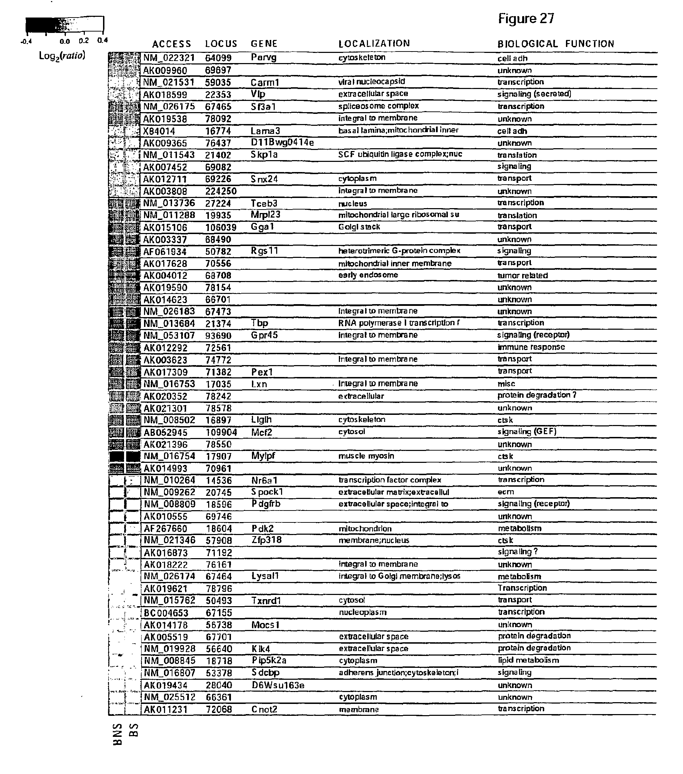

FIG. 27 shows the differentially expressed genes in midbrain of mice harboring lung carcinoma at 72 hours.

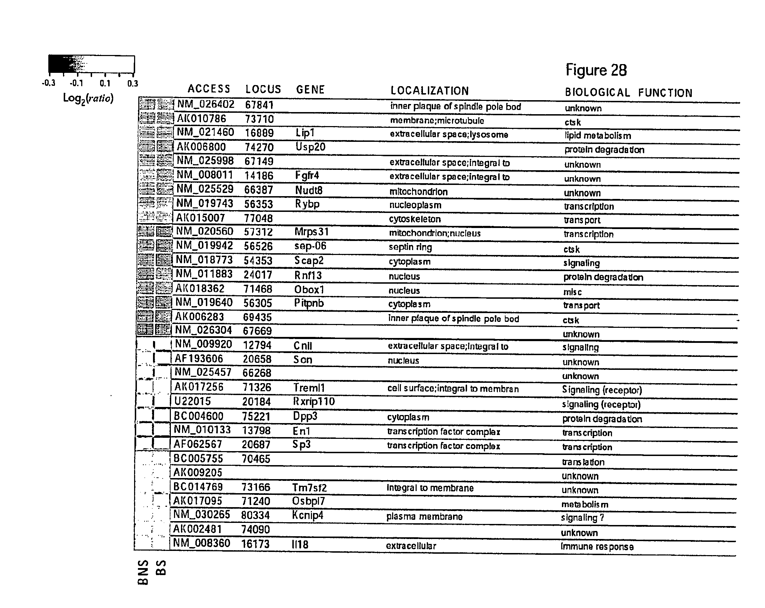

FIG. 28 shows the differentially expressed genes in midbrain of mice harboring lung carcinoma at 192 hours.

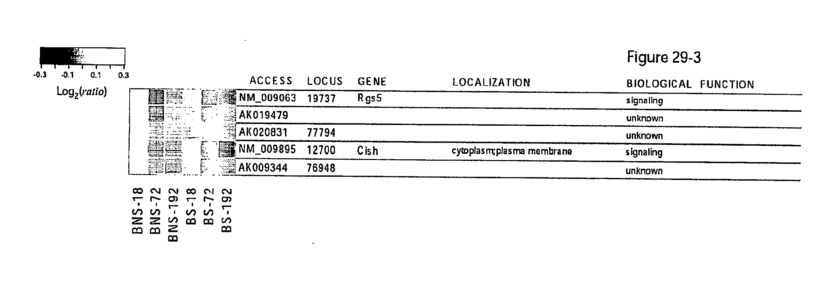

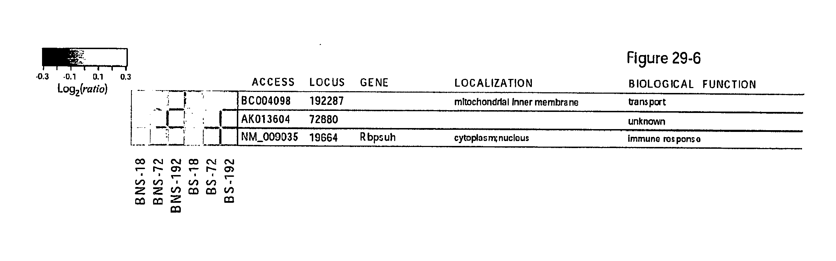

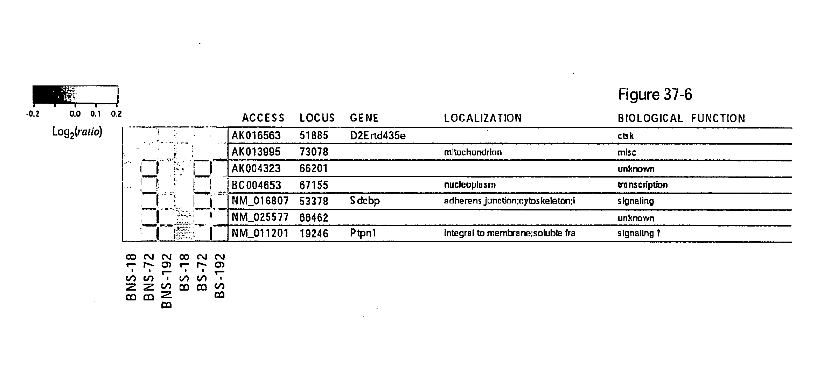

The following figures 29 to 37-6 are tables showing genes differentially expressed genes in mice harboring either breast, colon, or lung carcinoma compared to control mice (p < 0.01) after performing a hierarchical cluster analysis. Samples were obtained from either the prefrontal cortex, the hypothalamus, or the midbrain at 18, 72 and 192 hours post tumor cell injection. Differentially expressed genes were identified by a mixed model ANOVA, with tumor (or control) and time points as fixed effects. The base 2 logarithm of tumor vs. control ratio is shown as a gray scale. BNS corresponds to data obtained without background subtraction, and BS corresponds to data obtained after background subtraction.

FIG. 29 shows differentially expressed genes in mice harboring breast carcinoma after performing a hierarchical cluster analysis. Samples were obtained from prefrontal cortex.
FIG. 29-1 shows down-regulated genes in mice harboring breast carcinoma. Samples were obtained from prefrontal cortex. The list of genes corresponds to those that are down-regulated at all the time points.
FIG. 29-2 shows down-regulated genes in mice harboring breast carcinoma. Samples were obtained from prefrontal cortex. The list of genes corresponds to those that are down-regulated at 18 hours, or at 18 hours and 72 hours post tumor injection.
FIG. 29-3 shows down-regulated genes in mice harboring breast carcinoma. Samples were obtained from prefrontal cortex. The list of genes corresponds to those that are down-regulated at 72 hours and 192 hours post tumor injection.
FIG. 29- 4 shows up-regulated genes in mice harboring breast carcinoma. Samples were obtained from prefrontal cortex. The list of genes corresponds to those that are up-regulated at all the time points
FIG. 29-5 shows up-regulated genes in mice harboring breast carcinoma. Samples were obtained from prefrontal cortex. The list of genes corresponds to those that are up regulated at 18 hours, or at 18 hours and 72 hours post tumor injection.
FIG. 29-6 shows up-regulated genes in mice harboring breast carcinoma. Samples were obtained from prefrontal cortex. The list of genes corresponds to those that are up-regulated at 72 hours and 192 hours post tumor injection.

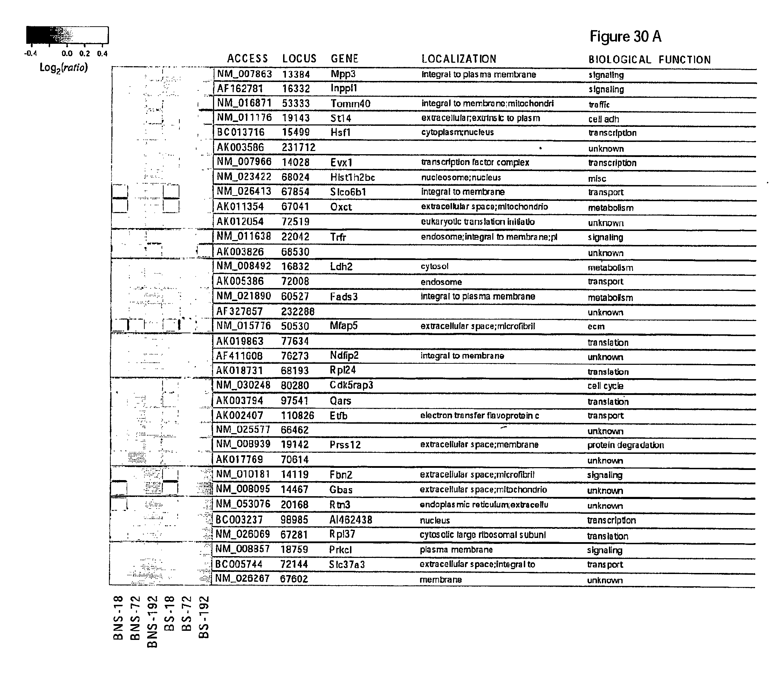

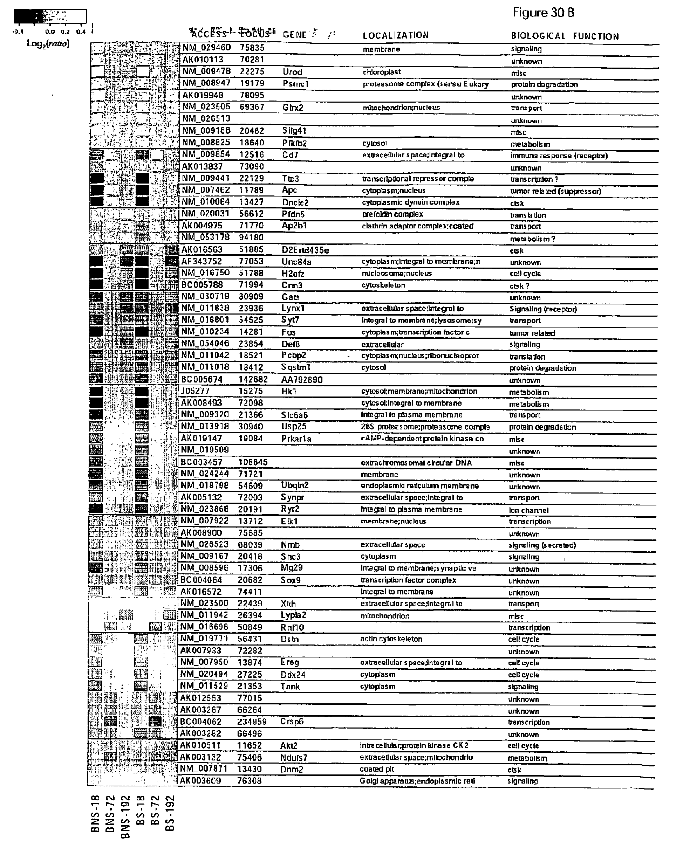

FIGS. 30A & 30B are tables showing genes differentially expressed genes in mice harboring colon carcinoma after performing a hierarchical cluster analysis. Samples were obtained from prefrontal cortex.
FIG. 30-1 shows down-regulated genes in mice harboring colon carcinoma. Samples were obtained from prefrontal cortex. The list of genes corresponds to those that are down-regulated at all the time points.
FIG. 30-2 shows down-regulated genes in mice harboring colon carcinoma. Samples were obtained from prefrontal cortex. The list of genes corresponds to those that are down-regulated at 18 hours, or at 18 hours and 72 hours post tumor injection.

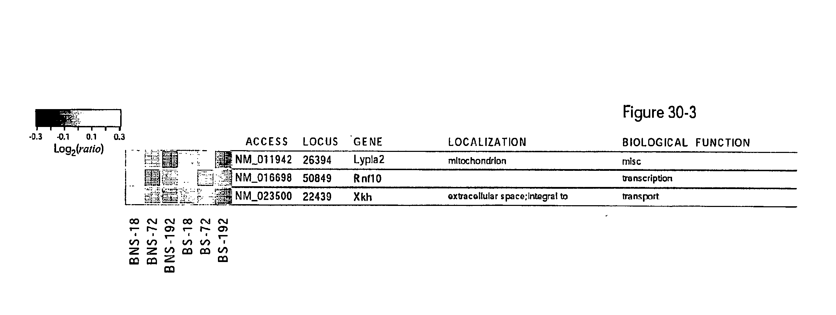

FIG. 30-3 shows down-regulated genes in mice harboring colon carcinoma. Samples were obtained from prefrontal cortex. The list of genes corresponds to those that are down-regulated at 72 hours and 192 hours post tumor injection.
FIG. 30-4 shows up-regulated in mice harboring colon carcinoma. Samples were obtained from prefrontal cortex. The list of genes corresponds to those that are up-regulated at all the time points
FIG. 30-5 shows up regulated genes in mice harboring colon carcinoma. Samples were obtained from prefrontal cortex. The list of genes corresponds to those that are up regulated at 18 hours, or at 18 hours and 72 hours post tumor injection.

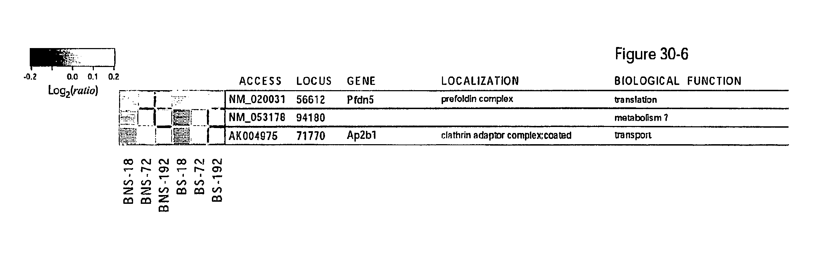

FIG. 30-6 shows up regulated genes in mice harboring colon carcinoma. Samples were obtained from prefrontal cortex. The list of genes corresponds to those that are up regulated at 72 hours and 192 hours post tumor injection.

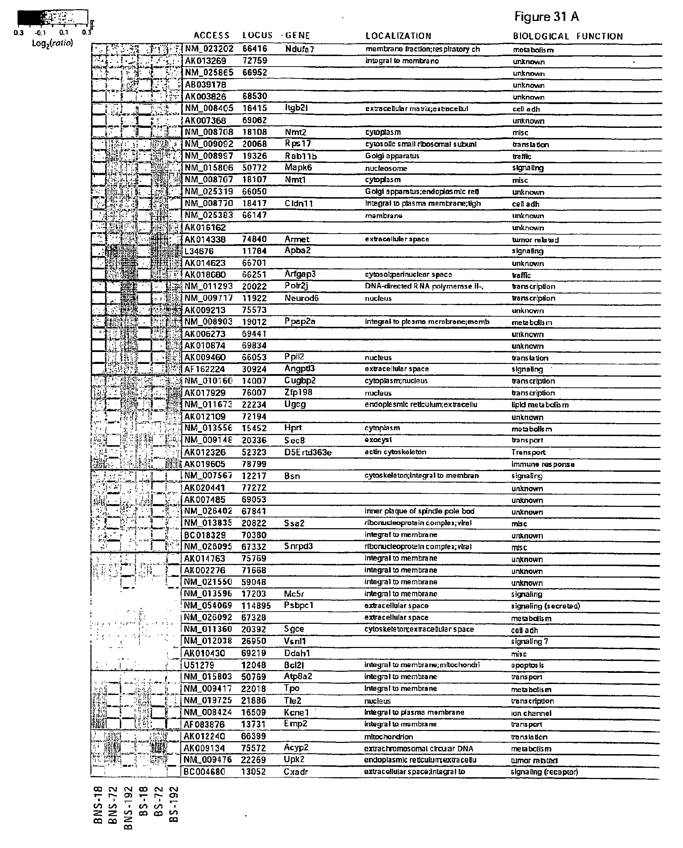

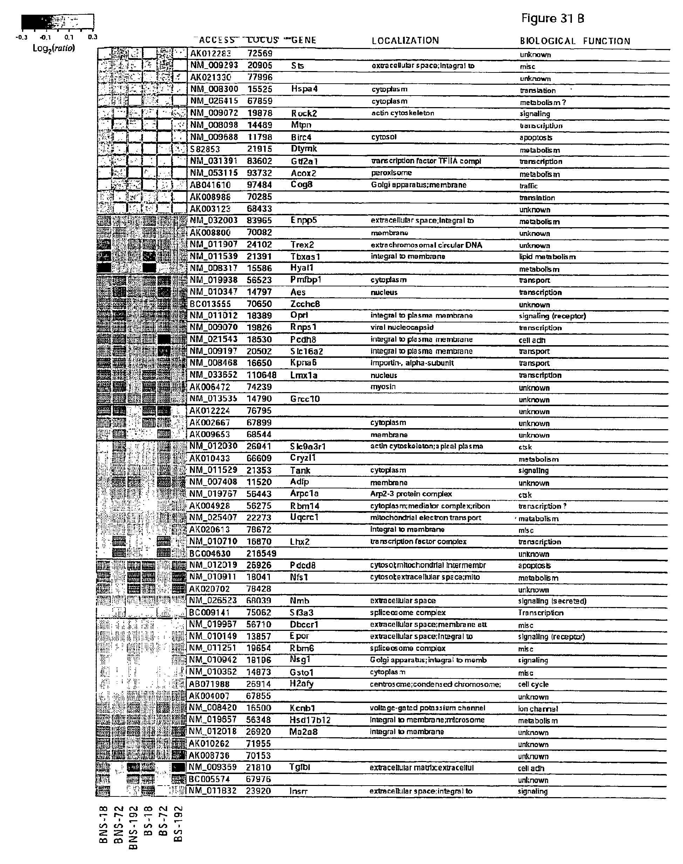

FIGS. 31A & 31B are tables showing differentially expressed genes in mice harboring lung carcinoma after hierarchical cluster analysis. Samples were obtained from prefrontal cortex.

FIG. 31-1 shows down regulated genes in mice harboring lung carcinoma. Samples were obtained from prefrontal cortex. The list of genes corresponds to those that are down regulated at all the time points.

FIG. 31-2 shows down regulated genes in mice harboring lung carcinoma. Samples were obtained from prefrontal cortex. The list of genes corresponds to those that are down regulated at 18 hours, or at 18 hours and 72 hours post tumor injection.

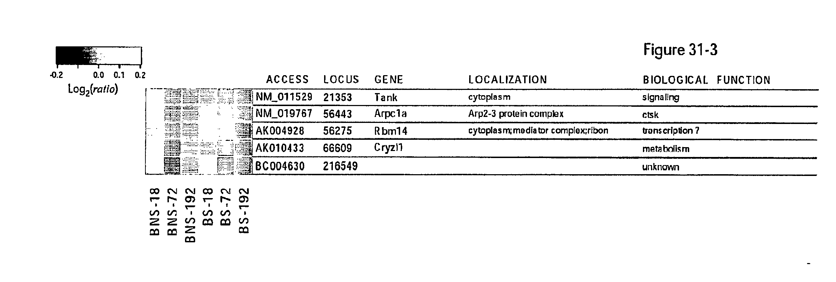

FIG. 31-3 shows down regulated genes in mice harboring lung carcinoma. Samples were obtained from prefrontal cortex. The list of genes corresponds to those that are down regulated at 72 hours and 192 hours post tumor injection.

FIG. 31-4 shows up regulated genes in mice harboring lung carcinoma. Samples were obtained from prefrontal cortex. The list of genes corresponds to those that are up regulated at all the time points.

FIG. 31-5 shows up regulated genes in mice harboring lung carcinoma. Samples were obtained from prefrontal cortex. The list of genes corresponds to those that are up regulated at 18 hours, or at 18 hours and 72 hours post tumor injection.

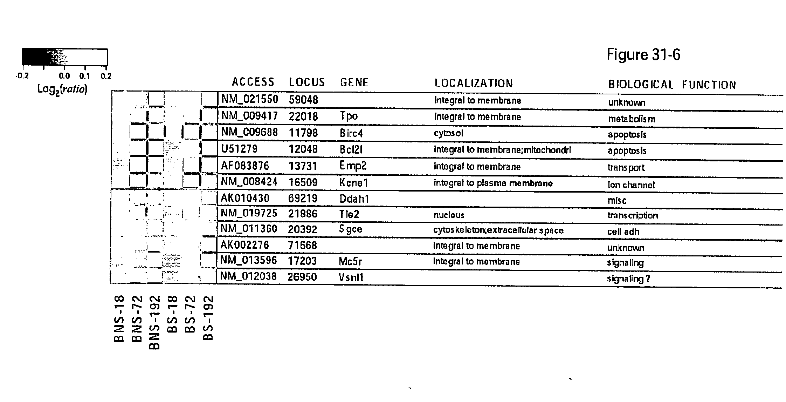

FIG. 31-6 shows up regulated genes in mice harboring lung carcinoma. Samples were obtained from prefrontal cortex. The list of genes corresponds to those that are up regulated at 72 hours and 192 hours post tumor injection.

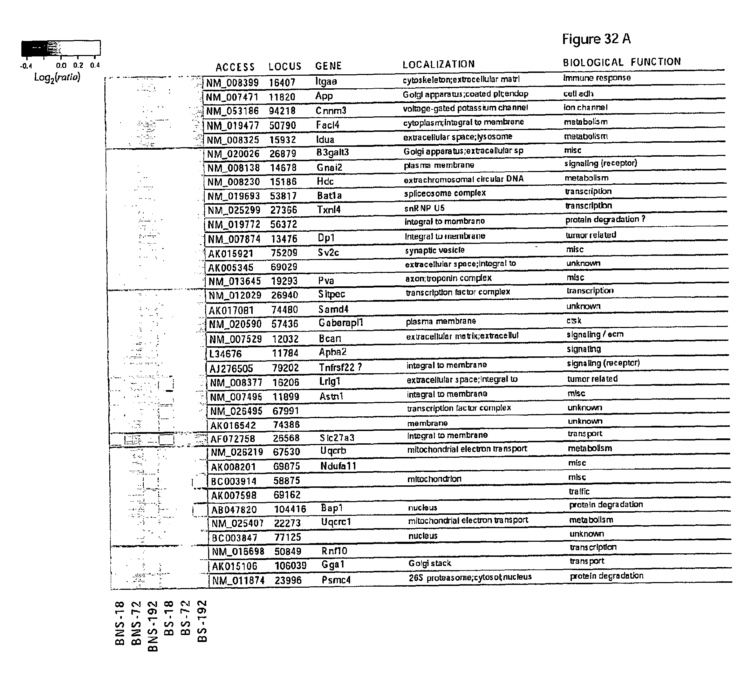

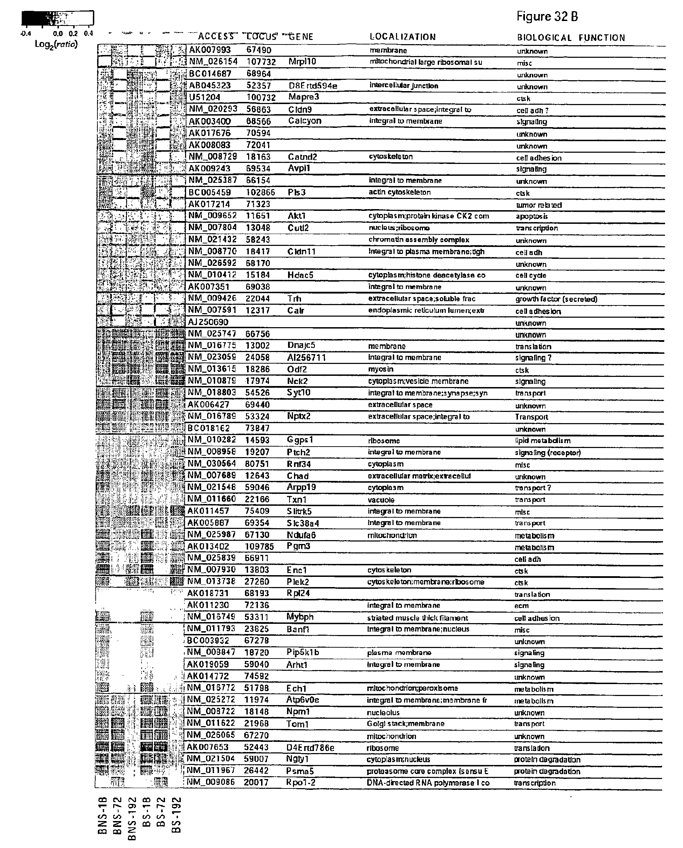

FIGS. 32A & 32B show differentially expressed genes in mice harboring breast carcinoma. Samples were obtained from hypothalamus.

FIG. 32-1 shows down regulated genes in mice harboring breast carcinoma. Samples were obtained from hypothalamus. The list of genes corresponds to those that are down regulated at all the time points.

FIG. 32-2 shows down regulated genes in mice harboring breast carcinoma. Samples were obtained from hypothalamus. The list of genes corresponds to those that are down regulated at 18 hours, or at 18 hours and 72 hours post tumor injection.

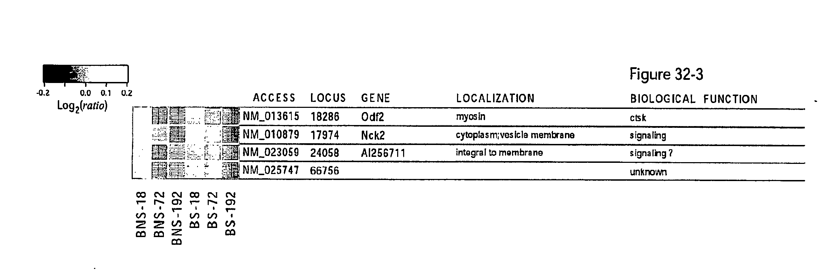

FIG. 32-3 shows down regulated genes in mice harboring breast carcinoma. Samples were obtained from hypothalamus. The list of genes corresponds to those that are down regulated at 72 hours and 192 hours post tumor injection.

FIG. 32-4 shows up regulated genes in mice harboring breast carcinoma. Samples were obtained from hypothalamus. The list of genes corresponds to those that are up regulated at all the time points.

FIG. 32-5 shows up regulated genes in mice harboring breast carcinoma. Samples were obtained from hypothalamus. The list of genes corresponds to those that are up regulated at 18 hours, or at 18 hours and 72 hours post tumor injection.

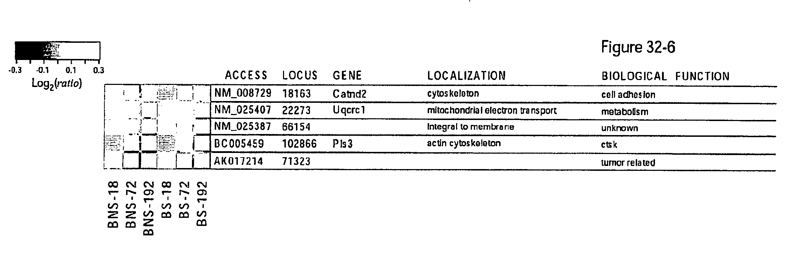

FIG. 32-6 shows up regulated genes in mice harboring breast carcinoma. Samples were obtained from hypothalamus. The list of genes corresponds to those that are up regulated at 72 hours and 192 hours post tumor injection.

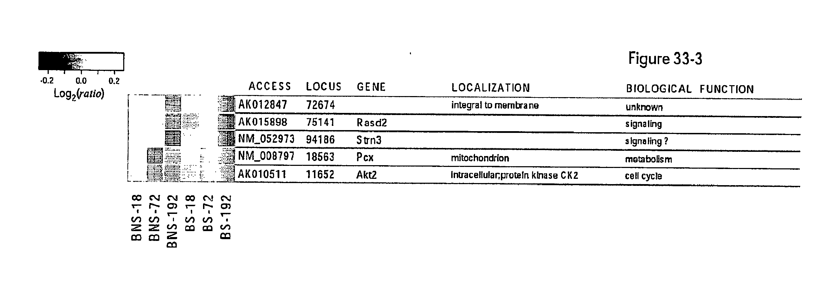

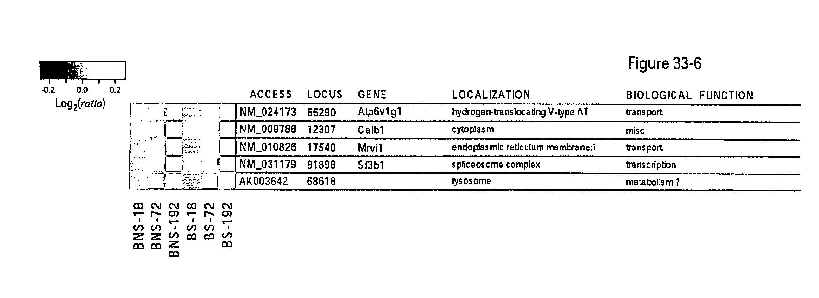

FIG. 33 shows differentially expressed genes in mice harboring colon carcinoma. Samples were obtained from hypothalamus.

FIG. 33-1 shows down regulated genes in mice harboring colon carcinoma. Samples were obtained from hypothalamus. The list of genes corresponds to those that are down regulated at all the time points.

FIG. 33-2 shows down regulated genes in mice harboring colon carcinoma. Samples were obtained from hypothalamus. The list of genes corresponds to those that are down regulated at 18 hours, or at 18 hours and 72 hours post tumor injection.

FIG. 33-3 shows down regulated genes in mice harboring colon carcinoma. Samples were obtained from hypothalamus. The list of genes corresponds to those that are down regulated at 72 hours and 192 hours post tumor injection.

FIG. 33-4 shows up regulated genes in mice harboring colon carcinoma. Samples were obtained from hypothalamus. The list of genes corresponds to those that are up regulated at all the time points.

FIG. 33-5 shows up regulated genes in mice harboring colon carcinoma. Samples were obtained from hypothalamus. The list of genes corresponds to those that are up regulated at 18 hours, or at 18 hours and 72 hours post tumor injection.

FIG. 33-6 shows up regulated genes in mice harboring colon carcinoma. Samples were obtained from hypothalamus. The list of genes corresponds to those that are up regulated at 72 hours and 192 hours post tumor injection.

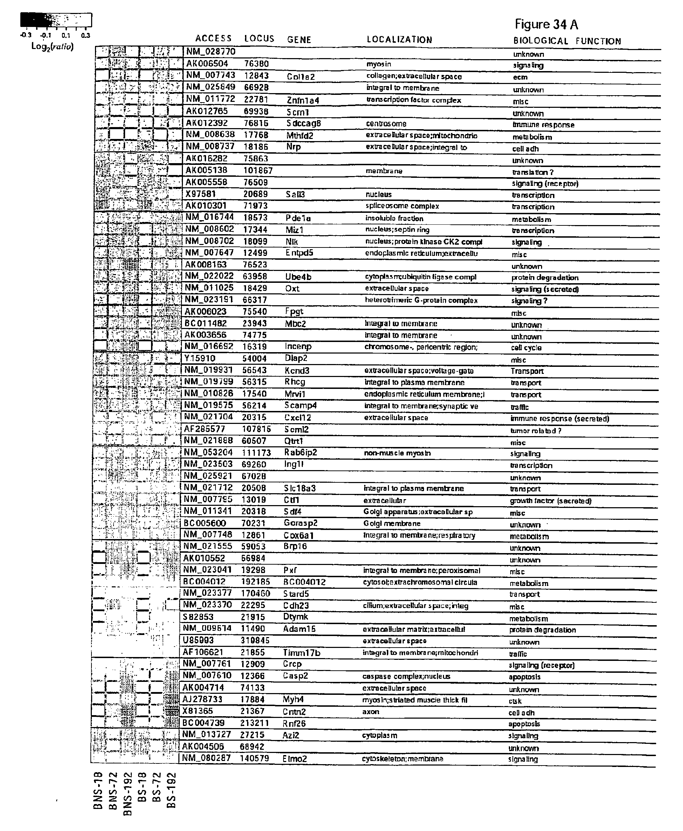

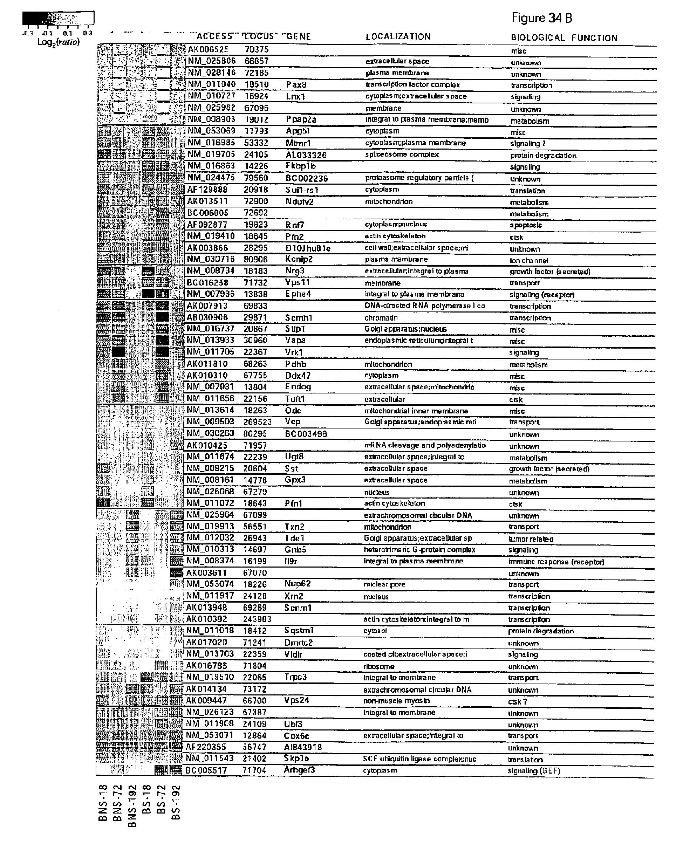

FIGS. 34A & 34-B show differentially expressed genes in mice harboring lung carcinoma. Samples were obtained from hypothalamus.

FIG. 34-1 shows down regulated genes in mice harboring lung carcinoma. Samples were obtained from hypothalamus. The list of genes corresponds to those that are down regulated at all the time points.

FIG. 34-2 shows down regulated genes in mice harboring lung carcinoma. Samples were obtained from hypothalamus. The list of genes corresponds to those that are down regulated at 18 hours, or at 18 hours and 72 hours post tumor injection.

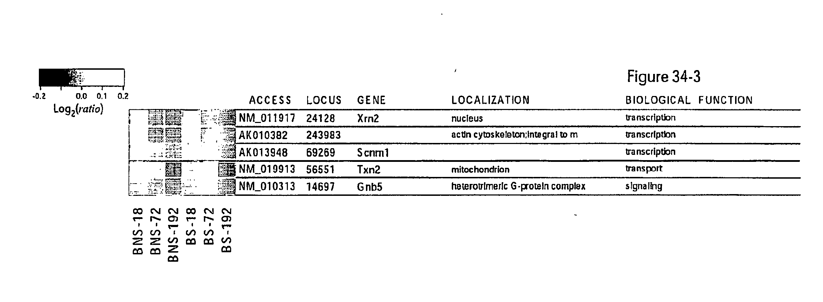

FIG. 34-3 shows down regulated genes in mice harboring lung carcinoma. Samples were obtained from hypothalamus. The list of genes corresponds to those that are down regulated at 72 hours and 192 hours post tumor injection

FIG. 34-4 shows up regulated genes in mice harboring lung carcinoma. Samples were obtained from hypothalamus.

The list of genes corresponds to those that are up regulated at all the time points.

FIG. 34-5 shows up regulated genes in mice harboring lung carcinoma. Samples were obtained from hypothalamus. The list of genes corresponds to those that are up regulated at 18 hours, or at 18 hours and 72 hours post tumor injection.

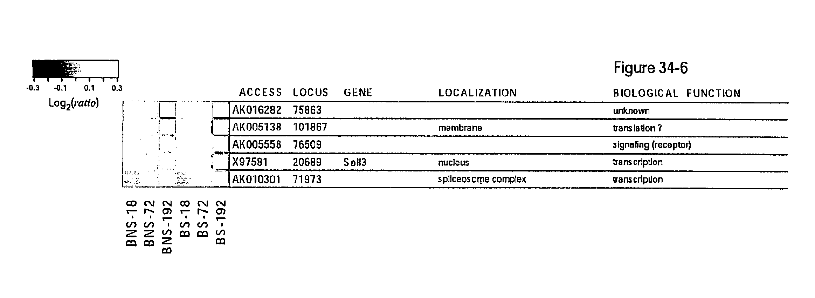

FIG. 34-6 shows up regulated genes in mice harboring lung carcinoma. Samples were obtained from hypothalamus. The list of genes corresponds to those that are up regulated at 72 hours and 192 hours post tumor injection.

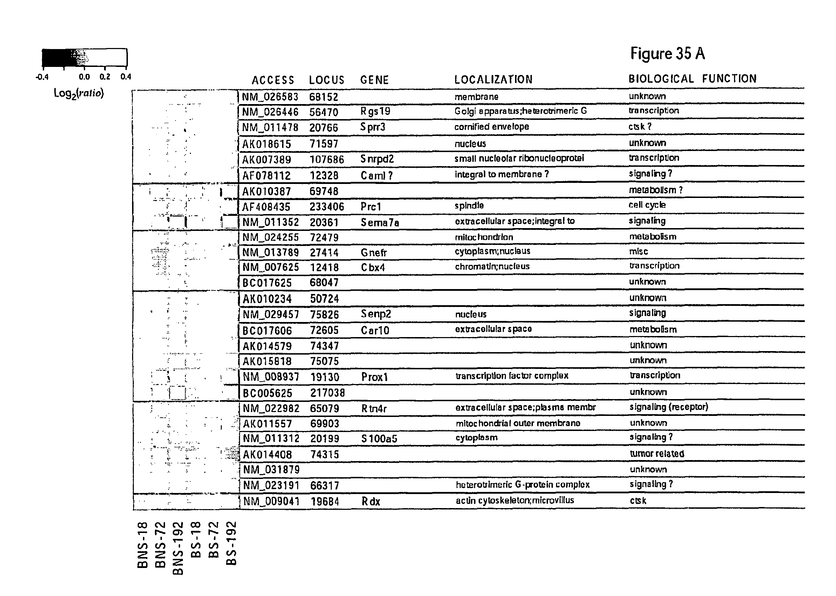

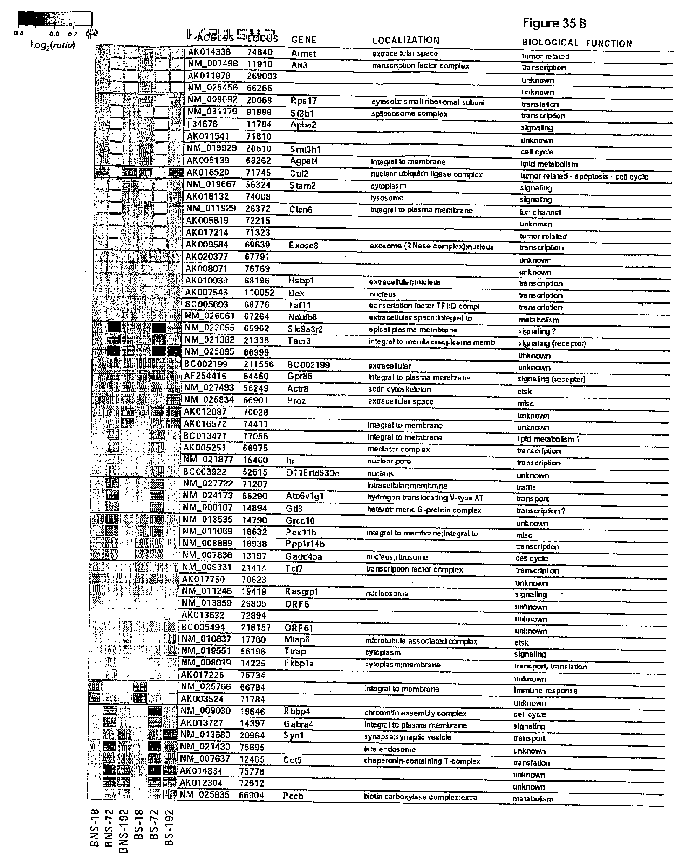

FIGS. 35A & 35B shows differentially expressed genes in mice harboring breast carcinoma after hierarchical cluster analysis. Samples were obtained from midbrain.

FIG. 35-1 shows down regulated genes in mice harboring breast carcinoma. Samples were obtained from midbrain. The list of genes corresponds to those that are down regulated at all the time points.

FIG. 35-2 shows down regulated genes in mice harboring breast carcinoma. Samples were obtained from midbrain. The list of genes corresponds to those that are down regulated at 18 hours, or at 18 hours and 72 hours post tumor injection.

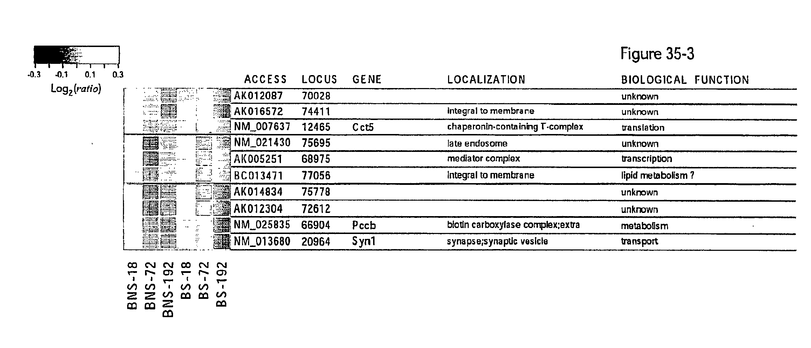

FIG. 35-3 shows down regulated genes in mice harboring breast carcinoma. Samples were obtained from midbrain. The list of genes corresponds to those that are down regulated at 72 hours and 192 hours post tumor injection.

FIG. 35-4 shows up regulated genes in mice harboring breast carcinoma. Samples were obtained from midbrain. The list of genes corresponds to those that are up regulated at all the time points

FIG. 35-5 shows up regulated genes in mice harboring breast carcinoma. Samples were obtained from midbrain. The list of genes corresponds to those that are up regulated at 18 hours, or at 18 hours and 72 hours post tumor injection.

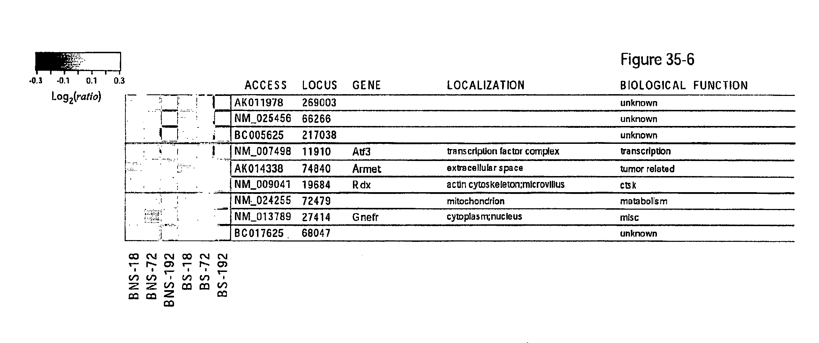

FIG. 35-6 shows up regulated genes in mice harboring breast carcinoma. Samples were obtained from midbrain. The list of genes corresponds to those that are up regulated at 72 hours and 192 hours post tumor injection.

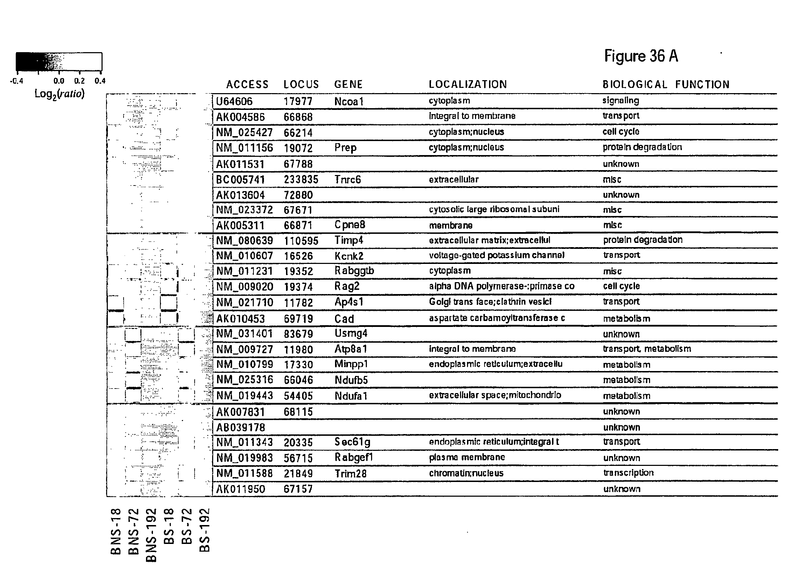

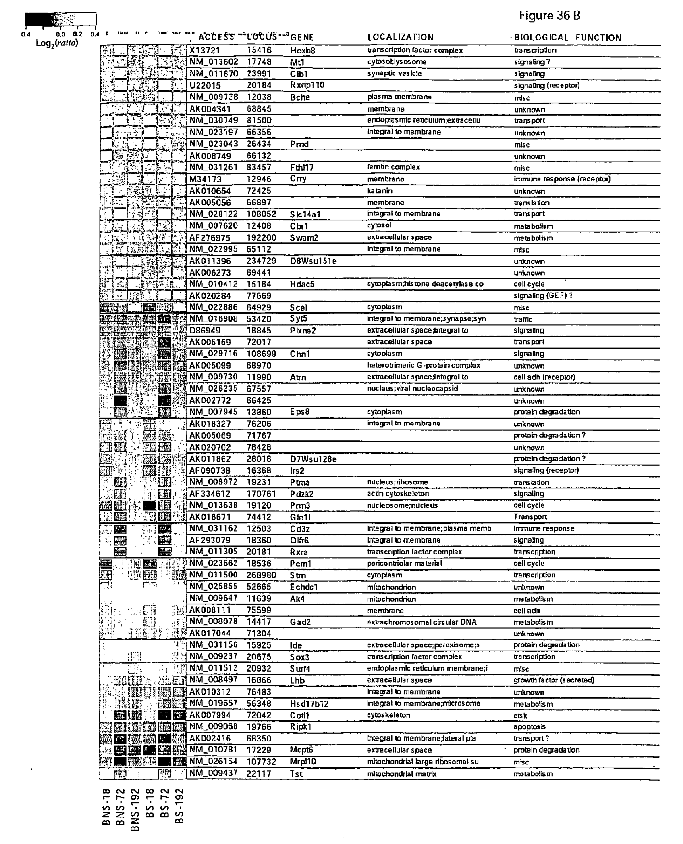

FIGS. 36A & 36B shows differentially expressed genes in mice harboring colon carcinoma after hierarchical cluster analysis. Samples were obtained from midbrain.

FIG. 36-1 shows down regulated genes in mice harboring colon carcinoma. Samples were obtained from midbrain. The list of genes corresponds to those that are down regulated at all the time points.

FIG. 36-2 shows down regulated genes in mice harboring colon carcinoma. Samples were obtained from midbrain. The list of genes corresponds to those that are down regulated at 18 hours, or at 18 hours and 72 hours post tumor injection.

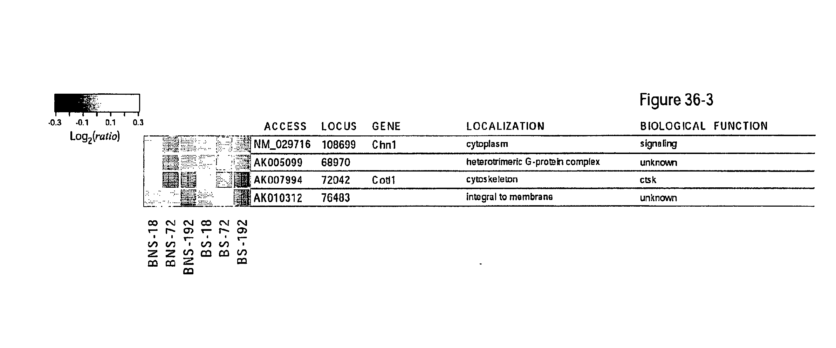

FIG. 36-3 shows down regulated genes in mice harboring colon carcinoma. Samples were obtained from midbrain. The list of genes corresponds to those that are down regulated at 72 hours and 192 hours post tumor injection.

FIG. 36-4 shows up regulated genes in mice harboring colon carcinoma. Samples were obtained from midbrain. The list of genes corresponds to those that are up regulated at all the time points.

FIG. 36-5 shows up regulated genes in mice harboring colon carcinoma. Samples were obtained from midbrain. The list of genes corresponds to those that are up regulated at 18 hours, or at 18 hours and 72 hours post tumor injection.

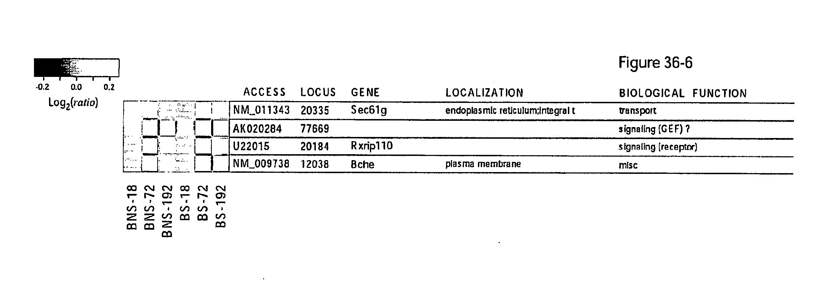

FIG. 36-6 shows up regulated genes in mice harboring colon carcinoma. Samples were obtained from midbrain. The list of genes corresponds to those that are up regulated at 72 hours and 192 hours post tumor injection.

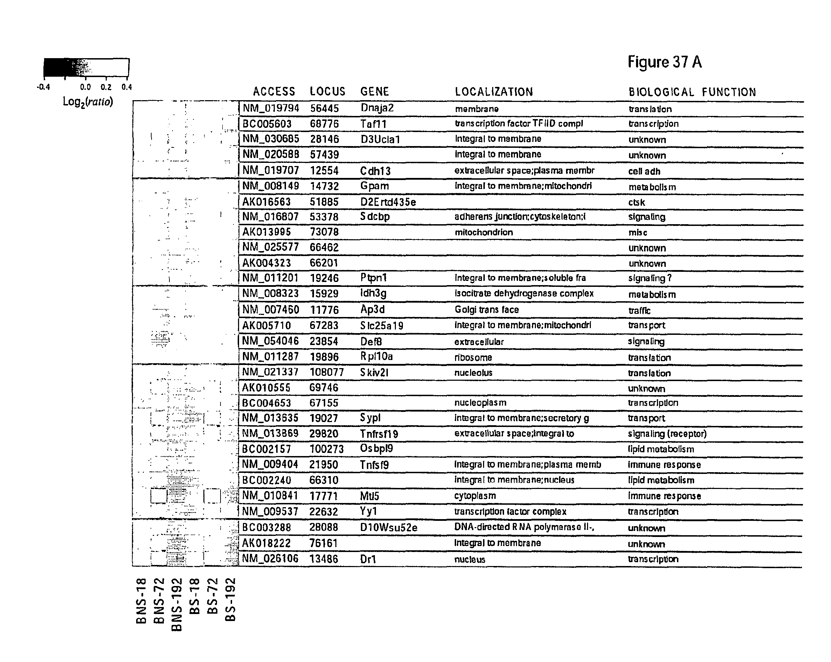

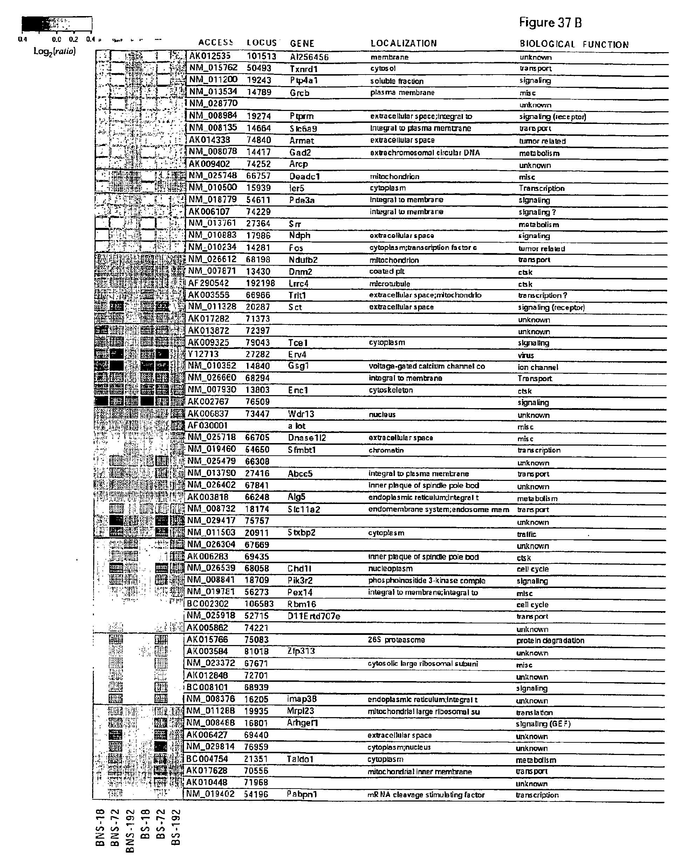

FIGS. 37A & 37B shows differentially expressed genes in mice harboring lung carcinoma after hierarchical cluster analysis. Samples were obtained from midbrain.

FIG. 37-1 shows down regulated genes in mice harboring lung carcinoma. Samples were obtained from midbrain. The list of genes corresponds to those that are down regulated at all the time points.

FIG. 37-2 shows down regulated genes in mice harboring lung carcinoma. Samples were obtained from midbrain. The list of genes corresponds to those that are down regulated at 18 hours, or at 18 hours and 72 hours post tumor injection.

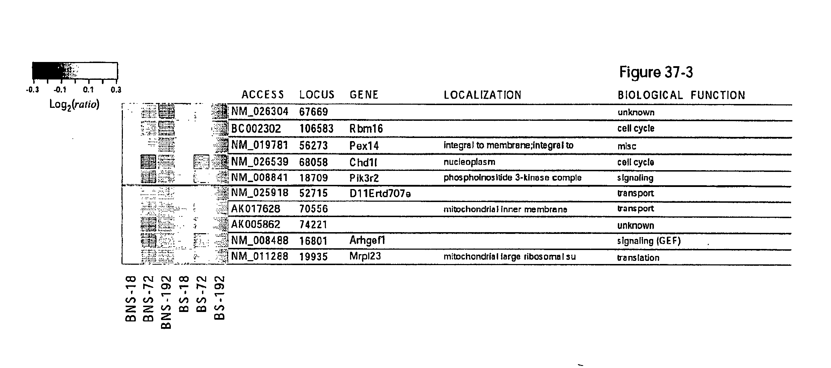

FIG. 37-3 shows down regulated genes in mice harboring lung carcinoma. Samples were obtained from midbrain. The list of genes corresponds to those that are down regulated at 72 hours and 192 hours post tumor injection.

FIG. 37-4 shows up regulated genes in mice harboring lung carcinoma. Samples were obtained from midbrain. The list of genes corresponds to those that are up regulated at all the time points.

FIG. 37-5 shows up regulated genes in mice harboring lung carcinoma. Samples were obtained from midbrain. The list of genes corresponds to those that are up regulated at 18 hours, or at 18 hours and 72 hours post tumor injection.

FIG. 37-6 shows up regulated genes in mice harboring lung carcinoma. Samples were obtained from midbrain at 18, 72 and 192 hours post tumor cell injection. The list of genes corresponds to those that are up regulated at 72 hours and 192 hours post tumor injection.

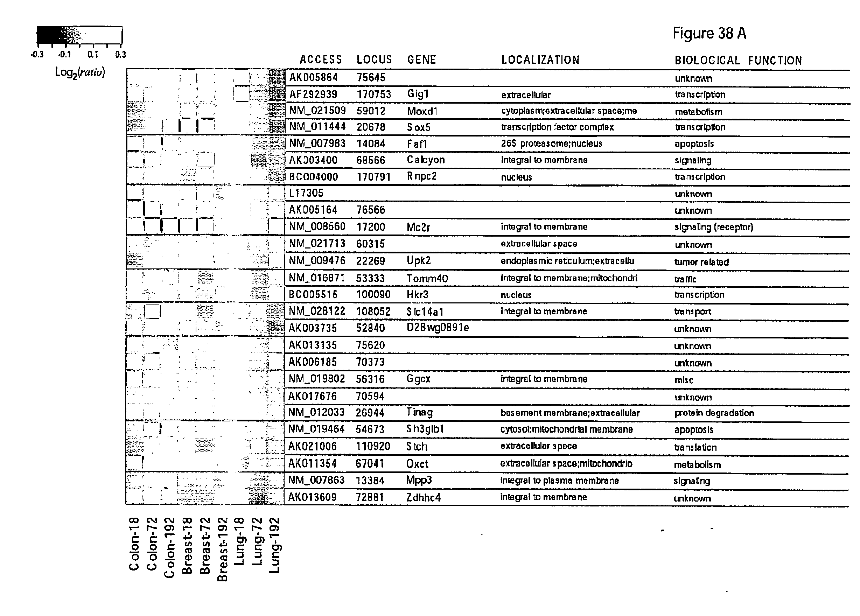

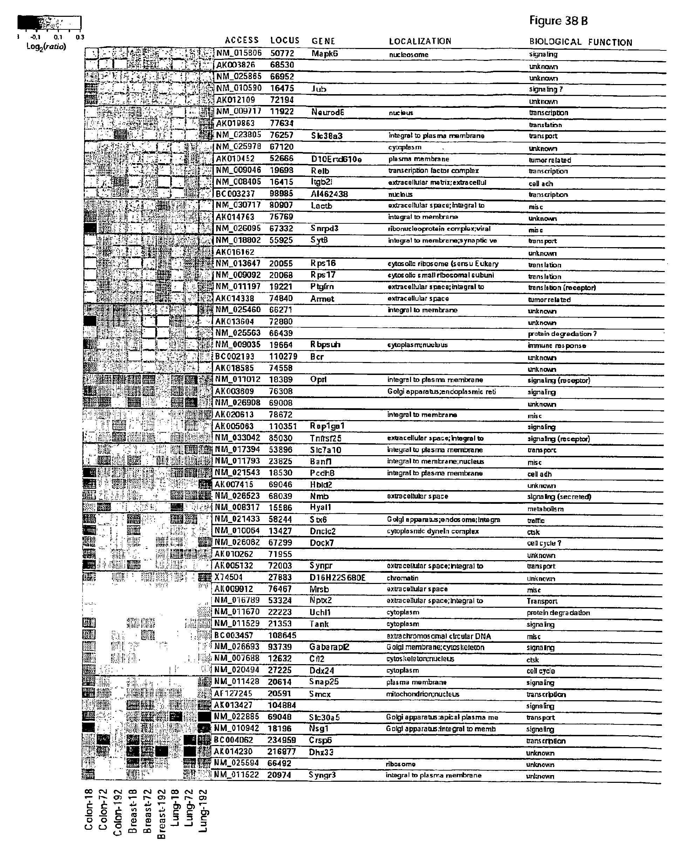

FIGS. 38A & 38B shows differentially expressed genes in mice harboring either breast, colon or lung carcinoma compared to control mice (p < 0.01) after hierarchical cluster analysis. Samples were obtained from prefrontal cortex at 18, 72 and 192 hours post tumor cell injection. Differentially expressed genes were identified by a mixed model ANOVA, with tumor (or control), tumor model, and time points as fixed effects. Only data obtained without background subtraction was included in the table. The base 2 logarithm of tumor vs. control ratio is shown as a gray scale.

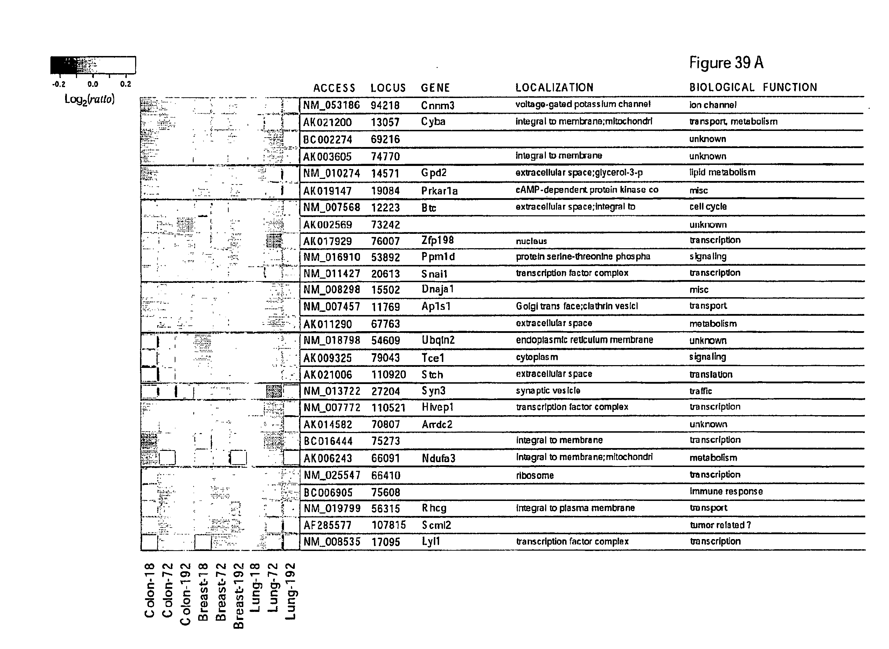

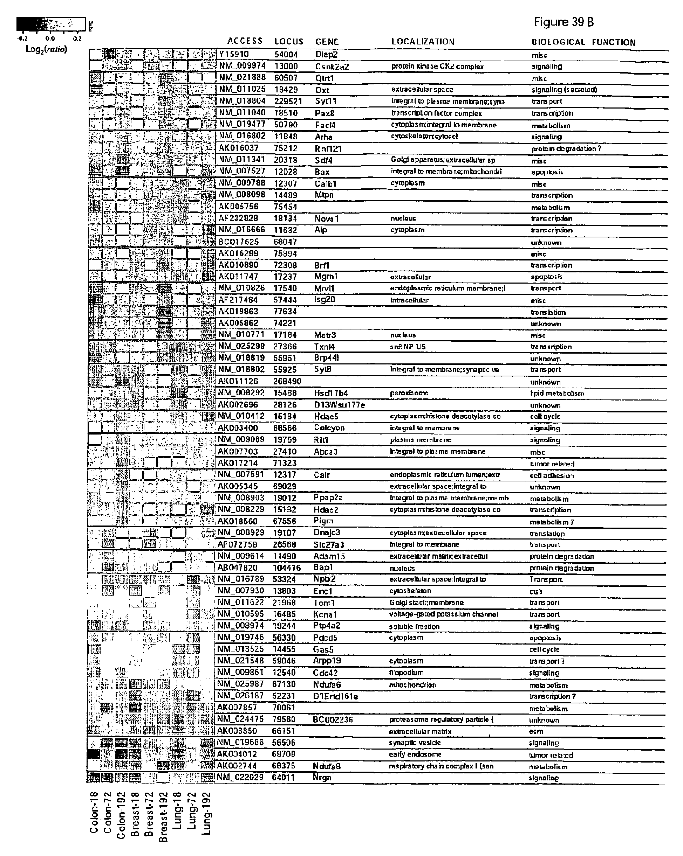

FIGS. 39A & 39B shows differentially expressed genes in mice harboring either breast, colon or lung carcinoma compared to control mice (p < 0.01). Samples were obtained from hypothalamus at 18, 72 and 192 hours post tumor cell injection. Differentially expressed genes were identified by a mixed model ANOVA, with tumor (or control), tumor model, and time points as fixed effects. Only data obtained without background subtraction was included in the table. The base 2 logarithm of tumor vs. control ratio is shown as a gray scale.

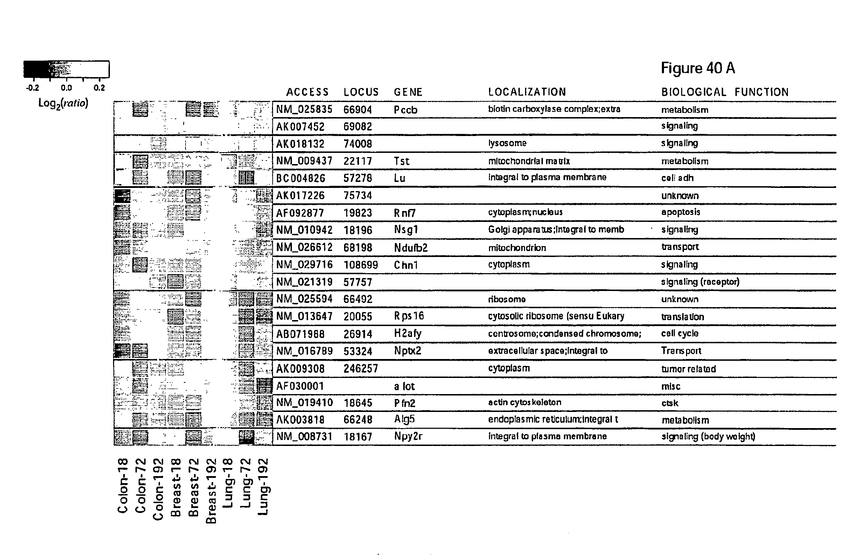

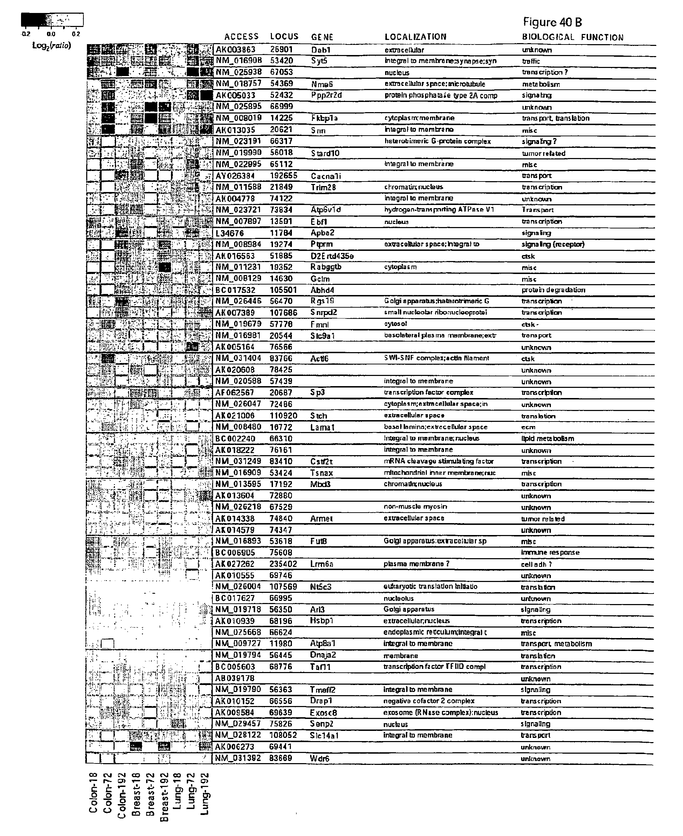

FIGS. 40A & 40B shows differentially expressed genes in mice harboring either breast, colon or lung carcinoma compared to control mice (p < 0.01). Samples were obtained from midbrain at 18, 72 and 192 hours post tumor cell injection. Differentially expressed genes were identified by a mixed model ANOVA, with tumor (or control), tumor model, and time points as fixed effects. Only data obtained without background subtraction was included in the table. The base 2 logarithm of tumor vs. control ratio is shown as a gray scale.

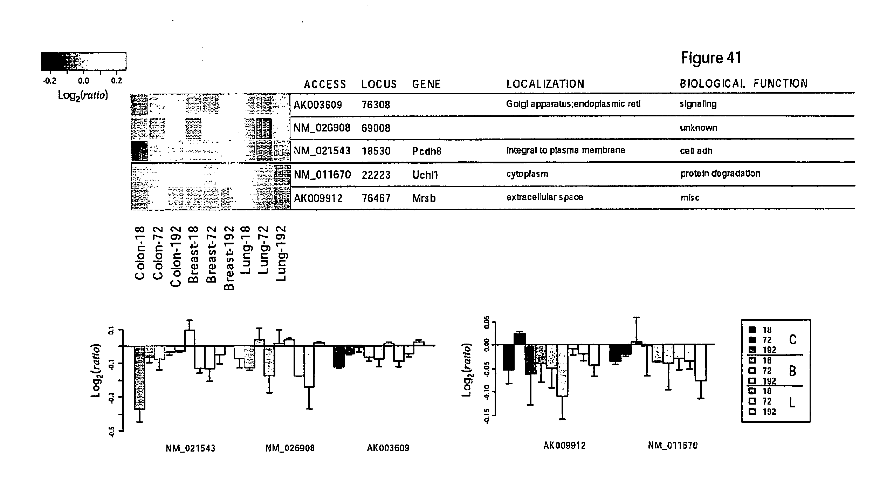

FIG. 41 (A) is a table showing down-regulated genes in mice harboring either breast, colon or lung carcinoma compared to control mice (p < 0.01). Samples were obtained from prefrontal cortex at 18, 72 and 192 hours post tumor cell injection. Differentially expressed genes were identified by a mixed model ANOVA, with tumor (or control), tumor model, and time points as fixed effects. Only genes that showed a similar temporal pattern of expression in at least two cancer models were included in the table. Results correspond to data obtained without background subtraction. The base 2 logarithm of tumor vs. control ratio is shown as a gray scale. (B) Base 2 logarithm of tumor vs. control ratio for genes in (A). Bars are the mean $\pm$ SEM.

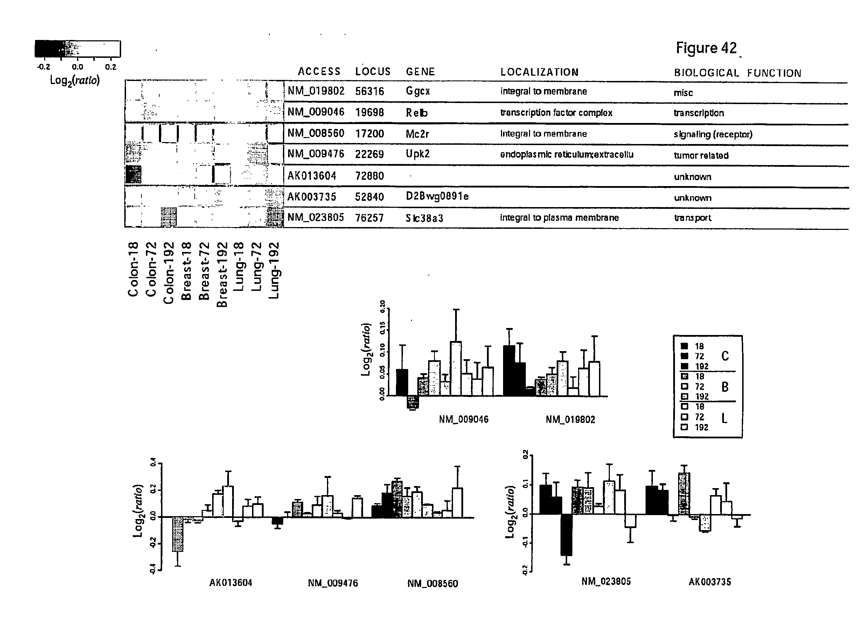

FIG. 42 (A) is a table showing up-regulated genes in mice harboring either breast, colon or lung carcinoma compared to control mice (p < 0.01). Samples were obtained from prefrontal cortex at 18, 72 and 192 hours post tumor cell injection. Differentially expressed genes were identified by a mixed model ANOVA, with tumor (or control), tumor model, and time points as fixed effects. Only genes that showed a similar temporal pattern of expression in at least two cancer models were included in the table. Results correspond to data obtained without background subtraction. The base 2 logarithm of tumor vs. control ratio is shown as a gray scale. (B) Base 2 logarithm of tumor vs. control ratio for genes in (A). Bars are the mean $\pm$ SEM.

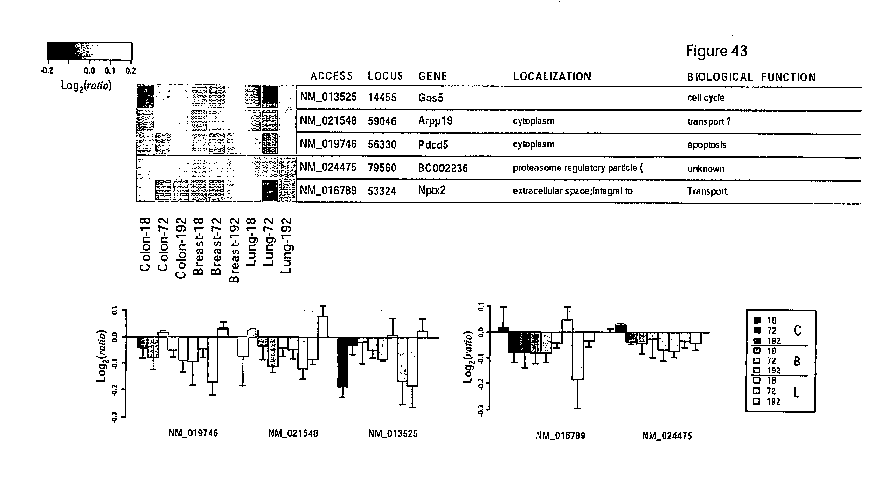

FIG. 43 (A) is a table showing down-regulated genes in mice harboring either breast, colon or lung carcinoma compared to control mice (p < 0.01). Samples were obtained from hypothalamus at 18, 72 and 192 hours post tumor cell injection. Differentially expressed genes were identified by a mixed model ANOVA, with tumor (or control), tumor model, and time points as fixed effects. Only genes that showed a similar temporal pattern of expression in at least two cancer models were included in the table. Results correspond to data obtained without background subtraction. The base 2 logarithm of tumor vs. control ratio is shown as a gray scale. (B) Base 2 logarithm of tumor vs. control ratio for genes in (A). Bars are the mean $\pm$ SEM.

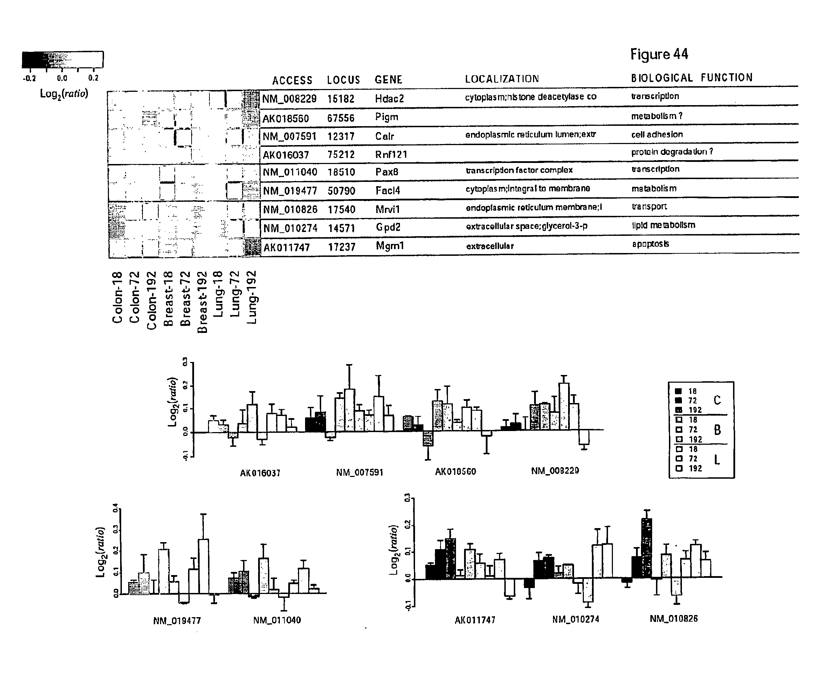

FIG. 44 (A) is a table showing up-regulated genes in mice harboring either breast, colon or lung carcinoma compared to control mice (p < 0.01). Samples were obtained from hypothalamus at 18, 72 and 192 hours post tumor cell injection. Differentially expressed genes were identified by a mixed model ANOVA, with tumor (or control), tumor model, and time points as fixed effects. Only genes that showed a similar temporal pattern of expression in at least two cancer models were included in the table. Results correspond to data obtained without background subtraction. The base 2 logarithm of tumor vs. control ratio is shown as a gray scale. (B) Base 2 logarithm of tumor vs. control ratio for genes in (A). Bars are the mean $\pm$ SEM.

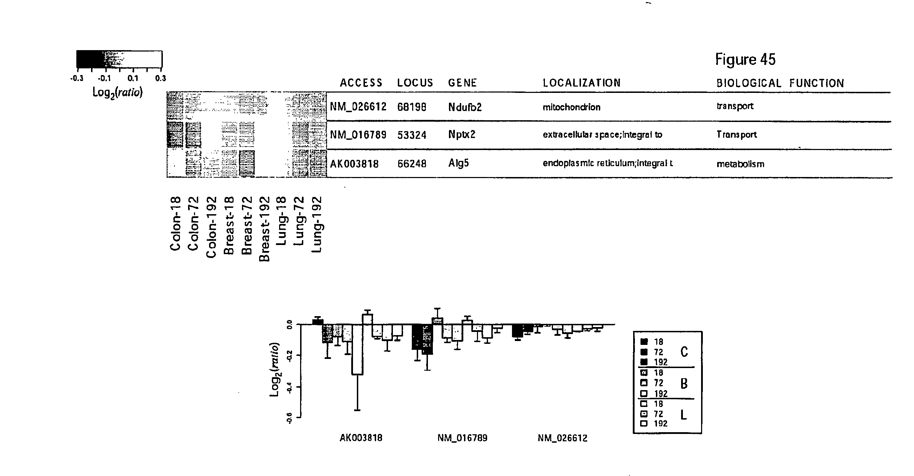

FIG. 45 (A) is a table showing down-regulated genes in mice harboring either breast, colon or lung carcinoma compared to control mice (p < 0.01). Samples were obtained from midbrain at 18, 72 and 192 hours post tumor cell injection. Differentially expressed genes were identified by a mixed model ANOVA, with tumor (or control), tumor model, and time points as fixed effects. Only genes that showed a similar temporal pattern of expression in at least two cancer models were included in the table. Results correspond to data obtained without background subtraction. The base 2 logarithm of tumor vs. control ratio is shown as a gray scale. (B) Base 2 logarithm of tumor vs. control ratio for genes in (A). Bars are the mean $\pm$ SEM.

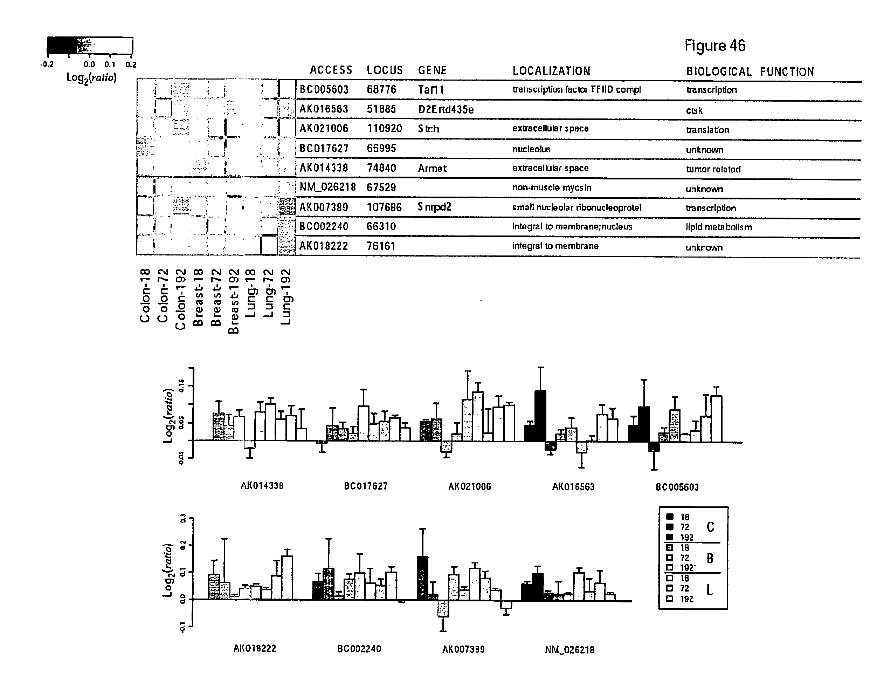

FIG. 46 (A) is a table showing up-regulated genes in mice harboring either breast, colon or lung carcinoma compared to control mice (p < 0.01). Samples were obtained from midbrain at 18, 72 and 192 hours post tumor cell injection. Differentially expressed genes were identified by a mixed model ANOVA, with tumor (or control), tumor model, and time points as fixed effects. Only genes that showed a similar temporal pattern of expression in at least two cancer models were included in the table. Results correspond to data obtained without background subtraction. The base 2 logarithm of tumor vs. control ratio is shown as a gray scale. (B) Base 2 logarithm of tumor vs. control ratio for genes in (A). Bars are the mean $\pm$ SEM.

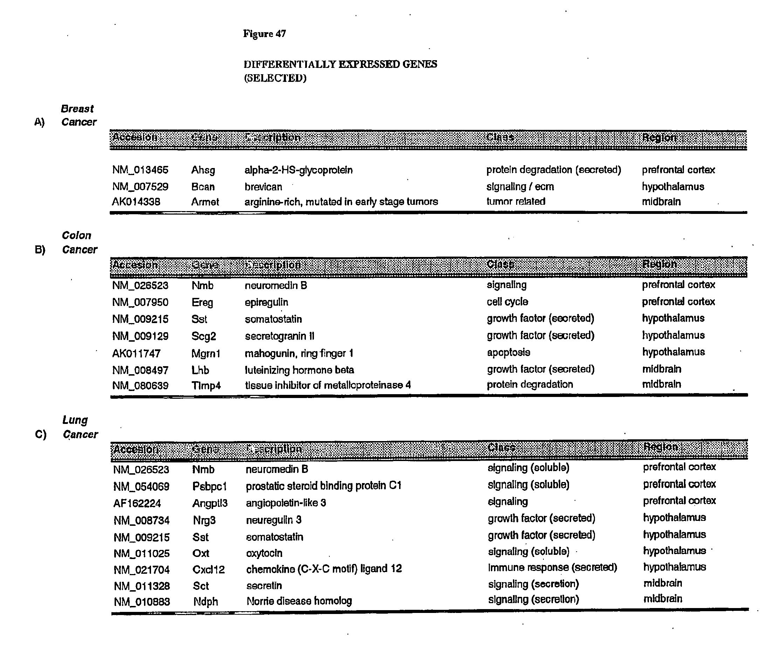

FIG. 47 (A)-(C) is a set of tables listing tumor-specific CNS markers differentially expressed, at any time tested, in three different cancer models: breast cancer, 47A; colon cancer, 47B; and lung cancer, 47C. Criteria for inclusion in this figure were (1) the marker corresponds to a secreted product; and (2) a p value below 0.01 for differential expression.

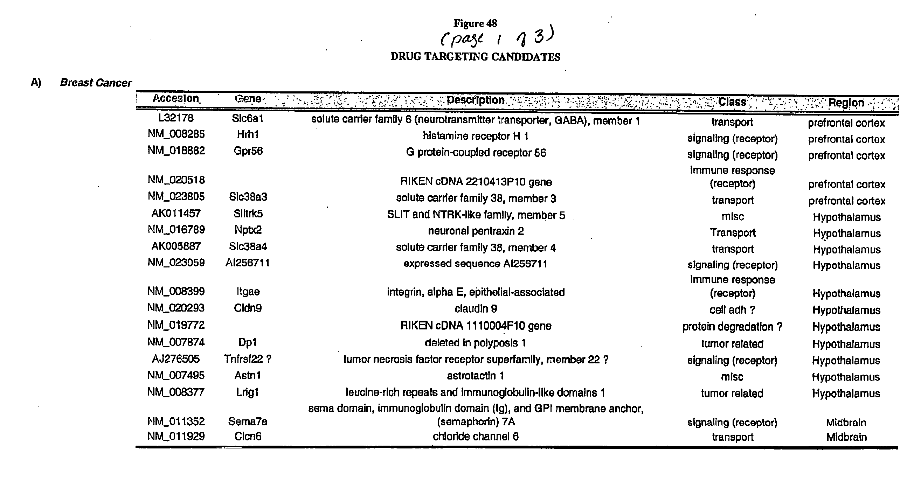

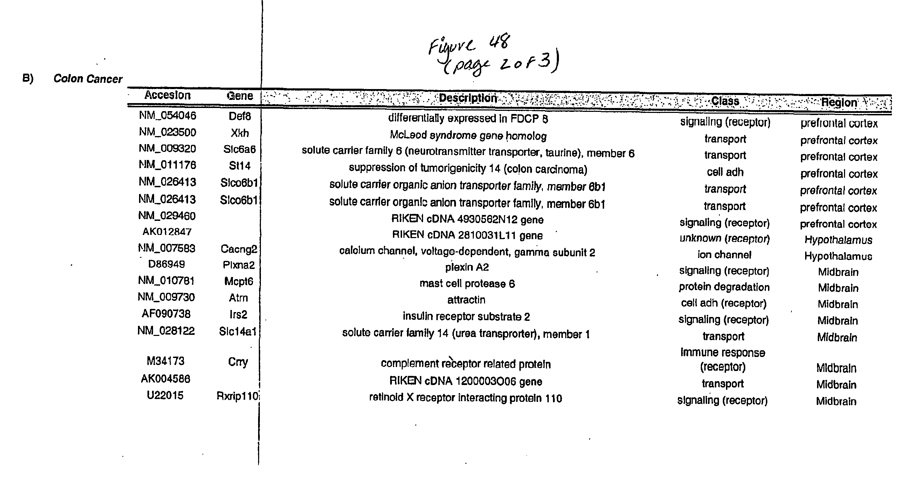

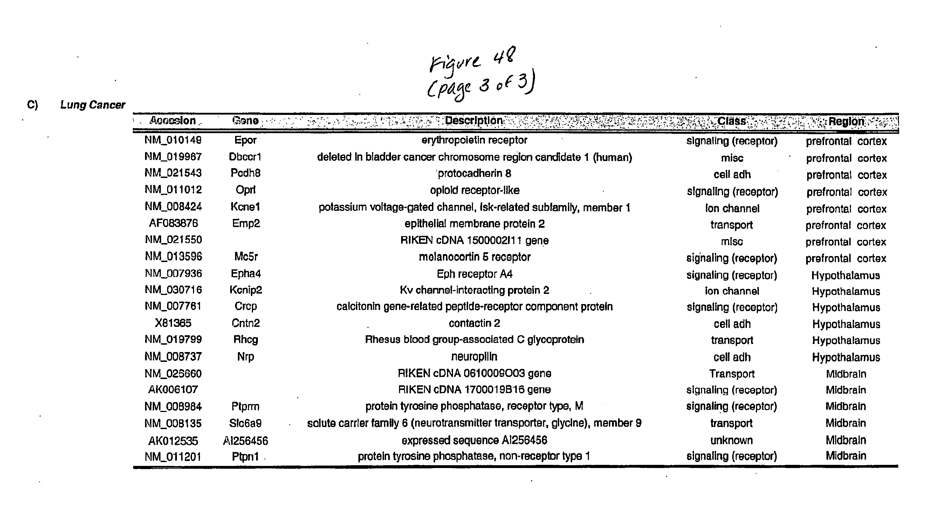

FIG. 48 (A)-(C) is a set of tables listing genes identified as CNS markers that are also potential targets for therapeutic intervention for each of breast, colon and lung cancer. Criteria for inclusion in this figure were (1) the marker corresponds to a signaling receptor such as a growth factor, hormone, or cytokine; and (2) a p value for differential expression below 0.01

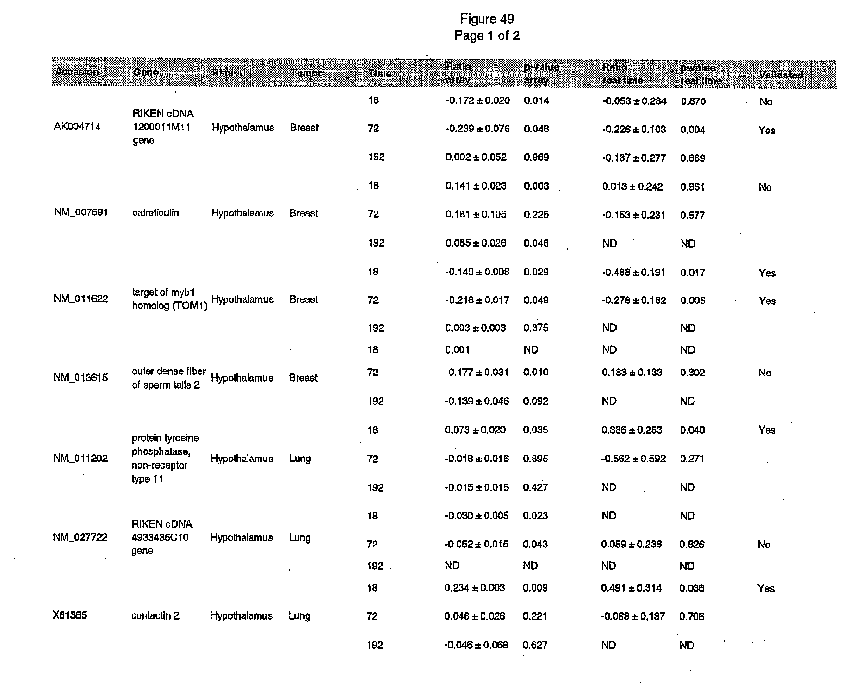

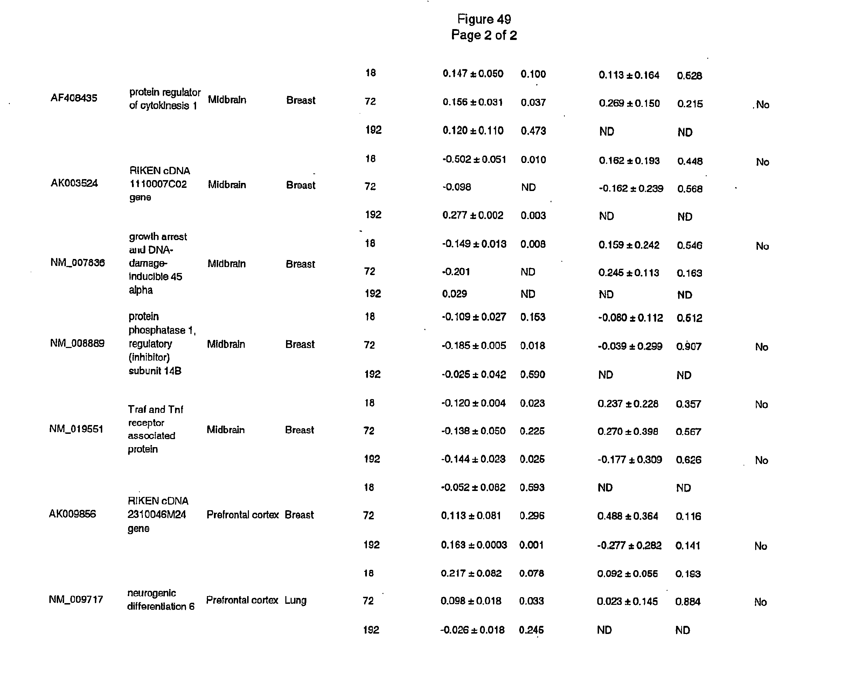

FIG. 49 is a table listing differentially expressed genes (p< 0.05) chosen at random for validation. 4 out of 14 (29%) were validated as differentially expressed genes by real time PCR indicating a good level of correlation between microarray and Real Time PCR according to Wurmbach et al., Methods 2003, 31: 306-316. Ratios are expressed as mean $\pm$ SEM. (ND) No data available. P-value ranks were calculated sorting the genes of microarray results according to their p-values in ascending order.

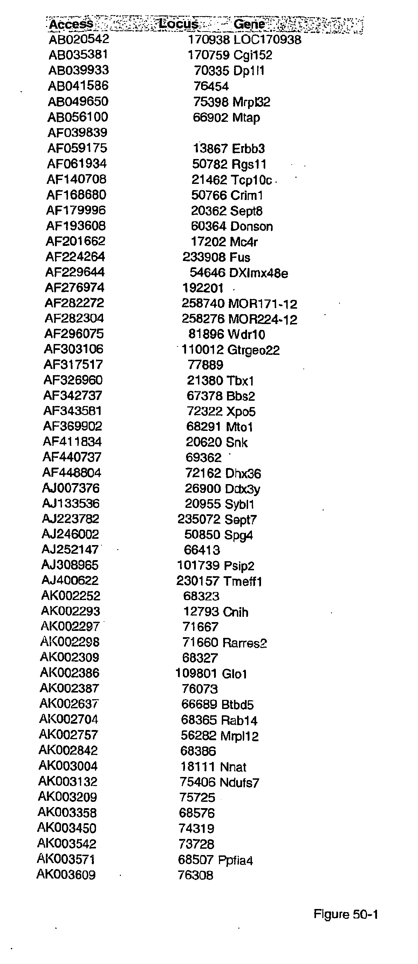

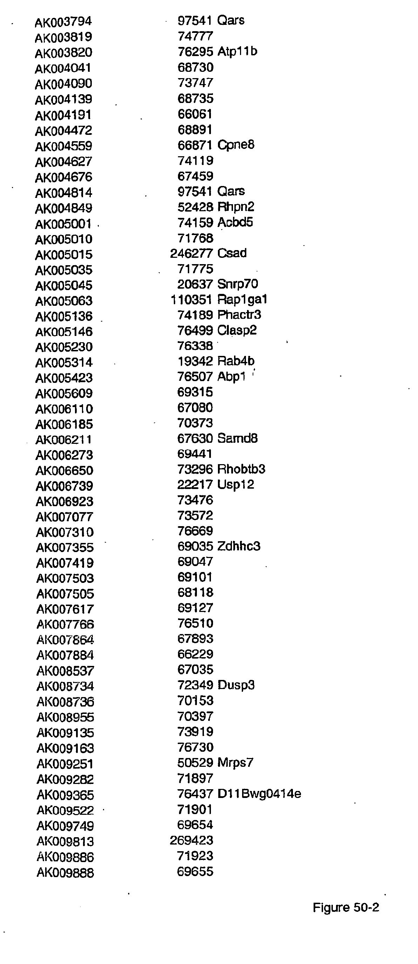

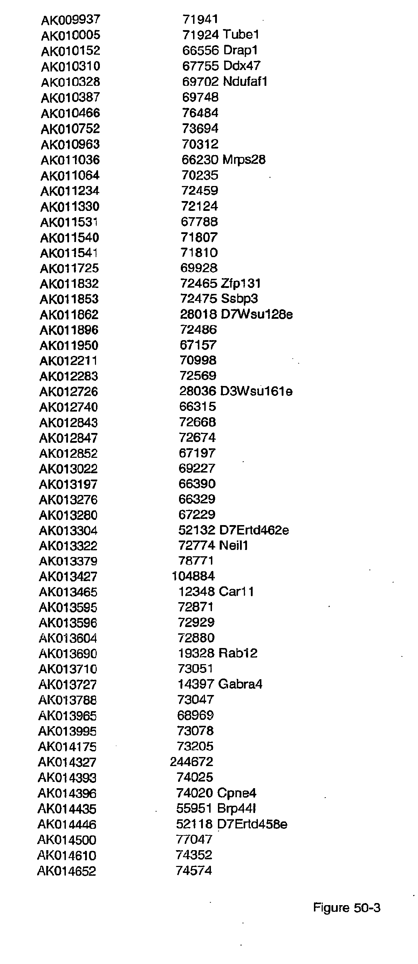

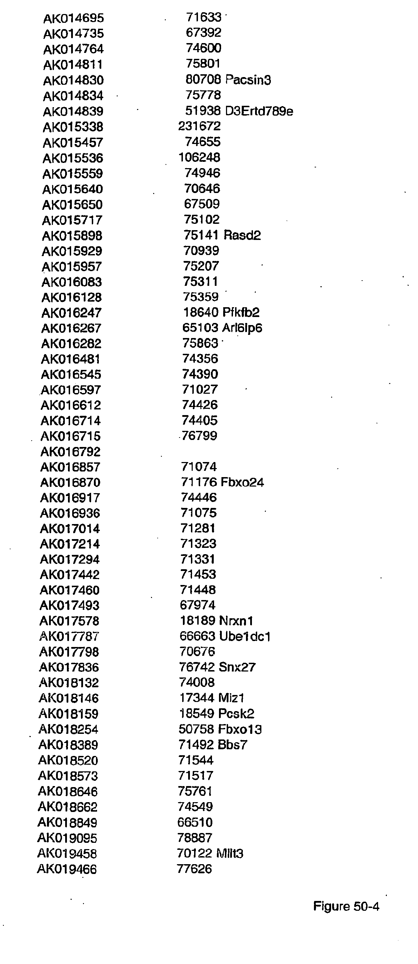

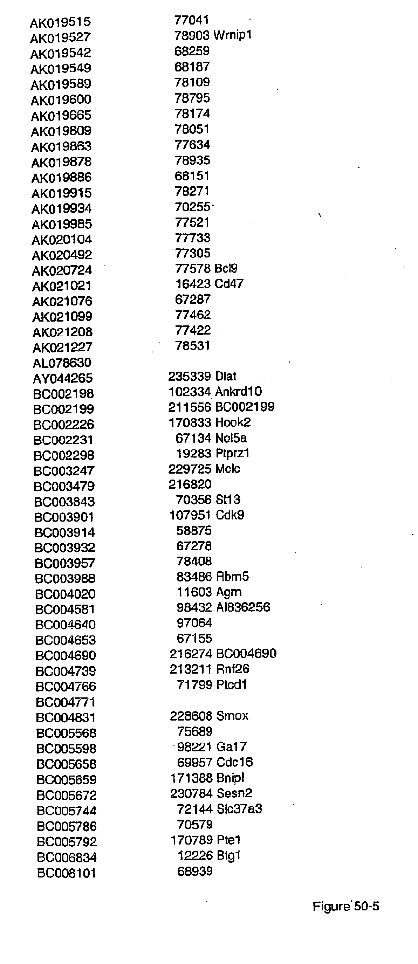

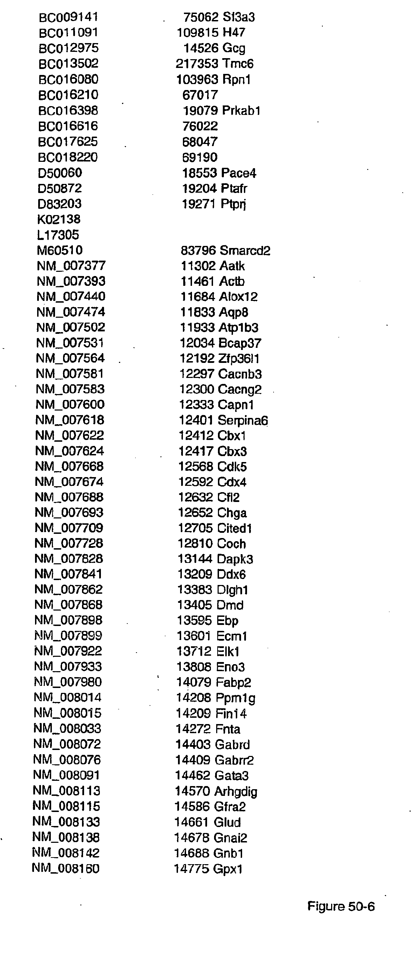

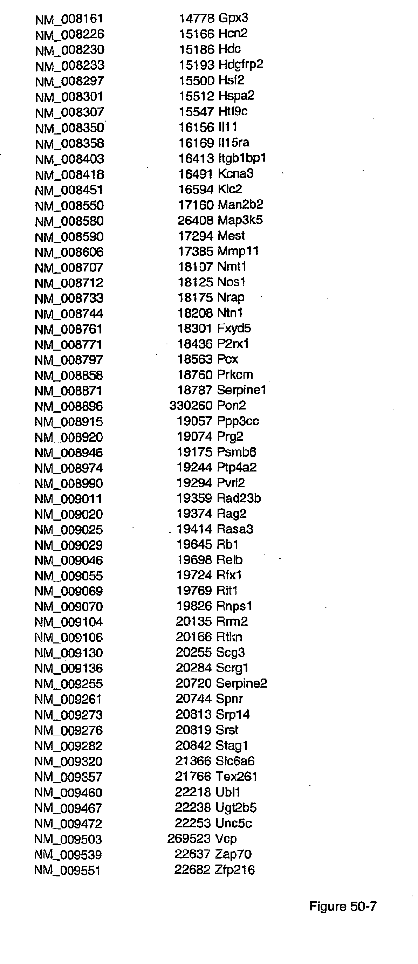

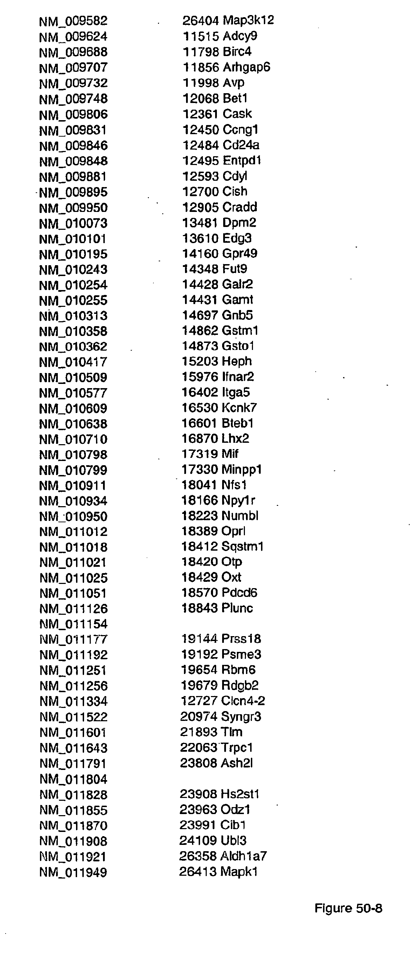

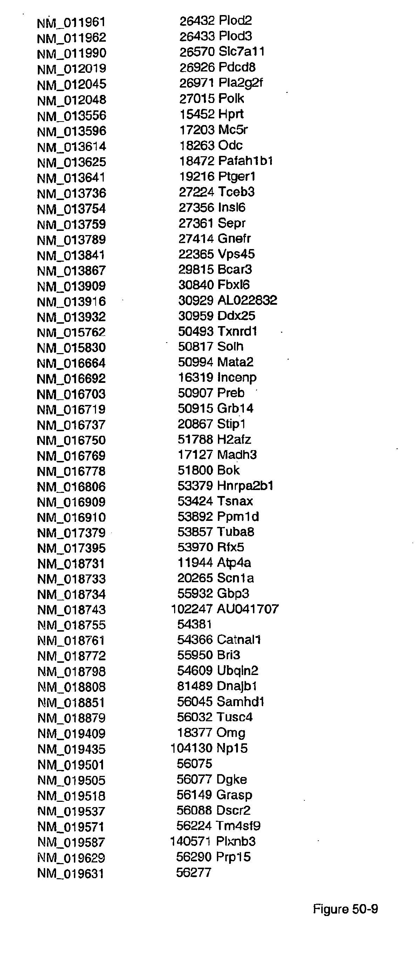

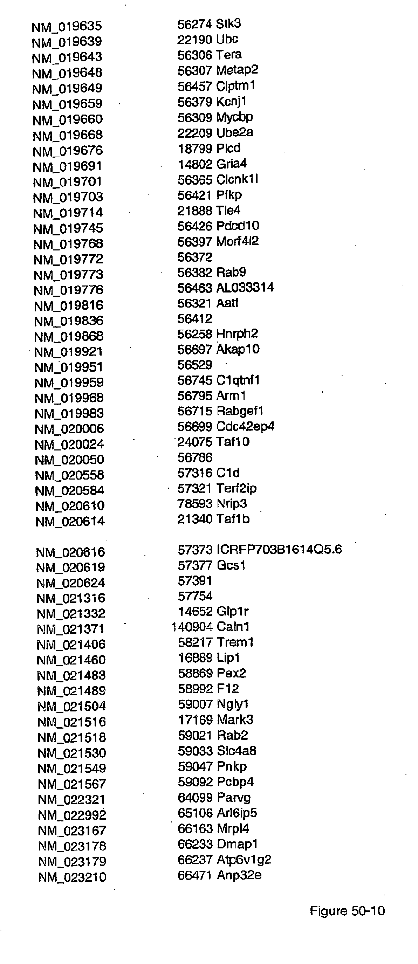

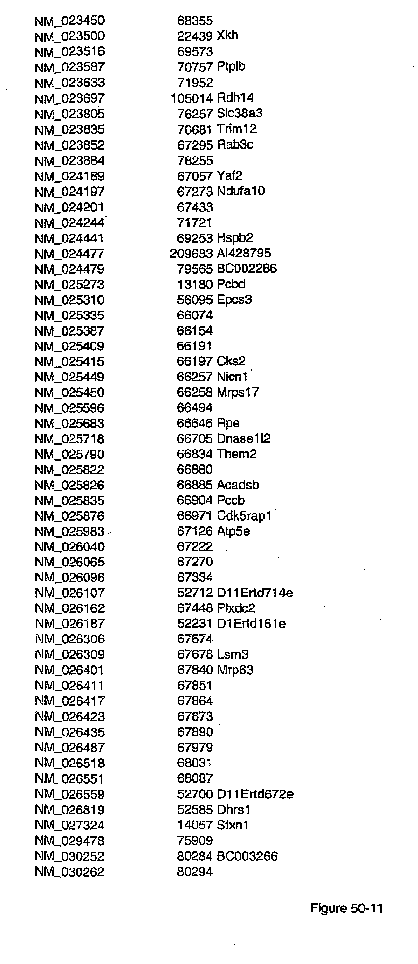

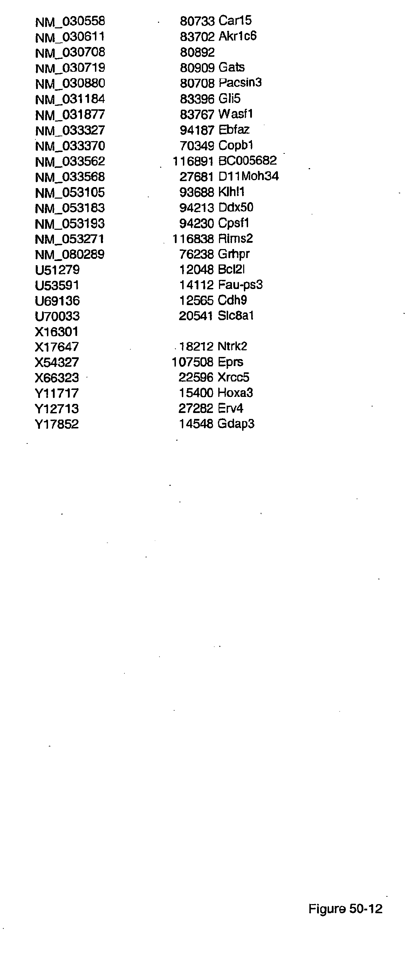

FIG. 50 is a table showing all the arthritis disease surveillance genes (differentially expressed at p < 0.05) identified in prefrontal cortex, hypothalamus and midbrain of relevant animal models. Data corresponds to genes differentially expressed in arthritic mice compared to control mice. Samples were obtained 24 days after the last LPS injection, when animals started to show arthritic symptoms.

FIGS. 51A, 51B, 51C & 51D are tables showing the differentially expressed genes (p< 0.05) in prefrontal cortex of arthritic mice. Samples were obtained 24 days after the last lipopolysaccharide injection, when animals started to show arthritic symptoms. Differentially expressed genes were identified by paired samples t-test. The base 2 logarithm of arthritic vs. control ratio is shown as a gray scale. BNS corresponds to data obtained without background subtraction, and BS corresponds to data obtained after background subtraction.

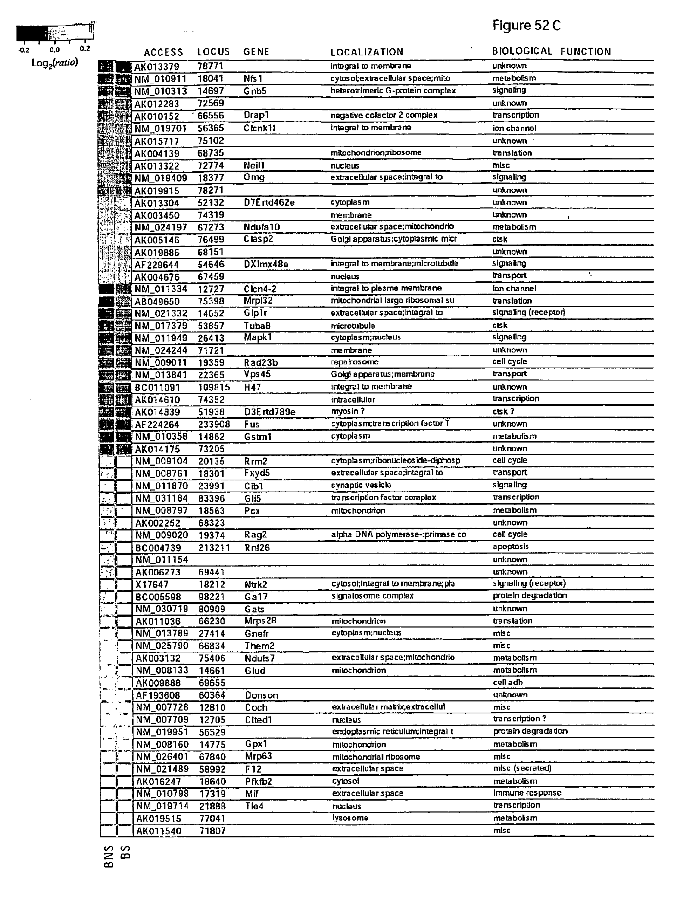

FIGS. 52A, 52B, 52C & 52D are tables showing the differentially expressed genes (p < 0.05) in hypothalamus of arthritic mice. Samples were obtained 24 days after the last lipopolysaccharide injection, when animals started to show arthritic symptoms. Differentially expressed genes were identified by paired samples t-test. The base 2 logarithm of arthritic vs. control ratio is shown as a gray scale. BNS corresponds to data obtained without background subtraction, and BS corresponds to data obtained after background subtraction.

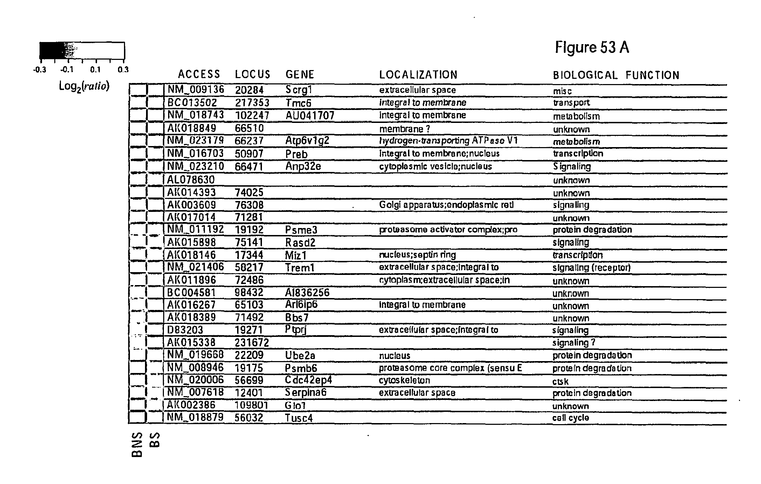

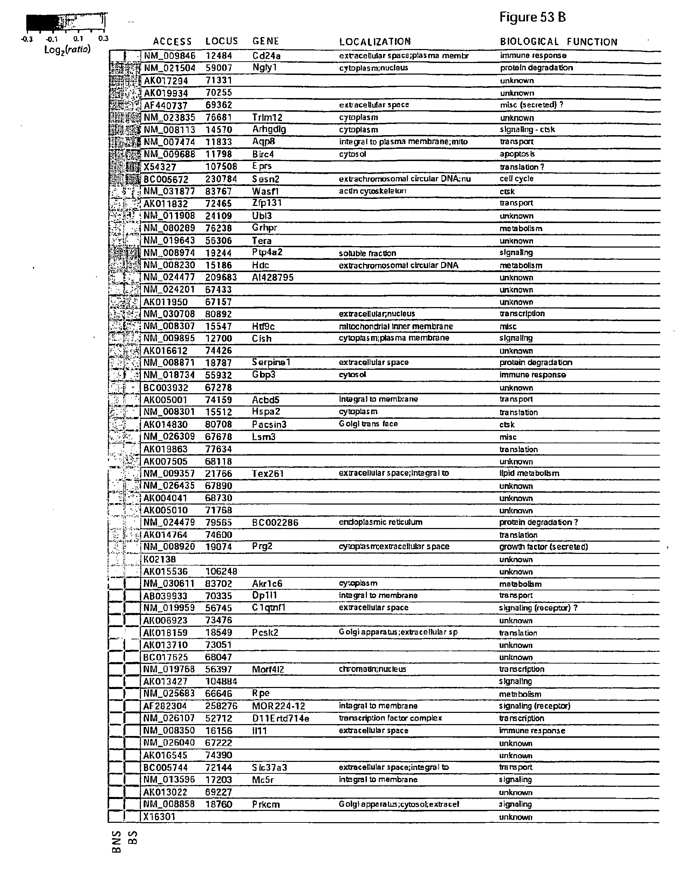

FIGS. 53A, 53B, 53C & 53D are tables showing the differentially expressed genes (p < 0.05) in midbrain of arthritic mice. Samples were obtained 24 days after the last lipopolysaccharide injection, when animals started to show arthritic symptoms. Differentially expressed genes were identified by paired samples t-test. The base 2 logarithm of arthritic vs. control ratio is shown as a gray scale. BNS corresponds to data obtained without background subtraction, and BS corresponds to data obtained after background subtraction.

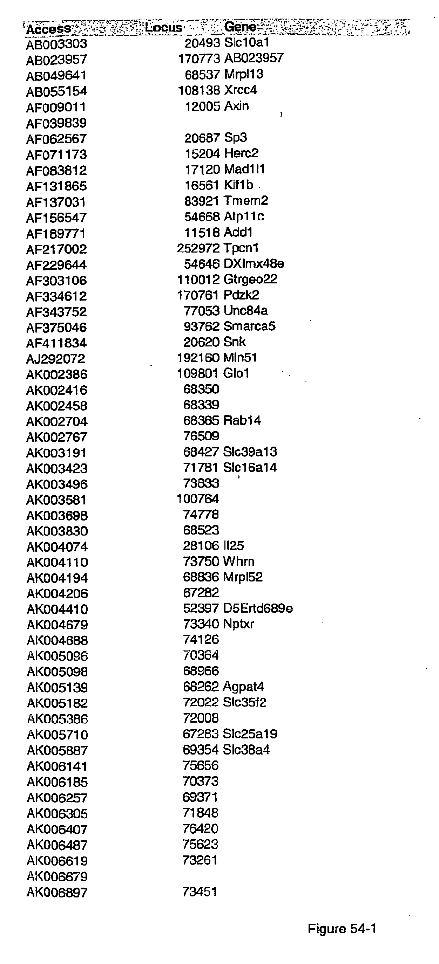

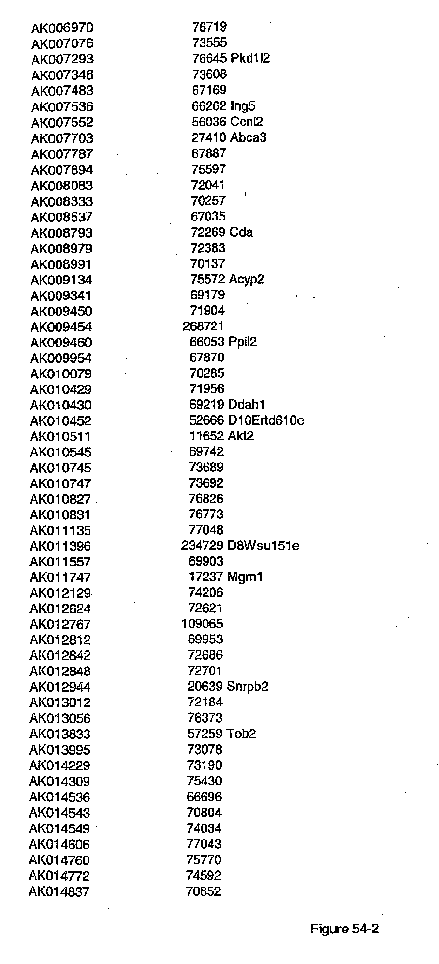

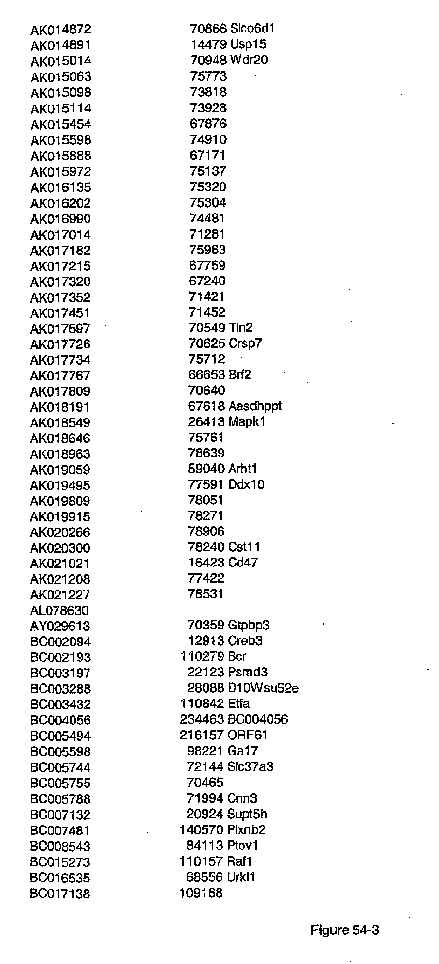

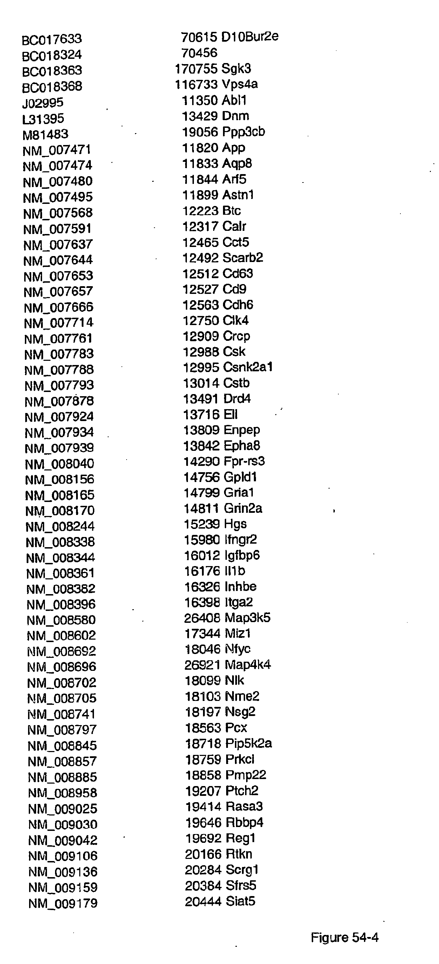

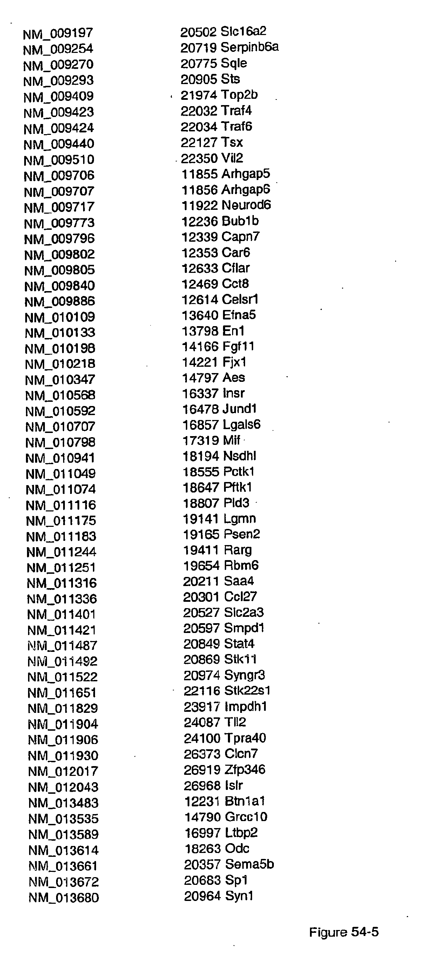

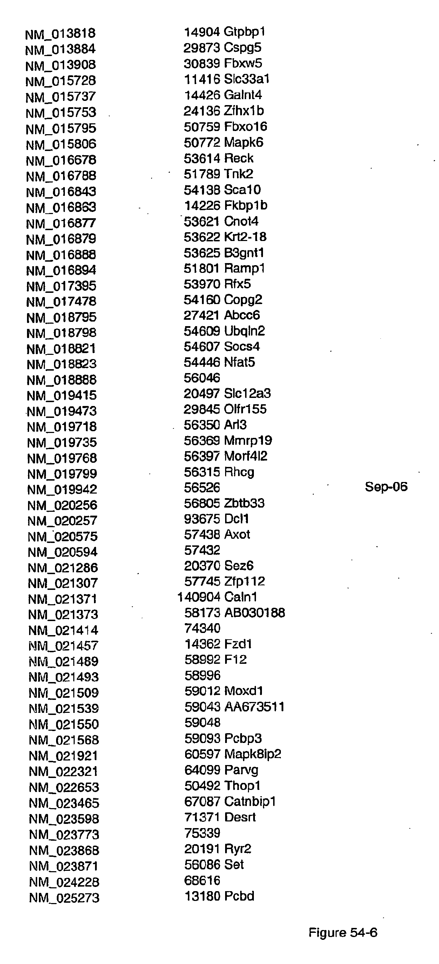

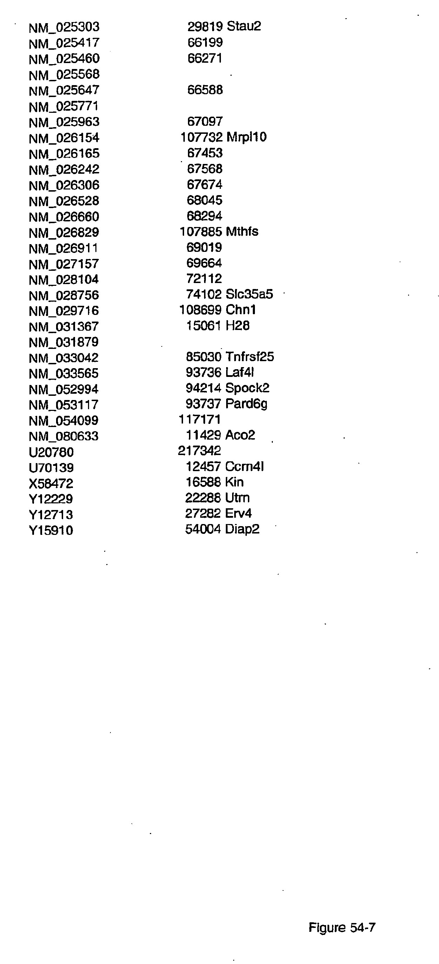

FIG. 54 is a table showing all the Asthma disease surveillance genes (differentially expressed at p < 0.05) identified in prefrontal cortex, hypothalamus and midbrain of relevant animal models. Data corresponds to genes differentially expressed in asthmatic mice compared to control mice. Samples were obtained two days after the last aerosol ovalbumin exposure.

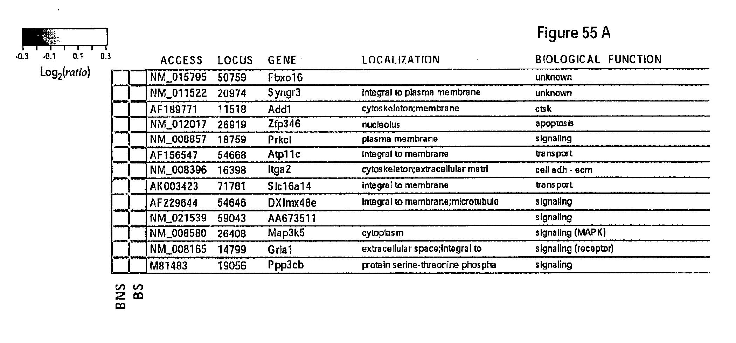

FIGS. 55A, 55B & 55C are tables showing the differentially expressed genes (p< 0.05) in prefrontal cortex of asthmatic mice. Samples were obtained two days after the last aerosol ovalbumin exposure. Differentially expressed genes were identified by paired samples t-test. The base 2 logarithm of asthmatic vs. control ratio is shown as a gray scale. BNS corresponds to data obtained without background subtraction, and BS corresponds to data obtained after background subtraction.

FIGS. 56A & 56B are tables showing the differentially expressed genes (p < 0.05) in hypothalamus of asthmatic mice. Samples were obtained two days after the last aerosol ovalbumin exposure. Differentially expressed genes were identified by paired samples t-test. The base 2 logarithm of asthmatic vs. control ratio is shown as a gray scale. BNS corresponds to data obtained without background subtraction, and BS corresponds to data obtained after background subtraction.

FIGS. 57A & 57B are tables showing the differentially expressed genes (p< 0.05) in midbrain of asthmatic mice. Samples were obtained two days after the last aerosol ovalbumin exposure. Differentially expressed genes were identified by paired samples t-test. The base 2 logarithm of asthmatic vs. control ratio is shown as a gray scale. BNS corresponds to data obtained without background subtraction, and BS corresponds to data obtained after background subtraction.

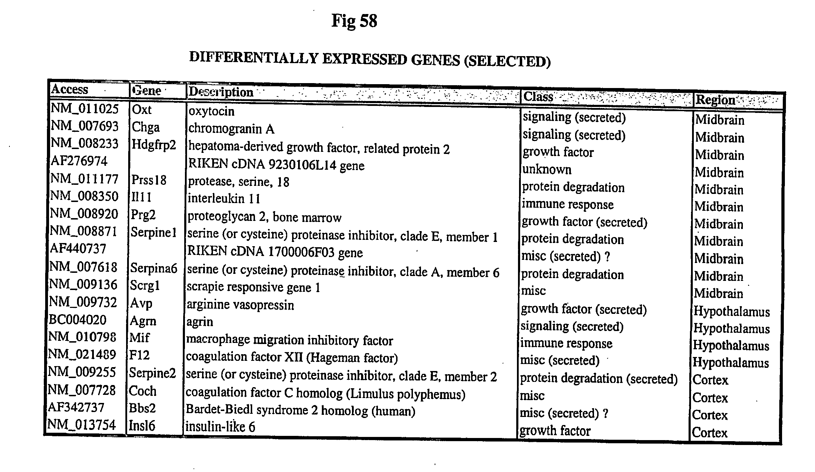

FIG. 58 is a table listing arthritis specific CNS markers differentially expressed, at the time tested. Criteria for inclusion in this figure were (1) the marker corresponds to a secreted product; and (2) a p value below 0.05 for differential expression.

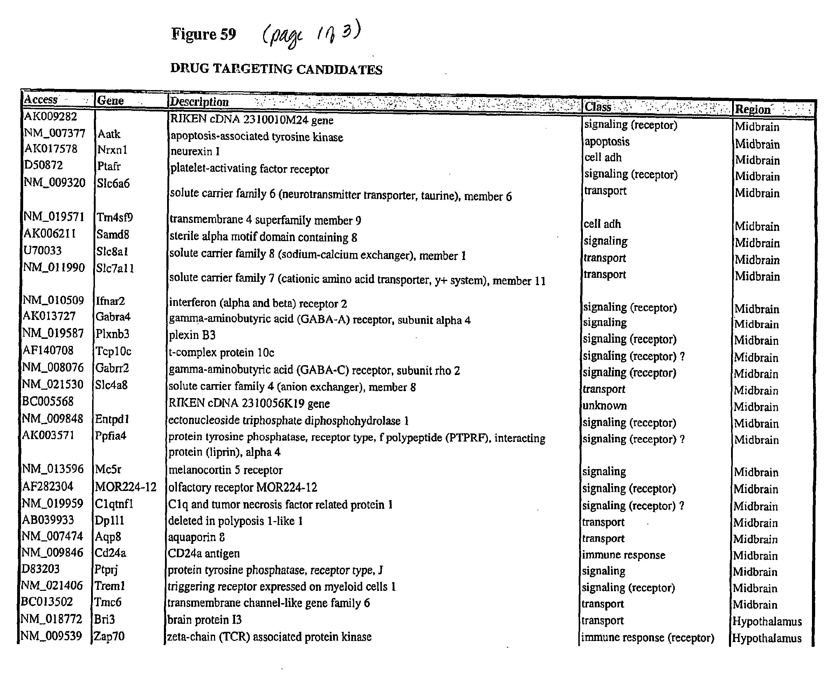

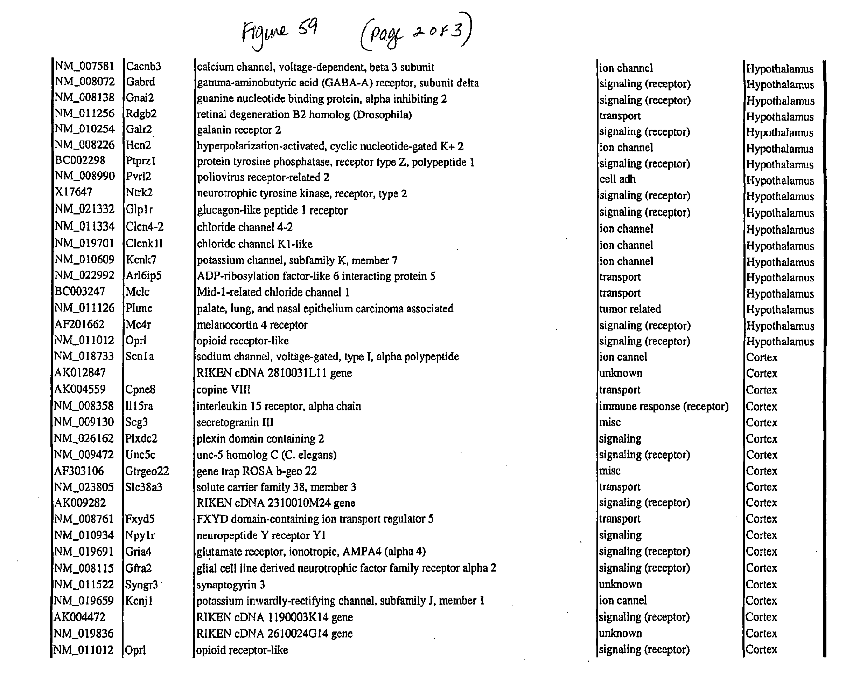

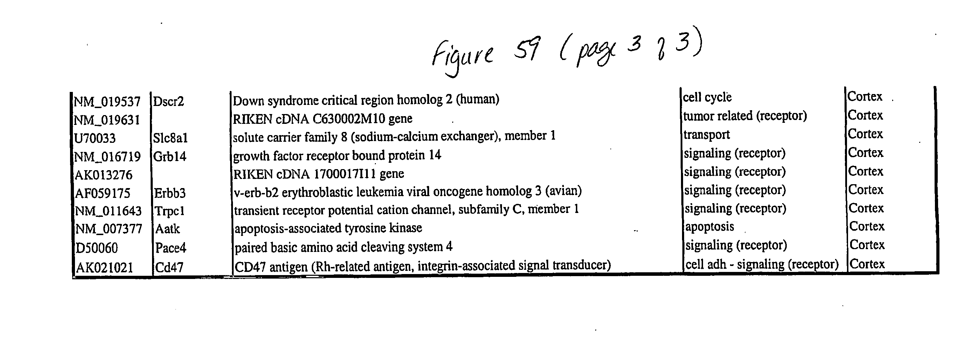

FIG. 59 is a table listing genes identified as CNS markers that are also potential targets for therapeutic intervention for arthritis. Criteria for inclusion in this figure were (1) the marker corresponds to a signaling receptor such as a growth factor, hormone, or cytokine; and (2) a p value for differential expression below 0.05.

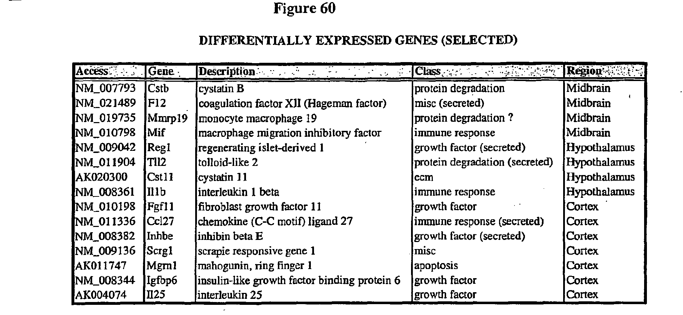

FIG. 60 is a table listing asthma specific CNS markers differentially expressed, at the time tested. Criteria for inclusion in this figure were (1) the marker corresponds to a secreted product; and (2) a p value below 0.05 for differential expression.

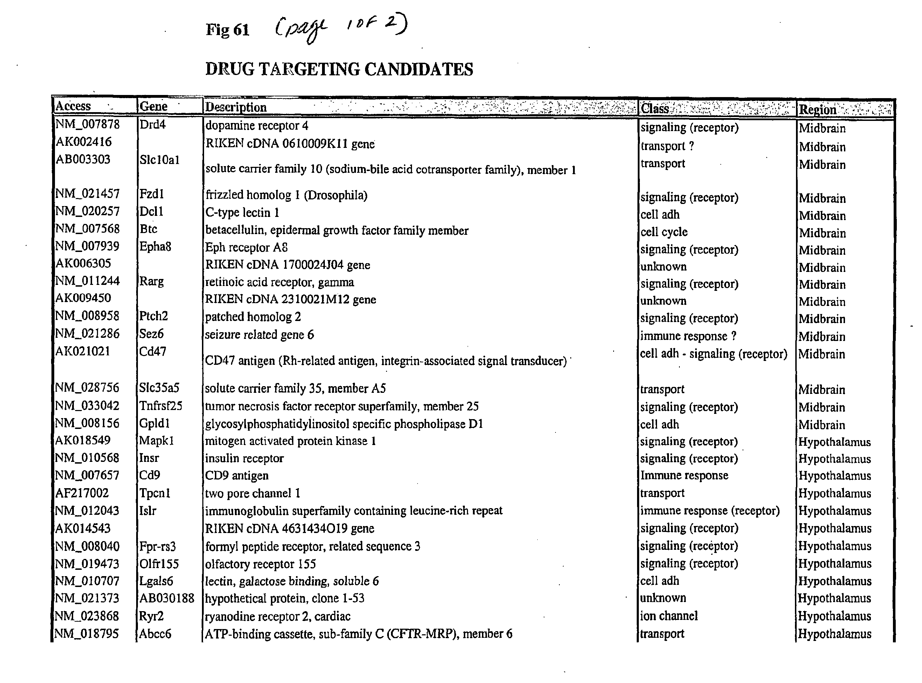

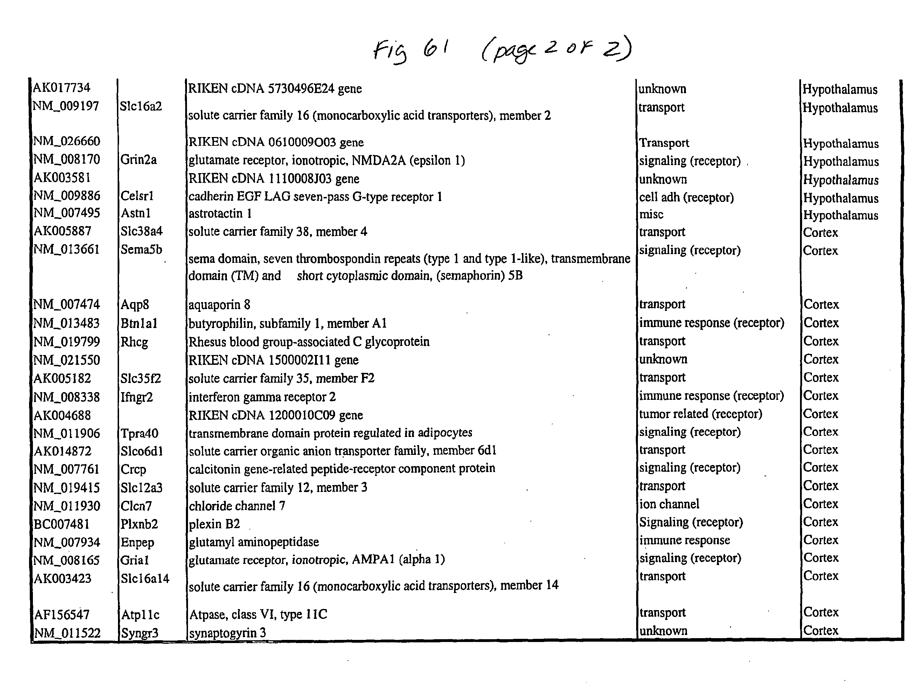

FIG. 61 is a table listing genes identified as CNS markers that are also potential targets for therapeutic intervention for asthma. Criteria for inclusion in this figure were (1) the marker corresponds to a signaling receptor such as a growth factor, hormone, or cytokine; and (2) a p value for differential expression below 0.05

**DETAILED DESCRIPTION**

The methods described herein rely, in part, on the detection of gene expression in the CNS to identify (e.g., diagnose or monitor) peripheral (non-CNS) tissues or organs for early stages of disease (e.g., in some cases, within hours, days, weeks or months of the appearance of disease). Early identification and/or diagnosis of disease provides an opportunity for early therapeutic intervention to target the disorder before it becomes overly advanced or aggressive.

General Methodology

The CNS is involved in the body's response to any internal or external stimulus that by its intensity or functional relevance could alter internal homeostasis. As part of this function, the CNS and the immune system interact to obtain a suitable immune response when necessary.

An immune response impacts the brain via neural and humoral mechanisms. Neural mechanisms primarily involve the activation of the vagal nerve. Humoral mechanisms can include cytokine-mediated action directly on brain structures, e.g., cytokine-mediated increases on neural firing rates (Rothwell and Hopkins, 1995, Trends Neurosci 18 (3):130-6; Wang et al., 2003, Nature, 421(6921):384-8). In one example, peripheral cytokines have been shown to bind and activate the vagal nerve, which in turn activates neurons of the nucleus of the tractus solitarius and the hypothalamus in the brain. (Watkins and Maier, 1999, Proc. Natl. Acad. Sci. USA, 96(14):7710-3).

Humoral signals from the periphery act as potent messengers to the brain. Cytokines in the brain can exert their action at a much lower dose than in the periphery. For example, intracerebral administration of interleukin-1 (IL-1) at a dose of 100 pg to 10 ng elicits maximal changes in fever, gastric function, increased metabolism and behavioral changes, while several micrograms of this cytokine are necessary to elicit similar responses when administered to the periphery (Rothwell and Hopkins, *supra*).

After sensing an internal immune signal, the brain reacts in different ways. A paradigm of CNS response to immune signals is the activation of neuroendocrine axes such as the hypothalamus-pituitary-adrenal axis. The activation of this axis results in the liberation of glucocorticoids, which in turn can modulate the ongoing immune response in under 10 minutes. Vagatomy has been shown to blunt the activation of the hypothalamus pituitary adrenal axis after intraperitoneal administration of cytokines (Watkins and Maier, *supra*). This feedback mechanism is of high physiological relevance; i.e., inhibition of glucocorticoid production after cytokine release in the periphery usually results in the death of the organism (Besedovsky and del Rey, 1996, Endocr. Rev., 17(1):64-102).

The brain can also sense signals that will affect the immune and other systems from the external milieu. For example, the triggering of a stress reaction can result in the release of glucocorticoids and the attenuation of an ongoing immune response. The effects of stress on the immune system are well documented in animal models and humans (Deinzer et al., 2000, Int. J. Psychophysiol., 37(3):219-32; Marshall et al., 1998, Brain Behav. Immun., 12(4):297-307; Benschop et al., 1996, FASEB J., 10(4):517-24; Sheridan et al., 1998, Ann. N.Y Acad. Sci., 840:803-8). In addition, there is anecdotal and preliminary evidence that mind/body interventions such as meditation or yoga could have an influence on the immune system (Cassileth, 1999, CA Cancer J. Clin., 49(6):362-75).

The new methods harness this natural reaction of the CNS as a way to detect peripheral disease at an early stage. While not limited by any theory, the methods described herein are based, in part, on the discovery that the CNS senses the presence of "alarm signals" from peripheral (non-CNS) disorders at an early stage in the development of disease progression. Thus, the methods described herein relate to diagnosing peripheral disorders by detecting gene expression in the CNS, e.g., in a CNS sample from a subject, such as a human, or from any other bodily fluid where CNS gene products or derivatives thereof could be detected. In one aspect, a non-CNS disorder can be identified based on a profile of gene expression in the CNS (e.g., the brain) within hours, weeks or months after disease progression is initiated in the body. In some embodiments, a non-CNS disorder can be identified based on a profile of gene expression in the CNS (e.g., the brain) within one or more years (e.g., 2, 3, 5, 7, 10 or more years) after disease progression is initiated in the body, but before a disorder is clinically detectable and/or in an advanced stage.

Cancer Development

It is generally accepted that a clinically detectable tumor mass is composed of cells that, although abnormal, evade immune surveillance and resist immune system attack. During the time of neoplastic progression, cells are characterized by high mutation rates, reflected, *inter alia,* in phenotypic changes such as down-regulation of histocompatibility antigens. A tumor may thus become resistant to a particular therapeutic by clonal selection and proliferation from the tumor mass of a cell clone having a mutation that allows the cell to resist the given therapeutic. The "natural selection" of tumor cell clones occurs at a given rate leading to the appearance of malignant cells having genetic and epigenetic traits that facilitate growth and escape from the immune system. It is estimated that the average malignancy contains more than 10,000 mutations (Stoler et al., 1999, Proc. Natl. Acad. Sci., USA., 96(26):15121-6). Therefore, it can be concluded that the antigen profile of established cancers by no means reflects the cell genotype and phenotype of very early stage neoplasia. Moreover, it is reasonable to assume that tumor antigens present in the established cancer and the response they can induce in the organism will be different than the antigens and responses induced by early stage neoplastic cells. The new methods described herein can detect such early stage neoplastic cells in spite of these obstacles.

Some neoplasms, e.g., some cancers (e.g., certain types of carcinoma) can grow for long periods (e.g., for 1, 2, 5, 10, 15, 20 or 25 years) before they are clinically detectable using prior known technology and/or before they become malignant. This period provides an extraordinary window of opportunity for detection of cancerous cells before the malignant tumor is clinically detectable by current strategies. During this period tumor cells undergo several modifications at the molecular level as a result of their genomic instability.

Each genetic change is potentially selective for proliferation and/or is capable of triggering a new "alarm signal" to recruit and activate local innate and adaptive immune responses. In a simple view, 10,000 alarm signals are produced during the 10 to 15 years of tumor development before the tumor is clinically detectable.

Development of Rheumatoid Arthritis

Rheumatoid arthritis (RA) is an acquired autoimmune disease in which genetic factors appear to play a role. RA occurs in 1-2 percent of the general population and is found world-wide. Females with RA outnumber males by 3:1. Onset of the disease in adults is usually between the ages of 40 to 60 years, although it can occur at any age.

RA involves Th1 lymphocytes and macrophage infiltration into joints as well as the presence of rheumatoid factors in patients' serum (Chernajovsky et al., 2000, Genes Immun., 1:295-307). Degradation of cartilage is accompanied by the outgrowth of synovial membrane (pannus). This process is generally regulated by IL-1 and TNF-$\alpha$, while TGF-$\beta$ and IL-10 counteract this effect (Chernajovsky et al., *ibid*). Susceptibility to arthritis has been correlated with MHC class II locus, in particular HLA-DR4 in 70 percent of patients with RA (Chernajovsky et al., *ibid*). Rheumatoid Factor(s) (RF) are antibodies to IgG, and are present in 60-80 percent of adults with the disease. High titers of RF are usually associated with more severe and active joint disease, greater systemic involvement, and a poorer prognosis for remission.

An unknown antigen is thought to initiate the autoimmune response resulting in RA. It has been suggested that there is a synovial antigen resembling a bacterial lipopolysaccharide (LPS) of arthritogenetic bacteria that initiates the autoimmune response (Kennedy, 2000, Med. Hypotheses, 54(5):723-5). TNF-$\alpha$ appears to be the driving force behind the chronic inflammation characteristic of RA. TNF-$\alpha$ plays also an important role in B cell maturation which appears to participate in disease progression (Chernajovsky et al., *ibid*). Some data also strongly indicate a role for Suppressor of Cytokine signaling (SOCS) in disease outcome (Egan et al., 2003, J. Clin. Invest. 111(6):915-24).

The initiation of the autoimmune response and/or the initiation of the inflammatory mechanisms in the early development of RA trigger signals detected by changes in gene expression in the CNS.

Development of Asthma

Asthma is an inflammatory airway disease characterized by the presence of cells such as eosinophils, mast cells, basophils, and CD25+ T lymphocytes in the airway walls. Chemokines attract cells to the site of inflammation and cytokines (Interleukin (IL)-4, IL-5, EL-10 and IL-13) activate them, resulting in inflammation and damage to the mucosa. When asthma becomes chronic, secondary changes occur, such as thickening of basement membrane and fibrosis. IL-4 and other cytokines such as TGF-$\beta$ may be involved in tissue remodeling and the fibrotic response.

In allergic asthma (also known as extrinsic asthma), the initiation event of airway inflammation is an immunological reaction to allergen. Continued exposure to allergen results in chronic inflammation. Allergic asthma affects about 3 million children (8 to 12 percent of all children) and 7 million adults in the United States at a cost estimated at $6.2 billion a year. It has been suggested that longitudinal studies based on yet unidentified inflammatory markers will guide asthma management in the future (Wilson, 2002, Curr. Opin. Pulm. Med., 8(1):25-32).

In the development of asthma, the initiation of the allergic or inflammatory response, e.g., release of cytokines and/or chemokines, can trigger signals detected by changes in gene expression in the CNS.

Development of Obesity

Body size and body weight are highly heritable traits. Association studies performed with populations of monozygotic and dizygotic twins, non-twin siblings and adoptive family members indicated that the variance for body mass index (body weight divided by height to the square) is much lower in identical twins that in any other group, indicating that genetic factors rather than environmental effects are the key determinant of human adiposity (Maes et al., 1997, Behav. Genet., 27:325-351; Allison et al., 1996, Int. J. Obes. Relat. Metab. Disord., 20:501-506). Diet-induced obesity is also highly heritable. A pioneer study performed in 12 pairs of young adult identical twins overfed by 1,000 kcal per day during a 100-day period demonstrated that overfeeding induced a variable increase in body weight in all volunteers. However, twin pairs had six times less variance in mass increase than non-twin pairs, indicating that adaptation to long-term overfeeding has important genetic factors (Bouchard et al., 1990, N. Engl. J. Med., 322:1477-1482). The strong genetic predisposition to gain weight after ingesting a fat-rich diet is even more clearly observed in the laboratory when testing mice or rats of different genetic backgrounds (Schaffhauser et al., 2002, Obes. Res., 10:1188-1196). Most strains of mice maintain their body weight throughout relatively long periods of time while being fed *ad libitum* with low fat diets. However, when fed *ad libitum* with a high fat diet, some strains develop a considerable increase in body mass and some other strains are resistant to this increase regardless of increase in food consumption (West et al., 1995, Am. J. Physiol., 268:R658-R665; Prpic et al., 2003, Endocrinology, 144:1155-1163).

The regulation of body weight involves a large number of interconnected peripheral and brain circuits that participate in the control of energy balance throughout the entire organism (Spiegelman and Flier, 2001, Cell, 104: 531-43). Information about the amount of energy stored in the whole body is transported into the brain by peripheral hormones such as leptin and insulin. The relative variation of the plasma concentration of these hormones is interpreted by central mechanisms to induce signals of appetite or satiety (Friedman and Halaas, 1998, Nature, 395:763-70). Other molecules such as ghrelin and cholecystokinin (CCK) enter into the brain after being released from different portions of the gastrointestinal tract and provide essential information to brain centers about the nutritional status of the organism (Murakami et al., 2002, J. Endocrinol., 174:283-288; Sheng and Moran, 2002, Neuropeptides, 36:171-181).

The hypothalamus, a critical brain area for the complicated control of energy homeostasis, integrates a variety of converging signals within a short time frame. In the ventral hypothalamus a group of appetite-inducing neurons expresses the neuropeptide Y (NPY) gene. As leptin levels drop from circulation NPY is released into the paraventricular nucleus of the hypothalamus to induce food intake (Widdowson et al., 1999, Peptides, 20:367-372). A single intracerebroventricular administration of NPY in mice or rats can dramatically increase food intake for many hours (Zarjevski et al., 1993, Endocrinology, 133:1753-1758). Conversely, another group of neurons located in the arcuate nucleus of the hypothalamus expresses the proopiomelanocortin gene (POMC). These neurons also express the leptin receptor gene. After an excessive intake of fatenriched food, the levels of triglycerides rise, filling peripheral adipocytes with fat stores. This leads to an increase in production of leptin, which is released into the circulation and eventually enters the brain by a selective uptake mechanism (Hileman et al., 2002, Endocrinology, 143:775-783). Leptin stimulates leptin receptors located in POMC neurons, thereby increasing their firing activity (Cowley et al., 2001, Nature, 411:480-484).

One of the active peptides produced by the POMC precursor is $\alpha$-melanocyte stimulating hormone ($\alpha$-MSH). Upon stimulation of leptin receptors, $\alpha$-MSH is released in the paraventricular nucleus of the hypothalamus to induce satiety. Intracerebroventricular injections of $\alpha$-MSH in mice or rats induce long lasting anorexia that can promote the death of the animals if they are not forced to feed (Fan et al., 1997, Nature, 385:165-168).

The hormones, neuropeptides and their receptors described above are only a few examples of the many gene products that participate in the central control of energy balance. Regulation of a molecule involved in energy control (e.g., a disruption associated with propensity or presence of obesity) can likely trigger signals that result in changes in gene expression in the CNS.

Methods Of Detecting Gene Expression

Gene expression in the CNS can be detected in vitro, e.g., in an isolated CNS sample, or in vivo, e.g., using in vivo imaging techniques.

Central Nervous System (CNS) Samples

The CNS refers to the brain (including the cranial nerves) and spinal cord. A CNS sample can be, e.g., a cell or tissue from the brain or spinal cord, or a sample of the cerebrospinal fluid (CSF) that fills the ventricles of the brain and the central canal of the spinal cord.

Where the detection of gene expression is to be done in a CNS sample isolated from the subject, a CNS sample can be obtained by any number of methods available to the skilled artisan. For example, a CNS cell or tissue sample can be obtained from the brain, e.g., by needle biopsy or by open surgical incision. Imaging of the brain can be performed to determine the precise positioning of the needle or scalpel to enter the brain.

In one example, known as stereotactic biopsy, a tiny hole is drilled into the skull with the patient under light sedation or general anesthesia, and a needle is inserted into the brain tissue guided by computer-assisted imaging techniques such as computerized tomography (CT) or magnetic resonance imaging (MRI) scans. The needle is used to remove a sample of cells, whose gene expression can then be detected by a routine assay, e.g., a gene expression assay described herein. In another example, a sample of CSF can be obtained by routine methods, such as by lumbar puncture. This procedure can be done on an outpatient basis, e.g., under local anesthetic.

The number of cells or amount of CSF needed to perform a particular gene expression assay on a CNS sample will vary; however, some techniques, such as PCR based techniques, will require a very small number of cells, e.g., as few as 10 to 100 cells (Klein et al., Nat. Biotechnol., 20(4):387-92, 2002). The CNS sample can be used immediately in a diagnostic test described herein, or it can be stored, e.g., cooled or frozen, and/or transported to a facility where the diagnostic test is performed.

Nucleic Acid-Based Methods

In one embodiment, the methods described herein will utilize techniques for detection of gene expression where a polynucleotide (such as an RNA, mRNA, DNA, cDNA, or other nucleic acid corresponding to the gene) is detected. It should be understood by the skilled artisan that many methods for nucleic-acid based detection of gene expression exist and that any suitable method for detection can be used. Typical assay formats utilize nucleic acid hybridization and include, e.g., 1) nuclear run-on assay, 2) slot blot assay, 3) northern blot assay, 4) magnetic particle separation, 5) nucleic acid or DNA arrays or chips (also discussed in more detail below), 6) reverse northern blot assay, 7) dot blot assay, 8) in situ hybridization, 9) RNase protection assay, 10) ligase chain reaction, 11) polymerase chain reaction (PCR), 12) reverse transcriptase (RT)-PCR, and 13) differential display RT-PCR (DDRT-PCR) or any combination of any two or more of these methods. Such assays can employ the use of detectable labels such as radioactive labels, enzyme labels, chemiluminescent labels, fluorescent labels, or other suitable labels, to detect, identify, or monitor the presence or level of a particular nucleic acid being detected. Such techniques and labels are known in the art and widely available to the skilled artisan.

In one embodiment, an RNase protection assay can be utilized in the methods described herein by hybridizing multiple DNA probes corresponding to one or more members of a panel of sequences to mRNA isolated from a CNS sample from a subject to be tested. The expression profile for one or more genes from the CNS sample can be compared to a reference gene expression profile, e.g., a basal pattern of expression, or other negative or positive control (e.g., a profile from a patient known to have no peripheral disease, or a standard or average profile derived from subjects known to not have the particular disorder being tested). In one example, the gene expression profile from the test CNS sample is compared to a reference gene expression profile that is associated with the presence of a non-CNS neoplasia. If the test gene expression profile matches the reference gene expression profile, it indicates that the subject has, or is at risk for developing, the non-CNS neoplastic disorder. As used herein, "matches" means that at least 75% of the genes in a test gene expression profile are either up- or down-regulated in the same manner as the genes in the reference expression profile.

The methods described herein are also well suited for polymerase chain reaction (PCR)-based methods. PCR-based methods include RT-PCR (U.S. Patent No. 4,683,202), ligase chain reaction (Barany, Proc. Natl. Acad. Sci. USA, 88:189-193, 1991), self-sustained sequence replication (Guatelli et al., Proc. Natl. Acad. Sci. USA, 87:1874-1878, 1990), transcriptional amplification system (Kwoh et al., Proc. Natl. Acad. Sci. USA, 86:1173-1177, 1989), Q-Beta Replicase (Lizardi et al., BioTechnology, 6:1197, 1988), rolling circle replication (Lizardi et al., U.S. Patent No. 5,854,033), or any other nucleic acid amplification method, followed by the detection of the amplified molecules using techniques known in the art. PCR amplification of mRNAs expressed in a CNS sample can be performed directly from mRNA isolated from the sample, or from cDNA reverse-transcribed from such isolated mRNA. The amplified nucleic acid can then be hybridized to a particular probe of interest, e.g., a probe for a CNS gene as described herein, to determine its expression. The probe can be disposed on an address of an array, *e.g.*, an array described herein. Such methods are routine and are particularly amendable to routine adaptation to automated systems employing computer controlled reagent aliquoting and signal detection. See, e.g., Klein et al., Nat. Biotechnol., 2002, 20(4):387-92.

In another embodiment, *in situ* methods are used to detect the presence or level of mRNA corresponding to a particular gene. In such methods, a CNS cell or tissue sample can be prepared/processed and immobilized on a support, typically a glass slide, and then contacted with a probe (e.g., a probe for a CNS gene described herein).

In still another embodiment, serial analysis of gene expression, as described in U.S. Patent No. 5,695,937, is used to detect transcript levels of a CNS gene described herein.

Polypeptide-Based Methods

In other embodiments, the methods described herein utilize techniques for detection of gene expression where a gene product (polypeptide) encoded by a gene is detected or where an activity of the polypeptide, e.g., an enzymatic activity, is detected. Such methods are particularly advantageous for detecting the expression of genes that encode polypeptides that are secreted from CNS cells, e.g., into the CSF.

A variety of methods can be used to determine the level of protein encoded by a CNS gene. In general, these methods include contacting a CNS sample (such as a brain cell sample or a CSF sample) with an agent, such as an antibody, that selectively binds to the protein of interest. In one embodiment, the antibody bears a detectable label. Antibodies can be polyclonal, or more preferably, monoclonal. An intact antibody, or a fragment thereof (e.g., Fab or F(ab')2) can be used. The term "labeled," with regard to the probe or antibody, is intended to encompass direct labeling of the probe or antibody by coupling (i.e., physically linking) a detectable substance to the probe or antibody, as well as indirect labeling of the probe or antibody by reactivity with a detectable substance. Such detection methods can be used to detect a CNS gene product in a CNS sample *in vitro* as well as *in vivo*.

*In vitro* techniques include immunoassays such as enzyme linked immunosorbent assays (ELISAs), immunoprecipitations, immunofluorescence, enzyme immunoassay (EIA), radioimmunoassay (RIA), Western blot analysis, and Luminex™ x MAP™ detection assay. Some immunoassays are "sandwich" type assays, in which a target analyte(s) is "sandwiched" between a labeled antibody and an antibody immobilized onto a solid support. The assay is read by observing the presence and amount of antigen-labeled antibody complex bound to the immobilized antibody.

Another immunoassay useful in the methods described herein is a "competition" type immunoassay, wherein an antibody bound to a solid surface is contacted with a sample (e.g., a CSF sample) containing both an unknown quantity of antigen analyte and labeled antigen of the same type. The amount of labeled antigen bound on the solid surface is then determined to provide an indirect measure of the amount of antigen analyte in the sample. Such immunoassays are readily performed in a "dipstick" format (e.g., a flow-through or migratory dipstick design) for convenient use. A dipstick-based assay optionally includes an internal negative or positive control. Numerous types of dipstick immunoassays are known in the art and are described, e.g., in U.S. Patent Nos. 5,656,448; 4,366,241; and 4,770,853. In other embodiments, antibody-based assays are performed in an array format. For example, a CNS sample is labeled, e.g., biotinylated, and then contacted to an antibody, e.g., an antibody positioned on an antibody array. The sample can be detected, e.g., with avidin coupled to a fluorescent label.

*In vivo* techniques include, e.g., introducing into a subject (e.g., into the CSF) a labeled antibody that binds to the gene product to be detected. The antibody can be labeled, e.g., with a radioactive marker, whose presence and location in a subject can be detected by standard imaging techniques.

Polyclonal and monoclonal antibodies to be used to detect a particular CNS gene product will, in certain cases, be available. For example, commercially available antibodies exist for many of the CNS marker genes described herein. Alternatively, a skilled artisan can make a suitable antibody for use in a diagnostic assay using routine techniques. Methods of making and using polyclonal and monoclonal antibodies to detect a particular target are described, e.g., in Harlow et al., Using Antibodies: A Laboratory Manual: Portable Protocol I. Cold Spring Harbor Laboratory (December 1, 1998). Methods for making modified antibodies and antibody fragments (e.g., chimeric antibodies, reshaped antibodies, humanized antibodies, or fragments thereof, e.g., Fab', Fab, F(ab')2 fragments); or biosynthetic antibodies (e.g., single chain antibodies, single domain antibodies (DABs), Fv, single chain Fv (scFv), and the like), are known in the art and can be found, e.g., in Zola, Monoclonal Antibodies: Preparation and Use of Monoclonal Antibodies and Engineered Antibody Derivatives, Springer Verlag (December 15, 2000; 1 st edition).

Imaging of CNS Gene Expression

In one embodiment, the methods described herein utilize techniques for imaging of gene expression, e.g., non-invasive imaging of gene expression, in the CNS. For example, a labeled probe that is capable of detecting the expression of a target gene can be delivered into the brain through the blood-brain barrier (BBB) by targeting the labeled probe to the brain via endogenous BBB transport systems, such as carrier-mediated transport systems that exist for the transport of nutrients across the BBB. Similarly, receptor-mediated transcytosis systems operate to transport circulating peptides across the BBB, such as insulin, transferrin, or insulin-like growth factors. These endogenous peptides can act as "transporting peptides," or "molecular Trojan horses," to ferry a labeled diagnostic probe as described herein, across the BBB. The label can then be detected by known brain imaging techniques. Such an approach is described, e.g., in U.S. Patent No. 6,372,250. In other embodiments, Shi et al., Proc. Natl. Acad. Sci. USA, 2000, 97(26):14709-14 and Lee et al., J. Nucl. Med. 2002, 43(7):948-56 describe imaging of gene expression in the brain *in vivo* using an antisense radiopharmaceutical combined with drug-targeting technology to traverse the BBB.

Other methods of delivering into the brain a labeled probe that is capable of detecting the expression of a target gene are described, e.g., in U.S. Pat. No. 5,720,720. This patent describes methods of delivering agents (such as labeled antibodies for imaging gene products) into the brain by high-flow microinfusion.

Detection of Changes in CNS Gene Expression in Bodily Fluids

In some cases, gene activation in the CNS can result in a measurable alteration in a gene product at a distant site, e.g., in a fluid such as blood, urine or semen. It is known, e.g., that the cerebral cortex, hippocampus, entorrhinal cortex, parts of the thalamus, basal ganglia, cerebellum, and the reticular formation influence the output of the autonomic nervous system (Kandel et al, Principles of Neural Science, Third Edition, Appleton & Lange). These influences can result in measurable alterations of gene expression at the mRNA or protein level in autonomic ganglia or in innervated organs. An example of this type of interaction is the immunomodulatory action of the activation of the vagus nerve after cytokine release in the periphery (Tracey, Nature, 420:853-9, 2002).

In addition, gene activation in the CNS can be detected by measuring changes in blood proteins in some cases. For example, neurons in the CNS can trigger the release of hormones in blood via the activation of several neuroendocrine axes such as the hypothalamus-pituitary-adrenal, -gonadal, or thyroid axes (Besedovsky and del Rey, Endocrine Reviews, 17:1-39, 1996). Moreover, brain extracellular fluid drains into blood and deep cervical lymph (Cserr et al, Brain Pathol., 2(4):269-76, 1992). Cerebral extracellular fluids drain from brain to blood across the arachnoid villi and to lymph along certain cranial nerves (primarily olfactory) and spinal nerve root ganglia. A minimum of 14 to 47% of protein injected into different regions of brain or cerebrospinal fluid passes through lymph. Thus, CSF markers drain into, and can be detected in, lymph, blood, or serum. Such markers found in blood may also be enriched, and thereby detectable, in urine, due to selective filtration of blood components by the kidneys.

The CNS is connected to the testis via the autonomic nervous system as well as the endocrine system. If a change in gene activity in the brain results in modifications in the activity of the hypothalamus-pituitary-gonadal axis or in the innervation of the testes, these changes could be then detected in fluids related to the testes, such as semen. For example, patients with spinal cord injury have been shown to have alterations in the composition of their semen (See Naderi and Safarinejad, Clin. Endocrinol., 58(2):177-84, 2003).

Routine methods can be used to identify gene products in peripheral tissues, such as peripheral bodily fluids, which are the result of changes in gene expression in the CNS. For example, a candidate marker gene can be disrupted in the brain of an experimental non human animal. A change in the expression of a candidate gene in a peripheral tissue in the experimental animal, compared to a wild type animal (i.e., an animal not disputed for the candidate marker gene) indicates that the expression of the candidate molecule in the peripheral tissue is tied to changes in gene expression in the CNS.

Arrays

The methods described herein are readily adapted for nucleic acid or protein arrays, e.g., nucleic acid and/or protein "chips," following the methods known in the art. In a typical embodiment, an array chip includes multiple probes (e.g., DNA probes and/or antibody probes) for detection of expression of multiple CNS genes. In one embodiment, the probes on a specific chip are chosen to detect the members of one or more specific panels or "clusters" of genes, each cluster being associated with a specific gene expression profile if a non-CNS neoplasia or other disorder is present in the subject from whom the CNS sample was taken. A chip can contain tens, hundreds, or thousands of individual probes immobilized (tethered) at discrete, predetermined locations (addresses or "spots") on a solid, planar support, e.g., glass, metal, or nylon. An array can be a macroarray or microarray, the difference being in the size of the spots. Macroarrays contain spots of about 300 microns in diameter or larger and can be imaged using gel or blot scanners. Microarrays contain spots less than 300 microns, typically less than 200 microns, in diameter.

For analysis and comparison of profiles of gene expression in the methods described herein, a nucleic acid array can be constructed using nucleic acid probes for at least four, e.g., at least 10, 20, 40, 60, 80 or 100 CNS genes. Such an array can include control probes (i.e., probes for genes whose expression is expected to remain unaffected in a negative sample, e.g., a sample from a subject not having a non-CNS disorder). Typically, such controls or "normal" non-disease samples are obtained from healthy volunteers. Longitudinal studies of healthy volunteers can be performed to confirm that the control samples are from individuals that remained disease free. Such studies provide the raw data for a database of control gene expression profiles. Such a database provides a source of normal or control "reference" profiles that can be used in the present methods. Control samples can also be obtained post-mortem from individuals who died for a reason unrelated to the disorder being diagnosed (e.g., individuals who died from an accidental trauma). In such cases, post-mortem samples should be taken as soon as possible after death, e.g., no later than 3 hours after death.

A population of labeled cDNA representing total mRNA from a sample of a tissue of interest, e.g., brain, spinal cord, or CSF, is contacted with the DNA array under suitable hybridization conditions. Hybridization of cDNAs with sequences in the array is detected, e.g., by fluorescence at particular addresses on the solid support. Thus, a pattern of fluorescence representing a gene expression pattern in the CNS sample of a particular subject or group of subjects is obtained. These patterns of gene expression can be digitized and stored electronically for computerized analysis and comparison. For example, an array can be used to compare expression of CNS genes in individuals being tested with one or more reference gene expression profiles stored electronically, e.g., in a digital database, where the reference gene expression profile is associated with either the presence (positive control) or absence (negative control) of a peripheral neoplasia or other disorder.

In some embodiments, cDNAs are used as probes to form the array. Suitable cDNAs can be obtained by conventional polymerase chain reaction (PCR) techniques, as described above. The length of the cDNAs can be from 20 to 2,000 nucleotides, e.g., from 100 to 1,000 nucleotides. Other methods known in the art for producing cDNAs can be used. For example, reverse transcription of a cloned sequence can be used (for example, as described in Sambrook et al., eds., Molecular Cloning: A Laboratory Manual. 2nd ed., Cold Spring Harbor Laboratory, Cold Spring Harbor Laboratory Press, Cold Spring Harbor, NY, 1989). The cDNA probes are deposited or placed ("printed" or "spotted") onto a suitable solid support (substrate), e.g., a coated glass microscope slide, at specific, predetermined locations (addresses) in a two-dimensional grid. A small volume, e.g., 5 nanoliters, of a concentrated DNA solution is used in each spot. Spotting can be carried out using a commercial microspotting device (sometimes called an arraying machine or gridding robot) according to the vendor's instructions. Commercial vendors of solid supports and equipment for producing DNA arrays include BioRobotics Ltd., Cambridge, UK; Corning Science Products Division, Acton, MA; GENPAK Inc., Stony Brook, NY; SciMatrix, Inc., Durham, NC; and TeleChem International, Sunnyvale, CA.

The cDNAs can be attached to the solid support by any suitable method. In general, the linkage is covalent. Suitable methods of covalently linking DNA molecules to the solid support include amino cross-linking and UV crosslinking. For guidance concerning construction of cDNA arrays , see, e.g., DeRisi et al., Nature Genetics, 1996, 14:457-460; Khan et al., Electrophoresis, 1999, 20:223-229; Lockhart et al., Nature Biotechnol., 1996, 14:1675-1680.

In some embodiments of the new methods, the immobilized DNA probes in the array are synthetic oligonucleotides. Preformed oligonucleotides can be spotted to form a DNA array, using techniques described herein with regard to cDNAs. In general, however, the oligonucleotides are synthesized directly on the solid support. Methods for synthesizing oligonucleotide arrays are known in the art. See, e.g., Fodor et al., U.S. Patent No. 5,744,305. The sequences of the oligonucleotides represent portions of the sequences of a particular gene to be detected. Generally, the lengths of oligonucleotides are 10 to 50 nucleotides, e.g., 15, 20, 25, 30, 35, 40, or 45 nucleotides.

Also useful in the methods are aptamer arrays. Aptamers are nucleic acid molecules that bind to specific target molecules based on their three-dimensional conformation rather than hybridization. The aptamers are selected, for example, by synthesizing an initial heterogeneous population of oligonucleotides, and then selecting oligonucleotides within the population that bind tightly to a particular target molecule. Once an aptamer that binds to a particular target molecule has been identified, it can be replicated using a variety of techniques known in biological and other arts, *e.g.,* by cloning and polymerase chain reaction (PCR) amplification followed by transcription. The target molecules can be nucleic acids, proteins, peptides, small organic or inorganic compounds, and even entire micro-organisms.

The synthesis of a heterogeneous population of oligonucleotides and the selection of aptamers within that population can be accomplished using a procedure known as the Systematic Evolution of Ligands by Exponential Enrichment or SELEX. The SELEX method is described in, *e.g.,* Gold et al., U.S. Patent Nos. 5,270,163 and 5,567,588; Fitzwater et al., ("A SELEX Primer," Methods in Enzymology, 267:275-301, 1996); and in Ellington and Szostak ("In Vitro Selection of RNA Molecules that Bind Specific Ligands," Nature, 346:818-22). Briefly, a heterogeneous DNA oligomer population is synthesized to provide candidate oligomers for the in vitro selection of aptamers. This initial DNA oligomer population is a set of random sequences 15 to 100 nucleotides in length flanked by fixed 5' and 3' sequences 10 to 50 nucleotides in length. The fixed regions provide sites for PCR primer hybridization and, in one implementation, for initiation of transcription by an RNA polymerase to produce a population of RNA oligomers. The fixed regions also contain restriction sites for cloning selected aptamers. Many examples of fixed regions can be used in aptamer evolution. See, e.g., Conrad et al. ("In Vitro Selection of Nucleic Acid Aptamers That Bind Proteins," Methods in Enzymology, 267:336-83, 1996); Ciesiolka et al., ("Affinity Selection-Amplification from Randomized Ribooligonucleotide Pools," Methods in Enzymology, 267:315-35, 1996); Fitzwater, *supra.*

Aptamers are generally selected in a 5 to 100 cycle procedure. In each cycle, oligomers are bound to the target molecule, purified by isolating the target to which they are bound, released from the target, and then replicated by 20 to 30 generations of PCR amplification.

Aptamer selection is similar to evolutionary selection of a function in biology. Subjecting the heterogeneous oligonucleotide population to the aptamer selection procedure described above is analogous to subjecting a continuously reproducing biological population to 10 to 20 severe selection events for the function, with each selection separated by 20 to 30 generations of replication

Heterogeneity is introduced, e.g., only at the beginning of the aptamer selection procedure, and does not occur throughout the replication process. Alternatively, heterogeneity can be introduced at later stages of the aptamer selection procedure.

Various oligomers can be used for aptamer selection, including, e.g., 2'-fluoro-ribonucleotide oligomers, NH2-substituted and OCH3-substituted ribose aptamers, and deoxyribose aptamers. RNA and DNA populations are equally capable of providing aptamers configured to bind to any type of target molecule. Within either population, the selected aptamers occur at a frequency of 109 to 1013, see Gold et al., ("Diversity of Oligonucleotide Functions," Annual Review of Biochemistry, 64:763-97, 1995), and most frequently have nanomolar binding affinities to the target, affinities as strong as those of antibodies to cognate antigens. See Griffiths et al., (EMBO J., 13:3245-60, 1994).

Using 2'-fluoro-ribonucleotide oligomers is likely to increase binding affinities ten to one hundred fold over those obtained with unsubstituted ribo- or deoxyribo-oligonucleotides. See Pagratis et al. ("Potent 2'-amino and 2' fluoro 2'deoxyribonucleotide RNA inhibitors of keratinocyte growth factor" Nature Biotechnology, 15:68-73). Such modified bases provide additional binding interactions and increase the stability of aptamer secondary structures. These modifications also make the aptamers resistant to nucleases, a significant advantage for real world applications of the system. See Lin et al. ("Modified RNA sequence pools for in vitro selection" Nucleic Acids Research, 22:5229-34, 1994); Pagratis, *supra.*

In the present invention, aptamers can be used to detect, e.g., mRNAs, cDNAs, or proteins corresponding to CNS marker genes.

In some embodiments of the invention, probes (e.g., nucleic acid probes, antibodies, or aptamers) for the human homologs of animal model CNS genes are used in the detection method. In other embodiments, the probe used for detection consists of highly conserved regions of a gene, e.g., a sequence that is highly conserved between homologous mouse and human sequence.

Sample Preparation and Analysis

In the new methods, the transcription level of one or more CNS genes is assumed to be reflected in the amount of its corresponding mRNA present in cells of an assayed CNS sample. In general, mRNA from the CNS cells or tissue is copied into cDNA under conditions such that the relative amounts of cDNA produced representing specific genes reflect the relative amounts of the mRNA in the sample. Comparative hybridization methods involve comparing the amounts of various, specific mRNAs in two tissue samples, as indicated by the amounts of corresponding cDNAs hybridized to sequences from the genes of interest.

The mRNA used to produce cDNA is generally isolated from other cellular contents and components. One useful approach for mRNA isolation is a two-step approach. In the first step, total RNA is isolated. The second step is based on hybridization of the poly(A) tails of mRNAs to oligo(dT) molecules bound to a solid support, e.g., a chromatographic column or magnetic beads. Total RNA isolation and mRNA isolation are known in the art and can be accomplished, for example, using commercial kits according to the vendor's instructions. Similarly, synthesis of cDNA from isolated mRNA is known in the art and can be accomplished using commercial kits according to the vendor's instructions. Fluorescent labeling of cDNA can be achieved by including a fluorescently labeled deoxynucleotide, e.g., Cy5-dUTP or Cy3-dUTP, in the cDNA synthesis reaction. For guidance concerning isolation of mRNA and synthesis of fluorescently labeled cDNA for analysis on a DNA array, see, e.g., Ross et al., Nature Genetics, 2000,24:227-235.

Conventional techniques for hybridization and washing of DNA arrays, detection of hybridization, and data analysis can be employed in the new methods without undue experimentation. Commercial vendors of hardware and software for scanning DNA arrays and analyzing data include Cartesian Technologies, Inc. (Irvine, CA); GSI Lumonics (Watertown, MA); Genetic Microsystems Inc. (Woburn, MA); and Scanalytics, Inc. (Fairfax, VA).

In other embodiments, the expression level of one or more CNS genes is reflected in the presence and/or level of protein present in cells of a CNS sample to be assayed. The presence or level of protein in a CNS sample can be detected by routine methods. For example, a CNS sample (e.g., a CSF sample) can be analyzed by gel electrophoresis techniques such as 2-dimensional (2D) PAGE. Once protein spots are separated on a 2D-PAGE gel, differentially expressed spots can be identified, e.g., by matrix assisted laser desorption ionization time of flight (MALDI-TOF) and electrospray ionization (ESI). This method can also be used for peptide analysis to provide the fingerprint of a particular protein in a sample.

A second proteomic approach can involve obtaining a proteomic spectrum by directly analyzing a CNS sample, such as a CSF sample, by mass spectroscopy. For example, surface enhanced laser desorption ionization time of flight (SELDI-TOF) analysis can be performed to generate a proteomic pattern from a CNS sample. SELDI-TOF analysis has been shown to be able to identify a cluster pattern that differentiates between normal and disease patients. See, Paweletz et al., Dis. Markers, 17(4):301-7,2001.

Generating Gene Expression Profiles

A gene expression profile used in the methods described herein is a pattern of expression of two or more CNS genes. In some cases, an expression profile can be a pattern of expression of 5, 10, 25, 50, 100, 200, 500, or more genes. A "reference gene expression profile" as used herein is a characteristic pattern (dataset) of expression (e.g., up or down regulated and/or level of expression) of two or more CNS genes, where the pattern of expression is associated with risk or presence of a particular disorder (e.g., a ratio of the level of expression associated with a particular disorder to the level of expression in a person without the disorder). The association between the characteristic profile and the particular disorder is determined through the generation and analysis of CNS gene expression data to identify correlations between particular patterns of CNS gene expression (e.g., relative increases and/or decreases of gene expression of particular genes compared to a negative control) and particular clinical states. For example, a reference gene expression profile can be data for a set of genes (also referred to herein as a "panel" or "cluster" of genes), where each gene of the set is either down-regulated or up-regulated when associated with a specific peripheral disorder or any peripheral disorder.

A reference profile can also include a value, e.g., a relative value, of gene expression for two or more genes in a panel, where at least one gene of the panel is down-regulated and at least one gene is up-regulated. An example of such a gene expression profile is a profile that includes a value for the relative differential expression of at least 2, e.g., between 5 and 50, of the genes shown in any of the tables of FIGS.47A-C or any number of the genes listed in FIGS. 58 and 60. Such a reference profile is associated with the presence of early stage carcinoma, arthritis or asthma. Other examples are provided by each of the figures disclosed herein. For example, FIG. 31-4 provides a profile or panel of genes that are significantly up-regulated in the cortex in response to the presence of lung cancer.

Exemplary gene expression profiles associated with non-CNS carcinoma (or particular types of non-CNS carcinoma, such as breast, lung or colon carcinoma) are shown in FIGS. 2-46. A reference gene expression profile can include data from at least a portion of the genes or gene products shown in these figures. For example, a reference gene expression profile associated with lung carcinoma can include a value for the differential expression of 1, 2, 5, 10, 20, 30, 40, 50, or more, genes or gene products listed as CNS markers for lung carcinoma in FIGS. 8, 9, 10, 17, 18, 19, 26, 27, and 28. The reference profiles that can be used with the methods of the invention are not limited by the CNS markers described herein.

Reference profiles can be generated by detecting changes in patterns of gene expression in the CNS in response to the presence of non-CNS disease in an experimental animal, and identifying the human homologs of the genes and gene clusters that are differentially expressed in a certain pattern in the experimental samples, as exemplified in Examples 1-3 described herein.

A reference gene expression profile can also be obtained by evaluating human CNS gene expression data. For example, a database is created and maintained where CNS gene expression data is obtained and stored, e.g., electronically e.g., digitally, for tens, hundreds, or thousands of individuals. The individuals can be followed and evaluated with regard to, e.g., cancer clinical state longitudinally (e.g., over at least 5 years, 10 years, 15 years, 20 years, 30 years, 50 years or a lifetime). The expression profiles of individuals who developed a particular disease, e.g., 5, years, 10 years, 15 years, 20 years, 30 years, or 50 years after the CNS gene expression data was obtained, are compared with the expression profiles of individuals who remained disease free. Similar comparison is made between individuals who developed one clinical type of the disorder compared to another, or individuals who developed the disease at an early age versus a late age. These analyses provide specific reference CNS gene expression profiles that are associated with different stages of disease, e.g., different stages of neoplasia, or different types of tumors. A "control gene expression profile" is a profile of a given set of genes in a healthy (normal) individual or animal model.

Both reference and control gene expression profiles are typically stored in electronic digital form, e.g., on a computer-readable medium; such as a CD, diskette, DVD, hard drive, computer memory, or memory cards, along with identifying information such as gender, type and stage of disorder, age group, and race of the subject.

A "test gene expression profile" is obtained from a CNS sample of a subject to be tested for the presence of peripheral disease. First, a CNS sample, e.g., a brain cell sample or CSF sample, is obtained from the subject by routine means such as brain needle biopsy (for a brain cell sample) or a lumbar puncture (for CSF), as described herein. The sample is then prepared for use in a method of detecting gene expression, e.g., any method of detecting gene expression described herein. In one embodiment, total RNA can be prepared from the sample, and reverse transcribed into cDNA for use in a nucleic acid array assay described herein. In another embodiment, total protein is prepared from the sample for use in an antibody assay described herein. The prepared sample can then be contacted with an array (e.g., an antibody or nucleic acid array) that can detect expression levels (or protein levels in the case of an antibody array) of at least one cluster or panel of CNS genes or gene products corresponding to the cluster or panel of CNS genes or gene products of one or more particular reference gene expression profiles to which the test sample will be compared. For example, a prepared CNS sample from the test subject can be contacted with a nucleic acid array containing nucleic acid probes or an antibody array containing antibody probes for two or more, e.g., between 2 and 150, between 10 and 50, or between 20 and 30, of the genes shown in FIGS. FIGS. 2-46. In one embodiment, the array can contain probes for each of the marker genes in a particular cluster disclosed in any of FIGS. 2-46.

The results of the array assay are obtained by routine techniques, such as fluorescence detection and measurement of bound antibody or hybridized nucleic acid for each position (each probe) on the array. A dataset of the values for the level of each polypeptide or gene detected in the CNS sample by each antibody or probe on the array can then be generated. The dataset can contain information such as patient identifier, and actual and/or relative levels of expression or protein detected. Such a dataset can be used directly as the "test" or "sample" gene expression profile or the dataset can be converted into a format comparable to the format of the reference profile.

Once the test expression profile is generated, a test profile can be compared to a reference expression profile as described herein.

Analyzing Gene Expression Profiles

The new methods any systems enable one to of evaluate a test subject by comparing a test gene expression profile from the test subject with a reference gene expression profile associated with the presence of a particular disorder and/or a control ("normal") gene expression profile associated with the absence of a particular non-CNS disorder. Longitudinal studies of CNS gene expression in multiple volunteers are performed to identify and confirm control gene expression profiles that are associated with individuals who remain disease free or reference profiles individuals who get the disease. Such studies provide the raw data for a database of negative and positive control gene expression profiles that can be used in the present methods.

Subject "test" and "reference" profiles can be obtained by methods described herein. In one embodiment, the methods include obtaining a CNS sample from a subject (either directly or indirectly from a caregiver or other party), creating an expression profile from the sample, and comparing the subject's expression profile to one or more control and/or reference profiles and/or selecting a reference profile most similar to that of the subject.

As with other detection methods, profile-based assays can be performed prior to the onset of symptoms (in which case they are diagnostic), prior to treatment (in which case they are prognostic) or during the course of treatment (in which case they serve as monitors.)

A variety of routine statistical measures can be used to compare two gene expression profiles. One possible metric is the length of the distance vector that is the difference between the two profiles. Each of the test and reference profile is represented as a multi-dimensional vector, wherein each dimension is a value in the profile, e.g., a value for the expression of a particular gene in a panel. A test profile and reference or control profile can be said to "match" if at least 75% of the genes in a test gene expression profile are either up- or down- regulated in the same manner as the genes in the reference expression profile. A "high level match" would mean that at least 75% of the genes come within at least plus or minus 50% of the expression level (or Log2 ratio of expression level) of the gene in the reference expression profile.

In one embodiment, test and reference profiles are said to match if their respective multi-dimensional vectors, as described above, have a 30% or lower variance with respect to each other. If the test and reference profile match, the test subject can be identified as having the peripheral disorder with which the reference profile is associated. If the test and normal control profile match, the subject is likely to be free of the peripheral disorder.

In one embodiment, pattern recognition software is used to identify matching profiles. For example, unsupervised clustering algorithms, such as hierarchical clustering, K-means clustering, and SOM (self-organizing maps) for pattern discovery can be used. Supervised techniques such as SVM (support vector machines) and SPLASH structural pattern localization analysis by sequential histograms) algorithms implemented in the Genes @Work software package (IBM Corp.) can also be used.

In another embodiment, gene expression profiles are analyzed by quantitative pattern comparison performed by applying a nearest neighbor classifier (see Jelinek et al., Mol. Cancer Res., 1:346-61, 2003). Based on the nearest neighbor classifier, a score is defined which, together with a permutations-derived distribution, can be used to estimate the probability of each test profile of belonging to a class defined by a reference gene expression pattern (see Jelinek, *supra*).

The result of the diagnostic test, which can be transmitted in paper or electronic form to the subject, a caregiver, or another interested party, can be the subject expression profile *per se,* a result of a comparison of the subject expression profile with another profile, a most similar reference profile, or a descriptor of any of these. Transmission can occur across a computer network (*e.g.*, in the form of a computer transmission such as a computer data signal embedded in a carrier wave). The new systems also include a computer-readable medium (such as a CD, diskette, or hard drive) having executable code for effecting the following steps: receive a subject expression profile; access a database of reference expression profiles; and either i) select a matching reference profile most similar to the subject expression profile, or ii) determine at least one comparison score for the similarity of the subject expression profile to at least one reference profile. The subject expression profile and the reference expression profile each include a value representing the level of expression of one or more of the identified genes or gene products or the proteins they encode.

Predictive Medicine

The methods described herein are generally useful in the field of predictive medicine and, more specifically, are useful in diagnostic and prognostic assays, in monitoring progression of a disease, e.g., neoplasia, or monitoring of response to treatment, e.g., in clinical trials. For example, one can determine whether a subject has a very early stage neoplasia, in the absence of other, e.g., clinical, indications of neoplasia. In another example, one can determine whether a subject is at risk for developing rheumatoid arthritis or whether the subject has early stage RA, in the absence of clinical indications of RA such as joint inflammation. The methods are particularly useful, e.g., for patients who have had surgery or treatment for the disease (e.g., to remove cancer), in which case the methods could be used to monitor recurrence or metastasis, for persons living in regions of high incidence of cancer due, e.g., to environmental factors, or for individuals who have a family history of a disease (e.g., diabetes, asthma or cancer) or are carriers of a disease susceptibility gene, e.g., a cancer susceptibility gene (e.g., BRCA1 or BRCA2, hMSH2, MLH1, MSH2, or MSH6). Other cancer susceptibility genes are described in The Genetic Basis of Human Cancer, 2nd edition (Vogelstein and Kinzler, Eds.), McGraw-Hill Professional (2002). Such individuals can be evaluated using the methods described herein.

In some cases, for example, where the risk of developing a disease is high (e.g., where an individual has a strong family history of asthma or cancer, or where an individual carries a cancer susceptibility gene or lives in a high risk area for cancer), an individual can be evaluated periodically (e.g., every 10 years, every 5 years, or every year) during his lifetime.

The "subject" referred to here, and that is referred to in the context of any of the methods, is a vertebrate animal, typically a mammal, or a human. The subject can be an experimental non human animal (e.g., an experimental rodent such as a rat or mouse), a domesticated animal (*e.g.,* a dog or cat); an animal kept as livestock (*e.g.*, a pig, cow, sheep, goat, or horse); a non-human primate (*e.g.*, an ape, monkey, or chimpanzee). The animal or human can be unborn (accordingly, the methods of the invention can be used to carry out genetic screening or to make prenatal diagnoses).

A System for Diagnosing a Non-CNS Disorder

A system for diagnosing a non-central nervous system (non-CNS) disorder in a subject can include the following elements: a sampling device to obtain a CNS sample, a gene expression detection device, a reference gene expression profile, and a means for comparing gene expression (e.g., a comparator) of one or more genes in the CNS sample with the reference gene expression profile.

A sampling device obtains a CNS sample by a minimally invasive technique, e.g., a form of neurosurgery. Minimally invasive neurosurgery techniques include computer-assisted stereo-taxis, intra-operative ultrasound, brain mapping and neuro-endoscopy, among other techniques. Stereo-taxis refers to a system of navigating to any area within the brain, with the aid of imaging techniques that display external reference landmarks and neural structures.

Alternatively, a "sample" can be taken by imaging gene expression, e.g., in the brain, rather than taking an actual sample. Brain imaging can be performed by Computer Tomography Scan (CT), Magnetic Resonance Imaging (MRI) or Positron Emission Tomography (PET), among other methods. Signals originated from these methods provide reference points from which a computer can calculate and present trajectories and depths to any target point within the brain. The latest generation of stereo-tactic systems, which includes the Cosman-Roberts-Wells (CRW) system, can be used with MRI and cerebral angiographic localization. Intra-operative ultrasound can be used either alone or in combination with stereo-taxis. Intra-operative ultrasound is used to identify structures such as the ventricles prior to dural opening. The ultrasound probe can also be used to guide a needle biopsy of a deep-seated lesion to obtain the CNS sample. Both the rigid and fiber-optic flexible endoscopes can be used to obtain a brain sample using minimally invasive techniques. Lasers and various other instruments (including biospy instruments) can be attached and used. A sampling device to obtain cerebrospinal fluid by lumbar puncture can be also guided by any of the imaging methods listed above.

Gene expression detection devices include those described herein under the subheading Nucleic Acid-Based Methods, Array, and, sample preparation and analysis. The comparator can be a computer loaded with pattern recognition software, as described herein.

Computer-Readable Medium

In another aspect, the new systems feature a computer-readable medium having a plurality of digitally encoded data records or data sets. Each data record or data set includes a value representing the level of expression of a CNS gene, and a descriptor of the sample. The descriptor can be, e.g., an identifier (e.g., an identifier for the patient from which the sample was obtained, e.g., a name or a reference code that can be matched with patient information only by those having access to a decoding table), a diagnosis made, or a treatment to be performed in the event the level of expression reaches a certain level or falls below a certain level. The data record can also include values representing the level of expression of related genes (e.g., the data record can include values for each of a plurality of genes in a gene "cluster," where a particular reference gene expression for the genes in the cluster is associated with a non-CNS disorder). The data record can also include values for control genes (e.g., genes whose expression is not changed in control samples or whose expression is not diagnostically correlated with a non-CNS disorder). The data record can be structured in various ways, e.g., as a table (e.g., a table that is part of a database such as a relational database (*e.g.*, a SQL database of the Oracle or Sybase database environments) or as a list.

Non-CNS Diseases

The methods described herein are not limiting in that they can be used to diagnose and monitor various non-CNS disorders, such as a neoplasia (e.g., tumor or cancer); immune disorders (e.g., an autoimmune disorder such as rheumatoid arthritis, multiple sclerosis, systemic lupus erythematosus, psoriasis, scleroderma); allergic or inflammatory disorders (e.g., asthma, inflammatory bowel disease, Crohn's disease); metabolic or endocrine disorders (e.g., diabetes, obesity, Addison's disease); pathogenic infections (e.g., a viral, parasitic or fungal infection, e.g., HIV infection); and cardiovascular disorders.

As used herein, "neoplasia" refers to the uncontrolled and progressive proliferation of cells under conditions that would not elicit, or would cause cessation of, proliferation of normal cells. Neoplasia results in the formation of a "neoplasm," which is defined herein to mean any new and abnormal growth, particularly a new growth of tissue, in which the growth is uncontrolled and progressive. Neoplasm, as used herein, is synonymous with "tumor." Malignant neoplasms or tumors are distinguished from benign in that the former show a greater degree of anaplasia, or loss of differentiation and orientation of cells, and have the properties of invasion and metastasis. Thus, neoplasia includes "cancer," which herein refers to a proliferation of cells having the unique trait of loss of normal controls, resulting in unregulated growth, lack of differentiation, local tissue invasion, and metastasis. The methods described herein can be used to diagnose neoplasia from any non-CNS cell or tissue type, such as neoplasia derived from epithelial or endocrine tissue, mesenchymal tissues, or hematopoietic tissue.

The term "carcinoma" is art recognized and refers to malignancies of epithelial or endocrine tissues including respiratory system carcinomas, gastrointestinal system carcinomas, genitourinary system carcinomas, testicular carcinomas; breast carcinomas, prostatic carcinomas, endocrine system carcinomas, and melanomas. Exemplary carcinomas include those forming from tissue of the colon, lung, prostate, breast, cervix, head and neck, and ovary. The term also includes carcinosarcomas, which include malignant tumors composed of carcinomatous and sarcomatous tissues. An "adenocarcinoma" refers to a carcinoma derived from glandular tissue or in which the tumor cells form recognizable glandular structures.

The term "sarcoma" is art recognized and refers to malignant tumors of mesenchymal derivation.

As used herein, the term "hematopoietic neoplastic disorders" includes diseases involving hyperplastic/neoplastic cells of hematopoietic origin, e.g., arising from myeloid, lymphoid or erythroid lineages, or precursor cells thereof. The disorders can arise from poorly differentiated acute leukemias, e.g., erythroblastic leukemia and acute megakaryoblastic leukemia. Exemplary myeloid disorders include, but are not limited to, acute promyeloid leukemia (APML), acute myelogenous leukemia (AML) and chronic myelogenous leukemia (CML) (reviewed in Vaickus, L. (1991) Crit Rev. in Oncol./Hemotol. 11:267-97); lymphoid malignancies include, but are not limited to acute lymphoblastic leukemia (ALL) which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Additional forms of malignant lymphomas include, but are not limited to non-Hodgkin lymphoma and variants thereof, peripheral T cell lymphomas, adult T cell leukemia/lymphoma (ATL), cutaneous T-cell lymphoma (CTCL), large granular lymphocytic leukemia (LGF), Hodgkin's disease and Reed-Sternberg disease.

Identification of Disease Surveillance Genes for Non-CNS Disorders

The new methods also include methods of identifying disease surveillance genes for-non-CNS disorders in a subject, as well as lists (in the Figures) of those CNS marker genes that have already been discovered. Generally, such methods involve detecting changes in gene expression in the CNS in response to the presence of a particular non-CNS disease condition in a subject, e.g., an experimental non human animal. The methods will generally involve inducing a disease condition or disorder in a test experimental non human animal; and comparing the expression of at least one gene in a CNS sample from the test experimental animal to expression of the gene in a CNS sample from a control experimental animal. A gene (or a human homolog of a gene) that is differentially expressed in the CNS sample from the test experimental animal compared to the CNS sample from the control experimental animal is a CNS diagnostic marker for a non-CNS disorder. Such markers are referred to herein as CNS "marker genes" or "disease surveillance genes" for non-CNS disease. It is understood, however, that the gene product of the marker gene can also serve as a diagnostic marker. In most cases, a plurality of differentially expressed markers are identified (e.g., a "profile" or "cluster" of markers is identified). The experimental animal is preferably an experimental mammal, and can be, e.g., an experimental rodent (e.g., a rat, mouse or guinea pig) or non-human primate (e.g., an ape, e.g., a monkey or chimpanzee).

The methods of detection of gene expression described herein, and particularly array and chip technology, are useful for methods of identifying Disease surveillance genes for non-CNS neoplasia. CNS samples are prepared from experimental and control animals (e.g., brains are biopsied or removed, or CSF samples are taken) and RNA, cDNA, or protein is prepared from the samples as described herein. A single chip (e.g., a commercially available chip having probes for a large number of genes in the genome of the experimental animal species) can allow measurement of the level at which hundreds, thousands, or even tens of thousands of genes are expressed in the CNS sample of a test experimental animal compared to a control experimental animal. Typically, clustering methodology or other bioinformatics tools are used to mine the data obtained from such large scale experiments and identify the genes or clusters of genes that are statistically significantly differentially expressed in an experimental sample compared to a control sample. Many such tools and programs are available to the skilled artisan. An exemplary method of data analysis is described herein and exemplified in the Examples below.

Disease Surveillance Genes for Neoplasia

In one embodiment, CNS diagnostic markers for non-CNS neoplastic disorders are identified by detecting changes in gene expression in the CNS in response to the presence of a non-CNS neoplasm in an experimental animal. For example, a neoplasm is induced in an experimental non human animal and gene expression in the CNS of the experimental animal is evaluated compared to a control animal. Methods for inducing growth of a non-CNS neoplasm, e.g., a cancer, in an experimental non human animal, are known in the art and include, e.g., chemical or radiation mutagenesis, or transplantation of a neoplastic cell (e.g., a neoplastic cultured cell or cell line) to the experimental animal. CNS genes or gene products whose expression is altered in the experimental animal compared to a control animal are identified as CNS markers or surveillance genes for neoplasia. Examples of CNS marker genes for cancer, particularly for carcinoma, are provided herein by FIGS. 2-48 and Examples 1-3.

In various embodiments, the diagnostic markers for breast cancer include Nedd8 (FIG. 29-1), Co14a3bp (FIG. 29-2), Bgn (FIG. 29-4), Sox5 (FIG. 29-5), Slc38a4 (FIG. 32-1), Tom1 (FIG. 32-2), Calr (FIG. 32-4), Itgae (FIG. 32-5), Ttrap (FIG. 35-1), P ex11b (FIG. 35-2), Sema7a (FIG. 35-4), Stam2 (FIG. 35-5)..

In other embodiments, the diagnostic markers for colon cancer include Nmb (FIG. 30-1), Ryr2 (FIG. 30-2), Trfr (FIG. 30-4), Mfap5 (FIG. 30-5), Prrg2 (FIG. 33-1), Faim (FIG. 33-2), Mgrn1 (FIG. 33-4), Stch (FIG. 33-5), Lhb (FIG. 36-1), Prm3 (FIG. 36-2), Crry (FIG. 36-4), Timp4 (FIG. 36-5).

Diagnostic markers for lung cancer include Nmb (FIG. 31-1), Pcdh8 (FIG. 31-2), Rock2 (FIG. 31-4), Angpt13 (FIG. 31-5), Sqstm1 (FIG. 34-1), Kcnip2 (FIG. 34-2), Oxt (FIG. 34-4), Myh4 (FIG. 34-5), Enc1 (FIG. 37-1), Gsg1 (FIG. 37-2), Srr (FIG. 37-4), Ndph (FIG. 37-5).

Any one of these disease surveillance genes can be used alone or in a set, e.g., of 2, 5, or 10 genes to create probes useful in the methods described herein to diagnose specific cancers.

Disease surveillance Genes for Rheumatoid Arthritis

In another embodiment, identifying CNS diagnostic markers for rheumatoid arthritis (RA) can be identified by detecting changes in gene expression in the CNS in an animal model of RA compared to a wild type animal. For example, the art-recognized rodent collagen induced arthritis (CIA) model can be used. In this model, arthritis is induced in a rodent, e.g., a DBA /1 mouse, by intradermal injection of purified collagen. 100 $\mu$g of purified type II collagen emulsified in complete adjuvant is typically injected at the base of the tail. Onset of arthritis is macroscopically visible as paw swelling or redness approximately three weeks after immunization (Williams et al., 1992, Proc. Natl. Acad. Sci. (USA), 89: 9784-9788). Clinical features of arthritis are monitored by quantitatively assessing paw swelling (e.g., with calipers) over a period of time. Severity of arthritis is assessed according to established clinical scores (Williams et al., 1995, Eur. J. Immunolo., 25:763-769). CNS genes or gene products whose expression is altered in the CIA animal compared to a control animal are identified as CNS markers or surveillance genes for RA.

Given the involvement of Th1 lymphocytes and B cells, pro-inflammatory cytokines, and a possible mimicry of bacterial LPS in disease evolvement, it is likely that genes that regulate these processes are candidates to be involved in early RA surveillance in the CNS. For example, pro-inflammatory cytokines produced in the brain such as IL-1$\beta$, TNF, IL-18, IFN-$\gamma$, IL-12, gp130; cytokines such as IL-6 and leukemia inhibitory factor (LIF); neurotransmitters and neurotrophic factors such as N-methyl-D-aspartate (NMDA), brain-derived neurotrophic factor (BDNF), glial cell line-derived neurotrophic factor (GDNF), nerve growth factor (NGF); inhibitors of cytokines such as prostaglandin E2 (PGE2) and SOCS-1 and -3; SOCS regulators such as cAMP-inducing central peptides; brain molecules that are produced as a result of cytokine action, such as pentraxin 3 (PTX3); hormone releasing factors such as cortocotropin; corticotropin-releasing hormone (CRH) and other hormones involved in the regulation of the HPA axis; pituitary corticotroph proteins such as POMC; molecules involved in NF-$\kappa$B-mediated signaling of inflammatory response; and other members of the families of these genes, as well as inducers and stimulators of these proteins, may be disease-surveillance genes for RA. See, e.g., See, e.g., Blond et al., 2002, Brain Res., 958(1):89-99; Suk et al., 2001, Immunol. Lett., 77(2):79-85; Losy et al., 2001, Acta Neurol. Scand., 104(3):171-3; Opp et al., 2001, Neuroendocrinology, 73(4):272-84; Chesnokova et al., 2002, Endocrinology, 143(5):1571-4; Bousquet et al., 2002, Mol. Endocrinol., 15(11):1880-90; Polentarutti et al., 2000, J. Neuroimmunol., 106(1-2):87-94; Bayas et al., 2003, Neurosci. Lett. 335(3):155-8; Xu et al., 2000, Acta Pharmacol. Sin. 21(7):600-4; Fang et al., 2000, Neuroreport, 11(4):737-41).

In various embodiments, the diagnostic markers for rheumatoid arthritis include Bc121 (FIG. 51A), P2rx1 (FIG. 51B), Pafah1b1 (FIG. 51B), Kcna3 (FIG. 51C), Taf1b (FIG. 51C), S1c38a3 (FIG. 51D), Hprt (FIG. 52A), C1d (FIG. 52B), Car11 (FIG. 52D), Dusp3 (FIG. 52D), Gabrr2 (FIG. 53C), Aatk (FIG. 53D).

Disease Surveillance Genes for Asthma

In another embodiment, CNS diagnostic markers for asthma can be identified by detecting changes in gene expression in the CNS in an animal model of asthma compared to a wild type animal. Several experimental models of asthma are known in the art, including rodent, sheep, and non-human primate models (for a review, see Isenberg-Feig et al., 2003, Curr. Allergy Asthma Rep. 3(1):70-8). Any of these can be used in the present methods. In one embodiment, the experimental model of asthma is performed according to Komai et al. (2003, Br. J. Pharmacol., 138(5):912-20). In brief, Balb/c mice are sensitized by intraperitoneal administration of 50 μg of ovalbumin combined with 1 mg of alum (A1(OH)3) on day 0 and 12. From day 22 to 43 animals are exposed to daily aerosol challenges of 1% w/v of ovalbumin for 30 minutes. Control animals can include saline-injected animals and animals sensitized with ovalbumin and alum and challenged with saline. Airway function is evaluated by measuring one or more of: airway responsiveness to acetylcholine; IL-4, IL-5, and/or IL-13 levels; interferon-γ levels; eosinophil numbers in bronchoalveolar fluids; specific IgG1 and IgG2a levels in sera; lung histology; and rectal temperature. CNS markers or surveillance genes for asthma are those whose expression is altered in the asthma model animal compared to a control animal, or those whose expression is altered after aerosol challenge compared to before aerosol challenge.

Several gene products associated with the CNS have been shown to influence the Th-2 response and are candidates as disease-surveillance genes. These include glucocorticoid, one of the main hormonal mediators of stress, which acts on antigen-presenting cells to suppress the production of IL-12 *in vitro* and *ex vivo;* neurotransmitters norepinephrine or epinephrine; β-adrenoreceptor (ARs) agonists and antagonists (e.g., propranolol); modulators of neurotransmission such as adenosine and adenosine analogues; opiod system components, which influence the immunological response in general and the Th-1/Th-2 balance in particular; mediators of allergic reactions, such as histamine; neuropeptides such as substance P, vasoactive intestinal peptide and somatostatin, which increase the release of histamine from mast cells. See Blotta et al., 1997, J. Immunol. 158: 5589-5595; Elenkov et al., 1996, Proc. Assoc. Am. Physicians, 108: 374-381;Cooper et al., The Biochemical Basis of Neuropharmacology, Oxford University Press, 1996, p. 123; Link et al., 1999, J. Immunol. 164: 436-442; Loizzo et al., 2002, Br. J. Pharmacol., 135(5):1219-26; Lowman et al., 1988, British Journal of Pharmacology, Vol 95:121-130; and Elenkov et al., Annals of the New York Academy of Sciences, 2000, 917:94-105.

In one embodiment, the diagnostic markers for asthma are Rasa3 (FIG. 55B), Tnk2 (FIG. 55B), H28 (FIG. 55C), Diap2 (FIG. 55C), Lga1s6 (FIG. 56A), Reck (FIG. 56A), Whrn (FIG. 56A), Stk22s1 (FIG. 56B), CD47 (FIG. 57A), Jund1 (FIG. 57A), Cstb (FIG. 57B), and Desrt (FIG. 57B).

Disease Surveillance Genes for Diabetes

In another embodiment, CNS diagnostic markers for diabetes can be identified by detecting changes in gene expression in the CNS in an animal model of diabetes compared to a wild type animal. Several experimental models of diabetes are known in the art, e.g., spontaneous models such as the NOD Mouse and BB Rat, and inducible models such as streptozotocin-induced (STZ) Diabetic Rats. These are reviewed in Cheta, 1998, J. Pediatr. Endocrinol. Metab., 11(1):11-9. CNS markers or surveillance genes for diabetes are those whose expression is identified to be altered in an induced non human animal compared to an uninduced animal (e.g., a streptozotocin-fed STZ rat compared to a control fed STZ rat), or those whose expression is altered in the early stages of spontaneous progression of disease.

Disease Surveillance Genes for Obesity

In yet another embodiment, CNS diagnostic markers for a propensity for obesity can be identified by detecting changes in gene expression in the CNS in an animal model of obesity, e.g., comparing CNS gene expression in an obesity-prone animal before and after obesity develops or is clinically detectable. The method can involve comparing differences in CNS gene expression between mouse strains that are either prone to obesity or resistant to obesity after being exposed to a fat-rich diet. For example, the method can employ the C57BL/KsJ(KsJ) or A/J strain of mice, both of which are resistant to the development of dietary obesity, or the obesity-prone strain C57BL/6J (B6).

Possible disease-surveillance genes for obesity or loss or body weight control include leptin, leptin receptor, ghrelin, cholecystokinin (CCK), CCK-A receptor, neuropeptide Y (NPY), proopiomelanocortin (POMC), α-melanocyte stimulating hormone (α-MSH), and other molecules that participate in the central control of energy balance. Given the fact that so many gene products orchestrate behaviors related to food intake, genetic deficiencies or the presence of particular polymorphic alleles in one or more of these genes may induce disorders in the control of energy homeostasis leading to obesity. Such a deficiency or disruption in the normal signaling of such molecules can likely trigger an early signal that alters CNS gene expression.

Isolating Homologous Sequences from Other Species

The human homologs of the genes listed in FIGS. 1,50, & 54 can be found on public databases such as GenBank and others that are available on the Internet.

The human homologs of CNS marker genes and their products (e.g., human homologs of CNS marker genes identified by experiments in non-human experimental animals) are useful for various embodiments of the methods described herein. Human homologs are known for most of the CNS marker genes provided herein. In those cases where a human homolog is not identified, several approaches can be used to identify such genes. These methods include low stringency hybridization screens of human libraries with a mouse marker gene nucleic acid sequence, polymerase chain reactions (PCR) of human DNA sequence primed with degenerate oligonucleotides derived from a mouse marker gene, two-hybrid screens, and database screens for homologous sequences.

Therapeutic Methods

The methods described herein can be used to identify or diagnose the presence of a non-CNS disorder in a subject at an early stage in the pathogenic process. As such, the methods allow for early intervention, which can be the key to successful treatment and/or management of many disorders. For example, if a propensity for obesity or diabetes can be diagnosed at an early stage using the methods described herein, simple lifestyle or nutritional changes may be sufficient to stop or slow the progress of the disease, where such changes would not be sufficient if the disease were diagnosed at a later, more progressive stage. Similarly, a neoplasia that is detected at an early stage is more likely to be treated with less toxic therapeutic agents, or lower doses of a therapeutic agent, than would be used at a stage of advanced neoplasia, e.g., cancer.

Chemotherapeutic Agents

In one embodiment, the methods described herein can identify or diagnose the presence of a non-CNS neoplasia in a subject at an early stage, e.g., before a neoplasm has formed, before a neoplasm is clinically detectable, and/or before a tumor has become malignant. As such, a neoplasm detected by a method described herein is amenable to treatment by an agent that targets neoplastic cells in general or targets specific neoplastic cells in particular. In one embodiment, a subject may be treated with a chemotherapeutic agent. Chemotherapeutic agents, as used herein, refer to chemical therapeutic agents or drugs used in the treatment of neoplasia. This term is used for simplicity notwithstanding the fact that other compounds may be technically described as chemotherapeutic agents in that they exert an anti-cancer effect. A number of exemplary chemotherapeutic agents are described below.

Suitable chemotherapeutic agents include: antitubulin/antimicrotubule drugs, e.g., paclitaxel, taxol, tamoxifen, vincristine, vinblastine, vindesine, vinorelbin, taxotere; topoisomerase I inhibitors, e.g., topotecan, camptothecin, doxorubicin, etoposide, mitoxantrone, daunorubicin, idarubicin, teniposide, amsacrine, epirubicin, merbarone, piroxantrone hydrochloride; antimetabolites, e.g., 5-fluorouracil (5-FU), methotrexate, 6-mercaptopurine, 6-thioguanine, fludarabine phosphate, cytarabine/Ara-C, trimetrexate, gemcitabine, acivicin, alanosine, pyrazofurin, N-Phosphoracetyl-L-Asparate=PALA, pentostatin, 5-azacitidine, 5-Aza 2'-deoxycytidine, ara-A, cladribine, 5 - fluorouridine, FUDR, tiazofurin, N-[5-[N-(3,4-dihydro-2-methyl-4-oxoquinazolin-6-ylmethyl)-N-methylamino]-2-thenoyl]-L-glutamic acid; alkylating agents, e.g., cisplatin, carboplatin, mitomycin C, BCNU=Carmustine, melphalan, thiotepa, busulfan, chlorambucil, plicamycin, dacarbazine, ifosfamide phosphate, cyclophosphamide, nitrogen mustard, uracil mustard, and pipobroman, 4-ipomeanol; estrogen modulators, e.g., raloxifene; piroxicam; 9-cis retinoic acid.

Suitable dosages for the selected chemotherapeutic agent are known to those of skill in the art. For example, where the agent is doxorubicin, suitable dosage may include 30 mg/m$^2$ of patient skin surface area, administered intravenously, twice at 1 week intervals. However, one of skill in the art can readily adjust the route of administration, the number of doses received, the timing of the doses, and the dosage amount, as needed. Bearing in mind these considerations, generally, a suitable dose for a given chemotherapeutic agent is between 10 mg/m$^2$ to about 500 mg/m$^2$, and more preferably, between 50 mg/m$^2$ to about 250 mg/m$^2$ of patient skin surface area (the skin surface of an average sized adult human is about 1.8 m$^2$). Such a dose, which may be readily adjusted depending upon the particular drug or agent selected, may be administered by any suitable route, including, e.g., intravenously, intradermally, by direct site injection, intraperitoneally, intranasally, or the like. Doses may be repeated as needed.

In one embodiment, because a method described herein can identify or diagnose the presence of a non-CNS neoplasia in a subject at an early stage, e.g., before a neoplasm has formed, before a neoplasm is clinically detectable, and/or before a tumor has become malignant, the dose of a chemotherapeutic agent may be lower than that typically used after a neoplasm, e.g., a cancer, is detected or diagnosed by clinical methods, such as visualization or palpation of a tumor mass.

Therapeutic Targets

A CNS marker gene for a non-CNS disorder, e.g., a CNS marker gene described herein, may not only "sense" the presence of the disorder, but also actively participate in responding to the presence of the disorder by generating a response, e.g., an antitumor response. Alternatively, a CNS marker gene may respond to the presence of non-CNS disorder by promoting progression of the disorder, e.g., inducing growth of a neoplasm or promoting malignant transformation of a neoplasm. As a therapeutic strategy, one would want to promote the expression or activity of the former type of gene, and/or inhibit the expression of activity of the latter type of gene, in the CNS. Thus, regardless of whether a CNS marker gene generates a response to curb or promote a specific disorder, its identification can provide a target for inhibiting progression of the disorder.

One way to identify such CNS marker genes that are also potential therapeutic targets is to identify CNS genes that are differentially expressed in animals that exhibit an inhibitory response against a disease compared to animals that do not exhibit an inhibitory response. For example, experimental non human animals can be injected with tumor inducing cells (e.g., colon cancer cells such as CT26) that express an interleukin (IL), e.g., IL-12. Injection of tumor cells genetically modified to express IL-12 is known to induce Th1 immune-mediated tumor rejection (Adris et al., 2000, Cancer Res., 60(23):6696-703). Control mice can be injected with tumor cells that do not express IL-12. At different times after injection, gene expression in the CNS is analyzed in the animals, as described herein, e.g., by microarray analysis. Thus, genes that "turn off' and "turn on" specifically in the CNS (e.g., brain) of the animals can be identified. Some of these genes will respond to the presence of the IL. Others will correspond to genes actively engaged in the "stimulation" of the antitumor immune response. This strategy can be used for any interleukin gene that may be involved in the stimulation of an antitumor immune response. Identification of brain genes actively involved in "stimulating" an antitumor response will provide a target for therapeutic intervention, e.g., by direct use of the gene or its gene product, or by screening for agents that block or stimulate their activity.

A second strategy for identifying CNS genes that are potential therapeutic targets is by using transgenic animals (e.g., knockout mice) having brain-specific disruptions (e.g., knockouts) in specific genes. A great number of CNS-specific knockout mice are currently available to the skilled artisan (see, e.g., the Jackson Laboratory web site, describing numerous JAX® mice models used in neurobiology), and many more can be expected to become routinely available. A role in the CNS response to non-CNS disease can be established for any particular gene for which a brain knockout animal can be obtained or produced, by inducing the disorder in the knockout mice (e.g., as described herein for cancer, RA, asthma or obesity), and evaluating disease outcome.

CNS marker genes and gene products that are also potential therapeutic targets are listed in FIGS. 48A-C, 59, and 61. These genes are or encode molecules involved in cell signaling, (e.g., growth factors, hormones, cytokines and their receptors) and are also differentially expressed markers in each of the tumors studied.

Vaccines

The methods described herein also provide targets for preventive vaccination. A set of brain genes that "senses" a disease may include receptors for known or unknown ligands. A disease cell might produce these ligands to inhibit the induction of a brain-derived anti-disease response. In such an instance, identifying a CNS gene that is involved in an anti-disease response can lead to the identification of a gene product secreted by the diseased cell that might impact in the brain to inhibit disease response. A genetic vaccine targeting these products could be a viable therapeutic strategy.

One approach to identify CNS targets for preventive vaccination in the treatment of non-CNS disorders is the following: obtain a CNS gene expression profile (using techniques such as those described herein above) from animals that exhibit an anti-disease response, e.g., in the case of a tumor, an IL-12 mediated antitumor response, in an experimental tumor model. It is expected that from the cluster of genes "sensing" the tumor, some will change their expression levels in the presence of IL-12. This subset of genes will likely be those involved in "generating" the antitumor response. This subset of genes is likely to have predictable modulators. For example, if a CNS gene that changes its expression profile in response to a non-CNS gene in the presence of IL-12 is a receptor, one could predict that the change in gene expression of such a receptor could be brought about by its ligand. Thus, a preventive genetic vaccine could be designed to generate a memory response to such a ligand.

A second experimental approach can involve identifying those CNS genes that change their activity in response to a non-tumorigenic dose of tumor cells (e.g., a condition where neoplasia exists in the body, but no neoplasm is yet formed). From this subset of CNS genes one can predict the modulating genes responsible for their changes in activity, as explained above. Such modulating genes, which may be derived from the neoplastic cells, are likely to be initial tumor-derived signals of alarm in the peripheral body. Thus, a preventive genetic vaccine could be designed to generate a memory response to such genes.

A vaccine can be, e.g., a polypeptide or nucleic acid corresponding to the gene to be targeted. Vaccines described herein can be administered, or inoculated, to an individual in physiologically compatible solution such as water, saline, Tris-EDTA (TE) buffer, or in phosphate buffered saline (PBS). They can also be administered in the presence of substances (e.g., facilitating agents and adjuvants) that have the capability of promoting uptake or recruiting immune system cells to the site of inoculation. Vaccines have many modes and routes of administration. They can be administered intradermally (ID), intramuscularly (IM), and by either route, they can be administered by needle injection, gene gun, or needleless jet injection (e.g., Biojector™, Bioject Inc., Portland, OR). Other modes of administration include oral, intravenous, intraperitoneal, intrapulmonary, intravitreal, and subcutaneous inoculation. Topical inoculation is also possible, and can be referred to as mucosal vaccination. These include, for example, intranasal, ocular, oral, vaginal, or rectal topical routes. Delivery by these topical routes can be by nose drops, eye drops, inhalants, suppositories, or microspheres.

The following examples are illustrative only and not intended to be limiting.

## EXAMPLES

Example 1: CNS Gene Expression Profiles Associated With Colon Carcinoma

CNS gene expression profiles associated with the presence of a peripheral tumor were identified using gene expression microarray analysis on brain tissue from experimental animals implanted peripherally with tumor cells. This example describes the identification of brain gene expression profiles associated with colon carcinoma.

Male BALB-C mice were injected subcutaneously with $5 \times 10^5$ CT-26 WT cells, a murine colon carcinoma cell line (ATCC cat # : CRL-2638), resuspended in 300 $\mu$l of PBS, as described below. Control mice were injected with the corresponding volume of PBS following the same procedure. After a specified time, the animals were sacrificed, their brains dissected, and first strand cDNA was synthesized from total or polyA+ RNA prepared from different brain regions, as described in detail below. Gene expression microarray analysis was performed with the first strand cDNA by hybridizing to preprinted slides (Corning's CMT-GAP™ II Coated Slides) containing Pan@ Mouse 10K Oligo set A (MWG Biotech). This slide set contains probes for 9,769 genes selected from mouse genes that have been functionally defined.

The data from the microarray experiments was analyzed with a Biorad Versarray chip reader 5 $\mu$m system, laser scanner (Biorad, Waterloo, ON, Canada) using then Versarray Analizer software, as described in more detail below.

Experimental Methodology

*Cell Lines:* The experimental work was based on the following murine cell lines: CT26WT colon carcinoma (ATCC cat #: CRL-2638), LL/2(LLC1) lung carcinoma (ATCC cat #: CRL-1642) and 4T1 breast carcinoma (ATCC cat #: CRL-2539). All cell lines were grown in P-100 plates with 10 ml of the corresponding medium. All culture media were sterilized by filtration using 0.22 $\mu$m CA filter. CT-26 cells were grown in DMEM containing 1.5 g/L Sodium Bicarbonate, 10 mM Hepes, and 1 mM Sodium pyruvate, supplemented with 10% Fetal Bovine Serum at 37°C with 5% $CO_2$. LL/2 (LLC1) cells were grown in DMEM containing 4.5 g/L Glucose, 1.5 g/L Sodium Bicarbonate, 10 mM Hepes, and 1 mM Sodium pyruvate, supplemented with 10% Fetal Bovine Serum at 37°C with 5% $CO_2$. 4T1 cells were grown in RPMI 1640 containing 4.5 g/L Glucose, 1.5 g/L Sodium Bicarbonate, 10 mM Hepes, and 1 mM Sodium pyruvate, supplemented with 10% Fetal Bovine Serum at 37°C with 5% $CO_2$.

*In vivo studies:* Six week-old animals were housed in an Hepa filtered air rack, 5 animals per cage (both tumor and control animals in the same cage) with food and water ad libitum for two weeks. At the age of 8 weeks Balb-C males were injected subcutaneously with $5 \times 10^5$ CT-26 WT cells resuspended in 300 $\mu$l of PBS. BALB-C female mice were injected subcutaneously with $1 \times 10^5$ 4T-1 cells resuspended in 100 $\mu$l of PBS. C-57/BL6 male were injected subcutaneously with $1 \times 10^6$ LL/2(LLC1) cells resuspended in 300 $\mu$l of PBS. Control animals were injected with the corresponding volume of PBS following the same procedure.

For each tumor type 4 different experiments were performed and 3 time points evaluated in quadruplicate. Each single time point corresponded to 30 mice (15 Tumor bearing mice and 15 control mice). All injections were done using a 27-G syringe. At the corresponding time, mice were killed by cervical dislocation. Mice were immediately decapitated, the brain extracted and dissected using the following procedure: the hypothalamus and the cerebellum were dissected, the brain was cut with a surgical razor blade leaving the right and left hemispheres separated, and two persons dissected the midbrain, the hippocampus, the prefrontal cortex and the striatum from each brain hemisphere. All brain regions were immediately frozen in dry ice and stored at -80°C until RNA extraction. ,

*Isolation ofTotal RNA:* Frozen tissue samples were homogenized in the presence of 6 ml of Trizol Reagent (Invitrogen, life technologies, Carlsbad, CA, USA), for Hypothalamus and Prefrontal Cortex and 10 ml for Mid Brain Total RNA was obtained following manufacturers instructions. The RNA was DNase treated with 10 $\mu$l of DNase I (2U/$\mu$l) (Ambion, Inc. Austin, TX, USA) for the hypothalamus and pre-frontal cortex and with 40 $\mu$l for the mid brain in the presence of RNase Out (Invitrogen, Life Technologies, Carlsbad, CA, USA) at 37°C for 30 min. DNA-free RNA was extracted with phenol-chloroform, and resuspended in RNase-free Milli-Q water.

*Preparation of Poly A+ RNA:* Poly A+ RNA was obtained from total RNA using the MicroPoly(A) Pure® kit from Ambion. In general, starting material was 400 $\mu$g total RNA to which a volume of 5M NaCl was added up to a final concentration of 0.45 M NaCl. After mixing, samples were transferred to an RNase-free microfuge tube. After adding binding buffer provided by the manufacturer, the RNA was heated for 5 minutes at 65°C and immediately chilled on ice for 1 minute. Oligo (dT) Cellulose was added to the sample, mixed by inversion and incubated for 60 minutes at room temperature with gentle agitation. This was followed by centrifugation at 4,000 rcf for 3 minutes. After the supernatant was removed, the pellet was treated with 1 ml binding buffer, mixed and spun down by centrifuging at 4,000 rcf for 3 minutes. After removing the supernatant, the pellet was washed 3 times with binding buffer followed by 4 washes with wash buffer. The Oligo(dT) Cellulose was then dissolved in 400 $\mu$l of wash buffer provided by the manufacturer and transferred to a spin column when the resin was washed 4 more times. When the flow-through of the column reached an absorbance of < 0.05 OD at A260, the mRNA was eluted from the Oligo(dT) Cellulose with 200 $\mu$l of Elution Buffer (provided by the manufacturer) pre-warmed at 65°C. The eluted polyA+ RNA was concentrated with a mixture containing 20 $\mu$l of 5 M Ammonium Acetate, 1 $\mu$l Glycogen and 550 $\mu$l of 100% ethanol. After overnight precipitation at -20°C samples were centrifuged at 14,000 rcf for 20 minutes at 4°C. After careful removal of the supernatant the pellet containing the polyA+ RNA was resuspended in 10 $\mu$l of DEPC treated Water/EDTA.

_Labeling of probes for microarray hybridization:_ Labeling was performed by an indirect method. The first method used aminoallyl labeled nucleotides via first strand cDNA synthesis using SuperScript Reverse Transcriptase followed by coupling of the aminoallyl to either Cyanine 3 or 5 (Cy3/Cy5) fluorescent molecules (Amersham Pharmacia). To 3 $\mu$g of poly(A) RNA were added 0.6 $\mu$l Random Primers (pd (N)6, Invitrogen) (3 $\mu$g/$\mu$l) and 1.2 $\mu$l Oligo (dT)12-18 (0.5 $\mu$g/$\mu$l). Milli-Q H$_2$O was added up to a final volume of 15.5 $\mu$l. The mixture was heated to 65°C for 5 minutes, chilled on ice and spun down. 12.5 $\mu$l of a master mix containing: 6 $\mu$l of 5X First Strand Buffer, 3 $\mu$l of 100 mM DTT, 0.6 $\mu$l of 50X aminoallyl (Sigma Co)-dNTP mix (Amersham Pharmacia), 1.5 $\mu$l of RNase Out (40 units/$\mu$l, Invitrogen), 1.4 $\mu$l Milli-Q H$_2$O were added to each tube, incubated at 37°C for 2 minutes, followed by the addition of 2 $\mu$l of SuperScript II Reverse Transcriptase (Invitrogen). After incubation for 2 hours at 37°C, the tubes were incubated for 15 minutes at 70°C and then were spun down. RNA was degraded by the sequential addition of 3 $\mu$l of 2.5 M NaOH incubated at 37°C for 15 minutes, then 15 $\mu$l of 2 M HEPES free acid, 4.8 $\mu$l of 3 M NaAcO (pH 5.2) and finally 150 $\mu$l of 100% EtOH. After mixing, tubes were incubated at -20°C for 1 hour. Tubes were centrifuged for 30 minutes at 4°C, the supernatant was removed and the pellet was washed twice in 70% ethanol. The pellet was dissolved in 2.25 $\mu$l Milli-Q H$_2$O. Coupling of fluorescent Cy3 and Cy5 was performed by adding to the 4.5 $\mu$l cDNA sample 2.25 $\mu$l of 0.2 M NaHCO$_3$ (pH 9.0) and then 4.5 $\mu$l of the DMSO/dye mixture. Tubes were mixed well and incubated for 1 hour at room temperature in the dark. For probe purification 500 $\mu$l of loading buffer were added to the sample and mixed. A SNAP Column (Invitrogen) was placed on a collection tube and the sample loaded on the column and incubated at room temperature for 2-5 minutes. The SNAP Column was centrifuged at maximum speed for 1 minute and the flow-through was discarded. After two more washes the SNAP column was put back in the collection tube and centrifuged at maximum speed for 30 seconds to remove residual wash buffer from the membrane filter. cDNA was eluted by adding 60 $\mu$l TE buffer to the SNAP column, incubated for 2-5 minutes and centrifuged at maximum speed at room temperature for 1-2 minutes. After saving the first eluate, the elution was repeated and both samples were combined.

_Quantification of the levels of incorporation of dyes and total DNA:_ The extent of dye incorporated was obtained by the absorbance at 550 nm and 650 nm for Cy3- and Cy5-probes, respectively. The amount of DNA was obtained by the absorbance at 260 nm. The percentage of dye incorporation was 3 - 5 %.


Microarrays and Data Analysis

_Prehybridization:_ The prehybridization buffer (5 ml of 20X SSC Buffer, 0.25 ml of 20% SDS, 5 ml of 10% BSA and 24.75 ml of Milli-Q H$_2$O) was preheated at 42°C. The printed slide was put in a 50 ml-Falcon polypropylene tube containing the preheated prehybridization buffer and incubated at 42°C for 40 min. After washing the slide five times, 1 minute each time, with Milli-Q H$_2$O preheated at 42°C in a Wash Station, slides were washed four or five times in 2-propanol. The slide was dried by centrifugation for 1 minute using a Microarray Centrifuge. Cover glasses were washed with Milli-Q H$_2$O and 2-propanol and dried. Slides were used immediately for hybridization.

_Hybridization._ All hybridization was done in dye swap manner. Each hybridization mix contained: 0.15% SDS, 30 % formamide, 3% SSC; 1 $\mu$g/$\mu$l Salmon Sperm DNA. To this mix 70 pmoles of Cy3 containing probe and 35 pmoles of Cy5 containing probe were added to give a total volume of 60 $\mu$l. The mixture incubated at 95°C for 3 minutes, snap cooled on ice for 1 minute, and centrifuged at 16.000 g for 1 minute. A pre-hybridized microarray slide (array side up) was placed in a hybridization chamber. The probe mixture was placed carefully on the top of the slide surface and covered by a cover slip. The edges of the cover slip were circumscribed with Immedge pen (Vector Laboratories Inc., Burlingame, CA, USA). 10 $\mu$l of Milli-Q H$_2$O (20 $\mu$l total) was added to the small wells at each end of the chamber to seal the chamber. Slides were incubated at 42°C for 16-20 hours in a 3D-rotator. At the end of the hybridization, the slide was carefully removed and washed with washing buffer (2 X SSC, 0.1 % SDS) preheated at 42°C for 5 minutes with agitation. Slides were washed twice more in different chambers, each time for 5 minutes (first in 1 X SSC and then in 0.1 X SSC). The slide was dried by centrifugation for 1 minute in a microarray centrifuge and placed in a light tight slide box until scanning.

_Data acquisition and image processing:_ The slides were scanned with a Virtek ChipReader laser scanner model A0-B0-05 (Virtek Vision Corp, Waterloo, ON, Canada) using the VersArray ChipReader software v3.0 build 1.63 (BioRad). Three images were obtained for each of the Cy3 and Cy5 channels with different detector sensitivity values for each image, with a resolution of 10 $\mu$m and a pixel depth of 16 bits. The images were stored as 16 bit TIFF files (Tagged Image File Format) and analyzed with VersArray Analyzer software v4.5 (BioRad). Image segmentation was performed with the "cross-correlated" algorithm, and "local corners" were used for background determination. The results were stored in plain text files with the following fields separated by tabulations: Grid, Row, Column, Signal Average for each channel, Signal Median for each channel, Background average for each channel, Area in pixels, and Quality score. The quality score (QS) was defined as the geometric mean between spot shape QS and signal-to-noise QS scores. Signal-to-noise QS was calculated as the percentage of pixels in a spot with values higher than 2*median (local background). Spot shape QS is defined as ratio of spot area to spot perimeter scaled to be in a range between 0 and 1.

*Data filtration and normalization:* All the data processing was performed under the R System v1.8.1 (The R Development core Team). To maximize the working dynamic range of the data, the nine possible combinations of channels were analyzed. The data was filtered to eliminate dust derived data points (spots with size less than 75 pixels or with a mean to median correlation less than 80% (Tran et al., Nucleic Acids Res. 30(12), e54, 2002), to eliminate saturated data points (spots with a proportion of saturated pixels greater than 20%), and to eliminate low signal data points (spots with signal to noise ratio below 1.2). Since spot intensity was not correlated to background, and in most images we observed that spot background was lower than slide background (Fang et al., Nucleic Acid Res. 31(16):e96), we decided to perform data analysis in two parallel ways, depending on whether background was subtracted (BS) or not (BNS) from spot intensity data. The base 2 logarithm of the ratio and the product between Cy5 and Cy3 was calculated as:

$$M = \log_2(Cy5/Cy3) \qquad\qquad (1)$$

$$A = \tfrac{1}{2} \cdot \log_2(Cy5 \times Cy3) \qquad\qquad (2)$$

Data for each of the nine replicates was globally normalized by subtraction of its own median value. Outlier data points were eliminated from the nine replicated data with a leave-one-out algorithm. Briefly, a data point was discarded as being outlier if it was outside the confidence interval defined by the remaining data points with a confidence level of 95% estimated from a t-student distribution with $n$-1 degrees of freedom. Here, n is the number of the remaining data points.

A gene expression dataset was then generated with the average of non-outlier data points.

For data normalization we assumed the following model:

$$M_{jk} = m_j + c_k + e_k(F_j) + e_k(A_{jk} \cdot P_j) + \varepsilon_{jk} \qquad\qquad (3)$$

Where $m_j$ (j = 1,2, ..., g) represents the true ratio of expression levels for the gene measured by spot *j*, and $M_{jk}$ (j = 1, 2, ..., g; k = 1, 2, ..., $n$) represents the measured ratio of expression levels for spot j on replicate k. This model states that the measured ratio M of replicate k is affected by a global measurement bias between the two channels $c_k$, a spot (or gene) specific bias $e_k(F_j)$, a spot intensity-dependent bias $e_k(A_{jk})$, a spot location-related bias $e_k(P_j)$, and a zero mean random error $\varepsilon_{jk}$. Since our experimental results showed that $e_k(P_j)$ and $e_k(A_{jk})$ were not independent, we modeled the intensity-dependent and location-dependent bias as $e_k(A_{jk} P_j) = f(x_j, y_j, A_{jk})$, where $x_j$ and $y_j$ define the coordinate of spot j in the slide. Data was corrected for global measurement bias between channels ($c_k$) by global median normalization. The gene specific bias ($e_k(F_j)$) was corrected by dye-swap analysis (see below). Finally, the intensity-dependent and location-dependent bias ($e_k(A_{jk} \cdot P_j)$) was corrected by a locally weighted 3D-polynomial surface regression of M vs. $x_j$, $y_j$ and $A_{jk}$ for the entire slide, followed by a 3D-polynomial surface regression for each grid, to correct for grid-specific intensity-dependent and location-dependent systematic bias. Locally weighted 3D-polynomial surface regression was carried out with the loess function of R system (modem regression package).

*Data integration between replicated slides (dye-swap analysis):*

Each labeled probe was hybridized at least twice in a dye-swap protocol (technical replicate). Genes that do not correlate in a dye-swap experiment were eliminated. Non-correlated genes were identified as follows: the product between the two ratios was calculated and sorted. The data points corresponding to the lower ratios were eliminated iteratively until the first quantile (in a total of 100 quantiles) was equal or greater than the 99th quantile.

If the scale (i.e., variance) between all the replicates of an experiment was different ($p < 0.05$, Fligner-Killeen test for homogeneity of variances), data was transformed to be equally scaled. Assuming that the ratios follow a normal distribution with mean zero and variance $a_i^2\sigma^2$, we estimated $a_i$ as follows:

$$a_i = \frac{MAD_i}{\sqrt[I]{\prod_{i=1}^{I} MAD_i}} \quad (4)$$

with I denoting the total number of slides, and the median absolute deviation (MAD) defined by,

$$MAD = \operatorname{median}_j \left| M_{ij} - \operatorname{median}_j \left( M_{ij} \right) \right| \quad (5)$$

where $M_{ij}$ denotes the $j$th spot in the $i$th slide.

An integrated data set was obtained as the average of A values from technical replicates weighted by their mean quality score, and the average of M values from technical replicates weighted by their mean quality score.

*Analysis and integration of biological replicates:* At least four biological replicates were prepared. The arithmetic mean (Mn) and SD were estimated from the integrated data for technical replicates. Differentially expressed genes for each experiment were identified ($p < 0.05$, t-student test for paired data).

*Multivariate analysis:*

Time analysis: A mixed-model design with two fixed effects (tumor cell injection or control treatment, and time points) and one random effect (biological replicates) without repetition was analyzed by Analysis of Variance (ANOVA) between groups (Pavlidis P, Methods, 31:282-289, 2003). Such a design allowed for the estimation of p-values for treatment, time points and their interaction.

Tumor and time analysis: A mixed-model design with three fixed effects and one random effect without repetition design was analyzed by ANOVA. For such a design, biological replicates were analyzed as random effects, and fixed factors were treatment (tumor vs. control), tumor model (breast, colon and lung cancer), and time (18, 72 and 192 hours). Such a design allowed for the estimation of p-values for treatment, tumor model, time, and interactions of treatment with tumor model and time.

*Cluster analysis:* Only genes differentially expressed were included in cluster analyses. A given gene was considered differentially expressed if its expression ratio was significantly different from zero for the two analyzed data sets (BNS and BS). Thus, genes differentially expressed ($p < 0.01$) in dataset BNS that were also differentially expressed ($p < 0.05$) in dataset BS were included in the cluster analysis. Similarly, genes differentially expressed in dataset BS ($p < 0.01$) that were also differentially expressed ($p < 0.05$) in dataset BNS were included in cluster analysis. Figures 5, 6, and 7 list the genes that were considered differentially expressed in the prefrontal cortex at 18 hours, 72 hours, and 192 hours, respectively, after tumor cell injection. Figures 14, 15, and 16 list the genes that were considered differentially expressed in the hypothalamus at 18 hours, 72 hours, and 192 hours, respectively. Similarly, Figures 23, 24, and 25 list the genes that were considered differentially expressed in the midbrain at 18 hours, 72 hours, and 192 hours, respectively.

Before cluster analysis, the data was scaled as follows: Ms = (M - Mn(M)) / SD(M). A figure of merit algorithm (Yeung et al., Bioinformatics 17(4):309-18, 2001) was used to identify the clustering algorithm and the number of clusters that minimized the intra-cluster variability. After examining the figure of merit of all the datasets analyzed with seven different clustering algorithm and different variations of such algorithms that led to a total of 51 different clustering methods, we decided to perform a hierarchical algorithm using Euclidean distance between gene expression patterns and a Ward's minimum variance agglomeration method (Hartigan, Clustering Algorithms. Wiley, New York, 1975).

Figures 30A and 30B show the results of a clustering analysis that included data on genes that were differentially expressed at the 18, 72, and 192 hour time points in the prefrontal cortex. Figure 30-1 shows the result of a clustering analysis that included genes that were down-regulated in the prefrontal cortex at all time points. Figure 30-2 shows the result of a clustering analysis that included genes that were down-regulated at the 18 hour, or at the 18 hour and 72 hour time points. Figure 30-3 shows the result of a clustering analysis that included genes that were down-regulated at the 192 hour, or 72 and 192 hour time points. Figure 30-4 shows the result of a clustering analysis that included genes that were up-regulated at all time points. Figure 30-5 shows the result of a clustering analysis that included genes that were up regulated at the 18 hour, or the 18 and 72 hour time points. Figure 30-6 shows the result of a clustering analysis that included genes up-regulated at the 192 hour, or 72 and 192 hour time points. Figures 33 and 33-1 through 33-6 show the same kind of data except that the samples come from the hypothalamus. Figures 36A, 36B and 36-1 through 36-6 show the same kind of data except that the samples come from the midbrain.

_Secreted markers:_ Figure 47B lists the genes that were differentially expressed at any time (p < 0.01) and is predicted or known to be a secreted product related to colon cancer. Secreted markers are particularly useful in that their expression can be detected in cerebral or cerebrospinal fluid, avoiding the need for a solid tissue biopsy.

_Gene annotation:_ Gene information was obtained from:

Entrez Gene (on the internet at ncbi.nlm.nih.gov/entrez),
LocusLink (on the internet at ncbi.nlm.nih.gov/LocusLink),
UniGene (on the internet at ncbi.nlm.nih.gov/UniGene), and
Mouse Genome Informatics (on the internet at informatics.jax.org).

Fields for annotation are "locus" (LocusLink number), "gene" (gene name), "description", "localization" (component), "biochemical function" (function), "biological function" (process), and "class."

Example 2: CNS Gene Expression Profile Associated With Breast Carcinoma

This example describes the identification of brain gene expression profiles associated with breast carcinoma.
BALB-C mice were injected subcutaneously with 1 x $10^5$ 4T-1 breast carcinoma cells (ATCC cat #: CRL-2539) resuspended in 100 $\mu$l of PBS. All experimental methods, microarrays and data analysis were otherwise performed as described above for Example 1.

Results

Quality filtering, normalization, and analysis of the microarray data were performed as discussed above.
_Cluster analysis:_ Only genes differentially expressed were included in cluster analyses. A given gene was considered differentially expressed if its expression ratio was significantly different from zero for the two analyzed data sets (BNS and BS). Thus, genes differentially expressed (p < 0.01) in dataset BNS that were also differentially expressed (p < 0.05) in dataset BS were included in the cluster analysis. Similarly, genes differentially expressed in dataset BS (p < 0.01) that were also differentially expressed (p < 0.05) in dataset BNS were included in cluster analysis. Figures 2, 3, and 4 list the genes that were considered differentially expressed in the prefrontal cortex at 18 hours, 72 hours, and 192 hours, respectively, after tumor cell injection. Figures 11, 12, and 13 list the genes that were considered differentially expressed in the hypothalamus at 18 hours, 72 hours, and 192 hours, respectively. Similarly, Figures 20, 21, and 22 list the genes that were considered differentially expressed in the midbrain at 18 hours, 72 hours, and 192 hours, respectively.
Figure 29 shows the results of a clustering analysis that included data on genes that were differentially expressed at the 18, 72, and 192 hour time points in the prefrontal cortex. Figure 29-1 shows the result of a clustering analysis that included genes that were down-regulated in the prefrontal cortex at all time points. Figure 29-2 shows the result of a clustering analysis that included genes that were down-regulated at the 18 hour, or at the 18 hour and 72 hour time points. Figure 29-3 shows the result of a clustering analysis that included genes that were down-regulated at the 192 hour, or 72 and 192 hour time points. Figure 29-4 shows the result of a clustering analysis that included genes that were up-regulated at all time points. Figure 29-5 shows the result of a clustering analysis that included genes that were up-regulated at the 18 hour, or the 18 and 72 hour time points. Figure 29-6 shows the result of a clustering analysis that included genes up-regulated at the 192 hour, or 72 and 192 hour time points. Figures 32A, 32B, and 32-1 through 32-6 show the same kind of data except that the samples come from the hypothalamus. Figures 35A, 35B, and 35-1 through 35-6 show the same kind of data except that the samples come from the midbrain.
_Secreted markers:_ Figure 47A lists the genes that were differentially expressed at any time (p < 0.01) and is predicted or known to be a secreted product related to breast cancer. Secreted markers are particularly useful in that their expression can be detected in cerebral or cerebrospinal fluid, avoiding the need for a solid tissue biopsy.

Example 3: CNS Gene Expression Profile Associated With Lung Carcinoma

This example describes the identification of brain gene expression profiles associated with lung carcinoma.
Male C-57/BL6 mice were injected subcutaneously with 1x$10^6$ lung carcinoma LL/2(LLC1) cells (ATCC cat #: CRL-1642) resuspended in 300 $\mu$l of PBS. All experimental methods, microarray and data analysis were otherwise performed as described above for Example 1.

Results

Quality filtering, normalization, and analysis of the microarray data was performed as discussed above.

_Cluster analysis:_ Only genes differentially expressed were included in cluster analyses. A given gene was considered differentially expressed if its expression ratio was significantly different from zero for the two analyzed data sets (BNS and BS). Thus, genes differentially expressed ($p < 0.01$) in dataset BNS that were also differentially expressed ($p < 0.05$) in dataset BS were included in the cluster analysis. Similarly, genes differentially expressed in dataset BS ($p < 0.01$) that were also differentially expressed ($p < 0.05$) in dataset BNS were included in cluster analysis. Figures 8, 9, and 10 list the genes that were considered differentially expressed in the prefrontal cortex at 18 hours, 72 hours, and 192 hours, respectively, after tumor cell injection. Figures 17, 18, and 19 list the genes that were considered differentially expressed in the hypothalamus at 18 hours, 72 hours, and 192 hours, respectively. Similarly, Figures 26, 27, and 28 list the genes that were considered differentially expressed in the midbrain at 18 hours, 72 hours, and 192 hours, respectively.

Figures 31A and 31B show the results of a clustering analysis that included data on genes that were differentially expressed at the 18, 72, and 192 hour time points in the prefrontal cortex. Figure 31-1 shows the result of a clustering analysis that included genes that were down-regulated in the prefrontal cortex at all time points. Figure 31-2 shows the result of a clustering analysis that included genes that were down-regulated at the 18 hour, or at the 18 hour and 72 hour time points. Figure 31-3 shows the result of a clustering analysis that included genes that were down-regulated at the 192 hour, or 72 and 192 hour time points. Figure 31-4 shows the result of a clustering analysis that included genes that were up-regulated at all time points. Figure 31-5 shows the result of a clustering analysis that included genes that were up regulated at the 18 hour, or the 18 and 72 hour time points. Figure 31-6 shows the result of a clustering analysis that included genes up regulated at the 192 hour, or 72 and 192 hour time points. Figures 34A, 34B, and 34-1 through 34-6 show the same kind of data except that the samples come from the hypothalamus. Figures 37A, 37B, and 37-1 through 37-6 show the same kind of data except that the samples come from the midbrain.

_Secreted markers:_ Figure 47C lists the genes that were differentially expressed at any time ($p < 0.01$) and is predicted or known to be a secreted product related to lung cancer. Secreted markers are particularly useful in that their expression can be detected in cerebral or cerebrospinal fluid, avoiding the need for a solid tissue biopsy.

Example 4: CNS Gene Expression Profile Associated With Carcinoma

This example describes the identification of brain gene expression profiles associated with any two of the following three types of cancer: lung carcinoma, breast carcinoma, and colon carcinoma.

All experimental methods, microarray and data analysis were otherwise performed as described above for Examples 1, 2, & 3.

In a final analysis, the filtered data was re-clustered to select sequences that were differentially expressed in any two of the three tumors analyzed and showed a similar expression pattern for these two tumor models. Figure 41 shows genes that were down-regulated in any two of the three cancer models analyzed from prefrontal cortex samples. Figure 42 shows genes that were up-regulated in any two of the three cancer models analyzed from prefrontal cortex samples. Figure 43 shows genes that were down-regulated in any two of the three cancer models analyzed from hypothalamus samples. Figure 44 shows genes that were up-regulated in any two of the three cancer models analyzed from hypothalamus samples. Figure 45 shows genes that were down-regulated in any two of the three cancer models analyzed from midbrain samples. Figure 46 shows genes that were up-regulated in any two of the three cancer models analyzed from midbrain samples.

Example 5: Real-time PCR validation of the Microarray Data Real-Time RT-PCR Conditions

_Reverse Transcription Reaction:_ 0.5 $\mu$g of mRNA were reverse-transcribed using 0.5 $\mu$g oligo(dT)$_{12\text{-}18}$ (Invitrogen) and 200 U of Superscript II RNaseH Reverse Transcriptase (Invitrogen). mRNA and oligo(dT) were mixed first, heated at 65°C for 5 minutes, and placed on ice until addition of remaining reaction components. The reaction was incubated at 42°C for 50 minutes, and terminated by heat inactivation at 70°C for 15 minutes. For mRNA degradation, 2 $\mu$l of 2.5 M NaOH were added to each cDNA reaction and incubated at 37°C for 15 minutes. Reactions were neutralized with 10 $\mu$l of 2 M HEPES free acid, and cDNA was ethanol precipitated using 1 $\mu$l of 20 mg/ml glycogen as carrier. The amount of cDNA was quantified using Oligreen ssDNA Quantitation Reagent (Invitrogen) according to manufacturer instructions.

_Reaction Setup and Cycling Conditions:_ Primers were designed using Primer3 program (available free on the internet at genome.wi.mit.edu/cgi-bin/primer/primer3.cgi/primer3_www.cgi), and purchased from Invitrogen. Each gene analyzed for validation was analyzed by comparing the gene with two housekeeping genes (beta2-microglobulin and beta-actin) using SYBR Green I (Invitrogen) in 96-well optical plates on an iCycler IQ Real-Time Detection System (Bio-Rad). For each 25 $\mu$l reaction, 1 $\mu$l cDNA dilution, 2.5 $\mu$l 10X PCR Buffer, 1.5 $\mu$l 50 mM MgCl$_2$, 0.75 $\mu$l 10 mM dNTP

Mix, 0.5 μl of each primer (10 μM), 0.75 μl SYBR Green I (1:1000 dilution), 0.25 μl 10 mg/ml BSA, 0.25 μl mM fluorescein dye (Bio-Rad), 0.25 μl glycerol, 16.55 μl, and 0.2 μl Platinum Taq DNA Polymerase (Invitrogen) were employed. PCR conditions were set as follows: 2.5 minutes at 94°C, and 40 cycles of 45 seconds at 94°C, 30 seconds at 58°C and 15 seconds at 72°C.

_Calculations:_ All samples were assayed by triplicate (n = 3), and each experiment performed by duplicate (n = 2). For analysis, first corrected Tm of each PCR product was checked. Then, efficiency of each reaction was tested using LinRegPCR program (available free on the internet at bioinfo@amc.uva.nl) according to Ramakers et.al., 2003, Neurosci. Lett., 339:62-66. Efficiencies between 85% and 100% were considered appropriate. One standard curve was constructed for each gene (generally 1000 ng, 100 ng, and 10 ng of cDNA dilution were employed), and the relative level of expression calculated according to Rajeevan et.al., 2001, Methods, 25(4):443-51. Normalization versus housekeeping expression were performed using geNorm program (available free on the internet at medgen31.ugent.be/jvdesomp/genorm) according to Vandesompele et.al., 2002, Genome Biol., 18;3(7):RESEARCH0034.

_Real-time PCR:_ The results obtained from a microarray experiment are influenced by each step in the experimental procedure, from array manufacturing to sample preparation and application to image analysis Brazma et al., 2000, FEBS Lett, 480:17-24. These factors affect the representation of transcripts in the sample, creating the need for validations by complementary techniques. Different techniques may be used for validation. Traditionally, measurements of mRNA levels have been achieved using hybridization-based techniques such as Northern blot, in situ hybridization and ribonuclease protection assay (RPA). However, these approaches are limited by hybridization kinetics and require large amounts of RNA. Additionally, the number of samples that can be handled simultaneously is very limited.

The accuracy of quantitative RT-PCR combined with its potential for high sample throughput makes it an ideal complement to microarray analysis. Real time quantitative PCR is a technique optimized to monitor the progress of the reaction by measuring the accumulation of the amplification products during each cycle via a change in fluorescence, (Gibson UE et al. Genome Res 1996,6:995-1001; Heid CA et al. Genome Res 1996, 6:986-994). SYBR Green was used for detection of PCR products. In solution, SYBR Green I exhibits very little fluorescence, however, fluorescence is greatly enhanced upon binding to the minor groove of the DNA double helix.

The analysis of gene expression in the brain is very complex. Although the brain has a few primary cell types, these show immense phenotypic diversity, and gene expression changes may affect only small cell subpopulations. Consequently, even profound transcriptome changes in a small subpopulation of brain cells may not be detected; more abundant sources of transcripts can mask these changes. As a result, the magnitude of expression changes found with microarray is often only modest and hard to separate from experimental noise (Mirnics K et al., 2004, Nature Neurosc, 7:434-439). For example, Wurmbach et al., 2003, Methods, 31:306-316 have shown that in mouse cerebral cortex after hallucinogens treatment, there was a 43% gene validation when microarray fold difference was greater than 1.6, but only 14.3% gene validation when the fold difference was between 1.3 and 1.6.

We started validating our results by using Real Time PCR analysis. As a first approach for validation we chosen ad random 14 differentially expressed genes. FIG. 49 shows a table comparing the fold difference obtained by microarray analysis versus the fold change obtained by real time PCR. Four genes out of 14 (29 %) were validated, when microarray folds were between 1.15 and 1.35. The genes that were validated were the following:

a) TOM1 (target of myb1 homolog) which has been reported to function in inflammatory cytokine-dependent signaling pathways induced by IL-1 beta and TNF-alpha (Yamakami M, 2004, Biol. Pharm.Bull., 27:564-566)
b) Ptpn11 (protein tyrosine phosphatase, non-receptor type 11) which has been reported to be involved in several signal transduction pathway, among them, a pathway required for neurite growth (Chen B. et al., 2002, Dev Biol., 15;252(2):170-87).
c) Cntn2 (Contactin 2) which has been reported to be involved in organization of mielydated fibers. (Traka M. et al., 2003, J. Cell Biol., 15;162(6):1161-72).
d) RIKEN cDNA 1200011M11, a novel gene with unknown function

Example 6: CNS Gene Expression Profile Associated With Asthma

This example describes the identification of brain gene expression profiles associated with asthma.

Eight week Balb-c males were intraperitoneally injected with 50 μg of ovalbumin (250 μl of a 200 μg/ml solution of ovalbumin in physiologic saline) for seven consecutive days. Negative control animals were injected with the corresponding volume of physiologic saline alone. All injections were done using a 27-G syringe. Three weeks after the last injection, the animals were exposed to repeated ovalbumin (2 mg/ml) aerosols for the asthma group or physiologic saline alone for the negative control group, once a day for 8 days. The aerosol was applied in one cage for each experimental group coupled to a nebulizer. Exposure was performed in groups of 5 animals for 5 minutes.

_ELISA for Detection of Ovalbumin-specific Antibodies in Serum:_ Blood samples were obtained after the last nebulization, stored 1 hour at room temperature centrifuged at 10,000 g for 10 minutes at room temperature. The supernatant (serum) was stored at - 80°C until use. 100 $\mu$l of Rat anti-mouse IgE 2$\mu$g/ml in PBS (pH 7.5) was added to a 96 well plate and incubated overnight at 4°C with agitation. The plate was washed 3x with 100 $\mu$l of wash buffer (PBS pH 7.5; 0.05% Tween 20). Blocking was done with 100 $\mu$l of blocking buffer (PBS pH 7.5; 1% BSA), and incubated 30 minutes at room temperature with agitation, then washed 3x with 100 $\mu$l of Wash buffer. Serum was added in appropriate dilution series in PBS (pH 7.5) and incubated overnight at 4°C with agitation. The next day 100 $\mu$l of a solution containing ovalbumin coupled to Digoxigenin (4 $\mu$g/ml) in blocking buffer was added and incubated 2 hours and 30 minutes with agitation at room temperature. The plate was washed 3x with 100 $\mu$l of wash buffer and 100 $\mu$l of anti-Digoxigenin-POD, Fab Fragments, diluted 1:1000 from the stock solution in wash buffer was added, and incubated 1hour and 30minutes at room temperature with agitation. The plate was washed 3x with 100 $\mu$l of wash buffer. Developing was done by adding 100 $\mu$l of developing solution (Citric Acid 48.8 mM; Sodium Phosphate basic 0.102 M; one O.P.D. pill to 7 ml of solution, H2O2 150X to make it 1X). The reaction was stopped with 100 $\mu$l of sulphuric acid 4N and read on an ELISA reader at 420 nm. Animals from the asthma group with levels of anti-ovalbumin IgE similar to controls animals were not included for dissection.

Methods for isolating total RNA, for labeling probes, for microarray hybridization and for data analysis were otherwise performed as described above for Example 1.

Results

Quality filtering, normalization and analysis of the microarray data were performed as discussed above.

A given gene was considered differentially expressed if its expression ratio was significantly different from zero for the two analyzed data sets (BNS and BS). Thus, genes differentially expressed ($p < 0.05$) in dataset BNS that were also differentially expressed ($p < 0.1$) in dataset BS were included in the cluster analysis. Similarly, genes differentially expressed in dataset BS ($p < 0.05$) that were also differentially expressed ($p < 0.1$) in dataset BNS were included in cluster analysis. Figure 55 lists the genes that were considered differentially expressed in the prefrontal cortex 2 days after exposure to ovalbumin. Figure 56 lists the genes that were considered differentially expressed in the hypothalamus 2 days after exposure to ovalbumin. Similarly, figures 57 lists the genes that were considered differentially expressed in the midbrain 2 days after exposure to ovalbumin.

*Secreted Markers:* Figure 60 lists the genes that were differentially expressed at any time ($p < 0.05$) and is predicted or known to be a secreted product related to asthma. Secreted markers are particularly useful in that their expression can be detected in cerebral or cerebrospinal fluid, avoiding the need for a solid tissue biopsy.

Example 7: CNS Gene Expression Profile Associated With Arthritis

This example describes the identification of brain gene expression profiles associated with arthritis.

Ten weeks C57BL/6J mice were intradermal injected at the base of the tail with 0.1 ml of chicken collagen type II (CII) emulsified with complete Freund's adjuvant at a final concentration of 2 mg/ml. Twenty-one days later, a booster (0.1 ml) consisting of CII emulsified with incomplete Freunds adjuvant (2 mg/ml) was injected intradermally too. A further three days later animals were injected with lipopolysacharide (40 mg in 0.1 ml phosphate-buffered saline (PBS); *E. coli* serotype 055:B5) intra-peritoneally.

*Clinical assessment of arthritis:* The development and progression of arthritis was monitored and a clinical score was assigned based on visual signs of arthritis (0.5 = swelling in the digits, difficulty to walk or pain (paw retraction); 1 = swelling of the paw; 2 = swelling of the paw and the ankle; 3 = complete inflammation). After three weeks, mice were killed by cervical dislocation, immediately decapitated, and the brain extracted and dissected as described below.

Methods for isolating total RNA, for labeling probes, for microarray hybridization and for data analysis were otherwise performed as described above for Example 1.

Results

Quality filtering, normalization and analysis of the microarray data were performed as discussed above.

A given gene was considered differentially expressed if its expression ratio was significantly different from zero for the two analyzed data sets (BNS and BS). Thus, genes differentially expressed ($p < 0.05$) in data set BNS that were also differentially expressed ($p < 0.1$) in data set BS were included in the cluster analysis. Similarly, genes differentially expressed in data set BS ($p < 0.05$) that were also differentially expressed ($p < 0.1$) in data set BNS were included in cluster analysis. Figure 51 lists the genes that were considered differentially expressed in the prefrontal cortex 24 days after the last lipopolysaccharide injection. Figure 52 lists the genes that were considered differentially expressed in the hypothalamus 24 days after the last lipopolysaccharide injection. Similarly, Figures 53 lists the genes that were considered differentially expressed in the midbrain 24 days after the last lipopolysaccharide injection.

*Secreted Markers:* Figure 58 lists the genes that were differentially expressed at any time ($p < 0.05$) and are predicted or known to be a secreted product related to arthritis. Secreted markers are particularly useful in that their expression can be detected in cerebral or cerebrospinal fluid, avoiding the need for a solid tissue biopsy.

Example 8: Diagnosis of Breast Cancer in a Human by Detecting a Gene Product Profile

This example describes a diagnostic test for non-CNS carcinoma performed on a human subject. The subject is a carrier of the BRCA1 breast cancer susceptibility gene.

A CSF sample is obtained from the subject by means of a lumbar puncture. This procedure is done on an outpatient basis under local anesthetic. The CSF sample is used immediately in the diagnostic assay, or is cooled or frozen and stored or transported to a facility where the diagnostic test is performed.

The diagnostic test involves contacting the CSF sample to an antibody array containing a panel of 3 antibodies that can detect a set (cluster) of CNS gene products that are associated with the presence of breast cancer when secreted in a characteristic profile in the CSF. The panel includes antibody probes for the three CNS markers for breast carcinoma listed in FIG. 47(A). Thus, in this example, the characteristic profile is the CNS "reference profile" for breast carcinoma.

The results of the antibody array are obtained by routine techniques, such as fluorescence detection and measurement of bound antibody vs. unbound antibody for each position (each antibody) on the array. A dataset of the value for the level of each polypeptide detected in the CSF sample by each antibody on the array is generated. The dataset is used directly as the test expression profile. A control expression profile is generated from the average results from antibody arrays of persons without breast carcinoma.

Once the test expression profile is generated, the test profile is compared to the reference expression profile and the control profile. In this example, the reference profile is a dataset that includes relative values of expression for a panel of 3 CNS gene products secreted into the CSF, all of which are known to be up-regulated in subjects who have early stage breast cancer. The Log2 ratios for those three genes are depicted as grey-scale levels in FIGS. 29, 32A, and 35B respectively. If the test profile shows a match, as defined herein, with the reference profile and the subject is determined to have (or be at risk for) early stage breast cancer.

Example 9: Diagnosis of Colon Cancer in a Human by Detecting a Gene Product Profile

This example describes a diagnostic test for colon carcinoma performed on a human subject. The subject is a person who has early stage colon cancer. Methods for obtaining a CSF sample from a subject is the same as in Example 8.

The diagnostic test involves contacting the CSF sample to an antibody array containing a panel of 3 antibodies that can detect a set (cluster) of CNS gene products that are associated with the presence of breast cancer when secreted in a characteristic profile in the CSF. The panel includes antibody probes for three of the seven CNS markers for colon carcinoma listed in FIG. 47(B). Thus, in this example, the characteristic profile is the CNS "reference profile" for colon carcinoma.

The results of the antibody array are obtained by routine techniques, such as fluorescence detection and measurement of bound antibody vs. unbound antibody for each position (each antibody) on the array. A dataset of the value for the level of each polypeptide detected in the CSF sample by each antibody on the array is generated. The dataset is used directly as the test expression profile. A control expression profile is generated from the average results from antibody arrays of persons without colon carcinoma.

Once the test expression profile is generated, the test profile is compared to the reference expression profile and the control profile. In this example, the reference profile is a dataset that includes relative values of expression for Ereg, Mgrn1, and Lhb), all of which are known to be up-regulated in subjects who have early stage breast cancer. The Log2 ratios for those three genes are depicted as grey-scale levels in FIGS. 30, 33, and 36 respectively (Ereg ratio = 0.67, Cortex 18 hr; Mgrn1 ratio = 1.095, average of 1.08 and 1.11, hypothalamus at 72 hr and 192 hr respectively; Lhb ratio = 0.92, average of 0.94 and 0.90, midbrain at 72 hr and 192 hr respectively. If the test profile shows a match, as defined herein, with the reference profile and the subject is determined to have (or be at risk for) early stage colon cancer.

**Claims**

1. A system for diagnosing a non-central nervous system (non-CNS) disorder in a subject, the system comprising:

a sampling device to obtain a central nervous system (CNS) sample comprising a cell from the brain of the subject, a cell from the spinal cord of the subject, or cerebrospinal fluid (CSF) from the subject; a gene expression detection device that generates gene expression data for one or more genes in the CNS sample, thereby providing a subject CNS gene expression profile; and

a computer loaded with a reference CNS gene expression profile for a specific non-CNS disorder; wherein the computer receives and compares the subject CNS gene expression profile with the reference CNS gene expression profile.

2. A method of diagnosing a non-central nervous system (non-CNS) disorder in a subject, the method comprising:

detecting expression of one or more genes in a CNS sample comprising a cell from the brain of the subject, a cell from the spinal cord of the subject, or cerebrospinal fluid (CSF) from the subject;
generating gene expression data from the detected expression, thereby providing a subject CNS gene expression profile;
obtaining a reference CNS gene expression profile for a specific non-CNS disorder; and
comparing the subject CNS gene expression profile with the reference CNS gene expression profile;

wherein a match of the subject CNS gene expression profile to the reference CNS gene expression profile indicates that the subject has or will develop the non-CNS disorder.

3. The system of claim 1 or the method of claim 2, wherein the CNS sample is a cerebrospinal fluid (CSF) sample.

4. The system of claim 1 or the method of claim 2, wherein the CNS sample is a cell from the brain of the subject.

5. The system of claim 1 or the method of claim 2, wherein the CNS sample is a cell from the spinal cord of the subject.

6. A system for diagnosing a non-central nervous system (non-CNS) disorder in a subject, the system comprising:

a sampling device to obtain a central nervous system (CNS) sample comprising a cell from the brain of the subject, a cell from the spinal cord of the subject, or cerebrospinal fluid (CSF) from the subject;
a protein detection device that generates expression data for one or more proteins in the CNS sample, thereby providing a subject CNS protein profile; and
a computer loaded with a reference CNS protein profile for a specific non-CNS disorder;

wherein the computer receives and compares the subject CNS protein profile with the reference CNS protein profile.

7. The system of claim 6, wherein the sample is CSF.

8. The system of claim 6, wherein the protein is selected from the group consisting of a hormone, a growth factor, an immune system component, and a cytokine.

9. The system of claim 6, wherein the protein is encoded by any of the genes listed in any of FIGS. 1, 50 and 54, or a human or other mammalian homolog thereof.

10. The system of claim 6, wherein the protein is selected from the group consisting of hepatocyte growth factor (HGF), apherin A3, chemokine (C-C motif) ligand 4, growth differentiation factor-9b (GDF-9b); bone morphogenetic protein 15 (BMP 15), neuroblastoma suppressor of tumorigenicity 1, melanocyte proliferating gene 1, and fibroblast growth factor 22 (FGF 22).

11. The system of claim 1 or the method of claim 2, wherein the gene expression profile includes gene expression data corresponding to the presence or level of a protein, wherein one protein is encoded by any of the genes listed in any of FIGS. 1, 50 and 54, or a human or other mammalian homolog thereof.

12. The system of claim 1 or the method of claim 2, wherein the brain cell is selected from the group consisting of a cell from: the hypothalamus, the midbrain, the prefrontal cortex and the striatum.

13. The method of claim 2, wherein detecting expression of a gene in a CNS sample comprises detecting the presence or level of mRNA corresponding to the gene.

14. The system of claim 1 or the method of claim 2, wherein two or more reference gene expression profiles are used, each specific for a different non-CNS disorder.

15. The system of claim 1 or 6 or the method of claim 2, wherein the non-CNS disorder is selected from the group consisting of cancer, rheumatoid arthritis, asthma, diabetes, and obesity.

16. The system of claim 1 or 6 or the method of claim 2, wherein the non-CNS disorder is a carcinoma.

17. The system of claim 1 or 6 or the method of claim 2, wherein the non-CNS disorder is a solid tumor less than 0.5 cm in diameter.

18. The system of claim 1 or 6 or the method of claim 2, wherein the gene expression data comprises data for a plurality of genes in the CNS sample.

19. The method of claim 2, further comprising:

obtaining a control CNS gene expression profile corresponding to one or more healthy subjects; and comparing the subject CNS gene expression profile with the control CNS gene expression profile, wherein a match of the subject CNS gene expression profile to the control CNS gene expression profile indicates the subject does not have and is not likely to develop the non-CNS disorder.

20. The system of claim 1 or the method of claim 2, wherein the gene expression is detected using a microarray assay.

21. The system of claim 1 or 6 or the method of claim 2, wherein the subject is a human.

22. The method of claim 2, wherein the subject CNS gene expression profile comprises gene expression data for two or more genes.

23. The method of claim 2, further comprising generating a record of the result of the comparing step; and optionally transmitting the record to the subject, health care provider, or other party.

24. The method of claim 2, wherein the subject CNS gene expression profile comprises gene expression data for five or more genes.

25. The method of claim 2, wherein the subject CNS gene expression profile comprises gene expression data for ten or more genes.

26. The method of claim 2, wherein detecting expression of one or more genes comprises performing a microarray assay.

27. A comparator loaded with a data set corresponding to a reference gene expression profile comprising expression data of five or more genes, wherein each of the five or more genes is differentially expressed in a central nervous system (CNS) sample of a mammal having a specific non-CNS disorder compared to the same five or more genes in a mammal not having the specific non-CNS disorder; wherein the central nervous system (CNS) sample comprises a cell from the brain of the mammal, a cell from the spinal cord of the mammal, or cerebrospinal fluid (CSF) from the mammal, and the data set is used to diagnose a non-CNS disorder.

28. The comparator of claim 27, wherein the reference gene expression profile comprises expression data of five or more genes selected from any of the genes listed in one or more of FIGs. 29-1 to 29-6; 32-1 to 32-6; or 35-1 to 35-6 for breast cancer; FIGs. 30-1 to 30-6; 33-1 to 33-6; or 36-1 to 36-6 for colon cancer; FIGS. 31-1 to 31-6; 34-1 to 34-6; or 37-1 to 37-6 for lung cancer; FIG. 50 for arthritis; or FIG. 54 for asthma.

29. The comparator of claim 27, wherein the five or more genes are selected from any one of the following groups of genes:

Breast Cancer: Nedd8 (FIG. 29-1), Col4a3bp (FIG. 29-2), Bgn (FIG. 29-4), Sox5 (FIG. 29-5), Slc38a4 (FIG. 32-1), Tom1 (FIG. 32-2), Cair (FIG. 32-4), Itgae (FIG. 32-5), Ttrap (FIG. 35-1), P ex11b (FIG. 35-2), Sema7a (FIG. 35-4), and Stam2 (FIG. 35-5);
Colon Cancer: Nmb (FIG. 30-1), Ry2 (FIG. 30-2), Trfr (FIG. 30-4), Mfap5 (FIG. 30-5), Prrg2 (FIG. 33-1), Faim (FIG. 33-2), Mgrn1 (FIG. 33-4), Stch (FIG. 33-5), Lhb (FIG. 36-1), Prm3 (FIG. 36-2), Crry (FIG. 36-4), and Timp4 (FIG. 36-5);
Lung cancer. Nmb (FIG. 31-1), Pcdh8 (FIG. 31-2), Rock2 (FIG. 31-4), Angpti3 (FIG. 31-5), Sqstm1 (FIG. 34-1), Kcnip2 (FIG. 34-2), Oxt (FIG. 34-4), Myh4 (FIG. 34-5), Enc1 (FIG. 37-1), Gsg1 (FIG. 37-2), Srr (FIG. 37-4), and Ndph (FIG. 37-5);

Arthritis: Bcl2l (FIG. 51A), P2rx1 (FIG. 51B), Pafah1b1 (FIG. 51B), Kcna3 (FIG. 51C), Taf1 b (FIG. 51 C), Slc38a3 (FIG. 51D). Hprt (FIG. 52A), C1d (FIG. 52B), Car11 (FIG. 52D), Dusp3 (FIG. 52D), Gabrr2 (FIG. 53C), and Aatk (FIG. 53D); and

Asthma: Rasa3 (FIG. 55B), Tnk2 (FIG. 55B), H28 (FIG. 55C), Diap2 (FIG. 55C), Lgals6 (FIG. 56A), Reck (FIG. 56A), Whrn (FIG. 56A), Stk22s1 (FIG. 568), CD47 (FIG. 57A), Jund1 (FIG. 57A), Cstb (FIG. 57B), and Desrt (FIG. 578).

30. A method of identifying a disease surveillance gene in the central nervous system (CNS) that indicates the presence of a non-CNS disorder in a human, the method comprising:

inducing a non-CNS disorder in a test non-human experimental animal; comparing expression of a gene in a CNS sample from the test experimental animal to expression of the gene in a CNS sample from a control experimental animal in which the disorder was not induced,

wherein the CNS samples comprise a cell from the brain of the subject, a cell from the spinal cord of the subject, or cerebrospinal fluid (CSF); and selecting as a disease surveillance gene a human homolog of a gene that is differentially expressed in the CNS sample from the test experimental animal compared to the CNS sample from the control experimental animal.

31. The method of claim 30, wherein a non-CNS neoplasm is induced by chemical or radiation mutagenesis.

32. The method of claim 30, wherein a non-CNS neoplasm is induced by administering a neoplastic cell to the experimental animal.

33. The method of claim 30, wherein the experimental animal is an animal model of rheumatoid arthritis, diabetes, asthma, obesity, or diabetes.

34. The method of claim 30, wherein the experimental animal is a mouse or non-human primate.

35. The system or method of any of the preceding claims, wherein the subject lacks a clinical sign of a non-CNS disorder as evaluated by imaging analysis.

36. The system or method of any of the preceding claims, wherein the subject has a family history of the non-CNS disorder.

37. The system or method of any of the preceding claims, wherein the subject is a carrier of a gene associated with an increased risk of developing the non-CNS disorder.

38. The system or method of claim 37, wherein the subject is a carrier of a gene selected from the group consisting of BRCA1, BRCA2, hMSH2, hMLH1, or hMSH6.

39. A method of generating a reference gene expression profile of one or more genes that are differentially expressed in a CNS sample of a mammal having a specific non-CNS disorder, the method comprising:

generating a first CNS gene expression profile comprising gene expression data from a sample from a control mammal not having the specific non-CNS disorder, wherein the sample comprises a cell from the brain of the control mammal, a cell from the spinal cord of the control mammal, or cerebrospinal fluid (CSF) from the control mammal;

generating a second CNS gene expression profile comprising gene expression data from a sample from a diseased mammal that has the specific non-CNS disorder, wherein the mammal is the same type as the control mammal, and wherein the sample comprises a cell from the brain of the diseased mammal, a cell from the spinal cord of the diseased mammal, or cerebrospinal fluid (CSF) from the diseased mammal;

comparing the first and second CNS gene expression profiles;

selecting a set of genes from the second CNS gene expression profile that are differentially expressed; and

preparing the reference gene expression profile from expression data from the selected genes.

**Patentansprüche**

1. System zur Diagnose einer nicht das Zentralnervensystem (Nicht-ZNS) betreffenden Störung bei einem Subjekt, wobei das System folgendes umfasst:

   eine Probennahmevorrichtung zur Gewinnung einer Probe des Zentralnervensystems (ZNS), die eine Zelle aus dem Gehirn des Subjekts, eine Zelle aus dem Rückenmark des Subjekts oder zerebrospinale Flüssigkeit (ZSF) des Subjekts umfasst;
   eine Genexpression-Nachweisvorrichtung, die Genexpressionsdaten für ein oder mehr Gene in der ZNS-Probe erzeugt, wodurch ein ZNS-Genexpressionsprofil des Subjekts bereitgestellt wird; und
   einen Rechner, der mit einem Referenz-ZNS-Genexpressionsprofil für eine spezifische Nicht-ZNS-Störung beladen ist; wobei der Rechner das ZNS-Genexpressionsprofil des Subjekts empfängt und mit dem Referenz-ZNS-Genexpressionsprofil vergleicht.

2. Verfahren zur Diagnose einer nicht das Zentralnervensystem (Nicht-ZNS) betreffenden Störung bei einem Subjekt, wobei das Verfahren folgendes umfasst:

   das Nachweisen der Expression von einem oder mehreren Genen in einer ZNS-Probe, die eine Zelle aus dem Gehirn des Subjekts, eine Zelle aus dem Rückenmark des Subjekts oder zerebrospinale Flüssigkeit (ZSF) des Subjekts umfasst;
   das Erzeugen von Genexpressionsdaten aus der nachgewiesenen Expression, wodurch ein ZNS-Genexpressionsprofil des Subjekts bereitgestellt wird;
   das Gewinnen eines Referenz-ZNS-Genexpressionsprofils für eine spezifische Nicht-ZNS-Störung und
   das Vergleichen des ZNS-Genexpressionsprofils des Subjekts mit dem Referenz-ZNS-Genexpressionsprofil;

   wobei eine Übereinstimmung des ZNS-Genexpressionsprofils des Subjekts mit dem Referenz-ZNS-Genexpressionsprofil anzeigt, dass das Subjekt die Nicht-ZNS-Störung aufweist oder diese zukünftig entwickelt.

3. System nach Anspruch 1 oder Verfahren nach Anspruch 2, wobei es sich bei der ZNS-Probe um eine Probe von zerebrospinaler Flüssigkeit (ZSF) handelt.

4. System nach Anspruch 1 oder Verfahren nach Anspruch 2, wobei es sich bei der ZNS-Probe um eine Zelle aus dem Gehirn des Subjekts handelt.

5. System nach Anspruch 1 oder Verfahren nach Anspruch 2, wobei es sich bei der ZNS-Probe um eine Zelle aus dem Rückenmark des Subjekts handelt.

6. System zur Diagnose einer nicht das Zentralnervensystem (Nicht-ZNS) betreffenden Störung bei einem Subjekt, wobei das System folgendes umfasst:

   eine Probennahmevorrichtung zur Gewinnung einer Probe des Zentralnervensystems (ZNS), die eine Zelle aus dem Gehirn des Subjekts, eine Zelle aus dem Rückenmark des Subjekts oder zerebrospinale Flüssigkeit (ZSF) des Subjekts umfasst;
   eine Protein-Nachweisvorrichtung, die Expressionsdaten für ein oder mehr Proteine in der ZNS-Probe erzeugt, wodurch ein ZNS-Proteinprofil des Subjekts bereitgestellt wird; und
   einen Rechner, der mit einem Referenz-ZNS-Proteinprofil für eine spezifische Nicht-ZNS-Störung beladen ist;

   wobei der Rechner das ZNS-Proteinprofil des Subjekts empfängt und mit dem Referenz-ZNS-Proteinprofil vergleicht.

7. System nach Anspruch 6, wobei es sich bei der Probe um ZSF handelt.

8. System nach Anspruch 6, wobei das Protein aus der Gruppe ausgewählt ist, die aus einem Hormon, einem Wachstumsfaktor, einer Immunsystemkomponente und einem Cytokin besteht.

9. System nach Anspruch 6, wobei das Protein durch ein beliebiges der Gene, die in einer der Figuren 1, 50 und 54 aufgeführt sind, oder durch ein humanes oder anderes Säugetier-Homologes davon kodiert wird.

10. System nach Anspruch 6, wobei das Protein aus der Gruppe ausgewählt ist, die aus Hepatozyten-Wachstumsfaktor (HGF), Apherin A3, Chemokin (C-C-Motiv)-Ligand 4, Wachstumsdifferenzierungsfaktor 9b (GDF-9b), knochenmorphogenetischem Protein 15 (BMP 15), Neuroblastom-Suppressor von Tumorigenizität 1, Melanozyten-Proliferationsgen 1 und Fibroblasten-Wachstumsfaktor 22 (FGF 22) besteht.

11. System nach Anspruch 1 oder Verfahren nach Anspruch 2, wobei das Genexpressionsprofil Genexpressionsdaten einschließt, die der Anwesenheit oder der Konzentration eines Proteins entsprechen, wobei das Protein durch eines der in den Figuren 1, 50 und 54 aufgeführten Gene oder ein humanes oder ein anderes Säugetier-Homologes davon kodiert wird.

12. System nach Anspruch 1 oder Verfahren nach Anspruch 2, wobei die Gehirnzelle aus der Gruppe ausgewählt ist, die aus einer Zelle aus dem Hypothalamus, dem Mittelhirn, der präfrontalen Hirnrinde und dem Striatum besteht.

13. Verfahren nach Anspruch 2, wobei das Nachweisen der Expression eines Gens in einer ZNS-Probe das Nachweisen der Anwesenheit oder der Konzentration von mRNA, die dem Gen entspricht, umfasst.

14. System nach Anspruch 1 oder Verfahren nach Anspruch 2, wobei zwei oder mehr Referenz-Genexpressionsprofile verwendet werden, von denen jedes für eine unterschiedliche Nicht-ZNS-Störung spezifisch ist.

15. System nach Anspruch 1 oder 6 oder Verfahren nach Anspruch 2, wobei die Nicht-ZNS-Störung aus der Gruppe ausgewählt ist, die aus Krebs, rheumatoider Arthritis, Asthma, Diabetes und Fettleibigkeit besteht.

16. System nach Anspruch 1 oder 6 oder Verfahren nach Anspruch 2, wobei es sich bei Nicht-ZNS-Störung um ein Karzinom handelt.

17. System nach Anspruch 1 oder 6 oder Verfahren nach Anspruch 2, wobei es sich bei der Nicht-ZNS-Störung um einen soliden Tumor mit einem Durchmesser von weniger als 0,5 cm handelt.

18. System nach Anspruch 1 oder 6 oder Verfahren nach Anspruch 2, wobei die Genexpressionsdaten Daten für eine Mehrzahl von Genen in der ZNS-Probe umfassen.

19. Verfahren nach Anspruch 2, ferner umfassend:

das Gewinnen eines Kontroll-ZNS-Genexpressionsprofils, entsprechend einem oder mehreren gesunden Subjekten; und

das Vergleichen des ZNS-Genexpressionsprofils des Subjekts mit dem Kontroll-ZNS-Genexpressionsprofil, wobei eine Übereinstimmung des ZNS-Genexpressionsprofils des Subjekts mit dem Kontroll-ZNS-Genexpressionsprofil ein Anzeichen dafür darstellt, dass das Subjekt die Nicht-ZNS-Störung nicht aufweist und vermutlich nicht entwickeln wird.

20. System nach Anspruch 1 oder Verfahren nach Anspruch 2, wobei die Genexpression unter Verwendung eines Mikroarray-Tests nachgewiesen wird.

21. System nach Anspruch 1 oder 6 oder Verfahren nach Anspruch 2, wobei es sich bei dem Subjekt um einen Menschen handelt.

22. Verfahren nach Anspruch 2, wobei das ZNS-Genexpressionsprofil des Subjekts Genexpressionsdaten für zwei oder mehr Gene umfasst.

23. Verfahren nach Anspruch 2, ferner umfassend das Erzeugen eines Berichts über das Ergebnis der Vergleichsstufe und gegebenenfalls das Übermitteln des Berichts an das Subjekt, an eine medizinische Institution oder an Dritte.

24. Verfahren nach Anspruch 2, wobei das ZNS-Genexpressionsprofil des Subjekts Genexpressionsdaten für fünf oder mehr Gene umfasst.

25. Verfahren nach Anspruch 2, wobei das ZNS-Genexpressionsprofil des Subjekts Genexpressionsdaten für zehn oder mehr Gene umfasst.

26. Verfahren nach Anspruch 2, wobei das Nachweisen von einem oder mehreren Genen die Durchführung eines Mikroarray-Tests umfasst.

27. Komparator, beladen mit einem Datensatz, der einem Referenz-Genexpressionsprofil entspricht, das Expressiondaten für fünf oder mehr Gene umfasst, wobei die fünf oder mehr Gene differentiell in einer Probe des Zentralnervensystems (ZNS) eines Säugers mit einer spezifischen, nicht das Zentralnervensystem betreffenden Störung exprimiert werden, verglichen mit den gleichen fünf oder mehr Genen in einem Säuger, der nicht die spezifische Nicht-ZNS-Störung aufweist, wobei die Probe des Zentralnervensystems (ZNS) eine Zelle aus dem Gehirn des Säugers, eine Zelle aus dem Rükkenmark des Säugers oder zerebrospinale Flüssigkeit (ZSF) des Säugers umfasst und wobei der Datensatz zur Diagnose einer Nicht-ZNS-Störung verwendet wird.

28. Komparator nach Anspruch 27, wobei das Referenz-Genexpressionsprofil Genexpressionsdaten von fünf oder mehr Genen umfasst, die aus beliebigen der Gene ausgewählt sind, die in einer oder mehreren der Figuren 29-1 bis 29-6; 32-1 bis 32-6; oder 35-1 bis 35-6 für Brustkrebs; Figuren 30-1 bis 30-6; 33-1 bis 33-6; oder 36-1 bis 36-6 für Kolonkrebs; Figuren 31-1 bis 31-6; 34-1 bis 34-6; oder 37-1 bis 37-6 für Lungenkrebs; Figur 50 für Arthritis; oder Figur 54 für Asthma aufgeführt sind.

29. Komparator nach Anspruch 27, wobei die fünf oder mehr Gene aus einer beliebigen der folgenden Gruppen von Genen ausgewählt sind:

Brustkrebs: Nedd8 (FIG. 29-1), Co14a3bp (FIG. 29-2), Bgn (FIG. 29-4), Sox5 (FIG. 29-5), Slc38a4 (FIG. 32-1), Tom1 (FIG. 32-2), Calr (FIG. 32-4), Itgae (FIG. 32-5), Ttrap (FIG. 35-1), Pex11b (FIG. 35-2), Sema7a (FIG. 35-4) und Stam2 (FIG. 35-5);
Kolonkrebs: Nmb (FIG. 30-1), Ryr2 (FIG. 30-2), Trfr (FIG. 30-4), Mfap5 (FIG. 30-5), Prrg2 (FIG. 33-1), Faim (FIG. 33-2), Mgm1 (FIG. 33-4), Stch (FIG. 33-5), Lhb (FIG. 36-1), Prm3 (FIG. 36-2), Crry (FIG. 36-4) und Timp4 (FIG. 36-5);
Lungenkrebs: Nmb (FIG. 31-1), Pcdh8 (FIG. 31-2), Rock2 (FIG. 31-4), Angpt13 (FIG 31-5), Sqstm1 (FIG. 34-1), Kcnip2 (FIG. 34.-2), Oxt (FIG. 34-4), Myh4 (FIG. 34- 5), Enc1 (FIG. 37-1), Gsg1 (FIG. 37-2), Srr (FIG. 37-4) und Ndph (FIG. 37-5);
Arthritis: Bc121 (FIG. 51A), P2rx1 (FIG. 51B), Pafahlb1 (FIG. 51B), Kcna3 (FIG. 51C), Taflb (FIG. 51C), Slc38a3 (FIG. 51D), Hprt (FIG. 52A), C1d (FIG. 52B), Carl1 (FIG. 52D), Dusp3 (FIG. 52D), Gabrr2 (FIG. 53C) und Aatk (FIG. 53D); und
Asthma: Rasa3 (FIG. 55B), Tnk2 (FIG. 55B), H28 (FIG. 55C), Diap2 (FIG.55C), Lgals66 (FIG. 56A), Reck (FIG. 56A), Whrn (FIG. 56A), Stk22s1 (FIG. 56B), CD47 (FIG. 57A), Jund1 (FIG. 57A), Cstb (FIG. 57B) und Desrt (FIG. 57B).

30. Verfahren zum Identifizieren eines Krankheitsüberwachungsgens im Zentralnervensystem (ZNS), das die Anwesenheit einer Nicht-ZNS-Störung bei einem Menschen anzeigt, wobei das Verfahren folgendes umfasst:

das Induzieren einer Nicht-ZNS-Störung in einem nicht-humanen Versuchstier;
das Vergleichen der Expression eines Gens in einer ZNS-Probe aus dem Testversuchstier mit der Expression des Gens in einer ZNS-Probe aus einem Kontrollversuchstier, bei dem die Störung nicht induziert worden ist, wobei die ZNS-Probe eine Zelle aus dem Gehirn des Subjekts, eine Zelle aus dem Rückenmark des Subjekts oder zerebrospinale Flüssigkeit (ZSF) umfasst; und
das Auswählen eines humanen Homologen eines Gens, das differentiell in der ZNS-Probe des Versuchstiers exprimiert wird, als Krankheitsüberwachungsgen, verglichen mit der ZNS-Probe aus dem Kontrollversuchstier.

31. Verfahren nach Anspruch 30, wobei ein Nicht-ZNS-Neoplasma durch chemische Mutagenese oder Strahlungsmutagenese induziert wird.

32. Verfahren nach Anspruch 30, wobei ein Nicht-ZNS-Neoplasma durch Verabreichen einer neoplastischen Zelle an das Versuchstier induziert wird.

33. Verfahren nach Anspruch 30, wobei es sich beim Versuchstier um ein Tiermodell für rheumatoide Arthritis, Diabetes, Asthma, Fettleibigkeit oder Diabetes handelt.

34. Verfahren nach Anspruch 30, wobei es sich beim Versuchstier um eine Maus oder einen nicht-humanen Primaten handelt.

**35.** System oder Verfahren nach einem der vorstehenden Ansprüche, wobei dem Subjekt ein klinisches Anzeichen für eine Nicht-ZNS-Störung gemäß Ermittlung durch bildgebende Analyse fehlt.

**36.** System oder Verfahren nach einem der vorstehenden Ansprüche, wobei das Subjekt eine Familienkrankengeschichte der Nicht-ZNS-Störung aufweist.

**37.** System oder Verfahren nach einem der vorstehenden Ansprüche, wobei das Subjekt ein Träger eines Gens ist, das mit einem erhöhten Risiko zur Entwicklung der Nicht-ZNS-Störung verbunden ist.

**38.** System oder Verfahren nach Anspruch 37, wobei es sich bei dem Subjekt um einen Träger eines Gens handelt, das aus der Gruppe BRCA1, BRCA2, hMSH2, hMLH1 oder hMSH6 ausgewählt ist.

**39.** Verfahren zur Erzeugung eines Referenz-Genexpressionspsrofils von einem oder mehreren Genen, die differentiell in einer ZNS-Probe eines Säugers mit einer spezifischen Nicht-ZNS-Störung exprimiert werden, wobei das Verfahren folgendes umfasst:

das Erzeugen eines ersten ZNS-Genexpressionsprofils, das Genexpressionsdaten aus einer Probe eines Kontrollsäugers, der nicht die spezifische Nicht-ZNS-Störung aufweist, umfasst, wobei die Probe eine Zelle aus dem Gehirn des Kontrollsäugers, eine Zelle aus dem Rückenmark des Kontrollsäugers oder zerebrospinale Flüssigkeit (ZSF) des Kontrollsäugers umfasst;
das Erzeugen eines zweiten ZNS-Genexpressionsprofils, das Genexpressionsdaten aus einer Probe eines erkrankten Säugers, der die spezifische Nicht-ZNS-Störung aufweist, umfasst, wobei der Säuger dem gleichen Typ wie der Kontrollsäuger angehört und wobei die Probe eine Zelle aus dem Gehirn des erkrankten Säugers, eine Zelle aus dem Rückenmark des erkrankten Säugers oder zerebrospinale Flüssigkeit (ZSF) des erkrankten Säugers umfasst;
das Vergleichen des ersten und des zweiten ZNS-Genexpressionsprofils;
das Auswählen eines Satzes von Genen aus dem zweiten ZNS-Genexpressionsprofil, die differentiell exprimiert werden; und
das Erstellen des Referenz-Genexpressionsprofils aus den Expressionsdaten der ausgewählten Gene.

**Revendications**

**1.** Système pour diagnostiquer des troubles d'un système nerveux non central (SN non-C) chez un sujet, le système comprenant :

un dispositif de prélèvement pour obtenir un échantillon du système nerveux central (SNC) comprenant une cellule cérébrale du sujet, une cellule de la moelle épinière du sujet ou du liquide céphalorachidien (LCR) du sujet ;
un dispositif de détection d'expression génétique qui génère des données d'expression génétique pour un ou plusieurs gènes dans l'échantillon du SNC, fournissant ainsi un profil d'expression génétique du SNC du sujet ; et
un ordinateur contenant un profil d'expression génétique de SNC de référence pour un trouble spécifique de SN non-C ; dans lequel l'ordinateur reçoit le profil d'expression génétique du SNC du sujet et le compare au profil d'expression génétique de SNC de référence.

**2.** Méthode de diagnostic des troubles d'un système nerveux non central (SN non-C) chez un sujet, la méthode comprenant les étapes consistant à :

détecter l'expression d'un ou plusieurs gènes dans un échantillon de SNC comprenant une cellule cérébrale du sujet, une cellule de la moelle épinière du sujet ou du liquide céphalorachidien (LCR) du sujet ;
générer des données d'expression génétique à partir de l'expression détectée, fournissant ainsi un profil d'expression génétique du SNC du sujet ;
obtenir un profil d'expression génétique de SNC de référence pour un trouble spécifique de SN non-C ; et
comparer le profil d'expression génétique du SNC du sujet au profil d'expression génétique de SNC de référence ;

dans laquelle une correspondance du profil d'expression génétique du SNC du sujet et du profil d'expression génétique de SNC de référence indique que le sujet est atteint du trouble du SN non-C ou qu'il est susceptible de le développer.

3. Système selon la revendication 1, ou méthode selon la revendication 2, dans lesquels l'échantillon de SNC est un échantillon de liquide céphalorachidien (LCR).

4. Système selon la revendication 1, ou méthode selon la revendication 2, dans lesquels l'échantillon de SNC est une cellule cérébrale du sujet.

5. Système selon la revendication 1, ou méthode selon la revendication 2, dans lesquels l'échantillon de SNC est une cellule de la moelle épinière du sujet.

6. Système pour diagnostiquer des troubles d'un système nerveux non central (SN non-C) chez un sujet, le système comprenant :

   un dispositif de prélèvement pour obtenir un échantillon du système nerveux central (SNC) comprenant une cellule cérébrale du sujet, une cellule de la moelle épinière du sujet ou du liquide céphalorachidien (LCR) du sujet ;
   un dispositif de détection de protéines qui génère des données d'expression pour une ou plusieurs protéines dans l'échantillon de SNC, fournissant ainsi un profil protéique du SNC du sujet ; et
   un ordinateur contenant un profil protéique de SNC de référence pour un trouble spécifique de SN non-C ;

   dans lequel l'ordinateur reçoit le profil protéique du SNC du sujet et le compare au profil protéique de SNC de référence.

7. Système selon la revendication 6, dans lequel l'échantillon est du LCR.

8. Système selon la revendication 6, dans lequel la protéine est choisie dans le groupe comprenant une hormone, un facteur de croissance, un composant de système immunitaire et une cytokine.

9. Système selon la revendication 6, dans lequel la protéine est codée par n'importe lequel des gènes énumérés sur n'importe laquelle des figures 1,50 et 54, ou un équivalent de ceux-ci, humain ou d'un autre mammifère.

10. Système selon la revendication 6, dans lequel la protéine est choisie dans le groupe comprenant un facteur de croissance hépatocytaire (HGF), l'apherine A3, un ligand 4 de chimiokine (motif C-C), un facteur de différenciation de croissance 9b (GDF-9b), une protéine morphogène osseuse 15 (BMP 15), un suppresseur 1 d'oncogenèse du neuroblastome, un gène 1 de prolifération de mélanocyte, et un facteur de croissance fibroblastique 22 (FGF 22).

11. Système selon la revendication 1 ou méthode selon la revendication 2, dans lesquels le profil d'expression génétique comprend des données d'expression génétique correspondant à la présence ou au niveau d'une protéine, dans lequel une protéine est codée par n'importe lequel des gènes énumérés sur n'importe laquelle des figures 1, 50 et 54 ou un équivalent de ceux-ci, humain ou d'un autre mammifère.

12. Système selon la revendication 1 ou méthode selon la revendication 2, dans lesquels la cellule cérébrale est choisie dans le groupe comprenant une cellule issue de : l'hypothalamus, le mésencéphale, le cortex préfrontal et le striatum.

13. Méthode selon la revendication 2, dans laquelle la détection de l'expression d'un gène dans un échantillon de SNC comprend la détection de la présence ou d'un niveau de ARNm correspondant au gène.

14. Système selon la revendication 1 ou méthode selon la revendication 2, dans lesquels deux ou plusieurs profils d'expression génétique de référence sont utilisés, chacun d'entre eux étant spécifique pour un trouble différent de SN non-C.

15. Système selon la revendication 1 ou 6 ou méthode selon la revendication 2, dans lesquels le trouble de SN non-C est choisi dans le groupe comprenant le cancer, la polyarthrite rhumatoïde, l'asthme, le diabète et l'obésité.

16. Système selon la revendication 1 ou 6 ou méthode selon la revendication 2, dans lesquels le trouble de SN non-C est un carcinome.

17. Système selon la revendication 1 ou 6 ou méthode selon la revendication 2, dans lesquels le trouble de SN non-C est une tumeur solide de diamètre inférieur à 0,5 cm.

**18.** Système selon la revendication 1 ou 6 ou méthode selon la revendication 2, dans lesquels les données d'expression génétique comprennent des données concernant une pluralité de gènes dans l'échantillon de SNC.

**19.** Méthode selon la revendication 2, comprenant en outre les étapes consistant à :

obtenir un profil témoin d'expression génétique de SNC correspondant à un ou plusieurs sujets sains ; et comparer le profil d'expression génétique du SNC du sujet au profil témoin d'expression génétique de SNC, dans laquelle une correspondance entre le profil d'expression génétique du SNC du sujet et le profil témoin d'expression génétique de SNC indique que le sujet n'est pas atteint du trouble du SN non-C et qu'il n'est pas susceptible de le développer.

**20.** Système selon la revendication 1 ou méthode selon la revendication 2, dans lesquels l'expression génétique est détectée en utilisant une analyse par microréseaux.

**21.** Système selon la revendication 1 ou 6 ou méthode selon la revendication 2, dans lesquels le sujet est un humain.

**22.** Méthode selon la revendication 2, dans laquelle le profil d'expression génétique du SNC du sujet comprend des données d'expression génétique pour deux ou plusieurs gènes.

**23.** Méthode selon la revendication 2, comprenant en outre les étapes consistant à :

générer un enregistrement du résultat de l'étape de comparaison ; et transmettre optionnellement l'enregistrement au sujet, au prestataire de soins de santé ou autre.

**24.** Méthode selon la revendication 2, dans laquelle le profil d'expression génétique du SNC du sujet comprend des données d'expression génétique pour cinq gènes ou plus.

**25.** Méthode selon la revendication 2, dans laquelle le profil d'expression génétique du SNC du sujet comprend des données d'expression génétique pour dix gènes ou plus.

**26.** Méthode selon la revendication 2, dans laquelle la détection de l'expression d'un ou plusieurs gènes comprend l'exécution d'une analyse par microréseaux.

**27.** Comparateur chargé d'un ensemble de données correspondant à un profil d'expression génétique de référence comprenant des données d'expression de cinq gènes ou plus, dans lequel chacun des cinq gènes ou plus est exprimé de manière différentielle dans un échantillon de système nerveux central (SNC) d'un mammifère atteint d'un trouble spécifique du SN non-C comparativement aux mêmes cinq gènes ou plus chez un mammifère non atteint du trouble spécifique du SN non-C, dans lequel l'échantillon du système nerveux central (SNC) comprend une cellule cérébrale du mammifère, une cellule de la moelle épinière du mammifère, ou du liquide céphalorachidien (LCR) du mammifère, et l'ensemble de données est utilisé pour diagnostiquer un trouble du SN non-C.

**28.** Comparateur selon la revendication 27, dans lequel le profil d'expression génétique de référence comprend des données d'expression de cinq gènes ou plus, choisis parmi n'importe lesquels des gènes énumérés sur une ou plusieurs des figures 29-1 à 29-6, 32-1 à 32-6 ou 35-1 à 35-6 pour le cancer du sein ; des figures 30-1 à 30-6, 33-1 à 33-6 ou 36-1 à 36-6 pour le cancer du côlon ; des figures 31-1 à 31-6, 34-1 à 34-6 ou 37-1 à 37-6 pour le cancer du poumon ; la figure 50 pour l'arthrite ; ou la figure 54 pour l'asthme.

**29.** Comparateur selon la revendication 27, dans lequel les cinq gènes ou plus sont choisis dans n'importe lequel des groupes de gènes suivants :

cancer du poumon : Nedd8 (figure 29-1), Col4a3bp (figure 29-2), Bgn (figure 29-4), Sox5 (figure 29-5), Sic38a4 (figure 32-1), Tom1 (figure 32-2), Cair (figure 32-4), Itgae (figure 32-5), Ttrap (figure 35-1), Pex11b (figure 35-2), Sema7a (figure 35-4) et Stam2 (figure 35-5) ;
cancer du côlon : Nmb (figure 30-1), Ryr2 (figure 30-2), Trfr (figure 30-4), Mfap5 (figure 30-5), Prrg2 (figure 33-1), Faim (figure 33-2), Mgml (figure 33-4), Stch (figure 33-5), Lhb (figure 36-1), Prm3 (figure 36-2), Crry (figure 36-4) et Timp4 (figure 36-5) ;
cancer du poumon : Nmb (figure 31-1), Pcdh8 (figure 31-2), Rock2 (figure 31-4), Angpt13 (figure 31-5), Sqstm1 (figure 34-1), Kcnip2 (figure 34-2), Oxt (figure 34-4), Myh4 (figure 34-5), Enc1 (figure 37-1), Gsg1 (figure 37-2),

**EP 1 649 062 B1**

Srr (figure 37-4) et Ndph (figure 37-5) ;

arthrite : B c121 (figure 51 A), P2rx1 (figure 51B), Pafahlb1 (figure 51B), Kcna3 (figure 51C), Taflb (figure 51C), Sic3 8a3 (figure 51D), Hprt (figure 52A), C1d (figure 52B), Carl1 (figure 52D), Dusp3 (figure 52D), Gabrr2 (figure 53C) et Aatk (figure 53D) ; et

asthme : Rasa3 (figure 55B), Tnk2 (figure 55B), H28 (figure 55C), Diap2 (figure 55C), Lgals6 (figure 56A), Reck (figure 56A), Whm (figure 56A), Stk22s1 (figure 56B), CD47 (figure 57A), Jund1 (figure 57A), Cstb (figure 57B) et Desrt (figure 57B).

30. Méthode d'identification d'un gène indicateur de maladie dans le système nerveux central (SNC), qui indique la présence d'un trouble de SN non-C chez un humain, la méthode comprenant les étapes consistant à :

induire un trouble de SN non-C chez un animal de laboratoire d'essai non humain ;

comparer l'expression d'un gène dans un échantillon de SNC de l'animal de laboratoire d'essai à l'expression du gène dans un échantillon de SNC appartenant à un animal de laboratoire témoin chez lequel le trouble n'a pas été induit,

dans laquelle les échantillons de SNC comprennent une cellule cérébrale du sujet, une cellule de la moelle épinière du sujet ou du liquide céphalorachidien (LCR) ; et choisir en guise de gène indicateur de maladie un équivalent humain d'un gène qui est exprimé de manière différentielle dans l'échantillon du SNC de l'animal de laboratoire d'essai comparativement à l'échantillon du SNC de l'animal de laboratoire témoin.

31. Méthode selon la revendication 30, dans laquelle un néoplasme de SN non-C est induit par mutagenèse chimique ou mutagenèse par radiation.

32. Méthode selon la revendication 30, dans laquelle un néoplasme de SN non-C est induit en administrant une cellule néoplasique à l'animal de laboratoire.

33. Méthode selon la revendication 30, dans laquelle l'animal de laboratoire est un modèle animal pour la polyarthrite rhumatoïde, le diabète, l'asthme, l'obésité ou le diabète.

34. Méthode selon la revendication 30, dans laquelle l'animal de laboratoire est une souris ou un primate non humain.

35. Système ou méthode selon l'une quelconque des revendications précédentes, dans lesquels le sujet ne présente pas de signe clinique de trouble de SN non-C tel qu'il a été évalué par une analyse par imagerie.

36. Système ou méthode selon l'une quelconque des revendications précédentes, dans lesquels le sujet a une histoire familiale du trouble de SN non-C.

37. Système ou méthode selon l'une quelconque des revendications précédentes, dans lesquels le sujet est porteur d'un gène associé à un risque accru de développement du trouble de SN non-C.

38. Système ou méthode selon la revendication 37, dans lesquels le sujet est porteur d'un gène choisi dans le groupe comprenant le BRCA1, le BRCA2, le hMSH2, le hMLH1, ou le hMSH6.

39. Méthode de génération d'un profil d'expression génétique de référence d'un ou plusieurs gènes qui sont exprimés de manière différentielle dans un échantillon de SNC d'un mammifère atteint d'un trouble spécifique du SN non-C, la méthode comprenant les étapes consistant à :

générer un premier profil d'expression génétique de SNC comprenant des données d'expression génétique issues d'un échantillon prélevé sur un mammifère témoin non atteint du trouble spécifique de SN non-C, dans laquelle l'échantillon comprend une cellule cérébrale du mammifère témoin, une cellule de la moelle épinière du mammifère témoin, ou du liquide céphalorachidien (LCR) du mammifère témoin ;

générer un second profil d'expression génétique de SNC comprenant des données d'expression génétique issues d'un échantillon prélevé sur un mammifère malade atteint du trouble spécifique de SN non-C, dans laquelle le mammifère est du même type que le mammifère témoin, et dans laquelle l'échantillon comprend une cellule cérébrale du mammifère malade, une cellule de la moelle épinière du mammifère malade, ou du liquide céphalorachidien (LCR) du mammifère malade ;

comparer le premier et le second profil d'expression génétique de SNC ;

choisir un ensemble de gènes exprimés de manière différentielle dans le second profil d'expression génétique de SNC ; et

préparer le profil d'expression génétique de référence à partir des données d'expression des gènes sélectionnés.

| Access | Locus | Gene |
|---|---|---|
| AB023957 | 170773 | AB023957 |
| AB030906 | 29871 | Scmh1 |
| AB035322 | 108897 | |
| AB036708 | 170828 | Vgll1 |
| AB039178 | | |
| AB041576 | 58242 | Nudt11 |
| AB041610 | 97484 | Cog8 |
| AB042402 | 14695 | Gnb3 |
| AB045323 | 52357 | D8Ertd594e |
| AB047323 | | |
| AB047820 | 104416 | Bap1 |
| AB047921 | 67210 | |
| AB048834 | 64435 | Fcamr |
| AB052945 | 109904 | Mcf2 |
| AB055154 | 108138 | Xrcc4 |
| AB055635 | 26931 | Ppp2r5c |
| AB056100 | 66902 | Mtap |
| AB071988 | 26914 | H2afy |
| AC079832 | 286947 | Adh6b |
| AF030001 | | a lot |
| AF035526 | 20534 | Slc4a1ap |
| AF060088 | 26440 | Psma1 |
| AF061933 | 50779 | Rgs6 |
| AF061934 | 50782 | Rgs11 |
| AF062567 | 20687 | Sp3 |
| AF071173 | 15204 | Herc2 |
| AF072758 | 26568 | Slc27a3 |
| AF078112 | 12328 | Caml ? |
| AF083876 | 13731 | Emp2 |
| AF090738 | 16368 | Irs2 |
| AF092877 | 19823 | Rnf7 |
| AF100956 | | a lot |
| AF104414 | 16798 | Lats1 |
| AF105217 | 57813 | Tk2 |
| AF106621 | 21855 | Timm17b |
| AF121976 | 57250 | Ors19 |
| AF127245 | 20591 | Smcx |
| AF129888 | 20918 | Sui1-rs1 |
| AF133300 | 259102 | Olfr630 |
| AF150755 | 11426 | Macf1 |
| AF155583 | 18089 | Nkx2-3 |
| AF158744 | 67310 | Prlpc2 |
| AF162224 | 30924 | Angptl3 |
| AF162781 | 16332 | Inppl1 |
| AF177211 | 140795 | Gpr105 |
| AF190665 | 56873 | Lmbr1 |
| AF193606 | 20658 | Son |
| AF213381 | 27406 | Abcf3 |
| AF217484 | 57444 | Isg20 |
| AF220205 | 65116 | Prrg2 |
| AF220355 | 56747 | AI843918 |
| AF223953 | 54208 | Arl6ip1 |
| AF228925 | 72088 | Ush1c |
| AF232828 | 18134 | Nova1 |
| AF237628 | 93677 | Lmod2 |

Figure 1-1

| | |
|---|---|
| AF237917 | 109225 |
| AF246218 | 192192 Shkbp1 |
| AF254416 | 64450 Gpr85 |
| AF259674 | 107272 Psat1 |
| AF267660 | 18604 Pdk2 |
| AF276974 | 192201 |
| AF276975 | 192200 Swam2 |
| AF279458 | 19386 Ranbp2 |
| AF281870 | 64009 Syne1 |
| AF285577 | 107815 Scml2 |
| AF285590 | 73679 Tex19 |
| AF290542 | 192198 Lrrc4 |
| AF292939 | 170753 Gig1 |
| AF293079 | 18360 Olfr6 |
| AF297860 | 104776 Aldh6a1 |
| AF317552 | 108062 Cstf2 |
| AF320126 | 14816 Grm1 |
| AF320615 | |
| AF326960 | 21380 Tbx1 |
| AF327857 | 232288 |
| AF332051 | 104112 Acly |
| AF332067 | 75425 |
| AF334612 | 170761 Pdzk2 |
| AF343752 | 77053 Unc84a |
| AF346413 | 75526 Spinlw1 |
| AF369902 | 68291 Mto1 |
| AF375046 | 93762 Smarca5 |
| AF407579 | 140859 Nek8 |
| AF408435 | 233406 Prc1 |
| AF411608 | 76273 Ndfip2 |
| AF411834 | 20620 Snk |
| AJ006215 | 12764 Cmas |
| AJ131821 | 11938 Atp2a2 |
| AJ250690 | |
| AJ276505 | 79202 Tnfrsf22 ? |
| AJ278733 | 17884 Myh4 |
| AJ288061 | 76707 Clasp1 |
| AK002252 | 68323 |
| AK002276 | 71668 |
| AK002297 | 71667 |
| AK002360 | 64697 Keg1 |
| AK002407 | 110826 Etfb |
| AK002416 | 68350 |
| AK002441 | 66039 D14Ertd449e |
| AK002458 | 68339 |
| AK002465 | 71675 |
| AK002481 | 74090 |
| AK002490 | 20195 S100a11 |
| AK002569 | 73242 |
| AK002574 | 68348 |
| AK002637 | 66689 Btbd5 |
| AK002667 | 67899 |
| AK002696 | 28126 D13Wsu177e |
| AK002704 | 68365 Rab14 |
| AK002744 | 68375 Ndufa8 |
| AK002767 | 76509 |

Figure 1-2

| | | |
|---|---|---|
| AK002769 | 71683 | Gypc |
| AK002772 | 66425 | |
| AK002842 | 68386 | |
| AK003123 | 68433 | |
| AK003132 | 75406 | Ndufs7 |
| AK003154 | 68420 | |
| AK003179 | 66101 | D4Wsu43e |
| AK003201 | 68472 | |
| AK003223 | 68563 | Dpm3 |
| AK003232 | 109857 | Cbr3 |
| AK003256 | 74320 | |
| AK003278 | 52466 | D11Ertd18e |
| AK003282 | 66496 | |
| AK003287 | 66264 | |
| AK003337 | 68490 | |
| AK003388 | 76291 | |
| AK003400 | 68566 | Calcyon |
| AK003413 | 68001 | |
| AK003420 | 68567 | Cgref1 |
| AK003511 | 73724 | Mcee |
| AK003524 | 71784 | |
| AK003542 | 73728 | |
| AK003549 | 68499 | Mrpl53 |
| AK003556 | 66966 | Trit1 |
| AK003584 | 81018 | Zfp313 |
| AK003586 | 231712 | |
| AK003605 | 74770 | |
| AK003609 | 76308 | |
| AK003611 | 67070 | |
| AK003623 | 74772 | |
| AK003642 | 68618 | |
| AK003651 | 14000 | Etohi2 |
| AK003653 | 68644 | Dorz1 |
| AK003656 | 74775 | |
| AK003661 | 68520 | |
| AK003676 | 68581 | |
| AK003735 | 52840 | D2Bwg0891e |
| AK003794 | 97541 | Qars |
| AK003808 | 224250 | |
| AK003818 | 66248 | Alg5 |
| AK003819 | 74777 | |
| AK003826 | 68530 | |
| AK003850 | 66151 | |
| AK003863 | 26901 | Deb1 |
| AK003866 | 28295 | D10Jhu81e |
| AK003880 | 68647 | |
| AK003996 | 68668 | |
| AK004007 | 67855 | |
| AK004009 | 54151 | Cyhr1 |
| AK004012 | 68708 | |
| AK004139 | 68735 | |
| AK004323 | 66201 | |
| AK004341 | 68845 | |
| AK004342 | 66478 | |
| AK004431 | 66090 | |
| AK004506 | 68942 | |

Figure 1-3

| | |
|---|---|
| AK004550 | 71707 |
| AK004563 | |
| AK004586 | 66868 |
| AK004617 | 66861 Dnajc10 |
| AK004627 | 74119 |
| AK004632 | 74105 Gga2 |
| AK004650 | 67451 |
| AK004655 | 74107 |
| AK004657 | 74111 |
| AK004664 | 73830 |
| AK004679 | 73340 Nptxr |
| AK004703 | 71733 Susd2 |
| AK004714 | 74133 |
| AK004723 | 74144 Robo4 |
| AK004757 | 71728 |
| AK004778 | 74122 |
| AK004803 | 66875 |
| AK004813 | 71729 Rgs12 |
| AK004913 | 74158 |
| AK004928 | 56275 Rbm14 |
| AK004939 | 71753 Tmprss6 |
| AK004943 | 66627 |
| AK004975 | 71770 Ap2b1 |
| AK005015 | 246277 Csad |
| AK005033 | 52432 Ppp2r2d |
| AK005035 | 71775 |
| AK005044 | 74157 |
| AK005048 | 223776 |
| AK005056 | 66897 |
| AK005063 | 110351 Rap1ga1 |
| AK005069 | 71767 |
| AK005095 | 69757 Leng1 |
| AK005099 | 68970 |
| AK005132 | 72003 Synpr |
| AK005138 | 101867 |
| AK005139 | 68262 Agpat4 |
| AK005159 | 72017 |
| AK005160 | 72014 |
| AK005164 | 76566 |
| AK005166 | 76500 |
| AK005167 | 76686 |
| AK005205 | 67129 |
| AK005251 | 68975 |
| AK005267 | 68953 |
| AK005311 | 66871 Cpne8 |
| AK005345 | 69029 |
| AK005386 | 72008 |
| AK005431 | 67009 |
| AK005479 | 52076 D4Ertd89e |
| AK005519 | 67701 |
| AK005536 | 67883 Uxs1 |
| AK005558 | 76509 |
| AK005562 | 72205 |
| AK005588 | 69317 |
| AK005619 | 72215 |
| AK005661 | 69082 |

Figure 1-4

| | |
|---|---|
| AK005710 | 67283 Slc25a19 |
| AK005730 | 69318 |
| AK005734 | 75470 |
| AK005740 | 74215 |
| AK005756 | 75454 |
| AK005842 | 71840 |
| AK005852 | 71838 Phf7 |
| AK005862 | 74221 |
| AK005864 | 75645 |
| AK005887 | 69354 Slc38a4 |
| AK005888 | 69324 |
| AK005944 | 76920 Arrdc5 |
| AK005989 | 71853 Txndc7 |
| AK006009 | 75553 |
| AK006014 | 69428 |
| AK006023 | 75540 Fpgt |
| AK006079 | 76405 |
| AK006107 | 74229 |
| AK006141 | 75656 |
| AK006145 | 75642 |
| AK006173 | 75629 |
| AK006185 | 70373 |
| AK006222 | 72251 |
| AK006243 | 66091 Ndufa3 |
| AK006263 | 54613 Siat10 |
| AK006273 | 69441 |
| AK006283 | 69435 |
| AK006427 | 69440 |
| AK006467 | 75568 |
| AK006472 | 74239 |
| AK006487 | 75623 |
| AK006490 | 66938 |
| AK006504 | 76380 |
| AK006521 | 99349 |
| AK006525 | 70375 |
| AK006533 | 69519 Rwdd2 |
| AK006539 | 27660 |
| AK006617 | 73284 Ddit4l |
| AK006739 | 22217 Usp12 |
| AK006800 | 74270 Usp20 |
| AK006807 | 74271 |
| AK006837 | 73447 Wdr13 |
| AK006889 | 73410 |
| AK006938 | 73493 |
| AK006959 | 74279 |
| AK006993 | 73486 |
| AK007077 | 73572 |
| AK007097 | 14051 Eya4 |
| AK007130 | 73582 |
| AK007327 | 66144 Atp6v1f |
| AK007339 | 99730 Taf13 |
| AK007351 | 69038 |
| AK007368 | 69062 |
| AK007389 | 107686 Snrpd2 |
| AK007415 | 69046 Hbld2 |
| AK007452 | 69082 |

Figure 1-5

| | |
|---|---|
| AK007485 | 69053 |
| AK007494 | 69064 |
| AK007519 | 69090 Ascc1 |
| AK007546 | 110052 Dek |
| AK007552 | 56036 Ccnl2 |
| AK007598 | 69162 |
| AK007653 | 52443 D4Ertd786e |
| AK007678 | 69185 |
| AK007703 | 27410 Abca3 |
| AK007777 | 83490 Pik3ap1 |
| AK007822 | 107747 Fthfd |
| AK007831 | 68115 |
| AK007857 | 70061 |
| AK007913 | 69833 |
| AK007933 | 72282 |
| AK007941 | 67509 |
| AK007993 | 67490 |
| AK007994 | 72042 Cotl1 |
| AK007999 | 69815 |
| AK008008 | 69824 |
| AK008045 | 76952 Nt5c2 |
| AK008071 | 76769 |
| AK008080 | 76526 |
| AK008083 | 72041 |
| AK008111 | 75599 |
| AK008125 | 228880 |
| AK008151 | 70114 |
| AK008163 | 76523 |
| AK008201 | 69875 Ndufa11 |
| AK008333 | 70257 |
| AK008338 | 69894 |
| AK008415 | 67495 |
| AK008493 | 72098 |
| AK008537 | 67035 |
| AK008574 | 67939 |
| AK008703 | 75669 Pik3r4 |
| AK008724 | 70123 |
| AK008734 | 72349 Dusp3 |
| AK008736 | 70153 |
| AK008749 | 66132 |
| AK008753 | 70387 |
| AK008761 | 74257 Fbxo23 |
| AK008791 | 74245 |
| AK008800 | 70082 |
| AK008803 | 72281 Sh2d4a |
| AK008900 | 75685 |
| AK008988 | 70285 |
| AK008994 | 107358 |
| AK009014 | 69541 |
| AK009074 | 107734 Mrpl30 |
| AK009114 | 71885 |
| AK009123 | 69487 |
| AK009134 | 75572 Acyp2 |
| AK009163 | 76730 |
| AK009180 | 67484 |
| AK009187 | 69482 |

Figure 1-6

| | |
|---|---|
| AK009205 | |
| AK009213 | 75573 |
| AK009243 | 69534 Avpi1 |
| AK009267 | 28019 Ing4 |
| AK009308 | 246257 |
| AK009325 | 79043 Tce1 |
| AK009343 | 71907 Serpina9 |
| AK009344 | 76948 |
| AK009362 | 99650 |
| AK009365 | 76437 D11Bwg0414e |
| AK009375 | 52685 D11Ertd736e |
| AK009402 | 74252 Arcp |
| AK009436 | 67345 |
| AK009447 | 66700 Vps24 |
| AK009450 | 71904 |
| AK009454 | 268721 |
| AK009460 | 66053 Ppil2 |
| AK009487 | 67863 Slc25a11 |
| AK009497 | 69568 Vkorc1l1 |
| AK009584 | 69639 Exosc8 |
| AK009653 | 68544 |
| AK009669 | 72309 |
| AK009759 | 66958 |
| AK009856 | 77039 |
| AK009886 | 71923 |
| AK009912 | 76467 Mrsb |
| AK009937 | 71941 |
| AK009957 | 69692 |
| AK009960 | 69697 |
| AK010038 | 269587 Epb4.1 |
| AK010113 | 70281 |
| AK010147 | 73373 |
| AK010152 | 66556 Drap1 |
| AK010202 | 66968 |
| AK010234 | 50724 |
| AK010262 | 71955 |
| AK010301 | 71973 |
| AK010310 | 67755 Ddx47 |
| AK010312 | 76483 |
| AK010382 | 243983 |
| AK010387 | 69748 |
| AK010396 | 71986 Ddx28 |
| AK010425 | 71957 |
| AK010430 | 69219 Ddah1 |
| AK010433 | 66609 Cryzl1 |
| AK010448 | 71968 |
| AK010452 | 52666 D10Ertd610e |
| AK010453 | 69719 Cad |
| AK010466 | 76484 |
| AK010476 | 71979 |
| AK010511 | 11652 Akt2 |
| AK010552 | 66984 |
| AK010555 | 69746 |
| AK010654 | 72425 |
| AK010752 | 73694 |
| AK010786 | 73710 |

Figure 1-7

| | |
|---|---|
| AK010874 | 69834 |
| AK010890 | 72308 Brf1 |
| AK010898 | 72320 |
| AK010939 | 68196 Hsbp1 |
| AK011034 | 19139 Prps1 |
| AK011126 | 268490 |
| AK011127 | 67031 Upf3a |
| AK011160 | 72117 Mak3p |
| AK011184 | 67039 |
| AK011196 | 67042 |
| AK011230 | 72136 |
| AK011231 | 72068 Cnot2 |
| AK011290 | 67763 |
| AK011354 | 67041 Oxct |
| AK011396 | 234729 D8Wsu151e |
| AK011446 | 70348 |
| AK011457 | 75409 Slitrk5 |
| AK011531 | 67788 |
| AK011541 | 71810 |
| AK011544 | 66254 |
| AK011557 | 69903 |
| AK011565 | 69904 |
| AK011747 | 17237 Mgrn1 |
| AK011781 | 72482 Acbd6 |
| AK011810 | 68263 Pdhb |
| AK011832 | 72465 Zfp131 |
| AK011862 | 28018 D7Wsu128e |
| AK011881 | 67148 |
| AK011950 | 67157 |
| AK011978 | 269003 |
| AK012054 | 72519 |
| AK012087 | 70028 |
| AK012097 | 72155 |
| AK012109 | 72194 |
| AK012134 | 72195 |
| AK012159 | 72542 |
| AK012212 | 107581 Col16a1 |
| AK012224 | 76795 |
| AK012240 | 66399 |
| AK012283 | 72569 |
| AK012292 | 72561 |
| AK012304 | 72612 |
| AK012326 | 52323 D5Ertd363e |
| AK012392 | 76816 Sdccag8 |
| AK012475 | 330203 |
| AK012503 | 72563 |
| AK012535 | 101513 AI256456 |
| AK012553 | 77015 |
| AK012601 | 67622 |
| AK012610 | 72654 |
| AK012624 | 72621 |
| AK012635 | 69920 Polr2i |
| AK012711 | 69226 Snx24 |
| AK012733 | 76366 |
| AK012765 | 69938 Scrn1 |
| AK012842 | 72686 |

Figure 1-8

| | |
|---|---|
| AK012847 | 72674 |
| AK012848 | 72701 |
| AK012885 | 67212 Mrpl55 |
| AK012922 | 72681 |
| AK012969 | 74200 |
| AK012971 | 74201 |
| AK013003 | 67610 |
| AK013012 | 72184 |
| AK013035 | 20621 Snn |
| AK013044 | 106200 |
| AK013055 | 67059 |
| AK013135 | 75620 |
| AK013145 | 72174 |
| AK013166 | 67607 |
| AK013175 | 28027 D4Wsu132e |
| AK013202 | 20467 Sin3b |
| AK013245 | 72748 |
| AK013267 | 72747 |
| AK013269 | 72759 |
| AK013280 | 67229 |
| AK013309 | 170707 Usp31 |
| AK013367 | 72792 |
| AK013393 | 72843 Prdm4 |
| AK013402 | 109785 Pgm3 |
| AK013427 | 104884 |
| AK013464 | 67515 |
| AK013511 | 72900 Ndufv2 |
| AK013595 | 72871 |
| AK013604 | 72880 |
| AK013609 | 72881 Zdhhc4 |
| AK013632 | 72894 |
| AK013727 | 14397 Gabra4 |
| AK013800 | 73053 |
| AK013837 | 73090 |
| AK013856 | 23938 Map2k5 |
| AK013872 | 72397 |
| AK013880 | 70223 Nars |
| AK013924 | 12301 Cacybp |
| AK013927 | 73099 |
| AK013948 | 69269 Scnm1 |
| AK013995 | 73078 |
| AK014029 | 73068 Fut11 |
| AK014134 | 73172 |
| AK014142 | 67287 |
| AK014178 | 56738 Mocs1 |
| AK014230 | 216877 Dhx33 |
| AK014235 | 73192 Xpot |
| AK014285 | 76965 |
| AK014338 | 74840 Armet |
| AK014408 | 74315 |
| AK014487 | 257635 Sdsl |
| AK014579 | 74347 |
| AK014582 | 70807 Arrdc2 |
| AK014623 | 66701 |
| AK014680 | 74617 |
| AK014709 | 74585 4833416I09Rik |

Figure 1-9

| | |
|---|---|
| AK014752 | 75768 |
| AK014763 | 75769 |
| AK014772 | 74592 |
| AK014834 | 75778 |
| AK014863 | 70853 |
| AK014891 | 14479 Usp15 |
| AK014942 | 76856 |
| AK014948 | 66719 |
| AK014993 | 70961 |
| AK015007 | 77048 |
| AK015063 | 75773 |
| AK015086 | 73809 |
| AK015106 | 106039 Gga1 |
| AK015275 | 73945 D8Ertd69e |
| AK015309 | 75792 |
| AK015402 | 74862 |
| AK015452 | 75796 Cdyl2 |
| AK015555 | 74919 |
| AK015642 | 75036 |
| AK015650 | 67509 |
| AK015766 | 75083 |
| AK015803 | 225980 |
| AK015818 | 75075 |
| AK015898 | 75141 Rasd2 |
| AK015916 | 73866 |
| AK015921 | 75209 Sv2c |
| AK015947 | 68195 Rnaset2 |
| AK015971 | 75829 |
| AK015972 | 75137 |
| AK015988 | 75146 |
| AK016000 | 56428 Mtch2 |
| AK016018 | 75163 |
| AK016037 | 75212 Rnf121 |
| AK016128 | 75359 |
| AK016162 | |
| AK016214 | 75275 |
| AK016247 | 18640 Pfkfb2 |
| AK016267 | 65103 Arl6ip6 |
| AK016282 | 75863 |
| AK016299 | 75894 |
| AK016352 | 66722 |
| AK016374 | 75879 |
| AK016385 | 52348 D8Ertd531e |
| AK016397 | 66362 |
| AK016466 | 70965 |
| AK016475 | 70981 |
| AK016480 | |
| AK016509 | 71003 |
| AK016517 | 75725 |
| AK016520 | 71745 Cul2 |
| AK016542 | 74386 |
| AK016563 | 51885 D2Ertd435e |
| AK016572 | 74411 |
| AK016584 | 72230 |
| AK016622 | 74438 |
| AK016671 | 74412 Gle1l |

Figure 1-10

58

| | |
|---|---|
| AK016685 | 74443 |
| AK016704 | 74434 |
| AK016786 | 71804 |
| AK016812 | 74085 |
| AK016865 | 71164 Zdhhc20 |
| AK016873 | 71192 |
| AK016890 | 71099 |
| AK016916 | 109242 Kif24 |
| AK016990 | 74481 |
| AK017020 | 71241 Dmrtc2 |
| AK017044 | 71304 |
| AK017081 | 74480 Samd4 |
| AK017095 | 71240 Osbpl7 |
| AK017138 | 74471 |
| AK017149 | 75964 |
| AK017158 | 75965 |
| AK017214 | 71323 |
| AK017226 | 75734 |
| AK017249 | 11842 Arf3 |
| AK017256 | 71326 Treml1 |
| AK017270 | 237943 |
| AK017282 | 71373 |
| AK017309 | 71382 Pex1 |
| AK017464 | 75747 Sesn3 |
| AK017484 | 67023 |
| AK017491 | 70478 Mipep |
| AK017528 | 69227 |
| AK017530 | 70502 |
| AK017531 | 73998 Herc3 |
| AK017550 | 59032 |
| AK017577 | 74741 |
| AK017628 | 70556 |
| AK017676 | 70594 |
| AK017726 | 70625 Crsp7 |
| AK017736 | 70616 Sf4 |
| AK017750 | 70623 |
| AK017761 | 70619 |
| AK017769 | 70614 |
| AK017801 | 68021 |
| AK017808 | 70652 |
| AK017848 | 74329 |
| AK017929 | 76007 Zfp198 |
| AK018027 | 277250 |
| AK018060 | 76131 |
| AK018089 | 76137 |
| AK018115 | 74007 Btbd11 |
| AK018132 | 74008 |
| AK018155 | 70750 |
| AK018159 | 18549 Pcsk2 |
| AK018222 | 76161 |
| AK018327 | 76206 |
| AK018331 | 76205 Stard3nl |
| AK018362 | 71468 Obox1 |
| AK018495 | 74536 |
| AK018541 | 71564 |
| AK018551 | 107022 |

Figure 1-11

| | |
|---|---|
| AK018560 | 67556 Pigm |
| AK018561 | 71546 |
| AK018585 | 74558 |
| AK018594 | 68145 |
| AK018599 | 22353 Vip |
| AK018615 | 71597 |
| AK018680 | 66251 Arfgap3 |
| AK018731 | 68193 Rpl24 |
| AK018937 | 78636 |
| AK018953 | 68214 |
| AK019059 | 59040 Arht1 |
| AK019147 | 19084 Prkar1a |
| AK019168 | 68991 |
| AK019176 | 93687 Csnk1a1 |
| AK019216 | 78832 |
| AK019434 | 28040 D6Wsu163e |
| AK019462 | 20643 Snrpe |
| AK019479 | |
| AK019481 | 68260 |
| AK019514 | 78104 |
| AK019527 | 78903 Wrnip1 |
| AK019531 | 78902 |
| AK019538 | 78092 |
| AK019547 | |
| AK019590 | 78154 |
| AK019600 | 78795 |
| AK019605 | 78799 |
| AK019621 | 78796 |
| AK019664 | 78801 |
| AK019665 | 78174 |
| AK019744 | 78931 |
| AK019803 | 78217 |
| AK019863 | 77634 |
| AK019886 | 68151 |
| AK019929 | 78283 |
| AK019938 | 321015 |
| AK019948 | 78095 |
| AK019985 | 77521 |
| AK020024 | 106504 Stk38 |
| AK020090 | 68176 |
| AK020099 | 68180 |
| AK020225 | 77506 |
| AK020284 | 77669 |
| AK020352 | 78242 |
| AK020377 | 67791 |
| AK020410 | 52120 D8Ertd354e |
| AK020441 | 77272 |
| AK020471 | 12631 Cfl1 |
| AK020492 | 77305 |
| AK020608 | 78425 |
| AK020613 | 78672 |
| AK020653 | 78558 |
| AK020687 | 78083 |
| AK020700 | 77763 |
| AK020702 | 78428 |
| AK020724 | 77578 Bcl9 |

Figure 1-12

| | | |
|---|---|---|
| AK020727 | 77754 | |
| AK020780 | 77929 | |
| AK020831 | 77794 | |
| AK020837 | 77799 | Sla2 |
| AK020859 | 68146 | |
| AK020868 | 68073 | |
| AK021006 | 110920 | Stch |
| AK021023 | 78688 | Nol3 |
| AK021033 | 77219 | |
| AK021066 | 77318 | |
| AK021099 | 77462 | |
| AK021152 | 77643 | |
| AK021166 | 77609 | |
| AK021190 | 77644 | |
| AK021200 | 13057 | Cyba |
| AK021301 | 78578 | |
| AK021330 | 77996 | |
| AK021336 | 77994 | |
| AK021396 | 78550 | |
| AK027262 | 235402 | Lrrn6a |
| AY007717 | 70603 | Mutyh |
| AY026384 | 192655 | Cacna1i |
| BC002157 | 100273 | Osbpl9 |
| BC002178 | 93747 | Echs1 |
| BC002191 | 108902 | |
| BC002193 | 110279 | Bcr |
| BC002199 | 211556 | BC002199 |
| BC002230 | 217827 | BC002230 |
| BC002240 | 66310 | |
| BC002274 | 69216 | |
| BC002298 | 19283 | Ptprz1 |
| BC002302 | 106583 | Rbm16 |
| BC002306 | 245841 | Polr2h |
| BC002307 | 27878 | D1Ertd251e |
| BC003235 | 71665 | Fuca |
| BC003237 | 98985 | AI462438 |
| BC003247 | 229725 | Mclc |
| BC003255 | 228355 | Madd |
| BC003284 | 73828 | Wdr21 |
| BC003288 | 28088 | D10Wsu52e |
| BC003335 | 106344 | Rfc4 |
| BC003451 | 232087 | Mat2a |
| BC003457 | 108645 | |
| BC003842 | 97112 | C87860 |
| BC003847 | 77125 | |
| BC003914 | 58875 | |
| BC003922 | 52615 | D11Ertd530e |
| BC003932 | 67278 | |
| BC003959 | 286940 | Flnb |
| BC003975 | 30853 | Mlf2 |
| BC003986 | | |
| BC004000 | 170791 | Rnpc2 |
| BC004012 | 192185 | BC004012 |
| BC004062 | 234959 | Crsp6 |
| BC004064 | 20682 | Sox9 |
| BC004098 | 192287 | |

Figure 1-13

| | |
|---|---|
| BC004600 | 75221 Dpp3 |
| BC004604 | 104318 Csnk1d |
| BC004626 | 72722 |
| BC004630 | 216549 |
| BC004635 | 102607 Snx19 |
| BC004648 | 74737 |
| BC004653 | 67155 |
| BC004680 | 13052 Cxadr |
| BC004739 | 213211 Rnf26 |
| BC004754 | 21351 Taldo1 |
| BC004759 | 171095 Il17rc |
| BC004761 | 233066 AI428936 |
| BC004764 | 56690 Mlycd |
| BC004766 | 71799 Ptcd1 |
| BC004768 | |
| BC004811 | 224742 Abcf1 |
| BC004826 | 57278 Lu |
| BC005459 | 102866 Pls3 |
| BC005485 | 99543 Olfml3 |
| BC005494 | 216157 ORF61 |
| BC005515 | 100090 Hkr3 |
| BC005517 | 71704 Arhgef3 |
| BC005518 | 76867 |
| BC005574 | 67976 |
| BC005598 | 98221 Ga17 |
| BC005600 | 70231 Gorasp2 |
| BC005603 | 68776 Taf11 |
| BC005625 | 217038 |
| BC005658 | 69957 Cdc16 |
| BC005674 | 142682 AA792890 |
| BC005741 | 233835 Tnrc6 |
| BC005744 | 72144 Slc37a3 |
| BC005755 | 70465 |
| BC005774 | |
| BC005788 | 71994 Cnn3 |
| BC006063 | 75570 |
| BC006805 | 72692 |
| BC006905 | 75608 |
| BC008101 | 68939 |
| BC009123 | 72704 |
| BC009141 | 75062 Sf3a3 |
| BC011091 | 109815 H47 |
| BC011482 | 23943 Mbc2 |
| BC012407 | 110695 Aldh7a1 |
| BC012512 | 67398 Srpr |
| BC012878 | 77034 |
| BC013471 | 77056 |
| BC013555 | 70650 Zcchc8 |
| BC013716 | 15499 Hsf1 |
| BC013800 | 67063 |
| BC014687 | 68964 |
| BC014769 | 73166 Tm7sf2 |
| BC015253 | 11688 Alox15b |
| BC015273 | 110157 Raf1 |
| BC016073 | 68916 |
| BC016080 | 103963 Rpn1 |

Figure 1-14

| | | |
|---|---|---|
| BC016210 | . | 67017 |
| BC016258 | | 71732 Vps11 |
| BC016444 | | 75273 |
| BC017130 | | 224938 Pja2 |
| BC017138 | | 109168 |
| BC017532 | | 105501 Abhd4 |
| BC017606 | | 72605 Car10 |
| BC017615 | | 94249 Slc24a3 |
| BC017625 | | 68047 |
| BC017627 | . | 66995 |
| BC017636 | | 110796 Sdccag33 |
| BC018162 | | 73847 |
| BC018329 | | 70380 |
| BC018468 | | 66753 |
| BC018470 | | 23960 Oas1g |
| D13903 | | 19266 Ptprd |
| D29797 | | 20908 Stx3 |
| D50366 | | 16579 Kifap3 |
| D86949 | | 18845 Plxna2 |
| J05277 | | 15275 Hk1 |
| L17069 | | 214162 Mll |
| L17305 | | |
| L20343 | | 12296 Cacnb2 |
| L21027 | | 236539 Phgdh |
| L32178 | | 232333 Slc6a1 |
| L34676 | | 11784 Apba2 |
| L38281 | | 16365 Irg1 |
| M12289 | | 17885 Myh8 |
| M34173 | | 12946 Crry |
| M36514 | | 22688 Zfp26 |
| M36654 | | 15412 Hoxb4 |
| M63245 | | 11655 Alas1 |
| M69293 | | 15902 Idb2 |
| NM_007393 | . | 11461 Actb |
| NM_007399 | | 11487 Adam10 |
| NM_007404 | | 11502 Adam9 |
| NM_007408 | | 11520 Adfp |
| NM_007427 | | 11604 Agrp |
| NM_007429 | | 11609 Agtr2 |
| NM_007457 | | 11769 Ap1s1 |
| NM_007458 | | 11771 Ap2a1 |
| NM_007460 | | 11776 Ap3d |
| NM_007462 | | 11789 Apc |
| NM_007471 | | 11820 App |
| NM_007474 | | 11833 Aqp8 |
| NM_007495 | | 11899 Astn1 |
| NM_007498 | | 11910 Atf3 |
| NM_007508 | | 11964 Atp6v1a1 |
| NM_007524 | | 12020 Bapx1 |
| NM_007525 | | 12021 Bard1 |
| NM_007527 | | 12028 Bax |
| NM_007529 | | 12032 Bcan |
| NM_007538 | | 12057 Opn1sw |
| NM_007542 | | 12111 Bgn |
| NM_007555 | | 12160 Bmp5 |
| NM_007557 | | 12162 Bmp7 |

Figure 1-15

| | |
|---|---|
| NM_007567 | 12217 Bsn |
| NM_007568 | 12223 Btc |
| NM_007574 | 12262 C1qg |
| NM_007583 | 12300 Cacng2 |
| NM_007591 | 12317 Calr |
| NM_007607 | 12351 Car4 |
| NM_007610 | 12366 Casp2 |
| NM_007614 | 12387 Catnb |
| NM_007620 | 12408 Cbr1 |
| NM_007625 | 12418 Cbx4 |
| NM_007627 | 12426 Cckbr |
| NM_007628 | 12427 Ccna1 |
| NM_007633 | 12447 Ccne1 |
| NM_007637 | 12465 Cct5 |
| NM_007638 | 12468 Cct7 |
| NM_007647 | 12499 Entpd5 |
| NM_007667 | 12564 Cdh8 |
| NM_007682 | 12616 Cenpb |
| NM_007686 | 12630 Cfi . |
| NM_007688 | 12632 Cfl2 |
| NM_007689 | 12643 Chad |
| NM_007699 | 12672 Chrm4 |
| NM_007701 | 12677 Chx10 |
| NM_007702 | 12683 Cidea |
| NM_007703 | 12686 Elovl3 |
| NM_007731 | 12817 Col13a1 |
| NM_007733 | 12823 Col19a1 |
| NM_007743 | 12843 Col1a2 |
| NM_007748 | 12861 Cox6a1 |
| NM_007761 | 12909 Crcp |
| NM_007772 | 110521 Hivep1 |
| NM_007791 | 13007 Csrp1 |
| NM_007795 | 13019 Ctf1 |
| NM_007804 | 13048 Cutl2 |
| NM_007833 | 13179 Dcn |
| NM_007836 | 13197 Gadd45a |
| NM_007863 | 13384 Mpp3 |
| NM_007871 | 13430 Dnm2 |
| NM_007874 | 13476 Dp1 |
| NM_007889 | 13544 Dvl3 |
| NM_007897 | 13591 Ebf1 |
| NM_007900 | 13605 Ect2 |
| NM_007917 | 13684 Eif4e |
| NM_007922 | 13712 Elk1 |
| NM_007930 | 13803 Enc1 |
| NM_007931 | 13804 Endog |
| NM_007936 | 13838 Epha4 |
| NM_007945 | 13860 Eps8 |
| NM_007948 | 13870 Ercc1 |
| NM_007950 | 13874 Ereg |
| NM_007966 | 14028 Evx1 |
| NM_007983 | 14084 Faf1 |
| NM_007995 | 14133 Fcna |
| NM_007997 | 14149 Fdxr |
| NM_008011 | 14186 Fgfr4 |
| NM_008016 | 14210 Fin15 |

Figure 1-16

| | |
|---|---|
| NM_008019 | 14225 Fkbp1a |
| NM_008020 | 14227 Fkbp2 |
| NM_008030 | 14262 Fmo3 |
| NM_008040 | 14290 Fpr-rs3 |
| NM_008060 | 14376 G2an |
| NM_008064 | 14387 Gaa |
| NM_008066 | 14395 Gabra2 |
| NM_008078 | 14417 Gad2 |
| NM_008082 | 14427 Galr1 |
| NM_008083 | 14432 Gap43 |
| NM_008085 | 14447 Gapds |
| NM_008095 | 14467 Gbas |
| NM_008098 | 14489 Mtpn |
| NM_008106 | 14539 Opn1mw |
| NM_008120 | 14612 Gja4 |
| NM_008121 | 14613 Gja5 |
| NM_008125 | 14619 Gjb2 |
| NM_008126 | 14620 Gjb3 |
| NM_008128 | 14623 Gjb6 |
| NM_008129 | 14630 Gclm |
| NM_008133 | 14661 Glud |
| NM_008135 | 14664 Slc6a9 |
| NM_008138 | 14678 Gnai2 |
| NM_008142 | 14688 Gnb1 |
| NM_008149 | 14732 Gpam |
| NM_008158 | 14761 Gpr27 |
| NM_008161 | 14778 Gpx3 |
| NM_008167 | 14804 Grid2 |
| NM_008171 | 14812 Grin2b |
| NM_008172 | 14814 Grin2d |
| NM_008187 | 14894 Gtl3 |
| NM_008222 | 15159 Hccs |
| NM_008229 | 15182 Hdac2 |
| NM_008230 | 15186 Hdc |
| NM_008235 | 15205 Hes1 |
| NM_008249 | 15278 Tfb2m |
| NM_008254 | 15356 Hmgcl |
| NM_008285 | 15465 Hrh1 |
| NM_008288 | 15483 Hsd11b1 |
| NM_008292 | 15488 Hsd17b4 |
| NM_008298 | 15502 Dnaja1 |
| NM_008300 | 15525 Hspa4 |
| NM_008317 | 15586 Hyal1 |
| NM_008323 | 15929 Idh3g |
| NM_008325 | 15932 Idua |
| NM_008329 | 15951 Ifi16 |
| NM_008339 | 15985 Cd79b |
| NM_008357 | 16168 Il15 |
| NM_008360 | 16173 Il18 |
| NM_008372 | 16197 Il7r |
| NM_008373 | 16198 Il9 |
| NM_008374 | 16199 Il9r |
| NM_008376 | 16205 Imap38 |
| NM_008377 | 16206 Lrig1 |
| NM_008382 | 16326 Inhbe |
| NM_008399 | 16407 Itgae |

Figure 1-17

| | |
|---|---|
| NM_008401 | 16409 Itgam |
| NM_008405 | 16415 Itgb2l |
| NM_008420 | 16500 Kcnb1 |
| NM_008421 | 16502 Kcnc1 |
| NM_008424 | 16509 Kcne1 |
| NM_008442 | 16563 Kif2a |
| NM_008443 | 16568 Kif3a |
| NM_008451 | 16594 Klc2 |
| NM_008453 | 16599 Klf3 |
| NM_008468 | 16650 Kpna6 |
| NM_008480 | 16772 Lama1 |
| NM_008488 | 16801 Arhgef1 |
| NM_008492 | 16832 Ldh2 |
| NM_008497 | 16866 Lhb |
| NM_008502 | 16897 Llglh |
| NM_008535 | 17095 Lyl1 |
| NM_008537 | 17117 Amacr |
| NM_008538 | 17118 Marcks |
| NM_008560 | 17200 Mc2r |
| NM_008571 | 17225 Mcpt2 |
| NM_008574 | 17235 Mcsp |
| NM_008585 | 17287 Mep1a |
| NM_008589 | 17293 Mesp2 |
| NM_008591 | 17295 Met |
| NM_008596 | 17306 Mg29 |
| NM_008602 | 17344 Miz1 |
| NM_008604 | 17380 Mme |
| NM_008617 | 17448 Mdh2 |
| NM_008638 | 17768 Mthfd2 |
| NM_008642 | 17777 Mttp |
| NM_008663 | 17921 Myo7a |
| NM_008676 | 17966 Nbr1 |
| NM_008683 | 18002 Nedd8 |
| NM_008702 | 18099 Nlk |
| NM_008703 | 18101 Nmbr |
| NM_008707 | 18107 Nmt1 |
| NM_008708 | 18108 Nmt2 |
| NM_008722 | 18148 Npm1 |
| NM_008729 | 18163 Catnd2 |
| NM_008731 | 18167 Npy2r |
| NM_008732 | 18174 Slc11a2 |
| NM_008734 | 18183 Nrg3 |
| NM_008737 | 18186 Nrp |
| NM_008738 | 18188 Nrtn |
| NM_008742 | 18205 Ntf3 |
| NM_008756 | 18260 Ocln |
| NM_008770 | 18417 Cldn11 |
| NM_008791 | 18546 Pcp4 |
| NM_008797 | 18563 Pcx |
| NM_008799 | 18567 Pdcd2 |
| NM_008809 | 18596 Pdgfrb |
| NM_008812 | 18600 Padi2 |
| NM_008821 | 18630 Pet2 |
| NM_008825 | 18640 Pfkfb2 |
| NM_008839 | 18706 Pik3ca |
| NM_008841 | 18709 Pik3r2 |

Figure 1-18

| | |
|---|---|
| NM_008845 | 18718 Pip5k2a |
| NM_008847 | 18720 Pip5k1b |
| NM_008852 | 18742 Pitx3 |
| NM_008855 | 18751 Prkcb |
| NM_008857 | 18759 Prkcl |
| NM_008866 | 18777 Lypla1 |
| NM_008877 | 18815 Plg |
| NM_008883 | 18846 Plxna3 |
| NM_008885 | 18858 Pmp22 |
| NM_008889 | 18938 Ppp1r14b |
| NM_008890 | 18948 Pnmt |
| NM_008902 | 19011 Pp11r |
| NM_008903 | 19012 Ppap2a |
| NM_008923 | 19085 Prkar1b |
| NM_008925 | 19089 Prkcsh |
| NM_008929 | 19107 Dnajc3 |
| NM_008937 | 19130 Prox1 |
| NM_008939 | 19142 Prss12 |
| NM_008946 | 19175 Psmb6 |
| NM_008947 | 19179 Psmc1 |
| NM_008955 | 19202 Psx1 |
| NM_008956 | 19205 Ptbp1 |
| NM_008957 | 19206 Ptch |
| NM_008958 | 19207 Ptch2 |
| NM_008969 | 19224 Ptgs1 |
| NM_008971 | 19230 Ptk9 |
| NM_008972 | 19231 Ptma |
| NM_008974 | 19244 Ptp4a2 |
| NM_008983 | 19272 Ptprk |
| NM_008984 | 19274 Ptprm |
| NM_008995 | 19305 Pex5 |
| NM_008997 | 19326 Rab11b |
| NM_008998 | 19329 Rab17 |
| NM_009015 | 19366 Rad54l |
| NM_009020 | 19374 Rag2 |
| NM_009030 | 19646 Rbbp4 |
| NM_009035 | 19664 Rbpsuh |
| NM_009041 | 19684 Rdx |
| NM_009046 | 19698 Relb |
| NM_009047 | 19700 Rem |
| NM_009063 | 19737 Rgs5 |
| NM_009068 | 19766 Ripk1 |
| NM_009069 | 19769 Rit1 |
| NM_009070 | 19826 Rnps1 |
| NM_009072 | 19878 Rock2 |
| NM_009079 | 19934 Rpl22 |
| NM_009084 | 19981 Rpl37a |
| NM_009086 | 20017 Rpo1-2 |
| NM_009092 | 20068 Rps17 |
| NM_009100 | 20129 Rptn |
| NM_009128 | 20250 Scd2 |
| NM_009129 | 20254 Scg2 |
| NM_009131 | 20256 Scgf |
| NM_009147 | 20334 Sec23a |
| NM_009148 | 20336 Sec8 |
| NM_009153 | 20347 Sema3b |

Figure 1-19

| | |
|---|---|
| NM_009155 | 20363 Sepp1 |
| NM_009166 | 20411 Sorbs1 |
| NM_009167 | 20418 Shc3 |
| NM_009171 | 20425 Shmt1 |
| NM_009186 | 20462 Silg41 |
| NM_009197 | 20502 Slc16a2 |
| NM_009213 | 20598 Smpd2 |
| NM_009215 | 20604 Sst |
| NM_009228 | 20648 Snta1 |
| NM_009237 | 20675 Sox3 |
| NM_009257 | 20724 Serpinb5 |
| NM_009262 | 20745 Spock1 |
| NM_009278 | 20823 Ssb |
| NM_009282 | 20842 Stag1 |
| NM_009293 | 20905 Sts |
| NM_009314 | 21337 Tacr2 |
| NM_009320 | 21366 Slc6a6 |
| NM_009325 | 21390 Tbxa2r |
| NM_009331 | 21414 Tcf7 |
| NM_009352 | 21749 Terf1 |
| NM_009369 | 21810 Tgfbi |
| NM_009377 | 21823 Th |
| NM_009390 | 21892 Tll |
| NM_009398 | 21930 Tnfaip6 |
| NM_009404 | 21950 Tnfsf9 |
| NM_009417 | 22018 Tpo |
| NM_009420 | 22024 Crisp2 |
| NM_009421 | 22029 Traf1 |
| NM_009425 | 22035 Tnfsf10 |
| NM_009426 | 22044 Trh |
| NM_009427 | 22057 Tob1 |
| NM_009437 | 22117 Tst |
| NM_009440 | 22127 Tsx |
| NM_009441 | 22129 Ttc3 |
| NM_009465 | 26362 Axl |
| NM_009476 | 22269 Upk2 |
| NM_009478 | 22275 Urod |
| NM_009481 | 22284 Usp9x |
| NM_009482 | 22286 Utf1 |
| NM_009503 | 269523 Vcp |
| NM_009536 | 22627 Ywhae |
| NM_009537 | 22632 Yy1 |
| NM_009551 | 22682 Zfp216 |
| NM_009555 | 22700 Zfp40 |
| NM_009557 | 22704 Zfp46 |
| NM_009579 | 22782 Slc30a1 |
| NM_009584 | 22792 Zrf2 |
| NM_009614 | 11490 Adam15 |
| NM_009624 | 11515 Adcy9 |
| NM_009632 | 11546 Adprtl2 |
| NM_009634 | 11564 Adsl |
| NM_009647 | 11639 Ak4 |
| NM_009652 | 11651 Akt1 |
| NM_009660 | 11687 Alox15 |
| NM_009671 | 11736 Ankfy1 |
| NM_009679 | 11773 Ap2m1 |

Figure 1-20

| | |
|---|---|
| NM_009680 | 11774 Ap3b1 |
| NM_009688 | 11798 Birc4 |
| NM_009717 | 11922 Neurod6 |
| NM_009727 | 11980 Atp8a1 |
| NM_009729 | 11984 Atp6v0c |
| NM_009730 | 11990 Atrn |
| NM_009738 | 12038 Bche |
| NM_009746 | 12055 Bcl7c |
| NM_009748 | 12068 Bet1 |
| NM_009779 | 12267 C3ar1 |
| NM_009781 | 12288 Cacna1c |
| NM_009788 | 12307 Calb1 |
| NM_009819 | 12386 Catna2 |
| NM_009852 | 12511 Cd6 |
| NM_009854 | 12516 Cd7 |
| NM_009860 | 12532 Cdc25c |
| NM_009861 | 12540 Cdc42 |
| NM_009864 | 12550 Cdh1 |
| NM_009873 | 12571 Cdk6 |
| NM_009883 | 12608 Cebpb |
| NM_009895 | 12700 Cish |
| NM_009920 | 12794 Cnil |
| NM_009964 | 12955 Cryab |
| NM_009974 | 13000 Csnk2a2 |
| NM_009983 | 13033 Ctsd |
| NM_010010 | 13116 Cyp46a1 |
| NM_010064 | 13427 Dncic2 |
| NM_010133 | 13798 En1 |
| NM_010149 | 13857 Epor |
| NM_010157 | 13983 Esr2 |
| NM_010160 | 14007 Cugbp2 |
| NM_010181 | 14119 Fbn2 |
| NM_010200 | 14168 Fgf13 |
| NM_010234 | 14281 Fos |
| NM_010242 | 14345 Fut4 |
| NM_010250 | 14394 Gabra1 |
| NM_010255 | 14431 Gamt |
| NM_010259 | 14468 Gbp1 |
| NM_010264 | 14536 Nr6a1 |
| NM_010274 | 14571 Gpd2 |
| NM_010278 | 14581 Gfi1 |
| NM_010282 | 14593 Ggps1 |
| NM_010309 | 14683 Gnas |
| NM_010313 | 14697 Gnb5 |
| NM_010321 | 14711 Gnmt |
| NM_010347 | 14797 Aes |
| NM_010352 | 14840 Gsg1 |
| NM_010362 | 14873 Gsto1 |
| NM_010377 | 107970 Hist1h1t |
| NM_010389 | 15002 H2-Ob |
| NM_010412 | 15184 Hdac5 |
| NM_010431 | 15251 Hif1a |
| NM_010451 | 15399 Hoxa2 |
| NM_010471 | 15444 Hpca |
| NM_010473 | 15464 Hrc |
| NM_010476 | 15490 Hsd17b7 |

Figure 1-21

69

| NM_010500 | 15939 Ier5 | |
| NM_010590 | 16475 Jub | |
| NM_010595 | 16485 Kcna1 | |
| NM_010599 | 16499 Kcnab3 | |
| NM_010607 | 16526 Kcnk2 | |
| NM_010661 | 268482 Krt1-12 | |
| NM_010710 | 16870 Lhx2 | |
| NM_010727 | 16924 Lnx1 | |
| NM_010765 | 17165 Mapkapk5 | |
| NM_010769 | 17180 Matn1 | |
| NM_010770 | 17182 Matn3 | |
| NM_010771 | 17184 Matr3 | |
| NM_010781 | 17229 Mcpt6 | |
| NM_010799 | 17330 Minpp1 | |
| NM_010817 | 17463 Psmd7 | |
| NM_010826 | 17540 Mrvi1 | |
| NM_010837 | 17760 Mtap6 | |
| NM_010841 | 17771 Mtl5 | |
| NM_010879 | 17974 Nck2 | |
| NM_010883 | 17986 Ndph | |
| NM_010891 | 18000 | Sep-02 |
| NM_010911 | 18041 Nfs1 | |
| NM_010942 | 18196 Nsg1 | |
| NM_010946 | 18203 Ntan1 | |
| NM_010991 | 18348 Olfr49 | |
| NM_011010 | 18378 Omp | |
| NM_011012 | 18389 Oprl | |
| NM_011018 | 18412 Sqstm1 | |
| NM_011025 | 18429 Oxt | |
| NM_011040 | 18510 Pax8 | |
| NM_011042 | 18521 Pcbp2 | |
| NM_011049 | 18555 Pctk1 | |
| NM_011069 | 18632 Pex11b | |
| NM_011072 | 18643 Pfn1 | |
| NM_011102 | 18752 Prkcc | |
| NM_011104 | 18754 Prkce | |
| NM_011109 | 18782 Pla2g2d | |
| NM_011116 | 18807 Pld3 | |
| NM_011156 | 19072 Prep | |
| NM_011165 | 19110 Prlpa | |
| NM_011176 | 19143 St14 | |
| NM_011196 | 19218 Ptger3 | |
| NM_011197 | 19221 Ptgfrn | |
| NM_011200 | 19243 Ptp4a1 | |
| NM_011201 | 19246 Ptpn1 | |
| NM_011202 | 19247 Ptpn11 | |
| NM_011203 | 19248 Ptpn12 | |
| NM_011219 | | |
| NM_011231 | 19352 Rabggtb | |
| NM_011242 | 19395 Rasgrp2 | |
| NM_011244 | 19411 Rarg | |
| NM_011246 | 19419 Rasgrp1 | |
| NM_011251 | 19654 Rbm6 | |
| NM_011287 | 19896 Rpl10a | |
| NM_011288 | 19935 Mrpl23 | |
| NM_011292 | 20005 Rpl9 | |

Figure 1-22

70

| | |
|---|---|
| NM_011293 | 20022 Polr2j |
| NM_011305 | 20181 Rxra |
| NM_011310 | 20197 S100a3 |
| NM_011312 | 20199 S100a5 |
| NM_011328 | 20287 Sct |
| NM_011341 | 20318 Sdf4 |
| NM_011343 | 20335 Sec61g |
| NM_011352 | 20361 Sema7a |
| NM_011356 | 20378 Frzb |
| NM_011360 | 20392 Sgce |
| NM_011389 | 20495 Slc12a1 |
| NM_011421 | 20597 Smpd1 |
| NM_011423 | 20601 Smr3 |
| NM_011427 | 20613 Snai1 |
| NM_011428 | 20614 Snap25 |
| NM_011443 | 20674 Sox2 |
| NM_011444 | 20678 Sox5 |
| NM_011478 | 20766 Sprr3 |
| NM_011492 | 20869 Stk11 |
| NM_011500 | 268980 Strn |
| NM_011503 | 20911 Stxbp2 |
| NM_011512 | 20932 Surf4 |
| NM_011522 | 20974 Syngr3 |
| NM_011529 | 21353 Tank |
| NM_011539 | 21391 Tbxas1 |
| NM_011541 | 21399 Tcea1 |
| NM_011543 | 21402 Skp1a |
| NM_011550 | 21428 Tcfl4 |
| NM_011552 | 21453 Tcof1 |
| NM_011577 | 21803 Tgfb1 |
| NM_011585 | 21841 Tia1 |
| NM_011588 | 21849 Trim28 |
| NM_011595 | 21859 Timp3 |
| NM_011597 | 21873 Tjp2 |
| NM_011601 | 21893 Tlm |
| NM_011607 | 21923 Tnc |
| NM_011617 | 21948 Tnfsf7 |
| NM_011622 | 21968 Tom1 |
| NM_011623 | 21973 Top2a |
| NM_011626 | 21982 Tparl |
| NM_011638 | 22042 Trfr |
| NM_011656 | 22156 Tuft1 |
| NM_011660 | 22166 Txn1 |
| NM_011665 | 22196 Ube2i |
| NM_011670 | 22223 Uchl1 |
| NM_011673 | 22234 Ugcg |
| NM_011674 | 22239 Ugt8 |
| NM_011705 | 22367 Vrk1 |
| NM_011714 | 22385 Baz1b |
| NM_011740 | 22631 Ywhaz |
| NM_011772 | 22781 Znfn1a4 |
| NM_011787 | 23802 Amfr |
| NM_011791 | 23808 Ash2l |
| NM_011793 | 23825 Banf1 |
| NM_011801 | 23837 Cfdp |
| NM_011802 | 270166 Clpx |

Figure 1-23

71

| | |
|---|---|
| NM_011810 | 23873 Faim |
| NM_011813 | 23877 Fiz1 |
| NM_011832 | 23920 Insrr |
| NM_011835 | 23924 Katna1 |
| NM_011838 | 23936 Lynx1 |
| NM_011857 | 23965 Odz3 |
| NM_011870 | 23991 Cib1 |
| NM_011874 | 23996 Psmc4 |
| NM_011883 | 24017 Rnf13 |
| NM_011893 | 24055 Sh3bp2 |
| NM_011907 | 24102 Trex2 |
| NM_011908 | 24109 Ubl3 |
| NM_011909 | 24110 Usp18 |
| NM_011917 | 24128 Xrn2 |
| NM_011929 | 26372 Clcn6 |
| NM_011936 | 26383 Fto |
| NM_011942 | 26394 Lypla2 |
| NM_011946 | 26405 Map3k2 |
| NM_011955 | 26425 Nubp1 |
| NM_011961 | 26432 Plod2 |
| NM_011962 | 26433 Plod3 |
| NM_011967 | 26442 Psma5 |
| NM_011971 | 26446 Psmb3 |
| NM_011986 | 26562 Ncdn |
| NM_011990 | 26570 Slc7a11 |
| NM_011994 | 26874 Abcd2 |
| NM_012018 | 26920 Ma2a8 |
| NM_012019 | 26926 Pdcd8 |
| NM_012029 | 26940 Sitpec |
| NM_012030 | 26941 Slc9a3r1 |
| NM_012032 | 26943 Tde1 |
| NM_012033 | 26944 Tinag |
| NM_012038 | 26950 Vsnl1 |
| NM_012040 | 93843 Pnck |
| NM_012047 | 26992 Brd7 |
| NM_013465 | 11625 Ahsg |
| NM_013481 | 12181 Bop1 |
| NM_013519 | 14234 Foxc2 |
| NM_013525 | 14455 Gas5 |
| NM_013534 | 14789 Grcb |
| NM_013535 | 14790 Grcc10 |
| NM_013541 | 14870 Gstp2 |
| NM_013542 | 14939 Gzmb |
| NM_013556 | 15452 Hprt |
| NM_013559 | 15505 Hsp105 |
| NM_013569 | 16511 Kcnh2 |
| NM_013584 | 16880 Lifr |
| NM_013589 | 16997 Ltbp2 |
| NM_013595 | 17192 Mbd3 |
| NM_013596 | 17203 Mc5r |
| NM_013602 | 17748 Mt1 |
| NM_013614 | 18263 Odc |
| NM_013615 | 18286 Odf2 |
| NM_013617 | 18366 Olfr65 |
| NM_013627 | 18508 Pax6 |
| NM_013635 | 19027 Sypl |

Figure 1-24

72

| | |
|---|---|
| NM_013638 | 19120 Prm3 |
| NM_013641 | 19216 Ptger1 |
| NM_013645 | 19293 Pva |
| NM_013647 | 20055 Rps16 |
| NM_013666 | 225742 Siat8e |
| NM_013680 | 20964 Syn1 |
| NM_013684 | 21374 Tbp |
| NM_013703 | 22359 Vldlr |
| NM_013718 | 27096 Trappc3 |
| NM_013722 | 27204 Syn3 |
| NM_013723 | 27205 Podxl |
| NM_013727 | 27215 Azi2 |
| NM_013736 | 27224 Tceb3 |
| NM_013738 | 27260 Plek2 |
| NM_013750 | 27280 Phlda3 |
| NM_013759 | 27361 Sepr |
| NM_013761 | 27364 Srr |
| NM_013762 | 27367 Rpl3 |
| NM_013779 | 27385 Magel2 |
| NM_013789 | 27414 Gnefr |
| NM_013790 | 27416 Abcc5 |
| NM_013826 | 17434 Mocs2 |
| NM_013835 | 20822 Ssa2 |
| NM_013843 | 24132 Zfp118 |
| NM_013844 | 24135 Zfp68 |
| NM_013859 | 29805 ORF6 |
| NM_013865 | 29812 Ndr3 |
| NM_013867 | 29815 Bcar3 |
| NM_013869 | 29820 Tnfrsf19 |
| NM_013890 | 30050 Fbxw2 |
| NM_013892 | 30052 Pcsk1n |
| NM_013895 | 30055 Timm9 |
| NM_013906 | 30806 Adamts8 |
| NM_013909 | 30840 Fbxl6 |
| NM_013918 | 30940 Usp25 |
| NM_013932 | 30959 Ddx25 |
| NM_013933 | 30960 Vapa |
| NM_015762 | 50493 Txnrd1 |
| NM_015764 | 268527 |
| NM_015776 | 50530 Mfap5 |
| NM_015803 | 50769 Atp8a2 |
| NM_015806 | 50772 Mapk6 |
| NM_015819 | 50786 Hs6st2 |
| NM_016658 | 14430 Galt |
| NM_016665 | 20892 Stra13 |
| NM_016666 | 11632 Aip |
| NM_016673 | 12804 Cntfr |
| NM_016676 | 19325 Rab10 |
| NM_016692 | 16319 Incenp |
| NM_016696 | 14733 Gpc1 |
| NM_016698 | 50849 Rnf10 |
| NM_016705 | 16564 Kif21a |
| NM_016714 | 18141 Nup50 |
| NM_016717 | 50880 Scly |
| NM_016737 | 20867 Stip1 |
| NM_016744 | 18573 Pde1a |

Figure 1-25

73

| | |
|---|---|
| NM_016749 | 53311 Mybph |
| NM_016750 | 51788 H2afz |
| NM_016751 | 51811 Clecsf13 |
| NM_016753 | 17035 Lxn |
| NM_016754 | 17907 Mylpf |
| NM_016757 | 22377 Wbp1 |
| NM_016766 | 51812 Mcrs1 |
| NM_016772 | 51798 Ech1 |
| NM_016775 | 13002 Dnajc5 |
| NM_016782 | 53321 Cntnap1 |
| NM_016789 | 53324 Nptx2 |
| NM_016798 | 53318 Pdlim3 |
| NM_016800 | 53612 Vti1b |
| NM_016802 | 11848 Arha |
| NM_016806 | 53379 Hnrpa2b1 |
| NM_016807 | 53378 Sdcbp |
| NM_016813 | 53319 Nxf1 |
| NM_016849 | 54131 Irf3 |
| NM_016859 | 53414 Bysl |
| NM_016863 | 14226 Fkbp1b |
| NM_016871 | 53333 Tomm40 |
| NM_016875 | 53422 Ybx2 |
| NM_016893 | 53618 Fut8 |
| NM_016908 | 53420 Syt5 |
| NM_016909 | 53424 Tsnax |
| NM_016910 | 53892 Ppm1d |
| NM_016914 | 53856 Prg3 |
| NM_016960 | 20297 Ccl20 |
| NM_016973 | 50935 Siat7f |
| NM_016981 | 20544 Slc9a1 |
| NM_016985 | 53332 Mtmr1 |
| NM_017394 | 53896 Slc7a10 |
| NM_017461 | 54204           Sep-01 |
| NM_017462 | 18975 Polg |
| NM_017477 | 54161 Copg |
| NM_018731 | 11944 Atp4a |
| NM_018754 | 55948 Sfn |
| NM_018757 | 54369 Nme6 |
| NM_018764 | 54216 Pcdh7 |
| NM_018773 | 54353 Scap2 |
| NM_018776 | 54394 Crlf3 |
| NM_018779 | 54611 Pde3a |
| NM_018788 | 54616 Extl3 |
| NM_018798 | 54609 Ubqln2 |
| NM_018801 | 54525 Syt7 |
| NM_018802 | 55925 Syt8 |
| NM_018803 | 54526 Syt10 |
| NM_018804 | 229521 Syt11 |
| NM_018811 | 54608 Abhd2 |
| NM_018812 | 229615 Pias3 |
| NM_018819 | 55951 Brp44l |
| NM_018826 | 54352 Irx5 |
| NM_018855 | 104346 Gas8 |
| NM_018871 | 22628 Ywhag |
| NM_018872 | 56030 D1Bwg0491e |
| NM_018874 | 18946 Pnliprp1 |

Figure 1-26

| | |
|---|---|
| NM_018882 | 14766 Gpr56 |
| NM_018884 | 55983 Pdzrn3 |
| NM_018887 | 56050 Cyp39a1 |
| NM_019402 | 54196 Pabpn1 |
| NM_019409 | 18377 Omg |
| NM_019410 | 18645 Pfn2 |
| NM_019425 | 54342 Gnpnat1 |
| NM_019432 | 170706 Pr1 |
| NM_019435 | 104130 Np15 |
| NM_019443 | 54405 Ndufa1 |
| NM_019458 | 54624 |
| NM_019460 | 54650 Sfmbt1 |
| NM_019464 | 54673 Sh3glb1 |
| NM_019475 | 29847 Olfr157 |
| NM_019477 | 50790 Facl4 |
| NM_019487 | 56016 Hebp2 |
| NM_019509 | |
| NM_019510 | 22065 Trpc3 |
| NM_019512 | 56070 Tcerg1 |
| NM_019535 | 20404 Sh3gl2 |
| NM_019550 | 56195 Ptbp2 |
| NM_019551 | 56196 Ttrap |
| NM_019571 | 56224 Tm4sf9 |
| NM_019572 | 56233 Hdac7a |
| NM_019575 | 56214 Scamp4 |
| NM_019635 | 56274 Stk3 |
| NM_019638 | 56449 Csda |
| NM_019639 | 22190 Ubc |
| NM_019640 | 56305 Pitpnb |
| NM_019643 | 56306 Tera |
| NM_019657 | 56348 Hsd17b12 |
| NM_019662 | 56437 Rrad |
| NM_019663 | 56469 Pias1 |
| NM_019667 | 56324 Stam2 |
| NM_019679 | 57778 Fmnl |
| NM_019686 | 56506 |
| NM_019693 | 53817 Bat1a |
| NM_019695 | 56513 Pard6a |
| NM_019697 | 16508 Kcnd2 |
| NM_019703 | 56421 Pfkp |
| NM_019705 | 24105 AL033326 |
| NM_019707 | 12554 Cdh13 |
| NM_019714 | 21888 Tle4 |
| NM_019718 | 56350 Arl3 |
| NM_019724 | 17389 Mmp16 |
| NM_019725 | 21886 Tle2 |
| NM_019741 | 56485 Slc2a5 |
| NM_019743 | 56353 Rybp |
| NM_019746 | 56330 Pdcd5 |
| NM_019754 | 56370 Tagln3 |
| NM_019759 | 56429 Dpt |
| NM_019764 | 56332 Amotl2 |
| NM_019767 | 56443 Arpc1a |
| NM_019768 | 56397 Morf4l2 |
| NM_019771 | 56431 Dstn |
| NM_019772 | 56372 |

Figure 1-27

75

| NM_019781 | 56273 Pex14 |
| NM_019790 | 56363 Tmeff2 |
| NM_019794 | 56445 Dnaja2 |
| NM_019799 | 56315 Rhcg |
| NM_019802 | 56316 Ggcx |
| NM_019808 | 56376 |
| NM_019825 | 56406 Ncoa6 |
| NM_019831 | 56364 Zfp261 |
| NM_019836 | 56412 |
| NM_019913 | 56551 Txn2 |
| NM_019914 | 56772 |
| NM_019921 | 56697 Akap10 |
| NM_019925 | 56696 Gpr132 |
| NM_019928 | 56640 Klk4 |
| NM_019929 | 20610 Smt3h1 |
| NM_019931 | 56543 Kcnd3 |
| NM_019932 | 56744 Cxcl4 |
| NM_019938 | 56523 Pmfbp1 |
| NM_019942 | 56526 Sep-06 |
| NM_019948 | 56619 Clecsf9 |
| NM_019950 | 56773 Chst5 |
| NM_019957 | 56629 Dnase2b |
| NM_019967 | 56710 Dbccr1 |
| NM_019977 | 56727 Aldrl6 |
| NM_019983 | 56715 Rabgef1 |
| NM_019990 | 56018 Stard10 |
| NM_020004 | 14534 Gcn5l2 |
| NM_020024 | 24075 Taf10 |
| NM_020025 | 26878 B3galt2 |
| NM_020026 | 26879 B3galt3 |
| NM_020031 | 56612 Pfdn5 |
| NM_020293 | 56863 Cldn9 |
| NM_020494 | 27225 Ddx24 |
| NM_020506 | 57258 Xpo4 |
| NM_020518 | 57276 |
| NM_020558 | 57316 C1d |
| NM_020560 | 57312 Mrps31 |
| NM_020567 | 57441 Gmnn |
| NM_020588 | 57439 |
| NM_020590 | 57436 Gabarapl1 |
| NM_020612 | 57358 Cmar |
| NM_021291 | 30962 Slc7a9 |
| NM_021319 | 57757 |
| NM_021325 | 57781 Mox2r |
| NM_021332 | 14652 Glp1r |
| NM_021337 | 108077 Skiv2l |
| NM_021346 | 57908 Zfp318 |
| NM_021354 | 13495 Drg2 |
| NM_021371 | 140904 Caln1 |
| NM_021380 | 58181 Il20 |
| NM_021382 | 21338 Tacr3 |
| NM_021385 | 58186 Rad18 |
| NM_021401 | 58210 Sectm1 |
| NM_021414 | 74340 |
| NM_021430 | 75695 |
| NM_021432 | 58243 |

Figure 1-28

76

| | |
|---|---|
| NM_021433 | 58244 Stx6 |
| NM_021446 | 58520 ORF11 |
| NM_021449 | 58799 AF229032 |
| NM_021452 | 58802 Kcnmb4 |
| NM_021453 | 58803 |
| NM_021458 | 14365 Fzd3 |
| NM_021459 | 16392 Isl1 |
| NM_021460 | 16889 Lip1 |
| NM_021472 | 58809 Rnase4 |
| NM_021483 | 58869 Pex2 |
| NM_021504 | 59007 Ngly1 |
| NM_021505 | 59008 Anapc5 |
| NM_021509 | 59012 Moxd1 |
| NM_021527 | 59030 Mkks |
| NM_021530 | 59033 Slc4a8 |
| NM_021531 | 59035 Carm1 |
| NM_021537 | 59041 Stk25 |
| NM_021543 | 18530 Pcdh8 |
| NM_021547 | 59045 Stard3 |
| NM_021548 | 59046 Arpp19 |
| NM_021550 | 59048 |
| NM_021555 | 59053 Brp16 |
| NM_021560 | 59058 Bhlhb5 |
| NM_021565 | 59090 Midn |
| NM_021567 | 59092 Pcbp4 |
| NM_021606 | 59126 Nek6 |
| NM_021704 | 20315 Cxcl12 |
| NM_021710 | 11782 Ap4s1 |
| NM_021712 | 20508 Slc18a3 |
| NM_021713 | 60315 |
| NM_021789 | 60409 Trappc4 |
| NM_021793 | 60455 Tmem8 |
| NM_021877 | 15460 hr |
| NM_021879 | 18431 p |
| NM_021888 | 60507 Qtrt1 |
| NM_021890 | 60527 Fads3 |
| NM_021898 | 60600 Halapx |
| NM_022007 | 57780 Fxyd7 |
| NM_022011 | 23894 Gtf2h2 |
| NM_022022 | 63958 Ube4b |
| NM_022029 | 64011 Nrgn |
| NM_022030 | 64051 Sv2a |
| NM_022318 | 64082 Popdc2 |
| NM_022320 | 64095 Gpr35 |
| NM_022321 | 64099 Parvg |
| NM_022331 | 64209 Herpud1 |
| NM_022418 | 64295 |
| NM_022813 | 24044 Scamp2 |
| NM_022882 | 64898 Lpin2 |
| NM_022885 | 69048 Slc30a5 |
| NM_022886 | 64929 Scel |
| NM_022982 | 65079 Rtn4r |
| NM_022992 | 65106 Arl6ip5 |
| NM_022995 | 65112 |
| NM_023041 | 19298 Pxf |
| NM_023043 | 26434 Prnd |

Figure 1-29

| | |
|---|---|
| NM_023055 | 65962 Slc9a3r2 |
| NM_023059 | 24058 AI256711 |
| NM_023066 | 65973 Asph |
| NM_023133 | 20085 Rps19 |
| NM_023153 | 66070 |
| NM_023191 | 66317 |
| NM_023197 | 66356 |
| NM_023202 | 66416 Ndufa7 |
| NM_023217 | 66522 |
| NM_023266 | 104348 Zfp120 |
| NM_023290 | 67027 Mkrn2 |
| NM_023305 | 67123 Ubap1 |
| NM_023336 | 67382 Brd3 |
| NM_023370 | 22295 Cdh23 |
| NM_023372 | 67671 |
| NM_023377 | 170460 Stard5 |
| NM_023383 | 67758 Aadac |
| NM_023420 | 68018 Col4a3bp |
| NM_023422 | 68024 Hist1h2bc |
| NM_023450 | 68355 |
| NM_023485 | 68828 Sync |
| NM_023500 | 22439 Xkh |
| NM_023503 | 69260 Ing1l |
| NM_023505 | 69367 Glrx2 |
| NM_023526 | 69721 |
| NM_023530 | 69836 Pla2b12b |
| NM_023537 | 69908 Rab3b |
| NM_023596 | 71279 Slc29a3 |
| NM_023597 | 71354 Wdr31 |
| NM_023605 | 71538 Fbxo9 |
| NM_023633 | 71952 |
| NM_023649 | 72088 Ush1c |
| NM_023662 | 18536 Pcm1 |
| NM_023670 | 140488 Igf2bp3 |
| NM_023721 | 73834 Atp6v1d |
| NM_023788 | 75625 |
| NM_023790 | 75659 |
| NM_023805 | 76257 Slc38a3 |
| NM_023814 | 76365 Tbx18 |
| NM_023868 | 20191 Ryr2 |
| NM_023873 | 68121 |
| NM_023876 | 77766 Elp4 |
| NM_023893 | |
| NM_024173 | 66290 Atp6v1g1 |
| NM_024199 | 67337 Cstf1 |
| NM_024244 | 71721 |
| NM_024255 | 72479 |
| NM_024273 | 76916 |
| NM_024471 | 79464 Lias |
| NM_024472 | 79554 BC002216 |
| NM_024474 | 140709 Col26a1 |
| NM_024475 | 79560 BC002236 |
| NM_025272 | 11974 Atp6v0e |
| NM_025274 | 13915 Dppa5 |
| NM_025282 | 17260 Mef2c |
| NM_025299 | 27366 Txnl4 |

Figure 1-30

| NM_025301 | 27397 Mrpl17 |
| NM_025302 | 27398 Mrpl2 |
| NM_025315 | 108098 Surb7 |
| NM_025316 | 66046 Ndufb5 |
| NM_025319 | 66050 |
| NM_025323 | 66055 |
| NM_025367 | 20698 Sphk1 |
| NM_025368 | 66124 |
| NM_025383 | 66147 |
| NM_025386 | 66153 Fbxo36 |
| NM_025387 | 66154 |
| NM_025389 | 66156 Anapc11 |
| NM_025407 | 22273 Uqcrc1 |
| NM_025419 | 66202 |
| NM_025424 | 66208 |
| NM_025427 | 66214 |
| NM_025456 | 66266 |
| NM_025457 | 66268 |
| NM_025460 | 66271 |
| NM_025479 | 66308 |
| NM_025512 | 66361 |
| NM_025520 | 66373 Lsm5 |
| NM_025529 | 66387 Nudt8 |
| NM_025547 | 66410 |
| NM_025561 | |
| NM_025563 | 66439 |
| NM_025566 | 66443 |
| NM_025573 | 108014 Sfrs9 |
| NM_025577 | 66462 |
| NM_025590 | 329910 Thea |
| NM_025594 | 66492 |
| NM_025612 | |
| NM_025628 | 110323 Cox6b |
| NM_025633 | 66559 Metapl1 |
| NM_025654 | 66599 Rad52b |
| NM_025658 | 66607 Ms4a4d |
| NM_025668 | 66624 |
| NM_025706 | 66687 Tbc1d15 |
| NM_025708 | 66690 |
| NM_025714 | 52184 D3Ertd250e |
| NM_025718 | 66705 Dnase1l2 |
| NM_025730 | 66725 |
| NM_025736 | 66736 |
| NM_025747 | 66756 |
| NM_025748 | 66757 Deadc1 |
| NM_025766 | 66784 |
| NM_025779 | 66815 |
| NM_025785 | 66822 Fbxo25 |
| NM_025806 | 66857 |
| NM_025808 | 66863 Lztr1 |
| NM_025834 | 66901 Proz |
| NM_025835 | 66904 Pccb |
| NM_025839 | 66911 |
| NM_025849 | 66928 |
| NM_025855 | 52665 Echdc1 |
| NM_025864 | 66950 |

Figure 1-31

| | |
|---|---|
| NM_025865 | 66952 |
| NM_025895 | 66999 |
| NM_025896 | 67000 |
| NM_025918 | 52715 D11Ertd707e |
| NM_025921 | 67028 |
| NM_025922 | 16434 Itpa |
| NM_025937 | 67050 |
| NM_025938 | 67053 |
| NM_025962 | 67096 |
| NM_025964 | 67099 |
| NM_025975 | 67117 Tcte1l |
| NM_025978 | 67120 |
| NM_025987 | 67130 Ndufa6 |
| NM_025989 | 67133 Gp2 |
| NM_025994 | 27984 D4Wsu27e |
| NM_025998 | 67149 |
| NM_026004 | 107569 Nt5c3 |
| NM_026015 | 67178 |
| NM_026047 | 72486 |
| NM_026061 | 67264 Ndufb8 |
| NM_026065 | 67270 |
| NM_026068 | 67279 |
| NM_026069 | 67281 Rpl37 |
| NM_026082 | 67299 Dock7 |
| NM_026091 | 67326 |
| NM_026092 | 67328 |
| NM_026095 | 67332 Snrpd3 |
| NM_026106 | 13486 Dr1 |
| NM_026123 | 67387 |
| NM_026154 | 107732 Mrpl10 |
| NM_026174 | 67464 Lysal1 |
| NM_026175 | 67465 Sf3a1 |
| NM_026177 | 67467 |
| NM_026183 | 67473 |
| NM_026187 | 52231 D1Ertd161e |
| NM_026217 | 67526 Apg12l |
| NM_026218 | 67529 |
| NM_026219 | 67530 Uqcrb |
| NM_026235 | 67557 |
| NM_026253 | 67580 |
| NM_026260 | 67590 |
| NM_026267 | 67602 |
| NM_026298 | 67661 Wim |
| NM_026304 | 67669 |
| NM_026312 | 67683 |
| NM_026318 | 67693 |
| NM_026338 | 67722 |
| NM_026348 | 67733 |
| NM_026367 | 67769 |
| NM_026402 | 67841 |
| NM_026413 | 67854 Slco6b1 |
| NM_026415 | 67859 |
| NM_026417 | 67864 |
| NM_026418 | 67865 Rgs10 |
| NM_026428 | 67880 Dcxr |
| NM_026433 | 67888 |

Figure 1-32

| | |
|---|---|
| NM_026434 | 67889 |
| NM_026437 | 67894 |
| NM_026446 | 56470 Rgs19 |
| NM_026495 | 67991 |
| NM_026507 | 68014 |
| NM_026510 | 68017 |
| NM_026513 | |
| NM_026514 | 260409 Cdc42ep3 |
| NM_026519 | 68032 |
| NM_026523 | 68039 Nmb |
| NM_026526 | 68043 |
| NM_026539 | 68058 Chd1l |
| NM_026556 | 68097 |
| NM_026559 | 52700 D11Ertd672e |
| NM_026580 | 68149 |
| NM_026583 | 68152 |
| NM_026592 | 68170 |
| NM_026612 | 68198 Ndufb2 |
| NM_026616 | 68209 |
| NM_026645 | 68265 |
| NM_026660 | 68294 |
| NM_026670 | 68310 |
| NM_026693 | 93739 Gabarapl2 |
| NM_026700 | 2 loci |
| NM_026908 | 69008 |
| NM_027019 | 69287 Odf3 |
| NM_027142 | 69623 |
| NM_027205 | 69761 |
| NM_027493 | 56249 Actr8 |
| NM_027722 | 71207 |
| NM_027910 | 71765 Klhdc3 |
| NM_028075 | 72049 Tnfrsf13c |
| NM_028122 | 108052 Slc14a1 |
| NM_028146 | 72185 |
| NM_028333 | 72713 Angptl1 |
| NM_028494 | 73297 |
| NM_028564 | 73534 |
| NM_028605 | 73682 |
| NM_028770 | |
| NM_029203 | 75199 |
| NM_029292 | 75453 |
| NM_029417 | 75757 |
| NM_029457 | 75826 Senp2 |
| NM_029460 | 75835 |
| NM_029583 | |
| NM_029636 | 104002 Ctsq |
| NM_029716 | 108699 Chn1 |
| NM_029814 | 76959 |
| NM_030248 | 80280 Cdk5rap3 |
| NM_030262 | 80294 |
| NM_030263 | 80295 BC003498 |
| NM_030265 | 80334 Kcnip4 |
| NM_030558 | 80733 Car15 |
| NM_030564 | 80751 Rnf34 |
| NM_030677 | 14776 Gpx2 |
| NM_030684 | 94094 Trim34 |

Figure 1-33

| | |
|---|---|
| NM_030685 | 28146 D3Ucla1 |
| NM_030696 | 80879 Slc16a3 |
| NM_030716 | 80906 Kcnip2 |
| NM_030717 | 80907 Lactb |
| NM_030719 | 80909 Gats |
| NM_030735 | 81010 V3R9 |
| NM_030744 | 76378 Ropn1 |
| NM_030749 | 81500 |
| NM_031156 | 15925 Ide |
| NM_031162 | 12503 Cd3z |
| NM_031174 | 13508 Dscam |
| NM_031179 | 81898 Sf3b1 |
| NM_031180 | 83379 |
| NM_031182 | 83383 Tfap4 |
| NM_031184 | 83396 Gli5 |
| NM_031201 | 22122 Tsta3 |
| NM_031248 | 83409 |
| NM_031249 | 83410 Cstf2t |
| NM_031261 | 83457 Fthl17 |
| NM_031391 | 83602 Gtf2a1 |
| NM_031392 | 83669 Wdr6 |
| NM_031401 | 83679 Usmg4 |
| NM_031404 | 83766 Actl6 |
| NM_031493 | 27084 Xlr5 |
| NM_031879 | |
| NM_032003 | 83965 Enpp5 |
| NM_033042 | 85030 Tnfrsf25 |
| NM_033269 | 12671 Chrm3 |
| NM_033327 | 94187 Ebfaz |
| NM_033370 | 70349 Copb1 |
| NM_033561 | 22384 Wbscr1 |
| NM_033613 | 112403 Dom3z |
| NM_033616 | 114564 Csprs |
| NM_033652 | 110648 Lmx1a |
| NM_052973 | 94186 Strn3 |
| NM_052975 | 94185 Tnfrsf21 |
| NM_053069 | 11793 Apg5l |
| NM_053071 | 12864 Cox6c |
| NM_053074 | 18226 Nup62 |
| NM_053075 | 19744 Rheb |
| NM_053076 | 20168 Rtn3 |
| NM_053107 | 93690 Gpr45 |
| NM_053115 | 93732 Acox2 |
| NM_053161 | 94064 Mrpl27 |
| NM_053178 | 94180 |
| NM_053186 | 94218 Cnnm3 |
| NM_053190 | 94226 Edg8 |
| NM_053204 | 111173 Rab6ip2 |
| NM_054046 | 23854 Def8 |
| NM_054066 | 114875 Plcz1 |
| NM_054069 | 114895 Psbpc1 |
| NM_080287 | 140579 Elmo2 |
| NM_080428 | 50754 Fbxw7 |
| NM_080639 | 110595 Timp4 |
| S67967 | 16976 Lrpap1 |
| S82853 | 21915 Dtymk |

Figure 1-34

| | | |
|---|---|---|
| U14172 | 13669 | Eif3s10 |
| U22015 | 20184 | Rxrip110 |
| U43892 | 11306 | Abcb7 |
| U51204 | 100732 | Mapre3 |
| U51279 | 12048 | Bcl2l |
| U61363 | 21888 | Tle4 |
| U64606 | 17977 | Ncoa1 |
| U70033 | 20541 | Slc8a1 |
| U85993 | 319845 | |
| U89513 | 73647 | Capn9 |
| X13721 | 15416 | Hoxb8 |
| X16493 | 22640 | Zfp1 |
| X61453 | 50884 | Nckap1 |
| X74504 | 27883 | D16H22S680E |
| X76850 | 17164 | Mapkapk2 |
| X81365 | 21367 | Cntn2 |
| X84014 | 16774 | Lama3 |
| X97581 | 20689 | Sall3 |
| X98848 | 18639 | Pfkfb1 |
| Y12713 | 27282 | Erv4 |
| Y15910 | 54004 | Diap2 |
| Y17793 | 19876 | Robo1 |

Figure 1-35

Figure 2

-0.4  0.0  0.2  0.4
Log$_2$(ratio)

| ACCESS | LOCUS | GENE | LOCALIZATION | BIOLOGICAL FUNCTION |
|---|---|---|---|---|
| NM_024471 | 79464 | Lias | mitochondrion | misc |
| NM_019487 | 56016 | Hebp2 | cytoplasm;extracellular | misc |
| NM_011793 | 23825 | Banf1 | integral to membrane;nucleus | misc |
| BC004811 | 224742 | Abcf1 | integral to plasma membrane | transport |
| NM_010250 | 14394 | Gabra1 | extracellular space;integral to | signaling |
| NM_009557 | 22704 | Zfp46 | transcription factor complex | transcription |
| AF104414 | 16798 | Lats1 | | signaling ? |
| L32178 | 232333 | Slc6a1 | integral to plasma membrane | transport |
| AF407579 | 140859 | Nek8 | | signaling ? |
| AK016018 | 75163 | | | unknown |
| NM_008703 | 18101 | Nmbr | integral to plasma membrane | signaling (receptor) |
| NM_007574 | 12262 | C1qg | extracellular space | signaling |
| AK005842 | 71840 | | membrane | ctsk |
| U14172 | 13669 | Eif3s10 | | translation |
| NM_008955 | 19202 | Psx1 | transcription factor complex | transcription |
| M36654 | 15412 | Hoxb4 | transcription factor complex | transcription |
| NM_018754 | 55948 | Sfn | cytoplasm;extracellular space | cell cycle ? |
| NM_007474 | 11833 | Aqp8 | integral to plasma membrane;mito | transport |
| AK018561 | 71546 | | | unknown |
| AK002458 | 68339 | | myosin | unknown |
| NM_054066 | 114875 | Plcz1 | | signaling |
| NM_019703 | 56421 | Pfkp | 6-phosphofructokinase complex | metabolism |
| NM_017461 | 54204 | sep-01 | mitochondrion | cell cycle |
| NM_009228 | 20648 | Snta1 | cytoskeleton;postsynaptic membra | signaling |
| NM_022418 | 64295 | | extracellular space;integral to | unknown |
| BC011091 | 109815 | H47 | integral to membrane | unknown |
| NM_007557 | 12162 | Bmp7 | extracellular space | signaling (secreted) |
| AK006467 | 75568 | | protein kinase CK2 complex | signaling |
| NM_011102 | 18752 | Prkcc | | signaling |
| AK011127 | 67031 | Upf3a | | misc |
| NM_008604 | 17380 | Mme | integral to plasma membrane | protein degradation |
| AK019929 | 78283 | | | unknown |
| AK019168 | 68991 | | membrane | unknown |
| AK018155 | 70750 | | extracellular space | tumor related (secretion) |
| AK007941 | 67509 | | | unknown |
| AF223953 | 54208 | Arl6ip1 | cytosol;integral to membrane;tra | translation |
| NM_015764 | 268527 | | | transport |
| NM_008538 | 17118 | Marcks | membrane | signaling |
| NM_019639 | 22190 | Ubc | nucleus | protein degradation |
| AK016247 | 18640 | Pfkfb2 | cytosol | metabolism |
| AK011544 | 66254 | | | transcription |
| NM_019509 | | | | unknown |
| NM_008120 | 14612 | Gja4 | connexon complex;integral to mem | signaling (receptor) |
| NM_008085 | 14447 | Gapds | | metabolism |
| AK015402 | 74862 | | | unknown |

BNS  BS

84

## Figure 3

Log₂(ratio) scale: -0.3  -0.1  0.1  0.3

| ACCESS | LOCUS | GENE | LOCALIZATION | BIOLOGICAL FUNCTION |
|---|---|---|---|---|
| NM_026318 | 67693 | | nascent polypeptide-associated c | unknown |
| AF035526 | 20534 | Slc4a1ap | integral to plasma membrane | transport |
| AK013145 | 72174 | | extracellular | unknown |
| NM_033613 | 112403 | Dom3z | integral to membrane | misc |
| NM_022318 | 64082 | Popdc2 | integral to membrane | unknown |
| NM_021879 | 18431 | p | integral to membrane | misc |
| AK008151 | 70114 | | | unknown |
| NM_025975 | 67117 | Tcte1l | cytoplasmic dynein complex | unknown |
| NM_008866 | 18777 | Lypla1 | mitochondrion | lipid metabolism |
| NM_008955 | 19202 | Psx1 | transcription factor complex | transcription |
| NM_020024 | 24075 | Taf10 | focal adhesion;transcription fac | transcription |
| AK012601 | 67622 | | | unknown |
| AF297860 | 104776 | Aldh6a1 | mitochondrion | metabolism |
| AK010466 | 76484 | | | unknown |
| NM_019921 | 56697 | Akap10 | mitochondrion | transport? |
| AK013055 | 67059 | | | unknown |
| NM_019759 | 56429 | Dpt | extracellular matrix;extracellul | cell adh |
| AK012922 | 72681 | | | unknown |
| NM_013843 | 24132 | Zfp118 | transcription factor complex | misc |
| NM_011994 | 26874 | Abcd2 | integral to plasma membrane;pero | lipid metabolism |
| NM_026304 | 67669 | | | unknown |
| NM_016960 | 20297 | Ccl20 | extracellular space | immune response |
| AK020225 | 77506 | | | unknown |
| NM_008133 | 14661 | Glud | mitochondrion | metabolism |
| AK021023 | 78688 | Nol3 | nucleus | apoptosis |
| X61453 | 50884 | Nckap1 | integral to membrane | unknown |
| NM_080428 | 50754 | Fbxw7 | Golgi apparatus;endoplasmic reti | protein degradation |
| NM_008821 | 18630 | Pet2 | extrachromosomal circular DNA | transcription |
| NM_023537 | 69908 | Rab3b | synaptic vesicle | signaling |
| NM_018811 | 54608 | Abhd2 | integral to membrane | unknown |
| AF121976 | 57250 | Ors19 | integral to membrane | signaling (receptor) |
| NM_025989 | 67133 | Gp2 | extracellular space;membrane | transport |
| AK019886 | 68151 | | | unknown |
| NM_011908 | 24109 | Ubl3 | | unknown |
| NM_025785 | 66822 | Fbxo25 | | unknown |
| BC009123 | 72704 | | | unknown |
| BC005600 | 70231 | Gorasp2 | Golgi membrane | unknown |
| AK009308 | 246257 | | cytoplasm | tumor related |

BNS  BS

Figure 4

Log$_2$(ratio)

-0.3  -0.1  0.1  0.3

| ACCESS | LOCUS | GENE | LOCALIZATION | BIOLOGICAL FUNCTION |
|---|---|---|---|---|
| U51204 | 100732 | Mapre3 | | ctsk |
| NM_023133 | 20085 | Rps19 | cytosolic ribosome (sensu Eukary | translation |
| AK011034 | 19139 | Prps1 | cytoplasm | metabolism |
| NM_021382 | 21338 | Tacr3 | integral to membrane;plasma memb | signaling (receptor) |
| NM_011801 | 23837 | Cfdp | basement membrane;integral to me | apoptosis |
| NM_019512 | 56070 | Tcerg1 | nuclear matrix | transcription |
| AK007415 | 69046 | Hbld2 | | unknown |
| NM_011665 | 22196 | Ube2i | | protein degradation |
| AF150755 | 11426 | Macf1 | actin cytoskeleton;microtubule c | ctsk |
| NM_025978 | 67120 | · | cytoplasm | unknown |
| BC012512 | 67398 | Srpr | integral to membrane;signal reco | transport |
| NM_008589 | 17293 | Mesp2 | extracellular space;membrane | signaling (development) |
| AK016385 | 52348 | D8Ertd531e | - | unknown |
| AK009856 | 77039 | | | unknown |
| AK003256 | 74320 | | | misc |
| NM_011541 | 21399 | Tcea1 | nucleus | Transcription |
| BC016210 | 67017 | | extracellular space;integral to | unknown |
| NM_026519 | 68032 | | | Immune response |
| AK004431 | 66090 | | | unknown |
| NM_008382 | 16326 | Inhbe | extracellular space | growth factor (secreted) |
| NM_025612 | | | | unknown |
| BC016080 | 103963 | Rpn1 | endoplasmic reticulum;extracellu | lipid metabolism |
| AK008703 | 75669 | Pik3r4 | | signaling |
| NM_021606 | 59126 | Nek6 | | signaling |
| AK009123 | 69487 | | extrachromosomal circular DNA | metabolism |
| AK009074 | 107734 | Mrpl30 | mitochondrial ribosome | misc |
| NM_015819 | 50786 | Hs6st2 | extracellular space | ecm |
| NM_011203 | 19248 | Ptpn12 | soluble fraction | Signaling |

BNS  BS

86

## Figure 5

| | ACCESS | LOCUS | GENE | LOCALIZATION | BIOLOGICAL FUNCTION |
|---|---|---|---|---|---|
| | NM_026267 | 67602 | | membrane | unknown |
| | AK021066 | 77318 | | | transcription |
| | NM_013627 | 18508 | Pax6 | transcription factor complex | transcription |
| | NM_007667 | 12564 | Cdh8 | extracellular space;integral to | cell adh |
| | AK006141 | 75656 | | | unknown |
| | AK006938 | 73493 | | | unknown |
| | AK003388 | 76291 | | | unknown |
| | NM_022007 | 57780 | Fxyd7 | integral to membrane | transport |
| | NM_012040 | 93843 | Pnck | calcium-calmodulin-dependent pro | signaling |
| | AK011196 | 67042 | | | signaling (secreted) |
| | NM_017477 | 54161 | Copg | Golgi apparatus;membrane | transport |
| | AK004723 | 74144 | Robo4 | integral to membrane;external si | signaling (receptor) |
| | AK007993 | 67490 | | membrane | unknown |
| | AF061933 | 50779 | Rgs6 | heterotrimeric G-protein complex | signaling |
| | NM_024472 | 79554 | BC002216 | protein kinase CK2 complex | translation ? |
| | AF127245 | 20591 | Smcx | mitochondrion;nucleus | transcription |
| | AK015555 | 74919 | | | unknown |

-0.3  -0.1  0.1  0.3

Log$_2$(ratio)

BNS  BS

EP 1 649 062 B1

EP 1 649 062 B1

Figure 6 A

Log$_2$(ratio) scale: -0.3  -0.1  0.1  0.3

| | ACCESS | LOCUS | GENE | LOCALIZATION | BIOLOGICAL FUNCTION |
|---|---|---|---|---|---|
| | NM_019695 | 56513 | Pard6a | tight junction | cell cycle |
| | AK015971 | 75829 | | | unknown |
| | NM_021530 | 59033 | Slc4a8 | integral to membrane | transport |
| | AK009669 | 72309 | | | unknown |
| | NM_016798 | 53318 | Pdlim3 | Z disc;actin cytoskeleton | ctsk |
| | NM_009465 | 26362 | Axl | extracellular space;integral to | signaling (receptor) |
| | NM_033269 | 12671 | Chrm3 | integral to plasma membrane | signaling (receptor) |
| | AK016128 | 75359 | | | unknown |
| | AK013055 | 67059 | | | unknown |
| | AK009454 | 268721 | | | unknown |
| | NM_026507 | 68014 | | | unknown |
| | AK013367 | 72792 | | | unknown |
| | NM_013909 | 30840 | Fbxl6 | | protein degradation |
| | NM_008799 | 18567 | Pdcd2 | 26S proteasome;cytoplasm;nucleus | apoptosis |

BNS  BS

EP 1 649 062 B1

Figure 6 B

| | ACCESS | LOCUS | GENE | LOCALIZATION | BIOLOGICAL FUNCTION |
|---|---|---|---|---|---|
| | NM_009153 | 20347 | Sema3b | endoplasmic reticulum;extracellu | growth factor (secreted) |
| | NM_008604 | 17380 | Mme | integral to plasma membrane | protein degradation |
| | NM_025922 | 16434 | Itpa | | metabolism |
| | AK009187 | 69482 | | | unknown |
| | NM_009551 | 22682 | Zfp216 | | transcription? |
| | AK008753 | 70387 | | | unknown |
| | NM_008339 | 15985 | Cd79b | extracellular space;integral to | signaling (receptor) |
| | AK005734 | 75470 | | | unknown |
| | NM_010010 | 13116 | Cyp46a1 | endoplasmic reticulum;extracellu | transport |
| | AK003132 | 75406 | Ndufs7 | extracellular space;mitochondrio | metabolism |
| | AB036708 | 170828 | Vgll1 | | transcription |
| | NM_019825 | 56406 | Ncoa6 | transcription factor complex | transcription |
| | NM_021459 | 16392 | Isl1 | nucleus | transcription |
| | NM_021291 | 30962 | Slc7a9 | integral to membrane | transport |
| | M36514 | 22688 | Zfp26 | nucleus | transcription |
| | NM_026908 | 69008 | | | unknown |
| | AK012733 | 76366 | | voltage-gated potassium channel | transcription? |
| | AK018089 | 76137 | | | unknown |
| | AK006222 | 72251 | | | unknown |
| | AB047921 | 67210 | | nucleus | transcription? |
| | NM_026298 | 67661 | Wim | kinesin complex | unknown |
| | NM_011202 | 19247 | Ptpn11 | cytoskeleton | signaling |
| | AK007933 | 72282 | | | unknown |
| | AK003282 | 66496 | | | unknown |
| | AK018495 | 74536 | | | unknown |
| | AK002769 | 71683 | Gypc | | unknown |
| | NM_019977 | 56727 | Aldrl6 | cytoplasm;inclusion body | metabolism |
| | NM_010473 | 15464 | Hrc | extracellular space;smooth endop | signaling |
| | AK010476 | 71979 | | | unknown |
| | NM_019475 | 29847 | Olfr157 | integral to plasma membrane | signaling (receptor) |
| | NM_019741 | 56485 | Slc2a5 | integral to membrane | transport |
| | NM_009660 | 11687 | Alox15 | plasma membrane | lipid metabolism |
| | NM_024199 | 67337 | Cstf1 | mRNA cleavage stimulating factor | transcription |
| | NM_053178 | 94180 | | | metabolism? |
| | NM_011638 | 22042 | Trfr | endosome;integral to membrane;pl | signaling |
| | AK004679 | 73340 | Nptxr | chromatin;extracellular space;in | transcription |
| | NM_008492 | 16832 | Ldh2 | cytosol | metabolism |
| | AK007494 | 69064 | | | unknown |
| | AK020410 | 52120 | D8Ertd354e | integral to membrane | unknown |
| | AK004803 | 66875 | | eukaryotic translation elongatio | translation? |
| | AJ131821 | 11938 | Atp2a2 | integral to plasma membrane;micr | transport |
| | NM_009079 | 19934 | Rpl22 | cytosolic ribosome (sensu Eukary | translation |
| | AK016352 | 66722 | | axoneme;microtubule | unknown |
| | NM_025736 | 66736 | | nucleus | unknown |
| | NM_016973 | 50935 | Siat7f | Golgi apparatus;integral to memb | misc |
| | NM_009854 | 12516 | Cd7 | extracellular space;integral to | immune response (receptor) |
| | AK019216 | 78832 | | | unknown |
| | NM_015776 | 50530 | Mfap5 | extracellular space;microfibril | ecm |
| | NM_007995 | 14133 | Fcna | extracellular space | immune response (secreted) |
| | AK019744 | 78931 | | | unknown |
| | AK008994 | 107358 | | extracellular space;integral to | transport |
| | AK017726 | 70625 | Crsp7 | transcription elongation factor | transcription |
| | AK004139 | 68735 | | mitochondrion;ribosome | translation |
| | AK010312 | 76483 | | integral to membrane | unknown |
| | AK005661 | 69082 | | | signaling (receptor) |
| | AK013837 | 73090 | | | unknown |
| | NM_009481 | 22284 | Usp9x | 26S proteasome | protein degradation |
| | BC005658 | 69957 | Cdc16 | | cell cycle |
| | NM_025427 | 66214 | | cytoplasm;nucleus | cell cycle |
| | NM_023422 | 68024 | Hist1h2bc | nucleosome;nucleus | misc |
| | AK012610 | 72654 | | | unknown |
| | NM_011740 | 22631 | Ywhaz | cytoplasm | signaling |
| | AF062567 | 20687 | Sp3 | transcription factor complex | transcription |

Log$_2$(ratio)  -0.3  -0.1  0.1  0.3

BNS  BS

Figure 7

-0.2  0.0  0.2
$\log_2(ratio)$

EP 1 649 062 B1

90

| ACCESS | LOCUS | GENE | LOCALIZATION | BIOLOGICAL FUNCTION |
|---|---|---|---|---|
| NM_007761 | 12909 | Crcp | | signaling (receptor) |
| AK013044 | 106200 | | | translation ? |
| NM_031174 | 13508 | Dscam | extracellular space;integral to | cell adh |
| NM_011492 | 20869 | Stk11 | microtubule cytoskeleton | Signaling |
| AK002490 | 20195 | S100a11 | cytoplasm | signaling |
| NM_019724 | 17389 | Mmp16 | extracellular matrix;extracellul | protein degradation |
| NM_009579 | 22782 | Slc30a1 | extracellular space;integral to | transport |
| AK017736 | 70616 | Sf4 | intracellular | transcription |
| NM_018779 | 54611 | Pde3a | integral to membrane | signaling |
| BC004766 | 71799 | Ptcd1 | ribosome | unknown |
| NM_018798 | 54609 | Ubqln2 | endoplasmic reticulum membrane | unknown |
| AK013995 | 73078 | | mitochondrion | misc |
| NM_011356 | 20378 | Frzb | extracellular space;membrane | signaling |
| NM_008969 | 19224 | Ptgs1 | cytoplasm;extracellular space;me | signaling |
| NM_025457 | 66268 | | | unknown |
| NM_016698 | 50849 | Rnf10 | | transcription |
| NM_023605 | 71538 | Fbxo9 | ubiquitin ligase complex | protein degradation |
| BC005788 | 71994 | Cnn3 | cytoskeleton | ctsk ? |
| AF237628 | 93677 | Lmod2 | cytoskeleton | ctsk |
| NM_023597 | 71354 | Wdr31 | membrane | transport |
| L17069 | 214162 | Mll | nucleus | Transcription |
| NM_019714 | 21888 | Tle4 | nucleus | transcription |
| X76850 | 17164 | Mapkapk2 | | signaling |
| AK012885 | 67212 | Mrpl55 | membrane | unknown |
| NM_025577 | 66462 | | | unknown |
| NM_018731 | 11944 | Atp4a | integral to membrane | metabolism |
| NM_007555 | 12160 | Bmp5 | extracellular space | ECM |
| AK003586 | 231712 | | | unknown |
| AK002574 | 68348 | | | unknown |
| AK007389 | 107686 | Snrpd2 | small nucleolar ribonucleoprotei | transcription |
| AK004975 | 71770 | Ap2b1 | clathrin adaptor complex;coated | transport |
| NM_020567 | 57441 | Gmnn | nucleus | cell cycle |
| AK017769 | 70614 | | | unknown |
| NM_007393 | 11461 | Actb | cytoskeleton | ctsk |
| AK010752 | 73694 | | | unknown |
| NM_009015 | 19366 | Rad54l | nucleoplasm | cell cycle |
| AK002704 | 68365 | Rab14 | | signaling |
| AK002252 | 68323 | | | unknown |
| NM_008537 | 17117 | Amacr | mitochondrion;peroxisome | metabolism |
| AB048834 | 64435 | Fcamr | integral to membrane | protein degradation |
| NM_026187 | 52231 | D1Ertd161e | | transcription ? |

BNS  BS

Figure 8 A

-0.3  -0.1  0.1  0.3
Log$_2$(ratio)

| | ACCESS | LOCUS | GENE | LOCALIZATION | BIOLOGICAL FUNCTION |
|---|---|---|---|---|---|
| | NM_025573 | 108014 | Sfrs9 | membrane;spliceosome complex | transcription |
| | NM_023290 | 67027 | Mkrn2 | intracellular;membrane | unknown |
| | AK003661 | 68520 | | | unknown |
| | AK016812 | 74085 | | | tumor related |
| | AK014680 | 74617 | | extracellular space;vacuole | ecm |
| | AF320126 | 14816 | Grm1 | integral to membrane | Signaling (receptor) |
| | NM_009100 | 20129 | Rptn | extracellular | ecm |
| | AK014134 | 73172 | | extrachromosomal circular DNA | unknown |
| | NM_011673 | 22234 | Ugcg | endoplasmic reticulum;extracellu | lipid metabolism |
| | BC002191 | 108902 | | extracellular space | misc |
| | NM_025628 | 110323 | Cox6b | respiratory chain complex IV (se | transport |
| | AF346413 | 75526 | Spinlw1 | acrosome;extracellular space | protein degradation |
| | NM_007524 | 12020 | Bapx1 | transcription factor complex | transcription |
| | AK013012 | 72184 | | | unknown |
| | NM_026700 | | 2 loci | | unknown |
| | NM_007748 | 12861 | Cox6a1 | integral to membrane;respiratory | metabolism |
| | NM_023530 | 69836 | Pla2b12b | extracellular space | lipid metabolism |
| | AK004655 | 74107 | | | translation |
| | AF228925 | 72088 | Ush1c | cilium | signaling |
| | NM_011813 | 23877 | Fiz1 | transcription factor complex | transcription |
| | NM_008997 | 19326 | Rab11b | Golgi apparatus | traffic |
| | NM_020590 | 57436 | Gabarapl1 | plasma membrane | ctsk |
| | NM_019932 | 56744 | Cxcl4 | extracellular space | immune response |
| | NM_028605 | 73682 | | | unknown |
| | AF259674 | 107272 | Psat1 | | metabolism |
| | NM_009352 | 21749 | Terf1 | chromosome-, telomeric region;nu | cell cycle |
| | NM_007791 | 13007 | Csrp1 | actin cytoskeleton;nucleus | ctsk |
| | AK004617 | 66861 | Dnajc10 | endoplasmic reticulum;mitochondr | translation |
| | NM_019950 | 56773 | Chst5 | Golgi apparatus;extracellular sp | translation |
| | NM_011293 | 20022 | Polr2j | DNA-directed RNA polymerase II-, | transcription |
| | NM_010770 | 17182 | Matn3 | extracellular matrix;extracellul | ecm |
| | NM_007607 | 12351 | Car4 | extracellular space;membrane | metabolism (secreted) |
| | L34676 | 11784 | Apba2 | | signaling |
| | NM_053115 | 93732 | Acox2 | peroxisome | metabolism |
| | AK005048 | 223776 | | extracellular space | metabolism |
| | NM_013519 | 14234 | Foxc2 | transcription factor complex | transcription |
| | NM_011197 | 19221 | Ptgfrn | extracellular space;integral to | translation (receptor) |
| | NM_011714 | 22385 | Baz1b | centric heterochromatin;condense | misc |
| | NM_011607 | 21923 | Tnc | extracellular space | cell adh |
| | BC002302 | 106583 | Rbm16 | | cell cycle |
| | NM_030744 | 76378 | Ropn1 | integral to membrane;mitochondri | transport |
| | NM_016705 | 16564 | Kif21a | kinesin complex | ctsk |
| | AK014623 | 66701 | | | unknown |
| | NM_011961 | 26432 | Plod2 | endoplasmic reticulum;extracellu | metabolism |
| | NM_013723 | 27205 | Podxl | extracellular space;integral to | immune response (receptor) |
| | NM_008357 | 16168 | Il15 | extracellular | growth factor (secreted) |

BNS  BS

Figure 8 B

| | ACCESS | LOCUS | GENE | LOCALIZATION | BIOLOGICAL FUNCTION |
|---|---|---|---|---|---|
| | BC018470 | 23960 | Oas1g | | immune response |
| | AB055635 | 26931 | Ppp2r5c | nucleus;protein phosphatase type | signaling |
| | AF105217 | 57813 | Tk2 | mitochondrion | metabolism |
| | NM_018871 | 22628 | Ywhag | | signaling |
| | AF155583 | 18089 | Nkx2-3 | transcription factor complex | transcription |
| | NM_025424 | 66208 | | extracellular space;membrane | transport |
| | NM_023191 | 66317 | | heterotrimeric G-protein complex | signaling ? |
| | NM_024273 | 76916 | | integral to membrane;mitochondri | unknown |
| | AK009460 | 66053 | Ppil2 | nucleus | translation |
| | NM_011857 | 23965 | Odz3 | integral to plasma membrane | unknown |
| | NM_011389 | 20495 | Slc12a1 | integral to plasma membrane | transport |
| | AK006490 | 66938 | | | immune response ? |
| | NM_008738 | 18188 | Nrtn | extracellular space | signaling (secreted) |
| | NM_021789 | 60409 | Trappc4 | Golgi apparatus;endoplasmic reti | traffic |
| | NM_009781 | 12288 | Cacna1c | T-tubule;voltage-gated calcium c | ion channel |
| | AK006079 | 76405 | | ubiquitin ligase complex | unknown |
| | NM_020560 | 57312 | Mrps31 | mitochondrion;nucleus | transcription |
| | AK002667 | 67899 | | cytoplasm | unknown |
| | NM_026514 | 260409 | Cdc42ep3 | plasma membrane | ctsk |
| | NM_009679 | 11773 | Ap2m1 | clathrin vesicle coat;coated pit | traffic |
| | NM_013906 | 30806 | Adamts8 | extracellular matrix;extracellul | ecm |
| | NM_016714 | 18141 | Nup50 | nuclear pore | transport |
| | AK012134 | 72195 | | nucleus | transcription |
| | NM_026908 | 69008 | | | unknown |
| | AK019803 | 78217 | | | unknown |
| | NM_026367 | 67769 | | extracellular matrix | unknown |
| | AK013880 | 70223 | Nars | cytoplasm;soluble fraction | metabolism |
| | AK010147 | 73373 | | | unknown |
| | NM_019764 | 56332 | Amotl2 | tight junction | cell adh |
| | NM_008317 | 15586 | Hyal1 | | metabolism |
| | BC003959 | 286940 | Flnb | focal adhesion;stress fibers | ctsk |
| | NM_026523 | 68039 | Nmb | extracellular space | signaling (secreted) |
| | NM_008135 | 14664 | Slc6a9 | integral to plasma membrane | transport |
| | AK019527 | 78903 | Wmip1 | DNA replication factor C complex | misc |
| | X16493 | 22640 | Zfp1 | nucleus | transcription |
| | NM_019425 | 54342 | Gnpnat1 | ER-Golgi intermediate compartmen | metabolism |
| | AK005562 | 72205 | | microtubule associated complex | traffic |
| | NM_021530 | 59033 | Slc4a8 | integral to membrane | transport |
| | NM_018874 | 18946 | Pnliprp1 | extracellular space | metabolism |
| | NM_013595 | 17192 | Mbd3 | chromatin;nucleus | transcription |
| | AK019481 | 68260 | | | unknown |
| | NM_009584 | 22792 | Zrf2 | nucleus | cell cycle |
| | NM_008925 | 19089 | Prkcsh | alpha-glucosidase complex;endopl | signaling |
| | NM_025301 | 27397 | Mrpl17 | mitochondrial large ribosomal su | misc |
| | NM_011251 | 19654 | Rbm6 | spliceosome complex | misc |
| | NM_009197 | 20502 | Slc16a2 | integral to plasma membrane | transport |
| | AK020024 | 106504 | Stk38 | | signaling ? |
| | AK014285 | 76965 | | | unknown |
| | AK008800 | 70082 | | membrane | unknown |
| | NM_009369 | 21810 | Tgfbi | extracellular matrix;extracellul | cell adh |
| | BC013800 | 67063 | | | unknown |
| | NM_031493 | 27084 | Xlr5 | synaptonemal complex | unknown |
| | NM_009746 | 12055 | Bcl7c | extrachromosomal circular DNA | tumor related |

-0.3   -0.1   0.1   0.3

Log$_2$(ratio)

BNS BS

Figure 9 A

-0.3   -0.1   0.1   0.3
Log$_2$(ratio)

| ACCESS | LOCUS | GENE | LOCALIZATION | BIOLOGICAL FUNCTION |
|--------|-------|------|--------------|---------------------|
| NM_021565 | 59090 | Midn | nucleolus | unknown |
| NM_011550 | 21428 | Tcfl4 | cytoplasm;transcription factor c | transcription |
| NM_025654 | 66599 | Rad52b | | unknown |
| AK007485 | 69053 | | | unknown |
| NM_031184 | 83396 | Gli5 | transcription factor complex | transcription |
| NM_019535 | 20404 | Sh3gl2 | cytoplasm | signaling |
| NM_016673 | 12804 | Cntfr | extracellular space;membrane | signaling (secreted) |
| NM_052975 | 94185 | Tnfrsf21 | integral to membrane | apoptosis |
| NM_026095 | 67332 | Snrpd3 | ribonucleoprotein complex;viral | misc |
| NM_016676 | 19325 | Rab10 | Golgi apparatus | tumor related |
| AK021166 | 77609 | | | unknown |
| AJ288061 | 76707 | Clasp1 | cytoplasmic microtubule;microtub | ctsk |
| AK006739 | 22217 | Usp12 | | protein degradation |
| NM_011595 | 21859 | Timp3 | basement membrane | protein degradation |
| AK013044 | 106200 | | | translation ? |
| AK005730 | 69318 | | | unknown |
| BC002191 | 108902 | | extracellular space | misc |
| AK019605 | 78799 | | | immune response |
| AK005044 | 74157 | | nucleolus | misc |
| NM_008574 | 17235 | Mcsp | mitochondrial outer membrane | misc |
| AK017801 | 68021 | | extrachromosomal circular DNA | unknown |
| NM_015803 | 50769 | AtpBa2 | integral to membrane | transport |
| AK018060 | 76131 | | myosin | signaling |

BNS  BS

93

EP 1 649 062 B1

Figure 9 B

-0.3  -0.1  0.1  0.3
Log$_2$(ratio)

| ACCESS | LOCUS | GENE | LOCALIZATION | BIOLOGICAL FUNCTION |
|--------|-------|------|--------------|---------------------|
| AK019462 | 20643 | Snrpe | small nucleolar ribonucleoprotei | transcription |
| AK011160 | 72117 | Mak3p | spliceosome complex | transcription |
| NM_010826 | 17540 | Mrvi1 | endoplasmic reticulum membrane;i | transport |
| AK019863 | 77634 | | | translation |
| NM_016806 | 53379 | Hnrpa2b1 | nucleus;ribonucleoprotein comple | transcription |
| AK018132 | 74008 | | lysosome | signaling |
| U61363 | 21888 | Tle4 | nucleus | transcription |
| NM_013890 | 30050 | Fbxw2 | cytoplasm;extrachromosomal circu | signaling |
| NM_017462 | 18975 | Polg | gamma DNA polymerase complex | cell cycle |
| NM_008857 | 18759 | Prkcl | plasma membrane | signaling |
| NM_019724 | 17389 | Mmp16 | extracellular matrix;extracellul | protein degradation |
| NM_008602 | 17344 | Miz1 | nucleus;septin ring | transcription |
| NM_029292 | 75453 | | | unknown |
| AK017929 | 76007 | Zfp198 | nucleus | transcription |
| AK002704 | 68365 | Rab14 | | signaling |
| NM_025282 | 17260 | Mef2c | mediator complex | transcription, |
| NM_019460 | 54650 | Sfmbt1 | chromatin | transcription |
| AK009213 | 75573 | | | unknown |
| AK004323 | 66201 | | | unknown |
| AK019803 | 78217 | | | unknown |
| NM_013535 | 14790 | Grcc10 | | unknown |
| AK008734 | 72349 | Dusp3 | | signaling |
| NM_011012 | 18389 | Oprl | integral to plasma membrane | signaling (receptor) |
| NM_023336 | 67382 | Brd3 | integral to membrane;nucleus | signaling ? |
| M63245 | 11655 | Alas1 | | metabolism |
| AK010433 | 66609 | Cryzl1 | | metabolism |
| NM_052973 | 94186 | Strn3 | | signaling ? |
| NM_008956 | 19205 | Ptbp1 | nucleolus;nucleoplasm | transcription |
| NM_026559 | 52700 | D11Ertd672e | | transport |
| NM_011522 | 20974 | Syngr3 | integral to plasma membrane | unknown |
| AK005063 | 110351 | Rap1ga1 | | signaling |
| AK009308 | 246257 | | cytoplasm | tumor related |
| BC006063 | 75570 | | | unknown |
| AK004664 | 73830 | | ' | unknown |
| BC004648 | 74737 | | | unknown |
| D86949 | 18845 | Plxna2 | extracellular space;integral to | signaling |
| AK004943 | 66627 | | endoplasmic reticulum | unknown |
| NM_029716 | 108699 | Chn1 | cytoplasm | signaling |
| AK006525 | 70375 | | | misc |
| NM_009084 | 19981 | Rpl37a | cytosolic ribosome (sensu Eukary | translation |
| AK006959 | 74279 | | | unknown |
| BC003986 | | | | unknown |
| AK016890 | 71099 | | microtubule cytoskeleton | signaling ? |
| NM_019836 | 56412 | | extracellular space;integral to | unknown |
| NM_025633 | 66559 | Metapl1 | integral to plasma membrane | protein degradation |
| AK007339 | 99730 | Taf13 | transcription factor TFIID compl | transcription |
| NM_021371 | 140904 | Caln1 | | signaling |
| NM_019754 | 56370 | Tagln3 | cytoskeleton | ctsk |
| NM_012030 | 26941 | Slc9a3r1 | actin cytoskeleton;apical plasma | ctsk |
| U89513 | 73647 | Capn9 | intracellular | misc |
| NM_007997 | 14149 | Fdxr | mitochondrion | metabolism |
| AK005095 | 69757 | Leng1 | extracellular space | unknown |
| AB035322 | 108897 | | centriole | unknown |

BNS  BS

Figure 10 A

-0.3 -0.1 0.1 0.3
$\log_2(ratio)$

| ACCESS | LOCUS | GENE | LOCALIZATION | BIOLOGICAL FUNCTION |
|---|---|---|---|---|
| AK017095 | 71240 | Osbpl7 | | metabolism |
| AK013393 | 72843 | Prdm4 | | transcription |
| AK002360 | 64697 | Keg1 | mitochondrion | unknown |
| NM_019512 | 56070 | Tcerg1 | nuclear matrix | transcription |
| NM_009257 | 20724 | Serpinb5 | cytoplasm | tumor related (suppressor) |
| NM_010837 | 17760 | Mtap6 | microtubule associated complex | ctsk |
| AK012212 | 107581 | Col16a1 | collagen | ecm |
| AF332067 | 75425 | | | metabolism |
| NM_010476 | 15490 | Hsd17b7 | endoplasmic reticulum;integral t | metabolism |
| AK012635 | 69920 | Polr2i | DNA-directed RNA polymerase II-, | transcription |
| AF237917 | 109225 | | | signaling |
| NM_011552 | 21453 | Tcof1 | nucleolus | misc |
| NM_025389 | 66156 | Anapc11 | anaphase-promoting complex | cell cycle |
| AF246218 | 192192 | Shkbp1 | voltage-gated potassium channel | ion channel |
| AK016214 | 75275 | | | unknown |
| AK005619 | 72215 | | | unknown |
| NM_010255 | 14431 | Gamt | cytoplasm | metabolism |
| AK003201 | 68472 | | | unknown |
| NM_008995 | 19305 | Pex5 | peroxisomal membrane | transport |
| AK004928 | 56275 | Rbm14 | cytoplasm;mediator complex;ribon | transcription ? |
| NM_019663 | 56469 | Pias1 | nucleus | transcription |
| AK005864 | 75645 | | | unknown |
| AK008008 | 69824 | | extrachromosomal circular DNA | misc |
| AB071988 | 26914 | H2afy | centrosome;condensed chromosome; | cell cycle |
| NM_011529 | 21353 | Tank | cytoplasm | signaling |
| NM_009325 | 21390 | Tbxa2r | integral to membrane | signaling (receptor) |
| NM_023383 | 67758 | Aadac | extracellular space;microsome | lipid metabolism |
| AK013464 | 67515 | | protein serine-threonine phospha | signaling (osmosis response) |
| NM_016914 | 53856 | Prg3 | extracellular space | ecm |
| NM_008957 | 19206 | Ptch | integral to plasma membrane | signaling (receptor) |
| AK006521 | 99349 | | cytoplasm | translation |
| AF327857 | 232288 | | | unknown |
| AK017464 | 75747 | Sesn3 | nucleus | cell cycle |

BNS BS

Figure 10 B

-0.3  -0.1  0.1  0.3

$\log_2(ratio)$

| ACCESS | LOCUS | GENE | LOCALIZATION | BIOLOGICAL FUNCTION |
|---|---|---|---|---|
| AK003850 | 66151 | | extracellular matrix | ecm |
| AK021190 | 77644 | | | unknown |
| AF190665 | 56873 | Lmbr1 | | unknown |
| NM_020004 | 14534 | Gcn5l2 | histone acetyltransferase comple | transcription |
| AK009937 | 71941 | | | transcription ? |
| NM_025590 | 329910 | Thea | cytoplasm | lipid metabolism |
| AK020702 | 78428 | | | unknown |
| NM_018776 | 54394 | Crlf3 | membrane | signaling (receptor) |
| NM_009536 | 22627 | Ywhae | | signaling |
| NM_021793 | 60455 | Tmem8 | extracellular space;integral to | cell adh |
| AK015007 | 77048 | | cytoskeleton | transport |
| BC005485 | 99543 | Olfml3 | extracellular space | ecm |
| NM_009476 | 22269 | Upk2 | endoplasmic reticulum;extracellu | tumor related |
| NM_007966 | 14028 | Evx1 | transcription factor complex | transcription |
| NM_009425 | 22035 | Tnfsf10 | extracellular space;integral to | apoptosis |
| NM_008229 | 15182 | Hdac2 | cytoplasm;histone deacetylase co | transcription |
| NM_021550 | 59048 | | integral to membrane | unknown |
| NM_011292 | 20005 | Rpl9 | cytosolic ribosome (sensu Eukary | translation |
| NM_022320 | 64095 | Gpr35 | integral to membrane | signaling (receptor) |
| NM_013750 | 27280 | Phlda3 | integral to membrane | unknown |
| AK005536 | 67883 | Uxs1 | integral to membrane | misc |
| AK003676 | 68581 | | Golgi apparatus;integral to plas | traffic |
| NM_013596 | 17203 | Mc5r | integral to membrane | signaling |
| AK014763 | 75769 | | integral to membrane | unknown |
| AK003413 | 68001 | | ribosome | unknown |
| NM_026428 | 67880 | Dcxr | brush border;microvillus | metabolism |
| AK012240 | 66399 | | mitochondrion | translation |
| NM_021567 | 59092 | Pcbp4 | cytoplasm;nucleus;ribonucleoprot | transcription |
| AF276975 | 192200 | Swam2 | extracellular space | metabolism |
| NM_030677 | 14776 | Gpx2 | cytosol | misc |
| NM_023450 | 68355 | | integral to membrane | unknown |
| NM_008998 | 19329 | Rab17 | early endosome | signaling |
| NM_018884 | 55983 | Pdzrn3 | peroxisome | signaling |
| AK009134 | 75572 | Acyp2 | extrachromosomal circular DNA | metabolism |
| U14172 | 13669 | Eif3s10 | | translation |
| AK019664 | 78801 | | | signaling ? |
| AF332051 | 104112 | Acly | intracellular | metabolism |
| NM_031184 | 83396 | Gli5 | transcription factor complex | transcription |
| NM_008663 | 17921 | Myo7a | myosin | ctsk |
| NM_033616 | 114564 | Csprs | nucleus | signaling |
| AK017848 | 74329 | | | unknown |
| NM_026217 | 67526 | Apg12l | cytoplasm | protein degradation |
| NM_019725 | 21886 | Tle2 | nucleus | transcription |
| AK003154 | 68420 | | | unknown |
| NM_009398 | 21930 | Tnfaip6 | extracellular space | cell adh |
| AK017158 | 75965 | | nucleus | unknown |
| AK018937 | 78636 | | | unknown |
| NM_025864 | 66950 | | integral to membrane | unknown |
| NM_010200 | 14168 | Fgf13 | extracellular space | signaling |
| NM_021712 | 20508 | Slc18a3 | integral to plasma membrane | transport |
| AK016267 | 65103 | Arl6ip6 | integral to membrane | unknown |
| NM_008083 | 14432 | Gap43 | axon;membrane | signaling |
| AK008791 | 74245 | | extracellular space;lysosome | protein degradation |

BNS   BS

EP 1 649 062 B1

Figure 11 A

Log$_2$(*ratio*)

-0.4　0.0　0.2　0.4

| ACCESS | LOCUS | GENE | LOCALIZATION | BIOLOGICAL FUNCTION |
|--------|-------|------|--------------|---------------------|
| NM_008254 | 15356 | Hmgcl | extracellular space;mitochondrio | misc |
| AK020724 | 77578 | Bcl9 | nucleus | immune response |
| NM_023066 | 65973 | Asph | integral to membrane | misc |
| AK005015 | 246277 | Csad | | metabolism |
| NM_026091 | 67326 | | | unknown |
| AK009759 | 66958 | | chloroplast;integral to membrane | unknown |
| NM_027019 | 69287 | Odf3 | extracellular space;outer dense | signaling (secreted) |
| NM_025368 | 66124 | | | cell adh |
| AK015275 | 73945 | D8Ertd69e | | unknown |
| AF317552 | 108062 | Cstf2 | mRNA cleavage stimulating factor | translation |
| NM_019772 | 56372 | | integral to membrane | protein degradation ? |

BNS BS

Figure 11 B

Log$_2$(ratio)

-0.4　0.0　0.2　0.4

| ACCESS | LOCUS | GENE | LOCALIZATION | BIOLOGICAL FUNCTION |
|---|---|---|---|---|
| AK019621 | 78796 | | | Transcription |
| NM_023670 | 140488 | Igf2bp3 | cytoplasm | growth factor |
| NM_008812 | 18600 | Padi2 | extracellular | translation |
| NM_011242 | 19395 | Rasgrp2 | cytosol;nucleosome;plasma membra | signaling |
| AK015947 | 68195 | Rnaset2 | | metabolism |
| AK011446 | 70348 | | | unknown |
| NM_007591 | 12317 | Calr | endoplasmic reticulum lumen;extr | cell adhesion |
| NM_022030 | 64051 | Sv2a | integral to membrane;synaptic ve | transport |
| AK018331 | 76205 | Stard3nl | integral to membrane | unknown |
| AF281870 | 64009 | Syne1 | nuclear membrane;sarcomere | ctsk |
| AK009114 | 71885 | | | cell adh ? |
| NM_025367 | 20698 | Sphk1 | soluble fraction | signaling |
| NM_023485 | 68828 | Sync | Z disc;cytosol;intermediate fila | ctsk |
| AK020492 | 77305 | | | transcription |
| NM_019477 | 50790 | Facl4 | cytoplasm;integral to membrane | metabolism |
| NM_026434 | 67889 | | viral nucleocapsid | unknown |
| NM_008064 | 14387 | Gaa | lysosome | misc |
| NM_016782 | 53321 | Cntnap1 | extracellular space;integral to | cell adh |
| AK003179 | 66101 | D4Wsu43e | spliceosome complex | transcription |
| AK019547 | | | | unknown |
| AK009014 | 69541 | | extracellular space | unknown |
| NM_016717 | 50880 | Scly | mitochondrial inner membrane | metabolism |
| NM_028333 | 72713 | Angptl1 | extracellular space | growth factor |
| NM_013541 | 14870 | Gstp2 | cytoplasm | metabolism |
| X76850 | 17164 | Mapkapk2 | | signaling |
| BC017138 | 109168 | | | immune response ? |
| NM_019643 | 56306 | Tera | | unknown |
| NM_008791 | 18546 | Pcp4 | | signaling |
| NM_010891 | 18000 | sep-02 | contractile ring | cell cycle |
| AK021099 | 77462 | | | unknown |
| AK014942 | 76856 | | integral to membrane | transport |
| NM_021548 | 59046 | Arpp19 | cytoplasm | transport ? |
| AK009267 | 28019 | Ing4 | | transcription |
| NM_016665 | 20892 | Stra13 | | transcription |
| AK013427 | 104884 | | | signaling |
| NM_007538 | 12057 | Opn1sw | integral to membrane;photorecept | signaling |
| NM_010010 | 13116 | Cyp46a1 | endoplasmic reticulum;extracellu | transport |
| NM_008421 | 16502 | Kcnc1 | voltage-gated potassium channel | ion channel |
| NM_008016 | 14210 | Fin15 | | misc |
| NM_011196 | 19218 | Ptger3 | integral to membrane;plasma memb | signaling (receptor) |
| NM_010259 | 14468 | Gbp1 | cytosol | immune response |
| NM_031162 | 12503 | Cd3z | integral to membrane;plasma memb | immune response |
| AF276974 | 192201 | | extracellular space | unknown |
| AK002667 | 67899 | | cytoplasm | unknown |
| AF369902 | 68291 | Mto1 | mitochondrion | metabolism |
| NM_023814 | 76365 | Tbx18 | microtubule;nucleus | transcription |
| AK015803 | 225980 | | | unknown |
| AK004550 | 71707 | | integral to membrane | misc |
| AK002360 | 64697 | Keg1 | mitochondrion | unknown |
| NM_023266 | 104348 | Zfp120 | nucleus | unknown |
| BC003255 | 228355 | Madd | plasma membrane | apoptosis |
| AK006487 | 75623 | | | unknown |
| NM_011423 | 20601 | Smr3 | extracellular | growth factor (secreted) |
| NM_008125 | 14619 | Gjb2 | connexon complex;integral to mem | signaling (receptor) |
| AK017484 | 67023 | | mitochondrial matrix | transcription |
| AK002842 | 68386 | | | unknown |
| NM_009555 | 22700 | Zfp40 | transcription factor complex | transport |
| AK015309 | 75792 | | | unknown |
| NM_007567 | 12217 | Bsn | cytoskeleton;integral to membran | signaling |
| NM_021354 | 13495 | Drg2 | mitochondrion | unknown |
| AK016584 | 72230 | | nucleus | unknown |
| AK017149 | 75964 | | | unknown |
| NM_008329 | 15951 | Ifi16 | nucleosome;nucleus | immune response |

BNS　BS

Figure 12

Log$_2$(ratio)
-0.4  0.0  0.2  0.4

| ACCESS | LOCUS | GENE | LOCALIZATION | BIOLOGICAL FUNCTION |
|---|---|---|---|---|
| NM_009860 | 12532 | Cdc25c | mitotic chromosome;nucleus | cell cycle |
| AF369902 | 68291 | Mto1 | mitochondrion | metabolism |
| NM_008729 | 18163 | Catnd2 | cytoskeleton | cell adhesion |
| NM_020567 | 57441 | Gmnn | nucleus | cell cycle |
| NM_009426 | 22044 | Trh | extracellular space;soluble frac | growth factor (secreted) |
| NM_010389 | 15002 | H2-Ob | extracellular space;integral to | immune response (receptor) |
| L21027 | 236539 | Phgdh | | metabolism |
| AK008083 | 72041 | | | unknown |
| NM_008451 | 16594 | Klc2 | kinesin complex | ctsk |
| AK003996 | 68668 | | extracellular space | protein degradation |
| NM_008106 | 14539 | Opn1mw | integral to plasma membrane;phot | signaling (receptor) |
| AK008125 | 228880 | | | unknown |
| NM_019957 | 56629 | Dnase2b | cytoplasm;extracellular space | misc |
| AK009243 | 69534 | Avpi1 | | signaling |
| AK007831 | 68115 | | | unknown |
| NM_008571 | 17225 | Mcpt2 | cytoplasm;extracellular space | protein degradation |
| AK011565 | 69904 | | | unknown |
| NM_011835 | 23924 | Katna1 | cytoskeleton;lipid particle;nucl | cell cycle ? |
| AK010396 | 71986 | Ddx28 | extracellular space;ribulose bis | unknown |
| NM_019695 | 56513 | Pard6a | tight junction | cell cycle |
| AK011290 | 67763 | | extracellular space | metabolism |
| NM_013542 | 14939 | Gzmb | cytoplasm;extracellular space | protein degradation |
| NM_011617 | 21948 | Tnfsf7 | integral to membrane | tumor related |
| AK006993 | 73486 | | ribosome | unknown |
| NM_026417 | 67864 | | integral to membrane | unknown |
| NM_016981 | 20544 | Slc9a1 | basolateral plasma membrane;extr | transport |
| NM_015819 | 50786 | Hs6st2 | extracellular space | ecm |
| AK004657 | 74111 | | | unknown |
| NM_016875 | 53422 | Ybx2 | cytoplasm | translation |
| AK013309 | 170707 | Usp31 | | protein degradation |
| NM_021380 | 58181 | Il20 | extracellular space | immune response |
| NM_023805 | 76257 | Slc38a3 | integral to plasma membrane | transport |
| AK004563 | | | | unknown |
| NM_028564 | 73534 | | | unknown |
| AK020727 | 77754 | | | misc |
| AK015972 | 75137 | | cytoplasm | unknown |
| AK004913 | 74158 | | | unknown |
| NM_025561 | | | | unknown |
| NM_026418 | 67865 | Rgs10 | | signaling |
| NM_016800 | 53612 | Vti1b | Golgi apparatus;integral to memb | traffic |
| AK019514 | 78104 | | | unknown |
| NM_031180 | 83379 | | integral to membrane | metabolism |
| AK005887 | 69354 | Slc38a4 | integral to membrane | transport |
| NM_013615 | 18286 | Odf2 | myosin | ctsk |
| AK007653 | 52443 | D4Ertd786e | ribosome | translation |
| AK006009 | 75553 | | | unknown |
| AK019938 | 321015 | | | unknown |
| AK016916 | 109242 | Kif24 | mitochondrion | misc |
| NM_008902 | 19011 | Pp11r | extracellular space | cell adh |
| NM_008756 | 18260 | Ocln | integral to membrane;tight junct | cell adh |
| AK002441 | 66039 | D14Ertd449e | | unknown |
| AK019176 | 93687 | Csnk1a1 | | signaling |
| BC017636 | 110796 | Sdccag33 | | transcription |
| BC002230 | 217827 | BC002230 | intracellular | unknown |
| NM_031201 | 22122 | Tsta3 | extrachromosomal circular DNA | metabolism |
| NM_009420 | 22024 | Crisp2 | extracellular space | unknown |
| NM_009086 | 20017 | Rpo1-2 | DNA-directed RNA polymerase I co | transcription |
| AK007777 | 83490 | Pik3ap1 | cytoplasm;nucleus;plasma membran | signaling |
| NM_010599 | 16499 | Kcnab3 | cytoplasm;integral to plasma mem | ion channel |
| NM_021332 | 14652 | Glp1r | extracellular space;integral to | signaling (receptor) |
| NM_007429 | 11609 | Agtr2 | integral to plasma membrane | signaling (receptor) |
| M12289 | 17885 | Myh8 | myosin;striated muscle thick fil | ctsk |
| NM_008317 | 15586 | Hyal1 | | metabolism |
| AK017530 | 70502 | | | unknown |
| AK002360 | 64697 | Keg1 | mitochondrion | unknown |

BNS  BS

99

Figure 13

-0.3  -0.1  0.1  0.3
Log₂(ratio) → $Log_2(ratio)$

| ACCESS | LOCUS | GENE | LOCALIZATION | BIOLOGICAL FUNCTION |
|---|---|---|---|---|
| NM_019662 | 56437 | Rrad | membrane | signaling |
| AK004939 | 71753 | Tmprss6 | integral to membrane;plasma memb | protein degradation |
| NM_031182 | 83383 | Tfap4 | membrane | transcription |
| NM_026177 | 67467 | | | unknown |
| NM_013589 | 16997 | Ltbp2 | extracellular matrix | ecm |
| AK009180 | 67484 | | integral to membrane;intracellul | misc |
| BC005518 | 76867 | | extracellular space;integral to | unknown |
| NM_011936 | 26383 | Fto | | unknown |
| NM_008958 | 19207 | Ptch2 | integral to membrane | signaling (receptor) |
| NM_030684 | 94094 | Trim34 | cytoplasm | unknown |
| AK012971 | 74201 | | cytoplasm | signaling ? |
| AK002842 | 68386 | | | unknown |
| NM_017462 | 18975 | Polg | gamma DNA polymerase complex | cell cycle |
| AK005167 | 76686 | | . | unnown |
| NM_009779 | 12267 | C3ar1 | integral to membrane | immune response |
| NM_008663 | 17921 | Myo7a | myosin | ctsk |
| BC003284 | 73828 | Wdr21 | heterotrimeric G-protein complex | signaling |
| AK017550 | 59032 | | membrane | cell cycle ? |
| AK020090 | 68176 | | | transcription |
| AK005431 | 67009 | | kinesin complex | unknown |
| NM_008877 | 18815 | Plg | extracellular space | apoptosis |
| AK005063 | 110351 | Rap1ga1 | | signaling |
| AF158744 | 67310 | Prlpc2 | extracellular space | growth factor (secreted) |
| X13721 | 15416 | Hoxb8 | transcription factor complex | transcription |
| NM_009680 | 11774 | Ap3b1 | Golgi trans face | transport |
| NM_007628 | 12427 | Ccna1 | cytosol;nucleus | cell cycle |
| NM_008249 | 15278 | Tfb2m | extracellular space;nucleolus | transcription |
| NM_029292 | 75453 | | | unknown |
| NM_019435 | 104130 | Np15 | integral to membrane;mitochondri | unknown |
| AK003549 | 68499 | Mrpl53 | | translation |
| AK013927 | 73099 | | | unknown |
| NM_008126 | 14620 | Gjb3 | connexon complex;integral to mem | signaling (receptor) |
| AK019531 | 78902 | | | unknown |
| BC004759 | 171095 | Il17rc | integral to plasma membrane | immune response (receptor) |
| NM_008372 | 16197 | Il7r | extracellular space;integral to | immune response (receptor) |
| NM_028494 | 73297 | | | unknown |
| AK004342 | 66478 | | | unknown |
| NM_026260 | 67590 | | cytoplasm;extracellular space | unknown |
| NM_019432 | 170706 | Pr1 | integral to membrane | ion channel |
| L34676 | 11784 | Apba2 | | signaling |
| NM_026183 | 67473 | | integral to membrane | unknown |
| NM_008222 | 15159 | Hccs | mitochondrion | metabolism |

BNS  BS

Figure 14

Log$_2$(ratio)
-0.3  -0.1  0.1  0.3

| ACCESS | LOCUS | GENE | LOCALIZATION | BIOLOGICAL FUNCTION |
|---|---|---|---|---|
| AK018560 | 67556 | Pigm | | metabolism ? |
| AK007485 | 69053 | | | unknown |
| NM_027205 | 69761 | | peroxisome | transport ? |
| BC018468 | 66753 | | | unknown |
| AK015642 | 75036 | | | misc (secreted) |
| NM_009983 | 13033 | Ctsd | extracellular space;lysosome | protein degradation |
| NM_008060 | 14376 | G2an | alpha-glucosidase complex;endopl | metabolism |
| D13903 | 19266 | Ptprd | integral to membrane;plasma memb | signaling (receptor) |
| AK003420 | 68567 | Cgref1 | extracellular space | cell cycle ? |
| NM_018819 | 55951 | Brp44l | | unknown |
| AF326960 | 21380 | Tbx1 | microtubule;nucleus | transcription |
| BC002298 | 19283 | Ptprz1 | extracellular matrix;extracellul | signaling (receptor) |
| AK021033 | 77219 | | cytoplasm | metabolism ? |
| AK013856 | 23938 | Map2k5 | extracellular | signaling |
| NM_010471 | 15444 | Hpca | | unknown |
| AK014285 | 76965 | | | unknown |
| NM_016717 | 50880 | Scly | mitochondrial inner membrane | metabolism |
| NM_013638 | 19120 | Prm3 | nucleosome;nucleus | cell cycle |
| AK013924 | 12301 | Cacybp | cytoplasm;nucleus | unknown |
| NM_009427 | 22057 | Tob1 | cytoplasm;extracellular | signaling |
| NM_007399 | 11487 | Adam10 | extracellular matrix;extracellul | protein degradation |
| AK020653 | 78558 | | extracellular | signaling |
| AK004655 | 74107 | | | translation |
| AK003256 | 74320 | | | misc |
| NM_023596 | 71279 | Slc29a3 | integral to membrane | transport |
| NM_019409 | 18377 | Omg | extracellular space;integral to | signaling |
| AK020700 | 77763 | | | ctsk |
| AK005205 | 67129 | | | unknown |
| NM_021547 | 59045 | Stard3 | integral to membrane;late endoso | metabolism |
| BC004635 | 102607 | Snx19 | | unknown |
| NM_025386 | 66153 | Fbxo36 | | transcription ? |
| AK017138 | 74471 | | | unknown |
| NM_016910 | 53892 | Ppm1d | protein serine-threonine phospha | signaling |
| AK013267 | 72747 | | integral to membrane | unknown |

BNS  BS

EP 1 649 062 B1

Log$_2$(ratio)

-0.2    0.0    0.2

# Figure 15

| ACCESS | LOCUS | GENE | LOCALIZATION | BIOLOGICAL FUNCTION |
|---|---|---|---|---|
| NM_009852 | 12511 | Cd6 | extracellular space;integral to | cell adh (receptor) |
| AK003642 | 68618 | | lysosome | metabolism ? |
| AF228925 | 72088 | Ush1c | cilium | signaling |
| NM_024199 | 67337 | Cstf1 | mRNA cleavage stimulating factor | transcription |
| AK012842 | 72686 | | | unknown |
| NM_008167 | 14804 | Grid2 | integral to membrane;synaptosome | ion channel |
| NM_019458 | 54624 | | nucleosome | unknown |
| NM_016658 | 14430 | Galt | cytoplasm | metabolism |
| NM_024474 | 140709 | Col26a1 | Golgi apparatus;endoplasmic reti | ecm |
| AK003651 | 14000 | Etohi2 | | translation |
| AB023957 | 170773 | AB023957 | | unknown |
| NM_025561 | | | | unknown |
| NM_025419 | 66202 | | cytoplasm | unknown |
| AK019481 | 68260 | | | unknown |
| NM_009047 | 19700 | Rem | membrane | signaling |
| BC003842 | 97112 | C87860 | extracellular | transport |
| BC005598 | 98221 | Ga17 | signalosome complex | protein degradation |
| NM_016859 | 53414 | Bysl | cytoplasm | cell adh |
| AK007327 | 66144 | Atp6v1f | hydrogen-transporting ATPase V1 | metabolism |
| NM_013614 | 18263 | Odc | mitochondrial inner membrane | misc |
| AK005852 | 71838 | Phf7 | | unknown |
| AK005479 | 52076 | D4Ertd89e | nucleosome;nucleus | misc |
| NM_030558 | 80733 | Car15 | | metabolism |
| NM_013559 | 15505 | Hsp105 | cytoplasm | translation |
| NM_023133 | 20085 | Rps19 | cytosolic ribosome (sensu Eukary | translation |
| NM_021385 | 58186 | Rad18 | nucleus | cell cycle |
| NM_023633 | 71952 | | cytosol | unknown |
| NM_021472 | 58809 | Rnase4 | | misc |
| NM_008742 | 18205 | Ntf3 | extracellular space | signaling (secreted) |
| NM_011838 | 23936 | Lynx1 | extracellular space;integral to | Signaling (receptor) |
| NM_011791 | 23808 | Ash2l | | transcription |
| AK013800 | 73053 | | | unknown |
| NM_007930 | 13803 | Enc1 | cytoskeleton | ctsk |
| AK014487 | 257635 | Sdsl | | metabolism |

BNS  BS

102

EP 1 649 062 B1

## Figure 16

-0.2  0.0  0.2
Log$_2$(ratio)

| ACCESS | LOCUS | GENE | LOCALIZATION | BIOLOGICAL FUNCTION |
|---|---|---|---|---|
| NM_022011 | 23894 | Gtf2h2 | transcription factor TFIIH compl | transcription |
| AK004632 | 74105 | Gga2 | | traffic |
| NM_013762 | 27367 | Rpl3 | nucleolus;ribosome | translation |
| NM_008128 | 14623 | Gjb6 | connexon complex;integral to mem | signaling |
| AK005267 | 68953 | | non-muscle myosin | tumor related |
| AF375046 | 93762 | Smarca5 | nucleus | misc |
| AK014891 | 14479 | Usp15 | | protein degradation |
| NM_053075 | 19744 | Rheb | spliceosome complex | signaling |
| NM_008642 | 17777 | Mttp | endoplasmic reticulum | traffic |
| NM_008066 | 14395 | Gabra2 | integral to plasma membrane | signaling (receptor) |
| AK003605 | 74770 | | integral to membrane | unknown |
| NM_023526 | 69721 | | extracellular space;membrane | signaling |
| NM_010826 | 17540 | Mrvi1 | endoplasmic reticulum membrane;i | transport |
| NM_016696 | 14733 | Gpc1 | extracellular matrix;extracellul | signaling (receptor) |
| AF332067 | 75425 | | | metabolism |
| NM_020612 | 57358 | Cmar | | cell adh |
| AK011781 | 72482 | Acbd6 | ribosome | signaling |
| S67967 | 16976 | Lrpap1 | extracellular space | tumor related |
| NM_009482 | 22286 | Utf1 | transcription factor complex | transcription |
| NM_019990 | 56018 | Stard10 | | tumor related |
| NM_011219 | | | | unknown |
| AK003880 | 68647 | | | unknown |
| NM_008172 | 14814 | Grin2d | extracellular space;integral to | signaling (receptor) |
| NM_008158 | 14761 | Gpr27 | extracellular space;integral to | signaling |
| NM_013759 | 27361 | Sepr | | metabolism |

BNS  BS

## Figure 17 A

Log₂(ratio) scale: -0.3  -0.1  0.1  0.3

| ACCESS | LOCUS | GENE | LOCALIZATION | BIOLOGICAL FUNCTION |
|---|---|---|---|---|
| NM_019990 | 56018 | Stard10 | | tumor related |
| AK015063 | 75773 | | | unknown |
| BC011482 | 23943 | Mbc2 | integral to membrane | unknown |
| BC004012 | 192185 | BC004012 | cytosol;extrachromosomal circula | metabolism |
| AK017808 | 70652 | | integral to membrane | transport? |
| NM_030696 | 80879 | Slc16a3 | integral to membrane | transport |
| NM_018788 | 54616 | Extl3 | endoplasmic reticulum;integral t | signaling |
| NM_023790 | 75659 | | endoplasmic reticulum | protein degradation |
| NM_007761 | 12909 | Crcp | | signaling (receptor) |
| AK006023 | 75540 | Fpgt | | misc |
| NM_021555 | 59053 | Brp16 | | unknown |
| NM_008078 | 14417 | Gad2 | extrachromosomal circular DNA | metabolism |
| NM_021458 | 14365 | Fzd3 | extracellular space;integral to | signaling (receptor) |
| NM_021414 | 74340 | | | unknown |
| NM_026556 | 68097 | | dynein complex | ctsk |
| NM_009964 | 12955 | Cryab | cytoplasm | misc |
| S82853 | 21915 | Dtymk | | metabolism |
| AK009487 | 67863 | Slc25a11 | inner membrane;integral to membr | traffic |
| NM_011109 | 18782 | Pla2g2d | extracellular space | metabolism |
| NM_011955 | 26425 | Nubp1 | mitochondrion;nucleus | metabolism |
| NM_009614 | 11490 | Adam15 | extracellular matrix;extracellul | protein degradation |
| X81365 | 21367 | Cntn2 | axon | cell adh |
| BC012878 | 77034 | | | transcription |
| U70033 | 20541 | Slc8a1 | integral to plasma membrane | transport |
| NM_023377 | 170460 | Stard5 | | transport |
| NM_008020 | 14227 | Fkbp2 | endoplasmic reticulum;extracellu | translation |
| AK019590 | 78154 | | | unknown |
| AK016374 | 75879 | | | unknown |
| AK013948 | 69269 | Scnm1 | | transcription |
| NM_053204 | 111173 | Rab6ip2 | non-muscle myosin | signaling |
| NM_008167 | 14804 | Grid2 | integral to membrane;synaptosome | ion channel |
| AK016480 | | | | unknown |

BNS  BS

EP 1 649 062 B1

## Figure 17 B

Log$_2$(ratio)  -0.3  -0.1  0.1  0.3

| ACCESS | LOCUS | GENE | LOCALIZATION | BIOLOGICAL FUNCTION |
|---|---|---|---|---|
| AK012503 | 72563 | | | unknown |
| NM_018801 | 54525 | Syt7 | integral to membrane;lysosome;sy | transport |
| NM_009352 | 21749 | Terf1 | chromosome-, telomeric region;nu | cell cycle |
| NM_019697 | 16508 | Kcnd2 | extracellular space;voltage-gate | ion channel |
| BC003975 | 30853 | Mif2 | nucleus | tumor related |
| AK017249 | 11842 | Arf3 | Golgi apparatus | transport |
| NM_019948 | 56619 | Clecsf9 | integral to plasma membrane | signaling (receptor) |
| AK013245 | 72748 | | spliceosome complex | transcription |
| NM_008676 | 17966 | Nbr1 | cytosol | unknown |
| NM_026348 | 67733 | | cytoplasm | signaling |
| AK008415 | 67495 | | | unknown |
| NM_009166 | 20411 | Sorbs1 | stress fiber | ctsk |
| AK011184 | 67039 | | cytoskeleton | unknown |
| U85993 | 319845 | | extracellular space | unknown |
| AK009450 | 71904 | | integral to membrane | unknown |
| U43892 | 11306 | Abcb7 | inner membrane;integral to plasm | transport |
| NM_025730 | 66725 | | cytoplasm | unknown |
| NM_009503 | 269523 | Vcp | Golgi apparatus;endoplasmic reti | transport |
| AK016247 | 18640 | Pfkfb2 | cytosol | metabolism |
| NM_026304 | 67669 | | | unknown |
| NM_008288 | 15483 | Hsd11b1 | microsome | metabolism |
| BC017615 | 94249 | Slc24a3 | integral to membrane;plasma memb | transport |
| NM_019410 | 18645 | Pfn2 | actin cytoskeleton | ctsk |
| AK003794 | 97541 | Qars | | translation |
| NM_010946 | 18203 | Ntan1 | cytoplasm;nucleus | misc |
| AK019481 | 68260 | | | unknown |
| AF220355 | 56747 | AI843918 | | unknown |
| NM_013614 | 18263 | Odc | mitochondrial inner membrane | misc |
| NM_009883 | 12608 | Cebpb | cytoplasm;nucleus | apoptosis |
| AK018551 | 107022 | | integral to membrane | unknown |
| NM_011929 | 26372 | Clcn6 | integral to plasma membrane | ion channel |
| AK020859 | 68146 | | | unknown |
| NM_016737 | 20867 | Stip1 | Golgi apparatus;nucleus | misc |
| NM_008983 | 19272 | Ptprk | extracellular space;integral to | signaling (receptor) |
| AK020099 | 68180 | | | unknown |
| NM_008161 | 14778 | Gpx3 | extracellular space | metabolism |
| NM_007702 | 12683 | Cidea | cytosol;mitochondrial membrane | apoptosis |
| AK020410 | 52120 | D8Ertd354e | integral to membrane | unknown |
| NM_011787 | 23802 | Amfr | integral to endoplasmic reticulu | protein degradation ? |
| AK005033 | 52432 | Ppp2r2d | protein phosphatase type 2A comp | signaling |
| AF279458 | 19386 | Ranbp2 | cytoplasm | metabolism |
| AK016267 | 65103 | Arl6ip6 | integral to membrane | unknown |
| AK014235 | 73192 | Xpot | nucleus | traffic |
| NM_011802 | 270166 | Clpx | mitochondrion | transport |
| AK010466 | 76484 | | | unknown |
| NM_007525 | 12021 | Bard1 | cytoplasm;nucleus | transcription |
| AK008080 | 76526 | | spliceosome complex | transcription |
| BC003247 | 229725 | Mclc | | transport |
| AK003866 | 28295 | D10Jhu81e | cell wall;extracellular space;mi | unknown |
| AF060088 | 26440 | Psma1 | membrane;proteasome core complex | protein degradation |
| AK020868 | 68073 | | | unknown |
| AK020780 | 77929 | | | traffic ? |
| AK009497 | 69568 | Vkorc1l1 | | metabolism |
| AF071173 | 15204 | Herc2 | | protein degradation |
| NM_019571 | 56224 | Tm4sf9 | integral to membrane | cell adh |
| AK014134 | 73172 | | extrachromosomal circular DNA | unknown |
| NM_025274 | 13915 | Dppa5 | cytoskeleton | misc |
| NM_019638 | 56449 | Csda | perinuclear space | transcription |
| NM_008591 | 17295 | Met | extracellular space;integral to | signaling (receptor) |
| AK014948 | 66719 | | | unknown |
| NM_009671 | 11736 | Ankfy1 | | transcription |
| AK005944 | 76920 | Arrdc5 | | unknown |
| AF320615 | | | | unknown |

BNS   BS

Figure 18 A

Log$_2$(ratio)

-0.3  -0.1  0.1  0.3

| ACCESS | LOCUS | GENE | LOCALIZATION | BIOLOGICAL FUNCTION |
|---|---|---|---|---|
| NM_010451 | 15399 | Hoxa2 | transcription factor complex | transcription |
| NM_008883 | 18846 | Plxna3 | extracellular space;integral to | signaling (receptor) |
| AK018953 | 68214 | | cytoplasm | metabolism ? |
| AK014752 | 75768 | | | unknown |
| NM_021325 | 57781 | Mox2r | integral to membrane | signaling (receptor) |
| AK009205 | | | | unknown |
| AK008045 | 76952 | Nt5c2 | cytosol | protein degradation |
| NM_025806 | 66857 | | extracellular space | unknown |
| NM_011010 | 18378 | Omp | cytosol | signaling |
| AK004627 | 74119 | | | unknown |
| NM_021483 | 58869 | Pex2 | peroxisomal membrane | unknown |
| NM_011577 | 21803 | Tgfb1 | extracellular matrix;extracellul | growth factor |
| NM_027142 | 69623 | | | unknown |
| AK006173 | 75629 | | | signaling |
| NM_016744 | 18573 | Pde1a | insoluble fraction | metabolism |
| AK006807 | 74271 | | | unknown |
| NM_009788 | 12307 | Calb1 | cytoplasm | misc |
| NM_007404 | 11502 | Adam9 | extracellular space;integral to | protein degradation |
| AK005035 | 71775 | | extracellular space | transport |

BNS
BS

EP 1 649 062 B1

106

Figure 18 B

-0.3  -0.1  0.1  0.3
Log$_2$(ratio)

| ACCESS | LOCUS | GENE | LOCALIZATION | BIOLOGICAL FUNCTION |
|---|---|---|---|---|
| NM_008971 | 19230 | Ptk9 | actin cytoskeleton | ctsk |
| NM_008300 | 15525 | Hspa4 | cytoplasm | translation |
| NM_011341 | 20318 | Sdf4 | Golgi apparatus;extracellular sp | misc |
| NM_008946 | 19175 | Psmb6 | proteasome core complex (sensu E | protein degradation |
| D29797 | 20908 | Stx3 | integral to membrane | transport |
| NM_009377 | 21823 | Th | ornithine carbamoyltransferase c | metabolism |
| NM_009729 | 11984 | Atp6v0c | hydrogen-translocating V-type AT | transport |
| NM_007917 | 13684 | Eif4e | cytoplasm | translation |
| NM_007761 | 12909 | Crcp | | signaling (receptor) |
| AK013595 | 72871 | | | unknown |
| NM_026580 | 68149 | | | protein degradation |
| NM_010727 | 16924 | Lnx1 | cytoplasm;extracellular space | signaling |
| AK018159 | 18549 | Pcsk2 | Golgi apparatus;extracellular sp | translation |
| AK004506 | 68942 | | | unknown |
| NM_026434 | 67889 | | viral nucleocapsid | unknown |
| NM_011990 | 26570 | Slc7a11 | integral to membrane | transport |
| NM_008903 | 19012 | Ppap2a | integral to plasma membrane;memb | metabolism |
| NM_025962 | 67096 | | membrane | unknown |
| NM_007731 | 12817 | Col13a1 | collagen type XIII | ecm |
| NM_009819 | 12386 | Catna2 | cytoskeleton | cell adh |
| NM_023305 | 67123 | Ubap1 | 26S proteasome | protein degradation |
| NM_008235 | 15205 | Hes1 | transcription factor complex | transcription |
| BC015273 | 110157 | Raf1 | cytosol;plasma membrane | signaling |
| AK003278 | 52466 | D11Ertd18e | integral to membrane | transport |
| NM_030717 | 80907 | Lactb | extracellular space;integral to | misc |
| NM_010769 | 17180 | Matn1 | extracellular matrix;extracellul | ecm |
| BC015253 | 11688 | Alox15b | | immune response |
| AK010382 | 243983 | | actin cytoskeleton;integral to m | transcription |
| BC003451 | 232087 | Mat2a | cytoplasm | metabolism |
| NM_011917 | 24128 | Xrn2 | nucleus | transcription |
| AK013309 | 170707 | Usp31 | | protein degradation |
| AK013166 | 67607 | | | unknown |
| AK003542 | 73728 | | mitochondrial inner membrane | unknown |
| AK017484 | 67023 | | mitochondrial matrix | transcription |
| NM_025779 | 66815 | | integral to membrane | unknown |
| AK016786 | 71804 | | ribosome | unknown |
| NM_011626 | 21982 | Tparl | extracellular space;integral to | unknown |
| NM_009421 | 22029 | Traf1 | cytoplasm | signaling |
| AC079832 | 286947 | Adh6b | cytosol ? | metabolism ? |
| AK021152 | 77643 | | | unknown |
| BC016073 | 68916 | | | unknown |
| NM_025808 | 66863 | Lztr1 | | transcription |
| NM_025566 | 66443 | | | unknown |
| NM_022418 | 64295 | | extracellular space;integral to | unknown |
| NM_008974 | 19244 | Ptp4a2 | soluble fraction | signaling |
| NM_007508 | 11964 | Atp6v1a1 | integral to plasma membrane;prot | metabolism |
| NM_011018 | 18412 | Sqstm1 | cytosol | protein degradation |
| NM_008617 | 17448 | Mdh2 | mitochondrial matrix | metabolism |
| NM_007699 | 12672 | Chrm4 | integral to plasma membrane | signaling (receptor) |
| NM_013867 | 29815 | Bcar3 | actin filament | signaling (GEF) |
| BC017130 | 224938 | Pja2 | | unknown |
| AK008333 | 70257 | | mitochondrion | unknown |
| NM_010250 | 14394 | Gabra1 | extracellular space;integral to | signaling |

BNS
BS

Figure 19

Log₂(ratio)  -0.3  -0.1  0.1  0.3

| ACCESS | LOCUS | GENE | LOCALIZATION | BIOLOGICAL FUNCTION |
|---|---|---|---|---|
| NM_010991 | 18348 | Olfr49 | integral to plasma membrane | signaling (receptor) |
| NM_010595 | 16485 | Kcna1 | voltage-gated potassium channel | transport |
| NM_025708 | 66690 | | | unknown |
| AK013948 | 69269 | Scnm1 | | transcription |
| NM_023721 | 73834 | Atp6v1d | hydrogen-transporting ATPase V1 | Transport |
| AK012159 | 72542 | | extracellular space | metabolism |
| NM_010412 | 15184 | Hdac5 | cytoplasm;histone deacetylase co | cell cycle |
| NM_008585 | 17287 | Mep1a | extracellular space;integral to | protein degradation (secreted) |
| NM_011917 | 24128 | Xrn2 | nucleus | transcription |
| NM_007889 | 13544 | Dvl3 | cytoplasm | signaling |
| NM_010661 | 268482 | Krt1-12 | intermediate filament | ctsk |
| NM_009634 | 11564 | Adsl | ribosome | metabolism |
| AK011747 | 17237 | Mgrn1 | extracellular | apoptosis |
| NM_023217 | 66522 | | cytoplasm | metabolism ? |
| NM_011049 | 18555 | Pctk1 | cytoplasm | misc |
| AK007822 | 107747 | Fthfd | | metabolism |
| AK003819 | 74777 | | | misc |
| AF100956 | | a lot | | unknown |
| AK005588 | 69317 | | chromatin;nucleus | transcription |
| AK020471 | 12631 | Cfl1 | cytoskeleton;nucleus | ctsk |
| NM_009128 | 20250 | Scd2 | endoplasmic reticulum;integral t | metabolism |
| NM_008707 | 18107 | Nmt1 | cytoplasm | misc |
| AK016685 | 74443 | | endoplasmic reticulum | metabolism |
| AK014142 | 67287 | | | unknown |
| NM_011585 | 21841 | Tia1 | lysosome;nucleus | apoptosis |
| AK011881 | 67148 | | microtubule | ctsk |
| AK002704 | 68365 | Rab14 | | signaling |
| NM_026670 | 68310 | | integral to membrane | unknown |
| NM_013892 | 30052 | Pcsk1n | Golgi trans face;extracellular s | misc |
| NM_007427 | 11604 | Agrp | extracellular space | signaling (secreted) |
| AK005887 | 69354 | Slc38a4 | integral to membrane | transport |
| NM_011623 | 21973 | Top2a | condensed chromosome;metaphase c | cell cycle |
| NM_019768 | 56397 | Morf4l2 | chromatin;nucleus | transcription |
| NM_010377 | 107970 | Hist1h1t | nucleosome;nucleus | cell cycle |
| AK016475 | 70981 | | | unknown |
| AK020837 | 77799 | Sla2 | cytoplasmic vesicle;late endosom | signaling |
| NM_054046 | 23854 | Defβ | extracellular | signaling |
| NM_008401 | 16409 | Itgam | cytoskeleton;external side of pl | cell adh (receptor) |
| NM_053074 | 18226 | Nup62 | nuclear pore | transport |
| NM_010278 | 14581 | Gfi1 | transcription factor complex | transcription |
| NM_013932 | 30959 | Ddx25 | | translation |
| NM_053204 | 111173 | Rab6lp2 | non-muscle myosin | signaling |
| AB055154 | 108138 | Xrcc4 | extracellular;nucleus | cell cycle |
| NM_010242 | 14345 | Fut4 | Golgi apparatus;integral to memb | translation |
| AF411834 | 20620 | Snk | phosphorylase kinase complex | cell cycle |
| NM_009748 | 12068 | Bet1 | Golgi membrane;endoplasmic retic | transport |
| AK009343 | 71907 | Serpina9 | extracellular space | protein degradation |
| NM_009147 | 20334 | Sec23a | Golgi membrane;endoplasmic retic | traffic |
| NM_009314 | 21337 | Tacr2 | integral to membrane;plasma memb | signaling (receptor) |
| NM_025323 | 66055 | | nucleus | transcription |
| NM_009873 | 12571 | Cdk6 | cytoplasm | cell cycle |
| NM_025520 | 66373 | Lsm5 | | transcription |
| NM_009213 | 20598 | Smpd2 | integral to membrane | lipid metabolism |
| NM_025714 | 52184 | D3Ertd250e | myosin | unknown |
| NM_008890 | 18948 | Pnmt | | metabolism |
| NM_023876 | 77766 | Elp4 | extracellular | unknown |
| AK019600 | 78795 | | transcription factor TFIID compl | unknown |
| NM_011962 | 26433 | Plod3 | endoplasmic reticulum;extracellu | metabolism |
| NM_009278 | 20823 | Ssb | nucleus;viral nucleocapsid | transcription |
| AK004757 | 71728 | | cytoplasm | signaling |
| AF177211 | 140795 | Gpr105 | integral to membrane | signaling (receptor) |
| AK016509 | 71003 | | extracellular space | protein degradation |
| AK016282 | 75863 | | | unknown |
| AK016517 | 75725 | | | unknown |

BNS  BS

Log$_2$(ratio)
-0.4  0.0  0.2  0.4

## Figure 20

| ACCESS | LOCUS | GENE | LOCALIZATION | BIOLOGICAL FUNCTION |
|---|---|---|---|---|
| NM_011522 | 20974 | Syngr3 | integral to plasma membrane | unknown |
| AK015916 | 73866 | | ribosome | translation? |
| NM_021888 | 60507 | Qtrt1 | | misc |
| NM_080639 | 110595 | Timp4 | extracellular matrix;extracellul | protein degradation |
| NM_013859 | 29805 | ORF6 | | unknown |
| AK009163 | 76730 | | | unknown |
| AK008201 | 69875 | Ndufa11 | | misc |
| AK004813 | 71729 | Rgs12 | nucleus | signaling |
| Y17793 | 19876 | Robo1 | extracellular space | ecm? |
| AK020687 | 78083 | | | unknown |
| AK018115 | 74007 | Btbd11 | nucleus? | transcription |
| NM_012029 | 26940 | Sitpec | transcription factor complex | transcription |
| BC004680 | 13052 | Cxadr | extracellular space;integral to | signaling (receptor) |
| AK015650 | 67509 | | | unknown |
| NM_010157 | 13983 | Esr2 | intermediate filament;nucleus | signaling (receptor) |
| NM_008373 | 16198 | Il9 | extracellular space | immune response (secreted) |
| AF121976 | 57250 | Ors19 | integral to membrane | signaling (receptor) |
| NM_026580 | 68149 | | | protein degradation |
| NM_025714 | 52184 | D3Ertd250e | myosin | unknown |
| NM_018802 | 55925 | Syt8 | integral to membrane;synaptic ve | transport |
| AK003232 | 109857 | Cbr3 | cytosol | metabolism |
| AF078112 | 12328 | Caml? | integral to membrane? | signaling? |
| NM_007836 | 13197 | Gadd45a | nucleus;ribosome | cell cycle |
| AK019665 | 78174 | | mitochondrion | metabolism |
| BC004761 | 233066 | AI428936 | | unknown |
| NM_023873 | 68121 | | cytoplasm | cell cycle |
| AK007678 | 69185 | | | unknown |
| AK015086 | 73809 | | | unknown |
| NM_025896 | 67000 | | extracellular space | growth factor |
| AK017577 | 74741 | | | traffic |
| AK017491 | 70478 | Mipep | mitochondrion | metabolism |
| NM_016751 | 51811 | Clecsf13 | integral to membrane | cell adh |
| NM_011165 | 19110 | Prlpa | extracellular space | growth factor (secreted) |
| AK004650 | 67451 | | cytoskeleton | protein degradation |
| NM_026433 | 67888 | | integral to membrane | unknown |

BNS  BS

EP 1 649 062 B1

109

Figure 21

-0.4   0.0  0.2  0.4
Log$_2$(ratio)

| ACCESS | LOCUS | GENE | LOCALIZATION | BIOLOGICAL FUNCTION |
|--------|-------|------|--------------|---------------------|
| NM_033652 | 110648 | Lmx1a | nucleus | transcription |
| NM_007900 | 13605 | Ect2 | cytoplasmic vesicle | signaling (receptor) |
| NM_025383 | 66147 | | membrane | unknown |
| NM_021527 | 59030 | Mkks | chaperonin-containing T-complex | translation |
| AK021023 | 78688 | Nol3 | nucleus | apoptosis |
| AK002637 | 66689 | Btbd5 | actin cytoskeleton | ctsk ? |
| BC005774 | | | | unknown |
| AK008338 | 69894 | | integral to membrane | unknown |
| NM_009440 | 22127 | Tsx | | unknown |
| NM_007863 | 13384 | Mpp3 | integral to plasma membrane | signaling |
| NM_030262 | 80294 | | | unknown |
| NM_019914 | 56772 | | | unknown |
| NM_018872 | 56030 | D1Bwg0491e | cytoplasm;integral to membrane | unknown |
| NM_008453 | 16599 | Klf3 | transcription factor complex | transcription |
| NM_013865 | 29812 | Ndr3 | cytoplasm | misc |
| NM_011946 | 26405 | Map3k2 | cytoplasm | signaling (MAPK) |
| AK010202 | 66968 | | Golgi apparatus | unknown |
| NM_053204 | 111173 | Rab6ip2 | non-muscle myosin | signaling |
| NM_011909 | 24110 | Usp18 | 26S proteasome | protein degradation |
| AK005139 | 68262 | Agpat4 | integral to membrane | lipid metabolism |
| NM_013718 | 27096 | Trappc3 | Golgi apparatus;endoplasmic reti | transport |
| AF408435 | 233406 | Prc1 | spindle | cell cycle |
| L34676 | 11784 | Apba2 | | signaling |
| AK018132 | 74008 | | lysosome | signaling |
| AK017528 | 69227 | | | unknown |
| NM_008121 | 14613 | Gja5 | connexon complex;integral to mem | signaling |
| NM_007833 | 13179 | Dcn | extracellular matrix;extracellul | Signaling (receptor) |
| NM_019808 | 56376 | | Z disc | ctsk |
| AK013727 | 14397 | Gabra4 | integral to plasma membrane | signaling |
| NM_009746 | 12055 | Bcl7c | extrachromosomal circular DNA | tumor related |
| NM_021898 | 60600 | Halapx | cytoplasm;nucleus | cell cycle |
| AK012624 | 72621 | | plasma membrane | signaling |
| NM_025457 | 66268 | | | unknown |
| NM_026645 | 68265 | | cytoplasm | misc |
| NM_026526 | 68043 | | ribulose bisphosphate carboxylas | unknown |
| NM_007948 | 13870 | Ercc1 | nucleotide excision repair compl | cell cycle |
| AK015452 | 75796 | Cdyl2 | chromatin;nucleus | misc |
| AK004009 | 54151 | Cyhr1 | cytoplasm | misc |

BNS  BS

EP 1 649 062 B1

## Figure 22

Log$_2$(ratio): -0.3  -0.1  0.1  0.3

| ACCESS | LOCUS | GENE | LOCALIZATION | BIOLOGICAL FUNCTION |
|---|---|---|---|---|
| NM_009282 | 20842 | Stag1 | nucleus | cell cycle |
| AK003287 | 66264 | | | unknown |
| AK006533 | 69519 | Rwdd2 | | unknown |
| NM_029636 | 104002 | Ctsq | | protein degradation |
| NM_026510 | 68017 | | | unknown |
| NM_026015 | 67178 | | nucleus | unknown |
| NM_025658 | 66607 | Ms4a4d | integral to membrane | immune response |
| AB041576 | 58242 | Nudt11 | | unknown |
| AK008574 | 67939 | | membrane | transport? |
| NM_024255 | 72479 | | mitochondrion | metabolism |
| NM_007686 | 12630 | Cfi | extracellular space;membrane | immune response |
| X98848 | 18639 | Pfkfb1 | | metabolism |
| NM_020506 | 57258 | Xpo4 | RAN-binding protein;nucleus | traffic |
| NM_025282 | 17260 | Mef2c | mediator complex | transcription |
| NM_013844 | 24135 | Zfp68 | transcription factor complex | transcription |
| NM_019929 | 20610 | Smt3h1 | | cell cycle |
| BC004764 | 56690 | Mlycd | mitochondrion;peroxisome | metabolism |
| NM_011893 | 24055 | Sh3bp2 | cytoplasm | signaling |
| NM_021505 | 59008 | Anapc5 | | cell cycle |
| NM_007633 | 12447 | Ccne1 | nuclear cyclin-dependent protein | cell cycle |
| AK012097 | 72155 | | | unknown |
| NM_027910 | 71765 | Klhdc3 | endosome;nuclear chromatin | unknown |
| AK016520 | 71745 | Cul2 | nuclear ubiquitin ligase complex | tumor related - apoptosis - cell cycle |
| NM_013641 | 19216 | Ptger1 | integral to membrane | signaling |
| AK006273 | 69441 | | | unknown |
| NM_007733 | 12823 | Col19a1 | collagen;extracellular space | ecm |

BNS  BS

EP 1 649 062 B1

Figure 23 A

-0.4  0.0  0.2  0.4

Log$_2$(ratio)

BNS BS

| ACCESS | LOCUS | GENE | LOCALIZATION | BIOLOGICAL FUNCTION |
|---|---|---|---|---|
| NM_025594 | 66492 | | ribosome | unknown |
| NM_023788 | 75625 | | plasma membrane | apoptosis |
| NM_019831 | 56364 | Zfp261 | integral to membrane | unknown |
| NM_026908 | 69008 | | | unknown |
| NM_008442 | 16563 | Kif2a | kinesin complex | ctsk |
| NM_012047 | 26992 | Brd7 | cytoplasm | transcription |
| AK014029 | 73068 | Fut11 | extracellular space;membrane | translation |
| NM_026312 | 67683 | | cytoplasm | unknown |
| AK007546 | 110052 | Dek | nucleus | transcription |
| AK009375 | 52685 | D11Ertd736e | integral to membrane | unknown |
| AF213381 | 27406 | Abcf3 | integral to plasma membrane | transport |
| NM_019679 | 57778 | Fmnl | cytosol | ctsk |

**Figure 23 B**

−0.4 0.0 0.2 0.4
Log$_2$(ratio)

| ACCESS | LOCUS | GENE | LOCALIZATION | BIOLOGICAL FUNCTION |
|---|---|---|---|---|
| BC005741 | 233835 | Tnrc6 | extracellular | misc |
| NM_019509 | | | | unknown |
| AB047921 | 67210 | | nucleus | transcription? |
| D86949 | 18845 | Plxna2 | extracellular space;integral to | signaling |
| AJ288061 | 76707 | Clasp1 | cytoplasmic microtubule;microtub | ctsk |
| NM_009864 | 12550 | Cdh1 | cell-cell adherens junction;extr | cell adh |
| BC004626 | 72722 | | membrane | misc |
| NM_021452 | 58802 | Kcnmb4 | integral to plasma membrane | ion channel |
| AK019985 | 77521 | | non-muscle myosin | signaling |
| NM_019635 | 56274 | Stk3 | cytoplasm;protein kinase CK2 com | apoptosis |
| AK019434 | 28040 | D6Wsu163e | | unknown |
| AK007999 | 69815 | | extracellular space;integral to | unknown |
| AK002416 | 68350 | | integral to membrane;lateral pla | transport? |
| NM_008078 | 14417 | Gad2 | extrachromosomal circular DNA | metabolism |
| AK017149 | 75964 | | | unknown |
| NM_021560 | 59058 | Bhlhb5 | transcription factor complex | transcription |
| NM_018812 | 229615 | Pias3 | nucleus | transcription |
| NM_025855 | 52665 | Echdc1 | mitochondrion | unknown |
| AK020702 | 78428 | | | unknown |
| NM_033327 | 94187 | Ebfaz | transcription factor complex | transcription |
| NM_018764 | 54216 | Pcdh7 | extracellular space;integral to | cell adh |
| AK018327 | 76206 | | integral to membrane | unknown |
| AK005069 | 71767 | | | protein degradation? |
| NM_013638 | 19120 | Prm3 | nucleosome;nucleus | cell cycle |
| NM_011500 | 268980 | Strn | cytoplasm | transcription |
| AK016466 | 70965 | | | signaling |
| NM_027019 | 69287 | Odf3 | extracellular space;outer dense | signaling (secreted) |
| BC004768 | | | | unknown |
| AK013464 | 67515 | | protein serine-threonine phospha | signaling (osmosis response) |
| AK006014 | 69428 | | | unknown |
| NM_019551 | 56196 | Ttrap | cytoplasm | signaling |
| AK006617 | 73284 | Ddit4l | membrane | immune response? |
| NM_022995 | 65112 | | integral to membrane | misc |
| NM_031261 | 83457 | Fthl17 | ferritin complex | misc |
| NM_009748 | 12068 | Bet1 | Golgi membrane;endoplasmic retic | transport |
| BC004766 | 71799 | Ptcd1 | ribosome | unknown |
| AF105217 | 57813 | Tk2 | mitochondrion | metabolism |
| AK004943 | 66627 | | endoplasmic reticulum | unknown |
| NM_026413 | 67854 | Slco6b1 | integral to membrane | transport |
| X74504 | 27883 | D16H22S680E | chromatin | unknown |
| NM_009390 | 21892 | Tll | extracellular space | protein degradation (secreted) |
| NM_007614 | 12387 | Catnb | adherens junction;basolateral pl | cell adh |
| NM_025315 | 108098 | Surb7 | DNA-directed RNA polymerase II-, | transcription |
| NM_022331 | 64209 | Herpud1 | endoplasmic reticulum membrane;i | signaling? |
| BC003235 | 71665 | Fuca | lysosome | metabolism |
| AK018594 | 68145 | | | misc |
| AK005015 | 246277 | Csad | | metabolism |
| NM_011156 | 19072 | Prep | cytoplasm;nucleus | protein degradation |
| AK009362 | 99650 | | | unknown |
| NM_018872 | 56030 | D1Bwg0491e | cytoplasm;integral to membrane | unknown |
| NM_030684 | 94094 | Trim34 | cytoplasm | unknown |
| AJ006215 | 12764 | Cmas | cytoplasm;nucleus | metabolism |
| NM_008839 | 18706 | Pik3ca | phosphoinositide 3-kinase comple | apoptosis |

BNS
BS

-0.3  -0.1  0.1  0.3

Log$_2$(ratio)

## Figure 24

| ACCESS | LOCUS | GENE | LOCALIZATION | BIOLOGICAL FUNCTION |
|---|---|---|---|---|
| NM_007863 | 13384 | Mpp3 | integral to plasma membrane | signaling |
| AK014582 | 70807 | Arrdc2 | | unknown |
| NM_025994 | 27984 | D4Wsu27e | | unknown |
| AK016374 | 75879 | | | unknown |
| NM_011801 | 23837 | Cfdp | basement membrane;integral to me | apoptosis |
| AK005888 | 69324 | | | unknown |
| BC004604 | 104318 | Csnk1d | cytoplasm | signaling |
| NM_008707 | 18107 | Nmt1 | cytoplasm | misc |
| NM_007627 | 12426 | Cckbr | integral to membrane | signaling (receptor) |
| NM_013569 | 16511 | Kcnh2 | membrane fraction;voltage-gated | ion channel |
| NM_007458 | 11771 | Ap2a1 | AP-2 adaptor complex;clathrin co | transport |
| NM_008841 | 18709 | Pik3r2 | phosphoinositide 3-kinase comple | signaling |
| AF133300 | 259102 | Olfr630 | | signaing (receptor) |
| AB056100 | 66902 | Mtap | | misc |
| AB039178 | | | | unknown |
| NM_016813 | 53319 | Nxf1 | nuclear pore | transport |
| NM_025706 | 66687 | Tbc1d15 | extrachromosomal circular DNA | unknown |
| NM_026217 | 67526 | Apg12l | cytoplasm | protein degradation |
| NM_011857 | 23965 | Odz3 | integral to plasma membrane | unknown |
| AK007831 | 68115 | | | unknown |
| AK015402 | 74862 | | | unknown |
| NM_023649 | 72088 | Ush1c | cilium | protein degradation |
| NM_019512 | 56070 | Tcerg1 | nuclear matrix | transcription |
| AK016000 | 56428 | Mtch2 | membrane | Transport |
| AK007097 | 14051 | Eya4 | cytoplasm | metabolism |
| NM_010765 | 17165 | Mapkapk5 | calcium-calmodulin-dependent pro | signaling |
| AK013003 | 67610 | | membrane | transport |
| NM_022995 | 65112 | | integral to membrane | misc |
| NM_013617 | 18366 | Olfr65 | integral to plasma membrane | signaling (receptor) |
| AK016865 | 71164 | Zdhhc20 | | unknown |
| AK017531 | 73998 | Herc3 | | unknown |
| NM_053190 | 94226 | Edg8 | integral to membrane;plasma memb | signaling (receptor) |
| AK014863 | 70853 | | | unknown |
| NM_019443 | 54405 | Ndufa1 | extracellular space;mitochondrio | metabolism |
| NM_009131 | 20256 | Scgf | extracellular space | signaling (secreted) |
| AK006889 | 73410 | | | unknown |
| AK008988 | 70285 | | | translation |
| NM_008171 | 14812 | Grin2b | extracellular space;integral to | Signaling (receptor) |
| AK009957 | 69692 | | | unknown |
| NM_033561 | 22384 | Wbscr1 | cytosol | translation |
| NM_009437 | 22117 | Tst | mitochondrial matrix | metabolism |
| NM_011104 | 18754 | Prkce | cytoskeleton | signaling |
| NM_008731 | 18167 | Npy2r | integral to plasma membrane | signaling (body weight) |
| AK020702 | 78428 | | | unknown |
| AK003223 | 68563 | Dpm3 | endoplasmic reticulum;integral t | metabolism |
| NM_029716 | 108699 | Chn1 | cytoplasm | signaling |
| NM_026235 | 67557 | | nucleus;viral nucleocapsid | unknown |
| NM_022813 | 24044 | Scamp2 | integral to membrane;membrane fr | transport |
| NM_009171 | 20425 | Shmt1 | cytosol | metabolism |
| NM_021531 | 59035 | Carm1 | viral nucleocapsid | transcription |
| AK019147 | 19084 | Prkar1a | cAMP-dependent protein kinase co | misc |
| NM_021446 | 58520 | ORF11 | integral to membrane | unknown |
| AK004703 | 71733 | Susd2 | extracellular space;integral to | cell adh |
| AK016990 | 74481 | | nucleus | transcription |
| NM_018887 | 56050 | Cyp39a1 | endoplasmic reticulum;membrane;m | metabolism |

BNS  BS

Figure 25

Log$_2$(ratio) scale: -0.2  0.0  0.2

| ACCESS | LOCUS | GENE | LOCALIZATION | BIOLOGICAL FUNCTION |
|---|---|---|---|---|
| NM_023893 | | | | unknown |
| AF100956 | | a lot | | unknown |
| NM_010321 | 14711 | Gnmt | endoplasmic reticulum | translation |
| NM_022992 | 65106 | Arl6ip5 | integral to membrane | transport |
| NM_007682 | 12616 | Cenpb | chromosome-, pericentric region; | misc |
| AK003511 | 73724 | Mcee | extracellular space;mitochondrio | unknown |
| NM_022022 | 63958 | Ube4b | cytoplasm;ubiquitin ligase compl | protein degradation |
| M69293 | 15902 | Idb2 | cytoplasm;transcription factor c | immune response |
| NM_026437 | 67894 | | extracellular | unknown |
| NM_013481 | 12181 | Bop1 | extrachromosomal circular DNA | transcription |
| NM_011936 | 26383 | Fto | | unknown |
| NM_008129 | 14630 | Gclm | | misc |
| AK005311 | 66871 | Cpne8 | membrane | misc |
| AK013604 | 72880 | | | unknown |
| NM_008142 | 14688 | Gnb1 | heterotrimeric G-protein complex | signaling |
| NM_029203 | 75199 | | transcription factor complex | transcription |
| NM_022882 | 64898 | Lpin2 | nucleus | unknown |
| NM_021401 | 58210 | Sectm1 | integral to plasma membrane | immune response |
| AK016247 | 18640 | Pfkfb2 | cytosol | metabolism |
| NM_013826 | 17434 | Mocs2 | | misc |
| NM_009624 | 11515 | Adcy9 | integral to plasma membrane | misc |
| AK010898 | 72320 | | | unknown |
| BC002306 | 245841 | Polr2h | DNA-directed RNA polymerase I co | transcription |
| AK019803 | 78217 | | | unknown |
| NM_019639 | 22190 | Ubc | nucleus | protein degradation |
| AK019985 | 77521 | | non-muscle myosin | signaling |
| NM_018776 | 54394 | Crlf3 | membrane | signaling (receptor) |
| AK014408 | 74315 | | | tumor related |
| NM_023336 | 67382 | Brd3 | integral to membrane;nucleus | signaling ? |
| AK005740 | 74215 | | | protein degradation |
| AK006539 | 27660 | | | unknown |
| NM_026616 | 68209 | | ribosome | unknown |
| NM_009237 | 20675 | Sox3 | transcription factor complex | transcription |
| AK017761 | 70619 | | | unknown |
| NM_013584 | 16880 | Lifr | extracellular space;integral to | signaling (receptor) |
| BC013471 | 77056 | | integral to membrane | lipid metabolism ? |
| AK009886 | 71923 | | | unknown |
| NM_011601 | 21893 | Tlm | extracellular;integral to membra | tumor related |
| AK007994 | 72042 | Cotl1 | cytoskeleton | ctsk |
| AK008761 | 74257 | Fbxo23 | integral to membrane | unknown |
| AF285590 | 73679 | Tex19 | | unknown |
| NM_031156 | 15925 | Ide | extracellular space;peroxisome;s | protein degradation |
| NM_008443 | 16568 | Kif3a | kinesin complex | ctsk |
| AK005989 | 71853 | Txndc7 | endoplasmic reticulum;extracellu | metabolism |
| NM_019657 | 56348 | Hsd17b12 | integral to membrane;microsome | metabolism |
| L38281 | 16365 | Irg1 | | unknown |
| BC013800 | 67063 | | | unknown |
| NM_025529 | 66387 | Nudt8 | mitochondrion | unknown |
| AB047323 | | | | unknown |
| NM_020558 | 57316 | C1d | transcriptional repressor comple | transcription |
| AK015921 | 75209 | Sv2c | synaptic vesicle | misc |
| NM_008852 | 18742 | Pitx3 | transcription factor complex | transcription |
| NM_007637 | 12465 | Cct5 | chaperonin-containing T-complex | translation |
| NM_013666 | 225742 | Slat8e | Golgi apparatus;integral to memb | translation |
| NM_009155 | 20363 | Sepp1 | extracellular space | misc |

BNS  BS

-0.4  0.0 0.2  0.4
Log$_2$(ratio)

## Figure 26

| ACCESS | LOCUS | GENE | LOCALIZATION | BIOLOGICAL FUNCTION |
|---|---|---|---|---|
| NM_016849 | 54131 | Irf3 | cytoplasm;nucleus | transcription |
| NM_008082 | 14427 | Gatr1 | integral to membrane;plasma memb | signaling (receptor) |
| NM_008129 | 14630 | Gclm | | misc |
| AK017270 | 237943 | | | unknown |
| AK012969 | 74200 | | ribulose bisphosphate carboxylas | unknown |
| NM_013895 | 30055 | Timm9 | mitochondrial inner membrane pre | transport |
| AK011544 | 66254 | | | transcription |
| NM_008923 | 19085 | Prkar1b | cAMP-dependent protein kinase co | signaling |
| AK016397 | 66362 | | | transcription |
| NM_023153 | 66070 | | membrane | unknown |
| NM_007638 | 12468 | Cct7 | cytoplasm | translation |
| NM_008855 | 18751 | Prkcb | | signaling |
| BC002307 | 27878 | D1Ertd251e | | transcription |
| NM_008885 | 18858 | Pmp22 | extracellular space;integral to | cell cycle |
| AK008803 | 72281 | Sh2d4a | | signaling ? |
| NM_021537 | 59041 | Stk25 | | signaling |
| AK003653 | 68644 | Dorz1 | extracellular space | metabolism |
| NM_033269 | 12671 | Chrm3 | integral to plasma membrane | signaling (receptor) |
| NM_016757 | 22377 | Wbp1 | integral to membrane | unknown |
| NM_009861 | 12540 | Cdc42 | filopodium | signaling |
| NM_025748 | 66757 | Deadc1 | mitochondrion | misc |
| AK002297 | 71667 | | | unknown |
| NM_029583 | | | | unknown |
| AK007130 | 73582 | | | unknown |
| NM_026253 | 67580 | | cytoplasm | unknown |
| NM_021550 | 59048 | | integral to membrane | unknown |
| NM_019572 | 56233 | Hdac7a | cytoplasm;histone deacetylase co | transcription |
| NM_009632 | 11546 | Adprtl2 | nucleus;telomere | misc |
| AK006145 | 75642 | | integral to membrane | unknown |
| AK005166 | 76500 | | | signaling |
| AK006807 | 74271 | | | unknown |
| NM_030716 | 80906 | Kcnip2 | plasma membrane | ion channel |
| AK016622 | 74438 | | | unknown |
| AK014709 | 74585 | 4833416I09Rik | | unknown |
| AK013202 | 20467 | Sin3b | nucleus | transcription |
| NM_019686 | 56506 | | synaptic vesicle | signaling |
| NM_025747 | 66756 | | | unknown |
| NM_011597 | 21873 | Tjp2 | nucleus;tight junction | cell adh |
| NM_021453 | 58803 | | extracellular space;soluble frac | protein degradation (secreted) |
| NM_009781 | 12288 | Cacna1c | T-tubule;voltage-gated calcium c | ion channel |
| AK011832 | 72465 | Zfp131 | | transport |
| NM_028075 | 72049 | Tnfrsf13c | integral to membrane | signaling (receptor) |
| AK013280 | 67229 | | spliceosome complex | transcription |
| NM_019550 | 56195 | Ptbp2 | spliceosome complex | transcription |
| AK003818 | 66248 | Alg5 | endoplasmic reticulum;integral t | metabolism |
| AK006263 | 54613 | Siat10 | Golgi apparatus;membrane | misc |
| NM_053161 | 94064 | Mrpl27 | mitochondrial large ribosomal su | translation |
| AK005160 | 72014 | | extracellular space | unknown |
| AK013175 | 28027 | D4Wsu132e | | unknown |
| NM_026612 | 68198 | Ndufb2 | mitochondrion | transport |
| AK007552 | 56036 | Ccnl2 | | cell cycle |
| NM_025937 | 67050 | | integral to endoplasmic reticulu | unknown |
| NM_007889 | 13544 | Dvl3 | cytoplasm | signaling |
| AF104414 | 16798 | Lats1 | | signaling ? |
| NM_029417 | 75757 | | | unknown |
| AK002744 | 68375 | Ndufa8 | respiratory chain complex I (sen | metabolism |
| BC002178 | 93747 | Echs1 | mitochondrion | metabolism |
| NM_016798 | 53318 | Pdlim3 | Z disc;actin cytoskeleton | ctsk |
| NM_008040 | 14290 | Fpr-rs3 | integral to membrane | signaling (receptor) |
| NM_013859 | 29805 | ORF6 | | unknown |
| BC012407 | 110695 | Aldh7a1 | | metabolism |

BNS  BS

Figure 27

-0.4 0.0 0.2 0.4

Log$_2$(ratio)

| ACCESS | LOCUS | GENE | LOCALIZATION | BIOLOGICAL FUNCTION |
|---|---|---|---|---|
| NM_022321 | 64099 | Parvg | cytoskeleton | cell adh |
| AK009960 | 69697 | | | unknown |
| NM_021531 | 59035 | Carm1 | viral nucleocapsid | transcription |
| AK018599 | 22353 | Vip | extracellular space | signaling (secreted) |
| NM_026175 | 67465 | Sf3a1 | spliceosome complex | transcription |
| AK019538 | 78092 | | integral to membrane | unknown |
| X84014 | 16774 | Lama3 | basal lamina;mitochondrial inner | cell adh |
| AK009365 | 76437 | D11Bwg0414e | | unknown |
| NM_011543 | 21402 | Skp1a | SCF ubiquitin ligase complex;nuc | translation |
| AK007452 | 69082 | | | signaling |
| AK012711 | 69226 | Srnx24 | cytoplasm | transport |
| AK003808 | 224250 | | integral to membrane | unknown |
| NM_013736 | 27224 | Tceb3 | nucleus | transcription |
| NM_011288 | 19935 | Mrpl23 | mitochondrial large ribosomal su | translation |
| AK015106 | 106039 | Gga1 | Golgi stack | transport |
| AK003337 | 68490 | | | unknown |
| AF061934 | 50782 | Rgs11 | heterotrimeric G-protein complex | signaling |
| AK017628 | 70556 | | mitochondrial inner membrane | transport |
| AK004012 | 68708 | | early endosome | tumor related |
| AK019590 | 78154 | | | unknown |
| AK014623 | 66701 | | | unknown |
| NM_026183 | 67473 | | Integral to membrane | unknown |
| NM_013684 | 21374 | Tbp | RNA polymerase I transcription f | transcription |
| NM_053107 | 93690 | Gpr45 | integral to membrane | signaling (receptor) |
| AK012292 | 72561 | | | immune response |
| AK003623 | 74772 | | Integral to membrane | transport |
| AK017309 | 71382 | Pex1 | | transport |
| NM_016753 | 17035 | Lxn | integral to membrane | misc |
| AK020352 | 78242 | | extracellular | protein degradation ? |
| AK021301 | 78578 | | | unknown |
| NM_008502 | 16897 | Llglh | cytoskeleton | ctsk |
| AB052945 | 109904 | Mcf2 | cytosol | signaling (GEF) |
| AK021396 | 78550 | | | unknown |
| NM_016754 | 17907 | Mylpf | muscle myosin | ctsk |
| AK014993 | 70961 | | | unknown |
| NM_010264 | 14536 | Nr6a1 | transcription factor complex | transcription |
| NM_009262 | 20745 | Spock1 | extracellular matrix;extracellul | ecm |
| NM_008809 | 18596 | Pdgfrb | extracellular space;integral to | signaling (receptor) |
| AK010555 | 69746 | | | unknown |
| AF267660 | 18604 | Pdk2 | mitochondrion | metabolism |
| NM_021346 | 57908 | Zfp318 | membrane;nucleus | ctsk |
| AK016873 | 71192 | | | signaling ? |
| AK018222 | 76161 | | integral to membrane | unknown |
| NM_026174 | 67464 | Lysal1 | integral to Golgi membrane;lysos | metabolism |
| AK019621 | 78796 | | | Transcription |
| NM_015762 | 50493 | Txnrd1 | cytosol | transport |
| BC004653 | 67155 | | nucleoplasm | transcription |
| AK014178 | 56738 | Mocs1 | | unknown |
| AK005519 | 67701 | | extracellular space | protein degradation |
| NM_019928 | 56640 | Klk4 | extracellular space | protein degradation |
| NM_008845 | 18718 | Pip5k2a | cytoplasm | lipid metabolism |
| NM_016807 | 53378 | Sdcbp | adherens junction;cytoskeleton;i | signaling |
| AK019434 | 28040 | D6Wsu163e | | unknown |
| NM_025512 | 66361 | | cytoplasm | unknown |
| AK011231 | 72068 | Cnot2 | membrane | transcription |

BNS  BS

## Figure 28

Log$_2$(ratio)  -0.3  -0.1  0.1  0.3

| ACCESS | LOCUS | GENE | LOCALIZATION | BIOLOGICAL FUNCTION |
|---|---|---|---|---|
| NM_026402 | 67841 | | inner plaque of spindle pole bod | unknown |
| AK010786 | 73710 | | membrane;microtubule | ctsk |
| NM_021460 | 16889 | Lip1 | extracellular space;lysosome | lipid metabolism |
| AK006800 | 74270 | Usp20 | | protein degradation |
| NM_025998 | 67149 | | extracellular space;integral to | unknown |
| NM_008011 | 14186 | Fgfr4 | extracellular space;integral to | unknown |
| NM_025529 | 66387 | Nudt8 | mitochondrion | unknown |
| NM_019743 | 56353 | Rybp | nucleoplasm | transcription |
| AK015007 | 77048 | | cytoskeleton | transport |
| NM_020560 | 57312 | Mrps31 | mitochondrion;nucleus | transcription |
| NM_019942 | 56526 | sep-06 | septin ring | ctsk |
| NM_018773 | 54353 | Scap2 | cytoplasm | signaling |
| NM_011883 | 24017 | Rnf13 | nucleus | protein degradation |
| AK018362 | 71468 | Obox1 | nucleus | misc |
| NM_019640 | 56305 | Pitpnb | cytoplasm | transport |
| AK006283 | 69435 | | inner plaque of spindle pole bod | ctsk |
| NM_026304 | 67669 | | | unknown |
| NM_009920 | 12794 | Cnil | extracellular space;integral to | signaling |
| AF193606 | 20658 | Son | nucleus | unknown |
| NM_025457 | 66268 | | | unknown |
| AK017256 | 71326 | Treml1 | cell surface;integral to membran | Signaling (receptor) |
| U22015 | 20184 | Rxrip110 | | signaling (receptor) |
| BC004600 | 75221 | Dpp3 | cytoplasm | protein degradation |
| NM_010133 | 13798 | En1 | transcription factor complex | transcription |
| AF062567 | 20687 | Sp3 | transcription factor complex | transcription |
| BC005755 | 70465 | | | translation |
| AK009205 | | | | unknown |
| BC014769 | 73166 | Tm7sf2 | integral to membrane | unknown |
| AK017095 | 71240 | Osbpl7 | | metabolism |
| NM_030265 | 80334 | Kcnip4 | plasma membrane | signaling ? |
| AK002481 | 74090 | | | unknown |
| NM_008360 | 16173 | Il18 | extracellular | immune response |

BNS  BS

Figure 29

Log$_2$(ratio)
-0.4  0.0  0.2  0.4

| ACCESS LOCUS | | GENE | LOCALIZATION | BIOLOGICAL FUNCTION |
|---|---|---|---|---|
| NM_023805 | 76257 | Slc38a3 | integral to plasma membrane | transport |
| NM_011244 | 19411 | Rarg | extrachromosomal circular DNA;nu | signaling (receptor) |
| L20343 | 12296 | Cacnb2 | voltage-gated calcium channel co | ion channel |
| NM_011971 | 26446 | Psmb3 | proteasome core complex (sensu E | protein degradation |
| AK021336 | 77994 | | | unknown |
| NM_018882 | 14766 | Gpr56 | extracellular space;integral to | signaling (receptor) |
| NM_016910 | 53892 | Ppm1d | protein serine-threonine phospha | signaling |
| AK016704 | 74434 | | extracellular | cell cycle ? |
| NM_007703 | 12686 | Elovl3 | endoplasmic reticulum;integral t | lipid metabolism |
| NM_018826 | 54352 | Irx5 | nucleus | transcription |
| AK003154 | 68420 | | | unknown |
| NM_019802 | 56316 | Ggcx | integral to membrane | misc |
| NM_008821 | 18630 | Pet2 | extrachromosomal circular DNA | transcription |
| NM_019925 | 56696 | Gpr132 | integral to plasma membrane | signaling (receptor) |
| NM_020025 | 26878 | B3galt2 | Golgi apparatus;integral to memb | translation |
| NM_016766 | 51812 | Mcrs1 | nucleus | cell cycle |
| AK021023 | 78688 | Nol3 | nucleus | apoptosis |
| NM_010431 | 15251 | Hif1a | flagellum (sensu Eukarya);transc | angiogenesis |
| AK010552 | 66984 | | | unknown |
| AK018027 | 277250 | | | unknown |
| AK019168 | 68991 | | membrane | unknown |
| NM_008538 | 17118 | Marcks | membrane | signaling |
| AK003735 | 52840 | D2Bwg0891e | | unknown |
| NM_026338 | 67722 | | actin cytoskeleton | ctsk ? |
| NM_020518 | 57276 | | integral to plasma membrane | immune response (receptor) |
| BC002193 | 110279 | Bcr | | unknown |
| NM_011444 | 20678 | Sox5 | transcription factor complex | transcription |
| AK007077 | 73572 | | | unknown |
| NM_010309 | 14683 | Gnas | Golgi trans cisterna;membrane fr | signaling |
| AB042402 | 14695 | Gnb3 | heterotrimeric G-protein complex | misc |
| NM_008030 | 14262 | Fmo3 | extracellular space;integral to | traffic |
| NM_009983 | 13033 | Ctsd | extracellular space;lysosome | protein degradation |
| AY007717 | 70603 | Mutyh | extrachromosomal circular DNA | misc |
| NM_013465 | 11625 | Ahsg | extracellular space | protein degradation (secreted) |
| BC003335 | 106344 | Rfc4 | DNA replication factor C complex | cell cycle |
| NM_007542 | 12111 | Bgn | extracellular matrix;extracellul | ecm |
| AK012475 | 330203 | | | unknown |
| AK010038 | 269587 | Epb4.1 | actin cytoskeleton | ctsk |
| BC004098 | 192287 | | mitochondrial inner membrane | transport |
| AK017214 | 71323 | | | tumor related |
| NM_009035 | 19664 | Rbpsuh | cytoplasm;nucleus | immune response |
| AK013604 | 72880 | | | unknown |
| AK019479 | | | | unknown |
| NM_009063 | 19737 | Rgs5 | | signaling |
| AK020831 | 77794 | | | unknown |
| NM_009895 | 12700 | Cish | cytoplasm;plasma membrane | signaling |
| AK009344 | 76948 | | | unknown |
| NM_030735 | 81010 | V3R9 | integral to membrane | signaling (receptor) |
| AK002465 | 71675 | | | unknown |
| AK007519 | 69090 | Ascc1 | transcription factor complex | transcription |
| NM_024471 | 79464 | Lias | mitochondrion | misc |
| NM_023420 | 68018 | Col4a3bp | | ECM |
| NM_021449 | 58799 | AF229032 | mitochondrion | unknown |
| AK015988 | 75146 | | | unknown |
| AK009436 | 67345 | | voltage-gated potassium channel | unknown |
| AK018541 | 71564 | | | unknown |
| NM_025594 | 66492 | | ribosome | unknown |
| L32178 | 232333 | Slc6a1 | integral to plasma membrane | transport |
| NM_008285 | 15465 | Hrh1 | integral to membrane | signaling (receptor) |
| NM_008683 | 18002 | Nedd8 | nucleus;ribosome | protein degradation |
| NM_007527 | 12028 | Bax | integral to membrane;mitochondri | apoptosis |
| D50366 | 16579 | Kifap3 | kinesin II complex | ctsk |
| NM_011793 | 23825 | Banf1 | integral to membrane;nucleus | misc |
| NM_007701 | 12677 | Chx10 | transcription factor complex | transcription |

BNS-18  BNS-72  BNS-192  BS-18  BS-72  BS-192

Figure 29-1

| ACCESS | LOCUS | GENE | LOCALIZATION | BIOLOGICAL FUNCTION |
|---|---|---|---|---|
| AK015988 | 75146 | | | unknown |
| AK009436 | 67345 | | voltage-gated potassium channel | unknown |
| NM_021449 | 58799 | AF229032 | mitochondrion | unknown |
| NM_008683 | 18002 | Nedd8 | nucleus;ribosome | protein degradation |

Log₂(ratio): -0.3  -0.1  0.1  0.3

BS-192  BS-72  BS-18  BNS-192  BNS-72  BNS-18

Figure 29-2

| ACCESS | LOCUS | GENE | LOCALIZATION | BIOLOGICAL FUNCTION |
|---|---|---|---|---|
| NM_007527 | 12028 | Bax | integral to membrane;mitochondri | apoptosis |
| D50366 | 16579 | Kifap3 | kinesin II complex | ctsk |
| NM_008285 | 15465 | Hrh1 | integral to membrane | signaling (receptor) |
| L32178 | 232333 | Slc6a1 | integral to plasma membrane | transport |
| AK018541 | 71564 | | | unknown |
| NM_025594 | 66492 | | ribosome | unknown |
| NM_024471 | 79464 | Llas | mitochondrion | misc |
| NM_023420 | 68018 | Col4a3bp | | ECM |

Sample columns: BS-192, BS-72, BS-18, BNS-192, BNS-72, BNS-18

$Log_2(ratio)$ scale: -0.2, 0.0, 0.2

Figure 29-3

Figure 29-4

Log$_2$(ratio): -0.4 0.0 0.2 0.4

| ACCESS | LOCUS | GENE | LOCALIZATION | BIOLOGICAL FUNCTION |
|---|---|---|---|---|
| NM_018826 | 54352 | Irx5 | nucleus | transcription |
| AK003154 | 68420 | | | unknown |
| NM_013465 | 11625 | Ahsg | extracellular space | protein degradation (secreted) |
| NM_016910 | 53892 | Ppm1d | protein serine-threonine phospha | signaling |
| BC002193 | 110279 | Bcr | | unknown |
| AK007077 | 73572 | | | unknown |
| NM_018882 | 14766 | Gpr56 | extracellular space;integral to | signaling (receptor) |
| NM_019802 | 56316 | Ggcx | integral to membrane | misc |
| BC003335 | 106344 | Rfc4 | DNA replication factor C complex | cell cycle |
| NM_007542 | 12111 | Bgn | extracellular matrix;extracellul | ecm |
| NM_009983 | 13033 | Ctsd | extracellular space;lysosome | protein degradation |
| AY007717 | 70603 | Mutyh | extrachromosomal circular DNA | misc |

BNS-18 BNS-72 BNS-192 BS-18 BS-72 BS-192

EP 1 649 062 B1

123

EP 1 649 062 B1

Figure 29-5

-0.4  0.0  0.2  0.4

Log$_2$(ratio)

| ACCESS | LOCUS | GENE | LOCALIZATION | BIOLOGICAL FUNCTION |
|---|---|---|---|---|
| AK019168 | 68991 | | membrane | unknown |
| AK003735 | 52840 | D2Bwg0891e | | unknown |
| NM_008538 | 17118 | Marcks | membrane | signaling |
| NM_026338 | 67722 | | actin cytoskeleton | ctsk ? |
| NM_023805 | 76257 | Slc38a3 | integral to plasma membrane | transport |
| NM_011244 | 19411 | Rarg | extrachromosomal circular DNA;nu | signaling (receptor) |
| NM_011971 | 26446 | Psmb3 | proteasome core complex (sensu E | protein degradation |
| AK021336 | 77994 | | | unknown |
| NM_010309 | 14683 | Gnas | Golgi trans cisterna;membrane fr | signaling |
| NM_008030 | 14262 | Fmo3 | extracellular space;integral to | traffic |
| AB042402 | 14695 | Gnb3 | heterotrimeric G-protein complex | misc |
| NM_011444 | 20678 | Sox5 | transcription factor complex | transcription |
| NM_020518 | 57276 | | integral to plasma membrane | immune response (receptor) |
| AK007077 | 73572 | | | unknown |

BNS-18  BNS-72  BNS-192  BS-18  BS-72  BS-192

EP 1 649 062 B1

Figure 29-6

-0.3   -0.1   0.1   0.3
Log$_2$(ratio)

BNS-18  BNS-72  BNS-192  BS-18  BS-72  BS-192

| ACCESS | LOCUS | GENE | LOCALIZATION | BIOLOGICAL FUNCTION |
|---|---|---|---|---|
| BC004098 | 192287 | | mitochondrial inner membrane | transport |
| AK013604 | 72880 | | | unknown |
| NM_009035 | 19664 | Rbpsuh | cytoplasm;nucleus | immune response |

# Figure 30 A

-0.4  0.0 0.2 0.4
Log₂(ratio)

| ACCESS | LOCUS | GENE | LOCALIZATION | BIOLOGICAL FUNCTION |
|--------|-------|------|--------------|---------------------|
| NM_007863 | 13384 | Mpp3 | integral to plasma membrane | signaling |
| AF162781 | 16332 | Inppl1 | | signaling |
| NM_016871 | 53333 | Tomm40 | integral to membrane;mitochondri | traffic |
| NM_011176 | 19143 | St14 | extracellular;extrinsic to plasm | cell adh |
| BC013716 | 15499 | Hsf1 | cytoplasm;nucleus | transcription |
| AK003586 | 231712 | | . | unknown |
| NM_007966 | 14028 | Evx1 | transcription factor complex | transcription |
| NM_023422 | 68024 | Hist1h2bc | nucleosome;nucleus | misc |
| NM_026413 | 67854 | Slco6b1 | integral to membrane | transport |
| AK011354 | 67041 | Oxct | extracellular space;mitochondrio | metabolism |
| AK012054 | 72519 | | eukaryotic translation initiatio | unknown |
| NM_011638 | 22042 | Trfr | endosome;integral to membrane;pl | signaling |
| AK003826 | 68530 | | | unknown |
| NM_008492 | 16832 | Ldh2 | cytosol | metabolism |
| AK005386 | 72008 | | endosome | transport |
| NM_021890 | 60527 | Fads3 | integral to plasma membrane | metabolism |
| AF327857 | 232288 | | | unknown |
| NM_015776 | 50530 | Mfap5 | extracellular space;microfibril | ecm |
| AK019863 | 77634 | | | translation |
| AF411608 | 76273 | Ndfip2 | integral to membrane | unknown |
| AK018731 | 68193 | Rpl24 | | translation |
| NM_030248 | 80280 | Cdk5rap3 | | cell cycle |
| AK003794 | 97541 | Qars | | translation |
| AK002407 | 110826 | Etfb | electron transfer flavoprotein c | transport |
| NM_025577 | 66462 | | — | unknown |
| NM_008939 | 19142 | Prss12 | extracellular space;membrane | protein degradation |
| AK017769 | 70614 | | | unknown |
| NM_010181 | 14119 | Fbn2 | extracellular space;microfibril | signaling |
| NM_008095 | 14467 | Gbas | extracellular space;mitochondrio | unknown |
| NM_053076 | 20168 | Rtn3 | endoplasmic reticulum;extracellu | unknown |
| BC003237 | 98985 | AI462438 | nucleus | transcription |
| NM_026069 | 67281 | Rpl37 | cytosolic large ribosomal subuni | translation |
| NM_008857 | 18759 | Prkci | plasma membrane | signaling |
| BC005744 | 72144 | Slc37a3 | extracellular space;integral to | transport |
| NM_026267 | 67602 | | membrane | unknown |

BNS-18
BNS-72
BNS-192
BS-18
BS-72
BS-192

EP 1 649 062 B1

Figure 30 B

-0.4  0.0 0.2 0.4
Log$_2$(ratio)

| ACCESS | FOCUS | GENE | LOCALIZATION | BIOLOGICAL FUNCTION |
|---|---|---|---|---|
| NM_029460 | 75835 | | membrane | signaling |
| AK010113 | 70281 | | | unknown |
| NM_009478 | 22275 | Urod | chloroplast | misc |
| NM_008947 | 19179 | Psmc1 | proteasome complex (sensu Eukary | protein degradation |
| AK019948 | 78095 | | | unknown |
| NM_023505 | 69367 | Glrx2 | mitochondrion;nucleus | transport |
| NM_026513 | | | | unknown |
| NM_009186 | 20462 | Silg41 | | misc |
| NM_008825 | 18640 | Pfkfb2 | cytosol | metabolism |
| NM_009854 | 12516 | Cd7 | extracellular space;integral to | immune response (receptor) |
| AK013837 | 73090 | | | unknown |
| NM_009441 | 22129 | Ttc3 | transcriptional repressor comple | transcription ? |
| NM_007462 | 11789 | Apc | cytoplasm;nucleus | tumor related (suppressor) |
| NM_010064 | 13427 | Dncic2 | cytoplasmic dynein complex | ctsk |
| NM_020031 | 56612 | Pfdn5 | prefoldin complex | translation |
| AK004975 | 71770 | Ap2b1 | clathrin adaptor complex;coated | transport |
| NM_053178 | 94180 | | | metabolism ? |
| AK016563 | 51885 | D2Ertd435e | | ctsk |
| AF343752 | 77053 | Unc84a | cytoplasm;integral to membrane;n | unknown |
| NM_016750 | 51788 | H2afz | nucleosome;nucleus | cell cycle |
| BC005788 | 71994 | Cnn3 | cytoskeleton | ctsk ? |
| NM_030719 | 80909 | Gats | | unknown |
| NM_011838 | 23936 | Lynx1 | extracellular space;integral to | Signaling (receptor) |
| NM_018801 | 54525 | Syt7 | integral to membrane;lysosome;sy | transport |
| NM_010234 | 14281 | Fos | cytoplasm;transcription factor c | tumor related |
| NM_054046 | 23854 | Def8 | extracellular | signaling |
| NM_011042 | 18521 | Pcbp2 | cytoplasm;nucleus;ribonucleoprot | translation |
| NM_011018 | 18412 | Sqstm1 | cytosol | protein degradation |
| BC005674 | 142682 | AA792890 | | unknown |
| J05277 | 15275 | Hk1 | cytosol;membrane;mitochondrion | metabolism |
| AK008493 | 72098 | | cytosol;integral to membrane | metabolism |
| NM_009320 | 21366 | Slc6a6 | integral to plasma membrane | transport |
| NM_013918 | 30940 | Usp25 | 26S proteasome;proteasome comple | protein degradation |
| AK019147 | 19094 | Prkar1a | cAMP-dependent protein kinase co | misc |
| NM_019509 | | | | unknown |
| BC003457 | 108645 | | extrachromosomal circular DNA | misc |
| NM_024244 | 71721 | | membrane | unknown |
| NM_018798 | 54609 | Ubqln2 | endoplasmic reticulum membrane | unknown |
| AK005132 | 72003 | Synpr | extracellular space;integral to | transport |
| NM_023868 | 20191 | Ryr2 | integral to plasma membrane | ion channel |
| NM_007922 | 13712 | Elk1 | membrane;nucleus | transcription |
| AK008900 | 75685 | | | unknown |
| NM_026523 | 68039 | Nmb | extracellular space | signaling (secreted) |
| NM_009167 | 20418 | Shc3 | cytoplasm | signaling |
| NM_008596 | 17306 | Mg29 | integral to membrane;synaptic ve | unknown |
| BC004064 | 20682 | Sox9 | transcription factor complex | unknown |
| AK016572 | 74411 | | integral to membrane | unknown |
| NM_023500 | 22439 | Xkh | extracellular space;integral to | transport |
| NM_011942 | 26394 | Lypla2 | mitochondrion | misc |
| NM_016698 | 50849 | Rnf10 | | transcription |
| NM_019771 | 56431 | Dstn | actin cytoskeleton | cell cycle |
| AK007933 | 72282 | | | unknown |
| NM_007950 | 13874 | Ereg | extracellular space;integral to | cell cycle |
| NM_020494 | 27225 | Ddx24 | cytoplasm | cell cycle |
| NM_011529 | 21353 | Tank | cytoplasm | signaling |
| AK012553 | 77015 | | | unknown |
| AK003287 | 66264 | | | unknown |
| BC004062 | 234959 | Crsp6 | | transcription |
| AK003282 | 66496 | | | unknown |
| AK010511 | 11652 | Akt2 | intracellular;protein kinase CK2 | cell cycle |
| AK003132 | 75406 | Nduts7 | extracellular space;mitochondrio | metabolism |
| NM_007871 | 13430 | Dnm2 | coated pit | ctsk |
| AK003609 | 76308 | | Golgi apparatus;endoplasmic reti | signaling |

BNS-18  BNS-72  BNS-192  BS-18  BS-72  BS-192

127

EP 1 649 062 B1

## Figure 30-1

Log$_2$(ratio): -0.3  -0.1  0.1  0.3

BNS-18  BNS-72  BNS-192  BS-18  BS-72  BS-192

| ACCESS | LOCUS | GENE | LOCALIZATION | BIOLOGICAL FUNCTION |
|---|---|---|---|---|
| NM_007922 | 13712 | Elk1 | membrane;nucleus | transcription |
| AK008900 | 75685 | | | unknown |
| NM_026523 | 68039 | Nmb | extracellular space | signaling (secreted) |
| NM_009167 | 20418 | Shc3 | cytoplasm | signaling |
| NM_018801 | 54525 | Syt7 | integral to membrane;lysosome;sy | transport |
| NM_011042 | 18521 | Pcbp2 | cytoplasm;nucleus;ribonucleoprot | translation |
| BC005674 | 142682 | AA792890 | | unknown |
| NM_054046 | 23854 | Def8 | extracellular | signaling |

## Figure 30-2

Log$_2$(ratio) scale bar: -0.6  -0.2  0.2  0.6

| ACCESS | LOCUS | GENE | LOCALIZATION | BIOLOGICAL FUNCTION |
|--------|-------|------|--------------|---------------------|
| NM_011529 | 21353 | Tank | cytoplasm | signaling |
| AK008493 | 72098 | | cytosol;integral to membrane | metabolism |
| J05277 | 15275 | Hk1 | cytosol;membrane;mitochondrion | metabolism |
| NM_009441 | 22129 | Ttc3 | transcriptional repressor comple | transcription ? |
| NM_013918 | 30940 | Usp25 | 26S proteasome;proteasome comple | protein degradation |
| NM_024244 | 71721 | | membrane | unknown |
| NM_009320 | 21366 | Slc6a6 | integral to plasma membrane | transport |
| AK019147 | 19084 | Prkar1a | cAMP-dependent protein kinase co | misc |
| NM_007462 | 11789 | Apc | cytoplasm;nucleus | tumor related (suppressor) |
| AK005132 | 72003 | Synpr | extracellular space;integral to | transport |
| NM_023868 | 20191 | Ryr2 | integral to plasma membrane | ion channel |
| NM_018798 | 54609 | Ubqln2 | endoplasmic reticulum membrane | unknown |
| NM_010234 | 14281 | Fos | cytoplasm;transcription factor c | tumor related |
| AK016572 | 74411 | | integral to membrane | unknown |
| AK007933 | 72282 | | | unknown |
| NM_020494 | 27225 | Ddx24 | cytoplasm | cell cycle |
| NM_008596 | 17306 | Mg29 | integral to membrane;synaptic ve | unknown |
| NM_019771 | 56431 | Dstn | actin cytoskeleton | cell cycle |
| NM_007950 | 13874 | Ereg | extracellular space;integral to | cell cycle |
| NM_007871 | 13430 | Dnm2 | coated pit | ctsk |
| AK003609 | 76308 | | Golgi apparatus;endoplasmic reti | signaling |
| AK003287 | 66264 | | | unknown |
| AK012553 | 77015 | | | unknown |
| AK003282 | 66496 | | | unknown |

BNS-18  BNS-72  BNS-192  BS-18  BS-72  BS-192

EP 1 649 062 B1

Figure 30-3

## Figure 30-4

Log$_2$(ratio)

-0.3  -0.1  0.1  0.3

| ACCESS | LOCUS | GENE | LOCALIZATION | BIOLOGICAL FUNCTION |
|---|---|---|---|---|
| AK018731 | 68.193 | Rpl24 | | translation |
| NM_023422 | 68024 | Hist1h2bc | nucleosome;nucleus | misc |
| NM_026413 | 67854 | Slco6b1 | integral to membrane | transport |
| AK011354 | 67041 | Oxct | extracellular space;mitochondrio | metabolism |
| BC013716 | 15499 | Hsf1 | cytoplasm;nucleus | transcription |
| AK003586 | 231712 | | | unknown |
| NM_007966 | 14028 | Evx1 | transcription factor complex | transcription |
| AF162781 | 16332 | Inppl1 | | signaling |
| AK012054 | 72519 | | eukaryotic translation initiatio | unknown |
| NM_011176 | 19143 | St14 | extracellular;extrinsic to plasm | cell adh |
| NM_007863 | 13384 | Mpp3 | integral to plasma membrane | signaling |
| AF327857 | 232288 | | | unknown |
| NM_011638 | 22042 | Trfr | endosome;integral to membrane;pl | signaling |
| AK003826 | 68530 | | | unknown |

BNS-18  BNS-72  BNS-192  BS-18  BS-72  BS-192

Figure 30-5

-0.4   0.0 0.2 0.4
$Log_2(ratio)$

| ACCESS | LOCUS | GENE | LOCALIZATION | BIOLOGICAL FUNCTION |
|---|---|---|---|---|
| NM_009478 | 22275 | Urod | chloroplast | misc |
| NM_010181 | 14119 | Fbn2 | extracellular space;microfibril | signaling |
| BC003237 | 98985 | AI462438 | nucleus | transcription |
| NM_053076 | 20168 | Rtn3 | endoplasmic reticulum;extracellu | unknown |
| NM_023505 | 69367 | Glrx2 | mitochondrion;nucleus | transport |
| NM_026267 | 67602 | | membrane | unknown |
| NM_029460 | 75835 | | membrane | signaling |
| NM_008095 | 14467 | Gbas | extracellular space;mitochondrio | unknown |
| NM_021890 | 60527 | Fads3 | integral to plasma membrane | metabolism |
| NM_026069 | 67281 | Rpl37 | cytosolic large ribosomal subuni | translation |
| NM_015776 | 50530 | Mfap5 | extracellular space;microfibril | ecm |
| NM_026413 | 67854 | Slco6b1 | integral to membrane | transport |
| AK011354 | 67041 | Oxct | extracellular space;mitochondrio | metabolism |

BNS-18 BNS-72 BNS-192 BS-18 BS-72 BS-192

EP 1 649 062 B1

Figure 30-6

-0.2    0.0   0.1   0.2

$\log_2(ratio)$

| ACCESS | LOCUS | GENE | LOCALIZATION | BIOLOGICAL FUNCTION |
|---|---|---|---|---|
| NM_020031 | 56612 | Pfdn5 | prefoldin complex | translation |
| NM_053178 | 94180 | | | metabolism ? |
| AK004975 | 71770 | Ap2b1 | clathrin adaptor complex;coated | transport |

BNS-18  BNS-72  BNS-192  BS-18  BS-72  BS-192

Figure 31 A

Log₂(ratio) scale: 0.3  -0.1  0.1  0.3

| ACCESS | LOCUS | GENE | LOCALIZATION | BIOLOGICAL FUNCTION |
|---|---|---|---|---|
| NM_023202 | 66416 | Ndufa7 | membrane fraction;respiratory ch | metabolism |
| AK013269 | 72759 | | integral to membrane | unknown |
| NM_025865 | 66952 | | | unknown |
| AB039178 | | | | unknown |
| AK003826 | 68530 | | | unknown |
| NM_008405 | 16415 | Itgb2l | extracellular matrix;extracellul | cell adh |
| AK007368 | 69062 | | | unknown |
| NM_008708 | 18108 | Nmt2 | cytoplasm | misc |
| NM_009092 | 20068 | Rps17 | cytosolic small ribosomal subuni | translation |
| NM_008997 | 19326 | Rab11b | Golgi apparatus | traffic |
| NM_015806 | 50772 | Mapk6 | nucleosome | signaling |
| NM_008707 | 18107 | Nmt1 | cytoplasm | misc |
| NM_025319 | 66050 | | Golgi apparatus;endoplasmic reti | unknown |
| NM_008770 | 18417 | Cldn11 | integral to plasma membrane;tigh | cell adh |
| NM_025383 | 66147 | | membrane | unknown |
| AK016162 | | | | unknown |
| AK014338 | 74840 | Armet | extracellular space | tumor related |
| L34676 | 11784 | Apba2 | | signaling |
| AK014623 | 66701 | | | unknown |
| AK018680 | 66251 | Arfgap3 | cytosol;perinuclear space | traffic |
| NM_011293 | 20022 | Polr2j | DNA-directed RNA polymerase II-, | transcription |
| NM_009717 | 11922 | Neurod6 | nucleus | transcription |
| AK009213 | 75573 | | | unknown |
| NM_008903 | 19012 | Ppap2a | integral to plasma membrane;memb | metabolism |
| AK006273 | 69441 | | | unknown |
| AK010874 | 69834 | | | unknown |
| AK009460 | 66053 | Ppil2 | nucleus | translation |
| AF162224 | 30924 | Angptl3 | extracellular space | signaling |
| NM_010160 | 14007 | Cugbp2 | cytoplasm;nucleus | transcription |
| AK017929 | 76007 | Zfp198 | nucleus | transcription |
| NM_011673 | 22234 | Ugcg | endoplasmic reticulum;extracellu | lipid metabolism |
| AK012109 | 72194 | | | unknown |
| NM_013556 | 15452 | Hprt | cytoplasm | metabolism |
| NM_009148 | 20336 | Sec8 | exocyst | transport |
| AK012326 | 52323 | D5Ertd363e | actin cytoskeleton | Transport |
| AK019605 | 78799 | | | immune response |
| NM_007567 | 12217 | Bsn | cytoskeleton;integral to membran | signaling |
| AK020441 | 77272 | | | unknown |
| AK007485 | 69053 | | | unknown |
| NM_026402 | 67841 | | inner plaque of spindle pole bod | unknown |
| NM_013835 | 20822 | Ssa2 | ribonucleoprotein complex;viral | misc |
| BC018329 | 70380 | | integral to membrane | unknown |
| NM_026095 | 67332 | Snrpd3 | ribonucleoprotein complex;viral | misc |
| AK014763 | 75769 | | integral to membrane | unknown |
| AK002276 | 71668 | | integral to membrane | unknown |
| NM_021550 | 59048 | | integral to membrane | unknown |
| NM_013596 | 17203 | Mc5r | integral to membrane | signaling |
| NM_054069 | 114895 | Psbpc1 | extracellular space | signaling (secreted) |
| NM_026092 | 67328 | | extracellular space | metabolism |
| NM_011360 | 20392 | Sgce | cytoskeleton;extracellular space | cell adh |
| NM_012038 | 26950 | Vsnl1 | | signaling ? |
| AK010430 | 69219 | Ddah1 | | misc |
| U51279 | 12048 | Bcl2l | integral to membrane;mitochondri | apoptosis |
| NM_015803 | 50769 | Atp8a2 | integral to membrane | transport |
| NM_009417 | 22018 | Tpo | integral to membrane | metabolism |
| NM_019725 | 21886 | Tle2 | nucleus | transcription |
| NM_008424 | 16509 | Kcne1 | integral to plasma membrane | ion channel |
| AF083876 | 13731 | Emp2 | integral to membrane | transport |
| AK012240 | 66399 | | mitochondrion | translation |
| AK009134 | 75572 | Acyp2 | extrachromosomal circular DNA | metabolism |
| NM_009476 | 22269 | Upk2 | endoplasmic reticulum;extracellu | tumor related |
| BC004680 | 13052 | Cxadr | extracellular space;integral to | signaling (receptor) |

Columns: BNS-18, BNS-72, BNS-192, BS-18, BS-72, BS-192

Figure 31 B

Log₂(ratio) scale: -0.3  -0.1  0.1  0.3

| ACCESS | LOCUS | GENE | LOCALIZATION | BIOLOGICAL FUNCTION |
|---|---|---|---|---|
| AK012283 | 72569 | | | unknown |
| NM_009293 | 20905 | Sts | extracellular space;integral to | misc |
| AK021330 | 77996 | | | unknown |
| NM_008300 | 15525 | Hspa4 | cytoplasm | translation |
| NM_026415 | 67859 | | cytoplasm | metabolism ? |
| NM_009072 | 19878 | Rock2 | actin cytoskeleton | signaling |
| NM_008098 | 14489 | Mtpn | | transcription |
| NM_009688 | 11798 | Birc4 | cytosol | apoptosis |
| S82853 | 21915 | Dtymk | | metabolism |
| NM_031391 | 83602 | Gtf2a1 | transcription factor TFIIA compl | transcription |
| NM_053115 | 93732 | Acox2 | peroxisome | metabolism |
| AB041610 | 97484 | Cog8 | Golgi apparatus;membrane | traffic |
| AK008988 | 70285 | | | translation |
| AK003123 | 68433 | | | unknown |
| NM_032003 | 83965 | Enpp5 | extracellular space;integral to | metabolism |
| AK008800 | 70082 | | membrane | unknown |
| NM_011907 | 24102 | Trex2 | extrachromosomal circular DNA | unknown |
| NM_011539 | 21391 | Tbxas1 | integral to membrane | lipid metabolism |
| NM_008317 | 15586 | Hyal1 | | metabolism |
| NM_019938 | 56523 | Pmfbp1 | cytoplasm | transport |
| NM_010347 | 14797 | Aes | nucleus | transcription |
| BC013555 | 70650 | Zcchc8 | | unknown |
| NM_011012 | 18389 | Oprl | integral to plasma membrane | signaling (receptor) |
| NM_009070 | 19826 | Rnps1 | viral nucleocapsid | transcription |
| NM_021543 | 18530 | Pcdh8 | integral to plasma membrane | cell adh |
| NM_009197 | 20502 | Slc16a2 | integral to plasma membrane | transport |
| NM_008468 | 16650 | Kpna6 | importin-, alpha-subunit | transport |
| NM_033652 | 110648 | Lmx1a | nucleus | transcription |
| AK006472 | 74239 | | myosin | unknown |
| NM_013535 | 14790 | Grcc10 | | unknown |
| AK012224 | 76795 | | | unknown |
| AK002667 | 67899 | | cytoplasm | unknown |
| AK009653 | 68544 | | membrane | unknown |
| NM_012030 | 26941 | Slc9a3r1 | actin cytoskeleton;apical plasma | ctsk |
| AK010433 | 66609 | Cryzl1 | | metabolism |
| NM_011529 | 21353 | Tank | cytoplasm | signaling |
| NM_007408 | 11520 | Adfp | membrane | unknown |
| NM_019767 | 56443 | Arpc1a | Arp2-3 protein complex | ctsk |
| AK004928 | 56275 | Rbm14 | cytoplasm;mediator complex;ribon | transcription ? |
| NM_025407 | 22273 | Uqcrc1 | mitochondrial electron transport | metabolism |
| AK020613 | 78672 | | integral to membrane | misc |
| NM_010710 | 16870 | Lhx2 | transcription factor complex | transcription |
| BC004630 | 216549 | | | unknown |
| NM_012019 | 26926 | Pdcd8 | cytosol;mitochondrial intermembr | apoptosis |
| NM_010911 | 18041 | Nfs1 | cytosol;extracellular space;mito | metabolism |
| AK020702 | 78428 | | | unknown |
| NM_026523 | 68039 | Nmb | extracellular space | signaling (secreted) |
| BC009141 | 75062 | Sf3a3 | spliceosome complex | Transcription |
| NM_019967 | 56710 | Dbccr1 | extracellular space;membrane att | misc |
| NM_010149 | 13857 | Epor | extracellular space;integral to | signaling (receptor) |
| NM_011251 | 19654 | Rbm6 | spliceosome complex | misc |
| NM_010942 | 18106 | Nsg1 | Golgi apparatus;integral to memb | signaling |
| NM_010362 | 14873 | Gsto1 | cytoplasm | misc |
| AB071988 | 26914 | H2afy | centrosome;condensed chromosome; | cell cycle |
| AK004007 | 67855 | | | unknown |
| NM_008420 | 16500 | Kcnb1 | voltage-gated potassium channel | ion channel |
| NM_019657 | 56348 | Hsd17b12 | integral to membrane;microsome | metabolism |
| NM_012018 | 26920 | Ma2a8 | integral to membrane | unknown |
| AK010262 | 71955 | | | unknown |
| AK008736 | 70153 | | | unknown |
| NM_009369 | 21810 | Tgfbi | extracellular matrix;extracellul | cell adh |
| BC005574 | 67976 | | | unknown |
| NM_011832 | 23920 | Insrr | extracellular space;integral to | signaling |

BNS-18  BNS-72  BNS-192  BS-18  BS-72  BS-192

Figure 31-1

-0.3  -0.1  0.1  0.3

Log$_2$(ratio)

BNS-18  BNS-72  BNS-192  BS-18  BS-72  BS-192

| ACCESS | LOCUS | GENE | LOCALIZATION | BIOLOGICAL FUNCTION |
|---|---|---|---|---|
| NM_019657 | 56348 | Hsd17b12 | integral to membrane;microsome | metabolism |
| NM_012018 | 26920 | Ma2a8 | integral to membrane | unknown |
| NM_026523 | 68039 | Nmb | extracellular space | signaling (secreted) |
| AK010262 | 71955 | | | unknown |
| AK008736 | 70153 | | | unknown |
| NM_007408 | 11520 | Adfp | membrane | unknown |
| BC009141 | 75062 | Sf3a3 | spliceosome complex | . Transcription |
| NM_019967 | 56710 | Dbccr1 | extracellular space;membrane att | misc |
| NM_010149 | 13857 | Epor | extracellular space;integral to | signaling (receptor) |
| NM_010362 | 14873 | Gsto1 | cytoplasm | misc |
| AB071988 | 26914 | H2afy | centrosome;condensed chromosome; | cell cycle |
| AK004007 | 67855 | | | unknown |

EP 1 649 062 B1

Figure 31-2

-0.3   -0.1   0.1   0.3
$Log_2(ratio)$

| ACCESS | LOCUS | GENE | LOCALIZATION | BIOLOGICAL FUNCTION |
|---|---|---|---|---|
| AK006472 | 74239 | | myosin | unknown |
| AK012224 | 76795 | | | unknown |
| NM_011012 | 18389 | Oprl | integral to plasma membrane | signaling (receptor) |
| NM_033652 | 110648 | Lmx1a | nucleus | transcription |
| AK002667 | 67899 | | cytoplasm | unknown |
| NM_009070 | 19826 | Rnps1 | viral nucleocapsid | transcription |
| NM_021543 | 18530 | Pcdh8 | integral to plasma membrane | cell adh |
| AK008800 | 70082 | | membrane | unknown |
| NM_032003 | 83965 | Enpp5 | extracellular space;integral to | metabolism |
| NM_008468 | 16650 | Kpna6 | importin-, alpha-subunit | transport |

BNS-18  BNS-72  BNS-192  BS-18  BS-72  BS-192

137

## Figure 31-3

| ACCESS | LOCUS | GENE | LOCALIZATION | BIOLOGICAL FUNCTION |
|---|---|---|---|---|
| NM_011529 | 21353 | Tank | cytoplasm | signaling |
| NM_019767 | 56443 | Arpc1a | Arp2-3 protein complex | ctsk |
| AK004928 | 56275 | Rbm14 | cytoplasm;mediator complex;ribon | transcription ? |
| AK010433 | 66609 | Cryzl1 | | metabolism |
| BC004630 | 216549 | | | unknown |

BS-192  BS-72  BS-18  BNS-192  BNS-72  BNS-18

Log$_2$(ratio)

-0.2   0.0   0.1   0.2

138

Figure 31-4

-0.3  -0.1   0.1   0.3

Log₂(ratio)

| ACCESS | LOCUS | GENE | LOCALIZATION | BIOLOGICAL FUNCTION |
|--------|-------|------|--------------|---------------------|
| AK013269 | 72759 | | integral to membrane | unknown |
| NM_054069 | 114895 | Psbpc1 | extracellular space | signaling (secreted) |
| NM_008300 | 15525 | Hspa4 | cytoplasm | translation |
| AK008988 | 70285 | | | translation |
| NM_009072 | 19878 | Rock2 | actin cytoskeleton | signaling |
| NM_031391 | 83602 | Gtf2a1 | transcription factor TFIIA compl | transcription |
| NM_013835 | 20822 | Ssa2 | ribonucleoprotein complex;viral | misc |
| NM_053115 | 93732 | Acox2 | peroxisome | metabolism |
| AK007368 | 69062 | | | unknown |
| NM_008708 | 18108 | Nmt2 | cytoplasm | misc |
| AK009460 | 66053 | Ppil2 | nucleus | translation |
| NM_025383 | 66147 | | membrane | unknown |
| AK016162 | | | | unknown |
| NM_008707 | 18107 | Nmt1 | cytoplasm | misc |
| NM_023202 | 66416 | Ndufa7 | membrane fraction;respiratory ch | metabolism |
| NM_025865 | 66952 | | | unknown |

BNS-18  BNS-72  BNS-192  BS-18  BS-72  BS-192

EP 1 649 062 B1

## Figure 31-5

-0.2  0.0  0.2
Log$_2$(ratio)

BNS-18 BNS-72 BNS-192 BS-18 BS-72 BS-192

| ACCESS | LOCUS | GENE | LOCALIZATION | BIOLOGICAL FUNCTION |
|---|---|---|---|---|
| NM_008997 | 19326 | Rab11b | Golgi apparatus | traffic |
| NM_008903 | 19012 | Ppap2a | integral to plasma membrane;memb | metabolism |
| AF162224 | 30924 | Angptl3 | extracellular space | signaling |
| AK006273 | 69441 | | | unknown |
| NM_009717 | 11922 | Neurod6 | nucleus | transcription |
| AK010874 | 69834 | | | unknown |
| NM_010160 | 14007 | Cugbp2 | cytoplasm;nucleus | transcription |
| AK012109 | 72194 | | | unknown |
| NM_011673 | 22234 | Ugcg | endoplasmic reticulum;extracellu | lipid metabolism |
| AK009213 | 75573 | | | unknown |
| NM_011293 | 20022 | Polr2j | DNA-directed RNA polymerase II-, | transcription |

Figure 31-6

Log$_2$(ratio)
-0.2  0.0  0.1  0.2

| ACCESS | LOCUS | GENE | LOCALIZATION | BIOLOGICAL FUNCTION |
|---|---|---|---|---|
| NM_021550 | 59048 | | integral to membrane | unknown |
| NM_009417 | 22018 | Tpo | integral to membrane | metabolism |
| NM_009688 | 11798 | Birc4 | cytosol | apoptosis |
| U51279 | 12048 | Bcl2l | integral to membrane;mitochondri | apoptosis |
| AF083876 | 13731 | Emp2 | integral to membrane | transport |
| NM_008424 | 16509 | Kcne1 | integral to plasma membrane | ion channel |
| AK010430 | 69219 | Ddah1 | | misc |
| NM_019725 | 21886 | Tle2 | nucleus | transcription |
| NM_011360 | 20392 | Sgce | cytoskeleton;extracellular space | cell adh |
| AK002276 | 71668 | | integral to membrane | unknown |
| NM_013596 | 17203 | Mc5r | integral to membrane | signaling |
| NM_012038 | 26950 | Vsnl1 | | signaling ? |

BNS-18  BNS-72  BNS-192  BS-18  BS-72  BS-192

141

## Figure 32 A

Log$_2$(ratio)
-0.4  0.0 0.2 0.4

| ACCESS | LOCUS | GENE | LOCALIZATION | BIOLOGICAL FUNCTION |
|---|---|---|---|---|
| NM_008399 | 16407 | Itgae | cytoskeleton;extracellular matri | immune response |
| NM_007471 | 11820 | App | Golgi apparatus;coated pit;endop | cell adh |
| NM_053186 | 94218 | Cnnm3 | voltage-gated potassium channel | ion channel |
| NM_019477 | 50790 | Facl4 | cytoplasm;integral to membrane | metabolism |
| NM_008325 | 15932 | Idua | extracellular space;lysosome | metabolism |
| NM_020026 | 26879 | B3galt3 | Golgi apparatus;extracellular sp | misc |
| NM_008138 | 14678 | Gnai2 | plasma membrane | signaling (receptor) |
| NM_008230 | 15186 | Hdc | extrachromosomal circular DNA | metabolism |
| NM_019693 | 53817 | Bat1a | spliceosome complex | transcription |
| NM_025299 | 27366 | Txnl4 | snRNP U5 | transcription |
| NM_019772 | 56372 | | integral to membrane | protein degradation ? |
| NM_007874 | 13476 | Dp1 | integral to membrane | tumor related |
| AK015921 | 75209 | Sv2c | synaptic vesicle | misc |
| AK005345 | 69029 | | extracellular space;integral to | unknown |
| NM_013645 | 19293 | Pva | axon;troponin complex | misc |
| NM_012029 | 26940 | Sitpec | transcription factor complex | transcription |
| AK017081 | 74480 | Samd4 | | unknown |
| NM_020590 | 57436 | Gabarapl1 | plasma membrane | ctsk |
| NM_007529 | 12032 | Bcan | extracellular matrix;extracellul | signaling / ecm |
| L34676 | 11784 | Apba2 | | signaling |
| AJ276505 | 79202 | Tnfrsf22 ? | integral to membrane | signaling (receptor) |
| NM_008377 | 16206 | Lrig1 | extracellular space;integral to | tumor related |
| NM_007495 | 11899 | Astn1 | integral to membrane | misc |
| NM_026495 | 67991 | | transcription factor complex | unknown |
| AK016542 | 74386 | | membrane | unknown |
| AF072758 | 26568 | Slc27a3 | integral to membrane | transport |
| NM_026219 | 67530 | Uqcrb | mitochondrial electron transport | metabolism |
| AK008201 | 69875 | Ndufa11 | | misc |
| BC003914 | 58875 | | mitochondrion | misc |
| AK007598 | 69162 | | | traffic |
| AB047820 | 104416 | Bap1 | nucleus | protein degradation |
| NM_025407 | 22273 | Uqcrc1 | mitochondrial electron transport | metabolism |
| BC003847 | 77125 | | nucleus | unknown |
| NM_016698 | 50849 | Rnf10 | | transcription |
| AK015106 | 106039 | Gga1 | Golgi stack | transport |
| NM_011874 | 23996 | Psmc4 | 26S proteasome;cytosol;nucleus | protein degradation |

BNS-18  BNS-72  BNS-192  BS-18  BS-72  BS-192

142

Figure 32 B

Log$_2$(ratio) scale: -0.4  0.0  0.2  0.4

| | ACCESS | LOCUS | GENE | LOCALIZATION | BIOLOGICAL FUNCTION |
|---|---|---|---|---|---|
| | AK007993 | 67490 | | membrane | unknown |
| | NM_026154 | 107732 | Mrpl10 | mitochondrial large ribosomal su | misc |
| | BC014687 | 68964 | | | unknown |
| | AB045323 | 52357 | D8Ertd594e | intercellular junction | unknown |
| | U51204 | 100732 | Mapre3 | | ctsk |
| | NM_020293 | 56863 | Cldn9 | extracellular space;integral to | cell adh ? |
| | AK003400 | 68566 | Calcyon | integral to membrane | signaling |
| | AK017676 | 70594 | | | unknown |
| | AK008083 | 72041 | | | unknown |
| | NM_008729 | 18163 | Catnd2 | cytoskeleton | cell adhesion |
| | AK009243 | 69534 | Avpi1 | | signaling |
| | NM_025387 | 66154 | | integral to membrane | unknown |
| | BC005459 | 102866 | Pls3 | actin cytoskeleton | ctsk |
| | AK017214 | 71323 | | | tumor related |
| | NM_009652 | 11651 | Akt1 | cytoplasm;protein kinase CK2 com | apoptosis |
| | NM_007804 | 13048 | Cutl2 | nucleus;ribosome | transcription |
| | NM_021432 | 58243 | | chromatin assembly complex | unknown |
| | NM_008770 | 18417 | Cldn11 | integral to plasma membrane;tigh | cell adh |
| | NM_026592 | 68170 | | | unknown |
| | NM_010412 | 15184 | Hdac5 | cytoplasm;histone deacetylase co | cell cycle |
| | AK007351 | 69038 | | integral to membrane | unknown |
| | NM_009426 | 22044 | Trh | extracellular space;soluble frac | growth factor (secreted) |
| | NM_007591 | 12317 | Calr | endoplasmic reticulum lumen;extr | cell adhesion |
| | AJ250690 | | | | unknown |
| | NM_025747 | 66756 | | | unknown |
| | NM_016775 | 13002 | Dnajc5 | membrane | translation |
| | NM_023059 | 24058 | AI256711 | integral to membrane | signaling ? |
| | NM_013615 | 18286 | Odf2 | myosin | ctsk |
| | NM_010879 | 17974 | Nck2 | cytoplasm;vesicle membrane | signaling |
| | NM_018803 | 54526 | Syt10 | integral to membrane;synapse;syn | transport |
| | AK006427 | 69440 | | extracellular space | unknown |
| | NM_016789 | 53324 | Nptx2 | extracellular space;integral to | Transport |
| | BC018162 | 73847 | | | unknown |
| | NM_010282 | 14593 | Ggps1 | ribosome | lipid metabolism |
| | NM_008958 | 19207 | Ptch2 | integral to membrane | signaling (receptor) |
| | NM_030564 | 80751 | Rnf34 | cytoplasm | misc |
| | NM_007689 | 12643 | Chad | extracellular matrix;extracellul | unknown |
| | NM_021548 | 59046 | Arpp19 | cytoplasm | transport ? |
| | NM_011660 | 22166 | Txn1 | vacuole | transport |
| | AK011457 | 75409 | Slitrk5 | integral to membrane | misc |
| | AK005887 | 69354 | Slc38a4 | integral to membrane | transport |
| | NM_025987 | 67130 | Ndufa6 | mitochondrion | metabolism |
| | AK013402 | 109785 | Pgm3 | | metabolism |
| | NM_025839 | 66911 | | | cell adh |
| | NM_007930 | 13803 | Enc1 | cytoskeleton | ctsk |
| | NM_013738 | 27260 | Plek2 | cytoskeleton;membrane;ribosome | ctsk |
| | AK018731 | 68193 | Rpl24 | | translation |
| | AK011230 | 72136 | | integral to membrane | ecm |
| | NM_016749 | 53311 | Mybph | striated muscle thick filament | cell adhesion |
| | NM_011793 | 23825 | Banf1 | integral to membrane;nucleus | misc |
| | BC003932 | 67278 | | | unknown |
| | NM_008847 | 18720 | Pip5k1b | plasma membrane | signaling |
| | AK019059 | 59040 | Arht1 | integral to membrane | signaling |
| | AK014772 | 74592 | | | unknown |
| | NM_016772 | 51798 | Ech1 | mitochondrion;peroxisome | metabolism |
| | NM_025272 | 11974 | Atp6v0e | integral to membrane;membrane fr | metabolism |
| | NM_008722 | 18148 | Npm1 | nucleolus | unknown |
| | NM_011622 | 21968 | Tom1 | Golgi stack;membrane | transport |
| | NM_026065 | 67270 | | mitochondrion | unknown |
| | AK007653 | 52443 | D4Ertd786e | ribosome | translation |
| | NM_021504 | 59007 | Ngly1 | cytoplasm;nucleus | protein degradation |
| | NM_011967 | 26442 | Psma5 | proteasome core complex (sensu E | protein degradation |
| | NM_009086 | 20017 | Rpo1-2 | DNA-directed RNA polymerase I co | transcription |

BNS-18　BNS-72　BNS-192　BS-18　BS-72　BS-192

Figure 32-1

Log$_2$(ratio)

-0.3  -0.1  0.1  0.3

| ACCESS | LOCUS | GENE | LOCALIZATION | BIOLOGICAL FUNCTION |
|---|---|---|---|---|
| AK013402 | 109785 | Pgm3 | | metabolism |
| AK006427 | 69440 | | extracellular space | unknown |
| NM_025987 | 67130 | Ndufa6 | mitochondrion | metabolism |
| AK005887 | 69354 | Slc38a4 | integral to membrane | transport |
| NM_011660 | 22166 | Txn1 | vacuole | transport |
| NM_016789 | 53324 | Nptx2 | extracellular space;integral to | Transport |
| NM_030564 | 80751 | Rnf34 | cytoplasm | misc |
| AK011457 | 75409 | Slitrk5 | integral to membrane | misc |
| NM_007930 | 13803 | Enc1 | cytoskeleton | ctsk |

BNS-18
BNS-72
BNS-192
BS-18
BS-72
BS-192

EP 1 649 062 B1

Figure 32-2

| | ACCESS | LOCUS | GENE | LOCALIZATION | BIOLOGICAL FUNCTION |
|---|---|---|---|---|---|
| | NM_021504 | 59007 | Ngly1 | cytoplasm;nucleus | protein degradation |
| | NM_011967 | 26442 | Psma5 | proteasome core complex (sensu E | protein degradation |
| | NM_025272 | 11974 | Atp6v0e | integral to membrane;membrane fr | metabolism |
| | NM_008722 | 18148 | Npm1 | nucleolus | unknown |
| | NM_011622 | 21968 | Tom1 | Golgi stack;membrane | transport |
| | NM_026065 | 67270 | | mitochondrion | unknown |
| | AK007653 | 52443 | D4Ertd786e | ribosome | translation |
| | NM_011793 | 23825 | Banf1 | integral to membrane;nucleus | misc |
| | NM_008847 | 18720 | Pip5k1b | plasma membrane | signaling |
| | BC003932 | 67278 | | | unknown |
| | NM_016772 | 51798 | Ech1 | mitochondrion;peroxisome | metabolism |

-0.3  -0.1  0.1  0.3

Log$_2$(ratio)

BNS-18  BNS-72  BNS-192  BS-18  BS-72  BS-192

Figure 32-3

-0.2  0.0  0.1  0.2
Log$_2$(ratio)

BNS-18  BNS-72  BNS-192  BS-18  BS-72  BS-192

| ACCESS | LOCUS | GENE | LOCALIZATION | BIOLOGICAL FUNCTION |
|---|---|---|---|---|
| NM_013615 | 18286 | Odf2 | myosin | ctsk |
| NM_010879 | 17974 | Nck2 | cytoplasm;vesicle membrane | signaling |
| NM_023059 | 24058 | AI256711 | integral to membrane | signaling ? |
| NM_025747 | 66756 | | | unknown |

EP 1 649 062 B1

Figure 32-4

Log$_2$(ratio)

-0.2   0.0   0.1   0.2

| ACCESS | LOCUS | GENE | LOCALIZATION | BIOLOGICAL FUNCTION |
|--------|-------|------|--------------|---------------------|
| AK015921 | 75209 | Sv2c | synaptic vesicle | misc |
| NM_007591 | 12317 | Calr | endoplasmic reticulum lumen;extr | cell adhesion |
| AK005345 | 69029 | | extracellular space;integral to | unknown |
| AK017081 | 74480 | Samd4 | | unknown |
| NM_020026 | 26879 | B3galt3 | Golgi apparatus;extracellular sp | misc |
| AK015106 | 106039 | Gga1 | Golgi stack | transport |
| NM_012029 | 26940 | Sitpec | transcription factor complex | transcription |
| NM_016698 | 50849 | Rnf10 | | transcription |
| NM_008770 | 18417 | Cldn11 | integral to plasma membrane;tigh | cell adh |
| NM_026592 | 68170 | | | unknown |
| NM_010412 | 15184 | Hdac5 | cytoplasm;histone deacetylase co | cell cycle |
| AK007351 | 69038 | | integral to membrane | unknown |
| NM_008138 | 14678 | Gnai2 | plasma membrane | signaling (receptor) |
| NM_021432 | 58243 | | chromatin assembly complex | unknown |
| NM_009652 | 11651 | Akt1 | cytoplasm;protein kinase CK2 com | apoptosis |

BNS-18   BNS-72   BNS-192   BS-18   BS-72   BS-192

Figure 32-5

| | ACCESS | LOCUS | GENE | LOCALIZATION | BIOLOGICAL FUNCTION |
|---|---|---|---|---|---|
| | NM_008377 | 16206 | Lrig1 | extracellular space;integral to | tumor related |
| | NM_007495 | 11899 | Astn1 | integral to membrane | misc |
| | NM_026495 | 67991 | | transcription factor complex | unknown |
| | NM_020590 | 57436 | Gabarapl1 | plasma membrane | ctsk |
| | NM_007529 | 12032 | Bcan | extracellular matrix;extracellul | signaling / ecm |
| | AJ276505 | 79202 | Tnfrsf22 ? | integral to membrane | signaling (receptor) |
| | NM_019477 | 50790 | Facl4 | cytoplasm;integral to membrane | metabolism |
| | NM_008325 | 15932 | Idua | extracellular space;lysosome | metabolism |
| | BC014687 | 68964 | | | unknown |
| | AB045323 | 52357 | D8Ertd594e | intercellular junction | unknown |
| | NM_008230 | 15186 | Hdc | extrachromosomal circular DNA | metabolism |
| | NM_007874 | 13476 | Dp1 | integral to membrane | tumor related |
| | NM_019772 | 56372 | | integral to membrane | protein degradation ? |
| | NM_020293 | 56863 | Cldn9 | extracellular space;integral to | cell adh ? |
| | NM_025299 | 27366 | Txnl4 | snRNP U5 | transcription |
| | NM_013645 | 19293 | Pva | axon;troponin complex | misc |
| | NM_008399 | 16407 | Itgae | cytoskeleton;extracellular matri | immune response |
| | NM_053186 | 94218 | Cnnm3 | voltage-gated potassium channel | ion channel |
| | NM_007471 | 11820 | App | Golgi apparatus;coated pit;endop | cell adh |

-0.3  -0.1  0.1  0.3

Log$_2$(ratio)

BNS-18 BNS-72 BNS-192 BS-18 BS-72 BS-192

EP 1 649 062 B1

148

Figure 32-6

Log$_2$(ratio)
-0.3  -0.1  0.1  0.3

| ACCESS | LOCUS | GENE | LOCALIZATION | BIOLOGICAL FUNCTION |
|---|---|---|---|---|
| NM_008729 | 18163 | Catnd2 | cytoskeleton | cell adhesion |
| NM_025407 | 22273 | Uqcrc1 | mitochondrial electron transport | metabolism |
| NM_025387 | 66154 | | integral to membrane | unknown |
| BC005459 | 102866 | Pls3 | actin cytoskeleton | ctsk |
| AK017214 | 71323 | | | tumor related |

BNS-18  BNS-72  BNS-192  BS-18  BS-72  BS-192

Fig 33

-0.3  -0.1  0.1  0.3
Log$_2$(ratio)

| ACCESS | LOCUS | GENE | LOCALIZATION | BIOLOGICAL FUNCTION |
|---|---|---|---|---|
| NM_009129 | 20254 | Scg2 | cytoplasm;extracellular space | growth factor (secreted) |
| AK003651 | 14000 | Etohi2 | | translation |
| AK008724 | 70123 | | | unknown |
| AK008537 | 67035 | | cytoplasm | translation |
| AK003642 | 68618 | | lysosome | metabolism ? |
| NM_024173 | 66290 | Atp6v1g1 | hydrogen-translocating V-type AT | transport |
| NM_010826 | 17540 | Mrvi1 | endoplasmic reticulum membrane;l | transport |
| NM_031179 | 81898 | Sf3b1 | spliceosome complex | transcription |
| NM_009788 | 12307 | Calb1 | cytoplasm | misc |
| AK011747 | 17237 | Mgm1 | extracellular | apoptosis |
| AK019147 | 19084 | Prkar1a | cAMP-dependent protein kinase co | misc |
| AK005852 | 71838 | Phf7 | | unknown |
| NM_008292 | 15488 | Hsd17b4 | peroxisome | lipid metabolism |
| AK005205 | 67129 | | | unknown |
| NM_013614 | 18263 | Odc | mitochondrial inner membrane | misc |
| NM_018855 | 104346 | Gas8 | flagellum (sensu Eukarya);microt | signaling |
| NM_018798 | 54609 | Ubqln2 | endoplasmic reticulum membrane | unknown |
| NM_007583 | 12300 | Cacng2 | extracellular space;tight juncti | ion channel |
| AK021006 | 110920 | Stch | extracellular space | translation |
| NM_011310 | 20197 | S100a3 | | transport |
| AK012109 | 72194 | | | unknown |
| NM_011443 | 20674 | Sox2 | cytoplasm;transcription factor c | transcription |
| NM_011986 | 26562 | Ncdn | | unknown |
| NM_010817 | 17463 | Psmd7 | cytosol | translation |
| AK004655 | 74107 | | | translation |
| NM_031248 | 83409 | | lysosome | traffic |
| NM_018871 | 22628 | Ywhag | | signaling |
| NM_011116 | 18807 | Pld3 | integral to membrane;membrane fr | metabolism |
| NM_033370 | 70349 | Copb1 | Golgi membrane;Golgi vesicle;cyt | transport |
| NM_009215 | 20604 | Sst | extracellular space | growth factor (secreted) |
| NM_011810 | 23873 | Faim | | apoptosis |
| NM_009861 | 12540 | Cdc42 | filopodium | signaling |
| AK004012 | 68708 | | early endosome | tumor related |
| NM_008797 | 18563 | Pcx | mitochondrion | metabolism |
| AK010511 | 11652 | Akt2 | intracellular;protein kinase CK2 | cell cycle |
| AF220205 | 65116 | Prrg2 | extracellular space;integral to | unknown |
| AK013202 | 20467 | Sin3b | nucleus | transcription |
| NM_025302 | 27398 | Mrpl2 | extracellular space;mitochondria | translation |
| AK011446 | 70348 | | | unknown |
| NM_007930 | 13803 | Enc1 | cytoskeleton | ctsk |
| NM_052973 | 94186 | Strn3 | | signaling ? |
| AK012847 | 72674 | | integral to membrane | unknown |
| NM_011421 | 20597 | Smpd1 | integral to membrane;lysosome | metabolism |
| AK015898 | 75141 | Rasd2 | | signaling |

BNS-18  BNS-72  BNS-192  BS-18  BS-72  BS-192

## Figure 33-1

Log$_2$(ratio)
-0.2　0.0　0.2

| ACCESS | LOCUS | GENE | LOCALIZATION | BIOLOGICAL FUNCTION |
|---|---|---|---|---|
| NM_011421 | 20597 | Smpd1 | integral to membrane;lysosome | metabolism |
| AF220205 | 65116 | Prrg2 | extracellular space;integral to | unknown |
| AK010511 | 11652 | Akt2 | intracellular;protein kinase CK2 | cell cycle |
| NM_007930 | 13803 | Enc1 | cytoskeleton | ctsk |
| NM_009215 | 20604 | Sst | extracellular space | growth factor (secreted) |
| NM_033370 | 70349 | Copb1 | Golgi membrane;Golgi vesicle;cyt | transport |
| AK011446 | 70348 | | | unknown |
| NM_018871 | 22628 | Ywhag | | signaling |

BNS-18　BNS-72　BNS-192　BS-18　BS-72　BS-192

Figure 33-2

$Log_2(ratio)$

-0.2  0.0  0.1  0.2

BNS-18  BNS-72  BNS-192  BS-18  BS-72  BS-192

| ACCESS | LOCUS | GENE | LOCALIZATION | BIOLOGICAL FUNCTION |
|---|---|---|---|---|
| NM_011810 | 23873 | Faim | | apoptosis |
| NM_025302 | 27398 | Mrpl2 | extracellular space;mitochondria | translation |

EP 1 649 062 B1

Figure 33-3

-0.2   0.0   0.2

Log$_2$(ratio)

| ACCESS | LOCUS | GENE | LOCALIZATION | BIOLOGICAL FUNCTION |
|---|---|---|---|---|
| AK012847 | 72674 | | integral to membrane | unknown |
| AK015898 | 75141 | Rasd2 | | signaling |
| NM_052973 | 94186 | Strn3 | | signaling ? |
| NM_008797 | 18563 | Pcx | mitochondrion | metabolism |
| AK010511 | 11652 | Akt2 | intracellular;protein kinase CK2 | cell cycle |

BNS-18
BNS-72
BNS-192
BS-18
BS-72
BS-192

154

Figure 33-4

| | -0.3 | -0.1 | 0.1 | 0.3 |
$\text{Log}_2(\text{ratio})$

| ACCESS | LOCUS | GENE | LOCALIZATION | BIOLOGICAL FUNCTION |
|---|---|---|---|---|
| NM_011443 | 20674 | Sox2 | cytoplasm;transcription factor c | transcription |
| AK005852 | 71838 | Phf7 | | unknown |
| NM_018855 | 104346 | Gas8 | flagellum (sensu Eukarya);microt | signaling |
| NM_007583 | 12300 | Cacng2 | extracellular space;tight juncti | ion channel |
| NM_018798 | 54609 | Ubqln2 | endoplasmic reticulum membrane | unknown |
| AK003651 | 14000 | Etohi2 | | translation |
| AK008724 | 70123 | | | unknown |
| AK011747 | 17237 | Mgrn1 | extracellular | apoptosis |
| NM_009129 | 20254 | Scg2 | cytoplasm;extracellular space | growth factor (secreted) |

BNS-18
BNS-72
BNS-192
BS-18
BS-72
BS-192

Figure 33-5

Log$_2$(ratio)

-0.3  -0.1  0.1  0.3

| | ACCESS | LOCUS | GENE | LOCALIZATION | BIOLOGICAL FUNCTION |
|---|---|---|---|---|---|
| | NM_008292 | 15488 | Hsd17b4 | peroxisome | lipid metabolism |
| | AK005205 | 67129 | | | unknown |
| | AK012109 | 72194 | | | unknown |
| | AK021006 | 110920 | Stch | extracellular space | translation |

BNS-18  BNS-72  BNS-192  BS-18  BS-72  BS-192

Figure 33-6

-0.2   0.0   0.2

Log$_2$(ratio)

| ACCESS | LOCUS | GENE | LOCALIZATION | BIOLOGICAL FUNCTION |
|--------|-------|------|--------------|---------------------|
| NM_024173 | 66290 | Atp6v1g1 | hydrogen-translocating V-type AT | transport |
| NM_009788 | 12307 | Calb1 | cytoplasm | misc |
| NM_010826 | 17540 | Mrvi1 | endoplasmic reticulum membrane;i | transport |
| NM_031179 | 81898 | Sf3b1 | spliceosome complex | transcription |
| AK003642 | 68618 | | lysosome | metabolism ? |

BNS-18  BNS-72  BNS-192  BS-18  BS-72  BS-192

**Figure 34 A**

-0.3  -0.1  0.1  0.3
Log₂(ratio)

| ACCESS | LOCUS | GENE | LOCALIZATION | BIOLOGICAL FUNCTION |
|---|---|---|---|---|
| NM_028770 | | | | unknown |
| AK006504 | 76380 | | myosin | signaling |
| NM_007743 | 12843 | Col1a2 | collagen;extracellular space | ecm |
| NM_025849 | 66928 | | integral to membrane | unknown |
| NM_011772 | 22781 | Znfn1a4 | transcription factor complex | misc |
| AK012765 | 69938 | Scrn1 | | unknown |
| AK012392 | 76816 | Sdccag8 | centrosome | immune response |
| NM_008638 | 17768 | Mthfd2 | extracellular space;mitochondrio | metabolism |
| NM_008737 | 18186 | Nrp | extracellular space;integral to | cell adh |
| AK016282 | 75863 | | | unknown |
| AK005138 | 101867 | | membrane | translation ? |
| AK005558 | 76509 | | | signaling (receptor) |
| X97581 | 20689 | Sall3 | nucleus | transcription |
| AK010301 | 71973 | | spliceosome complex | transcription |
| NM_016744 | 18573 | Pde1a | insoluble fraction | metabolism |
| NM_008602 | 17344 | Miz1 | nucleus;septin ring | transcription |
| NM_008702 | 18099 | Nlk | nucleus;protein kinase CK2 compl | signaling |
| NM_007647 | 12499 | Entpd5 | endoplasmic reticulum;extracellu | misc |
| AK008163 | 76523 | | | unknown |
| NM_022022 | 63958 | Ube4b | cytoplasm;ubiquitin ligase compl | protein degradation |
| NM_011025 | 18429 | Oxt | extracellular space | signaling (secreted) |
| NM_023191 | 66317 | | heterotrimeric G-protein complex | signaling ? |
| AK006023 | 75540 | Fpgt | | misc |
| BC011482 | 23943 | Mbc2 | integral to membrane | unknown |
| AK003656 | 74775 | | integral to membrane | unknown |
| NM_016692 | 16319 | Incenp | chromosome-, pericentric region; | cell cycle |
| Y15910 | 54004 | Diap2 | | misc |
| NM_019931 | 56543 | Kcnd3 | extracellular space;voltage-gate | Transport |
| NM_019799 | 56315 | Rhcg | integral to plasma membrane | transport |
| NM_010826 | 17540 | Mrvi1 | endoplasmic reticulum membrane;i | transport |
| NM_019575 | 56214 | Scamp4 | integral to membrane;synaptic ve | traffic |
| NM_021704 | 20315 | Cxcl12 | extracellular space | immune response (secreted) |
| AF285577 | 107816 | Scml2 | | tumor related ? |
| NM_021868 | 60507 | Qtrt1 | | misc |
| NM_053204 | 111173 | Rab6ip2 | non-muscle myosin | signaling |
| NM_023503 | 69260 | Ing1l | | transcription |
| NM_025921 | 67028 | | | unknown |
| NM_021712 | 20508 | Slc18a3 | integral to plasma membrane | transport |
| NM_007795 | 13019 | Ctf1 | extracellular | growth factor (secreted) |
| NM_011341 | 20318 | Sdf4 | Golgi apparatus;extracellular sp | misc |
| BC005600 | 70231 | Gorasp2 | Golgi membrane | unknown |
| NM_007748 | 12861 | Cox6a1 | integral to membrane;respiratory | metabolism |
| NM_021555 | 59053 | Brp16 | | unknown |
| AK010552 | 66984 | | | unknown |
| NM_023041 | 19298 | Pxf | integral to membrane;peroxisomal | misc |
| BC004012 | 192185 | BC004012 | cytosol;extrachromosomal circula | metabolism |
| NM_023377 | 170460 | Stard5 | | transport |
| NM_023370 | 22295 | Cdh23 | cilium;extracellular space;integ | misc |
| S82853 | 21915 | Dtymk | | metabolism |
| NM_009614 | 11490 | Adam15 | extracellular matrix;extracellul | protein degradation |
| U85993 | 319845 | | extracellular space | unknown |
| AF106621 | 21855 | Timm17b | integral to membrane;mitochondri | traffic |
| NM_007761 | 12909 | Crcp | | signaling (receptor) |
| NM_007610 | 12366 | Casp2 | caspase complex;nucleus | apoptosis |
| AK004714 | 74133 | | extracellular space | unknown |
| AJ278733 | 17884 | Myh4 | myosin;striated muscle thick fil | ctsk |
| X81365 | 21367 | Cntn2 | axon | cell adh |
| BC004739 | 213211 | Rnf26 | | apoptosis |
| NM_013727 | 27215 | Azi2 | cytoplasm | signaling |
| AK004506 | 68942 | | | unknown |
| NM_080287 | 140579 | Elmo2 | cytoskeleton;membrane | signaling |

BNS-18  BNS-72  BNS-192  BS-18  BS-72  BS-192

157

# EP 1 649 062 B1

Log$_2$(ratio): -0.3 -0.1 0.1 0.3

| ACCESS | LOCUS | GENE | LOCALIZATION | BIOLOGICAL FUNCTION |
|--------|-------|------|--------------|---------------------|
| AK006525 | 70375 | | | misc |
| NM_025806 | 66857 | | extracellular space | unknown |
| NM_028146 | 72185 | | plasma membrane | unknown |
| NM_011040 | 18510 | Pax8 | transcription factor complex | transcription |
| NM_010727 | 16924 | Lnx1 | cytoplasm;extracellular space | signaling |
| NM_025962 | 67096 | | membrane | unknown |
| NM_008903 | 19012 | Ppap2a | integral to plasma membrane;memb | metabolism |
| NM_053069 | 11793 | Apg5l | cytoplasm | misc |
| NM_016985 | 53332 | Mtmr1 | cytoplasm;plasma membrane | signaling ? |
| NM_019705 | 24105 | AL033326 | spliceosome complex | protein degradation |
| NM_016863 | 14226 | Fkbp1b | | signaling |
| NM_024475 | 79560 | BC002236 | proteasome regulatory particle ( | unknown |
| AF129888 | 20918 | Sui1-rs1 | cytoplasm | translation |
| AK013511 | 72900 | Ndufv2 | mitochondrion | metabolism |
| BC006805 | 72692 | | | metabolism |
| AF092877 | 19823 | Rnf7 | cytoplasm;nucleus | apoptosis |
| NM_019410 | 18645 | Pfn2 | actin cytoskeleton | ctsk |
| AK003866 | 28295 | D10Jhu81e | cell wall;extracellular space;mi | unknown |
| NM_030716 | 80906 | Kcnip2 | plasma membrane | ion channel |
| NM_008734 | 18183 | Nrg3 | extracellular;integral to plasma | growth factor (secreted) |
| BC016258 | 71732 | Vps11 | membrane | transport |
| NM_007936 | 13838 | Epha4 | integral to plasma membrane | signaling (receptor) |
| AK007913 | 69833 | | DNA-directed RNA polymerase I co | transcription |
| AB030906 | 29871 | Scmh1 | chromatin | transcription |
| NM_016737 | 20867 | Stip1 | Golgi apparatus;nucleus | misc |
| NM_013933 | 30960 | Vapa | endoplasmic reticulum;integral t | misc |
| NM_011705 | 22367 | Vrk1 | | signaling |
| AK011810 | 68263 | Pdhb | mitochondrion | metabolism |
| AK010310 | 67755 | Ddx47 | cytoplasm | misc |
| NM_007931 | 13804 | Endog | extracellular space;mitochondrio | misc |
| NM_011656 | 22156 | Tuft1 | extracellular | ctsk |
| NM_013614 | 18263 | Odc | mitochondrial inner membrane | misc |
| NM_009503 | 269523 | Vcp | Golgi apparatus;endoplasmic reti | transport |
| NM_030263 | 80295 | BC003498 | | unknown |
| AK010425 | 71957 | | mRNA cleavage and polyadenylatio | unknown |
| NM_011674 | 22239 | Ugt8 | extracellular space;integral to | metabolism |
| NM_009215 | 20604 | Sst | extracellular space | growth factor (secreted) |
| NM_008161 | 14778 | Gpx3 | extracellular space | metabolism |
| NM_026068 | 67279 | | nucleus | unknown |
| NM_011072 | 18643 | Pfn1 | actin cytoskeleton | ctsk |
| NM_025964 | 67099 | | extrachromosomal circular DNA | unknown |
| NM_019913 | 56551 | Txn2 | mitochondrion | transport |
| NM_012032 | 26943 | Tde1 | Golgi apparatus;extracellular sp | tumor related |
| NM_010313 | 14697 | Gnb5 | heterotrimeric G-protein complex | signaling |
| NM_008374 | 16199 | Il9r | integral to plasma membrane | immune response (receptor) |
| AK003611 | 67070 | | | unknown |
| NM_053074 | 18226 | Nup62 | nuclear pore | transport |
| NM_011917 | 24128 | Xrn2 | nucleus | transcription |
| AK013948 | 69269 | Scnm1 | | transcription |
| AK010382 | 243983 | | actin cytoskeleton;integral to m | transcription |
| NM_011018 | 18412 | Sqstm1 | cytosol | protein degradation |
| AK017020 | 71241 | Dmrtc2 | | unknown |
| NM_013703 | 22359 | Vldlr | coated pit;extracellular space;i | signaling |
| AK016786 | 71804 | | ribosome | unknown |
| NM_019510 | 22065 | Trpc3 | integral to membrane | transport |
| AK014134 | 73172 | | extrachromosomal circular DNA | unknown |
| AK009447 | 66700 | Vps24 | non-muscle myosin | ctsk ? |
| NM_026123 | 67387 | | integral to membrane | unknown |
| NM_011908 | 24109 | Ubl3 | | unknown |
| NM_053071 | 12864 | Cox6c | extracellular space;integral to | transport |
| AF220355 | 56747 | AI843918 | | unknown |
| NM_011543 | 21402 | Skp1a | SCF ubiquitin ligase complex;nuc | translation |
| BC005517 | 71704 | Arhgef3 | cytoplasm | signaling (GEF) |

BNS-18  BNS-72  BNS-192  BS-18  BS-72  BS-192

158

EP 1 649 062 B1

Figure 34-1

Log$_2$(ratio)

-0.2   0.0   0.2

| ACCESS | LOCUS | GENE | LOCALIZATION | BIOLOGICAL FUNCTION |
|---|---|---|---|---|
| NM_011018 | 18412 | Sqstm1 | cytosol | protein degradation |
| AK014134 | 73172 | | extrachromosomal circular DNA | unknown |
| NM_025964 | 67099 | | extrachromosomal circular DNA | unknown |
| AK013511 | 72900 | Ndufv2 | mitochondrion | metabolism |
| NM_026123 | 67387 | | integral to membrane | unknown |
| NM_011908 | 24109 | Ubl3 | | unknown |
| AF220355 | 56747 | AI843918 | | unknown |
| NM_019705 | 24105 | AL033326 | spliceosome complex | protein degradation |
| AK017020 | 71241 | Dmrtc2 | | unknown |
| AK016786 | 71804 | | ribosome | unknown |
| NM_016863 | 14226 | Fkbp1b | | signaling |
| AF129888 | 20918 | Sui1-rs1 | cytoplasm | translation |
| NM_053069 | 11793 | Apg5l | cytoplasm | misc |

BNS-18  BNS-72  BNS-192  BS-18  BS-72  BS-192

## Figure 34-2

Log₂(ratio) scale: -0.3  -0.1  0.1  0.3

| ACCESS | LOCUS | GENE | LOCALIZATION | BIOLOGICAL FUNCTION |
|--------|-------|------|--------------|---------------------|
| NM_009503 | 269523 | Vcp | Golgi apparatus;endoplasmic reti | transport |
| AK010425 | 71957 | | mRNA cleavage and polyadenylatio | unknown |
| NM_013614 | 18263 | Odc | mitochondrial inner membrane | misc |
| NM_009215 | 20604 | Sst | extracellular space | growth factor (secreted) |
| NM_008161 | 14778 | Gpx3 | extracellular space | metabolism |
| NM_016985 | 53332 | Mtmr1 | cytoplasm;plasma membrane | signaling ? |
| NM_026068 | 67279 | | nucleus | unknown |
| NM_011072 | 18643 | Pfn1 | actin cytoskeleton | ctsk |
| AF092877 | 19823 | Rnf7 | cytoplasm;nucleus | apoptosis |
| NM_016737 | 20867 | Stip1 | Golgi apparatus;nucleus | misc |
| NM_030716 | 80906 | Kcnip2 | plasma membrane | ion channel |
| NM_019410 | 18645 | Pfn2 | actin cytoskeleton | ctsk |
| BC016258 | 71732 | Vps11 | membrane | transport |
| AB030906 | 29871 | Scmh1 | chromatin | transcription |
| BC006805 | 72692 | | | metabolism |
| NM_008734 | 18183 | Nrg3 | extracellular;integral to plasma | growth factor (secreted) |
| NM_007936 | 13838 | Epha4 | integral to plasma membrane | signaling (receptor) |

BNS-18  BNS-72  BNS-192  BS-18  BS-72  BS-192

Figure 34-3

-0.2   0.0  0.1  0.2
$\text{Log}_2(ratio)$

| ACCESS | LOCUS | GENE | LOCALIZATION | BIOLOGICAL FUNCTION |
|---|---|---|---|---|
| NM_011917 | 24128 | Xrn2 | nucleus | transcription |
| AK010382 | 243983 | | actin cytoskeleton;integral to m | transcription |
| AK013948 | 69269 | Scnm1 | | transcription |
| NM_019913 | 56551 | Txn2 | mitochondrion | transport |
| NM_010313 | 14697 | Gnb5 | heterotrimeric G-protein complex | signaling |

BNS-18  BNS-72  BNS-192  BS-18  BS-72  BS-192

EP 1 649 062 B1

161

Figure 34-4

Log$_2$(ratio)

-0.3  -0.1  0.1  0.3

| ACCESS | LOCUS | GENE | LOCALIZATION | BIOLOGICAL FUNCTION |
|--------|-------|------|--------------|---------------------|
| AK006504 | 76380 | | myosin | signaling |
| NM_011772 | 22781 | Znfn1a4 | transcription factor complex | misc |
| NM_007743 | 12843 | Col1a2 | collagen;extracellular space | ecm |
| AK012392 | 76816 | Sdccag8 | centrosome | immune response |
| AK006023 | 75540 | Fpgt | | misc |
| NM_021704 | 20315 | Cxcl12 | extracellular space | immune response (secreted) |
| NM_021888 | 60507 | Qtrt1 | | misc |
| AK012765 | 69938 | Scrn1 | | unknown |
| NM_008638 | 17768 | Mthfd2 | extracellular space;mitochondrio | metabolism |
| NM_008737 | 18186 | Nrp | extracellular space;integral to | cell adh |
| NM_022022 | 63958 | Ube4b | cytoplasm;ubiquitin ligase compl | protein degradation |
| BC011482 | 23943 | Mbc2 | integral to membrane | unknown |
| NM_023191 | 66317 | | heterotrimeric G-protein complex | signaling ? |
| AK003656 | 74775 | | integral to membrane | unknown |
| NM_011025 | 18429 | Oxt | extracellular space | signaling (secreted) |
| Y15910 | 54004 | Diap2 | | misc |
| NM_019575 | 56214 | Scamp4 | integral to membrane;synaptic ve | traffic |
| NM_023503 | 69260 | Ing1l | | transcription |
| NM_011341 | 20318 | Sdf4 | Golgi apparatus;extracellular sp | misc |
| NM_008702 | 18099 | Nlk | nucleus;protein kinase CK2 compl | signaling |
| NM_007748 | 12861 | Cox6a1 | integral to membrane;respiratory | metabolism |
| NM_019799 | 56315 | Rhcg | integral to plasma membrane | transport |
| AF285577 | 107815 | Scml2 | | tumor related ? |
| NM_010826 | 17540 | Mrvi1 | endoplasmic reticulum membrane;i | transport |

BNS-18  BNS-72  BNS-192  BS-18  BS-72  BS-192

EP 1 649 062 B1

EP 1 649 062 B1

Figure 34-5

Log$_2$(ratio)

-0.3  -0.1  0.1  0.3

| ACCESS | LOCUS | GENE | LOCALIZATION | BIOLOGICAL FUNCTION |
|---|---|---|---|---|
| NM_021555 | 59053 | Brp16 | | unknown |
| AK010552 | 66984 | | | unknown |
| X81365 | 21367 | Cntn2 | axon | cell adh |
| BC004739 | 213211 | Rnf26 | | apoptosis |
| NM_023041 | 19298 | Pxf | integral to membrane;peroxisomal | misc |
| BC004012 | 192185 | BC004012 | cytosol;extrachromosomal circula | metabolism |
| NM_007610 | 12366 | Casp2 | caspase complex;nucleus | apoptosis |
| AK004714 | 74133 | | extracellular space | unknown |
| NM_007761 | 12909 | Crcp | | signaling (receptor) |
| AJ278733 | 17884 | Myh4 | myosin;striated muscle thick fil | ctsk |

BNS-18  BNS-72  BNS-192  BS-18  BS-72  BS-192

Figure 34-6

Figure 35 A

-0.4  0.0  0.2  0.4

Log$_2$(ratio)

| ACCESS | LOCUS | GENE | LOCALIZATION | BIOLOGICAL FUNCTION |
|--------|-------|------|--------------|---------------------|
| NM_026583 | 68152 | | membrane | unknown |
| NM_026446 | 56470 | Rgs19 | Golgi apparatus;heterotrimeric G | transcription |
| NM_011478 | 20766 | Sprr3 | cornified envelope | ctsk ? |
| AK018615 | 71597 | | nucleus | unknown |
| AK007389 | 107686 | Snrpd2 | small nucleolar ribonucleoprotei | transcription |
| AF078112 | 12328 | Caml ? | integral to membrane ? | signaling ? |
| AK010387 | 69748 | | | metabolism ? |
| AF408435 | 233406 | Prc1 | spindle | cell cycle |
| NM_011352 | 20361 | Sema7a | extracellular space;integral to | signaling |
| NM_024255 | 72479 | | mitochondrion | metabolism |
| NM_013789 | 27414 | Gnefr | cytoplasm;nucleus | misc |
| NM_007625 | 12418 | Cbx4 | chromatin;nucleus | transcription |
| BC017625 | 68047 | | | unknown |
| AK010234 | 50724 | | | unknown |
| NM_029457 | 75826 | Senp2 | nucleus | signaling |
| BC017606 | 72605 | Car10 | extracellular space | metabolism |
| AK014579 | 74347 | | | unknown |
| AK015818 | 75075 | | | unknown |
| NM_008937 | 19130 | Prox1 | transcription factor complex | transcription |
| BC005625 | 217038 | | | unknown |
| NM_022982 | 65079 | Rtn4r | extracellular space;plasma membr | signaling (receptor) |
| AK011557 | 69903 | | mitochondrial outer membrane | unknown |
| NM_011312 | 20199 | S100a5 | cytoplasm | signaling ? |
| AK014408 | 74315 | | | tumor related |
| NM_031879 | | | | unknown |
| NM_023191 | 66317 | | heterotrimeric G-protein complex | signaling ? |
| NM_009041 | 19684 | Rdx | actin cytoskeleton;microvillus | ctsk |

BNS-18  BNS-72  BNS-192  BS-18  BS-72  BS-192

165

## Figure 35 B

Log$_2$(ratio)   0.4  0.0  0.2  0.4

| ACCESS | LOCUS | GENE | LOCALIZATION | BIOLOGICAL FUNCTION |
|---|---|---|---|---|
| AK014338 | 74840 | Armet | extracellular space | tumor related |
| NM_007498 | 11910 | Atf3 | transcription factor complex | transcription |
| AK011978 | 269003 | | | unknown |
| NM_025456 | 66266 | | | unknown |
| NM_009092 | 20068 | Rps17 | cytosolic small ribosomal subuni | translation |
| NM_031179 | 81898 | Sf3b1 | spliceosome complex | transcription |
| L34676 | 11784 | Apba2 | | signaling |
| AK011541 | 71810 | | | unknown |
| NM_019929 | 20610 | Smt3h1 | | cell cycle |
| AK005139 | 68262 | Agpat4 | integral to membrane | lipid metabolism |
| AK016520 | 71745 | Cul2 | nuclear ubiquitin ligase complex | tumor related - apoptosis - cell cycle |
| NM_019667 | 56324 | Stam2 | cytoplasm | signaling |
| AK018132 | 74008 | | lysosome | signaling |
| NM_011929 | 26372 | Clcn6 | integral to plasma membrane | ion channel |
| AK005619 | 72215 | | | unknown |
| AK017214 | 71323 | | | tumor related |
| AK009584 | 69639 | Exosc8 | exosome (RNase complex);nucleus | transcription |
| AK020377 | 67791 | | · | unknown |
| AK008071 | 76769 | | | unknown |
| AK010939 | 68196 | Hsbp1 | extracellular;nucleus | transcription |
| AK007546 | 110052 | Dek | nucleus | transcription |
| BC005603 | 68776 | Taf11 | transcription factor TFIID compl | transcription |
| NM_026061 | 67264 | Ndufb8 | extracellular space;integral to | metabolism |
| NM_023055 | 65962 | Slc9a3r2 | apical plasma membrane | signaling? |
| NM_021382 | 21338 | Tacr3 | integral to membrane;plasma memb | signaling (receptor) |
| NM_025895 | 66999 | | | unknown |
| BC002199 | 211556 | BC002199 | extracellular | unknown |
| AF254416 | 64450 | Gpr85 | integral to plasma membrane | signaling (receptor) |
| NM_027493 | 56249 | Actr8 | actin cytoskeleton | ctsk |
| NM_025834 | 66901 | Proz | extracellular space | misc |
| AK012087 | 70028 | | | unknown |
| AK016572 | 74411 | | integral to membrane | unknown |
| BC013471 | 77056 | | integral to membrane | lipid metabolism? |
| AK005251 | 68975 | | mediator complex | transcription |
| NM_021877 | 15460 | hr | nuclear pore | transcription |
| BC003922 | 52615 | D11Ertd530e | nucleus | unknown |
| NM_027722 | 71207 | | intracellular;membrane | traffic |
| NM_024173 | 66290 | Atp6v1g1 | hydrogen-translocating V-type AT | transport |
| NM_008187 | 14894 | Gtl3 | heterotrimeric G-protein complex | transcription? |
| NM_013535 | 14790 | Grcc10 | | unknown |
| NM_011069 | 18632 | Pex11b | integral to membrane;integral to | misc |
| NM_008889 | 18938 | Ppp1r14b | | transcription |
| NM_007836 | 13197 | Gadd45a | nucleus;ribosome | cell cycle |
| NM_009331 | 21414 | Tcf7 | transcription factor complex | transcription |
| AK017750 | 70623 | | · | unknown |
| NM_011246 | 19419 | Rasgrp1 | nucleosome | signaling |
| NM_013859 | 29805 | ORF6 | | unknown |
| AK013632 | 72894 | | | unknown |
| BC005494 | 216157 | ORF61 | | unknown |
| NM_010837 | 17760 | Mtap6 | microtubule associated complex | ctsk |
| NM_019551 | 56196 | Ttrap | cytoplasm | signaling |
| NM_008019 | 14225 | Fkbp1a | cytoplasm;membrane | transport, translation |
| AK017226 | 75734 | | | unknown |
| NM_025766 | 66784 | | integral to membrane | immune response |
| AK003524 | 71784 | | | unknown |
| NM_009030 | 19646 | Rbbp4 | chromatin assembly complex | cell cycle |
| AK013727 | 14397 | Gabra4 | integral to plasma membrane | signaling |
| NM_013680 | 20964 | Syn1 | synapse;synaptic vesicle | transport |
| NM_021430 | 75695 | | late endosome | unknown |
| NM_007637 | 12465 | Cct5 | chaperonin-containing T-complex | translation |
| AK014834 | 75778 | | | unknown |
| AK012304 | 72612 | | | unknown |
| NM_025835 | 66904 | Pccb | biotin carboxylase complex;extra | metabolism |

BNS-18  BNS-72  BNS-192  BS-18  BS-72  BS-192

EP 1 649 062 B1

Log$_2$(ratio)

-0.2   0.0   0.2

## Figure 35-1

| | ACCESS | LOCUS | GENE | LOCALIZATION | BIOLOGICAL FUNCTION |
|---|---|---|---|---|---|
| | AK017226 | 75734 | | | unknown |
| | NM_008019 | 14225 | Fkbp1a | cytoplasm;membrane | transport, translation |
| | BC002199 | 211556 | BC002199 | extracellular | unknown |
| | AF254416 | 64450 | Gpr85 | integral to plasma membrane | signaling (receptor) |
| | BC005494 | 216157 | ORF61 | | unknown |
| | NM_010837 | 17760 | Mtap6 | microtubule associated complex | ctsk |
| | NM_025834 | 66901 | Proz | extracellular space | misc |
| | NM_009331 | 21414 | Tcf7 | transcription factor complex | transcription |
| | NM_011246 | 19419 | Rasgrp1 | nucleosome | signaling |
| | AK017750 | 70623 | | | unknown |
| | NM_019551 | 56196 | Ttrap | cytoplasm | signaling |

BNS-18  BNS-72  BNS-192  BS-18  BS-72  BS-192

Figure 35-2

Log$_2$(ratio)   -0.3   -0.1   0.1   0.3

| ACCESS | LOCUS | GENE | LOCALIZATION | BIOLOGICAL FUNCTION |
|--------|-------|------|--------------|---------------------|
| NM_013859 | 29805 | ORF6 | | unknown |
| NM_007836 | 13197 | Gadd45a | nucleus;ribosome | cell cycle |
| NM_013535 | 14790 | Grcc10 | | unknown |
| NM_011069 | 18632 | Pex11b | integral to membrane;integral to | misc |
| NM_008889 | 18938 | Ppp1r14b | | transcription |
| NM_025766 | 66784 | | integral to membrane | immune response |

BNS-18  BNS-72  BNS-192  BS-18  BS-72  BS-192

EP 1 649 062 B1

Figure 35-3

Log$_2$(ratio)

-0.3  -0.1  0.1  0.3

| ACCESS | LOCUS | GENE | LOCALIZATION | BIOLOGICAL FUNCTION |
|--------|-------|------|--------------|---------------------|
| AK012087 | 70028 | | | unknown |
| AK016572 | 74411 | | integral to membrane | unknown |
| NM_007637 | 12465 | Cct5 | chaperonin-containing T-complex | translation |
| NM_021430 | 75695 | | late endosome | unknown |
| AK005251 | 68975 | | mediator complex | transcription |
| BC013471 | 77056 | | integral to membrane | lipid metabolism ? |
| AK014834 | 75778 | | | unknown |
| AK012304 | 72612 | | | unknown |
| NM_025835 | 66904 | Pccb | biotin carboxylase complex;extra | metabolism |
| NM_013680 | 20964 | Syn1 | synapse;synaptic vesicle | transport |

BNS-18 BNS-72 BNS-192 BS-18 BS-72 BS-192

Figure 35-4

Log₂(ratio) scale: -0.4  0.0  0.2  0.4

| ACCESS | LOCUS | GENE | | LOCALIZATION | BIOLOGICAL FUNCTION |
|--------|-------|------|---|--------------|---------------------|
| AK007389 | 107686 | Snrpd2 | | small nucleolar ribonucleoprotei | transcription |
| AF078112 | 12328 | Caml ? | ~ | integral to membrane ? | signaling ? |
| AK014579 | 74347 | | | | unknown |
| BC017606 | 72605 | Car10 | | extracellular space | metabolism |
| AK015818 | 75075 | | | | unknown |
| AK010234 | 50724 | | | | unknown |
| NM_029457 | 75826 | Senp2 | | nucleus | signaling |
| AK010387 | 69748 | | | | metabolism ? |
| AF408435 | 233406 | Prc1 | | spindle | cell cycle |
| NM_011352 | 20361 | Sema7a | | extracellular space;integral to ~ | signaling |

BNS-18
BNS-72
BNS-192
BS-18
BS-72
BS-192

EP 1 649 062 B1

170

Figure 35-5

| | ACCESS | LOCUS | GENE | LOCALIZATION | BIOLOGICAL FUNCTION |
|---|---|---|---|---|---|
| | AK018132 | 74008 | | lysosome | signaling |
| | NM_011929 | 26372 | Clcn6 | integral to plasma membrane | ion channel |
| | NM_019667 | 56324 | Stam2 | cytoplasm | signaling |

$Log_2(ratio)$

-0.3   -0.1   0.1   0.3

BNS-18  BNS-72  BNS-192  BS-18  BS-72  BS-192

Figure 35-6

-0.3  -0.1  0.1  0.3

Log$_2$(ratio)

| ACCESS | LOCUS | GENE | LOCALIZATION | BIOLOGICAL FUNCTION |
|---|---|---|---|---|
| AK011978 | 269003 | | | unknown |
| NM_025456 | 66266 | | | unknown |
| BC005625 | 217038 | | | unknown |
| NM_007498 | 11910 | Atf3 | transcription factor complex | transcription |
| AK014338 | 74840 | Armet | extracellular space | tumor related |
| NM_009041 | 19684 | Rdx | actin cytoskeleton;microvillus | ctsk |
| NM_024255 | 72479 | | mitochondrion | metabolism |
| NM_013789 | 27414 | Gnefr | cytoplasm;nucleus | misc |
| BC017625 | 68047 | | | unknown |

BNS-18  BNS-72  BNS-192  BS-18  BS-72  BS-192

## Figure 36 A

Log$_2$(ratio) scale: -0.4  0.0  0.2  0.4

| ACCESS | LOCUS | GENE | LOCALIZATION | BIOLOGICAL FUNCTION |
|---|---|---|---|---|
| U64606 | 17977 | Ncoa1 | cytoplasm | signaling |
| AK004586 | 66868 | | integral to membrane | transport |
| NM_025427 | 66214 | | cytoplasm;nucleus | cell cycle |
| NM_011156 | 19072 | Prep | cytoplasm;nucleus | protein degradation |
| AK011531 | 67788 | | | unknown |
| BC005741 | 233835 | Tnrc6 | extracellular | misc |
| AK013604 | 72880 | | | unknown |
| NM_023372 | 67671 | | cytosolic large ribosomal subuni | misc |
| AK005311 | 66871 | Cpne8 | membrane | misc |
| NM_080639 | 110595 | Timp4 | extracellular matrix;extracellul | protein degradation |
| NM_010607 | 16526 | Kcnk2 | voltage-gated potassium channel | transport |
| NM_011231 | 19352 | Rabggtb | cytoplasm | misc |
| NM_009020 | 19374 | Rag2 | alpha DNA polymerase-;primase co | cell cycle |
| NM_021710 | 11782 | Ap4s1 | Golgi trans face;clathrin vesicl | transport |
| AK010453 | 69719 | Cad | aspartate carbamoyltransferase c | metabolism |
| NM_031401 | 83679 | Usmg4 | | unknown |
| NM_009727 | 11980 | Atp8a1 | integral to membrane | transport, metabolism |
| NM_010799 | 17330 | Minpp1 | endoplasmic reticulum;extracellu | metabolism |
| NM_025316 | 66046 | Ndufb5 | | metabolism |
| NM_019443 | 54405 | Ndufa1 | extracellular space;mitochondrio | metabolism |
| AK007831 | 68115 | | | unknown |
| AB039178 | | | | unknown |
| NM_011343 | 20335 | Sec61g | endoplasmic reticulum;integral t | transport |
| NM_019983 | 56715 | Rabgef1 | plasma membrane | unknown |
| NM_011588 | 21849 | Trim28 | chromatin;nucleus | transcription |
| AK011950 | 67157 | | | unknown |

BNS-18  BNS-72  BNS-192  BS-18  BS-72  BS-192

EP 1 649 062 B1

EP 1 649 062 B1

Figure 36 B

Log₂(ratio)

| ACCESS | LOCUS | GENE | LOCALIZATION | BIOLOGICAL FUNCTION |
|---|---|---|---|---|
| X13721 | 15416 | Hoxb8 | transcription factor complex | transcription |
| NM_013602 | 17748 | Mt1 | cytosol;lysosome | signaling ? |
| NM_011870 | 23991 | Cib1 | synaptic vesicle | signaling |
| U22015 | 20184 | Rxrip110 | | signaling (receptor) |
| NM_009738 | 12038 | Bche | plasma membrane | misc |
| AK004341 | 68845 | | membrane | unknown |
| NM_030749 | 81500 | | endoplasmic reticulum;extracellu | transport |
| NM_023197 | 66356 | | integral to membrane | unknown |
| NM_023043 | 26434 | Prnd | | misc |
| AK008749 | 66132 | | | unknown |
| NM_031261 | 83457 | Fthl17 | ferritin complex | misc |
| M34173 | 12946 | Crry | membrane | immune response (receptor) |
| AK010654 | 72425 | | katanin | unknown |
| AK005056 | 66897 | | membrane | translation |
| NM_028122 | 108052 | Slc14a1 | integral to membrane | transport |
| NM_007620 | 12408 | Cbr1 | cytosol | metabolism |
| AF276975 | 192200 | Swam2 | extracellular space | metabolism |
| NM_022995 | 65112 | | integral to membrane | misc |
| AK011396 | 234729 | D8Wsu151e | | unknown |
| AK006273 | 69441 | | | unknown |
| NM_010412 | 15184 | Hdac5 | cytoplasm;histone deacetylase co | cell cycle |
| AK020284 | 77669 | | | signaling (GEF) ? |
| NM_022886 | 64929 | Scel | cytoplasm | misc |
| NM_016908 | 53420 | Syt5 | integral to membrane;synapse;syn | traffic |
| D86949 | 18845 | Plxna2 | extracellular space;integral to | signaling |
| AK005159 | 72017 | | extracellular space | transport |
| NM_029716 | 108699 | Chn1 | cytoplasm | signaling |
| AK005099 | 68970 | | heterotrimeric G-protein complex | unknown |
| NM_009730 | 11990 | Atrn | extracellular space;integral to | cell adh (receptor) |
| NM_026235 | 67557 | | nucleus;viral nucleocapsid | unknown |
| AK002772 | 66425 | | | unknown |
| NM_007945 | 13860 | Eps8 | cytoplasm | protein degradation |
| AK018327 | 76206 | | integral to membrane | unknown |
| AK005069 | 71767 | | | protein degradation ? |
| AK020702 | 78428 | | | unknown |
| AK011862 | 28018 | D7Wsu128e | | protein degradation ? |
| AF090738 | 16368 | Irs2 | | signaling (receptor) |
| NM_008972 | 19231 | Ptma | nucleus;ribosome | translation |
| AF334612 | 170761 | Pdzk2 | actin cytoskeleton | signaling |
| NM_013638 | 19120 | Prm3 | nucleosome;nucleus | cell cycle |
| AK016671 | 74412 | Gle1l | integral to membrane;plasma memb | Transport |
| NM_031162 | 12503 | Cd3z | integral to membrane;plasma memb | immune response |
| AF293079 | 18360 | Olfr6 | integral to membrane | signaling |
| NM_011305 | 20181 | Rxra | transcription factor complex | transcription |
| NM_023662 | 18536 | Pcm1 | pericentriolar material | cell cycle |
| NM_011500 | 268980 | Strn | cytoplasm | transcription |
| NM_025855 | 52665 | Echdc1 | mitochondrion | unknown |
| NM_009647 | 11639 | Ak4 | mitochondrion | metabolism |
| AK008111 | 75599 | | membrane | cell adh |
| NM_008078 | 14417 | Gad2 | extrachromosomal circular DNA | metabolism |
| AK017044 | 71304 | | | unknown |
| NM_031156 | 15925 | Ide | extracellular space;peroxisome;s | protein degradation |
| NM_009237 | 20675 | Sox3 | transcription factor complex | transcription |
| NM_011512 | 20932 | Surf4 | endoplasmic reticulum membrane;i | misc |
| NM_008497 | 16866 | Lhb | extracellular space | growth factor (secreted) |
| AK010312 | 76483 | | integral to membrane | unknown |
| NM_019657 | 56348 | Hsd17b12 | integral to membrane;microsome | metabolism |
| AK007994 | 72042 | Cotl1 | cytoskeleton | ctsk |
| NM_009068 | 19766 | Ripk1 | | apoptosis |
| AK002416 | 68350 | | integral to membrane;lateral pla | transport ? |
| NM_010781 | 17229 | Mcpt6 | extracellular space | protein degradation |
| NM_026154 | 107732 | Mrpl10 | mitochondrial large ribosomal su | misc |
| NM_009437 | 22117 | Tst | mitochondrial matrix | metabolism |

BNS-18 BNS-72 BNS-192 BS-18 BS-72 BS-192

174

Figure 36-1

-0.3  -0.1  0.1  0.3
Log$_2$(ratio)

| ACCESS | LOCUS | GENE | LOCALIZATION | BIOLOGICAL FUNCTION |
|---|---|---|---|---|
| AK005159 | 72017 | | extracellular space | transport |
| NM_009730 | 11990 | Atrn | extracellular space;integral to | cell adh (receptor) |
| NM_026154 | 107732 | Mrpl10 | mitochondrial large ribosomal su | misc |
| AK002416 | 68350 | | integral to membrane;lateral pla | transport? |
| NM_029716 | 108699 | Chn1 | cytoplasm | signaling |
| NM_010781 | 17229 | Mcpt6 | extracellular space | protein degradation |
| NM_008497 | 16866 | Lhb | extracellular space | growth factor (secreted) |
| NM_019657 | 56348 | Hsd17b12 | integral to membrane;microsome | metabolism |
| AK008111 | 75599 | | membrane | cell adh |
| NM_008078 | 14417 | Gad2 | extrachromosomal circular DNA | metabolism |
| NM_016908 | 53420 | Syt5 | integral to membrane;synapse;syn | traffic |
| D86949 | 18845 | Plxna2 | extracellular space;integral to | signaling |

BNS-18  BNS-72  BNS-192  BS-18  BS-72  BS-192

EP 1 649 062 B1

## Figure 36-2

| ACCESS | LOCUS | GENE | LOCALIZATION | BIOLOGICAL FUNCTION |
|---|---|---|---|---|
| AK005069 | 71767 | | | protein degradation ? |
| AK016671 | 74412 | Gle1l | | Transport |
| AF334612 | 170761 | Pdzk2 | actin cytoskeleton | signaling |
| AK020702 | 78428 | | | unknown |
| NM_013638 | 19120 | Prm3 | nucleosome;nucleus | cell cycle |
| AK018327 | 76206 | | integral to membrane | unknown |
| AF090730 | 16368 | Irs2 | | signaling (receptor) |
| NM_025855 | 52665 | Echdc1 | mitochondrion | unknown |
| NM_023662 | 18536 | Pcm1 | pericentriolar material | cell cycle |
| AK011862 | 28018 | D7Wsu128e | | protein degradation ? |

Heatmap columns: BNS-18, BNS-72, BNS-192, BS-18, BS-72, BS-192

Log2(ratio) scale: -0.4   0.0   0.2   0.4

176

Figure 36-3

-0.3  -0.1  0.1  0.3
Log$_2$(ratio)

BNS-18  BNS-72  BNS-192  BS-18  BS-72  BS-192

| ACCESS | LOCUS | GENE | LOCALIZATION | BIOLOGICAL FUNCTION |
|---|---|---|---|---|
| NM_029716 | 108699 | Chn1 | cytoplasm | signaling |
| AK005099 | 68970 | | heterotrimeric G-protein complex | unknown |
| AK007994 | 72042 | Cotl1 | cytoskeleton | ctsk |
| AK010312 | 76483 | | integral to membrane | unknown |

Figure 36-4

Log$_2$(ratio) scale: -0.3, -0.1, 0.1, 0.3

| ACCESS | LOCUS | GENE | LOCALIZATION | BIOLOGICAL FUNCTION |
|---|---|---|---|---|
| NM_031261 | 83457 | Fthl17 | ferritin complex | misc |
| NM_010412 | 15184 | Hdac5 | cytoplasm;histone deacetylase co | cell cycle |
| NM_030749 | 81500 | | endoplasmic reticulum;extracellu | transport |
| AK011396 | 234729 | D8Wsu151e | | unknown |
| AK004341 | 68845 | | membrane | unknown |
| NM_023043 | 26434 | Prnd | | misc |
| M34173 | 12946 | Crry | membrane | immune response (receptor) |
| AK010654 | 72425 | | katanin | unknown |
| AK005056 | 66897 | | membrane | translation |
| NM_028122 | 108052 | Slc14a1 | integral to membrane | transport |
| NM_007620 | 12408 | Cbr1 | cytosol | metabolism |
| AF276975 | 192200 | Swam2 | extracellular space | metabolism |
| NM_022995 | 65112 | | integral to membrane | misc |

BNS-18  BNS-72  BNS-192  BS-18  BS-72  BS-192

EP 1 649 062 B1

Figure 36-5

Log$_2$(ratio)

-0.3  -0.1  0.1  0.3

| ACCESS | LOCUS | GENE | LOCALIZATION | BIOLOGICAL FUNCTION |
|--------|-------|------|--------------|---------------------|
| NM_080639 | 110595 | Timp4 | extracellular matrix;extracellul | protein degradation |
| NM_010607 | 16526 | Kcnk2 | voltage-gated potassium channel | transport |
| NM_009020 | 19374 | Rag2 | alpha DNA polymerase-;primase co | cell cycle |
| NM_011231 | 19352 | Rabggtb | cytoplasm | misc |
| AK011531 | 67788 | | | unknown |
| NM_021710 | 11782 | Ap4s1 | Golgi trans face;clathrin vesicl | transport |
| AK010453 | 69719 | Cad | aspartate carbamoyltransferase c | metabolism |
| AK004586 | 66868 | | integral to membrane | transport |
| NM_011156 | 19072 | Prep | cytoplasm;nucleus | protein degradation |
| NM_025427 | 66214 | | cytoplasm;nucleus | cell cycle |
| U64606 | 17977 | Ncoa1 | cytoplasm | signaling |

BNS-18  BNS-72  BNS-192  BS-18  BS-72  BS-192

Figure 36-6

-0.2    0.0    0.2

$Log_2(ratio)$

BNS-18   BNS-72   BNS-192   BS-18   BS-72   BS-192

| ACCESS | LOCUS | GENE | LOCALIZATION | BIOLOGICAL FUNCTION |
|---|---|---|---|---|
| NM_011343 | 20335 | Sec61g | endoplasmic reticulum;integral t | transport |
| AK020284 | 77669 | | | signaling (GEF) ? |
| U22015 | 20184 | Rxrip110 | | signaling (receptor) |
| NM_009738 | 12038 | Bche | plasma membrane | misc |

Figure 37 A

Log$_2$(ratio)   -0.4   0.0   0.2   0.4

| ACCESS | LOCUS | GENE | LOCALIZATION | BIOLOGICAL FUNCTION |
|--------|-------|------|--------------|---------------------|
| NM_019794 | 56445 | Dnaja2 | membrane | translation |
| BC005603 | 68776 | Taf11 | transcription factor TFIID compl | transcription |
| NM_030685 | 28146 | D3Ucla1 | integral to membrane | unknown |
| NM_020588 | 57439 | | integral to membrane | unknown |
| NM_019707 | 12554 | Cdh13 | extracellular space;plasma membr | cell adh |
| NM_008149 | 14732 | Gpam | integral to membrane;mitochondri | metabolism |
| AK016563 | 51885 | D2Ertd435e | | ctsk |
| NM_016807 | 53378 | Sdcbp | adherens junction;cytoskeleton;i | signaling |
| AK013995 | 73078 | | mitochondrion | misc |
| NM_025577 | 66462 | | | unknown |
| AK004323 | 66201 | | | unknown |
| NM_011201 | 19246 | Ptpn1 | integral to membrane;soluble fra | signaling ? |
| NM_008323 | 15929 | Idh3g | isocitrate dehydrogenase complex | metabolism |
| NM_007460 | 11776 | Ap3d | Golgi trans face | traffic |
| AK005710 | 67283 | Slc25a19 | integral to membrane;mitochondri | transport |
| NM_054046 | 23854 | Def8 | extracellular | signaling |
| NM_011287 | 19896 | Rpl10a | ribosome | translation |
| NM_021337 | 108077 | Skiv2l | nucleolus | translation |
| AK010555 | 69746 | | | unknown |
| BC004653 | 67155 | | nucleoplasm | transcription |
| NM_013635 | 19027 | Sypl | integral to membrane;secretory g | transport |
| NM_013869 | 29820 | Tnfrsf19 | extracellular space;integral to | signaling (receptor) |
| BC002157 | 100273 | Osbpl9 | | lipid metabolism |
| NM_009404 | 21950 | Tnfsf9 | integral to membrane;plasma memb | immune response |
| BC002240 | 66310 | | integral to membrane;nucleus | lipid metabolism |
| NM_010841 | 17771 | Mtl5 | cytoplasm | immune response |
| NM_009537 | 22632 | Yy1 | transcription factor complex | transcription |
| BC003288 | 28088 | D10Wsu52e | DNA-directed RNA polymerase II-, | unknown |
| AK018222 | 76161 | | integral to membrane | unknown |
| NM_026106 | 13486 | Dr1 | nucleus | transcription |

BNS-18   BNS-72   BNS-192   BS-18   BS-72   BS-192

EP 1 649 062 B1

181

Figure 37 B

Log₂(ratio)  0.4  0.0  0.2  0.4

| ACCESS | LOCUS | GENE | LOCALIZATION | BIOLOGICAL FUNCTION |
|---|---|---|---|---|
| AK012535 | 101513 | AI256456 | membrane | unknown |
| NM_015762 | 50493 | Txnrd1 | cytosol | transport |
| NM_011200 | 19243 | Ptp4a1 | soluble fraction | signaling |
| NM_013534 | 14789 | Grcb | plasma membrane | misc |
| NM_028770 | | | | unknown |
| NM_008984 | 19274 | Ptprm | extracellular space;integral to | signaling (receptor) |
| NM_008135 | 14664 | Slc6a9 | integral to plasma membrane | transport |
| AK014338 | 74840 | Armet | extracellular space | tumor related |
| NM_008078 | 14417 | Gad2 | extrachromosomal circular DNA | metabolism |
| AK009402 | 74252 | Arcp | | unknown |
| NM_025748 | 66757 | Deadc1 | mitochondrion | misc |
| NM_010500 | 15939 | Ier5 | cytoplasm | Transcription |
| NM_018779 | 54611 | Pde3a | integral to membrane | signaling |
| AK006107 | 74229 | | integral to membrane | signaling ? |
| NM_013761 | 27364 | Srr | | metabolism |
| NM_010883 | 17986 | Ndph | extracellular space | signaling |
| NM_010234 | 14281 | Fos | cytoplasm;transcription factor c | tumor related |
| NM_026612 | 68198 | Ndufb2 | mitochondrion | transport |
| NM_007871 | 13430 | Dnm2 | coated pit | ctsk |
| AF290542 | 192198 | Lrrc4 | microtubule | ctsk |
| AK003556 | 66966 | Trit1 | extracellular space;mitochondrio | transcription ? |
| NM_011328 | 20287 | Sct | extracellular space | signaling (receptor) |
| AK017282 | 71373 | | | unknown |
| AK013872 | 72397 | | | unknown |
| AK009325 | 79043 | Tce1 | cytoplasm | signaling |
| Y12713 | 27282 | Erv4 | | virus |
| NM_010352 | 14840 | Gsg1 | voltage-gated calcium channel co | ion channel |
| NM_026660 | 68294 | | integral to membrane | Transport |
| NM_007930 | 13803 | Enc1 | cytoskeleton | ctsk |
| AK002767 | 76509 | | | signaling |
| AK006837 | 73447 | Wdr13 | nucleus | unknown |
| AF030001 | | a lot | | misc |
| NM_025718 | 66705 | Dnase1l2 | extracellular space | misc |
| NM_019460 | 54650 | Sfmbt1 | chromatin | transcription |
| NM_025479 | 66308 | | | unknown |
| NM_013790 | 27416 | Abcc5 | integral to plasma membrane | transport |
| NM_026402 | 67841 | | inner plaque of spindle pole bod | unknown |
| AK003818 | 66248 | Alg5 | endoplasmic reticulum;integral t | metabolism |
| NM_008732 | 18174 | Slc11a2 | endomembrane system;endosome mem | transport |
| NM_029417 | 75757 | | | unknown |
| NM_011503 | 20911 | Stxbp2 | cytoplasm | traffic |
| NM_026304 | 67669 | | | unknown |
| AK006283 | 69435 | | inner plaque of spindle pole bod | ctsk |
| NM_026539 | 68058 | Chd1l | nucleoplasm | cell cycle |
| NM_008841 | 18709 | Pik3r2 | phosphoinositide 3-kinase comple | signaling |
| NM_019781 | 56273 | Pex14 | integral to membrane;integral to | misc |
| BC002302 | 106583 | Rbm16 | | cell cycle |
| NM_025918 | 52715 | D11Ertd707e | | transport |
| AK005862 | 74221 | | | unknown |
| AK015766 | 75083 | | 26S proteasome | protein degradation |
| AK003584 | 81018 | Zfp313 | | unknown |
| NM_023372 | 67671 | | cytosolic large ribosomal subuni | misc |
| AK012848 | 72701 | | | unknown |
| BC008101 | 68939 | | | signaling |
| NM_008376 | 16205 | Imap38 | endoplasmic reticulum;integral t | unknown |
| NM_011288 | 19935 | Mrpl23 | mitochondrial large ribosomal su | translation |
| NM_008488 | 16801 | Arhgef1 | | signaling (GEF) |
| AK006427 | 69440 | | extracellular space | unknown |
| NM_029814 | 76959 | | cytoplasm;nucleus | unknown |
| BC004754 | 21351 | Taldo1 | cytoplasm | metabolism |
| AK017628 | 70556 | | mitochondrial inner membrane | transport |
| AK010448 | 71968 | | | unknown |
| NM_019402 | 54196 | Pabpn1 | mRNA cleavage stimulating factor | transcription |

BNS-18  BNS-72  BNS-192  BS-18  BS-72  BS-192

Figure 37-1

Log$_2$(ratio)  -0.3  -0.1  0.1  0.3

| ACCESS | LOCUS | GENE | LOCALIZATION | BIOLOGICAL FUNCTION |
|---|---|---|---|---|
| NM_026660 | 68294 | | integral to membrane | Transport |
| NM_007930 | 13803 | Enc1 | cytoskeleton | ctsk |
| AK002767 | 76509 | | | signaling |
| NM_007871 | 13430 | Dnm2 | coated pit | ctsk |
| NM_025718 | 66705 | Dnase1l2 | extracellular space | misc |
| AF290542 | 192198 | Lrrc4 | microtubule | ctsk |
| AK003818 | 66248 | Alg5 | endoplasmic reticulum;integral t | metabolism |
| AK006837 | 73447 | Wdr13 | nucleus | unknown |
| NM_025479 | 66308 | | | unknown |
| NM_013790 | 27416 | Abcc5 | integral to plasma membrane | transport |
| NM_026402 | 67841 | | inner plaque of spindle pole bod | unknown |
| NM_029417 | 75757 | | | unknown |
| NM_011503 | 20911 | Stxbp2 | cytoplasm | traffic |

BNS-18  BNS-72  BNS-192  BS-18  BS-72  BS-192

EP 1 649 062 B1

Figure 37-2

Log$_2$(ratio)

-0.3  -0.1  0.1  0.3

| ACCESS | LOCUS | GENE | LOCALIZATION | BIOLOGICAL FUNCTION |
|---|---|---|---|---|
| NM_011328 | 20287 | Sct | extracellular space | signaling (receptor) |
| AK017282 | 71373 | | | unknown |
| AK003556 | 66966 | Trit1 | extracellular space;mitochondrio | transcription ? |
| AK009325 | 79043 | Tce1 | cytoplasm | signaling |
| NM_026660 | 68294 | | integral to membrane | Transport |
| AK013872 | 72397 | | | unknown |
| NM_010352 | 14840 | Gsg1 | voltage-gated calcium channel co | Ion channel |

BNS-18  BNS-72  BNS-192  BS-18  BS-72  BS-192

EP 1 649 062 B1

184

Figure 37-3

| ACCESS | LOCUS | GENE | LOCALIZATION | BIOLOGICAL FUNCTION |
|---|---|---|---|---|
| NM_026304 | 67669 | | | unknown |
| BC002302 | 106583 | Rbm16 | | cell cycle |
| NM_019781 | 56273 | Pex14 | integral to membrane;integral to | misc |
| NM_026539 | 68058 | Chd1l | nucleoplasm | cell cycle |
| NM_008841 | 18709 | Pik3r2 | phosphoinositide 3-kinase comple | signaling |
| NM_025918 | 52715 | D11Ertd707e | | transport |
| AK017628 | 70556 | | mitochondrial inner membrane | transport |
| AK005862 | 74221 | | | unknown |
| NM_008488 | 16801 | Arhgef1 | | signaling (GEF) |
| NM_011288 | 19935 | Mrpl23 | mitochondrial large ribosomal su | translation |

-0.3  -0.1  0.1  0.3

Log$_2$(ratio)

BNS-18 BNS-72 BNS-192 BS-18 BS-72 BS-192

EP 1 649 062 B1

185

EP 1 649 062 B1

## Figure 37-4

-0.2    0.0    0.2

$Log_2(ratio)$

| ACCESS | LOCUS | GENE | LOCALIZATION | BIOLOGICAL FUNCTION |
|---|---|---|---|---|
| NM_013534 | 14789 | Grcb | plasma membrane | misc |
| NM_028770 | | | | unknown |
| NM_013635 | 19027 | Sypl | integral to membrane;secretory g | transport |
| NM_008984 | 19274 | Ptprm | extracellular space;integral to | signaling (receptor) |
| NM_020588 | 57439 | | integral to membrane | unknown |
| NM_008323 | 15929 | Idh3g | isocitrate dehydrogenase complex | metabolism |
| NM_019794 | 56445 | Dnaja2 | membrane | translation |
| AK006107 | 74229 | | integral to membrane | signaling ? |
| NM_010883 | 17986 | Ndph | extracellular space | signaling |
| NM_018779 | 54611 | Pde3a | integral to membrane | signaling |
| NM_013761 | 27364 | Srr | | metabolism |

BNS-18  BNS-72  BNS-192  BS-18  BS-72  BS-192

Figure 37-5

Log$_2$(ratio)

-0.3  -0.1  0.1  0.3

| ACCESS | LOCUS | GENE | LOCALIZATION | BIOLOGICAL FUNCTION |
|---|---|---|---|---|
| AK012535 | 101513 | AI256456 | membrane | unknown |
| NM_008078 | 14417 | Gad2 | extrachromosomal circular DNA | metabolism |
| NM_008135 | 14664 | Slc6a9 | integral to plasma membrane | transport |
| AK009402 | 74252 | Arcp | | unknown |
| NM_026106 | 13486 | Dr1 | nucleus | transcription |
| NM_015762 | 50493 | Txnrd1 | cytosol | transport |
| BC003288 | 28088 | D10Wsu52e | DNA-directed RNA polymerase II-, | unknown |
| AK018222 | 76161 | | integral to membrane | unknown |
| NM_025748 | 66757 | Deadc1 | mitochondrion | misc |
| NM_010500 | 15939 | Ier5 | cytoplasm | Transcription |
| NM_010883 | 17986 | Ndph | extracellular space | signaling |
| NM_010234 | 14281 | Fos | cytoplasm;transcription factor c | tumor related |

BNS-18  BNS-72  BNS-192  BS-18  BS-72  BS-192

EP 1 649 062 B1

187

Figure 37-6

| | ACCESS | LOCUS | GENE | LOCALIZATION | BIOLOGICAL FUNCTION |
|---|---|---|---|---|---|
| | AK016563 | 51885 | D2Ertd435e | | ctsk |
| | AK013995 | 73078 | | mitochondrion | misc |
| | AK004323 | 66201 | | | unknown |
| | BC004653 | 67155 | | nucleoplasm | transcription |
| | NM_016807 | 53378 | Sdcbp | adherens junction;cytoskeleton;i | signaling |
| | NM_025577 | 66462 | | | unknown |
| | NM_011201 | 19246 | Ptpn1 | integral to membrane;soluble fra | signaling ? |

-0.2   0.0   0.1   0.2

Log$_2$(ratio)

BNS-18  BNS-72  BNS-192  BS-18  BS-72  BS-192

188

Figure 38 A

Log$_2$(ratio) scale: -0.3  -0.1  0.1  0.3

| ACCESS | LOCUS | GENE | LOCALIZATION | BIOLOGICAL FUNCTION |
|---|---|---|---|---|
| AK005864 | 75645 | | | unknown |
| AF292939 | 170753 | Gig1 | extracellular | transcription |
| NM_021509 | 59012 | Moxd1 | cytoplasm;extracellular space;me | metabolism |
| NM_011444 | 20678 | Sox5 | transcription factor complex | transcription |
| NM_007983 | 14084 | Faf1 | 26S proteasome;nucleus | apoptosis |
| AK003400 | 68566 | Calcyon | integral to membrane | signaling |
| BC004000 | 170791 | Rnpc2 | nucleus | transcription |
| L17305 | | | | unknown |
| AK005164 | 76566 | | | unknown |
| NM_008560 | 17200 | Mc2r | integral to membrane | signaling (receptor) |
| NM_021713 | 60315 | | extracellular space | unknown |
| NM_009476 | 22269 | Upk2 | endoplasmic reticulum;extracellu | tumor related |
| NM_016871 | 53333 | Tomm40 | integral to membrane;mitochondri | traffic |
| BC005515 | 100090 | Hkr3 | nucleus | transcription |
| NM_028122 | 108052 | Slc14a1 | integral to membrane | transport |
| AK003735 | 52840 | D2Bwg0891e | | unknown |
| AK013135 | 75620 | | | unknown |
| AK006185 | 70373 | | | unknown |
| NM_019802 | 56316 | Ggcx | integral to membrane | misc |
| AK017676 | 70594 | | | unknown |
| NM_012033 | 26944 | Tinag | basement membrane;extracellular | protein degradation |
| NM_019464 | 54673 | Sh3glb1 | cytosol;mitochondrial membrane | apoptosis |
| AK021006 | 110920 | Stch | extracellular space | translation |
| AK011354 | 67041 | Oxct | extracellular space;mitochondrio | metabolism |
| NM_007863 | 13384 | Mpp3 | integral to plasma membrane | signaling |
| AK013609 | 72881 | Zdhhc4 | integral to membrane | unknown |

Colon-18  Colon-72  Colon-192  Breast-18  Breast-72  Breast-192  Lung-18  Lung-72  Lung-192

189

# EP 1 649 062 B1

Figure 38 B

| | ACCESS | LOCUS | GENE | LOCALIZATION | BIOLOGICAL FUNCTION |
|---|---|---|---|---|---|
| | NM_015806 | 50772 | Mapk6 | nucleosome | signaling |
| | AK003826 | 68530 | | | unknown |
| | NM_025865 | 66952 | | | unknown |
| | NM_010590 | 16475 | Jub | | signaling ? |
| | AK012109 | 72194 | | | unknown |
| | NM_009717 | 11922 | Neurod6 | nucleus | transcription |
| | AK019863 | 77634 | | | translation |
| | NM_023805 | 76257 | Slc38a3 | integral to plasma membrane | transport |
| | NM_025978 | 67120 | | cytoplasm | unknown |
| | AK010452 | 52666 | D10Ertd610e | plasma membrane | tumor related |
| | NM_009046 | 19698 | Relb | transcription factor complex | transcription |
| | NM_008405 | 16415 | Itgb2l | extracellular matrix;extracellul | cell adh |
| | BC003237 | 98985 | AI462438 | nucleus | transcription |
| | NM_030717 | 80907 | Lactb | extracellular space;integral to | misc |
| | AK014763 | 75769 | | integral to membrane | unknown |
| | NM_026095 | 67332 | Snrpd3 | ribonucleoprotein complex;viral | misc |
| | NM_018802 | 55925 | Syt8 | integral to membrane;synaptic ve | transport |
| | AK016162 | | | | unknown |
| | NM_013647 | 20055 | Rps16 | cytosolic ribosome (sensu Eukary | translation |
| | NM_009092 | 20068 | Rps17 | cytosolic small ribosomal subuni | translation |
| | NM_011197 | 19221 | Ptgfrn | extracellular space;integral to | translation (receptor) |
| | AK014338 | 74840 | Armet | extracellular space | tumor related |
| | NM_025460 | 66271 | | integral to membrane | unknown |
| | AK013604 | 72880 | | | unknown |
| | NM_025563 | 66439 | | | protein degradation ? |
| | NM_009035 | 19664 | Rbpsuh | cytoplasm;nucleus | immune response |
| | BC002193 | 110279 | Bcr | | unknown |
| | AK018585 | 74558 | | | unknown |
| | NM_011012 | 18389 | Oprl | integral to plasma membrane | signaling (receptor) |
| | AK003809 | 76308 | | Golgi apparatus;endoplasmic reti | signaling |
| | NM_026908 | 69008 | | | unknown |
| | AK020613 | 78672 | | integral to membrane | misc |
| | AK005063 | 110351 | Rap1ga1 | | signaling |
| | NM_033042 | 85030 | Tnfrsf25 | extracellular space;integral to | signaling (receptor) |
| | NM_017394 | 53896 | Slc7a10 | integral to plasma membrane | transport |
| | NM_011793 | 23825 | Banfl | integral to membrane;nucleus | misc |
| | NM_021543 | 18530 | Pcdh8 | integral to plasma membrane | cell adh |
| | AK007415 | 69046 | Hbld2 | | unknown |
| | NM_026523 | 68039 | Nmb | extracellular space | signaling (secreted) |
| | NM_008317 | 15586 | Hyal1 | | metabolism |
| | NM_021433 | 58244 | Stx6 | Golgi apparatus;endosome;integra | traffic |
| | NM_010064 | 13427 | Dnclc2 | cytoplasmic dynein complex | ctsk |
| | NM_026082 | 67299 | Dock7 | | cell cycle ? |
| | AK010262 | 71955 | | | unknown |
| | AK005132 | 72003 | Synpr | extracellular space;integral to | transport |
| | X74504 | 27883 | D16H22S680E | chromatin | unknown |
| | AK009912 | 76467 | Mrsb | extracellular space | misc |
| | NM_016789 | 53324 | Nptx2 | extracellular space;integral to | Transport |
| | NM_011670 | 22223 | Uchl1 | cytoplasm | protein degradation |
| | NM_011529 | 21353 | Tank | cytoplasm | signaling |
| | BC003457 | 108645 | | extrachromosomal circular DNA | misc |
| | NM_026693 | 93739 | Gabarapl2 | Golgi membrane;cytoskeleton | signaling |
| | NM_007688 | 12632 | Cfl2 | cytoskeleton;nucleus | ctsk |
| | NM_020494 | 27225 | Ddx24 | cytoplasm | cell cycle |
| | NM_011428 | 20614 | Snap25 | plasma membrane | signaling |
| | AF127245 | 20591 | Smcx | mitochondrion;nucleus | transcription |
| | AK013427 | 104884 | | | signaling |
| | NM_022885 | 69048 | Slc30a5 | Golgi apparatus;apical plasma me | transport |
| | NM_010942 | 18196 | Nsg1 | Golgi apparatus;integral to memb | signaling |
| | BC004062 | 234959 | Crsp6 | | transcription |
| | AK014230 | 216877 | Dhx33 | | unknown |
| | NM_025594 | 66492 | | ribosome | unknown |
| | NM_011522 | 20974 | Syngr3 | integral to plasma membrane | unknown |

-0.1  0.1  0.3
Log2(ratio)

Colon-18 Colon-72 Colon-192 Breast-18 Breast-72 Breast-192 Lung-18 Lung-72 Lung-192

190

Figure 39 A

| | ACCESS | LOCUS | GENE | LOCALIZATION | BIOLOGICAL FUNCTION |
|---|---|---|---|---|---|
| | NM_053186 | 94218 | Cnnm3 | voltage-gated potassium channel | ion channel |
| | AK021200 | 13057 | Cyba | integral to membrane;mitochondri | transport, metabolism |
| | BC002274 | 69216 | | | unknown |
| | AK003605 | 74770 | | integral to membrane | unknown |
| | NM_010274 | 14571 | Gpd2 | extracellular space;glycerol-3-p | lipid metabolism |
| | AK019147 | 19084 | Prkar1a | cAMP-dependent protein kinase co | misc |
| | NM_007568 | 12223 | Btc | extracellular space;integral to | cell cycle |
| | AK002569 | 73242 | | | unknown |
| | AK017929 | 76007 | Zfp198 | nucleus | transcription |
| | NM_016910 | 53892 | Ppm1d | protein serine-threonine phospha | signaling |
| | NM_011427 | 20613 | Snai1 | transcription factor complex | transcription |
| | NM_008298 | 15502 | Dnaja1 | | misc |
| | NM_007457 | 11769 | Ap1s1 | Golgi trans face;clathrin vesicl | transport |
| | AK011290 | 67763 | | extracellular space | metabolism |
| | NM_018798 | 54609 | Ubqln2 | endoplasmic reticulum membrane | unknown |
| | AK009325 | 79043 | Tce1 | cytoplasm | signaling |
| | AK021006 | 110920 | Stch | extracellular space | translation |
| | NM_013722 | 27204 | Syn3 | synaptic vesicle | traffic |
| | NM_007772 | 110521 | Hivep1 | transcription factor complex | transcription |
| | AK014582 | 70807 | Arrdc2 | | unknown |
| | BC016444 | 75273 | | integral to membrane | transcription |
| | AK006243 | 66091 | Ndufa3 | integral to membrane;mitochondri | metabolism |
| | NM_025547 | 66410 | | ribosome | transcription |
| | BC006905 | 75608 | | | immune response |
| | NM_019799 | 56315 | Rhcg | integral to plasma membrane | transport |
| | AF285577 | 107815 | Scml2 | | tumor related ? |
| | NM_008535 | 17095 | Lyl1 | transcription factor complex | transcription |

-0.2   0.0   0.2

Log$_2$(ratio)

Colon-18
Colon-72
Colon-192
Breast-18
Breast-72
Breast-192
Lung-18
Lung-72
Lung-192

191

Figure 39 B

Log$_2$(ratio)  -0.2  0.0  0.2

| ACCESS | LOCUS | GENE | LOCALIZATION | BIOLOGICAL FUNCTION |
|---|---|---|---|---|
| Y15910 | 54004 | Diap2 | | misc |
| NM_009974 | 13000 | Csnk2a2 | protein kinase CK2 complex | signaling |
| NM_021888 | 60507 | Qtrt1 | | misc |
| NM_011025 | 18429 | Oxt | extracellular space | signaling (secreted) |
| NM_018804 | 229521 | Syt11 | integral to plasma membrane;syna | transport |
| NM_011040 | 18510 | Pax8 | transcription factor complex | transcription |
| NM_019477 | 50790 | Facl4 | cytoplasm;integral to membrane | metabolism |
| NM_016802 | 11848 | Arha | cytoskeleton;cytosol | signaling |
| AK016037 | 75212 | Rnf121 | | protein degradation ? |
| NM_011341 | 20318 | Sdf4 | Golgi apparatus;extracellular sp | misc |
| NM_007527 | 12028 | Bax | integral to membrane;mitochondri | apoptosis |
| NM_009788 | 12307 | Calb1 | cytoplasm | misc |
| NM_008098 | 14489 | Mtpn | | transcription |
| AK005756 | 75454 | | | metabolism |
| AF232828 | 18134 | Nova1 | nucleus | transcription |
| NM_016666 | 11632 | Aip | cytoplasm | transcription |
| BC017625 | 68047 | | | unknown |
| AK016299 | 75894 | | | misc |
| AK010890 | 72308 | Brf1 | | transcription |
| AK011747 | 17237 | Mgrn1 | extracellular | apoptosis |
| NM_010826 | 17540 | Mrvi1 | endoplasmic reticulum membrane;i | transport |
| AF217484 | 57444 | Isg20 | intracellular | misc |
| AK019863 | 77634 | | | translation |
| AK006862 | 74221 | | | unknown |
| NM_010771 | 17184 | Matr3 | nucleus | misc |
| NM_025299 | 27366 | Txnl4 | snRNP U5 | transcription |
| NM_018819 | 55951 | Brp44l | | unknown |
| NM_018802 | 55925 | Syt8 | integral to membrane;synaptic ve | transport |
| AK011126 | 268490 | | | unknown |
| NM_008292 | 15488 | Hsd17b4 | peroxisome | lipid metabolism |
| AK002696 | 28126 | D13Wsu177e | | unknown |
| NM_010412 | 15184 | Hdac5 | cytoplasm;histone deacetylase co | cell cycle |
| AK003400 | 68566 | Calcyon | integral to membrane | signaling |
| NM_009069 | 19769 | Rit1 | plasma membrane | signaling |
| AK007703 | 27410 | Abca3 | integral to plasma membrane | misc |
| AK017214 | 71323 | | | tumor related |
| NM_007591 | 12317 | Calr | endoplasmic reticulum lumen;extr | cell adhesion |
| AK005345 | 69029 | | extracellular space;integral to | unknown |
| NM_008903 | 19012 | Ppap2a | integral to plasma membrane;memb | metabolism |
| NM_008229 | 15182 | Hdac2 | cytoplasm;histone deacetylase co | transcription |
| AK018560 | 67556 | Pigm | | metabolism ? |
| NM_008929 | 19107 | Dnajc3 | cytoplasm;extracellular space | translation |
| AF072758 | 26568 | Slc27a3 | integral to membrane | transport |
| NM_009614 | 11490 | Adam15 | extracellular matrix;extracellul | protein degradation |
| AB047820 | 104416 | Bap1 | nucleus | protein degradation |
| NM_016789 | 53324 | Nptx2 | extracellular space;integral to | Transport |
| NM_007930 | 13803 | Enc1 | cytoskeleton | ctsk |
| NM_011622 | 21968 | Tom1 | Golgi stack;membrane | transport |
| NM_010595 | 16485 | Kcna1 | voltage-gated potassium channel | transport |
| NM_008974 | 19244 | Ptp4a2 | soluble fraction | signaling |
| NM_019746 | 56330 | Pdcd5 | cytoplasm | apoptosis |
| NM_013525 | 14455 | Gas5 | | cell cycle |
| NM_021548 | 59046 | Arpp19 | cytoplasm | transport ? |
| NM_009861 | 12540 | Cdc42 | filopodium | signaling |
| NM_025987 | 67130 | Ndufa6 | mitochondrion | metabolism |
| NM_026187 | 52231 | D1Ertd161e | | transcription ? |
| AK007857 | 70061 | | | metabolism |
| NM_024475 | 79560 | BC002236 | proteasome regulatory particle ( | unknown |
| AK003850 | 66151 | | extracellular matrix | ecm |
| NM_019686 | 56506 | | synaptic vesicle | signaling |
| AK004012 | 68708 | | early endosome | tumor related |
| AK002744 | 68375 | Ndufa8 | respiratory chain complex I (san | metabolism |
| NM_022029 | 64011 | Nrgn | | signaling |

Colon-18 Colon-72 Colon-192 Breast-18 Breast-72 Breast-192 Lung-18 Lung-72 Lung-192

EP 1 649 062 B1

Figure 40 A

$Log_2(ratio)$

-0.2 0.0 0.2

| ACCESS | LOCUS | GENE | LOCALIZATION | BIOLOGICAL FUNCTION |
|---|---|---|---|---|
| NM_025835 | 66904 | Pccb | biotin carboxylase complex;extra | metabolism |
| AK007452 | 69082 | | | signaling |
| AK018132 | 74008 | | lysosome | signaling |
| NM_009437 | 22117 | Tst | mitochondrial matrix | metabolism |
| BC004826 | 57278 | Lu | integral to plasma membrane | cell adh |
| AK017226 | 75734 | | | unknown |
| AF092877 | 19823 | Rnf7 | cytoplasm;nucleus | apoptosis |
| NM_010942 | 18196 | Nsg1 | Golgi apparatus;integral to memb | signaling |
| NM_026612 | 68198 | Ndufb2 | mitochondrion | transport |
| NM_029716 | 108699 | Chn1 | cytoplasm | signaling |
| NM_021319 | 57757 | | | signaling (receptor) |
| NM_025594 | 66492 | | ribosome | unknown |
| NM_013647 | 20055 | Rps16 | cytosolic ribosome (sensu Eukary | translation |
| AB071988 | 26914 | H2afy | centrosome;condensed chromosome; | cell cycle |
| NM_016789 | 53324 | Nptx2 | extracellular space;integral to | Transport |
| AK009308 | 246257 | | cytoplasm | tumor related |
| AF030001 | | a lot | | misc |
| NM_019410 | 18645 | Pfn2 | actin cytoskeleton | ctsk |
| AK003818 | 66248 | Alg5 | endoplasmic reticulum;integral t | metabolism |
| NM_008731 | 18167 | Npy2r | integral to plasma membrane | signaling (body weight) |

Colon-18 Colon-72 Colon-192 Breast-18 Breast-72 Breast-192 Lung-18 Lung-72 Lung-192

Figure 40 B

Log$_2$(ratio)  0.2  0.0  0.2

| ACCESS | LOCUS | GENE | LOCALIZATION | BIOLOGICAL FUNCTION |
|---|---|---|---|---|
| AK003863 | 26901 | Dab1 | extracellular | unknown |
| NM_016908 | 53420 | Syt5 | integral to membrane;synapse;syn | traffic |
| NM_025938 | 67053 | | nucleus | transcription ? |
| NM_018757 | 54369 | Nme6 | extracellular space;microtubule | metabolism |
| AK005033 | 52432 | Ppp2r2d | protein phosphatase type 2A comp | signaling |
| NM_025895 | 66999 | | | unknown |
| NM_008019 | 14225 | Fkbp1a | cytoplasm;membrane | transport, translation |
| AK013035 | 20621 | Snn | integral to membrane | misc |
| NM_023191 | 66317 | | heterotrimeric G-protein complex | signaling ? |
| NM_019990 | 56018 | Stard10 | | tumor related |
| NM_022995 | 65112 | | integral to membrane | misc |
| AY026384 | 192655 | Cacna1i | | transport |
| NM_011588 | 21849 | Trim28 | chromatin;nucleus | transcription |
| AK004778 | 74122 | | integral to membrane | unknown |
| NM_023721 | 73834 | Atp6v1d | hydrogen-transporting ATPase V1 | Transport |
| NM_007897 | 13591 | Ebf1 | nucleus | transcription |
| L34676 | 11784 | Apba2 | | signaling |
| NM_008984 | 19274 | Ptprm | extracellular space;integral to | signaling (receptor) |
| AK016563 | 51885 | D2Ertd435e | | ctsk |
| NM_011231 | 19352 | Rabggtb | cytoplasm | misc |
| NM_008129 | 14630 | Gclm | | misc |
| BC017532 | 105501 | Abhd4 | | protein degradation |
| NM_026446 | 56470 | Rgs19 | Golgi apparatus;heterotrimeric G | transcription |
| AK007389 | 107686 | Snrpd2 | small nucleolar ribonucleoprotei | transcription |
| NM_019679 | 57778 | Fmnl | cytosol | ctsk- |
| NM_016981 | 20544 | Slc9a1 | basolateral plasma membrane;extr | transport |
| AK005164 | 76566 | | | unknown |
| NM_031404 | 83766 | Actl6 | SWI-SNF complex;actin filament | ctsk |
| AK020608 | 78425 | | | unknown |
| NM_020588 | 57439 | | integral to membrane | unknown |
| AF062567 | 20687 | Sp3 | transcription factor complex | transcription |
| NM_026047 | 72486 | | cytoplasm;extracellular space;in | unknown |
| AK021006 | 110920 | Stch | extracellular space | translation |
| NM_008480 | 16772 | Lama1 | basal lamina;extracellular space | ecm |
| BC002240 | 66310 | | integral to membrane;nucleus | lipid metabolism |
| AK018222 | 76161 | | integral to membrane | unknown |
| NM_031249 | 83410 | Cstf2t | mRNA cleavage stimulating factor | transcription |
| NM_016909 | 53424 | Tsnax | mitochondrial inner membrane;nuc | misc |
| NM_013595 | 17192 | Mbd3 | chromatin;nucleus | transcription |
| AK013604 | 72880 | | | unknown |
| NM_026218 | 67529 | | non-muscle myosin | unknown |
| AK014338 | 74840 | Armet | extracellular space | tumor related |
| AK014579 | 74347 | | | unknown |
| NM_016893 | 53618 | Fut8 | Golgi apparatus;extracellular sp | misc |
| BC006905 | 75608 | | | immune response |
| AK027262 | 235402 | Lrrn6a | plasma membrane ? | cell adh ? |
| AK010555 | 69746 | | | unknown |
| NM_026004 | 107569 | Nt5c3 | eukaryotic translation initiatio | translation |
| BC017627 | 66995 | | nucleolus | unknown |
| NM_019718 | 56350 | Arl3 | Golgi apparatus | signaling |
| AK010939 | 68196 | Hsbp1 | extracellular;nucleus | transcription |
| NM_025668 | 66624 | | endoplasmic reticulum;integral t | misc |
| NM_009727 | 11980 | Atp8a1 | integral to membrane | transport, metabolism |
| NM_019794 | 56445 | Dnaja2 | membrane | translation |
| BC005603 | 68776 | Taf11 | transcription factor TFIID compl | transcription |
| AB039178 | | | | unknown |
| NM_019790 | 56363 | Tmeff2 | integral to membrane | signaling |
| AK010152 | 66556 | Drap1 | negative cofactor 2 complex | transcription |
| AK009584 | 69639 | Exosc8 | exosome (RNase complex);nucleus | transcription |
| NM_029457 | 75826 | Senp2 | nucleus | signaling |
| NM_028122 | 108052 | Slc14a1 | integral to membrane | transport |
| AK006273 | 69441 | | | unknown |
| NM_031392 | 83669 | Wdr6 | | unknown |

Colon-18 Colon-72 Colon-192 Breast-18 Breast-72 Breast-192 Lung-18 Lung-72 Lung-192

Figure 41

Figure 42

| ACCESS | LOCUS | GENE | LOCALIZATION | BIOLOGICAL FUNCTION |
|---|---|---|---|---|
| NM_019802 | 56316 | Ggcx | integral to membrane | misc |
| NM_009046 | 19698 | Relb | transcription factor complex | transcription |
| NM_008560 | 17200 | Mc2r | integral to membrane | signaling (receptor) |
| NM_009476 | 22269 | Upk2 | endoplasmic reticulum;extracellu | tumor related |
| AK013604 | 72880 | | | unknown |
| AK003735 | 52840 | D2Bwg0891e | | unknown |
| NM_023805 | 76257 | Slc38a3 | integral to plasma membrane | transport |

Figure 43

Figure 44

-0.2   0.0   0.2

Log$_2$(ratio)

| ACCESS | LOCUS | GENE | LOCALIZATION | BIOLOGICAL FUNCTION |
|---|---|---|---|---|
| NM_008229 | 15182 | Hdac2 | cytoplasm;histone deacetylase co | transcription |
| AK018560 | 67556 | Pigm | | metabolism ? |
| NM_007591 | 12317 | Calr | endoplasmic reticulum lumen;extr | cell adhesion |
| AK016037 | 75212 | Rnf121 | | protein degradation ? |
| NM_011040 | 18510 | Pax8 | transcription factor complex | transcription |
| NM_019477 | 50790 | Facl4 | cytoplasm;integral to membrane | metabolism |
| NM_010826 | 17540 | Mrvi1 | endoplasmic reticulum membrane;i | transport |
| NM_010274 | 14571 | Gpd2 | extracellular space;glycerol-3-p | lipid metabolism |
| AK011747 | 17237 | Mgrn1 | extracellular | apoptosis |

Colon-18
Colon-72
Colon-192
Breast-18
Breast-72
Breast-192
Lung-18
Lung-72
Lung-192

Log$_2$(ratio)

AK016037    NM_007591    AK018560    NM_008229

| | | |
|---|---|---|
| ■ 18 | | |
| ■ 72 | C | |
| ▩ 192 | | |
| ▢ 18 | | |
| ▢ 72 | B | |
| ▢ 192 | | |
| ▢ 18 | | |
| ▢ 72 | L | |
| ▢ 192 | | |

Log$_2$(ratio)

NM_019477    NM_011040

Log$_2$(ratio)

AK011747    NM_010274    NM_010826

EP 1 649 062 B1

## Figure 45

Log$_2$(ratio)

-0.3  -0.1  0.1  0.3

| ACCESS | LOCUS | GENE | LOCALIZATION | BIOLOGICAL FUNCTION |
|---|---|---|---|---|
| NM_026612 | 68198 | Ndufb2 | mitochondrion | transport |
| NM_016789 | 53324 | Nptx2 | extracellular space;integral to | Transport |
| AK003818 | 66248 | Alg5 | endoplasmic reticulum;integral t | metabolism |

Colon-18
Colon-72
Colon-192
Breast-18
Breast-72
Breast-192
Lung-18
Lung-72
Lung-192

Log$_2$(ratio)

0.0
-0.2
-0.4
-0.6

AK003818    NM_016789    NM_026612

| | 18 | |
|---|---|---|
| ■ | 72 | C |
| | 192 | |
| | 18 | |
| ▣ | 72 | B |
| □ | 192 | |
| | 18 | |
| ▣ | 72 | L |
| □ | 192 | |

Figure 46

Figure 47

DIFFERENTIALLY EXPRESSED GENES
(SELECTED)

A) *Breast Cancer*

| Accession | Gene | Description | Class | Region |
|---|---|---|---|---|
| NM_013465 | Ahsg | alpha-2-HS-glycoprotein | protein degradation (secreted) | prefrontal cortex |
| NM_007529 | Bcan | brevican | signaling / ecm | hypothalamus |
| AK014338 | Armet | arginine-rich, mutated in early stage tumors | tumor related | midbrain |

B) *Colon Cancer*

| Accession | Gene | Description | Class | Region |
|---|---|---|---|---|
| NM_026523 | Nmb | neuromedin B | signaling | prefrontal cortex |
| NM_007950 | Ereg | epiregulin | cell cycle | prefrontal cortex |
| NM_009215 | Sst | somatostatin | growth factor (secreted) | hypothalamus |
| NM_009129 | Scg2 | secretogranin II | growth factor (secreted) | hypothalamus |
| AK011747 | Mgrn1 | mahogunin, ring finger 1 | apoptosis | hypothalamus |
| NM_008497 | Lhb | luteinizing hormone beta | growth factor (secreted) | midbrain |
| NM_080639 | Timp4 | tissue inhibitor of metalloproteinase 4 | protein degradation | midbrain |

C) *Lung Cancer*

| Accession | Gene | Description | Class | Region |
|---|---|---|---|---|
| NM_026523 | Nmb | neuromedin B | signaling (soluble) | prefrontal cortex |
| NM_054069 | Psbpc1 | prostatic steroid binding protein C1 | signaling (soluble) | prefrontal cortex |
| AF162224 | Angptl3 | angiopoietin-like 3 | signaling | prefrontal cortex |
| NM_008734 | Nrg3 | neuregulin 3 | growth factor (secreted) | hypothalamus |
| NM_009215 | Sst | somatostatin | growth factor (secreted) | hypothalamus |
| NM_011025 | Oxt | oxytocin | signaling (soluble) | hypothalamus |
| NM_021704 | Cxcl12 | chemokine (C-X-C motif) ligand 12 | immune response (secreted) | hypothalamus |
| NM_011328 | Sct | secretin | signaling (secretion) | midbrain |
| NM_010883 | Ndph | Norrie disease homolog | signaling (secretion) | midbrain |

EP 1 649 062 B1

**Figure 48**

*(page 173)*

**DRUG TARGETING CANDIDATES**

A) *Breast Cancer*

| Accesion | Gene | Description | Class | Region |
|---|---|---|---|---|
| L32178 | Slc6a1 | solute carrier family 6 (neurotransmitter transporter, GABA), member 1 | transport | prefrontal cortex |
| NM_008285 | Hrh1 | histamine receptor H 1 | signaling (receptor) | prefrontal cortex |
| NM_018882 | Gpr56 | G protein-coupled receptor 56 | signaling (receptor) | prefrontal cortex |
| NM_020518 | | RIKEN cDNA 2210413P10 gene | Immune response (receptor) | prefrontal cortex |
| NM_023805 | Slc38a3 | solute carrier family 38, member 3 | transport | prefrontal cortex |
| AK011457 | Slitrk5 | SLIT and NTRK-like family, member 5 | misc | Hypothalamus |
| NM_016789 | Nptx2 | neuronal pentraxin 2 | Transport | Hypothalamus |
| AK005887 | Slc38a4 | solute carrier family 38, member 4 | transport | Hypothalamus |
| NM_023059 | AI256711 | expressed sequence AI256711 | signaling (receptor) | Hypothalamus |
| NM_008399 | Itgae | integrin, alpha E, epithelial-associated | Immune response (receptor) | Hypothalamus |
| NM_020293 | Cldn9 | claudin 9 | cell adh ? | Hypothalamus |
| NM_019772 | | RIKEN cDNA 1110004F10 gene | protein degradation ? | Hypothalamus |
| NM_007874 | Dp1 | deleted in polyposis 1 | tumor related | Hypothalamus |
| AJ276505 | Tnfrsf22 ? | tumor necrosis factor receptor superfamily, member 22 ? | signaling (receptor) | Hypothalamus |
| NM_007495 | Astn1 | astrotactin 1 | misc | Hypothalamus |
| NM_008377 | Lrig1 | leucine-rich repeats and Immunoglobulin-like domains 1 | tumor related | Hypothalamus |
| NM_011352 | Sema7a | sema domain, immunoglobulin domain (Ig), and GPI membrane anchor, (semaphorin) 7A | signaling (receptor) | Midbrain |
| NM_011929 | Clcn6 | chloride channel 6 | transport | Midbrain |

Figure 48
(page 2 of 3)

**B)** *Colon Cancer*

| Accesion | Gene | Description | Class | Region |
|---|---|---|---|---|
| NM_054046 | Def8 | differentially expressed in FDCP 8 | signaling (receptor) | prefrontal cortex |
| NM_023500 | Xkh | McLeod syndrome gene homolog | transport | prefrontal cortex |
| NM_009320 | Slc6a6 | solute carrier family 6 (neurotransmitter transporter, taurine), member 6 | transport | prefrontal cortex |
| NM_011176 | St14 | suppression of tumorigenicity 14 (colon carcinoma) | cell adh | prefrontal cortex |
| NM_026413 | Slco6b1 | solute carrier organic anion transporter family, member 6b1 | transport | prefrontal cortex |
| NM_026413 | Slco6b1 | solute carrier organic anion transporter family, member 6b1 | transport | prefrontal cortex |
| NM_029460 | | RIKEN cDNA 4930562N12 gene | signaling (receptor) | prefrontal cortex |
| AK012847 | | RIKEN cDNA 2810031L11 gene | unknown (receptor) | Hypothalamus |
| NM_007583 | Cacng2 | calcium channel, voltage-dependent, gamma subunit 2 | ion channel | Hypothalamus |
| D86949 | Plxna2 | plexin A2 | signaling (receptor) | Midbrain |
| NM_010781 | Mcpt6 | mast cell protease 6 | protein degradation | Midbrain |
| NM_009730 | Atrn | attractin | cell adh (receptor) | Midbrain |
| AF090738 | Irs2 | insulin receptor substrate 2 | signaling (receptor) | Midbrain |
| NM_028122 | Slc14a1 | solute carrier family 14 (urea transprorter), member 1 | transport | Midbrain |
| M34173 | Crry | complement receptor related protein | immune response (receptor) | Midbrain |
| AK004586 | | RIKEN cDNA 1200003O06 gene | transport | Midbrain |
| U22015 | Rxrip110 | retinoid X receptor interacting protein 110 | signaling (receptor) | Midbrain |

EP 1 649 062 B1

Figure 48
(page 3 of 3)

C) *Lung Cancer*

| Accession | Gene | Description | Class | Region |
|---|---|---|---|---|
| NM_010149 | Epor | erythropoietin receptor | signaling (receptor) | prefrontal cortex |
| NM_019967 | Dbccr1 | deleted in bladder cancer chromosome region candidate 1 (human) | misc | prefrontal cortex |
| NM_021543 | Pcdh8 | protocadherin 8 | cell adh | prefrontal cortex |
| NM_011012 | Oprl | opioid receptor-like | signaling (receptor) | prefrontal cortex |
| NM_008424 | Kcne1 | potassium voltage-gated channel, Isk-related subfamily, member 1 | ion channel | prefrontal cortex |
| AF083876 | Emp2 | epithelial membrane protein 2 | transport | prefrontal cortex |
| NM_021550 |  | RIKEN cDNA 1500002I11 gene | misc | prefrontal cortex |
| NM_013596 | Mc5r | melanocortin 5 receptor | signaling (receptor) | prefrontal cortex |
| NM_007936 | Epha4 | Eph receptor A4 | signaling (receptor) | Hypothalamus |
| NM_030716 | Kcnip2 | Kv channel-interacting protein 2 | ion channel | Hypothalamus |
| NM_007761 | Crcp | calcitonin gene-related peptide-receptor component protein | signaling (receptor) | Hypothalamus |
| X81365 | Cntn2 | contactin 2 | cell adh | Hypothalamus |
| NM_019799 | Rhcg | Rhesus blood group-associated C glycoprotein | transport | Hypothalamus |
| NM_008737 | Nrp | neuropilin | cell adh | Hypothalamus |
| NM_026660 |  | RIKEN cDNA 0610009O03 gene | Transport | Midbrain |
| AK006107 |  | RIKEN cDNA 1700019B16 gene | signaling (receptor) | Midbrain |
| NM_008984 | Ptprm | protein tyrosine phosphatase, receptor type, M | signaling (receptor) | Midbrain |
| NM_008135 | Slc6a9 | solute carrier family 6 (neurotransmitter transporter, glycine), member 9 | transport | Midbrain |
| AK012535 | AI256456 | expressed sequence AI256456 | unknown | Midbrain |
| NM_011201 | Ptpn1 | protein tyrosine phosphatase, non-receptor type 1 | signaling (receptor) | Midbrain |

Figure 49
Page 1 of 2

| Accession | Gene | Region | Tumor | Time | Ratio array | p-value array | Ratio real time | p-value real time | Validated |
|---|---|---|---|---|---|---|---|---|---|
| AK004714 | RIKEN cDNA 1200011M11 gene | Hypothalamus | Breast | 18 | -0.172 ± 0.020 | 0.014 | -0.053 ± 0.284 | 0.870 | No |
| | | | | 72 | -0.239 ± 0.076 | 0.048 | -0.226 ± 0.103 | 0.004 | Yes |
| | | | | 192 | 0.002 ± 0.052 | 0.969 | -0.137 ± 0.277 | 0.669 | |
| NM_007591 | calreticulin | Hypothalamus | Breast | 18 | 0.141 ± 0.023 | 0.003 | 0.013 ± 0.242 | 0.961 | No |
| | | | | 72 | 0.181 ± 0.105 | 0.226 | -0.153 ± 0.231 | 0.577 | |
| | | | | 192 | 0.085 ± 0.026 | 0.048 | ND | ND | |
| NM_011622 | target of myb1 homolog (TOM1) | Hypothalamus | Breast | 18 | -0.140 ± 0.006 | 0.029 | -0.488 ± 0.191 | 0.017 | Yes |
| | | | | 72 | -0.218 ± 0.017 | 0.049 | -0.278 ± 0.182 | 0.006 | Yes |
| | | | | 192 | 0.003 ± 0.003 | 0.375 | ND | ND | |
| NM_013615 | outer dense fiber of sperm tails 2 | Hypothalamus | Breast | 18 | 0.001 | ND | ND | ND | |
| | | | | 72 | -0.177 ± 0.031 | 0.010 | 0.183 ± 0.133 | 0.302 | No |
| | | | | 192 | -0.139 ± 0.046 | 0.092 | ND | ND | |
| NM_011202 | protein tyrosine phosphatase, non-receptor type 11 | Hypothalamus | Lung | 18 | 0.073 ± 0.020 | 0.035 | 0.386 ± 0.253 | 0.040 | Yes |
| | | | | 72 | -0.018 ± 0.016 | 0.395 | -0.562 ± 0.592 | 0.271 | |
| | | | | 192 | -0.015 ± 0.015 | 0.427 | ND | ND | |
| NM_027722 | RIKEN cDNA 4933436C10 gene | Hypothalamus | Lung | 18 | -0.030 ± 0.005 | 0.023 | ND | ND | |
| | | | | 72 | -0.052 ± 0.015 | 0.043 | 0.059 ± 0.236 | 0.826 | No |
| | | | | 192 | ND | ND | ND | ND | |
| X81365 | contactin 2 | Hypothalamus | Lung | 18 | 0.234 ± 0.003 | 0.009 | 0.491 ± 0.314 | 0.036 | Yes |
| | | | | 72 | 0.046 ± 0.026 | 0.221 | -0.068 ± 0.137 | 0.706 | |
| | | | | 192 | -0.046 ± 0.069 | 0.627 | ND | ND | |

EP 1 649 062 B1

Figure 49
Page 2 of 2

EP 1 649 062 B1

| | | | | | | | | | |
|---|---|---|---|---|---|---|---|---|---|
| AF408435 | protein regulator of cytokinesis 1 | Midbrain | Breast | 18 | 0.147 ± 0.050 | 0.100 | 0.113 ± 0.164 | 0.528 | |
| | | | | 72 | 0.156 ± 0.031 | 0.037 | 0.269 ± 0.150 | 0.215 | .No |
| | | | | 192 | 0.120 ± 0.110 | 0.473 | ND | ND | |
| AK003524 | RIKEN cDNA 1110007C02 gene | Midbrain | Breast | 18 | -0.502 ± 0.051 | 0.010 | 0.162 ± 0.193 | 0.448 | No |
| | | | | 72 | -0.098 | ND | -0.162 ± 0.239 | 0.568 | |
| | | | | 192 | 0.277 ± 0.002 | 0.003 | ND | ND | |
| NM_007836 | growth arrest and DNA-damage-inducible 45 alpha | Midbrain | Breast | 18 | -0.149 ± 0.013 | 0.008 | 0.159 ± 0.242 | 0.546 | No |
| | | | | 72 | -0.201 | ND | 0.245 ± 0.113 | 0.163 | |
| | | | | 192 | 0.029 | ND | ND | ND | |
| NM_008889 | protein phosphatase 1, regulatory (inhibitor) subunit 14B | Midbrain | Breast | 18 | -0.109 ± 0.027 | 0.153 | -0.080 ± 0.112 | 0.512 | |
| | | | | 72 | -0.185 ± 0.005 | 0.018 | -0.039 ± 0.299 | 0.907 | No |
| | | | | 192 | -0.025 ± 0.042 | 0.590 | ND | ND | |
| NM_019551 | Traf and Tnf receptor associated protein | Midbrain | Breast | 18 | -0.120 ± 0.004 | 0.023 | 0.237 ± 0.228 | 0.357 | No |
| | | | | 72 | -0.138 ± 0.050 | 0.225 | 0.270 ± 0.398 | 0.567 | |
| | | | | 192 | -0.144 ± 0.023 | 0.025 | -0.177 ± 0.309 | 0.626 | No |
| AK009856 | RIKEN cDNA 2310046M24 gene | Prefrontal cortex | Breast | 18 | -0.052 ± 0.082 | 0.593 | ND | ND | |
| | | | | 72 | 0.113 ± 0.081 | 0.296 | 0.488 ± 0.364 | 0.116 | |
| | | | | 192 | 0.163 ± 0.0003 | 0.001 | -0.277 ± 0.282 | 0.141 | No |
| NM_009717 | neurogenic differentiation 6 | Prefrontal cortex | Lung | 18 | 0.217 ± 0.082 | 0.078 | 0.092 ± 0.055 | 0.193 | |
| | | | | 72 | 0.098 ± 0.018 | 0.033 | 0.023 ± 0.145 | 0.884 | No |
| | | | | 192 | -0.026 ± 0.018 | 0.245 | ND | ND | |

| Access | Locus | Gene |
|---|---|---|
| AB020542 | 170938 | LOC170938 |
| AB035381 | 170759 | Cgi152 |
| AB039933 | 70335 | Dp1l1 |
| AB041586 | 76454 | |
| AB049650 | 75398 | Mrpl32 |
| AB056100 | 66902 | Mtap |
| AF039839 | | |
| AF059175 | 13867 | Erbb3 |
| AF061934 | 50782 | Rgs11 |
| AF140708 | 21462 | Tcp10c |
| AF168680 | 50766 | Crim1 |
| AF179996 | 20362 | Sept8 |
| AF193608 | 60364 | Donson |
| AF201662 | 17202 | Mc4r |
| AF224264 | 233908 | Fus |
| AF229644 | 54646 | DXImx48e |
| AF276974 | 192201 | |
| AF282272 | 258740 | MOR171-12 |
| AF282304 | 258276 | MOR224-12 |
| AF296075 | 81896 | Wdr10 |
| AF303106 | 110012 | Gtrgeo22 |
| AF317517 | 77889 | |
| AF326960 | 21380 | Tbx1 |
| AF342737 | 67378 | Bbs2 |
| AF343581 | 72322 | Xpo5 |
| AF369902 | 68291 | Mto1 |
| AF411834 | 20620 | Snk |
| AF440737 | 69362 | |
| AF448804 | 72162 | Dhx36 |
| AJ007376 | 26900 | Ddx3y |
| AJ133536 | 20955 | Sybl1 |
| AJ223782 | 235072 | Sept7 |
| AJ246002 | 50850 | Spg4 |
| AJ252147 | 66413 | |
| AJ308965 | 101739 | Psip2 |
| AJ400622 | 230157 | Tmeff1 |
| AK002252 | 68323 | |
| AK002293 | 12793 | Cnih |
| AK002297 | 71667 | |
| AK002298 | 71660 | Rarres2 |
| AK002309 | 68327 | |
| AK002386 | 109801 | Glo1 |
| AK002387 | 76073 | |
| AK002637 | 66689 | Btbd5 |
| AK002704 | 68365 | Rab14 |
| AK002757 | 56282 | Mrpl12 |
| AK002842 | 68386 | |
| AK003004 | 18111 | Nnat |
| AK003132 | 75406 | Ndufs7 |
| AK003209 | 75725 | |
| AK003358 | 68576 | |
| AK003450 | 74319 | |
| AK003542 | 73728 | |
| AK003571 | 68507 | Ppfia4 |
| AK003609 | 76308 | |

Figure 50-1

| | | |
|---|---|---|
| AK003794 | 97541 | Qars |
| AK003819 | 74777 | |
| AK003820 | 76295 | Atp11b |
| AK004041 | 68730 | |
| AK004090 | 73747 | |
| AK004139 | 68735 | |
| AK004191 | 66061 | |
| AK004472 | 68891 | |
| AK004559 | 66871 | Cpne8 |
| AK004627 | 74119 | |
| AK004676 | 67459 | |
| AK004814 | 97541 | Qars |
| AK004849 | 52428 | Rhpn2 |
| AK005001 | 74159 | Acbd5 |
| AK005010 | 71768 | |
| AK005015 | 246277 | Csad |
| AK005035 | 71775 | |
| AK005045 | 20637 | Snrp70 |
| AK005063 | 110351 | Rap1ga1 |
| AK005136 | 74189 | Phactr3 |
| AK005146 | 76499 | Clasp2 |
| AK005230 | 76338 | |
| AK005314 | 19342 | Rab4b |
| AK005423 | 76507 | Abp1 |
| AK005609 | 69315 | |
| AK006110 | 67080 | |
| AK006185 | 70373 | |
| AK006211 | 67630 | Samd8 |
| AK006273 | 69441 | |
| AK006650 | 73296 | Rhobtb3 |
| AK006739 | 22217 | Usp12 |
| AK006923 | 73476 | |
| AK007077 | 73572 | |
| AK007310 | 76669 | |
| AK007355 | 69035 | Zdhhc3 |
| AK007419 | 69047 | |
| AK007503 | 69101 | |
| AK007505 | 68118 | |
| AK007617 | 69127 | |
| AK007766 | 76510 | |
| AK007664 | 67893 | |
| AK007884 | 66229 | |
| AK008537 | 67035 | |
| AK008734 | 72349 | Dusp3 |
| AK008736 | 70153 | |
| AK008955 | 70397 | |
| AK009135 | 73919 | |
| AK009163 | 76730 | |
| AK009251 | 50529 | Mrps7 |
| AK009282 | 71897 | |
| AK009365 | 76437 | D11Bwg0414e |
| AK009522 | 71901 | |
| AK009749 | 69654 | |
| AK009813 | 269423 | |
| AK009886 | 71923 | |
| AK009888 | 69655 | |

Figure 50-2

| | |
|---|---|
| AK009937 | 71941 |
| AK010005 | 71924 Tube1 |
| AK010152 | 66556 Drap1 |
| AK010310 | 67755 Ddx47 |
| AK010328 | 69702 Ndufaf1 |
| AK010387 | 69748 |
| AK010466 | 76484 |
| AK010752 | 73694 |
| AK010963 | 70312 |
| AK011036 | 66230 Mrps28 |
| AK011064 | 70235 |
| AK011234 | 72459 |
| AK011330 | 72124 |
| AK011531 | 67788 |
| AK011540 | 71807 |
| AK011541 | 71810 |
| AK011725 | 69928 |
| AK011832 | 72465 Zfp131 |
| AK011853 | 72475 Ssbp3 |
| AK011862 | 28018 D7Wsu128e |
| AK011896 | 72486 |
| AK011950 | 67157 |
| AK012211 | 70998 |
| AK012283 | 72569 |
| AK012726 | 28036 D3Wsu161e |
| AK012740 | 66315 |
| AK012843 | 72668 |
| AK012847 | 72674 |
| AK012852 | 67197 |
| AK013022 | 69227 |
| AK013197 | 66390 |
| AK013276 | 66329 |
| AK013280 | 67229 |
| AK013304 | 52132 D7Ertd462e |
| AK013322 | 72774 Neil1 |
| AK013379 | 78771 |
| AK013427 | 104884 |
| AK013465 | 12348 Car11 |
| AK013595 | 72871 |
| AK013596 | 72929 |
| AK013604 | 72880 |
| AK013690 | 19328 Rab12 |
| AK013710 | 73051 |
| AK013727 | 14397 Gabra4 |
| AK013788 | 73047 |
| AK013965 | 68969 |
| AK013995 | 73078 |
| AK014175 | 73205 |
| AK014327 | 244672 |
| AK014393 | 74025 |
| AK014396 | 74020 Cpne4 |
| AK014435 | 55951 Brp44l |
| AK014446 | 52118 D7Ertd458e |
| AK014500 | 77047 |
| AK014610 | 74352 |
| AK014652 | 74574 |

Figure 50-3

| | |
|---|---|
| AK014695 | 71633 |
| AK014735 | 67392 |
| AK014764 | 74600 |
| AK014811 | 75801 |
| AK014830 | 80708 Pacsin3 |
| AK014834 | 75778 |
| AK014839 | 51938 D3Ertd789e |
| AK015338 | 231672 |
| AK015457 | 74655 |
| AK015536 | 106248 |
| AK015559 | 74946 |
| AK015640 | 70646 |
| AK015650 | 67509 |
| AK015717 | 75102 |
| AK015898 | 75141 Rasd2 |
| AK015929 | 70939 |
| AK015957 | 75207 |
| AK016083 | 75311 |
| AK016128 | 75359 |
| AK016247 | 18640 Pfkfb2 |
| AK016267 | 65103 Arl6ip6 |
| AK016282 | 75863 |
| AK016481 | 74356 |
| AK016545 | 74390 |
| AK016597 | 71027 |
| AK016612 | 74426 |
| AK016714 | 74405 |
| AK016715 | 76799 |
| AK016792 | |
| AK016857 | 71074 |
| AK016870 | 71176 Fbxo24 |
| AK016917 | 74446 |
| AK016936 | 71075 |
| AK017014 | 71281 |
| AK017214 | 71323 |
| AK017294 | 71331 |
| AK017442 | 71453 |
| AK017460 | 71448 |
| AK017493 | 67974 |
| AK017578 | 18189 Nrxn1 |
| AK017787 | 66663 Ube1dc1 |
| AK017798 | 70676 |
| AK017836 | 76742 Snx27 |
| AK018132 | 74008 |
| AK018146 | 17344 Miz1 |
| AK018159 | 18549 Pcsk2 |
| AK018254 | 50758 Fbxo13 |
| AK018389 | 71492 Bbs7 |
| AK018520 | 71544 |
| AK018573 | 71517 |
| AK018646 | 75761 |
| AK018662 | 74549 |
| AK018849 | 66510 |
| AK019095 | 78887 |
| AK019458 | 70122 Mllt3 |
| AK019466 | 77626 |

Figure 50-4

| | | |
|---|---|---|
| AK019515 | 77041 | |
| AK019527 | 78903 | Wrnip1 |
| AK019542 | 68259 | |
| AK019549 | 68187 | |
| AK019589 | 78109 | |
| AK019600 | 78795 | |
| AK019665 | 78174 | |
| AK019809 | 78051 | |
| AK019863 | 77634 | |
| AK019878 | 78935 | |
| AK019886 | 68151 | |
| AK019915 | 78271 | |
| AK019934 | 70255 | |
| AK019985 | 77521 | |
| AK020104 | 77733 | |
| AK020492 | 77305 | |
| AK020724 | 77578 | Bcl9 |
| AK021021 | 16423 | Cd47 |
| AK021076 | 67287 | |
| AK021099 | 77462 | |
| AK021208 | 77422 | |
| AK021227 | 78531 | |
| AL078630 | | |
| AY044265 | 235339 | Dlat |
| BC002198 | 102334 | Ankrd10 |
| BC002199 | 211556 | BC002199 |
| BC002226 | 170833 | Hook2 |
| BC002231 | 67134 | Nol5a |
| BC002298 | 19283 | Ptprz1 |
| BC003247 | 229725 | Mclc |
| BC003479 | 216820 | |
| BC003843 | 70356 | St13 |
| BC003901 | 107951 | Cdk9 |
| BC003914 | 58875 | |
| BC003932 | 67278 | |
| BC003957 | 78408 | |
| BC003988 | 83486 | Rbm5 |
| BC004020 | 11603 | Agm |
| BC004581 | 98432 | AI836256 |
| BC004640 | 97064 | |
| BC004653 | 67155 | |
| BC004690 | 216274 | BC004690 |
| BC004739 | 213211 | Rnf26 |
| BC004766 | 71799 | Ptcd1 |
| BC004771 | | |
| BC004831 | 228608 | Smox |
| BC005568 | 75689 | |
| BC005598 | 98221 | Ga17 |
| BC005658 | 69957 | Cdc16 |
| BC005659 | 171388 | Bnipl |
| BC005672 | 230784 | Sesn2 |
| BC005744 | 72144 | Slc37a3 |
| BC005786 | 70579 | |
| BC005792 | 170789 | Pte1 |
| BC006834 | 12226 | Btg1 |
| BC008101 | 68939 | |

Figure 50-5

211

| | |
|---|---|
| BC009141 | 75062 Sl3a3 |
| BC011091 | 109815 H47 |
| BC012975 | 14526 Gcg |
| BC013502 | 217353 Tmc6 |
| BC016080 | 103963 Rpn1 |
| BC016210 | 67017 |
| BC016398 | 19079 Prkab1 |
| BC016616 | 76022 |
| BC017625 | 68047 |
| BC018220 | 69190 |
| D50060 | 18553 Pace4 |
| D50872 | 19204 Ptafr |
| D83203 | 19271 Ptprj |
| K02138 | |
| L17305 | |
| M60510 | 83796 Smarcd2 |
| NM_007377 | 11302 Aatk |
| NM_007393 | 11461 Actb |
| NM_007440 | 11684 Alox12 |
| NM_007474 | 11833 Aqp8 |
| NM_007502 | 11933 Atp1b3 |
| NM_007531 | 12034 Bcap37 |
| NM_007564 | 12192 Zfp36l1 |
| NM_007581 | 12297 Cacnb3 |
| NM_007583 | 12300 Cacng2 |
| NM_007600 | 12333 Capn1 |
| NM_007618 | 12401 Serpina6 |
| NM_007622 | 12412 Cbx1 |
| NM_007624 | 12417 Cbx3 |
| NM_007668 | 12568 Cdk5 |
| NM_007674 | 12592 Cdx4 |
| NM_007688 | 12632 Cfl2 |
| NM_007693 | 12652 Chga |
| NM_007709 | 12705 Cited1 |
| NM_007728 | 12810 Coch |
| NM_007828 | 13144 Dapk3 |
| NM_007841 | 13209 Ddx6 |
| NM_007862 | 13383 Dlgh1 |
| NM_007868 | 13405 Dmd |
| NM_007898 | 13595 Ebp |
| NM_007899 | 13601 Ecm1 |
| NM_007922 | 13712 Elk1 |
| NM_007933 | 13808 Eno3 |
| NM_007980 | 14079 Fabp2 |
| NM_008014 | 14208 Ppm1g |
| NM_008015 | 14209 Fin14 |
| NM_008033 | 14272 Fnta |
| NM_008072 | 14403 Gabrd |
| NM_008076 | 14409 Gabrr2 |
| NM_008091 | 14462 Gata3 |
| NM_008113 | 14570 Arhgdig |
| NM_008115 | 14586 Gfra2 |
| NM_008133 | 14661 Glud |
| NM_008138 | 14678 Gnai2 |
| NM_008142 | 14688 Gnb1 |
| NM_008160 | 14775 Gpx1 |

Figure 50-6

212

| | | |
|---|---|---|
| NM_008161 | 14778 | Gpx3 |
| NM_008226 | 15166 | Hcn2 |
| NM_008230 | 15186 | Hdc |
| NM_008233 | 15193 | Hdgfrp2 |
| NM_008297 | 15500 | Hsf2 |
| NM_008301 | 15512 | Hspa2 |
| NM_008307 | 15547 | Htf9c |
| NM_008350 | 16156 | Il11 |
| NM_008358 | 16169 | Il15ra |
| NM_008403 | 16413 | Itgb1bp1 |
| NM_008418 | 16491 | Kcna3 |
| NM_008451 | 16594 | Klc2 |
| NM_008550 | 17160 | Man2b2 |
| NM_008580 | 26408 | Map3k5 |
| NM_008590 | 17294 | Mest |
| NM_008606 | 17385 | Mmp11 |
| NM_008707 | 18107 | Nmt1 |
| NM_008712 | 18125 | Nos1 |
| NM_008733 | 18175 | Nrap |
| NM_008744 | 18208 | Ntn1 |
| NM_008761 | 18301 | Fxyd5 |
| NM_008771 | 18436 | P2rx1 |
| NM_008797 | 18563 | Pcx |
| NM_008858 | 18760 | Prkcm |
| NM_008871 | 18787 | Serpine1 |
| NM_008896 | 330260 | Pon2 |
| NM_008915 | 19057 | Ppp3cc |
| NM_008920 | 19074 | Prg2 |
| NM_008946 | 19175 | Psmb6 |
| NM_008974 | 19244 | Ptp4a2 |
| NM_008990 | 19294 | Pvrl2 |
| NM_009011 | 19359 | Rad23b |
| NM_009020 | 19374 | Rag2 |
| NM_009025 | 19414 | Rasa3 |
| NM_009029 | 19645 | Rb1 |
| NM_009046 | 19698 | Relb |
| NM_009055 | 19724 | Rfx1 |
| NM_009069 | 19769 | Rit1 |
| NM_009070 | 19826 | Rnps1 |
| NM_009104 | 20135 | Rrm2 |
| NM_009106 | 20166 | Rtkn |
| NM_009130 | 20255 | Scg3 |
| NM_009136 | 20284 | Scrg1 |
| NM_009255 | 20720 | Serpine2 |
| NM_009261 | 20744 | Spnr |
| NM_009273 | 20813 | Srp14 |
| NM_009276 | 20819 | Srst |
| NM_009282 | 20842 | Stag1 |
| NM_009320 | 21366 | Slc6a6 |
| NM_009357 | 21766 | Tex261 |
| NM_009460 | 22218 | Ubl1 |
| NM_009467 | 22238 | Ugt2b5 |
| NM_009472 | 22253 | Unc5c |
| NM_009503 | 269523 | Vcp |
| NM_009539 | 22637 | Zap70 |
| NM_009551 | 22682 | Zfp216 |

Figure 50-7

| | |
|---|---|
| NM_009582 | 26404 Map3k12 |
| NM_009624 | 11515 Adcy9 |
| NM_009688 | 11798 Birc4 |
| NM_009707 | 11856 Arhgap6 |
| NM_009732 | 11998 Avp |
| NM_009748 | 12068 Bet1 |
| NM_009806 | 12361 Cask |
| NM_009831 | 12450 Ccng1 |
| NM_009846 | 12484 Cd24a |
| NM_009848 | 12495 Entpd1 |
| NM_009881 | 12593 Cdyl |
| NM_009895 | 12700 Cish |
| NM_009950 | 12905 Cradd |
| NM_010073 | 13481 Dpm2 |
| NM_010101 | 13610 Edg3 |
| NM_010195 | 14160 Gpr49 |
| NM_010243 | 14348 Fut9 |
| NM_010254 | 14428 Galr2 |
| NM_010255 | 14431 Gamt |
| NM_010313 | 14697 Gnb5 |
| NM_010358 | 14862 Gstm1 |
| NM_010362 | 14873 Gsto1 |
| NM_010417 | 15203 Heph |
| NM_010509 | 15976 Ifnar2 |
| NM_010577 | 16402 Itga5 |
| NM_010609 | 16530 Kcnk7 |
| NM_010638 | 16601 Bteb1 |
| NM_010710 | 16870 Lhx2 |
| NM_010798 | 17319 Mif |
| NM_010799 | 17330 Minpp1 |
| NM_010911 | 18041 Nfs1 |
| NM_010934 | 18166 Npy1r |
| NM_010950 | 18223 Numbl |
| NM_011012 | 18389 Oprl |
| NM_011018 | 18412 Sqstm1 |
| NM_011021 | 18420 Otp |
| NM_011025 | 18429 Oxt |
| NM_011051 | 18570 Pdcd6 |
| NM_011126 | 18843 Plunc |
| NM_011154 | |
| NM_011177 | 19144 Prss18 |
| NM_011192 | 19192 Psme3 |
| NM_011251 | 19654 Rbm6 |
| NM_011256 | 19679 Rdgb2 |
| NM_011334 | 12727 Clcn4-2 |
| NM_011522 | 20974 Syngr3 |
| NM_011601 | 21893 Tlm |
| NM_011643 | 22063 Trpc1 |
| NM_011791 | 23808 Ash2l |
| NM_011804 | |
| NM_011828 | 23908 Hs2st1 |
| NM_011855 | 23963 Odz1 |
| NM_011870 | 23991 Cib1 |
| NM_011908 | 24109 Ubl3 |
| NM_011921 | 26358 Aldh1a7 |
| NM_011949 | 26413 Mapk1 |

Figure 50-8

| | |
|---|---|
| NM_011961 | 26432 Plod2 |
| NM_011962 | 26433 Plod3 |
| NM_011990 | 26570 Slc7a11 |
| NM_012019 | 26926 Pdcd8 |
| NM_012045 | 26971 Pla2g2f |
| NM_012048 | 27015 Polk |
| NM_013556 | 15452 Hprt |
| NM_013596 | 17203 Mc5r |
| NM_013614 | 18263 Odc |
| NM_013625 | 18472 Pafah1b1 |
| NM_013641 | 19216 Ptger1 |
| NM_013736 | 27224 Tceb3 |
| NM_013754 | 27356 Insl6 |
| NM_013759 | 27361 Sepr |
| NM_013789 | 27414 Gnefr |
| NM_013841 | 22365 Vps45 |
| NM_013867 | 29815 Bcar3 |
| NM_013909 | 30840 Fbxl6 |
| NM_013916 | 30929 AL022832 |
| NM_013932 | 30959 Ddx25 |
| NM_015762 | 50493 Txnrd1 |
| NM_015830 | 50817 Solh |
| NM_016664 | 50994 Mata2 |
| NM_016692 | 16319 Incenp |
| NM_016703 | 50907 Preb |
| NM_016719 | 50915 Grb14 |
| NM_016737 | 20867 Stip1 |
| NM_016750 | 51788 H2afz |
| NM_016769 | 17127 Madh3 |
| NM_016778 | 51800 Bok |
| NM_016806 | 53379 Hnrpa2b1 |
| NM_016909 | 53424 Tsnax |
| NM_016910 | 53892 Ppm1d |
| NM_017379 | 53857 Tuba8 |
| NM_017395 | 53970 Rfx5 |
| NM_018731 | 11944 Atp4a |
| NM_018733 | 20265 Scn1a |
| NM_018734 | 55932 Gbp3 |
| NM_018743 | 102247 AU041707 |
| NM_018755 | 54381 |
| NM_018761 | 54366 Catnal1 |
| NM_018772 | 55950 Bri3 |
| NM_018798 | 54609 Ubqln2 |
| NM_018808 | 81489 Dnajb1 |
| NM_018851 | 56045 Samhd1 |
| NM_018879 | 56032 Tusc4 |
| NM_019409 | 18377 Omg |
| NM_019435 | 104130 Np15 |
| NM_019501 | 56075 |
| NM_019505 | 56077 Dgke |
| NM_019518 | 56149 Grasp |
| NM_019537 | 56088 Dscr2 |
| NM_019571 | 56224 Tm4sf9 |
| NM_019587 | 140571 Plxnb3 |
| NM_019629 | 56290 Prp15 |
| NM_019631 | 56277 |

Figure 50-9

| | |
|---|---|
| NM_019635 | 56274 Stk3 |
| NM_019639 | 22190 Ubc |
| NM_019643 | 56306 Tera |
| NM_019648 | 56307 Metap2 |
| NM_019649 | 56457 Clptm1 |
| NM_019659 | 56379 Kcnj1 |
| NM_019660 | 56309 Mycbp |
| NM_019668 | 22209 Ube2a |
| NM_019676 | 18799 Plcd |
| NM_019691 | 14802 Gria4 |
| NM_019701 | 56365 Clcnk1l |
| NM_019703 | 56421 Pfkp |
| NM_019714 | 21888 Tle4 |
| NM_019745 | 56426 Pdcd10 |
| NM_019768 | 56397 Morf4l2 |
| NM_019772 | 56372 |
| NM_019773 | 56382 Rab9 |
| NM_019776 | 56463 AL033314 |
| NM_019816 | 56321 Aatf |
| NM_019836 | 56412 |
| NM_019868 | 56258 Hnrph2 |
| NM_019921 | 56697 Akap10 |
| NM_019951 | 56529 |
| NM_019959 | 56745 C1qtnf1 |
| NM_019968 | 56795 Arm1 |
| NM_019983 | 56715 Rabgef1 |
| NM_020006 | 56699 Cdc42ep4 |
| NM_020024 | 24075 Taf10 |
| NM_020050 | 56786 |
| NM_020558 | 57316 C1d |
| NM_020584 | 57321 Terf2ip |
| NM_020610 | 78593 Nrip3 |
| NM_020614 | 21340 Taf1b |
| | |
| NM_020616 | 57373 ICRFP703B1614Q5.6 |
| NM_020619 | 57377 Gcs1 |
| NM_020624 | 57391 |
| NM_021316 | 57754 |
| NM_021332 | 14652 Glp1r |
| NM_021371 | 140904 Caln1 |
| NM_021406 | 58217 Trem1 |
| NM_021460 | 16889 Lip1 |
| NM_021483 | 58869 Pex2 |
| NM_021489 | 58992 F12 |
| NM_021504 | 59007 Ngly1 |
| NM_021516 | 17169 Mark3 |
| NM_021518 | 59021 Rab2 |
| NM_021530 | 59033 Slc4a8 |
| NM_021549 | 59047 Pnkp |
| NM_021567 | 59092 Pcbp4 |
| NM_022321 | 64099 Parvg |
| NM_022992 | 65106 Arl6ip5 |
| NM_023167 | 66163 Mrpl4 |
| NM_023178 | 66233 Dmap1 |
| NM_023179 | 66237 Atp6v1g2 |
| NM_023210 | 66471 Anp32e |

Figure 50-10

216

| | | |
|---|---|---|
| NM_023450 | 68355 | |
| NM_023500 | 22439 | Xkh |
| NM_023516 | 69573 | |
| NM_023587 | 70757 | Ptplb |
| NM_023633 | 71952 | |
| NM_023697 | 105014 | Rdh14 |
| NM_023805 | 76257 | Slc38a3 |
| NM_023835 | 76681 | Trim12 |
| NM_023852 | 67295 | Rab3c |
| NM_023884 | 78255 | |
| NM_024189 | 67057 | Yaf2 |
| NM_024197 | 67273 | Ndufa10 |
| NM_024201 | 67433 | |
| NM_024244 | 71721 | |
| NM_024441 | 69253 | Hspb2 |
| NM_024477 | 209683 | AI428795 |
| NM_024479 | 79565 | BC002286 |
| NM_025273 | 13180 | Pcbd |
| NM_025310 | 56095 | Epcs3 |
| NM_025335 | 66074 | |
| NM_025387 | 66154 | |
| NM_025409 | 66191 | |
| NM_025415 | 66197 | Cks2 |
| NM_025449 | 66257 | Nicn1 |
| NM_025450 | 66258 | Mrps17 |
| NM_025596 | 66494 | |
| NM_025683 | 66646 | Rpe |
| NM_025718 | 66705 | Dnase1l2 |
| NM_025790 | 66834 | Them2 |
| NM_025822 | 66880 | |
| NM_025826 | 66885 | Acadsb |
| NM_025835 | 66904 | Pccb |
| NM_025876 | 66971 | Cdk5rap1 |
| NM_025983 | 67126 | Atp5e |
| NM_026040 | 67222 | |
| NM_026065 | 67270 | |
| NM_026096 | 67334 | |
| NM_026107 | 52712 | D11Ertd714e |
| NM_026162 | 67448 | Plxdc2 |
| NM_026187 | 52231 | D1Ertd161e |
| NM_026306 | 67674 | |
| NM_026309 | 67678 | Lsm3 |
| NM_026401 | 67840 | Mrp63 |
| NM_026411 | 67851 | |
| NM_026417 | 67864 | |
| NM_026423 | 67873 | |
| NM_026435 | 67890 | |
| NM_026487 | 67979 | |
| NM_026518 | 68031 | |
| NM_026551 | 68087 | |
| NM_026559 | 52700 | D11Ertd672e |
| NM_026819 | 52585 | Dhrs1 |
| NM_027324 | 14057 | Sfxn1 |
| NM_029478 | 75909 | |
| NM_030252 | 80284 | BC003266 |
| NM_030262 | 80294 | |

Figure 50-11

| | | |
|---|---|---|
| NM_030558 | 80733 | Car15 |
| NM_030611 | 83702 | Akr1c6 |
| NM_030708 | 80892 | |
| NM_030719 | 80909 | Gats |
| NM_030880 | 80708 | Pacsin3 |
| NM_031184 | 83396 | Gli5 |
| NM_031877 | 83767 | Wasf1 |
| NM_033327 | 94187 | Ebfaz |
| NM_033370 | 70349 | Copb1 |
| NM_033562 | 116891 | BC005682 |
| NM_033568 | 27681 | D11Moh34 |
| NM_053105 | 93688 | Klhl1 |
| NM_053183 | 94213 | Ddx50 |
| NM_053193 | 94230 | Cpsf1 |
| NM_053271 | 116838 | Rims2 |
| NM_080289 | 76238 | Grhpr |
| U51279 | 12048 | Bcl2l |
| U53591 | 14112 | Fau-ps3 |
| U69136 | 12565 | Cdh9 |
| U70033 | 20541 | Slc8a1 |
| X16301 | | |
| X17647 | 18212 | Ntrk2 |
| X54327 | 107508 | Eprs |
| X66323 | 22596 | Xrcc5 |
| Y11717 | 15400 | Hoxa3 |
| Y12713 | 27282 | Erv4 |
| Y17852 | 14548 | Gdap3 |

Figure 50-12

Figure 5 A

Log₂(ratio)

-0.3  -0.1  0.1  0.3

| ACCESS | LOCUS | GENE | LOCALIZATION | BIOLOGICAL FUNCTION |
|---|---|---|---|---|
| AK013995 | 73078 | | mitochondrion | misc |
| AK008537 | 67035 | | cytoplasm | translation |
| NM_019773 | 56382 | Rab9 | Golgi apparatus | transport |
| AK013965 | 68969 | | cytoplasm | translation |
| NM_026559 | 52700 | D11Ertd672e | | transport |
| NM_019518 | 56149 | Grasp | cytoplasm;perinuclear space;plas | signaling |
| NM_023587 | 70757 | Ptplb | extracellular;integral to membra | signaling ? |
| NM_007440 | 11684 | Alox12 | plasma membrane | lipid metabolism |
| AK011853 | 72475 | Ssbp3 | nucleus | transcription |
| NM_008297 | 15500 | Hsf2 | cytoplasm;nucleus;polytene chrom | transcription |
| AK018254 | 50758 | Fbxo13 | | unknown |
| AK011862 | 28018 | D7Wsu128e | | protein degradation ? |
| NM_021549 | 59047 | Pnkp | telomere | misc |
| AK012843 | 72668 | | | traffic |
| NM_030558 | 80733 | Car15 | | metabolism |
| AK019458 | 70122 | Mllt3 | transcription factor TFIIF compl | tumor related |
| NM_023852 | 67295 | Rab3c | synaptic vesicle | transport |
| AK021021 | 16423 | Cd47 | integral to plasma membrane | cell adh - signaling (receptor) |
| NM_025826 | 66885 | Acadsb | mitochondrion | metabolism |
| NM_025387 | 66154 | | integral to membrane | unknown |
| NM_013916 | 30929 | AL022832 | | unknown |
| AF168680 | 50766 | Crim1 | extracellular | growth factor |
| D50060 | 18553 | Pace4 | membrane | signaling (receptor) |
| AK017798 | 70676 | | | signaling |
| NM_007377 | 11302 | Aatk | integral to membrane | apoptosis |
| NM_009055 | 19724 | Rfx1 | intermediate filament;nucleus | transcription |
| AK013604 | 72880 | | | unknown |
| AF061934 | 50782 | Rgs11 | heterotrimeric G-protein complex | signaling |
| NM_025409 | 66191 | | extracellular space;integral to | unknown |
| NM_010358 | 14862 | Gstm1 | cytoplasm | metabolism |
| NM_009046 | 19698 | Relb | transcription factor complex | transcription |
| AK011531 | 67788 | | | unknown |
| NM_022321 | 64099 | Parvg | cytoskeleton | cell adh |
| AK017493 | 67974 | | cytoplasm | cell cycle |
| NM_019435 | 104130 | Np15 | integral to membrane;mitochondri | unknown |
| AK009163 | 76730 | | | unknown |
| NM_019868 | 56258 | Hnrph2 | nucleus;ribonucleoprotein comple | traffic |
| AK012852 | 67197 | | cytoplasm | transcription ? |
| NM_011643 | 22063 | Trpc1 | integral to plasma membrane | signaling (receptor) |
| AF059175 | 13867 | Erbb3 | integral to membrane;intracellul | signaling (receptor) |
| NM_010950 | 18223 | Numbl | cytoplasm | misc |
| NM_009881 | 12593 | Cdyl | chromatin;nucleus | cell cycle |
| AK019915 | 78271 | | | unknown |
| AB056100 | 66902 | Mtap | | misc |
| U69136 | 12565 | Cdh9 | integral to membrane | cell adh |
| AK016792 | | | | unknown |
| AK012740 | 66315 | | integral to membrane | unknown |
| AF282272 | 258740 | MOR171-12 | integral to membrane | signaling |
| AK013276 | 66329 | | membrane | signaling (receptor) |
| NM_024441 | 69253 | Hspb2 | cytosol | translation |
| NM_016750 | 51788 | H2afz | nucleosome;nucleus | cell cycle |
| NM_023450 | 68355 | | integral to membrane | unknown |
| NM_013754 | 27356 | Insl6 | extracellular space | growth factor |
| AK004627 | 74119 | | | unknown |
| NM_013909 | 30840 | Fbxl6 | | protein degradation |
| NM_033370 | 70349 | Copb1 | Golgi membrane;Golgi vesicle;cyt | transport |
| NM_008091 | 14462 | Gata3 | microtubule;nucleus | transcription |
| BC005786 | 70579 | | | unknown |
| NM_020024 | 24075 | Taf10 | focal adhesion;transcription fac | transcription |
| U51279 | 12048 | Bcl2l | integral to membrane;mitochondri | apoptosis |

BNS  BS

Figure 51B

Log₂(ratio) -0.3 -0.1 0.1 0.3

| ACCESS' | LOCUS | GENE | LOCALIZATION | BIOLOGICAL FUNCTION |
|---|---|---|---|---|
| NM_008142 | 14688 | Gnb1 | heterotrimeric G-protein complex | signaling |
| Y12713 | 27282 | Erv4 | | virus |
| NM_021460 | 16889 | Lip1 | extracellular space;lysosome | lipid metabolism |
| NM_025335 | 66074 | | extracellular matrix | unknown |
| AK008955 | 70397 | | | unknown |
| NM_007980 | 14079 | Fabp2 | soluble fraction | transport |
| BC003843 | 70356 | St13 | integral to membrane | metabolism |
| AK014396 | 74020 | Cpne4 | mitochondrial inner membrane | metabolism |
| NM_007922 | 13712 | Elk1 | membrane;nucleus | transcription |
| AK005230 | 76338 | | Golgi apparatus;endoplasmic reti | transport - signaling |
| AK013788 | 73047 | | | unknown |
| NM_007622 | 12412 | Cbx1 | centric heterochromatin;nucleopl | misc |
| NM_007393 | 11461 | Actb | cytoskeleton | ctsk |
| NM_007898 | 13595 | Ebp | endoplasmic reticulum membrane;i | lipid metabolism |
| NM_016719 | 50915 | Grb14 | | signaling (receptor) |
| NM_007564 | 12192 | Zfp36l1 | cytoplasm;nucleus | transcription |
| AL078630 | | | | unknown |
| AK007310 | 76669 | | | transcription |
| AK020492 | 77305 | | | transcription |
| AK016714 | 74405 | | | unknown |
| NM_013625 | 18472 | Pafah1b1 | centrosome;microtubule associate | lipid metabolism |
| NM_007841 | 13209 | Ddx6 | eukaryotic translation initiatio | misc |
| AK003004 | 18111 | Nnat | | transport |
| U70033 | 20541 | Slc8a1 | integral to plasma membrane | transport |
| AK014695 | 71633 | | | unknown |
| NM_007624 | 12417 | Cbx3 | chromatin;nucleus | transcription |
| NM_018851 | 56045 | Samhd1 | | immune response |
| AK016128 | 75359 | | | unknown |
| AF342737 | 67378 | Bbs2 | integrin complex | misc (secreted) ? |
| NM_008606 | 17385 | Mmp11 | extracellular matrix;extracellul | protein degradation |
| NM_018808 | 81489 | Dnajb1 | cytoplasm | translation |
| AF369902 | 68291 | Mto1 | mitochondrion | metabolism |
| NM_016737 | 20867 | Stip1 | Golgi apparatus;nucleus | misc |
| AF411834 | 20620 | Snk | phosphorylase kinase complex | cell cycle |
| AK011725 | 69928 | | cytoskeleton | unknown |
| AK021227 | 78531 | | | unknown |
| NM_016692 | 16319 | Incenp | chromosome-, pericentric region; | cell cycle |
| AK018646 | 75761 | | | apoptosis |
| NM_011601 | 21893 | Tlm | extracellular;integral to membra | tumor related |
| NM_023167 | 66163 | Mrpl4 | ribosome | translation |
| AK018662 | 74549 | | | unknown |
| NM_027324 | 14057 | Sfxn1 | integral to membrane;mitochondri | transport |
| NM_019631 | 56277 | | integral to plasma membrane | tumor related (receptor) |
| NM_019537 | 56088 | Dscr2 | integral to plasma membrane;memb | cell cycle |
| NM_007600 | 12333 | Capn1 | cytoplasm | protein degradation |
| NM_026487 | 67979 | | endoplasmic reticulum | unknown |
| NM_022992 | 65106 | Arl6ip5 | integral to membrane | transport |
| NM_010362 | 14873 | Gsto1 | cytoplasm | misc |
| NM_013736 | 27224 | Tceb3 | nucleus | transcription |
| NM_011012 | 18389 | Oprl | integral to plasma membrane | signaling (receptor) |
| AK021099 | 77462 | | | unknown |
| NM_053183 | 94213 | Ddx50 | nucleolus | translation |
| NM_007828 | 13144 | Dapk3 | cytoplasm;nucleus | apoptosis |
| NM_009106 | 20166 | Rtkn | intracellular;membrane | signaling |
| AK017787 | 66663 | Ube1dc1 | chloroplast | protein degradation |
| NM_008707 | 18107 | Nmt1 | cytoplasm | misc |
| BC005658 | 69957 | Cdc16 | | cell cycle |
| NM_008771 | 18436 | P2rx1 | integral to membrane | apoptosis |
| AK008736 | 70153 | | | unknown |
| NM_026417 | 67864 | | integral to membrane | unknown |
| AB035381 | 170759 | Cgi152 | integral to membrane | metabolism |
| AK010466 | 76484 | | | unknown |
| NM_023697 | 105014 | Rdh14 | extracellular space | metabolism |

BNS
BS

Figure 5/ C

-0.3  -0.1  0.1  0.3

Log$_2$(ratio)

| ACCESS | LOCUS | GENE | LOCALIZATION | BIOLOGICAL FUNCTION |
|---|---|---|---|---|
| NM_020584 | 57321 | Terf2lp | chromosome-, telomeric region;nu | cell cycle |
| AK003609 | 76308 | | Golgi apparatus;endoplasmic reti | signaling |
| BC003957 | 78408 | | | transport |
| BC002198 | 102334 | Ankrd10 | | transcription |
| AF448804 | 72162 | Dhx36 | | translation |
| AK010387 | 69748 | | | metabolism ? |
| NM_021371 | 140904 | Caln1 | | signaling |
| AF326960 | 21380 | Tbx1 | microtubule;nucleus | transcription |
| NM_019816 | 56321 | Aatf | mitochondrial inner membrane;nuc | apoptosis |
| AK010328 | 69702 | Ndufaf1 | mitochondrion | translation |
| AK006739 | 22217 | Usp12 | | protein degradation |
| BC018220 | 69190 | | | unknown |
| AK003794 | 97541 | Qars | | translation |
| AK013596 | 72929 | | | unknown |
| AK014500 | 77047 | | dynein complex | ctsk |
| AK010752 | 73694 | | | unknown |
| NM_019836 | 56412 | | extracellular space;integral to | unknown |
| AK017214 | 71323 | | | tumor related |
| NM_020616 | 57373 | ICRFP703B1614Q5.6 | | unknown |
| NM_019648 | 56307 | Metap2 | large ribosomal subunit | protein degradation |
| L17305 | | | | unknown |
| NM_009806 | 12361 | Cask | actin cytoskeleton;basolateral p | signaling |
| NM_015830 | 50817 | Solh | cytoplasm | unknown |
| BC016398 | 19079 | Prkab1 | cytoplasm;nucleus | lipid metabolism |
| AK004472 | 68891 | | | signaling (receptor) |
| AK015559 | 74946 | | | unknown |
| AK007355 | 69035 | Zdhhc3 | Golgi apparatus;integral to memb | protein degradation |
| NM_025835 | 66904 | Pccb | biotin carboxylase complex;extra | metabolism |
| NM_019659 | 56379 | Kcnj1 | voltage-gated potassium channel | ion cannel |
| NM_033568 | 27681 | D11Moh34 | plasma membrane | unknown |
| AK017442 | 71453 | | | ctsk ? |
| AK011064 | 70235 | | membrane | unknown |
| NM_016909 | 53424 | Tsnax | mitochondrial inner membrane;nuc | misc |
| NM_020050 | 56786 | | extracellular space;integral to | signaling? |
| NM_011522 | 20974 | Syngr3 | integral to plasma membrane | unknown |
| NM_011018 | 18412 | Sqstm1 | cytosol | protein degradation |
| NM_008014 | 14208 | Ppm1g | nucleus;protein serine-threonine | cell cycle |
| NM_023633 | 71952 | | cytosol | unknown |
| AK017460 | 71448 | | | unknown |
| NM_008797 | 18563 | Pcx | mitochondrion | metabolism |
| NM_008115 | 14586 | Gfra2 | extracellular space;membrane | signaling (receptor) |
| NM_013759 | 27361 | Sepr | | metabolism |
| BC003901 | 107951 | Cdk9 | transcription elongation factor | cell cycle |
| AK003358 | 68576 | | | unknown |
| BC003914 | 58875 | | mitochondrion | misc |
| AK004090 | 73747 | | | unknown |
| AK009886 | 71923 | | | unknown |
| NM_019772 | 56372 | | integral to membrane | protein degradation ? |
| AK004191 | 66061 | | | ctsk ? |
| NM_019691 | 14802 | Gria4 | extracellular space;integral to | signaling (receptor) |
| NM_010255 | 14431 | Gamt | cytoplasm | metabolism |
| AK016917 | 74446 | | membrane | unknown |
| NM_010934 | 18166 | Npy1r | integral to plasma membrane | signaling |
| NM_009624 | 11515 | Adcy9 | integral to plasma membrane | misc |
| AK007766 | 76510 | | | unknown |
| AB041586 | 76454 | | | unknown |
| NM_018731 | 11944 | Atp4a | integral to membrane | metabolism |
| NM_008418 | 16491 | Kcna3 | voltage-gated potassium channel | transport |
| AK014811 | 75801 | | | unknown |
| NM_020614 | 21340 | Taf1b | RNA polymerase I transcription f | cell cycle |
| NM_016769 | 17127 | Madh3 | nucleus;plasma membrane | transcription |
| NM_007933 | 13808 | Eno3 | phosphopyruvate hydratase comple | metabolism |
| AK016857 | 71074 | | | unknown |

BNS  BS

221

Log$_2$(ratio)

-0.3  -0.1  0.1  0.3

| ACCESS | LOCUS | GENE | LOCALIZATION | BIOLOGICAL FUNCTION |
|---|---|---|---|---|
| NM_008761 | 18301 | Fxyd5 | extracellular space;integral to | transport |
| AK019466 | 77626 | | integral to membrane | misc |
| NM_018798 | 54609 | Ubqln2 | endoplasmic reticulum membrane | unknown |
| AK009282 | 71897 | | membrane | signaling (receptor) |
| NM_013867 | 29815 | Bcar3 | actin filament | signaling (GEF) |
| AK003819 | 74777 | | | misc |
| NM_024189 | 67057 | Yaf2 | nucleoplasm | unknown |
| NM_011921 | 26358 | Aldh1a7 | | metabolism |
| NM_009025 | 19414 | Rasa3 | plasma membrane | signaling (GAP) |
| NM_053271 | 116838 | Rims2 | | signaling |
| AK002842 | 68386 | | | unknown |
| NM_023805 | 76257 | Slc38a3 | integral to plasma membrane | transport |
| BC016616 | 76022 | | | unknown |
| AK018520 | 71544 | | | cell cycle |
| NM_008550 | 17160 | Man2b2 | extracellular space;lysosome | metabolism |
| NM_011961 | 26432 | Plod2 | endoplasmic reticulum;extracellu | metabolism |
| NM_007531 | 12034 | Bcap37 | mitochondrion | signaling |
| AF303106 | 110012 | Gtrgeo22 | integral to membrane | misc |
| X66323 | 22596 | Xrcc5 | cytoplasm;single-stranded DNA de | misc |
| AF317517 | 77889 | | nucleus | transcription |
| AF179996 | 20362 | sep-08 | | ctsk |
| NM_025415 | 66197 | Cks2 | | cell cycle |
| AK015457 | 74655 | | | unknown |
| NM_030880 | 80708 | Pacsin3 | Golgi trans face | transport |
| AK019549 | 68187 | | | unknown |
| NM_026306 | 67674 | | | unknown |
| NM_021516 | 17169 | Mark3 | microtubule cytoskeleton | ctsk ? |
| BC012975 | 14526 | Gcg | extracellular space | signaling |
| NM_009472 | 22253 | Unc5c | extracellular space;integral to | signaling (receptor) |
| AK005609 | 69315 | | | unknown |
| NM_053193 | 94230 | Cpsf1 | mRNA cleavage and polyadenylatio | transcription |
| NM_007502 | 11933 | Atp1b3 | sodium-potassium-exchanging ATPa | transport |
| AK013690 | 19328 | Rab12 | intracellular | tumor related |
| NM_008403 | 16413 | Itgb1bp1 | | signaling |
| AK015640 | 70646 | | | unknown |
| NM_016778 | 51800 | Bok | | apoptosis |
| NM_026162 | 67448 | Plxdc2 | extracellular space;integral to | signaling |
| NM_011791 | 23808 | Ash2l | | transcription |
| NM_019501 | 56075 | | chloroplast | misc |
| NM_010638 | 16601 | Bteb1 | transcription factor complex | transcription |
| NM_008915 | 19057 | Ppp3cc | protein serine-threonine phospha | signaling |
| AJ400622 | 230157 | Tmeff1 | | protein degradation |
| NM_007728 | 12810 | Coch | extracellular matrix;extracellul | misc |
| NM_008580 | 26408 | Map3k5 | cytoplasm | signaling (MAPK) |
| AK005063 | 110351 | Rap1ga1 | | signaling |
| NM_009255 | 20720 | Serpine2 | extracellular space | protein degradation (secreted) |
| NM_009130 | 20255 | Scg3 | cytoplasm;extracellular space | misc |
| NM_008358 | 16169 | Il15ra | extracellular space;integral to | immune response (receptor) |
| AK004559 | 66871 | Cpne8 | membrane | transport |
| AK012847 | 72674 | | integral to membrane | unknown |
| NM_026819 | 52585 | Dhrs1 | | metabolism |
| NM_026423 | 67873 | | eukaryotic translation initiatio | translation |
| NM_020624 | 57391 | | integral to membrane | unknown |
| NM_018733 | 20265 | Scn1a | integral to membrane;membrane fr | ion cannel |
| AY044265 | 235339 | Dlat | pyruvate dehydrogenase complex ( | metabolism |
| AK011541 | 71810 | | | unknown |
| NM_009503 | 269523 | Vcp | Golgi apparatus;endoplasmic reti | transport |
| AK011234 | 72459 | | | translation |
| NM_019703 | 56421 | Pfkp | 6-phosphofructokinase complex | metabolism |
| NM_019505 | 56077 | Dgke | integral to membrane | signaling, transport |
| NM_018761 | 54366 | Catnal1 | zonula adherens | cell adh |
| AK016267 | 65103 | Arl6ip6 | integral to membrane | unknown |
| AK019985 | 77521 | | non-muscle myosin | signaling |

BNS  BS

Figure 52 A

Log$_2$(ratio)  -0.2  0.0  0.2

BNS  BS

| ACCESS | LOCUS | GENE | LOCALIZATION | BIOLOGICAL FUNCTION |
|---|---|---|---|---|
| NM_025450 | 66258 | Mrps17 | mitochondrion;ribosome | translation |
| NM_008896 | 330260 | Pon2 | extracellular;membrane | misc |
| AK019549 | 68187 | | | unknown |
| AK007884 | 66229 | | large ribosomal subunit | translation |
| AK005423 | 76507 | Abp1 | extracellular space;membrane | transport |
| AF201662 | 17202 | Mc4r | integral to membrane;plasma memb | signaling (receptor) |
| NM_019983 | 56715 | Rabgef1 | plasma membrane | unknown |
| NM_011012 | 18389 | Oprl | integral to plasma membrane | signaling (receptor) |
| NM_021518 | 59021 | Rab2 | Golgi apparatus;endoplasmic reti | signaling |
| NM_013556 | 15452 | Hprt | cytoplasm | metabolism |

Figure 52 B

-0.2  0.0  0.2
Log$_2$(ratio)

| ACCESS | LOCUS | GENE | LOCALIZATION | BIOLOGICAL FUNCTION |
|---|---|---|---|---|
| AK002309 | 68327 | | cytoplasm | unknown |
| AK013197 | 66390 | | | translation ? |
| BC016616 | 76022 | | | unknown |
| AK014652 | 74574 | | | unknown |
| NM_011126 | 18843 | Plunc | extracellular;integral to plasma | tumor related |
| AJ133536 | 20955 | Sybl1 | Golgi apparatus;endoplasmic reti | translation |
| AK007864 | 67893 | | membrane ? | unknown |
| AJ308965 | 101739 | Psip2 | extracellular space | transcription ? |
| NM_009276 | 20819 | Srst | nucleus | transcription |
| AK011330 | 72124 | | nuclear pore | traffic |
| Y17852 | 14548 | Gdap3 | mitochondrion | unknown |
| BC003247 | 229725 | Mclc | | transport |
| NM_007688 | 12632 | Cfl2 | cytoskeleton;nucleus | ctsk |
| AF039839 | | | | unknown |
| AJ007376 | 26900 | Ddx3y | | cell cycle |
| NM_053105 | 93688 | Klhl1 | cytoskeleton | ctsk |
| NM_021483 | 58869 | Pex2 | peroxisomal membrane | unknown |
| NM_009282 | 20842 | Stag1 | nucleus | cell cycle |
| AK016267 | 65103 | Arl6ip6 | integral to membrane | unknown |
| AK006923 | 73476 | | | unknown |
| NM_023884 | 78255 | | nucleosome | signaling (GEF) |
| NM_010101 | 13610 | Edg3 | integral to plasma membrane | signaling |
| AK016715 | 76799 | | | signaling ? |
| AK003820 | 76295 | Atp11b | integral to membrane;plasma memb | metabolism |
| AK020724 | 77578 | Bcl9 | nucleus | immune response |
| NM_020610 | 78593 | Nrip3 | | unknown |
| AJ252147 | 66413 | | proteasome complex (sensu Eukary | unknown |
| AJ246002 | 50850 | Spg4 | nucleus | misc |
| NM_009831 | 12450 | Ccng1 | nuclear cyclin-dependent protein | cell cycle |
| NM_007668 | 12568 | Cdk5 | cytosol;filopodium;lamellipodium | cell cycle |
| NM_033562 | 116891 | BC005682 | integral to membrane | misc |
| BC002226 | 170833 | Hook2 | | unknown |
| AK018573 | 71517 | | membrane | unknown |
| NM_025449 | 66257 | Nicn1 | | unknown |
| NM_009460 | 22218 | Ubl1 | nuclear pore | cell cycle |
| AK014695 | 71633 | | | unknown |
| AF343581 | 72322 | Xpo5 | nucleus | transport |
| NM_016664 | 50994 | Mata2 | | tumor related |
| AK019589 | 78109 | | | unknown |
| AK014327 | 244672 | | | unknown |
| NM_020558 | 57316 | C1d | transcriptional repressor comple | transcription |
| NM_019635 | 56274 | Stk3 | cytoplasm;protein kinase CK2 com | apoptosis |
| BC004653 | 67155 | | nucleoplasm | transcription |
| AK012211 | 70998 | | | transcription |
| NM_019660 | 56309 | Mycbp | cytoplasm;nucleus | transcription |
| AK014435 | 55951 | Brp44l | | unknown |
| NM_017395 | 53970 | Rfx5 | intermediate filament | transcription |
| AK014735 | 67392 | | | unknown |
| NM_025409 | 66191 | | extracellular space;integral to | unknown |
| NM_021316 | 57754 | | integral to membrane | unknown |
| AK015640 | 70646 | | | unknown |
| NM_022992 | 65106 | Arl6ip5 | integral to membrane | transport |
| NM_009551 | 22682 | Zfp216 | | transcription ? |
| NM_009748 | 12068 | Bet1 | Golgi membrane;endoplasmic retic | transport |
| AJ223782 | 235072 | sep-07 | actin cytoskeleton | ctsk |
| NM_011051 | 18570 | Pdcd6 | cytoplasm | apoptosis |
| AK009251 | 50529 | Mrps7 | mitochondrial small ribosomal su | translation |
| NM_033327 | 94187 | Ebfaz | transcription factor complex | transcription |
| NM_026518 | 68031 | | cytoplasm | protein degradation ? |
| AK005136 | 74189 | Phactr3 | nucleus | unknown |
| NM_018755 | 54381 | | cytoplasm | protein degradation |
| NM_010609 | 16530 | Kcnk7 | integral to plasma membrane | ion channel |
| NM_008015 | 14209 | Fin14 | integral to membrane | unknown |

BNS  BS

Figure 52 C

-0.2  0.0  0.2
Log₂(ratio)

| ACCESS | LOCUS | GENE | LOCALIZATION | BIOLOGICAL FUNCTION |
|---|---|---|---|---|
| AK013379 | 78771 | | integral to membrane | unknown |
| NM_010911 | 18041 | Nfs1 | cytosol;extracellular space;mito | metabolism |
| NM_010313 | 14697 | Gnb5 | heterotrimeric G-protein complex | signaling |
| AK012283 | 72569 | | | unknown |
| AK010152 | 66556 | Drap1 | negative cofactor 2 complex | transcription |
| NM_019701 | 56365 | Clcnk1l | integral to membrane | ion channel |
| AK015717 | 75102 | | | unknown |
| AK004139 | 68735 | | mitochondrion;ribosome | translation |
| AK013322 | 72774 | Neil1 | nucleus | misc |
| NM_019409 | 18377 | Omg | extracellular space;integral to | signaling |
| AK019915 | 78271 | | | unknown |
| AK013304 | 52132 | D7Ertd462e | cytoplasm | unknown |
| AK003450 | 74319 | | membrane | unknown |
| NM_024197 | 67273 | Ndufa10 | extracellular space;mitochondrio | metabolism |
| AK005146 | 76499 | Clasp2 | Golgi apparatus;cytoplasmic micr | ctsk |
| AK019886 | 68151 | | | unknown |
| AF229644 | 54646 | DXImx48e | integral to membrane;microtubule | signaling |
| AK004676 | 67459 | | nucleus | transport |
| NM_011334 | 12727 | Clcn4-2 | integral to plasma membrane | ion channel |
| AB049650 | 75398 | Mrpl32 | mitochondrial large ribosomal su | translation |
| NM_021332 | 14652 | Glp1r | extracellular space;integral to | signaling (receptor) |
| NM_017379 | 53857 | Tuba8 | microtubule | ctsk |
| NM_011949 | 26413 | Mapk1 | cytoplasm;nucleus | signaling |
| NM_024244 | 71721 | | membrane | unknown |
| NM_009011 | 19359 | Rad23b | repairosome | cell cycle |
| NM_013841 | 22365 | Vps45 | Golgi apparatus;membrane | transport |
| BC011091 | 109815 | H47 | integral to membrane | unknown |
| AK014610 | 74352 | | intracellular | transcription |
| AK014839 | 51938 | D3Ertd789e | myosin ? | ctsk ? |
| AF224264 | 233908 | Fus | cytoplasm;transcription factor T | unknown |
| NM_010358 | 14862 | Gstm1 | cytoplasm | metabolism |
| AK014175 | 73205 | | | unknown |
| NM_009104 | 20135 | Rrm2 | cytoplasm;ribonucleoside-diphosp | cell cycle |
| NM_008761 | 18301 | Fxyd5 | extracellular space;integral to | transport |
| NM_011870 | 23991 | Cib1 | synaptic vesicle | signaling |
| NM_031184 | 83396 | Gli5 | transcription factor complex | transcription |
| NM_008797 | 18563 | Pcx | mitochondrion | metabolism |
| AK002252 | 68323 | | | unknown |
| NM_009020 | 19374 | Rag2 | alpha DNA polymerase-;primase co | cell cycle |
| BC004739 | 213211 | Rnf26 | | apoptosis |
| NM_011154 | | | | unknown |
| AK006273 | 69441 | | | unknown |
| X17647 | 18212 | Ntrk2 | cytosol;integral to membrane;pla | signaling (receptor) |
| BC005598 | 98221 | Ga17 | signalosome complex | protein degradation |
| NM_030719 | 80909 | Gats | | unknown |
| AK011036 | 66230 | Mrps28 | mitochondrion | translation |
| NM_013789 | 27414 | Gnefr | cytoplasm;nucleus | misc |
| NM_025790 | 66834 | Them2 | | misc |
| AK003132 | 75406 | Ndufs7 | extracellular space;mitochondrio | metabolism |
| NM_008133 | 14661 | Glud | mitochondrion | metabolism |
| AK009888 | 69655 | | | cell adh |
| AF193608 | 60364 | Donson | | unknown |
| NM_007728 | 12810 | Coch | extracellular matrix;extracellul | misc |
| NM_007709 | 12705 | Cited1 | nucleus | transcription ? |
| NM_019951 | 56529 | | endoplasmic reticulum;integral t | protein degradation |
| NM_008160 | 14775 | Gpx1 | mitochondrion | metabolism |
| NM_026401 | 67840 | Mrp63 | mitochondrial ribosome | misc |
| NM_021489 | 58992 | F12 | extracellular space | misc (secreted) |
| AK016247 | 18640 | Pfkfb2 | cytosol | metabolism |
| NM_010798 | 17319 | Mif | extracellular space | immune response |
| NM_019714 | 21888 | Tle4 | nucleus | transcription |
| AK019515 | 77041 | | lysosome | metabolism |
| AK011540 | 71807 | | | misc |

BNS  BS

Figure 52 D

Log$_2$(ratio)

-0.2　0.0　0.2

| ACCESS | LOCUS | GENE | LOCALIZATION | BIOLOGICAL FUNCTION |
|---|---|---|---|---|
| NM_010073 | 13481 | Dpm2 | endoplasmic reticulum;integral t | metabolism |
| BC004020 | 11603 | Agrn | extracellular space | signaling (secreted) |
| NM_008990 | 19294 | Pvrl2 | extracellular space;integral to | cell adh |
| BC004771 | | | | unknown |
| BC002298 | 19283 | Ptprz1 | extracellular matrix;extracellul | signaling (receptor) |
| NM_019676 | 18799 | Plcd | cytoplasm | signaling |
| AK019095 | 78887 | | | unknown |
| NM_025876 | 66971 | Cdk5rap1 | extracellular space | cell cycle |
| NM_009950 | 12905 | Cradd | caspase complex;intracellular | apoptosis |
| BC004690 | 216274 | BC004690 | cytoskeleton | unknown |
| AK005314 | 19342 | Rab4b | intracellular | Transport |
| NM_007899 | 13601 | Ecm1 | extracellular matrix;extracellul | ecm |
| AK007503 | 69101 | | | unknown |
| NM_011251 | 19654 | Rbm6 | spliceosome complex | misc |
| AK003209 | 75725 | | | unknown |
| NM_026187 | 52231 | D1Ertd161e | | transcription ? |
| AK003358 | 68576 | | | unknown |
| NM_008226 | 15166 | Hcn2 | integral to membrane | ion channel |
| NM_026096 | 67334 | | nucleosome;nucleus | unknown |
| AK016936 | 71075 | | | unknown |
| NM_010417 | 15203 | Heph | extracellular space | transport |
| NM_009582 | 26404 | Map3k12 | cytoplasm;plasma membrane | signaling (MAPK) |
| AK019527 | 78903 | Wrnip1 | DNA replication factor C complex | misc |
| NM_013932 | 30959 | Ddx25 | | translation |
| NM_016910 | 53892 | Ppm1d | protein serine-threonine phospha | signaling |
| BC008101 | 68939 | | | signaling |
| NM_010254 | 14428 | Galr2 | integral to membrane | signaling (receptor) |
| AK009937 | 71941 | | | transcription ? |
| NM_008590 | 17294 | Mest | extracellular space | protein degradation |
| AK019665 | 78174 | | mitochondrion | metabolism |
| BC004640 | 97064 | | ubiquitin ligase complex | transcription |
| NM_030252 | 80284 | BC003266 | extracellular space | unknown |
| AK006185 | 70373 | | | unknown |
| NM_011256 | 19679 | Rdgb2 | integral to membrane;intracellul | transport |
| AF061934 | 50782 | Rgs11 | heterotrimeric G-protein complex | signaling |
| NM_033370 | 70349 | Copb1 | Golgi membrane;Golgi vesicle;cyt | transport |
| NM_011962 | 26433 | Plod3 | endoplasmic reticulum;extracellu | metabolism |
| NM_025596 | 66494 | | mitochondrion | transport ? |
| AK002298 | 71660 | Rarres2 | extracellular space | misc |
| AK009522 | 71901 | | | unknown |
| AF296075 | 81896 | Wdr10 | cytoplasm | unknown |
| NM_019639 | 22190 | Ubc | nucleus | protein degradation |
| NM_008138 | 14678 | Gnai2 | plasma membrane | signaling (receptor) |
| NM_020619 | 57377 | Gcs1 | endoplasmic reticulum | misc |
| NM_010243 | 14348 | Fut9 | Golgi apparatus;integral to memb | metabolism |
| NM_008072 | 14403 | Gabrd | extracellular space;integral to | signaling (receptor) |
| AK020104 | 77733 | | integral to membrane ? | unknown |
| NM_007581 | 12297 | Cacnb3 | voltage-gated calcium channel co | ion channel |
| NM_030262 | 80294 | | | unknown |
| NM_009732 | 11998 | Avp | extracellular space | growth factor (secreted) |
| BC005792 | 170789 | Pte1 | peroxisome | metabolism |
| AB035381 | 170759 | Cgi152 | integral to membrane | metabolism |
| NM_026551 | 68087 | | | metabolism ? |
| NM_009539 | 22637 | Zap70 | phosphoinositide 3-kinase comple | immune response (receptor) |
| NM_008161 | 14778 | Gpx3 | extracellular space | metabolism |
| AK010963 | 70312 | | | unknown |
| BC004581 | 98432 | AI836256 | | unknown |
| AK013465 | 12348 | Car11 | cytoplasm | metabolism |
| NM_008451 | 16594 | Klc2 | kinesin complex | ctsk |
| NM_018772 | 55950 | Bri3 | integral to membrane | transport |
| AK003542 | 73728 | | mitochondrial inner membrane | unknown |
| AK008734 | 72349 | Dusp3 | | signaling |
| BC018220 | 69190 | | | unknown |

BNS  BS

Figure 53 A

-0.3  -0.1  0.1  0.3

$Log_2(ratio)$

| ACCESS | LOCUS | GENE | LOCALIZATION | BIOLOGICAL FUNCTION |
|---|---|---|---|---|
| NM_009136 | 20284 | Scrg1 | extracellular space | misc |
| BC013502 | 217353 | Tmc6 | integral to membrane | transport |
| NM_018743 | 102247 | AU041707 | integral to membrane | metabolism |
| AK018849 | 66510 | | membrane ? | unknown |
| NM_023179 | 66237 | Atp6v1g2 | hydrogen-transporting ATPase V1 | metabolism |
| NM_016703 | 50907 | Preb | integral to membrane;nucleus | transcription |
| NM_023210 | 66471 | Anp32e | cytoplasmic vesicle;nucleus | Signaling |
| AL078630 | | | | unknown |
| AK014393 | 74025 | | | unknown |
| AK003609 | 76308 | | Golgi apparatus;endoplasmic reti | signaling |
| AK017014 | 71281 | | | unknown |
| NM_011192 | 19192 | Psme3 | proteasome activator complex;pro | protein degradation |
| AK015898 | 75141 | Rasd2 | | signaling |
| AK018146 | 17344 | Miz1 | nucleus;septin ring | transcription |
| NM_021406 | 58217 | Trem1 | extracellular space;integral to | signaling (receptor) |
| AK011896 | 72486 | | cytoplasm;extracellular space;in | unknown |
| BC004581 | 98432 | AI836256 | | unknown |
| AK016267 | 65103 | Arl6ip6 | integral to membrane | unknown |
| AK018389 | 71492 | Bbs7 | | unknown |
| D83203 | 19271 | Ptprj | extracellular space;integral to | signaling |
| AK015338 | 231672 | | | signaling ? |
| NM_019668 | 22209 | Ube2a | nucleus | protein degradation |
| NM_008946 | 19175 | Psmb6 | proteasome core complex (sensu E | protein degradation |
| NM_020006 | 56699 | Cdc42ep4 | cytoskeleton | ctsk |
| NM_007618 | 12401 | Serpina6 | extracellular space | protein degradation |
| AK002386 | 109801 | Glo1 | | unknown |
| NM_018879 | 56032 | Tusc4 | | cell cycle |

BNS BS

**Figure 53 B**

-0.3  -0.1  0.1  0.3
Log$_2$(ratio)

| ACCESS | LOCUS | GENE | LOCALIZATION | BIOLOGICAL FUNCTION |
|---|---|---|---|---|
| NM_009846 | 12484 | Cd24a | extracellular space;plasma membr | immune response |
| NM_021504 | 59007 | Ngly1 | cytoplasm;nucleus | protein degradation |
| AK017294 | 71331 | | | unknown |
| AK019934 | 70255 | | | unknown |
| AF440737 | 69362 | | extracellular space | misc (secreted) ? |
| NM_023835 | 76681 | Trim12 | cytoplasm | unknown |
| NM_008113 | 14570 | Arhgdig | cytoplasm | signaling - ctsk |
| NM_007474 | 11833 | Aqp8 | integral to plasma membrane;mito | transport |
| NM_009688 | 11798 | Birc4 | cytosol | apoptosis |
| X54327 | 107508 | Eprs | | translation ? |
| BC005672 | 230784 | Sesn2 | extrachromosomal circular DNA;nu | cell cycle |
| NM_031877 | 83767 | Wasf1 | actin cytoskeleton | ctsk |
| AK011832 | 72465 | Zfp131 | | transport |
| NM_011908 | 24109 | Ubl3 | | unknown |
| NM_080289 | 76238 | Grhpr | | metabolism |
| NM_019643 | 56306 | Tera | | unknown |
| NM_008974 | 19244 | Ptp4a2 | soluble fraction | signaling |
| NM_008230 | 15186 | Hdc | extrachromosomal circular DNA | metabolism |
| NM_024477 | 209683 | AI428795 | | unknown |
| NM_024201 | 67433 | | | unknown |
| AK011950 | 67157 | | | unknown |
| NM_030708 | 80892 | | extracellular;nucleus | transcription |
| NM_008307 | 15547 | Htf9c | mitochondrial inner membrane | misc |
| NM_009895 | 12700 | Cish | cytoplasm;plasma membrane | signaling |
| AK016612 | 74426 | | | unknown |
| NM_008871 | 18787 | Serpine1 | extracellular space | protein degradation |
| NM_018734 | 55932 | Gbp3 | cytosol | immune response |
| BC003932 | 67278 | | | unknown |
| AK005001 | 74159 | Acbd5 | integral to membrane | transport |
| NM_008301 | 15512 | Hspa2 | cytoplasm | translation |
| AK014830 | 80708 | Pacsin3 | Golgi trans face | ctsk |
| NM_026309 | 67678 | Lsm3 | | misc |
| AK019863 | 77634 | | | translation |
| AK007505 | 68118 | | | unknown |
| NM_009357 | 21766 | Tex261 | extracellular space;integral to | lipid metabolism |
| NM_026435 | 67890 | | | unknown |
| AK004041 | 68730 | | | unknown |
| AK005010 | 71768 | | | unknown |
| NM_024479 | 79565 | BC002286 | endoplasmic reticulum | protein degradation ? |
| AK014764 | 74600 | | | translation |
| NM_008920 | 19074 | Prg2 | cytoplasm;extracellular space | growth factor (secreted) |
| K02138 | | | | unknown |
| AK015536 | 106248 | | | unknown |
| NM_030611 | 83702 | Akr1c6 | cytoplasm | metabolism |
| AB039933 | 70335 | Dp1l1 | integral to membrane | transport |
| NM_019959 | 56745 | C1qtnf1 | extracellular space | signaling (receptor) ? |
| AK006923 | 73476 | | | unknown |
| AK018159 | 18549 | Pcsk2 | Golgi apparatus;extracellular sp | translation |
| AK013710 | 73051 | | | unknown |
| BC017625 | 68047 | | | unknown |
| NM_019768 | 56397 | Morf4l2 | chromatin;nucleus | transcription |
| AK013427 | 104884 | | | signaling |
| NM_025683 | 66646 | Rpe | | metabolism |
| AF282304 | 258276 | MOR224-12 | integral to membrane | signaling (receptor) |
| NM_026107 | 52712 | D11Ertd714e | transcription factor complex | transcription |
| NM_008350 | 16156 | Il11 | extracellular space | immune response |
| NM_026040 | 67222 | | | unknown |
| AK016545 | 74390 | | | unknown |
| BC005744 | 72144 | Slc37a3 | extracellular space;integral to | transport |
| NM_013596 | 17203 | Mc5r | integral to membrane | signaling |
| AK013022 | 69227 | | | unknown |
| NM_008858 | 18760 | Prkcm | Golgi apparatus;cytosol;extracel | signaling |
| X16301 | | | | unknown |

BNS  BS

228

Figure 53 C

| ACCESS | LOCUS | GENE | LOCALIZATION | BIOLOGICAL FUNCTION |
|--------|-------|------|--------------|---------------------|
| NM_009070 | 19826 | Rnps1 | viral nucleocapsid | transcription |
| BC005659 | 171388 | Bnipl | membrane | apoptosis |
| NM_025718 | 66705 | Dnase1l2 | extracellular space | misc |
| NM_011177 | 19144 | Prss18 | cytoplasm;extracellular space | protein degradation |
| AK021208 | 77422 | | | unknown |
| AK005015 | 246277 | Csad | | metabolism |
| NM_009069 | 19769 | Rlt1 | plasma membrane | signaling |
| AK006110 | 67080 | | | unknown |
| AF276974 | 192201 | | extracellular space | unknown |
| NM_016806 | 53379 | Hnrpa2b1 | nucleus;ribonucleoprotein comple | transcription |
| BC003479 | 216820 | | mitochondrion | metabolism |
| AK002757 | 56282 | Mrpl12 | mitochondrial ribosome | translation |
| NM_010799 | 17330 | Minpp1 | endoplasmic reticulum;extracellu | metabolism |
| NM_007674 | 12592 | Cdx4 | transcription factor complex | transcription |
| AK016481 | 74356 | | | unknown |
| NM_023516 | 69573 | | extracellular space;integral to | unknown |
| AK003571 | 68507 | Ppfia4 | | signaling (receptor) ? |
| NM_019649 | 56457 | Clptm1 | aspartate carbamoyltransferase c | misc |
| M60510 | 83796 | Smarcd2 | nucleus ? | transcription ? |
| AK016870 | 71176 | Fbxo24 | | misc |
| NM_025273 | 13180 | Pcbd | cytoplasm;nucleus | misc |
| AK019878 | 78935 | | | unknown |
| NM_008712 | 18125 | Nos1 | cytoplasm;synapse | signaling |
| AK016597 | 71027 | | | unknown |
| AK002293 | 12793 | Cnih | integral to membrane | signaling |
| AK009135 | 73919 | | | unknown |
| NM_019629 | 56290 | Prp15 | | unknown |
| NM_009848 | 12495 | Entpd1 | basal lamina;integral to membran | signaling (receptor) |
| AK014834 | 75778 | | | unknown |
| BC005568 | 75689 | | integral to membrane | unknown |
| NM_021567 | 59092 | Pcbp4 | cytoplasm;nucleus;ribonucleoprot | transcription |
| NM_021530 | 59033 | Slc4a8 | integral to membrane | transport |
| NM_008076 | 14409 | Gabrr2 | axon;extracellular space;integra | signaling (receptor) |
| AK007077 | 73572 | | | unknown |
| NM_009273 | 20813 | Srp14 | signal recognition particle | signaling |
| BC016080 | 103963 | Rpn1 | endoplasmic reticulum;extracellu | lipid metabolism |
| NM_012019 | 26926 | Pdcd8 | cytosol;mitochondrial intermembr | apoptosis |
| AF140708 | 21462 | Tcp10c | | signaling (receptor) ? |
| NM_029478 | 75909 | | membrane | unknown |
| NM_019587 | 140571 | Plxnb3 | extracellular space;integral to | signaling (receptor) |
| AK004814 | 97541 | Qars | | misc |
| AK018132 | 74008 | | lysosome | signaling |
| BC002199 | 211556 | BC002199 | extracellular | unknown |
| BC009141 | 75062 | Sf3a3 | spliceosome complex | Transcription |
| AK013280 | 67229 | | spliceosome complex | transcription |
| Y11717 | 15400 | Hoxa3 | transcription factor complex | transcription |
| BC004766 | 71799 | Ptcd1 | ribosome | unknown |
| AK012726 | 28036 | D3Wsu161e | | unknown |
| NM_009261 | 20744 | Spnr | microtubule cytoskeleton | ctsk |
| AK015650 | 67509 | | | unknown |
| AK009365 | 76437 | D11Bwg0414e | | unknown |
| NM_008233 | 15193 | Hdgfrp2 | extracellular space;nucleus | growth factor |
| AK013727 | 14397 | Gabra4 | integral to plasma membrane | signaling |
| AK015957 | 75207 | | | unknown |
| AB020542 | 170938 | LOC170938 | transcription factor complex | unknown |
| NM_011855 | 23963 | Odz1 | extracellular;integral to plasma | protein degradation |
| NM_007868 | 13405 | Dmd | cytoskeleton;membrane;synapse | ctsk |
| AK009813 | 269423 | | | unknown |
| NM_011804 | | | | unknown |
| BC016210 | 67017 | | extracellular space;integral to | unknown |
| NM_026065 | 67270 | | mitochondrion | unknown |
| NM_019745 | 56426 | Pdcd10 | | apoptosis |
| NM_019776 | 56463 | AL033314 | | transcription |

BNS BS

**Figure 53 D**

-0.3  -0.1  0.1  0.3

Log$_2$(ratio)

| ACCESS | LOCUS | GENE | LOCALIZATION | BIOLOGICAL FUNCTION |
|---|---|---|---|---|
| NM_011021 | 18420 | Otp | transcription factor complex | transcription |
| NM_010509 | 15976 | Ifnar2 | extracellular space;integral to | signaling (receptor) |
| NM_012045 | 26971 | Pla2g2f | | signaling |
| NM_011990 | 26570 | Slc7a11 | integral to membrane | transport |
| NM_008033 | 14272 | Fnta | cytoplasm | misc |
| AK007617 | 69127 | | | tumor related |
| NM_025310 | 56095 | Epcs3 | membrane | misc |
| AK004849 | 52428 | Rhpn2 | cytosol | signaling |
| AK014446 | 52118 | D7Ertd458e | | tumor related |
| NM_026411 | 67851 | | | unknown |
| NM_025835 | 66904 | Pccb | biotin carboxylase complex;extra | metabolism |
| AK013595 | 72871 | | | unknown |
| U70033 | 20541 | Slc8a1 | integral to plasma membrane | transport |
| BC006834 | 12226 | Btg1 | | apoptosis |
| AK010005 | 71924 | Tube1 | pericentriolar material | ctsk |
| AK006211 | 67630 | Samd8 | integral to membrane | signaling |
| AK021076 | 67287 | | | unknown |
| NM_023500 | 22439 | Xkh | extracellular space;integral to | transport |
| AK005035 | 71775 | | extracellular space | transport |
| NM_019968 | 56795 | Arm1 | Golgi apparatus | misc |
| NM_008744 | 18208 | Ntn1 | extracellular matrix;extracellul | ecm |
| NM_010577 | 16402 | Itga5 | cytoskeleton;extracellular matri | cell adh |
| NM_009467 | 22238 | Ugt2b5 | integral to membrane;microsome | lipid metabolism |
| AK016083 | 75311 | | | unknown |
| AK010310 | 67755 | Ddx47 | cytoplasm | misc |
| NM_009707 | 11856 | Arhgap6 | actin filament | signaling |
| NM_007531 | 12034 | Bcap37 | mitochondrion | signaling |
| AK016282 | 75863 | | | unknown |
| NM_008733 | 18175 | Nrap | fascia adherens;striated muscle | ctsk |
| NM_019571 | 56224 | Tm4sf9 | integral to membrane | cell adh |
| NM_011828 | 23908 | Hs2st1 | extracellular space;integral to | metabolism |
| AK019542 | 68259 | | | unknown |
| AK006650 | 73296 | Rhobtb3 | | Signaling |
| NM_026162 | 67448 | Plxdc2 | extracellular space;integral to | signaling |
| NM_007583 | 12300 | Cacng2 | extracellular space;tight juncti | ion channel |
| U53591 | 14112 | Fau-ps3 | | unknown |
| NM_015762 | 50493 | Txnrd1 | cytosol | transport |
| NM_010710 | 16870 | Lhx2 | transcription factor complex | transcription |
| NM_009029 | 19645 | Rb1 | chromatin;transcription factor c | cell cycle |
| NM_026096 | 67334 | | nucleosome;nucleus | unknown |
| BC004831 | 228608 | Smox | membrane | misc |
| NM_019921 | 56697 | Akap10 | mitochondrion | transport? |
| NM_012048 | 27015 | Polk | extrachromosomal circular DNA;nu | cell cycle |
| AF061934 | 50782 | Rgs11 | heterotrimeric G-protein complex | signaling |
| NM_009320 | 21366 | Slc6a6 | integral to plasma membrane | transport |
| D50872 | 19204 | Ptafr | integral to membrane | signaling (receptor) |
| AF224264 | 233908 | Fus | cytoplasm;transcription factor T | unknown |
| AK017578 | 18189 | Nrxn1 | integral to membrane;membrane fr | cell adh |
| AK015929 | 70939 | | | unknown |
| NM_007693 | 12652 | Chga | extracellular space;synaptic ves | signaling (secreted) |
| NM_007377 | 11302 | Aatk | integral to membrane | apoptosis |
| BC003988 | 83486 | Rbm5 | intracellular | tumor related (suppressor) |
| AK009282 | 71897 | | membrane | signaling (receptor) |
| NM_007862 | 13383 | Dlgh1 | basolateral plasma membrane;cyto | misc |
| NM_023178 | 66233 | Dmap1 | nucleus | transcription |
| NM_011025 | 18429 | Oxt | extracellular space | signaling (secreted) |
| AK017836 | 76742 | Snx27 | | unknown |
| AK002637 | 66689 | Btbd5 | actin cytoskeleton | ctsk? |
| BC002231 | 67134 | Nol5a | nucleus | misc |
| NM_013614 | 18263 | Odc | mitochondrial inner membrane | misc |
| AK002704 | 68365 | Rab14 | | signaling |
| NM_013641 | 19216 | Ptger1 | integral to membrane | signaling |
| AK002297 | 71667 | | . | unknown |

BNS  BS

| Access | Locus | Gene |
|---|---|---|
| AB003303 | 20493 | Slc10a1 |
| AB023957 | 170773 | AB023957 |
| AB049641 | 68537 | Mrpl13 |
| AB055154 | 108138 | Xrcc4 |
| AF009011 | 12005 | Axin |
| AF039839 | | |
| AF062567 | 20687 | Sp3 |
| AF071173 | 15204 | Herc2 |
| AF083812 | 17120 | Mad1l1 |
| AF131865 | 16561 | Kif1b |
| AF137031 | 83921 | Tmem2 |
| AF156547 | 54668 | Atp11c |
| AF189771 | 11518 | Add1 |
| AF217002 | 252972 | Tpcn1 |
| AF229644 | 54646 | DXImx48e |
| AF303106 | 110012 | Gtrgeo22 |
| AF334612 | 170761 | Pdzk2 |
| AF343752 | 77053 | Unc84a |
| AF375046 | 93762 | Smarca5 |
| AF411834 | 20620 | Snk |
| AJ292072 | 192160 | Mln51 |
| AK002386 | 109801 | Glo1 |
| AK002416 | 68350 | |
| AK002458 | 68339 | |
| AK002704 | 68365 | Rab14 |
| AK002767 | 76509 | |
| AK003191 | 68427 | Slc39a13 |
| AK003423 | 71781 | Slc16a14 |
| AK003496 | 73833 | |
| AK003581 | 100764 | |
| AK003698 | 74778 | |
| AK003830 | 68523 | |
| AK004074 | 28106 | Il25 |
| AK004110 | 73750 | Whrn |
| AK004194 | 68836 | Mrpl52 |
| AK004206 | 67282 | |
| AK004410 | 52397 | D5Ertd689e |
| AK004679 | 73340 | Nptxr |
| AK004688 | 74126 | |
| AK005096 | 70364 | |
| AK005098 | 68966 | |
| AK005139 | 68262 | Agpat4 |
| AK005182 | 72022 | Slc35f2 |
| AK005386 | 72008 | |
| AK005710 | 67283 | Slc25a19 |
| AK005887 | 69354 | Slc38a4 |
| AK006141 | 75656 | |
| AK006185 | 70373 | |
| AK006257 | 69371 | |
| AK006305 | 71848 | |
| AK006407 | 76420 | |
| AK006487 | 75623 | |
| AK006619 | 73261 | |
| AK006679 | | |
| AK006897 | 73451 | |

Figure 54-1

| | |
|---|---|
| AK006970 | 76719 |
| AK007076 | 73555 |
| AK007293 | 76645 Pkd1l2 |
| AK007346 | 73608 |
| AK007483 | 67169 |
| AK007536 | 66262 Ing5 |
| AK007552 | 56036 Ccnl2 |
| AK007703 | 27410 Abca3 |
| AK007787 | 67887 |
| AK007894 | 75597 |
| AK008083 | 72041 |
| AK008333 | 70257 |
| AK008537 | 67035 |
| AK008793 | 72269 Cda |
| AK008979 | 72383 |
| AK008991 | 70137 |
| AK009134 | 75572 Acyp2 |
| AK009341 | 69179 |
| AK009450 | 71904 |
| AK009454 | 268721 |
| AK009460 | 66053 Ppil2 |
| AK009954 | 67870 |
| AK010079 | 70285 |
| AK010429 | 71956 |
| AK010430 | 69219 Ddah1 |
| AK010452 | 52666 D10Ertd610e |
| AK010511 | 11652 Akt2 |
| AK010545 | 69742 |
| AK010745 | 73689 |
| AK010747 | 73692 |
| AK010827 | 76826 |
| AK010831 | 76773 |
| AK011135 | 77048 |
| AK011396 | 234729 D8Wsu151e |
| AK011557 | 69903 |
| AK011747 | 17237 Mgrn1 |
| AK012129 | 74206 |
| AK012624 | 72621 |
| AK012767 | 109065 |
| AK012812 | 69953 |
| AK012842 | 72686 |
| AK012848 | 72701 |
| AK012944 | 20639 Snrpb2 |
| AK013012 | 72184 |
| AK013056 | 76373 |
| AK013833 | 57259 Tob2 |
| AK013995 | 73078 |
| AK014229 | 73190 |
| AK014309 | 75430 |
| AK014536 | 66696 |
| AK014543 | 70804 |
| AK014549 | 74034 |
| AK014606 | 77043 |
| AK014760 | 75770 |
| AK014772 | 74592 |
| AK014837 | 70852 |

Figure 54-2

| | |
|---|---|
| AK014872 | 70866 Slco6d1 |
| AK014891 | 14479 Usp15 |
| AK015014 | 70948 Wdr20 |
| AK015063 | 75773 |
| AK015098 | 73818 |
| AK015114 | 73928 |
| AK015454 | 67876 |
| AK015598 | 74910 |
| AK015888 | 67171 |
| AK015972 | 75137 |
| AK016135 | 75320 |
| AK016202 | 75304 |
| AK016990 | 74481 |
| AK017014 | 71281 |
| AK017182 | 75963 |
| AK017215 | 67759 |
| AK017320 | 67240 |
| AK017352 | 71421 |
| AK017451 | 71452 |
| AK017597 | 70549 Tln2 |
| AK017726 | 70625 Crsp7 |
| AK017734 | 75712 |
| AK017767 | 66653 Brf2 |
| AK017809 | 70640 |
| AK018191 | 67618 Aasdhppt |
| AK018549 | 26413 Mapk1 |
| AK018646 | 75761 |
| AK018963 | 78639 |
| AK019059 | 59040 Arht1 |
| AK019495 | 77591 Ddx10 |
| AK019809 | 78051 |
| AK019915 | 78271 |
| AK020266 | 78906 |
| AK020300 | 78240 Cst11 |
| AK021021 | 16423 Cd47 |
| AK021208 | 77422 |
| AK021227 | 78531 |
| AL078630 | |
| AY029613 | 70359 Gtpbp3 |
| BC002094 | 12913 Creb3 |
| BC002193 | 110279 Bcr |
| BC003197 | 22123 Psmd3 |
| BC003288 | 28088 D10Wsu52e |
| BC003432 | 110842 Etfa |
| BC004056 | 234463 BC004056 |
| BC005494 | 216157 ORF61 |
| BC005598 | 98221 Ga17 |
| BC005744 | 72144 Slc37a3 |
| BC005755 | 70465 |
| BC005788 | 71994 Cnn3 |
| BC007132 | 20924 Supt5h |
| BC007481 | 140570 Plxnb2 |
| BC008543 | 84113 Ptov1 |
| BC015273 | 110157 Raf1 |
| BC016535 | 68556 Urkl1 |
| BC017138 | 109168 |

Figure 54-3

| | |
|---|---|
| BC017633 | 70615 D10Bur2e |
| BC018324 | 70456 |
| BC018363 | 170755 Sgk3 |
| BC018368 | 116733 Vps4a |
| J02995 | 11350 Abl1 |
| L31395 | 13429 Dnm |
| M81483 | 19056 Ppp3cb |
| NM_007471 | 11820 App |
| NM_007474 | 11833 Aqp8 |
| NM_007480 | 11844 Arf5 |
| NM_007495 | 11899 Astn1 |
| NM_007568 | 12223 Btc |
| NM_007591 | 12317 Calr |
| NM_007637 | 12465 Cct5 |
| NM_007644 | 12492 Scarb2 |
| NM_007653 | 12512 Cd63 |
| NM_007657 | 12527 Cd9 |
| NM_007666 | 12563 Cdh6 |
| NM_007714 | 12750 Clk4 |
| NM_007761 | 12909 Crcp |
| NM_007783 | 12988 Csk |
| NM_007788 | 12995 Csnk2a1 |
| NM_007793 | 13014 Cstb |
| NM_007878 | 13491 Drd4 |
| NM_007924 | 13716 Ell |
| NM_007934 | 13809 Enpep |
| NM_007939 | 13842 Epha8 |
| NM_008040 | 14290 Fpr-rs3 |
| NM_008156 | 14756 Gpld1 |
| NM_008165 | 14799 Gria1 |
| NM_008170 | 14811 Grin2a |
| NM_008244 | 15239 Hgs |
| NM_008338 | 15980 Ifngr2 |
| NM_008344 | 16012 Igfbp6 |
| NM_008361 | 16176 Il1b |
| NM_008382 | 16326 Inhbe |
| NM_008396 | 16398 Itga2 |
| NM_008580 | 26408 Map3k5 |
| NM_008602 | 17344 Miz1 |
| NM_008692 | 18046 Nfyc |
| NM_008696 | 26921 Map4k4 |
| NM_008702 | 18099 Nlk |
| NM_008705 | 18103 Nme2 |
| NM_008741 | 18197 Nsg2 |
| NM_008797 | 18563 Pcx |
| NM_008845 | 18718 Pip5k2a |
| NM_008857 | 18759 Prkcl |
| NM_008885 | 18858 Pmp22 |
| NM_008958 | 19207 Ptch2 |
| NM_009025 | 19414 Rasa3 |
| NM_009030 | 19646 Rbbp4 |
| NM_009042 | 19692 Reg1 |
| NM_009106 | 20166 Rtkn |
| NM_009136 | 20284 Scrg1 |
| NM_009159 | 20384 Sfrs5 |
| NM_009179 | 20444 Siat5 |

Figure 54-4

| | |
|---|---|
| NM_009197 | 20502 Slc16a2 |
| NM_009254 | 20719 Serpinb6a |
| NM_009270 | 20775 Sqle |
| NM_009293 | 20905 Sts |
| NM_009409 | 21974 Top2b |
| NM_009423 | 22032 Traf4 |
| NM_009424 | 22034 Traf6 |
| NM_009440 | 22127 Tsx |
| NM_009510 | 22350 Vil2 |
| NM_009706 | 11855 Arhgap5 |
| NM_009707 | 11856 Arhgap6 |
| NM_009717 | 11922 Neurod6 |
| NM_009773 | 12236 Bub1b |
| NM_009796 | 12339 Capn7 |
| NM_009802 | 12353 Car6 |
| NM_009805 | 12633 Cflar |
| NM_009840 | 12469 Cct8 |
| NM_009886 | 12614 Celsr1 |
| NM_010109 | 13640 Efna5 |
| NM_010133 | 13798 En1 |
| NM_010198 | 14166 Fgf11 |
| NM_010218 | 14221 Fjx1 |
| NM_010347 | 14797 Aes |
| NM_010568 | 16337 Insr |
| NM_010592 | 16478 Jund1 |
| NM_010707 | 16857 Lgals6 |
| NM_010798 | 17319 Mif |
| NM_010941 | 18194 Nsdhl |
| NM_011049 | 18555 Pctk1 |
| NM_011074 | 18647 Pftk1 |
| NM_011116 | 18807 Pld3 |
| NM_011175 | 19141 Lgmn |
| NM_011183 | 19165 Psen2 |
| NM_011244 | 19411 Rarg |
| NM_011251 | 19654 Rbm6 |
| NM_011316 | 20211 Saa4 |
| NM_011336 | 20301 Ccl27 |
| NM_011401 | 20527 Slc2a3 |
| NM_011421 | 20597 Smpd1 |
| NM_011487 | 20849 Stat4 |
| NM_011492 | 20869 Stk11 |
| NM_011522 | 20974 Syngr3 |
| NM_011651 | 22116 Stk22s1 |
| NM_011829 | 23917 Impdh1 |
| NM_011904 | 24087 Tll2 |
| NM_011906 | 24100 Tpra40 |
| NM_011930 | 26373 Clcn7 |
| NM_012017 | 26919 Zfp346 |
| NM_012043 | 26968 Islr |
| NM_013483 | 12231 Btn1a1 |
| NM_013535 | 14790 Grcc10 |
| NM_013589 | 16997 Ltbp2 |
| NM_013614 | 18263 Odc |
| NM_013661 | 20357 Sema5b |
| NM_013672 | 20683 Sp1 |
| NM_013680 | 20964 Syn1 |

Figure 54-5

235

| | | |
|---|---|---|
| NM_013818 | 14904 Gtpbp1 | |
| NM_013884 | 29873 Cspg5 | |
| NM_013908 | 30839 Fbxw5 | |
| NM_015728 | 11416 Slc33a1 | |
| NM_015737 | 14426 Galnt4 | |
| NM_015753 | 24136 Zfhx1b | |
| NM_015795 | 50759 Fbxo16 | |
| NM_015806 | 50772 Mapk6 | |
| NM_016678 | 53614 Reck | |
| NM_016788 | 51789 Tnk2 | |
| NM_016843 | 54138 Sca10 | |
| NM_016863 | 14226 Fkbp1b | |
| NM_016877 | 53621 Cnot4 | |
| NM_016879 | 53622 Krt2-18 | |
| NM_016888 | 53625 B3gnt1 | |
| NM_016894 | 51801 Ramp1 | |
| NM_017395 | 53970 Rfx5 | |
| NM_017478 | 54160 Copg2 | |
| NM_018795 | 27421 Abcc6 | |
| NM_018798 | 54609 Ubqln2 | |
| NM_018821 | 54607 Socs4 | |
| NM_018823 | 54446 Nfat5 | |
| NM_018888 | 56046 | |
| NM_019415 | 20497 Slc12a3 | |
| NM_019473 | 29845 Olfr155 | |
| NM_019718 | 56350 Arl3 | |
| NM_019735 | 56369 Mmrp19 | |
| NM_019768 | 56397 Morf4l2 | |
| NM_019799 | 56315 Rhcg | |
| NM_019942 | 56526 | Sep-06 |
| NM_020256 | 56805 Zbtb33 | |
| NM_020257 | 93675 Dcl1 | |
| NM_020575 | 57438 Axot | |
| NM_020594 | 57432 | |
| NM_021286 | 20370 Sez6 | |
| NM_021307 | 57745 Zfp112 | |
| NM_021371 | 140904 Caln1 | |
| NM_021373 | 58173 AB030188 | |
| NM_021414 | 74340 | |
| NM_021457 | 14362 Fzd1 | |
| NM_021489 | 58992 F12 | |
| NM_021493 | 58996 | |
| NM_021509 | 59012 Moxd1 | |
| NM_021539 | 59043 AA673511 | |
| NM_021550 | 59048 | |
| NM_021568 | 59093 Pcbp3 | |
| NM_021921 | 60597 Mapk8ip2 | |
| NM_022321 | 64099 Parvg | |
| NM_022653 | 50492 Thop1 | |
| NM_023465 | 67087 Catnbip1 | |
| NM_023598 | 71371 Desrt | |
| NM_023773 | 75339 | |
| NM_023868 | 20191 Ryr2 | |
| NM_023871 | 56086 Set | |
| NM_024228 | 68616 | |
| NM_025273 | 13180 Pcbd | |

Figure 54-6

236

| | | |
|---|---|---|
| NM_025303 | 29819 | Stau2 |
| NM_025417 | 66199 | |
| NM_025460 | 66271 | |
| NM_025568 | | |
| NM_025647 | 66588 | |
| NM_025771 | | |
| NM_025963 | 67097 | |
| NM_026154 | 107732 | Mrpl10 |
| NM_026165 | 67453 | |
| NM_026242 | 67568 | |
| NM_026306 | 67674 | |
| NM_026528 | 68045 | |
| NM_026660 | 68294 | |
| NM_026829 | 107885 | Mthfs |
| NM_026911 | 69019 | |
| NM_027157 | 69664 | |
| NM_028104 | 72112 | |
| NM_028756 | 74102 | Slc35a5 |
| NM_029716 | 108699 | Chn1 |
| NM_031367 | 15061 | H28 |
| NM_031879 | | |
| NM_033042 | 85030 | Tnfrsf25 |
| NM_033565 | 93736 | Laf4l |
| NM_052994 | 94214 | Spock2 |
| NM_053117 | 93737 | Pard6g |
| NM_054099 | 117171 | |
| NM_080633 | 11429 | Aco2 |
| U20780 | 217342 | |
| U70139 | 12457 | Ccrn4l |
| X58472 | 16588 | Kin |
| Y12229 | 22288 | Utrn |
| Y12713 | 27282 | Erv4 |
| Y15910 | 54004 | Diap2 |

Figure 54-7

EP 1 649 062 B1

-0.3  -0.1  0.1  0.3

$Log_2(ratio)$

## Figure 55 A

| ACCESS | LOCUS | GENE | LOCALIZATION | BIOLOGICAL FUNCTION |
|--------|-------|------|--------------|---------------------|
| NM_015795 | 50759 | Fbxo16 | | unknown |
| NM_011522 | 20974 | Syngr3 | integral to plasma membrane | unknown |
| AF189771 | 11518 | Add1 | cytoskeleton;membrane | ctsk |
| NM_012017 | 26919 | Zfp346 | nucleolus | apoptosis |
| NM_008857 | 18759 | Prkcl | plasma membrane | signaling |
| AF156547 | 54668 | Atp11c | integral to membrane | transport |
| NM_008396 | 16398 | Itga2 | cytoskeleton;extracellular matri | cell adh - ecm |
| AK003423 | 71781 | Slc16a14 | integral to membrane | transport |
| AF229644 | 54646 | DXImx48e | integral to membrane;microtubule | signaling |
| NM_021539 | 59043 | AA673511 | | signaling |
| NM_008580 | 26408 | Map3k5 | cytoplasm | signaling (MAPK) |
| NM_008165 | 14799 | Gria1 | extracellular space;integral to | signaling (receptor) |
| M81483 | 19056 | Ppp3cb | protein serine-threonine phospha | signaling |

BNS  BS

**Figure 55 B**

Log$_2$(ratio) — -0.3 -0.1 0.1 0.3

| ACCESS | LOCUS | GENE | LOCALIZATION | BIOLOGICAL FUNCTION |
|---|---|---|---|---|
| NM_011421 | 20597 | Smpd1 | integral to membrane;lysosome | metabolism |
| NM_009159 | 20384 | Sfrs5 | spliceosome complex | transcription |
| NM_008845 | 18718 | Pip5k2a | cytoplasm | lipid metabolism |
| AK012767 | 109065 | | | unknown |
| AK003496 | 73833 | | | unknown |
| NM_013908 | 30839 | Fbxw5 | anaphase-promoting complex | protein degradation |
| NM_008797 | 18563 | Pcx | mitochondrion | metabolism |
| NM_007783 | 12988 | Csk | cytoplasm | signaling |
| NM_026154 | 107732 | Mrpl10 | mitochondrial large ribosomal su | misc |
| NM_025303 | 29819 | Stau2 | microtubule associated complex | transport |
| NM_008244 | 15239 | Hgs | endosome | transport |
| AK019809 | 78051 | | | unknown |
| NM_016788 | 51789 | Tnk2 | cytoplasm | signaling |
| NM_009025 | 19414 | Rasa3 | plasma membrane | signaling (GAP) |
| NM_007934 | 13809 | Enpep | integral to membrane;plasma memb | immune response |
| AF071173 | 15204 | Herc2 | | protein degradation |
| BC007481 | 140570 | Ptxnb2 | integral to plasma membrane | Signaling (receptor) |
| NM_011930 | 26373 | Clcn7 | integral to plasma membrane | ion channel |
| AK004206 | 67262 | | | unknown |
| NM_011829 | 23917 | Impdh1 | cytoplasm | metabolism |
| NM_010347 | 14797 | Aes | nucleus | transcription |
| BC005744 | 72144 | Slc37a3 | extracellular space;integral to | transport |
| BC003197 | 22123 | Psmd3 | 26S proteasome;cytosol | protein degradation |
| AK012129 | 74206 | | | unknown |
| AK003698 | 74778 | | membrane | misc |
| AK012842 | 72686 | | | unknown |
| NM_017478 | 54160 | Copg2 | Golgi apparatus;membrane;viral c | transport |
| BC005494 | 216157 | ORF61 | | unknown |
| NM_019415 | 20497 | Slc12a3 | integral to plasma membrane | transport |
| AF343752 | 77053 | Unc84a | cytoplasm;integral to membrane;n | unknown |
| AK010429 | 71956 | | cytoplasm | unknown |
| NM_016879 | 53622 | Krt2-18 | intermediate filament | ctsk |
| AK010452 | 52666 | D10Ertd610e | plasma membrane | tumor related |
| NM_007761 | 12909 | Crcp | | signaling (receptor) |
| NM_007471 | 11820 | App | Golgi apparatus;coated pit;endop | cell adh |
| AK017014 | 71281 | | | unknown |
| NM_011492 | 20869 | Stk11 | microtubule cytoskeleton | Signaling |
| AK004074 | 28106 | Il25 | extracellular space | growth factor |
| NM_080633 | 11429 | Aco2 | mitochondrion | metabolism |
| AK019495 | 77591 | Ddx10 | | misc |
| AK009954 | 67870 | | extracellular | unknown |
| BC005788 | 71994 | Cnn3 | cytoskeleton | ctsk ? |
| NM_016863 | 14226 | Fkbp1b | | signaling |
| AK016202 | 75304 | | | unknown |
| NM_022653 | 50492 | Thop1 | mitochondrion | protein degradation |
| AK014872 | 70856 | Slco6d1 | integral to membrane | transport |
| AK016990 | 74481 | | nucleus | transcription |
| AK010511 | 11652 | Akt2 | intracellular;protein kinase CK2 | cell cycle |
| AK007703 | 27410 | Abca3 | integral to plasma membrane | misc |
| NM_008692 | 18046 | Nfyc | transcription factor complex | transcription |
| NM_031879 | | | | unknown |
| NM_011906 | 24100 | Tpra40 | integral to membrane | signaling (receptor) |
| NM_007666 | 12563 | Cdh6 | extracellular space;integral to | cell adh |
| BC008543 | 84113 | Ptov1 | | tumor related |
| NM_008344 | 16012 | Igfbp6 | extracellular space | growth factor |
| AK019059 | 59040 | Arht1 | integral to membrane | signaling |
| AK011747 | 17237 | Mgrn1 | extracellular | apoptosis |
| AK004688 | 74126 | | endoplasmic reticulum;integral t | tumor related (receptor) |
| NM_021371 | 140904 | Caln1 | | signaling |
| AK005386 | 72008 | | endosome | transport |
| NM_011487 | 20849 | Stat4 | cytoplasm;nucleus | transcription |
| NM_010218 | 14221 | Fjx1 | extracellular space | unknown |
| NM_009136 | 20284 | Scrg1 | extracellular space | misc |

BNS BS

Figure 55 C

Log$_2$(ratio)
-0.3  -0.1  0.1  0.3

| ACCESS | LOCUS | GENE | LOCALIZATION | BIOLOGICAL FUNCTION |
|---|---|---|---|---|
| AF375046 | 93762 | Smarca5 | nucleus | misc |
| NM_009424 | 22034 | Traf6 | | protein degradation |
| AK008537 | 67035 | | cytoplasm | translation |
| NM_009510 | 22350 | Vil2 | cytoskeleton;microvillus | ctsk |
| NM_013589 | 16997 | Ltbp2 | extracellular matrix | ecm |
| NM_008338 | 15980 | Ifngr2 | extracellular space;integral to | immune response (receptor) |
| NM_009805 | 12633 | Cflar | | apoptosis |
| NM_025647 | 66588 | | cytoplasm;extracellular space;nu | unknown |
| NM_016894 | 51801 | Ramp1 | extracellular space;integral to | signaling |
| AK005182 | 72022 | Slc35f2 | integral to membrane | transport |
| NM_013535 | 14790 | Grcc10 | | unknown |
| AK010831 | 76773 | | | unknown |
| AF334612 | 170761 | Pdzk2 | actin cytoskeleton | signaling |
| NM_019718 | 56350 | Arl3 | Golgi apparatus | signaling |
| NM_013818 | 14904 | Gtpbp1 | membrane | immune response |
| AL078630 | | | | unknown |
| AK014837 | 70852 | | | unknown |
| AK014772 | 74592 | | | unknown |
| AK014549 | 74034 | | | unknown |
| AK004410 | 52397 | D5Ertd689e | | unknown |
| AK017767 | 66653 | Brf2 | transcription factor complex | transcription |
| NM_021550 | 59048 | | integral to membrane | unknown |
| NM_008382 | 16326 | Inhbe | extracellular space | growth factor (secreted) |
| NM_026911 | 69019 | | integral to endoplasmic reticulu | protein degradation |
| NM_009840 | 12469 | Cct8 | cytosol | translation |
| NM_019799 | 56315 | Rhcg | integral to plasma membrane | transport |
| NM_013483 | 12231 | Btnla1 | extracellular space;integral to | immune response (receptor) |
| NM_007474 | 11833 | Aqp8 | integral to plasma membrane;mito | transport |
| NM_009409 | 21974 | Top2b | chromosome;metaphase chromosome; | misc |
| NM_011336 | 20301 | Ccl27 | extracellular space;nucleus | immune response (secreted) |
| AK012812 | 69953 | | | unknown |
| NM_007714 | 12750 | Clk4 | cytoplasm;nucleus | signaling |
| AK005098 | 68966 | | | unknown |
| AK012624 | 72621 | | plasma membrane | signaling |
| NM_009717 | 11922 | Neurod6 | nucleus | transcription |
| AK017215 | 67759 | | | unknown |
| NM_009796 | 12339 | Capn7 | nucleus | protein degradation |
| NM_011251 | 19654 | Rbm6 | spliceosome complex | misc |
| BC005598 | 98221 | Ga17 | signalosome complex | protein degradation |
| BC017138 | 109168 | | | immune response ? |
| NM_013661 | 20357 | Sema5b | extracellular space;integral to | signaling (receptor) |
| AK021208 | 77422 | | | unknown |
| AK008333 | 70257 | | mitochondrion | unknown |
| NM_020575 | 57438 | Axot | ribosome | unknown |
| AK005887 | 69354 | Slc38a4 | integral to membrane | transport |
| AK012944 | 20639 | Snrpb2 | snRNP U2;viral nucleocapsid | transcription |
| AK006679 | | | | unknown |
| NM_015737 | 14426 | Galnt4 | Golgi apparatus;extracellular sp | misc |
| AK015098 | 73818 | | | unknown |
| AK007293 | 76645 | Pkd1l2 | | unknown |
| NM_010198 | 14166 | Fgf11 | extracellular space | growth factor |
| AK008979 | 72383 | | | unknown |
| NM_025460 | 66271 | | integral to membrane | unknown |
| AK010827 | 76826 | | extracellular space | unknown |
| AK007787 | 67887 | | integral to membrane | unknown |
| Y15910 | 54004 | Diap2 | | misc |
| AK010079 | 70285 | | | transcription ? |
| AK015888 | 67171 | | ribosome | unknown |
| AK015454 | 67876 | | mitochondrion | transcription |
| AK011135 | 77048 | | cytoskeleton | transcription |
| X58472 | 16588 | Kin | | misc |
| NM_031367 | 15061 | H28 | cytoplasm | immune response |
| AK006185 | 70373 | | | unknown |

BNS  BS

Figure 56 A

Log$_2$(ratio)  -0.2  0.0  0.2

| ACCESS | LOCUS | GENE | LOCALIZATION | BIOLOGICAL FUNCTION |
|--------|-------|------|--------------|---------------------|
| NM_008361 | 16176 | Il1b | extracellular | immune response |
| AK020300 | 78240 | Cst11 | extracellular space | ecm |
| NM_007495 | 11899 | Astn1 | integral to membrane | misc |
| NM_018821 | 54607 | Socs4 | | signaling |
| AK014760 | 75770 | | | unknown |
| Y12713 | 27282 | Erv4 | | virus |
| AF009011 | 12005 | Axin | cytoplasmic vesicle | signaling |
| NM_021493 | 58996 | | membrane | unknown |
| NM_011401 | 20527 | Slc2a3 | extracellular space;integral to | transport |
| NM_021414 | 74340 | | | unknown |
| NM_023465 | 67087 | Catnbip1 | cytoplasm;nucleus | signaling |
| NM_009886 | 12614 | Celsr1 | extracellular space;integral to | cell adh (receptor) |
| AK016135 | 75320 | | | signaling |
| NM_020256 | 56805 | Zbtb33 | transcription factor complex | transcription |
| J02995 | 11350 | Abl1 | cytoplasm;nucleus | tumor related |
| NM_011049 | 18555 | Pctk1 | cytoplasm | misc |
| AK006970 | 76719 | | | unknown |
| NM_009423 | 22032 | Traf4 | nucleus | apoptosis |
| AK010747 | 73692 | | | unknown |
| AK003581 | 100764 | | integral to membrane | unknown |
| AK009341 | 69179 | | | unknown |
| NM_009179 | 20444 | Siat5 | Golgi apparatus;integral to memb | lipid metabolism |
| NM_008170 | 14811 | Grin2a | extracellular space;integral to | signaling (receptor) |
| NM_028104 | 72112 | | cytoplasm | signaling |
| BC015273 | 110157 | Raf1 | cytosol;plasma membrane | signaling |
| AB023957 | 170773 | AB023957 | | unknown |
| U70139 | 12457 | Ccrn4l | | unknown |
| NM_025771 | | | | unknown |
| NM_026660 | 68294 | | integral to membrane | Transport |
| NM_009030 | 19646 | Rbbp4 | chromatin assembly complex | cell cycle |
| NM_008702 | 18099 | Nlk | nucleus;protein kinase CK2 compl | signaling |
| NM_009197 | 20502 | Slc16a2 | integral to plasma membrane | transport |
| AK014606 | 77043 | | | unknown |
| NM_018823 | 54446 | Nfat5 | cytoplasm;nucleus | transcription |
| NM_008602 | 17344 | Miz1 | nucleus;septin ring | transcription |
| AK017734 | 75712 | | integral to membrane | unknown |
| AK009460 | 66053 | Ppil2 | nucleus | translation |
| NM_015728 | 11416 | Slc33a1 | endoplasmic reticulum membrane;i | transport |
| AK017597 | 70549 | Tln2 | | ctsk |
| NM_018795 | 27421 | Abcc6 | integral to plasma membrane | transport |
| AK007552 | 56036 | Ccnf2 | | cell cycle |
| NM_023868 | 20191 | Ryr2 | integral to plasma membrane | ion channel |
| NM_021921 | 60597 | Mapk8ip2 | cytoplasm | signaling (MAPK) |
| NM_054099 | 117171 | | | unknown |
| BC002193 | 110279 | Bcr | | unknown |
| AK017320 | 67240 | | | unknown |
| NM_009270 | 20775 | Sqle | extracellular space;integral to | lipid metabolism |
| AK015598 | 74910 | | | unknown |
| U20780 | 217342 | | | protein degradation |
| NM_033565 | 93736 | Laf4l | mitochondrion | tumor related |
| AK014549 | 74034 | | | unknown |
| BC004056 | 234463 | BC004056 | integral to membrane | unknown |
| AF303106 | 110012 | Gtrgeo22 | integral to membrane | misc |
| BC007132 | 20924 | Supt5h | | transcription |
| NM_021373 | 58173 | AB030188 | integral to membrane | unknown |
| BC017633 | 70615 | D10Bur2e | | unknown |
| AK008991 | 70137 | | o | unknown |
| NM_011904 | 24087 | Tll2 | extracellular space;membrane | protein degradation (secreted) |
| NM_009293 | 20905 | Sts | extracellular space;integral to | misc |
| NM_007924 | 13716 | Ell | transcription elongation factor | transcription |
| AK004110 | 73750 | Whrn | | unknown |
| NM_010707 | 16857 | Lgals6 | plasma membrane | cell adh |
| NM_016678 | 53614 | Reck | extracellular space;membrane | protein degradation |

BNS  BS

$Log_2(ratio)$

Figure 56 B

| ACCESS | LOCUS | GENE | LOCALIZATION | BIOLOGICAL FUNCTION |
|---|---|---|---|---|
| NM_011316 | 20211 | Saa4 | extracellular space | misc |
| NM_052994 | 94214 | Spock2 | extracellular matrix;extracellul | ecm |
| NM_007480 | 11844 | Arf5 | Golgi apparatus;plasma membrane | transport |
| AK002767 | 76509 | | | signaling |
| NM_024228 | 68616 | | extrachromosomal circular DNA;in | unknown |
| AK017352 | 71421 | | | unknown |
| NM_018888 | 56046 | | cytoplasmic vesicle | unknown |
| AK006679 | | | | unknown |
| AK017809 | 70640 | | | unknown |
| NM_021307 | 57745 | Zfp112 | transcription factor complex | transcription |
| NM_008885 | 18858 | Pmp22 | extracellular space;integral to | cell cycle |
| NM_019473 | 29845 | Olfr155 | integral to plasma membrane | signaling (receptor) |
| AK006257 | 69371 | | | unknown |
| NM_009042 | 19692 | Reg1 | extracellular space | growth factor (secreted) |
| NM_009440 | 22127 | Tsx | | unknown |
| NM_015753 | 24136 | Zfhx1b | transcription factor complex | transcription |
| NM_026528 | 68045 | | | unknown |
| AK010745 | 73689 | | | unknown |
| NM_008040 | 14290 | Fpr-rs3 | integral to membrane | signaling (receptor) |
| AK015014 | 70948 | Wdr20 | extrachromosomal circular DNA | misc |
| AK006897 | 73451 | | intracellular | transcription ? |
| AK013012 | 72184 | | | unknown |
| AK007483 | 67169 | | integral to membrane | signaling |
| AK013833 | 57259 | Tob2 | extracellular | signaling |
| AK006487 | 75623 | | | unknown |
| BC018324 | 70456 | | | unknown |
| AK014309 | 75430 | | | unknown |
| AK002386 | 109801 | Glo1 | | unknown |
| AK018963 | 78639 | | | unknown |
| NM_020594 | 57432 | | membrane;nucleus | unknown |
| NM_016888 | 53625 | B3gnt1 | Golgi apparatus;integral to memb | translation |
| AK013995 | 73078 | | mitochondrion | misc |
| AK014536 | 66696 | | plasma membrane | traffic |
| NM_007637 | 12465 | Cct5 | chaperonin-containing T-complex | translation |
| AK007536 | 66262 | Ing5 | microtubule | transcription |
| NM_009802 | 12353 | Car6 | extracellular space;integral to | metabolism |
| NM_011651 | 22116 | Stk22s1 | | signaling ? |
| AK014543 | 70804 | | | signaling (receptor) |
| AK009134 | 75572 | Acyp2 | extrachromosomal circular DNA | metabolism |
| NM_025273 | 13180 | Pcbd | cytoplasm;nucleus | misc |
| AK012624 | 72621 | | plasma membrane | signaling |
| NM_025568 | | | | unknown |
| AK004194 | 68836 | Mrpl52 | | unknown |
| NM_013614 | 18263 | Odc | mitochondrial inner membrane | misc |
| NM_022321 | 64099 | Parvg | cytoskeleton | cell adh |
| NM_012043 | 26968 | Islr | extracellular space | immune response (receptor) |
| NM_023871 | 56086 | Set | chromatin assembly complex | tumor related |
| NM_025417 | 66199 | | membrane | unknown |
| NM_017478 | 54160 | Copg2 | Golgi apparatus;membrane;viral c | transport |
| NM_007788 | 12995 | Csnk2a1 | plasma membrane;protein kinase C | signaling |
| AK008793 | 72269 | Cda | | misc |
| NM_019942 | 56526 | sep-06 | septin ring | ctsk |
| AK018646 | 75761 | | | apoptosis |
| AK007894 | 75597 | | | unknown |
| NM_023773 | 75339 | | chromatin;nucleus | transcription |
| AF217002 | 252972 | Tpcn1 | integral to membrane | transport |
| NM_017395 | 53970 | Rfx5 | intermediate filament | transcription |
| NM_016843 | 54138 | Sca10 | | unknown |
| NM_007657 | 12527 | Cd9 | integral to membrane;plasma memb | immune response |
| BC002094 | 12913 | Creb3 | extrachromosomal circular DNA;nu | transcription |
| AK008083 | 72041 | | | unknown |
| NM_010568 | 16337 | Insr | integral to plasma membrane | signaling (receptor) |
| AK018549 | 26413 | Mapk1 | cytoplasm;nucleus | signaling (receptor) |
| AK018191 | 67618 | Aasdhppt | extrachromosomal circular DNA | unknown |

Figure 57 A

Log$_2$(ratio)  -0.2  0.0  0.2

| ACCESS | LOCUS | GENE | LOCALIZATION | BIOLOGICAL FUNCTION |
|---|---|---|---|---|
| AK006619 | 73261 | | | unknown |
| AK002458 | 68339 | | myosin | unknown |
| AB055154 | 108138 | Xrcc4 | extracellular;nucleus | cell cycle |
| NM_010109 | 13640 | Efna5 | extracellular space;membrane | signaling |
| NM_009717 | 11922 | Neurod6 | nucleus | transcription |
| AF039839 | | | | unknown |
| NM_008156 | 14756 | Gpld1 | extracellular space;integrin com | cell adh |
| AK020266 | 78906 | | extracellular | unknown |
| NM_033042 | 85030 | Tnfrsf25 | extracellular space;integral to | signaling (receptor) |
| NM_028756 | 74102 | Slc35a5 | integral to membrane | transport |
| AF137031 | 83921 | Tmem2 | | unknown |
| AK021021 | 16423 | Cd47 | integral to plasma membrane | cell adh - signaling (receptor) |
| AK007346 | 73608 | | integral to membrane | unknown |
| NM_021509 | 59012 | Moxd1 | cytoplasm;extracellular space;me | metabolism |
| NM_011074 | 18647 | Pftk1 | nucleus | transport |
| AK003191 | 68427 | Slc39a13 | integral to membrane | transport |
| NM_010592 | 16478 | Jund1 | chromatin;nucleus | cell cycle |
| NM_013680 | 20964 | Syn1 | synapse;synaptic vesicle | transport |
| NM_010133 | 13798 | En1 | transcription factor complex | transcription |
| NM_021286 | 20370 | Sez6 | extracellular space;integral to | immune response ? |
| AK013056 | 76373 | | | Transcription |
| NM_019768 | 56397 | Morf4l2 | chromatin;nucleus | transcription |
| NM_008958 | 19207 | Ptch2 | integral to membrane | signaling (receptor) |
| L31395 | 13429 | Dnm | membrane coat | ctsk |
| BC018368 | 116733 | Vps4a | | transport |
| AK002704 | 68365 | Rab14 | | signaling |
| AF411834 | 20620 | Snk | phosphorylase kinase complex | cell cycle |
| NM_029716 | 108699 | Chn1 | cytoplasm | signaling |
| NM_009254 | 20719 | Serpinb6a | | protein degradation |
| NM_018798 | 54609 | Ubqln2 | endoplasmic reticulum membrane | unknown |
| NM_013672 | 20683 | Sp1 | aspartate carbamoyltransferase c | transcription |
| AK009450 | 71904 | | integral to membrane | unknown |
| NM_013884 | 29873 | Cspg5 | extracellular space;integral to | traffic |
| AK014229 | 73190 | | | unknown |
| AK005710 | 67283 | Slc25a19 | integral to membrane;mitochondri | transport |
| BC005755 | 70465 | | | translation |
| NM_010798 | 17319 | Mif | extracellular space | immune response |
| NM_007653 | 12512 | Cd63 | integral to membrane;lysosome;pl | signaling |
| NM_021568 | 59093 | Pcbp3 | cytoplasm;nucleus;ribonucleoprot | transcription |

BNS  BS

Figure 57 B

| | ACCESS | LOCUS | GENE | LOCALIZATION | BIOLOGICAL FUNCTION |
|---|---|---|---|---|---|
| | NM_008705 | 18103 | Nme2 | microtubule | misc |
| | NM_026829 | 107885 | Mthfs | | metabolism |
| | AK006407 | 76420 | | | unknown |
| | NM_008741 | 18197 | Nsg2 | Golgi apparatus;integral to memb | transport |
| | AB049641 | 68537 | Mrpl13 | mitochondrial large ribosomal su | translation |
| | NM_011183 | 19165 | Psen2 | Golgi apparatus;endoplasmic reti | signaling |
| | BC003288 | 28088 | D10Wsu52e | DNA-directed RNA polymerase II-, | unknown |
| | BC003432 | 110842 | Etfa | electron transfer flavoprotein c | metabolism |
| | AK015014 | 70948 | Wdr20 | extrachromosomal circular DNA | misc |
| | NM_025963 | 67097 | | nucleus | misc |
| | NM_019735 | 56369 | Mmrp19 | extracellular | protein degradation ? |
| | AK003830 | 68523 | | | unknown |
| | NM_021489 | 58992 | F12 | extracellular space | misc (secreted) |
| | NM_026306 | 67674 | | | unknown |
| | NM_007793 | 13014 | Cstb | extracellular | protein degradation |
| | AK017451 | 71452 | | | unknown |
| | AK005096 | 70364 | | integral to membrane | transport |
| | AK015598 | 74910 | | | unknown |
| | AK005139 | 68262 | Agpat4 | integral to membrane | lipid metabolism |
| | NM_010941 | 18194 | Nsdhl | endoplasmic reticulum;integral t | metabolism |
| | AK004679 | 73340 | Nptxr | chromatin;extracellular space;in | transcription |
| | BC016535 | 68556 | Urkl1 | cytoplasm | metabolism |
| | AK010430 | 69219 | Ddah1 | | misc |
| | NM_026242 | 67568 | | | immune response |
| | NM_011244 | 19411 | Rarg | extrachromosomal circular DNA;nu | signaling (receptor) |
| | NM_011116 | 18807 | Pld3 | integral to membrane;membrane fr | metabolism |
| | NM_008696 | 26921 | Map4k4 | | signaling |
| | AK017182 | 75963 | | | unknown |
| | AY029613 | 70359 | Gtpbp3 | extracellular space;membrane | Signaling |
| | AK015114 | 73928 | | | unknown |
| | NM_016877 | 53621 | Cnot4 | membrane | transcription |
| | AK012848 | 72701 | | | unknown |
| | AK006305 | 71848 | | | unknown |
| | NM_027157 | 69664 | | | ctsk |
| | AJ292072 | 192160 | Mln51 | | unknown |
| | AK007076 | 73555 | | | immune response |
| | AK011557 | 69903 | | mitochondrial outer membrane | unknown |
| | AK006141 | 75656 | | | unknown |
| | NM_007939 | 13842 | Epha8 | extracellular space;integral to | signaling (receptor) |
| | NM_053117 | 93737 | Pard6g | tight junction | cell cycle |
| | AK011396 | 234729 | D8Wsu151e | | unknown |
| | BC018363 | 170755 | Sgk3 | cytoplasmic vesicle | apoptosis |
| | AK015063 | 75773 | | | unknown |
| | NM_007591 | 12317 | Calr | endoplasmic reticulum lumen;extr | cell adhesion |
| | NM_009106 | 20166 | Rtkn | intracellular;membrane | signaling |
| | AK019915 | 78271 | | | unknown |
| | NM_011175 | 19141 | Lgmn | extracellular space;lysosome | protein degradation |
| | AF062567 | 20687 | Sp3 | transcription factor complex | transcription |
| | AL078630 | | | | unknown |
| | AF083812 | 17120 | Mad1l1 | | unknown |
| | NM_009706 | 11855 | Arhgap5 | membrane | ctsk - signaling (GAP) |
| | NM_015806 | 50772 | Mapk6 | nucleosome | signaling |
| | NM_007568 | 12223 | Btc | extracellular space;integral to | cell cycle |
| | Y12713 | 27282 | Erv4 | | virus |
| | NM_023598 | 71371 | Desrt | intracellular | transcription |
| | NM_020257 | 93675 | Dcl1 | integral to plasma membrane | cell adh |
| | NM_021457 | 14362 | Fzd1 | integral to membrane | signaling (receptor) |
| | AF131865 | 16561 | Kif1b | kinesin complex;mitochondrion | ctsk |
| | AB003303 | 20493 | Slc10a1 | integral to plasma membrane | transport |
| | AK008083 | 72041 | | | unknown |
| | AK002416 | 68350 | | integral to membrane;lateral pla | transport ? |
| | NM_007878 | 13491 | Drd4 | integral to membrane | signaling (receptor) |
| | AK009454 | 268721 | | | unknown |

BNS
BS

# Fig 58

## DIFFERENTIALLY EXPRESSED GENES (SELECTED)

| Access | Gene | Description | Class | Region |
|---|---|---|---|---|
| NM_011025 | Oxt | oxytocin | signaling (secreted) | Midbrain |
| NM_007693 | Chga | chromogranin A | signaling (secreted) | Midbrain |
| NM_008233 | Hdgfrp2 | hepatoma-derived growth factor, related protein 2 | growth factor | Midbrain |
| AF276974 | | RIKEN cDNA 9230106L14 gene | unknown | Midbrain |
| NM_011177 | Prss18 | protease, serine, 18 | protein degradation | Midbrain |
| NM_008350 | Il11 | interleukin 11 | immune response | Midbrain |
| NM_008920 | Prg2 | proteoglycan 2, bone marrow | growth factor (secreted) | Midbrain |
| NM_008871 | Serpine1 | serine (or cysteine) proteinase inhibitor, clade E, member 1 | protein degradation | Midbrain |
| AF440737 | | RIKEN cDNA 1700006F03 gene | misc (secreted) ? | Midbrain |
| NM_007618 | Serpina6 | serine (or cysteine) proteinase inhibitor, clade A, member 6 | protein degradation | Midbrain |
| NM_009136 | Scrg1 | scrapie responsive gene 1 | misc | Midbrain |
| NM_009732 | Avp | arginine vasopressin | growth factor (secreted) | Hypothalamus |
| BC004020 | Agrn | agrin | signaling (secreted) | Hypothalamus |
| NM_010798 | Mif | macrophage migration inhibitory factor | immune response | Hypothalamus |
| NM_021489 | F12 | coagulation factor XII (Hageman factor) | misc (secreted) | Hypothalamus |
| NM_009255 | Serpine2 | serine (or cysteine) proteinase inhibitor, clade E, member 2 | protein degradation (secreted) | Cortex |
| NM_007728 | Coch | coagulation factor C homolog (Limulus polyphemus) | misc | Cortex |
| AF342737 | Bbs2 | Bardet-Biedl syndrome 2 homolog (human) | misc (secreted) ? | Cortex |
| NM_013754 | Insl6 | insulin-like 6 | growth factor | Cortex |

# Figure 59 (page in 3)

## DRUG TARGETING CANDIDATES

| Access | Gene | Description | Class | Region |
|--------|------|-------------|-------|--------|
| AK009282 | | RIKEN cDNA 2310010M24 gene | signaling (receptor) | Midbrain |
| NM_007377 | Aatk | apoptosis-associated tyrosine kinase | apoptosis | Midbrain |
| AK017578 | Nrxn1 | neurexin I | cell adh | Midbrain |
| D50872 | Ptafr | platelet-activating factor receptor | signaling (receptor) | Midbrain |
| NM_009320 | Slc6a6 | solute carrier family 6 (neurotransmitter transporter, taurine), member 6 | transport | Midbrain |
| NM_019571 | Tm4sf9 | transmembrane 4 superfamily member 9 | cell adh | Midbrain |
| AK006211 | Samd8 | sterile alpha motif domain containing 8 | signaling | Midbrain |
| U70033 | Slc8a1 | solute carrier family 8 (sodium-calcium exchanger), member 1 | transport | Midbrain |
| NM_011990 | Slc7a11 | solute carrier family 7 (cationic amino acid transporter, y+ system), member 11 | transport | Midbrain |
| NM_010509 | Ifnar2 | interferon (alpha and beta) receptor 2 | signaling (receptor) | Midbrain |
| AK013727 | Gabra4 | gamma-aminobutyric acid (GABA-A) receptor, subunit alpha 4 | signaling | Midbrain |
| NM_019587 | Plxnb3 | plexin B3 | signaling (receptor) | Midbrain |
| AF140708 | Tcp10c | t-complex protein 10c | signaling (receptor) ? | Midbrain |
| NM_008076 | Gabrr2 | gamma-aminobutyric acid (GABA-C) receptor, subunit rho 2 | signaling (receptor) | Midbrain |
| NM_021530 | Slc4a8 | solute carrier family 4 (anion exchanger), member 8 | transport | Midbrain |
| BC005568 | | RIKEN cDNA 2310056K19 gene | unknown | Midbrain |
| NM_009848 | Entpd1 | ectonucleoside triphosphate diphosphohydrolase 1 | signaling (receptor) | Midbrain |
| AK003571 | Ppfia4 | protein tyrosine phosphatase, receptor type, f polypeptide (PTPRF), interacting protein (liprin), alpha 4 | signaling (receptor) ? | Midbrain |
| NM_013596 | Mc5r | melanocortin 5 receptor | signaling | Midbrain |
| AF282304 | MOR224-12 | olfactory receptor MOR224-12 | signaling (receptor) | Midbrain |
| NM_019959 | C1qtnf1 | C1q and tumor necrosis factor related protein 1 | signaling (receptor) ? | Midbrain |
| AB039933 | Dpl11 | deleted in polyposis 1-like 1 | transport | Midbrain |
| NM_007474 | Aqp8 | aquaporin 8 | transport | Midbrain |
| NM_009846 | Cd24a | CD24a antigen | immune response | Midbrain |
| D83203 | Ptprj | protein tyrosine phosphatase, receptor type, J | signaling | Midbrain |
| NM_021406 | Trem1 | triggering receptor expressed on myeloid cells 1 | signaling (receptor) | Midbrain |
| BC013502 | Tmc6 | transmembrane channel-like gene family 6 | transport | Midbrain |
| NM_018772 | Bri3 | brain protein I3 | transport | Hypothalamus |
| NM_009539 | Zap70 | zeta-chain (TCR) associated protein kinase | immune response (receptor) | Hypothalamus |

Figure 59 (page 2 of 3)

| | | | | |
|---|---|---|---|---|
| NM_007581 | Cacnb3 | calcium channel, voltage-dependent, beta 3 subunit | ion channel | Hypothalamus |
| NM_008072 | Gabrd | gamma-aminobutyric acid (GABA-A) receptor, subunit delta | signaling (receptor) | Hypothalamus |
| NM_008138 | Gnai2 | guanine nucleotide binding protein, alpha inhibiting 2 | signaling (receptor) | Hypothalamus |
| NM_011256 | Rdgb2 | retinal degeneration B2 homolog (Drosophila) | transport | Hypothalamus |
| NM_010254 | Galr2 | galanin receptor 2 | signaling (receptor) | Hypothalamus |
| NM_008226 | Hcn2 | hyperpolarization-activated, cyclic nucleotide-gated K+ 2 | ion channel | Hypothalamus |
| BC002298 | Ptprz1 | protein tyrosine phosphatase, receptor type Z, polypeptide 1 | signaling (receptor) | Hypothalamus |
| NM_008990 | Pvrl2 | poliovirus receptor-related 2 | cell adh | Hypothalamus |
| X17647 | Ntrk2 | neurotrophic tyrosine kinase, receptor, type 2 | signaling (receptor) | Hypothalamus |
| NM_021332 | Glp1r | glucagon-like peptide 1 receptor | signaling (receptor) | Hypothalamus |
| NM_011334 | Clcn4-2 | chloride channel 4-2 | ion channel | Hypothalamus |
| NM_019701 | Clcnk1l | chloride channel K1-like | ion channel | Hypothalamus |
| NM_010609 | Kcnk7 | potassium channel, subfamily K, member 7 | ion channel | Hypothalamus |
| NM_022992 | Arl6ip5 | ADP-ribosylation factor-like 6 interacting protein 5 | transport | Hypothalamus |
| BC003247 | Mclc | Mid-1-related chloride channel 1 | transport | Hypothalamus |
| NM_011126 | Plunc | palate, lung, and nasal epithelium carcinoma associated | tumor related | Hypothalamus |
| AF201662 | Mc4r | melanocortin 4 receptor | signaling (receptor) | Hypothalamus |
| NM_011012 | Oprl | opioid receptor-like | signaling (receptor) | Hypothalamus |
| NM_018733 | Scn1a | sodium channel, voltage-gated, type I, alpha polypeptide | ion cannel | Cortex |
| AK012847 | | RIKEN cDNA 2810031L11 gene | unknown | Cortex |
| AK004559 | Cpne8 | copine VIII | transport | Cortex |
| NM_008358 | Il15ra | interleukin 15 receptor, alpha chain | immune response (receptor) | Cortex |
| NM_009130 | Scg3 | secretogranin III | misc | Cortex |
| NM_026162 | Plxdc2 | plexin domain containing 2 | signaling | Cortex |
| NM_009472 | Unc5c | unc-5 homolog C (C. elegans) | signaling (receptor) | Cortex |
| AF303106 | Gtrgeo22 | gene trap ROSA b-geo 22 | misc | Cortex |
| NM_023805 | Slc38a3 | solute carrier family 38, member 3 | transport | Cortex |
| AK009282 | | RIKEN cDNA 2310010M24 gene | signaling (receptor) | Cortex |
| NM_008761 | Fxyd5 | FXYD domain-containing ion transport regulator 5 | transport | Cortex |
| NM_010934 | Npy1r | neuropeptide Y receptor Y1 | signaling | Cortex |
| NM_019691 | Gria4 | glutamate receptor, ionotropic, AMPA4 (alpha 4) | signaling (receptor) | Cortex |
| NM_008115 | Gfra2 | glial cell line derived neurotrophic factor family receptor alpha 2 | signaling (receptor) | Cortex |
| NM_011522 | Syngr3 | synaptogyrin 3 | unknown | Cortex |
| NM_019659 | Kcnj1 | potassium inwardly-rectifying channel, subfamily J, member 1 | ion cannel | Cortex |
| AK004472 | | RIKEN cDNA 1190003K14 gene | signaling (receptor) | Cortex |
| NM_019836 | | RIKEN cDNA 2610024G14 gene | unknown | Cortex |
| NM_011012 | Oprl | opioid receptor-like | signaling (receptor) | Cortex |

EP 1 649 062 B1

Figure 59 (page 3 ? 3)

| NM_019537 | Dscr2 | Down syndrome critical region homolog 2 (human) | cell cycle | Cortex |
| NM_019631 | | RIKEN cDNA C630002M10 gene | tumor related (receptor) | Cortex |
| U70033 | Slc8a1 | solute carrier family 8 (sodium-calcium exchanger), member 1 | transport | Cortex |
| NM_016719 | Grb14 | growth factor receptor bound protein 14 | signaling (receptor) | Cortex |
| AK013276 | | RIKEN cDNA 1700017I11 gene | signaling (receptor) | Cortex |
| AF059175 | Erbb3 | v-erb-b2 erythroblastic leukemia viral oncogene homolog 3 (avian) | signaling (receptor) | Cortex |
| NM_011643 | Trpc1 | transient receptor potential cation channel, subfamily C, member 1 | signaling (receptor) | Cortex |
| NM_007377 | Aatk | apoptosis-associated tyrosine kinase | apoptosis | Cortex |
| D50060 | Pace4 | paired basic amino acid cleaving system 4 | signaling (receptor) | Cortex |
| AK021021 | Cd47 | CD47 antigen (Rh-related antigen, integrin-associated signal transducer) | cell adh - signaling (receptor) | Cortex |

EP 1 649 062 B1

EP 1 649 062 B1

Figure 60

DIFFERENTIALLY EXPRESSED GENES (SELECTED)

| Access | Gene | Description | Class | Region |
|---|---|---|---|---|
| NM_007793 | Cstb | cystatin B | protein degradation | Midbrain |
| NM_021489 | F12 | coagulation factor XII (Hageman factor) | misc (secreted) | Midbrain |
| NM_019735 | Mmrp19 | monocyte macrophage 19 | protein degradation ? | Midbrain |
| NM_010798 | Mif | macrophage migration inhibitory factor | immune response | Midbrain |
| NM_009042 | Reg1 | regenerating islet-derived 1 | growth factor (secreted) | Hypothalamus |
| NM_011904 | Tll2 | tolloid-like 2 | protein degradation (secreted) | Hypothalamus |
| AK020300 | Cst11 | cystatin 11 | ecm | Hypothalamus |
| NM_008361 | Il1b | interleukin 1 beta | immune response | Hypothalamus |
| NM_010198 | Fgf11 | fibroblast growth factor 11 | growth factor | Cortex |
| NM_011336 | Ccl27 | chemokine (C-C motif) ligand 27 | immune response (secreted) | Cortex |
| NM_008382 | Inhbe | inhibin beta E | growth factor (secreted) | Cortex |
| NM_009136 | Scrg1 | scrapie responsive gene 1 | misc | Cortex |
| AK011747 | Mgrn1 | mahogunin, ring finger 1 | apoptosis | Cortex |
| NM_008344 | Igfbp6 | insulin-like growth factor binding protein 6 | growth factor | Cortex |
| AK004074 | Il25 | interleukin 25 | growth factor | Cortex |

249

## Fig 61  (page 1 of 2)

## DRUG TARGETING CANDIDATES

| Access | Gene | Description | Class | Region |
|---|---|---|---|---|
| NM_007878 | Drd4 | dopamine receptor 4 | signaling (receptor) | Midbrain |
| AK002416 | | RIKEN cDNA 0610009K11 gene | transport ? | Midbrain |
| AB003303 | Slc10a1 | solute carrier family 10 (sodium-bile acid cotransporter family), member 1 | transport | Midbrain |
| NM_021457 | Fzd1 | frizzled homolog 1 (Drosophila) | signaling (receptor) | Midbrain |
| NM_020257 | Dcl1 | C-type lectin 1 | cell adh | Midbrain |
| NM_007568 | Btc | betacellulin, epidermal growth factor family member | cell cycle | Midbrain |
| NM_007939 | Epha8 | Eph receptor A8 | signaling (receptor) | Midbrain |
| AK006305 | | RIKEN cDNA 1700024J04 gene | unknown | Midbrain |
| NM_011244 | Rarg | retinoic acid receptor, gamma | signaling (receptor) | Midbrain |
| AK009450 | | RIKEN cDNA 2310021M12 gene | unknown | Midbrain |
| NM_008958 | Ptch2 | patched homolog 2 | signaling (receptor) | Midbrain |
| NM_021286 | Sez6 | seizure related gene 6 | immune response ? | Midbrain |
| AK021021 | Cd47 | CD47 antigen (Rh-related antigen, integrin-associated signal transducer) | cell adh - signaling (receptor) | Midbrain |
| NM_028756 | Slc35a5 | solute carrier family 35, member A5 | transport | Midbrain |
| NM_033042 | Tnfrsf25 | tumor necrosis factor receptor superfamily, member 25 | signaling (receptor) | Midbrain |
| NM_008156 | Gpld1 | glycosylphosphatidylinositol specific phospholipase D1 | cell adh | Midbrain |
| AK018549 | Mapk1 | mitogen activated protein kinase 1 | signaling (receptor) | Hypothalamus |
| NM_010568 | Insr | insulin receptor | signaling (receptor) | Hypothalamus |
| NM_007657 | Cd9 | CD9 antigen | Immune response | Hypothalamus |
| AF217002 | Tpcn1 | two pore channel 1 | transport | Hypothalamus |
| NM_012043 | Islr | immunoglobulin superfamily containing leucine-rich repeat | immune response (receptor) | Hypothalamus |
| AK014543 | | RIKEN cDNA 4631434O19 gene | signaling (receptor) | Hypothalamus |
| NM_008040 | Fpr-rs3 | formyl peptide receptor, related sequence 3 | signaling (receptor) | Hypothalamus |
| NM_019473 | Olfr155 | olfactory receptor 155 | signaling (receptor) | Hypothalamus |
| NM_010707 | Lgals6 | lectin, galactose binding, soluble 6 | cell adh | Hypothalamus |
| NM_021373 | AB030188 | hypothetical protein, clone 1-53 | unknown | Hypothalamus |
| NM_023868 | Ryr2 | ryanodine receptor 2, cardiac | ion channel | Hypothalamus |
| NM_018795 | Abcc6 | ATP-binding cassette, sub-family C (CFTR-MRP), member 6 | transport | Hypothalamus |

Fig 61 (page 2 of 2)

| | | | | |
|---|---|---|---|---|
| AK017734 | | RIKEN cDNA 5730496E24 gene | unknown | Hypothalamus |
| NM_009197 | Slc16a2 | solute carrier family 16 (monocarboxylic acid transporters), member 2 | transport | Hypothalamus |
| NM_026660 | | RIKEN cDNA 0610009O03 gene | Transport | Hypothalamus |
| NM_008170 | Grin2a | glutamate receptor, ionotropic, NMDA2A (epsilon 1) | signaling (receptor) | Hypothalamus |
| AK003581 | | RIKEN cDNA 1110008J03 gene | unknown | Hypothalamus |
| NM_009886 | Celsr1 | cadherin EGF LAG seven-pass G-type receptor 1 | cell adh (receptor) | Hypothalamus |
| NM_007495 | Astn1 | astrotactin 1 | misc | Hypothalamus |
| AK005887 | Slc38a4 | solute carrier family 38, member 4 | transport | Cortex |
| NM_013661 | Sema5b | sema domain, seven thrombospondin repeats (type 1 and type 1-like), transmembrane domain (TM) and    short cytoplasmic domain, (semaphorin) 5B | signaling (receptor) | Cortex |
| NM_007474 | Aqp8 | aquaporin 8 | transport | Cortex |
| NM_013483 | Btnla1 | butyrophilin, subfamily 1, member A1 | immune response (receptor) | Cortex |
| NM_019799 | Rhcg | Rhesus blood group-associated C glycoprotein | transport | Cortex |
| NM_021550 | | RIKEN cDNA 1500002I11 gene | unknown | Cortex |
| AK005182 | Slc35f2 | solute carrier family 35, member F2 | transport | Cortex |
| NM_008338 | Ifngr2 | interferon gamma receptor 2 | immune response (receptor) | Cortex |
| AK004688 | | RIKEN cDNA 1200010C09 gene | tumor related (receptor) | Cortex |
| NM_011906 | Tpra40 | transmembrane domain protein regulated in adipocytes | signaling (receptor) | Cortex |
| AK014872 | Slco6d1 | solute carrier organic anion transporter family, member 6d1 | transport | Cortex |
| NM_007761 | Crcp | calcitonin gene-related peptide-receptor component protein | signaling (receptor) | Cortex |
| NM_019415 | Slc12a3 | solute carrier family 12, member 3 | transport | Cortex |
| NM_011930 | Clcn7 | chloride channel 7 | ion channel | Cortex |
| BC007481 | Plxnb2 | plexin B2 | Signaling (receptor) | Cortex |
| NM_007934 | Enpep | glutamyl aminopeptidase | immune response | Cortex |
| NM_008165 | Gria1 | glutamate receptor, ionotropic, AMPA1 (alpha 1) | signaling (receptor) | Cortex |
| AK003423 | Slc16a14 | solute carrier family 16 (monocarboxylic acid transporters), member 14 | transport | Cortex |
| AF156547 | Atp11c | Atpase, class VI, type 11C | transport | Cortex |
| NM_011522 | Syngr3 | synaptogyrin 3 | unknown | Cortex |

251

EP 1 649 062 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 60484683 B
- US 60484726 P
- US 4683202 A
- US 5854033 A, Lizardi
- US 5695937 A
- US 5656448 A
- US 4366241 A
- US 4770853 A
- US 6372250 B
- US 5720720 A
- US 5744305 A, Fodor
- US 5270163 A, Gold
- US 5567588 A

### Non-patent literature cited in the description

- **Bas et al.** *Rheumatology (Oxford),* 2002, vol. 41 (7), 809-14
- **Scott.** *Rheumatology (Oxford),* 2000, vol. 39 (1), 24-9
- **Beer et al.** *Nat. Med.,* 2002, vol. 8 (8), 816-24
- **Shipp et al.** *Nat. Med.,* 2002, vol. 8 (1), 68-74
- **Petricoin et al.** *Lancet,* 2002, vol. 359 (9306), 572-7
- **Petricoin et al.** *J. Natl. Cancer Inst.,* 2002, vol. 94 (20), 1576-8
- **Wurmbach et al.** *Methods,* 2003, vol. 31, 306-316
- **Rothwell ; Hopkins.** *Trends Neurosci,* 1995, vol. 18 (3), 130-6
- **Wang et al.** *Nature,* 2003, vol. 421 (6921), 384-8
- **Watkins ; Maier.** *Proc. Natl. Acad. Sci. USA,* 1999, vol. 96 (14), 7710-3
- **Besedovsky ; del Rey.** *Endocr. Rev.,* 1996, vol. 17 (1), 64-102
- **Deinzer et al.** *Int. J. Psychophysiol.,* 2000, vol. 37 (3), 219-32
- **Marshall et al.** *Brain Behav. Immun.,* 1998, vol. 12 (4), 297-307
- **Benschop et al.** *FASEB J.,* 1996, vol. 10 (4), 517-24
- **Sheridan et al.** *Ann. N.Y Acad. Sci.,* 1998, vol. 840, 803-8
- **Cassileth.** *CA Cancer J. Clin.,* 1999, vol. 49 (6), 362-75
- **Stoler et al.** *Proc. Natl. Acad. Sci., USA,* 1999, vol. 96 (26), 15121-6
- **Chernajovsky et al.** *Genes Immun.,* 2000, vol. 1, 295-307
- **Kennedy.** *Med. Hypotheses,* 2000, vol. 54 (5), 723-5
- **Egan et al.** *J. Clin. Invest.,* 2003, vol. 111 (6), 915-24
- **Wilson.** *Curr. Opin. Pulm. Med.,* 2002, vol. 8 (1), 25-32
- **Maes et al.** *Behav. Genet.,* 1997, vol. 27, 325-351
- **Allison et al.** *Int. J. Obes. Relat. Metab. Disord.,* 1996, vol. 20, 501-506
- **Bouchard et al.** *N. Engl. J. Med.,* 1990, vol. 322, 1477-1482
- **Schaffhauser et al.** *Obes. Res.,* 2002, vol. 10, 1188-1196
- **West et al.** *Am. J. Physiol.,* 1995, vol. 268, R658-R665
- **Prpic et al.** *Endocrinology,* 2003, vol. 144, 1155-1163
- **Spiegelman ; Flier.** *Cell,* 2001, vol. 104, 531-43
- **Friedman ; Halaas.** *Nature,* 1998, vol. 395, 763-70
- **Murakami et al.** *J. Endocrinol.,* 2002, vol. 174, 283-288
- **Sheng ; Moran.** *Neuropeptides,* 2002, vol. 36, 171-181
- **Widdowson et al.** *Peptides,* 1999, vol. 20, 367-372
- **Zarjevski et al.** *Endocrinology,* 1993, vol. 133, 1753-1758
- **Hileman et al.** *Endocrinology,* 2002, vol. 143, 775-783
- **Cowley et al.** *Nature,* 2001, vol. 411, 480-484
- **Fan et al.** *Nature,* 1997, vol. 385, 165-168
- **Klein et al.** *Nat. Biotechnol.,* 2002, vol. 20 (4), 387-92
- **Barany.** *Proc. Natl. Acad. Sci. USA,* 1991, vol. 88, 189-193
- **Guatelli et al.** *Proc. Natl. Acad. Sci. USA,* 1990, vol. 87, 1874-1878
- **Kwoh et al.** *Proc. Natl. Acad. Sci. USA,* 1989, vol. 86, 1173-1177

- **Lizardi et al.** *BioTechnology,* 1988, vol. 6, 1197
- **Harlow et al.** Using Antibodies: A Laboratory Manual: Portable Protocol I. Cold Spring Harbor Laboratory, 01 December 1998
- **Zola.** Monoclonal Antibodies: Preparation and Use of Monoclonal Antibodies and Engineered Antibody Derivatives. Springer Verlag, 15 December 2000
- **Shi et al.** *Proc. Natl. Acad. Sci. USA,* 2000, vol. 97 (26), 14709-14
- **Lee et al.** *J. Nucl. Med.,* 2002, vol. 43 (7), 948-56
- **Tracey.** *Nature,* 2002, vol. 420, 853-9
- **Besedovsky ; del Rey.** *Endocrine Reviews,* 1996, vol. 17, 1-39
- **Cserr et al.** *Brain Pathol.,* 1992, vol. 2 (4), 269-76
- **Naderi ; Safarinejad.** *Clin. Endocrinol.,* 2003, vol. 58 (2), 177-84
- Molecular Cloning: A Laboratory Manual. Cold Spring Harbor Laboratory Press, 1989
- **DeRisi et al.** *Nature Genetics,* 1996, vol. 14, 457-460
- **Khan et al.** *Electrophoresis,* 1999, vol. 20, 223-229
- **Lockhart et al.** *Nature Biotechnol.,* 1996, vol. 14, 1675-1680
- **Fitzwater et al.** A SELEX Primer. *Methods in Enzymology,* 1996, vol. 267, 275-301
- **Ellington ; Szostak.** In Vitro Selection of RNA Molecules that Bind Specific Ligands. *Nature,* vol. 346, 818-22
- **Conrad et al.** In Vitro Selection of Nucleic Acid Aptamers That Bind Proteins. *Methods in Enzymology,* 1996, vol. 267, 336-83
- **Ciesiolka et al.** Affinity Selection-Amplification from Randomized Ribooligonucleotide Pools. *Methods in Enzymology,* 1996, vol. 267, 315-35
- **Gold et al.** Diversity of Oligonucleotide Functions. *Annual Review of Biochemistry,* 1995, vol. 64, 763-97
- **Griffiths et al.** *EMBO J.,* 1994, vol. 13, 3245-60
- **Pagratis et al.** Potent 2'-amino and 2' fluoro 2'deoxyribonucleotide RNA inhibitors of keratinocyte growth factor. *Nature Biotechnology,* vol. 15, 68-73
- **Lin et al.** Modified RNA sequence pools for in vitro selection. *Nucleic Acids Research,* 1994, vol. 22, 5229-34
- **Ross et al.** *Nature Genetics,* 2000, vol. 24, 227-235
- **Paweletz et al.** *Dis. Markers,* 2001, vol. 17 (4), 301-7
- **Jelinek et al.** *Mol. Cancer Res.,* 2003, vol. 1, 346-61
- The Genetic Basis of Human Cancer. McGraw-Hill Professional, 2002
- **Vaickus, L.** *Crit Rev. in Oncol./Hemotol.,* 1991, vol. 11, 267-97
- **Williams et al.** *Proc. Natl. Acad. Sci. (USA),* 1992, vol. 89, 9784-9788
- **Williams et al.** *Eur. J. Immunolo.,* 1995, vol. 25, 763-769
- **Blond et al.** *Brain Res.,* 2002, vol. 958 (1), 89-99
- **Suk et al.** *Immunol. Lett.,* 2001, vol. 77 (2), 79-85
- **Losy et al.** *Acta Neurol. Scand.,* 2001, vol. 104 (3), 171-3
- **Opp et al.** *Neuroendocrinology,* 2001, vol. 73 (4), 272-84
- **Chesnokova et al.** *Endocrinology,* 2002, vol. 143 (5), 1571-4
- **Bousquet et al.** *Mol. Endocrinol.,* 2002, vol. 15 (11), 1880-90
- **Polentarutti et al.** *J. Neuroimmunol.,* 2000, vol. 106 (1-2), 87-94
- **Bayas et al.** *Neurosci. Lett.,* 2003, vol. 335 (3), 155-8
- **Xu et al.** *Acta Pharmacol. Sin.,* 2000, vol. 21 (7), 600-4
- **Fang et al.** *Neuroreport,* 2000, vol. 11 (4), 737-41
- **Isenberg-Feig et al.** *Curr. Allergy Asthma Rep.,* 2003, vol. 3 (1), 70-8
- **Komai et al.** *Br. J. Pharmacol.,* 2003, vol. 138 (5), 912-20
- **Blotta et al.** *J. Immunol.,* 1997, vol. 158, 5589-5595
- **Elenkov et al.** *Proc. Assoc. Am. Physicians,* 1996, vol. 108, 374-381
- **Cooper et al.** Biochemical Basis of Neuropharmacology. Oxford University Press, 1996, 123
- **Link et al.** *J. Immunol.,* 1999, vol. 164, 436-442
- **Loizzo et al.** *Br. J. Pharmacol.,* 2002, vol. 135 (5), 1219-26
- **Lowman et al.** *British Journal of Pharmacology,* 1988, vol. 95, 121-130
- **Elenkov et al.** *Annals of the New York Academy of Sciences,* 2000, vol. 917, 94-105
- **Cheta.** *J. Pediatr. Endocrinol. Metab.,* 1998, vol. 11 (1), 11-9
- **Adris et al.** *Cancer Res.,* 2000, vol. 60 (23), 6696-703
- **Tran et al.** *Nucleic Acids Res.,* 2002, vol. 30 (12), e54
- **Fang et al.** *Nucleic Acid Res.,* vol. 31 (16), e96
- **Pavlidis P.** *Methods,* 2003, vol. 31, 282-289
- **Yeung et al.** *Bioinformatics,* 2001, vol. 17 (4), 309-18
- **Hartigan.** Clustering Algorithms. Wiley, 1975
- **Ramakers.** *Neurosci. Lett.,* 2003, vol. 339, 62-66
- **Rajeevan.** *Methods,* 2001, vol. 25 (4), 443-51

- **Vandesompele.** *Genome Biol.,* 2002, vol. 3 (7
- **Brazma et al.** *FEBS Lett,* 2000, vol. 480, 17-24
- **Gibson UE et al.** *Genome Res,* 1996, vol. 6, 995-1001
- **Heid CA et al.** *Genome Res,* 1996, vol. 6, 986-994
- **Mirnics K et al.** *Nature Neurosc,* 2004, vol. 7, 434-439
- **Yamakami M.** *Biol. Pharm.Bull.,* 2004, vol. 27, 564-566
- **Chen B. et al.** *Dev Biol.,* 2002, vol. 252 (2), 170-87
- **Traka M. et al.** *J. Cell Biol.,* 2003, vol. 162 (6), 1161-72